# EUROPEAN PATENT APPLICATION

(11) **EP 3 903 788 A1**
(43) Date of publication of application: **03.11.2021**
(21) Application number: 19903563.5
(22) Date of filing: 24.12.2019
(51) Int. Cl.: A61K 31/529, A61K 31/5377, A61K 31/5383, A61P 31/12, C07D 471/22, C07D 513/18, C07D 513/22, C07D 515/22, C07D 487/18, C07D 487/20, C07D 498/18, C07D 498/22

(54) **PYRAZOLO[1,5-A]PYRIMIDINE MACROCYCLIC COMPOUND**

(30) Priority: 27.12.2018 JP 2018244312
(71) Applicant: Taisho Pharmaceutical Co., Ltd., Tokyo 170-8633 (JP)
(72) Inventor: KAWAGUCHI, Takanori, Tokyo 170-8633 (JP); OGATA, Yuya, Tokyo 170-8633 (JP); TANAKA, Nozomi, Tokyo 170-8633 (JP); TAKEUCHI, Tomoki, Tokyo 170-8633 (JP); SASAKI, Toru, Tokyo 170-8633 (JP); TAKAHASHI, Ryo, Tokyo 170-8633 (JP); HATANAKA, Kanako, Tokyo 170-8633 (JP); TSURUTA, Risa, Tokyo 170-8633 (JP); TAMAOKI, Tomokazu, Tokyo 170-8633 (JP)
(74) Representative: Hoffmann Eitle
(86) International application number: PCT/JP2019/050455
(87) International publication number: WO 2020/138015

(57) **Abstract**

The present invention provides a novel compound that has anti-RSV activity and that is useful in the prevention or treatment of an infection in which viruses of the subfamily Pneumovirinae, including respiratory syncytial virus (RSV), are involved, or a pharmaceutically acceptable salt thereof. Specifically, the present invention provides a compound represented by formula (I): 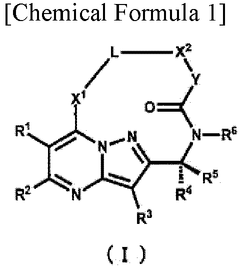 or a pharmaceutically acceptable salt thereof.

## Description

### TECHNICAL FIELD

The present invention relates to a compound that is useful in the prevention or treatment of an infection in which viruses of the subfamily Pneumovirinae are involved, and a pharmaceutically acceptable salt thereof.

### BACKGROUND ART

Viruses of the subfamily Pneumovirinae are negative sense single-stranded RNA viruses included in the family Paramyxoviridae. The viruses of the subfamily Pneumovirinae, including respiratory syncytial virus (RSV), are implicated in a number of epidemic human and animal diseases.

Human respiratory syncytial virus (HRSV) is one of the causes of the most frequent respiratory infections in infants, and severe lower respiratory infections such as bronchiolitis and pneumonia can lead to hospitalization and even death. Especially in high risk groups, such as low birth weight infants and infants with congenital heart or lung diseases, the risk of exacerbation and complications is high.

There are currently no effective therapeutic drugs or vaccines for viral infections of the subfamily Pneumovirinae. Palivizumab, a humanized monoclonal antibody with high neutralizing capacity, has been used as a prophylactic drug to prevent the development of serious lower respiratory infections caused by HRSV in high risk infants, but its drug price is very high. Therefore, an effective therapeutic drug or vaccine has been needed.

In recent years, research and development of RSV F protein inhibitors such as GS-5806, MDT-637, JNJ-2408068, TMC353121, BMS-433771, and BTA-C585 have been conducted aiming at RSV therapeutic drugs. From the analysis of mutant strains generated in the presence of these compounds, it has been known that D486N, E487D, K399I, and T400A are the main common mutant strains (Non Patent Literature 2). Among these mutant strains, multiple clinical isolates of the D486N mutant strain have been reported (Non Patent Literatures 3, 4, and 5), suggesting the possibility that the D486N mutant strain may become prevalent after these agents are marketed.

Recently, compounds that exhibit anti-RSV activity and have a pyrazolo[1,5-a]pyrimidine skeleton are disclosed in Patent Literatures 1, 2, 3, 4, 5, 6, and 7, and Non Patent Literature 1. However, the structure of the compound of the present application is not disclosed in these literatures.

Also, it is disclosed that the activity of GS-5806 against the D486N mutant strain described in Non Patent Literature 1 is reduced by approximately 1000 times compared to the activity against the wild strain (Non Patent Literature 6), but the activity of other compounds is not disclosed.

### CITATION LIST

### PATENT LITERATURE

PTL 1: International Publication No. WO 2011/163518
PTL 2: International Publication No. WO 2013/096681
PTL 3: International Publication No. WO 2013/158776
PTL 4: International Publication No. WO 2016/091774
PTL 5: International Publication No. WO 2016/091791
PTL 6: International Publication No. WO 2016/174079
PTL 7: International Publication No. WO 2016/148145

### NON PATENT LITERATURE

NPL 1: Journal of Medicinal Chemistry vol. 58, p. 1630-1643 (2015)
NPL 2: Biochemical Pharmacology vol. 127, p. 1-12 (2017)
NPL 3: Emerging Infectious disease vol. 18, p. 120-124 (2012)
NPL 4: https://www.ncbi.nlm.nih.gov/nuccore/JX069801
NPL 5: https://www.ncbi.nlm.nih.gov/nuccore/KP258741
NPL 6: Antimicrobial Agents and Chemotherapy vol. 60, p. 1264-1273

### SUMMARY OF INVENTION

### TECHNICAL PROBLEM

An object of the present invention is to provide a novel compound that is useful in the prevention or treatment of an infection in which viruses of the subfamily Pneumovirinae, including respiratory syncytial virus (RSV), are involved, or a pharmaceutically acceptable salt thereof.

### SOLUTION TO PROBLEM

As a result of diligent investigations, the present inventors have found that a pyrazolo[1,5-a]pyrimidine macrocyclic compound represented by formula (I) below (hereinafter, also referred to as a "present inventive compound") has excellent anti-RSV activity, and based on this finding, the present invention has been completed.

That is, the present invention relates to the following:

### (1) A compound represented by formula (I):

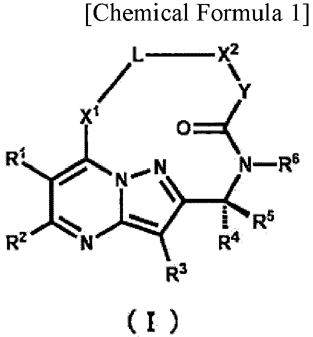

wherein
Y represents phenylene {the phenylene is optionally substituted with one to three substituents selected from the group consisting of a halogen atom, a C₁₋₆ alkyl group (the C₁₋₆ alkyl group is optionally substituted with one to five substituents selected from the group consisting of a deuterium atom, a halogen atom, an amino group, and a hydroxy group), a C₁₋₆ alkoxy group, a C₂₋₆ alkenyl group, a C₁₋₆ alkanoyl group, a C₃₋₆ cycloalkyl group, a cyano group, and a tetramethylguanidyl group}, naphthalenediyl, pyridinediyl, pyridonediyl, thiophenediyl, pyrazolediyl {the naphthalenediyl, the pyridinediyl, the pyridonediyl, the thiophenediyl, and the pyrazolediyl are optionally substituted with one to three substituents selected from the group consisting of a halogen atom, a C₁₋₆ alkyl group (the C₁₋₆ alkyl group is optionally substituted with one to five substituents selected from the group consisting of a deuterium atom, a halogen atom, an amino group, and a hydroxy group), a C₁₋₆ alkoxy group, a C₂₋₆ alkenyl group, a C₁₋₆ alkanoyl group, a C₃₋₆ cycloalkyl group, a cyano group, and a tetramethylguanidyl group}, methylene {the methylene is optionally substituted with one to two substituents selected from the group consisting of a C₁₋₆ alkyl group (the C₁₋₆ alkyl group is optionally substituted with one phenyl group) and a phenyl group (the phenyl group is optionally substituted with one to three substituents selected from the group consisting of a halogen atom and a C₁₋₆ alkyl group)}, indenediyl, or ethylene {the ethylene is optionally substituted with one to three substituents selected from the group consisting of a C₁₋₆ alkyl group (the C₁₋₆ alkyl group is optionally substituted with one phenyl group), a phenyl group (the phenyl group is optionally substituted with one to three substituents selected from the group consisting of a halogen atom and a C₁₋₆ alkyl group), and a pyridyl group, or one methylene in the ethylene is optionally replaced with C₃₋₆ cycloalkanediyl or phenylene}; X¹ represents a single bond or NR^{X11} {R^{X11} represents a hydrogen atom, a C₁₋₆ alkyl group (the C₁₋₆ alkyl group is optionally substituted with one to five substituents selected from the group consisting of a deuterium atom, a hydroxy group, an amino group, and a C₁₋₆ alkoxy group), or a C₃₋₆ cycloalkyl group};
X² represents O, CH₂, or NR^{X21} {R^{X21} represents a hydrogen atom, a C₁₋₆ alkyl group, or a C₁₋₆ alkylsulfonyl group (the C₁₋₆ alkylsulfonyl group is optionally substituted with one to three halogen atoms)};
L represents:
   CHR^{X12}-CH₂-CH₂-NHCO-CH₂, CR^{X12}=CH-CH₂-NHCO-CH₂, or CH₂-NR^{X12}-(CH₂)₃ {R^{X12} represents a hydrogen atom, a C₁₋₆ alkyl group (the C₁₋₆ alkyl group is optionally substituted with one to five substituents selected from the group consisting of a deuterium atom, a hydroxy group, an amino group, and a C₁₋₆ alkoxy group), or a C₃₋₆ cycloalkyl group}, when X¹ is a single bond; and
   C₃₋₆ alkanediyl, C₂₋₄ alkanediyl-CO, C₂₋₄ alkanediyl-SO₂, or Z¹-Z²-Z³, or any of the structures represented by formula group (II) below, when X¹ is NR^{X11};
   Z¹ represents C₁₋₄ alkanediyl (the C₁₋₄ alkanediyl is optionally substituted with one to three substituents selected from the group consisting of a halogen atom and oxo);
   Z² represents O or NR^{Z2} {R^{Z2} represents a hydrogen atom, a C₁₋₆ alkyl group (the C₁₋₆ alkyl group is optionally substituted with one to three substituents selected from the group consisting of a phenyl group, a hydroxy group, and a C₃₋₆ cycloalkyl group), a C₁₋₆ alkylsulfonyl group, a C₁₋₆ alkanoyl group (the C₁₋₆ alkanoyl group is optionally substituted with one amino group), a sulfamoyl group, a C₃₋₆ cycloalkyl group, a pyridyl group, or a C₁₋₆ alkoxycarbonyl group (the C₁₋₆ alkoxycarbonyl group is optionally substituted with one to two phenyl groups)}; and
   Z³ represents C₁₋₄ alkanediyl {the C₁₋₄ alkanediyl is optionally substituted with one to five substituents selected from the group consisting of a halogen atom, oxo, and a hydroxy group, or one methylene in the C₁₋₄ alkanediyl is optionally replaced with C₃₋₆ cycloalkanediyl (in the C₃₋₆ cycloalkanediyl, one methylene in the ring is optionally replaced with an oxygen atom) or sulfonyl}; 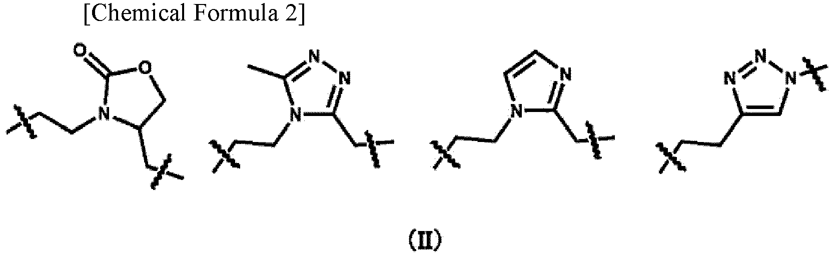
R¹ represents a hydrogen atom, a halogen atom, a C₁₋₆ alkyl group, or a C₃₋₆ cycloalkyl group; R² represents a hydrogen atom, a halogen atom, a cyano group, a C₁₋₆ alkyl group (the
C₁₋₆ alkyl group is optionally substituted with one substituent selected from the group consisting of an amino group, a hydroxy group, and a di-C₁₋₆ alkylamino group), a C₂₋₆ alkenyl group (the C₂₋₆ alkenyl group is optionally substituted with one substituent selected from the group consisting of a hydroxy group and a carboxy group), a C₂₋₆ alkynyl group (the C₂₋₆ alkynyl group is optionally substituted with one substituent selected from the group consisting of a hydroxy group and an amino group), a C₁₋₆ alkoxy group (the C₁₋₆ alkoxy group is optionally substituted with one amino group), a C₁₋₆ alkylsulfanyl group (the C₁₋₆ alkylsulfanyl group is optionally substituted with one amino group), a C₃₋₆ cycloalkyl group {the C₃₋₆ cycloalkyl group is optionally substituted with one substituent selected from the group consisting of a C₁₋₆ alkyl group (the C₁₋₆ alkyl group is optionally substituted with one hydroxy group), an amino group, a hydroxy group, and a carbamoyl group}, a heterocyclyloxy group, a heterocyclylamino group, a C₁₋₆ alkylamino group {the C₁₋₆ alkylamino group is optionally substituted with one to two substituents selected from the group consisting of a hydroxy group, an amino group, a C₁₋₆ alkyl group, and a C₃₋₆ cycloalkyl group (the C₃₋₆ cycloalkyl group is optionally substituted with one amino group)}, a C₃₋₆ cycloalkylamino group (the C₃₋₆ cycloalkylamino group is optionally substituted with one amino group), or a heterocyclyl group {the heterocyclyl group is optionally substituted with one to three substituents selected from the group consisting of a halogen atom, a hydroxy group, an amino group, a C₁₋₆ alkoxy group, a mono-C₁₋₆ alkylamino group, a di-C₁₋₆ alkylamino group, a C₂₋₆ alkanoylamino group, and a C₁₋₆ alkyl group (the C₁₋₆ alkyl group is optionally substituted with one amino group or a hydroxy group)};
R³ represents a hydrogen atom or a halogen atom;
R⁴ and R⁵ are the same or different, and each represent a hydrogen atom or a C₁₋₆ alkyl group; and
R⁶ represents a C₁₋₆ alkyl group {the C₁₋₆ alkyl group is optionally substituted with one to three substituents selected from the group consisting of a halogen atom, a hydroxy group, an amino group, a C₁₋₆ alkoxy group (the C₁₋₆ alkoxy group is optionally substituted with one to three halogen atoms), an aryloxy group, a C₁₋₆ alkylsulfonylamino group, a C₁₋₆ alkylsulfonyl group, a heterocyclyl group (the heterocyclyl group is optionally substituted with one to two oxo), an aryl group, and a heteroaryl group}; or
R⁵ and R⁶ optionally form a 5- to 6-membered cyclic amine optionally containing one oxygen atom in the ring (the cyclic amine is optionally substituted with one to three halogen atoms), together with the adjacent carbon atom and nitrogen atom, or a pharmaceutically acceptable salt thereof;

### (2) The compound according to (1), wherein:

in formula (I) above,
Y represents phenylene {the phenylene is optionally substituted with one to three substituents selected from the group consisting of a halogen atom, a C₁₋₆ alkyl group (the C₁₋₆ alkyl group is optionally substituted with one to five substituents selected from the group consisting of a deuterium atom, a halogen atom, an amino group, and a hydroxy group), a C₁₋₆ alkoxy group, a C₂₋₆ alkenyl group, a C₁₋₆ alkanoyl group, a C₃₋₆ cycloalkyl group, a cyano group, and a tetramethylguanidyl group}, naphthalenediyl, pyridinediyl, pyridonediyl, thiophenediyl, pyrazolediyl {the naphthalenediyl, the pyridinediyl, the pyridonediyl, the thiophenediyl, and the pyrazolediyl are optionally substituted with one to three substituents selected from the group consisting of a halogen atom, a C₁₋₆ alkyl group (the C₁₋₆ alkyl group is optionally substituted with one to five substituents selected from the group consisting of a deuterium atom, a halogen atom, an amino group, and a hydroxy group), a C₁₋₆ alkoxy group, a C₂₋₆ alkenyl group, a C₁₋₆ alkanoyl group, a C₃₋₆ cycloalkyl group, a cyano group, and a tetramethylguanidyl group}, methylene {the methylene is optionally substituted with one to two substituents selected from the group consisting of a C₁₋₆ alkyl group (the C₁₋₆ alkyl group is optionally substituted with one phenyl group) and a phenyl group (the phenyl group is optionally substituted with one to three substituents selected from the group consisting of a halogen atom and a C₁₋₆ alkyl group)}, indenediyl, or ethylene {the ethylene is optionally substituted with one to three substituents selected from the group consisting of a C₁₋₆ alkyl group, a phenyl group (the phenyl group is optionally substituted with one to three halogen atoms), and a pyridyl group, or one methylene in the ethylene is optionally replaced with C₃₋₆ cycloalkanediyl or phenylene}; and
R² represents a hydrogen atom, a halogen atom, a cyano group, a C₁₋₆ alkyl group (the
C₁₋₆ alkyl group is optionally substituted with one substituent selected from the group consisting of an amino group, a hydroxy group, and a di-C₁₋₆ alkylamino group), a C₂₋₆ alkenyl group (the C₂₋₆ alkenyl group is optionally substituted with one substituent selected from the group consisting of a hydroxy group and a carboxy group), a C₂₋₆ alkynyl group (the C₂₋₆ alkynyl group is optionally substituted with one substituent selected from the group consisting of a hydroxy group and an amino group), a C₁₋₆ alkoxy group (the C₁₋₆ alkoxy group is optionally substituted with one amino group), a C₁₋₆ alkylsulfanyl group (the C₁₋₆ alkylsulfanyl group is optionally substituted with one amino group), a C₃₋₆ cycloalkyl group {the C₃₋₆ cycloalkyl group is optionally substituted with one substituent selected from the group consisting of a C₁₋₆ alkyl group (the C₁₋₆ alkyl group is optionally substituted with one hydroxy group), an amino group, a hydroxy group, and a carbamoyl group}, a heterocyclyloxy group, a heterocyclylamino group, a C₁₋₆ alkylamino group {the C₁₋₆ alkylamino group is optionally substituted with one to two substituents selected from the group consisting of an amino group, a C₁₋₆ alkyl group, and a C₃₋₆ cycloalkyl group (the C₃₋₆ cycloalkyl group is optionally substituted with one amino group)}, a
C₃₋₆ cycloalkylamino group (the C₃₋₆ cycloalkylamino group is optionally substituted with one amino group), or a heterocyclyl group {the heterocyclyl group is optionally substituted with one to three substituents selected from the group consisting of a halogen atom, a hydroxy group, an amino group, a mono-C₁₋₆ alkylamino group, a di-C₁₋₆ alkylamino group, and a C₁₋₆ alkyl group (the C₁₋₆ alkyl group is optionally substituted with one amino group)},
or a pharmaceutically acceptable salt thereof;

### (3) The compound according to (1) or (2), wherein:

in formula (I) above,
Y is phenylene {the phenylene is optionally substituted with one to three substituents selected from the group consisting of a halogen atom, a C₁₋₆ alkyl group (the C₁₋₆ alkyl group is optionally substituted with one to five substituents selected from the group consisting of a deuterium atom, a halogen atom, an amino group, and a hydroxy group), a C₁₋₆ alkoxy group, a C₂₋₆ alkenyl group, an acetyl group, a cyclopropyl group, a cyano group, and a tetramethylguanidyl group}, naphthalenediyl, pyridinediyl (the pyridinediyl is optionally substituted with one to three C₁₋₆ alkyl groups), pyridonediyl, thiophenediyl, pyrazolediyl (the pyrazolediyl is optionally substituted with one to three C₁₋₆ alkyl groups), methylene {the methylene is optionally substituted with one to two substituents selected from the group consisting of a C₁₋₆ alkyl group (the C₁₋₆ alkyl group is optionally substituted with one phenyl group) and a phenyl group (the phenyl group is optionally substituted with one to three substituents selected from the group consisting of a halogen atom and a C₁₋₆ alkyl group)}, indenediyl, or ethylene {the ethylene is optionally substituted with one to three substituents selected from the group consisting of a C₁₋₆ alkyl group, a phenyl group (the phenyl group is optionally substituted with one to three halogen atoms), and a pyridyl group, or one methylene in the ethylene is optionally replaced with cyclohexanediyl or phenylene};
X¹ is a single bond or NR^{X11} {R^{X11} represents a hydrogen atom, a C₁₋₆ alkyl group (the
C₁₋₆ alkyl group is optionally substituted with one to five substituents selected from the group consisting of a deuterium atom, a hydroxy group, an amino group, and a C₁₋₆ alkoxy group), or a cyclopropyl group};
Z³ is C₁₋₄ alkanediyl (the C₁₋₄ alkanediyl is optionally substituted with one to five substituents selected from the group consisting of a halogen atom, oxo, and a hydroxy group, or one methylene in the C₁₋₄ alkanediyl is optionally replaced with cyclopropanediyl, oxetanediyl, or sulfonyl);
R⁴ and R⁵ are the same or different, and are each a hydrogen atom or a C₁₋₆ alkyl group; and R⁶ is a C₁₋₆ alkyl group; or
R⁵ and R⁶ optionally form a piperidine ring (the piperidine ring is optionally substituted with one to three halogen atoms) or a morpholine ring, together with the adjacent carbon atom and nitrogen atom,
or a pharmaceutically acceptable salt thereof;

### (4) The compound according to any one of (1) to (3), wherein:

in formula (I) above,
Y is phenylene {the phenylene is optionally substituted with one to three substituents selected from the group consisting of a halogen atom, a C₁₋₆ alkyl group (the C₁₋₆ alkyl group is optionally substituted with one to five substituents selected from the group consisting of a deuterium atom, a halogen atom, an amino group, and a hydroxy group), a C₁₋₆ alkoxy group, a C₂₋₆ alkenyl group, a C₁₋₆ alkanoyl group, a C₃₋₆ cycloalkyl group, a cyano group, and a tetramethylguanidyl group}, naphthalenediyl, pyridinediyl (the pyridinediyl is optionally substituted with one to three C₁₋₆ alkyl groups), pyridonediyl, thiophenediyl, or pyrazolediyl (the pyrazolediyl is optionally substituted with one to three C₁₋₆ alkyl groups),
or a pharmaceutically acceptable salt thereof;

### (5) The compound according to any one of (1) to (4), wherein:

in formula (I) above,
Y is phenylene {the phenylene is optionally substituted with one to three substituents selected from the group consisting of a halogen atom, a C₁₋₆ alkyl group (the C₁₋₆ alkyl group is optionally substituted with one to five substituents selected from the group consisting of a deuterium atom, a halogen atom, an amino group, and a hydroxy group), a C₁₋₆ alkoxy group,
a C₂₋₆ alkenyl group, a C₁₋₆ alkanoyl group, a C₃₋₆ cycloalkyl group, a cyano group, and a tetramethylguanidyl group},
or a pharmaceutically acceptable salt thereof;

### (6) The compound according to any one of (1) to (5), wherein:

in formula (I) above,
X¹ is NR^{X11} {R^{X11} is a C₁₋₆ alkyl group (the C₁₋₆ alkyl group is optionally substituted with one to three deuterium atoms)};
X² is O or NH;
L is (CH₂)₂-NR^{Z2}CO-CH₂, CH₂C(CH₃)₂-NR^{Z2}CO-CH₂, (CH₂)₂-NR^{Z2}CO-CH(CH₃), CH₂-CONR^{Z2}-(CH₂)₂, (CH₂)₃-NR^{Z2}CO-CH₂, (CH₂)₂-NR^{Z2}-(CH₂)₂, CH₂CH(CF₃)-NR^{Z2}(CH₂)₂, (CH₂)₂-NR^{Z2}CH(CF₃)-CH₂, (CH₂)₂-NR^{Z2}-C(-CH₂OCH₂-)CH₂, (CH₂)₄, (CH₂)₅, (CH₂)₂-NR^{Z2}SO₂-CH₂, (CH₂)₂-O-(CH₂)₂, (CH₂)₃-SO₂, or (CH₂)₄-SO₂, or has any of the structural formulas represented by formula (III) below:

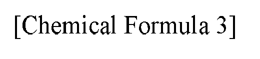

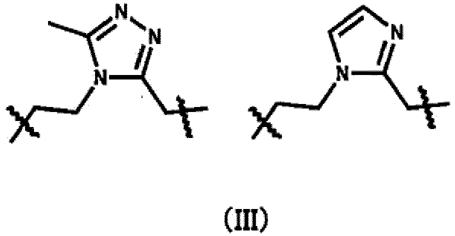

R^{Z2} is a hydrogen atom or a C₁₋₆ alkyl group;
R¹ is a hydrogen atom, a halogen atom, or a C₁₋₆ alkyl group;
R² is a C₁₋₆ alkyl group (the C₁₋₆ alkyl group is optionally substituted with one substituent selected from the group consisting of an amino group, a hydroxy group, and a di-C₁₋₆ alkylamino group), a C₂₋₆ alkenyl group (the C₂₋₆ alkenyl group is optionally substituted with one substituent selected from the group consisting of a hydroxy group and a carboxy group), a C₂₋₆ alkynyl group (the C₂₋₆ alkynyl group is optionally substituted with one substituent selected from the group consisting of a hydroxy group and an amino group), a C₁₋₆ alkoxy group (the C₁₋₆ alkoxy group is optionally substituted with one amino group), a C₃₋₆ cycloalkyl group {the C₃₋₆ cycloalkyl group is optionally substituted with one substituent selected from the group consisting of a C₁₋₆ alkyl group (the C₁₋₆ alkyl group is optionally substituted with one hydroxy group), an amino group, a hydroxy group, and a carbamoyl group}, a C₁₋₆ alkylamino group {the C₁₋₆ alkylamino group is optionally substituted with one to two substituents selected from the group consisting of an amino group, a C₁₋₆ alkyl group, and a C₃₋₆ cycloalkyl group (the C₃₋₆ cycloalkyl group is optionally substituted with one amino group)}, or a heterocyclyl group {the heterocyclyl group is optionally substituted with one to three substituents selected from the group consisting of a halogen atom, a hydroxy group, an amino group, a mono-C₁₋₆ alkylamino group, a di-C₁₋₆ alkylamino group, and a C₁₋₆ alkyl group (the C₁₋₆ alkyl group is optionally substituted with one amino group)};
R³ is a hydrogen atom;
R⁴ is a hydrogen atom;
R⁵ is a C₁₋₆ alkyl group; and
R⁶ is a C₁₋₆ alkyl group; or
R⁵ and R⁶ optionally form a piperidine ring (the piperidine ring is optionally substituted with one to three halogen atoms) or a morpholine ring, together with the adjacent carbon atom and nitrogen atom,
or a pharmaceutically acceptable salt thereof;

### (7) The compound according to any one of (1) to (6), wherein:

in formula (I) above,
X¹ is NR^{X11} {R^{X11} is a C₁₋₆ alkyl group (the C₁₋₆ alkyl group is optionally substituted with one to three deuterium atoms)};
X² is O; and
L is (CH₂)₂-NR^{Z2}CO-CH₂, CH₂C(CH₃)₂-NR^{Z2}CO-CH₂, CH₂-CONR^{Z2}-(CH₂)₂, (CH₂)₃-NR^{Z2}CO-CH₂, (CH₂)₂-NR^{Z2}-(CH₂)₂, (CH₂)₄, (CH₂)₅, (CH₂)₂-NR^{Z2}SO₂-CH₂, (CH₂)₂-NR^{Z2}CH(CF₃)-CH₂, CH₂CH(CF₃)-NR^{Z2}-(CH₂)₂, (CH₂)₂-NR^{Z2}-C(-CH₂OCH₂-)CH₂, or (CH₂)₂-O-(CH₂)₂, or has any of the structural formulas represented by formula (III) below: 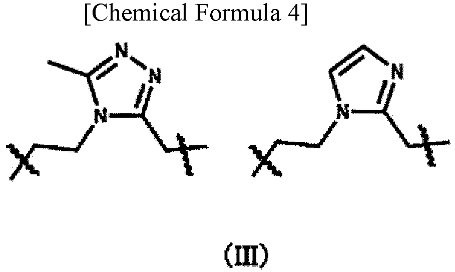 or a pharmaceutically acceptable salt thereof.

### (8) The compound according to any one of (1) to (6), wherein:

in formula (I) above,
X¹ is NR^{X11} {R^{X11} is a C₁₋₆ alkyl group (the C₁₋₆ alkyl group is optionally substituted with one to three deuterium atoms)};
X² is NH; and
L is (CH₂)₃-SO₂ or (CH₂)₄-SO₂,
or a pharmaceutically acceptable salt thereof;

### (9) The compound according to any one of (1) to (5), wherein:

in formula (I) above,
X¹ is a single bond;
X² is O;
L is CH(CH₃)-CH₂-CH₂-NHCO-CH₂ or C(CH₃)=CH-CH₂-NHCO-CH₂;
R¹ is a hydrogen atom, a halogen atom, or a C₁₋₆ alkyl group;
R² is a C₁₋₆ alkyl group (the C₁₋₆ alkyl group is optionally substituted with one substituent selected from the group consisting of an amino group, a hydroxy group, and a di-C₁₋₆ alkylamino group), a C₂₋₆ alkenyl group (the C₂₋₆ alkenyl group is optionally substituted with one substituent selected from the group consisting of a hydroxy group and a carboxy group), a C₂₋₆ alkynyl group (the C₂₋₆ alkynyl group is optionally substituted with one substituent selected from the group consisting of a hydroxy group and an amino group), a C₁₋₆ alkoxy group (the C₁₋₆ alkoxy group is optionally substituted with one amino group), a C₁₋₆ alkylsulfanyl group (the C₁₋₆ alkylsulfanyl group is optionally substituted with one amino group), a C₃₋₆ cycloalkyl group {the C₃₋₆ cycloalkyl group is optionally substituted with one substituent selected from the group consisting of a C₁₋₆ alkyl group (the C₁₋₆ alkyl group is optionally substituted with one hydroxy group), an amino group, a hydroxy group, and a carbamoyl group}, a C₁₋₆ alkylamino group {the C₁₋₆ alkylamino group is optionally substituted with one to two substituents selected from the group consisting of an amino group, a C₁₋₆ alkyl group, and a C₃₋₆ cycloalkyl group (the C₃₋₆ cycloalkyl group is optionally substituted with one amino group)}, or a heterocyclyl group {the heterocyclyl group is optionally substituted with one to three substituents selected from the group consisting of a halogen atom, a hydroxy group, an amino group, a mono-C₁₋₆ alkylamino group, a di-C₁₋₆ alkylamino group, and a C₁₋₆ alkyl group (the C₁₋₆ alkyl group is optionally substituted with one amino group)};
R³ is a hydrogen atom;
R⁴ is a hydrogen atom;
R⁵ is a C₁₋₆ alkyl group; and
R⁶ is a C₁₋₆ alkyl group; or
R⁵ and R⁶ optionally form a piperidine ring (the piperidine ring is optionally substituted with one to three halogen atoms) or a morpholine ring, together with the adjacent carbon atom and nitrogen atom,
or a pharmaceutically acceptable salt thereof;

### (10) The compound according to any one of (1) to (3), wherein:

in formula (I) above,
Y is methylene or ethylene {the methylene and the ethylene are optionally substituted with one to two substituents selected from the group consisting of a C₁₋₆ alkyl group (the C₁₋₆ alkyl group is optionally substituted with one phenyl group) and a phenyl group (the phenyl group is optionally substituted with one to three substituents selected from the group consisting of a halogen atom and a C₁₋₆ alkyl group)},
or a pharmaceutically acceptable salt thereof;

### (11) The compound according to any one of (1) to (10), wherein:

in formula (I) above,
R¹ is a hydrogen atom, a fluorine atom, a chlorine atom, or a methyl group,
or a pharmaceutically acceptable salt thereof;

### (12) The compound according to any one of (1) to (11), wherein:

in formula (I) above,
R² is a cyclopropyl group, a cyclobutyl group (the cyclobutyl group is optionally substituted with one amino group or hydroxyl group), an azetidinyl group (the azetidinyl group is optionally substituted with one amino group or hydroxyl group), a pyrrolidinyl group substituted with one amino group, an aminoethylamino group, or a hydroxyethylamino group,
or a pharmaceutically acceptable salt thereof;

### (13) The compound according to any one of (1) to (12), wherein:

in formula (I) above,
R³ is a hydrogen atom or a fluorine atom,
or a pharmaceutically acceptable salt thereof;

### (14) The compound according to any one of (1) to (13), wherein:

in formula (I) above,
R⁴ is a hydrogen atom; and
R⁵ and R⁶ form a piperidine ring (the piperidine ring is optionally substituted with one to three halogen atoms), together with the adjacent carbon atom and nitrogen atom,
or a pharmaceutically acceptable salt thereof.

(15) The compound according to (1), selected from the group consisting of:
(18aS)-13-[(3S)-3-aminopyrrolidin-1-yl]-2-fluoro-11-methyl-8,9,10,11,19,20,21,22-octahydr o-18aH,24H-18,15-(metheno)pyrido[2,1-l]pyrimido[6,1-h][1,4,7,9,10,13]benzoxapentaazacy clohexadecine-7,24(6H)-dione;
(18aS)-13-(azetidin-1-yl)-2-fluoro-11-methyl-8,9,10,11,19,20,21,22-octahydro-18aH,24H-18 ,15-(metheno)pyrido[2,1-l]pyrimido[6,1-h][1,4,7,9,10,13]benzoxapentaazacyclohexadecine-7 ,24(6H)-dione;
(18aS)-2-fluoro-13-(3-hydroxyazetidin-1-yl)-11-methyl-8,9,10,11,19,20,21,22-octahydro-18a H,24H-18,15-(metheno)pyrido[2,1-l]pyrimido[6,1-h][1,4,7,9,10,13]benzoxapentaazacyclohe xadecine-7,24(6H)-dione;
(18aS)-13-(3-aminoazetidin-1-yl)-2-fluoro-11-methyl-8,9,10,11,19,20,21,22-octahydro-18aH ,24H-18,15-(metheno)pyrido[2,1-l]pyrimido[6,1-h][1,4,7,9,10,13]benzoxapentaazacyclohexa decine-7,24(6H)-dione;
(18aS)-13-(azetidin-1-yl)-2-fluoro-8,11-dimethyl-8,9,10,11,19,20,21,22-octahydro-18aH,24 H-18,15-(metheno)pyrido[2,1-l]pyrimido[6,1-h][1,4,7,9,10,13]benzoxapentaazacyclohexadec ine-7,24(6H)-dione;
(18aS)-13-(3-aminoazetidin-1-yl)-2-fluoro-9,9,11-trimethyl-8,9,10,11,19,20,21,22-octahydro -18aH,24H-18,15-(metheno)pyrido[2,1-l]pyrimido[6,1-h][1,4,7,9,10,13]benzoxapentaazacycl ohexadecine-7,24(6H)-dione;
(18aS)-13-(3-aminoazetidin-1-yl)-2-fluoro-6,11-dimethyl-8,9,10,11,19,20,21,22-octahydro-1 8aH,24H-18,15-(metheno)pyrido[2,1-l]pyrimido[6,1-h][1,4,7,9,10,13]benzoxapentaazacyclo hexadecine-7,24(6H)-dione;
(18aS)-13-(3-aminoazetidin-1-yl)-11-cyclopropyl-2-fluoro-8,9,10,11,19,20,21,22-octahydro-18aH,24H-18,15-(metheno)pyrido[2,1-l]pyrimido[6,1-h][1,4,7,9,10,13]benzoxapentaazacycl ohexadecine-7,24(6H)-dione;
(18aS)-13-(3-aminoazetidin-1-yl)-2-fluoro-11-methyl-9-(trifluoromethyl)-8,9,10,11,19,20,21, 22-octahydro-18aH,24H-18,15-(metheno)pyrido[2,1-l]pyrimido[6,1-h] [1,4,7,9,10,13]benzox apentaazacyclohexadecine-7,24(6H)-dione;
(18aS)-13-(cyclopropylamino)-2-fluoro-11-methyl-8,9,10,11,19,20,21,22-octahydro-18aH,24 H-18,15-(metheno)pyrido[2,1-l]pyrimido[6,1-h][1,4,7,9,10,13]benzoxapentaazacyclohexadec ine-7,24(6H)-dione;
(18aS)-13-{[(1-aminocyclopropyl)methyl]amino}-2-fluoro-11-methyl-8,9,10,11,19,20,21,22-octahydro-18aH,24H-18,15-(metheno)pyrido[2,1-l]pyrimido[6,1-h][1,4,7,9,10,13]benzoxape ntaazacyclohexadecine-7,24(6H)-dione;
(18aS)-2-fluoro-13-methoxy-11-methyl-8,9,10,11,19,20,21,22-octahydro-18aH,24H-18,15-( metheno)pyrido[2,1-l]pyrimido[6,1-h][1,4,7,9,10,13]benzoxapentaazacyclohexadecine-7,24( 6H)-dione;
(18aS)-13-cyclobutyl-2-fluoro-11-methyl-8,9,10,11,19,20,21,22-octahydro-18aH,24H-18,15-(metheno)pyrido[2,1-l]pyrimido[6,1-h][1,4,7,9,10,13]benzoxapentaazacyclohexadecine-7,24( 6H)-dione;
(18aS)-13-(1-aminocyclopropyl)-2-fluoro-11-methyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione;
(18aS)-2-fluoro-13-(3-hydroxyprop-1-yn-1-yl)-11-methyl-8,9,10,11,19,20,21,22-octahydro-1 8aH,24H-18,15-(metheno)pyrido[2,1-l]pyrimido[6,1-h][1,4,7,9,10,13]benzoxapentaazacyclo hexadecine-7,24(6H)-dione;
(18aS)-2-fluoro-13-(3-hydroxypropyl)-11-methyl-8,9,10,11,19,20,21,22-octahydro-18aH,24 H-18,15-(metheno)pyrido[2,1-l]pyrimido[6,1-h][1,4,7,9,10,13]benzoxapentaazacyclohexadec ine-7,24(6H)-dione;
(18aS)-2-fluoro-13-[(1E)-3-hydroxyprop-1-en-1-yl]-11-methyl-8,9,10,11,19,20,21,22-octahy dro-18aH,24H-18,15-(metheno)pyrido[2,1-l]pyrimido[6,1-h][1,4,7,9,10,13]benzoxapentaaza cyclohexadecine-7,24(6H)-dione;
(18aS)-13 -cyclopropyl-2,25-difluoro-11-methyl-8,9,10,11,19,20,21,22-octahydro-18aH,24H-18,15-(metheno)pyrido[2,1-l]pyrimido[6,1-h][1,4,7,9,10,13]benzoxapentaazacyclohexadecin e-7,24(6H)-dione;
(18aS)-13-(azetidin-1-yl)-2-fluoro-11-methyl-7,8,10,11,19,20,21,22-octahydro-18aH,24H-18 ,15-(metheno)pyrido[2,1-l]pyrimido[6,1-h][1,4,7,9,10,13]benzoxapentaazacyclohexadecine-9 ,24(6H)-dione;
(18aS)-13-(azetidin-1-yl)-2-chloro-11-methyl-8,9,10,11,19,20,21,22-octahydro-18aH,24H-18 ,15-(metheno)pyrido[2,1-l]pyrimido[6,1-h][1,4,7,9,10,13]benzoxapentaazacyclohexadecine-7 ,24(6H)-dione;
(18aS)-2-chloro-13 -(3 -hydroxyazetidin-1 -yl)-11 -methyl-8,9,10,11,19,20,21,22-octahydro-18 aH,24H-18,15-(metheno)pyrido[2,1-l]pyrimido[6,1-h][1,4,7,9,10,13]benzoxapentaazacycloh exadecine-7,24(6H)-dione;
(18aS)-13-(3-aminoazetidin-1-yl)-2-chloro-11-methyl-8,9,10,11,19,20,21,22-octahydro-18aH ,24H-18,15-(metheno)pyrido[2,1-l]pyrimido[6,1-h][1,4,7,9,10,13]benzoxapentaazacyclohexa decine-7,24(6H)-dione;
(18aS)-13-(azetidin-1-yl)-2-chloro-8,11-dimethyl-8,9,10,11,19,20,21,22-octahydro-18aH,24 H-18,15-(metheno)pyrido[2,1-l]pyrimido[6,1-h][1,4,7,9,10,13]benzoxapentaazacyclohexadec ine-7,24(6H)-dione;
(18aS)-13-(3-hydroxyazetidin-1-yl)-2,11-dimethyl-8,9,10,11,19,20,21,22-octahydro-18aH,24 H-18,15-(metheno)pyrido[2,1-l]pyrimido[6,1-h][1,4,7,9,10,13]benzoxapentaazacyclohexadec ine-7,24(6H)-dione;
(18aS)-13-(3-aminoazetidin-1-yl)-2,11-dimethyl-8,9,10,11,19,20,21,22-octahydro-18aH,24H -18,15-(metheno)pyrido[2,1-l]pyrimido[6,1-h][1,4,7,9,10,13]benzoxapentaazacyclohexadecin e-7,24(6H)-dione;
(18aS)-13-(azetidin-1-yl)-2,11-dimethyl-8,9,10,11,19,20,21,22-octahydro-18aH,24H-18,15-( metheno)pyrido[2,1-l]pyrimido[6,1-h][1,4,7,9,10,13]benzoxapentaazacyclohexadecine-7,24( 6H)-dione;
(18aS)-13-(azetidin-1-yl)-2-ethyl-11-methyl-8,9,10,11,19,20,21,22-octahydro-18aH,24H-18, 15-(metheno)pyrido[2,1-l]pyrimido[6,1-h][1,4,7,9,10,13]benzoxapentaazacyclohexadecine-7, 24(6H)-dione;
(18aS)-13-(2-aminoethoxy)-2-ethyl-11-methyl-8,9,10,11,19,20,21,22-octahydro-18aH,24H-1 8,15-(metheno)pyrido[2,1-l]pyrimido[6,1-h][1,4,7,9,10,13]benzoxapentaazacyclohexadecine-7,24(6H)-dione;
(18aS)-13-(azetidin-1-yl)-2-methoxy-11-methyl-8,9,10,11,19,20,21,22-octahydro-18aH,24H-18,15-(metheno)pyrido[2,1-l]pyrimido[6,1-h][1,4,7,9,10,13]benzoxapentaazacyclohexadecin e-7,24(6H)-dione;
(18aS)-13-(3-hydroxyazetidin-1-yl)-11-methyl-2-trideuteriomethyl-8,9,10,11,19,20,21,22-oct ahydro-18aH,24H-18,15-(metheno)pyrido[2,1-l]pynmido[6,1-h][1,4,7,9,10,13]benzoxapenta azacyclohexadecine-7,24(6H)-dione;
(19aS)-14-(3-aminoazetidin-1-yl)-2-fluoro-12-methyl-9,10,11,12,20,21,22,23-octahydro-6H, 19aH,25H-19,16-(metheno)pyrido[2,1-m]pyrimido[6,1-i][1,4,8,10,11,14]benzoxapentaazacy cloheptadecine-7,25(8H)-dione;
(18aS)-13-(azetidin-1-yl)-2-fluoro-11,12-dimethyl-8,9,10,11,19,20,21,22-octahydro-18aH,24 H-18,15-(metheno)pyrido[2,1-l]pyrimido[6,1-h][1,4,7,9,10,13]benzoxapentaazacyclohexadec ine-7,24(6H)-dione;
(18aS)-2-fluoro-13-(3-hydroxyazetidin-1-yl)-11,12-dimethyl-8,9,10,11,19,20,21,22-octahydr o-18aH,24H-18,15-(metheno)pyrido[2,1-l]pyrimido[6,1-h][1,4,7,9,10,13]benzoxapentaazacy clohexadecine-7,24(6H)-dione;
(18aS)-13-(3-aminoazetidin-1-yl)-2-fluoro-11,12-dimethyl-8,9,10,11,19,20,21,22-octahydro-18aH,24H-18,15-(metheno)pyrido[2,1-l]pyrimido[6,1-h][1,4,7,9,10,13]benzoxapentaazacycl ohexadecine-7,24(6H)-dione;
(18aS)-13-(2-aminoethoxy)-2-fluoro-11,12-dimethyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione;
(18aS)-13-(azetidin-1-yl)-2-fluoro-8,11,12-trimethyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione;
(18aS)-13-(3-aminoazetidin-1-yl)-2-fluoro-12-methyl-8,9,10,11,19,20,21,22-octahydro-18aH ,24H-18,15-(metheno)pyrido[2,1-l]pynmido[6,1-h][1,4,7,9,10,13]benzoxapentaazacyclohexa decine-7,24(6H)-dione;
(18aS)-13-(3-aminoazetidin-1-yl)-2-chloro-11,12-dimethyl-8,9,10,11,19,20,21,22-octahydro-18aH,24H-18,15-(metheno)pyrido[2,1-l]pyrimido[6,1-h][1,4,7,9,10,13]benzoxapentaazacycl ohexadecine-7,24(6H)-dione;
(18aS)-2-chloro-13-(3-hydroxyazetidin-1-yl)-11,12-dimethyl-8,9,10,11,19,20,21,22-octahydr o-18aH,24H-18,15-(metheno)pyrido[2,1-l]pyrimido[6,1-h][1,4,7,9,10,13]benzoxapentaazacy clohexadecine-7,24(6H)-dione;
(18aS)-13-[(2-aminoethyl)amino]-2-chloro-11,12-dimethyl-8,9,10,11,19,20,21,22-octahydro-18aH,24H-18,15-(metheno)pyrido[2,1-l]pyrimido[6,1-h][1,4,7,9,10,13]benzoxapentaazacycl ohexadecine-7,24(6H)-dione;
(18aS)-13-[(2-aminoethyl)sulfanyl]-2-chloro-11,12-dimethyl-8,9,10,11,19,20,21,22-octahydr o-18aH,24H-18,15-(metheno)pyrido[2,1-l]pyrimido[6,1-h][1,4,7,9,10,13]benzoxapentaazacy clohexadecine-7,24(6H)-dione;
(18aS)-13 -(2-aminoethoxy)-2-chloro-11,12-dimethyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione;
(18aS)-2-chloro-13-(3-hydroxypropyl)-11,12-dimethyl-8,9,10,11,19,20,21,22-octahydro-18a H,24H-18,15-(metheno)pyrido[2,1-l]pyrimido[6,1-h][1,4,7,9,10,13]benzoxapentaazacyclohe xadecine-7,24(6H)-dione;
(18aS)-13-(3-aminopropyl)-2-chloro-11,12-dimethyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione;
(18aS)-13-(2-aminoethyl)-2-chloro-11,12-dimethyl-8,9,10,11,19,20,21,22-octahydro-18aH,2 4H-18,15-(metheno)pyrido[2,1-l]pyrimido[6,1-h][1,4,7,9,10,13]benzoxapentaazacyclohexade cine-7,24(6H)-dione;
(18aS)-13-(4-aminobutyl)-2-chloro-11,12-dimethyl-8,9,10,11,19,20,21,22-octahydro-18aH,2 4H-18,15-(metheno)pyrido[2,1-l]pyrimido[6,1-h][1,4,7,9,10,13]benzoxapentaazacyclohexade cine-7,24(6H)-dione;
(18aS)-13-(3-aminoazetidin-1-yl)-2,11,12-trimethyl-8,9,10,11,19,20,21,22-octahydro-18aH,2 4H-18,15-(metheno)pyrido[2,1-l]pyrimido[6,1-h][1,4,7,9,10,13]benzoxapentaazacyclohexade cine-7,24(6H)-dione;
(18aS)-13-(2-aminoethoxy)-2,11,12-trimethyl-8,9,10,11,19,20,21,22-octahydro-18aH,24H-1 8,15-(metheno)pyrido[2,1-l]pyrimido[6,1-h][1,4,7,9,10,13]benzoxapentaazacyclohexadecine-7,24(6H)-dione;
(18aS)-13-(3-aminoazetidin-1-yl)-2,12-difluoro-11-methyl-8,9,10,11,19,20,21,22-octahydro-18aH,24H-18,15-(metheno)pyrido[2,1-l]pyrimido[6,1-h][1,4,7,9,10,13]benzoxapentaazacycl ohexadecine-7,24(6H)-dione;
(18aS)-2,12-difluoro-13-(3-hydroxyazetidin-1-yl)-11-methyl-8,9,10,11,19,20,21,22-octahydr o-18aH,24H-18,15-(metheno)pyrido[2,1-l]pyrimido[6,1-h][1,4,7,9,10, 13]benzoxapentaazacy clohexadecine-7,24(6H)-dione;
(18aS)-13-(3-aminoazetidin-1-yl)-2-chloro-12-fluoro-11-methyl-8,9,10,11,19,20,21,22-octah ydro-18aH,24H-18,15-(metheno)pyrido[2,1-l]pyrimido[6,1-h][1,4,7,9,10,13]benzoxapentaaz acyclohexadecine-7,24(6H)-dione;
(18aS)-13-(3-aminoazetidin-1-yl)-12-chloro-2-fluoro-11-methyl-8,9,10,11,19,20,21,22-octah ydro-18aH,24H-18,15-(metheno)pyrido[2,1-l]pyrimido[6,1-h][1,4,7,9,10,13]benzoxapentaaz acyclohexadecine-7,24(6H)-dione;
(18aS)-13-cyclopropyl-2-fluoro-11-methyl-8,9,10,11,19,20,21,22-octahydro-18aH,24H-18,1 5-(metheno)pyrido[2,1-l]pyrimido[6,1-h][1,4,7,9,10,13]benzoxapentaazacyclohexadecine-7,2 4(6H)-dione;
(18aS)-13-cyclopropyl-11-ethyl-2-fluoro-8,9,10,11,19,20,21,22-octahydro-18aH,24H-18,15-( metheno)pyrido[2,1-l]pyrimido[6,1-h][1,4,7,9,10, 13]benzoxapentaazacyclohexadecine-7,24( 6H)-dione;
(18aS)-13-cyclopropyl-2-fluoro-11-trideuteriomethyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione;
(18aS)-13-cyclopropyl-2-ethenyl-11-methyl-8,9,10,11,19,20,21,22-octahydro-18aH,24H-18, 15-(metheno)pyrido[2,1-l]pyrimido[6, 1-h][1,4,7,9,10,13]benzoxapentaazacyclohexadecine-7, 24(6H)-dione;
(18aS)-13-cyclopropyl-2-iodo-11-methyl-8,9,10,11,19,20,21,22-octahydro-18aH,24H-18,15-(metheno)pyrido[2,1-l]pyrimido[6,1-h][1,4,7,9,10,13]benzoxapentaazacyclohexadecine-7,24( 6H)-dione;
(18aS)-13-cyclopropyl-11-methyl-7,24-dioxo-6,7,8,9,10,11,19,20,21,22-decahydro-18aH,24 H-18,15-(metheno)pyrido[2,1-l]pyrimido[6,1-h][1,4,7,9,10,13]benzoxapentaazacyclohexadec ine-2-carbonitrile;
(18aS)-2,13-dicyclopropyl-11-methyl-8,9,10,11,19,20,21,22-octahydro-18aH,24H-18,15-(me theno)pyrido[2,1-l]pyrimido[6,1-h] [1,4,7,9,10,13]benzoxapentaazacyclohexadecine-7,24(6H) -dione;
(18aS)-13 -cyclopropyl-11-methyl-2-trideuteriomethyl-8,9,10,11,19,20,21,22-octahydro-18a H,24H-18,15-(metheno)pyrido[2,1-l]pyrimido[6,1-h][1,4,7,9,10,13]benzoxapentaazacyclohe xadecine-7,24(6H)-dione; (18aS)-13-cyclopropyl-2,11-dimethyl-8,9,10,11,19,20,21,22-octahydro-18aH,24H-18,15-(me theno)pyrido[2,1-l]pyrimido[6, 1-h][1,4,7,9,10,13]benzoxapentaazacyclohexadecine-7,24(6H) -dione;
(18aS)-2-chloro-13-cyclopropyl-11-methyl-8,9,10,11,19,20,21,22-octahydro-18aH,24H-18,1 5-(metheno)pyrido[2,1-l]pyrimido[6,1-h][1,4,7,9,10,13]benzoxapentaazacyclohexadecine-7,2 4(6H)-dione;
(18S)-3-cyclopropyl-18-ethyl-14-fluoro-5,17-dimethyl-5,6,7,8,17,18-hexahydro-16H-19,1-( metheno)pyrimido[6,1-h][1,4,7,9,10,13]benzoxapentaazacyclohexadecine-9,16(10H)-dione; (18aS)-13-(trans-3-aminocyclobutyl)-2-fluoro-11-methyl-8,9,10,11,19,20,21,22-octahydro-1 8aH,24H-18,15-(metheno)pyrido[2,1-l]pyrimido[6,1-h][1,4,7,9,10, 13]benzoxapentaazacyclo hexadecine-7,24(6H)-dione;
(18aS)-13-(cis-3-aminocyclobutyl)-2-fluoro-11-methyl-8,9,10,11,19,20,21,22-octahydro-18a H,24H-18,15-(metheno)pyrido[2,1-l]pyrimido[6,1-h][1,4,7,9,10, 13]benzoxapentaazacyclohe xadecine-7,24(6H)-dione;
(18aS)-2-fluoro-13-(cis-3-hydroxycyclobutyl)-11-methyl-8,9,10,11,19,20,21,22-octahydro-1 8aH,24H-18,15-(metheno)pyrido[2,1-l]pyrimido[6,1-h][1,4,7,9,10, 13]benzoxapentaazacyclo hexadecine-7,24(6H)-dione;
(18aS)-2-fluoro-13-(trans-3-hydroxycyclobutyl)-11-methyl-8,9,10,11,19,20,21,22-octahydro-18aH,24H-18,15-(metheno)pyrido[2,1-l]pyrimido[6,1-h][1,4,7,9,10,13]benzoxapentaazacycl ohexadecine-7,24(6H)-dione;
(18aS)-2-chloro-13-(cis-3-hydroxycyclobutyl)-11-methyl-8,9 10,11, 19,20,21,22-octahydro-1 8aH,24H-18,15-(metheno)pyrido[2,1-l]pyrimido[6,1-h][1,4,7,9,10,13]benzoxapentaazacyclo hexadecine-7,24(6H)-dione;
(18aS)-2-chloro-13-(trans-3-hydroxycyclobutyl)-11-methyl-8,9,10,11,19,20,21,22-octahydro -18aH,24H-18,15-(metheno)pyrido[2,1-l]pyrimido[6,1-h][1,4,7,9,10,13]benzoxapentaazacycl ohexadecine-7,24(6H)-dione;
(18aS)-2-chloro-13-(cis-3-hydroxycyclobutyl)-11-trideuteriomethyl-8,9,10,11,19,20,21,22-oc tahydro-18aH,24H-18,15-(metheno)pyrido[2,1-l]pynmido[6,1-h][1,4,7,9,10,13]benzoxapenta azacyclohexadecine-7,24(6H)-dione;
(18aS)-2-chloro-13-(trans-3-hydroxycyclobutyl)-11-trideuteriomethyl-8,9,10,11,19,20,21,22-octahydro- 18aH,24H-18,15-(metheno)pyrido[2,1-l]pyrimido[6,1-h][1,4,7,9,10,13]benzoxape ntaazacyclohexadecine-7,24(6H)-dione;
(18aS)-13-(cis-3-hydroxycyclobutyl)-2,11-dimethyl-8,9,10,11,19,20,21,22-octahydro-18aH,2 4H-18,15-(metheno)pyrido[2,1-l]pyrimido[6,1-h][1,4,7,9,10,13]benzoxapentaazacyclohexade cine-7,24(6H)-dione;
(18aS)-13-(trans-3-hydroxycyclobutyl)-2,11-dimethyl-8,9,10,11,19,20,21,22-octahydro-18aH ,24H-18,15-(metheno)pyrido[2,1-l]pyrimido[6,1-h][1,4,7,9,10,13]benzoxapentaazacyclohexa decine-7,24(6H)-dione;
(18aS)-13-(cis-3-hydroxycyclobutyl)-11-methyl-2-trideuteriomethyl-8,9,10,11,19,20,21,22-o ctahydro-18aH,24H-18,15-(metheno)pyrido[2,1-l]pyrimido[6,1-h][1,4,7,9,10,13]benzoxapent aazacyclohexadecine-7,24(6H)-dione;
(18aS)-13-(trans-3-hydroxycyclobutyl)-11-methyl-2-trideuteriomethyl-8,9,10,11,19,20,21,22 -octahydro-18aH,24H-18,15-(metheno)pyrido[2,1-l]pyrimido[6,1-h][1,4,7,9,10,13]benzoxap entaazacyclohexadecine-7,24(6H)-dione; (18aS)-13-(cis-3-hydroxycyclobutyl)-2,11-bis[trideuteriomethyl]-8,9,10,11,19,20,21,22-octa hydro-18aH,24H-18,15-(metheno)pyrido[2,1-l]pyrimido[6,1-h][1,4,7,9,10,13]benzoxapentaa zacyclohexadecine-7,24(6H)-dione; (18aS)-13-(trans-3-hydroxycyclobutyl)-2,11-bis[trideuteriomethyl]-8,9,10,11,19,20,21,22-oct ahydro-18aH,24H-18,15-(metheno)pyrido[2,1-l]pyrimido[6,1-h][1,4,7,9,10,13]benzoxapenta azacyclohexadecine-7,24(6H)-dione;
(18aS)-13-cyclopropyl-2-fluoro-11-methyl-8,9,10,11,19,20,21,22-octahydro-18aH,24H-18,1 5-(metheno)pyrido[2,1-l]pyrimido[6,1-h][1,4,9,10,13]benzoxatetraazacyclohexadecine-7,24( 6H)-dione;
(18aS)-2-chloro-13-cyclopropyl-11-methyl-8,9,10,11,19,20,21,22-octahydro-18aH,24H-18,1 5-(metheno)pyrido[2,1-l]pyrimido[6,1-h][1,4,9,10,13]benzoxatetraazacyclohexadecine-7,24( 6H)-dione;
(18aS)-2-chloro-13-(3-hydroxyazetidin-1-yl)-11-methyl-8,9,10,11,19,20,21,22-octahydro-18 aH,24H-18,15-(metheno)pyrido[2,1-l]pyrimido[6,1-h][1,4,9,10,13]benzoxatetraazacyclohexa decine-7,24(6H)-dione;
(18aS)-13-cyclopropyl-2,11-dimethyl-8,9,10,11,19,20,21,22-octahydro-18aH,24H-18,15-(me theno)pyrido[2,1-l]pyrimido[6,1-h][1,4,9,10,13]benzoxatetraazacyclohexadecine-7,24(6H)-di one;
(18aS)-13-(3-hydroxyazetidin-1-yl)-2,11-dimethyl-8,9,10,11,19,20,21,22-octahydro-18aH,24 H-18,15-(metheno)pyrido[2,1-l]pyrimido[6,1-h][1,4,9,10,13]benzoxatetraazacyclohexadecin e-7,24(6H)-dione;
(18aS)-13-(3-hydroxyazetidin-1-yl)-11-methyl-2-trideuteriomethyl-8,9,10,11,19,20,21,22-oct ahydro-18aH,24H-18,15-(metheno)pyrido[2,1-1]pyrimido[6,1-h][1,4,9,10,13]benzoxatetraaza cyclohexadecine-7,24(6H)-dione; (18aS)-13-(azetidin-1-yl)-2-fluoro-11-methyl-6,7,8,9,10,11,19,20,21,22-decahydro-18aH,24 H-18,15-(metheno)pyrido[2,1-1]pyrimido[6,1-h][1,4,7,9,10,13]benzoxapentaazacyclohexadec in-24-one;
(18aS)-13-(azetidin-1-yl)-2-chloro-8,11-dimethyl-6,7,8,9,10,11,19,20,21,22-decahydro-18aH ,24H-18,15-(metheno)pyrido[2,1-l]pyrimido[6,1-h][1,4,7,9,10,13]benzoxapentaazacyclohexa decin-24-one; (18aS)-13-(azetidin-1-yl)-2,11-dimethyl-6,7,8,9,10,11,19,20,21,22-decahydro-18aH,24H-18, 15-(metheno)pyrido[2,1-l]pyrimido[6,1-h][1,4,7,9,10,13]benzoxapentaazacyclohexadecin-24 -one;
(18aS)-2,11-dimethyl-13-(pyrrolidin-1-yl)-6,7,8,9,10,11,19,20,21,22-decahydro-18aH,24H-1 8,15-(metheno)pyrido[2,1-l]pyrimido[6,1-h][1,4,7,9,10,13]benzoxapentaazacyclohexadecin-2 4-one;
(18aS)-13-(azetidin-1-yl)-2,8,11-trimethyl-6,7,8,9,10,11,19,20,21,22-decahydro-18aH,24H-1 8,15-(metheno)pyrido[2,1-l]pyrimido[6,1-h][1,4,7,9,10,13]benzoxapentaazacyclohexadecin-2 4-one;
(18aS)-13-(azetidin-1-yl)-8-ethyl-2,11-dimethyl-6,7,8,9,10,11,19,20,21,22-decahydro-18aH, 24H-18,15-(metheno)pyrido[2,1-l]pyrimido[6,1-h][1,4,7,9,10,13]benzoxapentaazacyclohexa decin-24-one;
(18aS)-13-(azetidin-1-yl)-8-(2-hydroxyethyl)-2,11-dimethyl-6,7,8,9,10,11,19,20,21,22-decah ydro-18aH,24H-18,15-(metheno)pyrido[2,1-l]pyrimido[6,1-h][1,4,7,9,10,13]benzoxapentaaz acyclohexadecin-24-one;
(18aS)-13-cyclopropyl-2,11-dimethyl-6H,8H,9H,10H,11H,18aH,19H,20H,21H,22H,24H-spi ro[18,15-(metheno)pyrido[2,1-l]pyrimido[6,1-h][1,4,7,9,10,13]benzoxapentaazacyclohexade cine-7,3'-oxetan]-24-one;
(18aS)-13-(azetidin-1-yl)-2,12-difluoro-11-methyl-6,7,8,9,10,11,19,20,21,22-decahydro-18a H,24H-18,15-(metheno)pyrido[2,1-l]pyrimido[6,1-h][1,4,7,9,10,13]benzoxapentaazacyclohe xadecin-24-one;
(17S)-3-[(3S)-3-aminopyrrolidin-1-yl]-13-chloro-17-ethyl-5,16-dimethyl-6,7,8,9,16,17-hexah ydro-5H,15H-18,1-(metheno)pyrimido[6,1-g][1,6,8,9,12]benzoxatetraazacyclopentadecin-15 -one;
(17S)-3-[(3S)-3-aminopyrrolidin-1-yl]-13-chloro-5,17-diethyl-16-methyl-6,7,8,9,16,17-hexah ydro-5H,15H-18,1-(metheno)pyrimido[6,1-g][1,6,8,9,12]benzoxatetraazacyclopentadecin-15 -one;
(17S)-3-[(3S)-3-aminopyrrolidin-1-yl]-17-ethyl-5,13,16-trimethyl-6,7,8,9,16,17-hexahydro-5 H,15H-18,1-(metheno)pyrimido[6,1-g][1,6,8,9,12]benzoxatetraazacyclopentadecin-15-one;
(17S)-3-[(2-aminoethyl)amino]-17-ethyl-5,13,16-trimethyl-6,7,8,9,16,17-hexahydro-5H,15H-18,1-(metheno)pyrimido[6,1-g][1,6,8,9,12]benzoxatetraazacyclopentadecin-15-one;
(17S)-3-(3-aminopropyl)-17-ethyl-5,13,16-trimethyl-6,7,8,9,16,17-hexahydro-5H,15H-18,1-( metheno)pyrimido[6,1-g][1,6,8,9,12]benzoxatetraazacyclopentadecin-15-one;
(17S)-3-[(3S)-3-aminopyrrolidin-1-yl]-17-ethyl-5,13,16-trimethyl-5,6,7,8,9,10,16,17-octahyd ro-15H-18,1-(metheno)pyrimido[6,1-g][1,6,8,9,12]benzopentaazacyclopentadecin-15-one;
(17S)-3-[(3S)-3-aminopyrrolidin-1-yl]-17-ethyl-4,13,16-trimethyl-6,7,8,9,16,17-hexahydro-5 H,15H-18,1-(metheno)pyrimido[6,1-g][1,6,8,9,12]benzoxatetraazacyclopentadecin-15-one;
(17S)-3-[(3S)-3-aminopyrrolidin-1-yl]-17-ethyl-4,5,13,16-tetramethyl-5,6,7,8,9,10,16,17-oct ahydro-15H-18,1-(metheno)pyrimido[6,1-g][1,6,8,9,12]benzopentaazacyclopentadecin-15-on e;
(23aS)-18-(3-aminoazetidin-1-yl)-8,16-dimethyl-1,3,4,13,14,15,16,23a-octahydro-2H,6H,12 H-23,20-(metheno)pyrido[2,1-k]pyrimido[6,1-g][1,6,8,9,12]benzoxatetraazacyclopentadecin-6-one;
(23aS)-18-(3-hydroxyazetidin-1-yl)-8,16-dimethyl-1,3,4,13,14,15,16,23a-octahydro-2H,6H,1 2H-23,20-(metheno)pyrido[2,1-k]pyrimido[6,1-g][1,6,8,9,12]benzoxatetraazacyclopentadeci n-6-one;
(23aS)-18-[(3S)-3-aminopyrrolidin-1-yl]-7-chloro-16-methyl-1,3,4,13,14,15,16,23a-octahydr o-2H,6H,12H-23,20-(metheno)pyrido[2,1-k]pyrimido[6,1-g][1,6,8,9,12]benzoxatetraazacycl opentadecin-6-one;
(23aS)-18-[(3S)-3-aminopyrrolidin-1-yl]-7-fluoro-16-methyl-1,3,4,13,14,15,16,23a-octahydr o-2H,6H,12H-23,20-(metheno)pyrido[2,1-k]pyrimido[6,1-g][1,6,8,9,12]benzoxatetraazacycl opentadecin-6-one;
(23aS)-18-(3-aminopropyl)-8-chloro-16-methyl-1,3,4,13,14,15,16,23a-octahydro-2H,6H,12H -23,20-(metheno)pyrido[2,1-k]pyrimido[6,1-g][1,6,8,9,12]benzoxatetraazacyclopentadecin-6 -one;
(23aS)-18-[(3S)-3-aminopyrrolidin-1-yl]-8-fluoro-16-methyl-1,3,4,13,14,15,16,23a-octahydr o-2H,6H,12H-23,20-(metheno)pyrido[2,1-k]pyrimido[6,1-g][1,6,8,9,12]benzoxatetraazacycl opentadecin-6-one;
(23aS)-18-(3-aminoazetidin-1-yl)-8-fluoro-16-methyl-1,3,4,13,14,15,16,23a-octahydro-2H,6 H,12H-23,20-(metheno)pyrido[2,1-k]pyrimido[6,1-g][1,6,8,9,12]benzoxatetraazacyclopentad ecin-6-one;
(23aS)-18-[(3S)-3-aminopyrrolidin-1-yl]-9-fluoro-16-methyl-1,3,4,13,14,15,16,23a-octahydr o-2H,6H,12H-23,20-(metheno)pyrido[2,1-k]pyrimido[6,1-g][1,6,8,9,12]benzoxatetraazacycl opentadecin-6-one;
(23aS)-18-[(3S)-3-aminopyrrolidin-1-yl]-16-methyl-1,3,4,13,14,15,16,23a-octahydro-2H,6H, 12H-23,20-(metheno)pyrido[2,1-k]pyrimido[6,1-g][1,6,8,9,12]benzoxatetraazacyclopentadec in-6-one;
(23aS)-18-(azetidin-1-yl)-8-chloro-15-methyl-1,3,4,13,14,15,16,23a-octahydro-2H,6H,12H-2 3,20-(metheno)pyrido[2,1-k]pyrimido[6,1-g][1,5,8,9,12]benzoxatetraazacyclopentadecin-6-o ne;
(17S)-3-[(3S)-3-aminopyrrolidin-1-yl]-17-ethyl-4,13,16-trimethyl-7,8,16,17-tetrahydro-5H-1 8,1-(metheno)-9λ⁶-pyrimido[6,1-g][2,1,6,8,9,12]benzothiapentaazacyclopentadecine-9,9,15( 6H,10H)-trione;
(17S)-3-[(2-aminoethyl)amino]-17-ethyl-4,13,16-trimethyl-7,8,16,17-tetrahydro-5H-18,1-(m etheno)-9λ⁶-pyrimido[6,1-g][2,1,6,8,9,12]benzothiapentaazacyclopentadecine-9,9,15(6H,10 H)-trione;
(23aS)-18-[(2-aminoethyl)amino]-8-chloro-16,17-dimethyl-1,3,4,13,14,15,16,23a-octahydro-2H,6H-23,20-(metheno)-12λ⁶-pyrido[2,1-k]pyrimido[6,1-g][2,1,6,8,9,12]benzothiapentaazac yclopentadecine-6,12,12(11H)-trione;
(20aS)-15-(azetidin-1-yl)-2-chloro-9,13-dimethyl-12,13,21,22,23,24-hexahydro-6H,11H,20a H,26H-20,17-(metheno)pyrido[2,1-l]pyrimido[6,1-h][1,2,4]triazolo[3,4-c][1,4,7,9,10,13]benz oxapentaazacyclohexadecin-26-one;
(20aS)-15-(azetidin-1-yl)-2-chloro-13-methyl-12,13,21,22,23,24-hexahydro-6H,11H,20aH,2 6H-20,17-(metheno)imidazo[2,1-c]pyrido[2,1-l]pyrimido[6,1-h][1,4,7,9,10,13]benzoxapenta azacyclohexadecin-26-one;
(18aS)-13-(3-aminoazetidin-1-yl)-2-chloro-11-methyl-7-(trifluoromethyl)-6,7,8,9,10,11,19,2 0,21,22-decahydro-18aH,24H-18,15-(metheno)pyrido[2,1-l]pyrimido[6,1-h][1,4,7,9,10,13]be nzoxapentaazacyclohexadecin-24-one;
(18aS)-2-chloro-13-(3-hydroxyazetidin-1-yl)-11-methyl-7-(trifluoromethyl)-6,7,8,9,10,11,19, 20,21,22-decahydro-18aH,24H-18,15-(metheno)pyrido[2,1-l]pyrimido[6,1-h][1,4,7,9,10,13]b enzoxapentaazacyclohexadecin-24-one;
(18aS)-13-(3-hydroxyazetidin-1-yl)-2,11-dimethyl-7-(trifluoromethyl)-6,7,8,9,10,11,19,20,21 ,22-decahydro-18aH,24H-18,15-(metheno)pyrido[2,1-l]pyrimido[6,1-h][1,4,7,9,10,13]benzo xapentaazacyclohexadecin-24-one;
(18aS)-13-[(3 S)-3-aminopyrrolidin-1-yl]-2,11-dimethyl-7-(trifluoromethyl)-6,7,8,9,10,11,19, 20,21,22-decahydro-18aH,24H-18,15-(metheno)pyrido[2,1-l]pyrimido[6,1-h][1,4,7,9,10,13]b enzoxapentaazacyclohexadecin-24-one;
(18aS)-13-(3-hydroxyazetidin-1-yl)-2,11-dimethyl-9-(trifluoromethyl)-6,7,8,9,10,11,19,20,21 ,22-decahydro-18aH,24H-18,15-(metheno)pyrido[2,1-l]pyrimido[6,1-h][1,4,7,9,10,13]benzo xapentaazacyclohexadecin-24-one;
(18aS)-3-(azetidin-1-yl)-5,12-dimethyl-12-phenyl-6,7,8,9,11,12,16,17,18,18a-decahydro-5H, 15H-19,1-(metheno)-10λ⁶-pyrido[1,2-e]pyrimido[1,6-i][1,2,5,8,9,11]thiapentaazacyclopentad ecine-10,10,13-trione;
(18aS)-3-[(3S)-3-aminopyrrolidin-1-yl]-5,12-dimethyl-12-phenyl-6,7,8,9,11,12,16,17,18,18a-decahydro-5H,15H-19,1-(metheno)-10λ⁶-pyrido[1,2-e]pyrimido[1,6-i][1,2,5,8,9,11]thiapenta azacyclopentadecine-10,10,13-trione; and (18a'S)-3'-[(3S)-3-aminopyrrolidin-1-yl]-5'-methyl-2H,3H,5'H,6'H,7'H,8'H,9'H,10'H,11'H,13' H,15'H,16'H,17'H,18'H,18a'H-spiro[indene-1,12'-[19,1](metheno)[10λ⁶]pyrido[1,2-e]pyrimid o[1,6-i][1,2,5,8,9,11]thiapentaazacyclopentadecine]-10',10',13'-trione,
or a pharmaceutically acceptable salt thereof;

(16) A medicament comprising the compound according to any one of the above (1) to (15) or a pharmaceutically acceptable salt thereof as an active ingredient; and

(17) A drug for preventing or treating an infection in which viruses of the subfamily Pneumovirinae, including respiratory syncytial virus (RSV), are involved, wherein the drug comprises the compound according to any one of the above (1) to (15) or a pharmaceutically acceptable salt thereof as an active ingredient.

### ADVANTAGEOUS EFFECTS OF INVENTION

The present inventive compound was demonstrated to have anti-RSV activity. Medicaments containing the present inventive compound are useful as a drug for preventing or treating an infection in which viruses of the subfamily Pneumovirinae, including respiratory syncytial virus (RSV), are involved.

### DESCRIPTION OF EMBODIMENTS

The terms used in the present specification are as defined below.

The term "halogen atom" refers to a fluorine atom, a chlorine atom, a bromine atom, or an iodine atom.

The term "C₁₋₆ alkyl group" refers to a linear alkyl group or branched alkyl group having 1 to 6 carbon atoms. Examples of the linear alkyl group include alkyl groups such as a methyl group, an ethyl group, a propyl group, a butyl group, a pentyl group, and a hexyl group. Examples of the branched alkyl group include, for example, isopropyl, isobutyl, tert-butyl, isopentyl, 1-ethylpropyl, and isohexyl.

The term "C₃₋₆ cycloalkyl group" refers to a cyclic alkyl group having 3 to 6 carbon atoms, and examples thereof include a cyclopropyl group, a cyclobutyl group, a cyclopentyl group, and a cyclohexyl group.

The term "C₂₋₆ alkenyl group" refers to an alkenyl group having 2 to 6 carbon atoms, and examples thereof include, for example, an ethenyl group, a propenyl group, a butenyl group, a pentenyl group, and a hexenyl group.

The term "C₂₋₆ alkynyl group" refers to an alkynyl group having 2 to 6 carbon atoms, and examples thereof include, for example, an ethynyl group, a propynyl group, a butynyl group, a pentynyl group, and a hexynyl group.

Examples of the "aryl group" include, for example, a phenyl group, a 1-naphthyl group, and a 2-naphthyl group.

The term "heteroaryl group" refers to a monocyclic heteroaryl group or fused ring heteroaryl group containing 1 to 4 heteroatoms that are the same or different, selected from the group consisting of an oxygen atom, a nitrogen atom, and a sulfur atom, and is preferably a 5- to 10-membered ring. Examples thereof include, for example, a pyrrolyl group, a furyl group, a thienyl group, an imidazolyl group, a pyrazolyl group, a thiazolyl group, an isothiazolyl group, an oxazolyl group, an isoxazolyl group, a triazolyl group, a tetrazolyl group, an oxadiazolyl group, a pyridyl group, a pyrazinyl group, a pyrimidinyl group, a pyridazinyl group, a triazinyl group, an indolyl group, a benzofuranyl group, a dihydrobenzofuranyl group, a benzothienyl group, a tetrahydrobenzothienyl group, a dihydrobenzodioxynyl group, a benzimidazolyl group, a benzoxazolyl group, a benzisoxazolyl group, a benzothiazolyl group, a benzisothiazolyl group, an indazolyl group, a quinolyl group, an isoquinolyl group, and a tetrahydroimidazopyridyl group. Other examples thereof include a pyrrolyl group, a furyl group, a thienyl group, an imidazolyl group, a pyrazolyl group, a thiazolyl group, an isothiazolyl group, an oxazolyl group, an isoxazolyl group, a triazolyl group, a tetrazolyl group, an oxadiazolyl group, a pyridyl group, a pyrazinyl group, a pyrimidinyl group, a pyridazinyl group, a triazinyl group, an indolyl group, a benzofuranyl group, a benzothienyl group, a benzimidazolyl group, a benzoxazolyl group, a benzisoxazolyl group, a benzothiazolyl group, a benzisothiazolyl group, an indazolyl group, a quinolyl group, and an isoquinolyl group.

The term "C₃₋₆ cycloalkanediyl" refers to a cyclic divalent alkane having 3 to 6 carbon atoms, and examples thereof include cyclopropane-1,1-diyl, cyclobutane-1,1-diyl, cyclopentane-1,1-diyl, and cyclohexane-1,1-diyl.

The term "C₁₋₄ alkanediyl" refers to a divalent hydrocarbon group having 1 to 4 carbon atoms, and examples thereof include methanediyl, ethane-1,1-diyl, ethane-1,2-diyl, propane-1,1-diyl, propane-1,2-diyl, propane-1,3-diyl, propane-2,2-diyl, butane-1,2-diyl, butane-1,3-diyl, and butane-1,4-diyl.

The term "C₂₋₄ alkanediyl" refers to a divalent hydrocarbon group having 2 to 4 carbon atoms, and examples thereof include ethane-1,1-diyl, ethane-1,2-diyl, propane-1,1-diyl, propane-1,2-diyl, propane-1,3-diyl, propane-2,2-diyl, butane-1,2-diyl, butane-1,3-diyl, and butane-1,4-diyl.

The term "C₃₋₆ alkanediyl" refers to a divalent hydrocarbon group having 3 to 6 carbon atoms, and examples thereof include propane-1,1-diyl, propane-1,2-diyl, propane-1,3-diyl, propane-2,2-diyl, butane-1,2-diyl, butane-1,3-diyl, butane-1,4-diyl, pentane-1,5-diyl, and hexane-1,6-diyl.

The term "C₁₋₆ alkoxy group" refers to a linear alkoxy group or branched alkoxy group having 1 to 6 carbon atoms. Examples of the linear alkoxy group include alkoxy groups such as a methoxy group, an ethoxy group, a propoxy group, a butoxy group, a pentyloxy group, and a hexyloxy group. Examples of the branched alkoxy group include, for example, an isopropoxy group, an isobutoxy group, a tert-butoxy group, an isopentyloxy group, a 1-ethylpropoxy group, and an isohexyloxy group.

The term "C₁₋₆ alkylsulfanyl group" refers to a linear alkylsulfanyl group or branched alkylsulfanyl group having 1 to 6 carbon atoms. Examples of the linear alkylsulfanyl group include alkylsulfanyl groups such as a methylsulfanyl group, an ethylsulfanyl group, a propylsulfanyl group, a butylsulfanyl group, a pentylsulfanyl group, and a hexylsulfanyl group. Examples of the branched alkylsulfanyl group include, for example, an isopropylsulfanyl group, an isobutylsulfanyl group, a tert-butylsulfanyl group, an isopentylsulfanyl group, a 1-ethylpropylsulfanyl group, and an isohexylsulfanyl group.

The term "C₁₋₆ alkanoyl group" refers to an alkanoyl group having 1 to 6 carbon atoms, and examples thereof include, for example, a formyl group, an acetyl group, a propionyl group, a butyryl group, an isobutyryl group, a valeryl group, an isovaleryl group, and a pivaloyl group.

The term "C₂₋₆ alkanoylamino group" refers to a group in which the "C₂₋₆ alkanoyl group" described above and an amino group are bonded, and examples thereof include, for example, an acetylamino group, a propionylamino group, a butyrylamino group, an isobutyrylamino group, a valerylamino group, an isovalerylamino group, and a pivaloylamino group.

The term "C₁₋₆ alkoxycarbonyl group" refers to a group in which the "C₁₋₆ alkoxy group" described above and a carbonyl group are bonded, and examples thereof include, for example, a methoxycarbonyl group, an ethoxycarbonyl group, a propoxycarbonyl group, an isopropoxycarbonyl group, a butoxycarbonyl group, an isobutoxycarbonyl group, a tert-butoxycarbonyl group, and a pentyloxycarbonyl group.

The term "C₁₋₆ alkylamino group" refers to a group in which one or two of the same or different "C₁₋₆ alkyl groups" described above and an amino group are bonded, and examples thereof include, for example, a methylamino group, an ethylamino group, a propylamiono group, a butylamino group, a pentylamino group, a hexylamino group, an isopropylamino group, an isobutylamino group, a tert-butylamino group, an isopentylamino group, a 1-ethylpropylamino group, an isohexylamino group, a dimethylamino group, a diethylamino group, a dipropylamino group, an ethylmethylamino group, a methylpropylamino group, and an ethylpropylamino group.

The term "mono-C₁₋₆ alkylamino group" refers to a group in which one of the "C₁₋₆ alkyl groups" described above and an amino group are bonded, and examples thereof include, for example, a methylamino group, an ethylamino group, a propylamiono group, a butylamino group, a pentylamino group, a hexylamino group, an isopropylamino group, an isobutylamino group, a tert-butylamino group, an isopentylamino group, a 1-ethylpropylamino group, and an isohexylamino group.

The term "di-C₁₋₆ alkylamino group" refers to a group in which two of the same or different "C₁₋₆ alkyl groups" described above and an amino group are bonded, and examples thereof include, for example, a dimethylamino group, a diethylamino group, a dipropylamino group, an ethylmethylamino group, a methylpropylamino group, and an ethylpropylamino group.

The term "C₁₋₆ alkylsulfonyl group" refers to a group in which the "C₁₋₆ alkyl group" described above and a sulfonyl group are bonded, and examples thereof include, for example, a methylsulfonyl group, an ethylsulfonyl group, a propylsulfonyl group, a butylsulfonyl group, a pentylsulfonyl group, a hexylsulfonyl group, an isopropylsulfonyl group, an isobutylsulfonyl group, a tert-butylsulfonyl group, an isopentylsulfonyl group, a 1-ethylpropylsulfonyl group, and an isohexylsulfonyl group.

The term "C₁₋₆ alkylsulfonylamino group" refers to a group in which the "C₁₋₆ alkylsulfonyl group" described above and an amino group are bonded, and examples thereof include, for example, a methylsulfonylamino group, an ethylsulfonylamino group, a propylsulfonylamino group, a butylsulfonylamino group, a pentylsulfonylamino group, a hexylsulfonylamino group, an isopropylsulfonylamino group, an isobutylsulfonylamino group, a tert-butylsulfonylamino group, an isopentylsulfonylamino group, a 1-ethylpropylsulfonylamino group, and an isohexylsulfonylamino group.

The term "C₃₋₆ cycloalkylamino group" refers to a group in which the "C₃₋₆ cycloalkyl group" described above and an amino group are bonded, and examples thereof include a cyclopropylamino group, a cyclobutylamino group, a cyclopentylamino group, and a cyclohexylamino group.

The term "aryloxy group" refers to a group in which the "aryl group" described above and an oxy group are bonded, and examples thereof include, for example, a phenoxy group, a 1-naphthoxy group, and a 2-naphthoxy group.

The term "heterocyclyl group" refers to a heterocyclyl group containing 1 to 3 heteroatoms that are the same or different, selected from the group consisting of an oxygen atom, a nitrogen atom, and a sulfur atom, and preferably a 4- to 10-membered, monocyclic or fused ring heterocyclyl group. Examples thereof include, for example, an azetidinyl group, a pyrrolidinyl group, a piperidinyl group, an oxetanyl group, a tetrahydrofuranyl group, a tetrahydropyranyl group, a piperazinyl group, a morpholinyl group, a thiomorpholinyl group, a 1,1-dioxidethiomorpholinyl group, an indolinyl group, an isoindolinyl group, a 3,4-dihydro-2H-benzoxazinyl group, and a 2-azaspiro[3.3]heptan-2-yl group.

The term "heterocyclyloxy group" refers to a group in which the "heterocyclyl group" described above and an oxy group are bonded, and examples thereof include, for example, an azetidinyloxy group, a pyrrolidinyloxy group, a piperidinyloxy group, an oxetanyloxy group, a tetrahydrofuranyloxy group, a tetrahydropyranyloxy group, a piperazinyloxy group, a morpholinyloxy group, a thiomorpholinyloxy group, a 1,1-dioxidethiomorpholinyloxy group, an indolinyloxy group, an isoindolinyloxy group, and a 3,4-dihydro-2H-benzoxazinyloxy group.

The term "heterocyclylamino group" refers to a group in which the "heterocyclyl group" described above and an amino group are bonded, and examples thereof include, for example, an azetidinylamino group, a pyrrolidinylamino group, a piperidinylamino group, an oxetanylamino group, a tetrahydrofuranylamino group, a tetrahydropyranylamino group, a piperazinylamino group, a morpholinylamino group, a thiomorpholinylamino group, a 1,1-dioxidethiomorpholinylamino group, an indolinylamino group, an isoindolinylamino group, and a 3,4-dihydro-2H-benzoxazinylamino group.

Examples of the "5- to 6-membered cyclic amine optionally containing one oxygen atom in the ring" include pyrrolidine, piperidine, and morpholine.

As described for confirmation, the term "ethylene group" in the present specification refers to a divalent group represented by the structural formula: -CH₂-CH₂-.

In the present specification, it is preferable that the two bonding hands that phenylene, naphthalenediyl, pyridinediyl, pyridonediyl, thiophenediyl, and pyrazolediyl have should protrude from two adjacent atoms. For example, phenylene is benzene-1,2-diyl or benzene-1,3-diyl, naphthalenediyl is naphthalene-2,3-diyl, pyridonediyl is pyridone-1,6-diyl, thiophenediyl is thiophene-2,3-diyl, and pyrazolediyl is pyrazole-3,4-diyl.

The term "indenediyl" in the present specification is preferably indene-1,1-diyl.

In addition, as described for confirmation, the following structural formulas are applied to the structure represented by formula I without reversing the left and right sides: CHR^{X12}-CH₂-CH₂-NHCO-CH₂, CR^{X12}=CH-CH₂-NHCO-CH₂, CH₂-NR¹²-(CH₂)₃, C₂₋₄ alkanediyl-CO, C₂₋₄ alkanediyl-SO₂, Z¹-Z²-Z³, (CH₂)₂-NR^{Z2}CO-CH₂, CH₂C(CH₃)₂-NR^{Z2}CO-CH₂, (CH₂)₂-NR^{Z2}CO-CH(CH₃), CH₂-CONR^{Z2}-(CH₂)₂, (CH₂)₃-NR^{Z2}CO-CH₂, (CH₂)₂-NR^{Z2}-(CH₂)₂, CH₂CH(CF₃)-NR^{Z2}(CH₂)₂, (CH₂)₂-NR^{Z2}CH(CF₃)-CH₂, (CH₂)₂-NR^{Z2}-C(-CH₂OCH₂-)CH₂, (CH₂)₄, (CH₂)₅, (CH₂)₂-NR^{Z2}SO₂-CH₂, (CH₂)₂-O-(CH₂)₂, (CH₂)₃-SO₂, (CH₂)₄-SO₂, CH(CH₃)-CH₂-CH₂-NHCO-CH₂, C(CH₃)=CH-CH₂-NHCO-CH₂, formula II, and formula III. Note that the structure: -C(-CH₂OCH₂-) represents oxetane-3,3-diyl.

The viruses of the subfamily Pneumovirinae, including respiratory syncytial virus (RSV), are negative sense single-stranded RNA viruses included in the family Paramyxoviridae, and examples thereof include human respiratory syncytial virus (HRSV) and human metapneumovirus (HMPV).

The infections in which viruses of the subfamily Pneumovirinae, including respiratory syncytial virus (RSV), are involved are epidemic human and animal infections, including RSV infection and HMPV infection. The RSV infection may be an RSV respiratory infection.

When the present inventive compound (I) forms a salt and it is used as a medical product, it is preferably a pharmaceutically acceptable salt. As the "pharmaceutically acceptable salt", for example, a salt with an inorganic acid such as hydrochloride, sulfate, hydrobromide, nitrate, or phosphate, or a salt with an organic acid such as acetate, oxalate, lactate, citrate, malate, tartrate, maleate, fumarate, succinate, methanesulfonate, ethanesulfonate, benzenesulfonate, or p-toluenesulfonate is used, but it is not limited to these salts. The conversion of the free form to the relevant salt can be carried out by conventional methods.

In addition, examples of the "pharmaceutically acceptable salt" include salts with inorganic bases and salts with organic bases. Suitable examples of the salts with inorganic bases include, for example, alkali metal salts (for example, sodium salts, potassium salts, and the like), alkaline earth metal salts (for example, calcium salts, magnesium salts, barium salts, and the like), aluminum salts, and ammonium salts. Suitable examples of the salts with organic bases include, for example, salts with trimethylamine, triethylamine, pyridine, picoline, ethanolamine, diethanolamine, triethanolamine, dicyclohexylamine, and N,N'-dibenzylethylenediamine.

Preferred aspects in the present inventive compound are as follows.
Y is preferably phenylene {the phenylene is optionally substituted with one to two substituents selected from the group consisting of a halogen atom, a C₁₋₂ alkyl group (the C₁₋₂ alkyl group is optionally substituted with one to five deuterium atoms), a methoxy group, a vinyl group, an acetyl group, a cyano group, and a cyclopropyl group}, methylene {the methylene is optionally substituted with one to two substituents selected from the group consisting of a methyl group and a phenyl group (the phenyl group is optionally substituted with one to two substituents selected from the group consisting of a halogen atom and a methyl group)}, or indenediyl, and is more preferably any of the structures of formula group (IV) below: 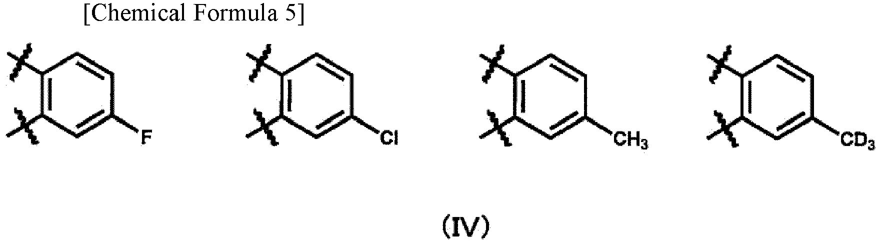
X¹ is preferably a single bond or NR^{X11} {R^{X11} represents a C₁₋₂ alkyl group (the C₁₋₂ alkyl group is optionally substituted with one to five deuterium atoms) or a cyclopropyl group}, and
it is more preferably a single bond, NCH₃, or NCD₃.
X² is preferably O or NH
L is preferably:
   CH(CH₃)-CH₂-CH₂-NHCO-CH₂, when X¹ is a single bond; and
   (CH₂)₂-NHCO-CH₂, (CH₂)₂-N(CH₃)CO-CH₂, CH₂C(CH₃)₂-NHCO-CH₂, CH₂CH(CF₃)-NHCO-CH₂, (CH₂)₂-NHCO-CH(CH₃), CH₂-CONH-(CH₂)₂, (CH₂)₃-NHCO-CH₂, (CH₂)₂-NH-(CH₂)₂, (CH₂)₂-N(CH₃)-(CH₂)₂, (CH₂)₂-NH-C(-CH₂OCH₂-)CH₂, (CH₂)₄, (CH₂)₅, CH₂CH(CF₃)-NH(CH₂)₂, (CH₂)₂-NHCH(CF₃)-CH₂, (CH₂)₃-SO₂, (CH₂)₄-SO₂, or any of the structures represented by formula (III) below, when X¹ is NR^{X11}, and
it is more preferably (CH₂)₂-NHCO-CH₂, (CH₂)₂-NH-(CH₂)₂, (CH₂)₄, or (CH₂)₂-NHCH(CF₃)-CH₂. 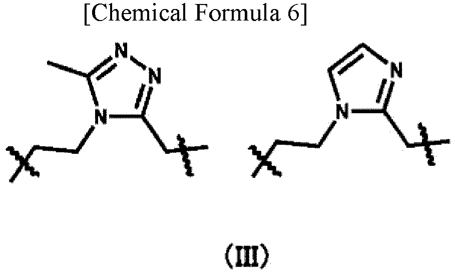
R¹ is preferably a hydrogen atom, a fluorine atom, or a methyl group.
R² is more preferably a C₁₋₃ alkyl group (the C₁₋₃ alkyl group is optionally substituted with one substituent selected from the group consisting of an amino group, a hydroxy group, and a di-C₁₋₃ alkylamino group), a C₂₋₄ alkenyl group (the C₂₋₄ alkenyl group is optionally substituted with one substituent selected from the group consisting of a hydroxy group and a carboxy group), a C₂₋₄ alkynyl group (the C₂₋₄ alkynyl group is optionally substituted with one substituent selected from the group consisting of a hydroxy group and an amino group), a C₁₋₃ alkoxy group (the C₁₋₃ alkoxy group is optionally substituted with one amino group), a C₃₋₅ cycloalkyl group {the C₃₋₅ cycloalkyl group is optionally substituted with one substituent selected from the group consisting of a C₁₋₃ alkyl group (the C₁₋₃ alkyl group is optionally substituted with one hydroxy group), an amino group, and a hydroxy group}, a heterocyclyloxy group, a heterocyclylamino group, a C₁₋₃ alkylamino group {the C₁₋₃ alkylamino group is optionally substituted with one to two substituents selected from the group consisting of an amino group, a C₁₋₃ alkyl group, and a C₃₋₆ cycloalkyl group (the C₃₋₆ cycloalkyl group is optionally substituted with one amino group)}, a C₃₋₆ cycloalkylamino group (the C₃₋₆ cycloalkylamino group is optionally substituted with one amino group), or a heterocyclyl group {the heterocyclyl group is optionally substituted with one to two substituents selected from the group consisting of a hydroxy group, an amino group, a mono-C₁₋₂ alkylamino group, a di-C₁₋₆ alkylamino group, and a C₁₋₆ alkyl group (the C₁₋₆ alkyl group is optionally substituted with one amino group)}; and
it is further preferably any of the structures of formula group (V) below: 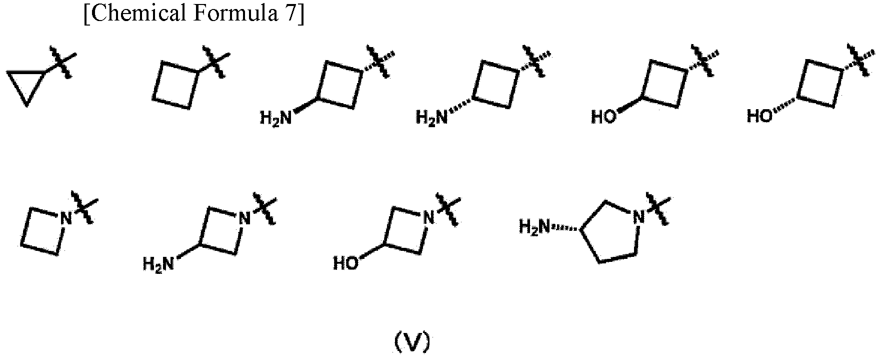
R³ is preferably a hydrogen atom.
R⁴ is preferably a hydrogen atom.

The case where R⁵ is a C₁₋₃ alkyl group and R⁶ is a C₁₋₃ alkyl group, or the case where R⁵ and R⁶ form a cyclic amine optionally containing one oxygen atom in the ring (the cyclic amine is optionally substituted with one to two halogen atoms), together with the adjacent carbon atom and nitrogen atom, is more preferable; and
the case where R⁵ and R⁶ form a piperidine ring, together with the adjacent carbon atom and nitrogen atom, is further preferable.

In the present inventive compound, examples of preferred compounds include the following:
(18aS)-13-[(3S)-3-aminopyrrolidin-1-yl]-2-fluoro-11-methyl-8,9,10,11,19,20,21,22-octahydr o-18aH,24H-18,15-(metheno)pyrido[2,1-l]pyrimido[6,1-h][1,4,7,9,10,13]benzoxapentaazacy clohexadecine-7,24(6H)-dione;
(18aS)-13-(azetidin-1-yl)-2-fluoro-11-methyl-8,9,10,11,19,20,21,22-octahydro-18aH,24H-18 ,15-(metheno)pyrido[2,1-l]pyrimido[6,1-h][1,4,7,9,10,13]benzoxapentaazacyclohexadecine-7 ,24(6H)-dione;
(18aS)-2-fluoro-13-(3-hydroxyazetidin-1-yl)-11-methyl-8,9,10,11,19,20,21,22-octahydro-18a H,24H-18,15-(metheno)pyrido[2,1-l]pyrimido[6,1-h][1,4,7,9,10,13]benzoxapentaazacyclohe xadecine-7,24(6H)-dione;
(18aS)-13-(3-aminoazetidin-1-yl)-2-fluoro-11-methyl-8,9,10,11,19,20,21,22-octahydro-18aH ,24H-18,15-(metheno)pyrido[2,1-l]pyrimido[6,1-h][1,4,7,9,10,13]benzoxapentaazacyclohexa decine-7,24(6H)-dione;
(18aS)-13-(azetidin-1-yl)-2-fluoro-8,11-dimethyl-8,9,10,11,19,20,21,22-octahydro-18aH,24 H-18,15-(metheno)pyrido[2,1-1]pyrimido[6,1-h][1,4,7,9,10,13]benzoxapentaazacyclohexadec ine-7,24(6H)-dione;
(18aS)-13-(3-aminoazetidin-1-yl)-2-fluoro-9,9,11-trimethyl-8,9,10,11,19,20,21,22-octahydro -18aH,24H-18,15-(metheno)pyrido[2,1-l]pyrimido[6,1-h][1,4,7,9,10,13]benzoxapentaazacycl ohexadecine-7,24(6H)-dione;
(18aS)-13-(3-aminoazetidin-1-yl)-2-fluoro-6,11-dimethyl-8,9,10,11,19,20,21,22-octahydro-1 8aH,24H-18,15-(metheno)pyrido[2,1-l]pyrimido[6,1-h][1,4,7,9,10,13]benzoxapentaazacyclo hexadecine-7,24(6H)-dione;
(18aS)-13-(3-aminoazetidin-1-yl)-11-cyclopropyl-2-fluoro-8,9,10,11,19,20,21,22-octahydro-18aH,24H-18,15-(metheno)pyrido[2,1-l]pyrimido[6,1-h][1,4,7,9,10,13]benzoxapentaazacycl ohexadecine-7,24(6H)-dione;
(18aS)-13-(3-aminoazetidin-1-yl)-2-fluoro-11-methyl-9-(trifluoromethyl)-8,9,10,11,19,20,21, 22-octahydro-18aH,24H-18,15-(metheno)pyrido[2,1-l]pyrimido[6,1-h][1,4,7,9,10,13]benzox apentaazacyclohexadecine-7,24(6H)-dione;
(18aS)-13-(cyclopropylamino)-2-fluoro-11-methyl-8,9,10,11,19,20,21,22-octahydro-18aH,24 H-18,15-(metheno)pyrido[2,1-1]pyrimido[6,1-h][1,4,7,9,10,13]benzoxapentaazacyclohexadec ine-7,24(6H)-dione;
(18aS)-13-{[(1-aminocyclopropyl)methyl]amino}-2-fluoro-11-methyl-8,9,10,11,19,20,21,22-octahydro-18aH,24H-18,15-(metheno)pyrido[2,1-l]pyrimido[6,1-h][1,4,7,9,10,13]benzoxape ntaazacyclohexadecine-7,24(6H)-dione;
(18aS)-2-fluoro-13-methoxy-11-methyl-8,9,10,11,19,20,21,22-octahydro-18aH,24H-18,15-( metheno)pyrido[2,1-l]pyrimido[6,1-h][1,4,7,9,10,13]benzoxapentaazacyclohexadecine-7,24( 6H)-dione;
(18aS)-13-cyclobutyl-2-fluoro-11-methyl-8,9,10,11,19,20,21,22-octahydro-18aH,24H-18,15-(metheno)pyrido[2,1-l]pyrimido[6,1-h][1,4,7,9,10,13]benzoxapentaazacyclohexadecine-7,24( 6H)-dione;
(18aS)-13-(1-aminocyclopropyl)-2-fluoro-11-methyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione;
(18aS)-2-fluoro-13-(3-hydroxyprop-1-yn-1-yl)-11-methyl-8,9,10,11,19,20,21,22-octahydro-1 8aH,24H-18,15-(metheno)pyrido[2,1-l]pyrimido[6,1-h][1,4,7,9,10,13]benzoxapentaazacyclo hexadecine-7,24(6H)-dione;
(18aS)-2-fluoro-13-(3-hydroxypropyl)-11-methyl-8,9,10,11,19,20,21,22-octahydro-18aH,24 H-18,15-(metheno)pyrido[2,1-l]pyrimido[6,1-h][1,4,7,9,10,13]benzoxapentaazacyclohexadec ine-7,24(6H)-dione;
(18aS)-2-fluoro-13-[(lE)-3-hydroxyprop-1-en-1-yl]-11-methyl-8,9,10,11,19,20,21,22-octahy dro-18aH,24H-18,15-(metheno)pyrido[2,1-l]pyrimido[6,1-h][1,4,7,9,10,13]benzoxapentaaza cyclohexadecine-7,24(6H)-dione;
(18aS)-13-cyclopropyl-2,25-difluoro-11-methyl-8,9,10,11,19,20,21,22-octahydro-18aH,24H-18,15-(metheno)pyrido[2,1-l]pyrimido[6,1-h][1,4,7,9,10,13]benzoxapentaazacyclohexadecin e-7,24(6H)-dione;
(18aS)-13-(azetidin-1-yl)-2-fluoro-11-methyl-7,8,10,11,19,20,21,22-octahydro-18aH,24H-18 ,15-(metheno)pyrido[2,1-l]pyrimido[6,1-h][1,4,7,9,10,13]benzoxapentaazacyclohexadecine-9 ,24(6H)-dione;
(18aS)-13-(azetidin-1-yl)-2-chloro-11-methyl-8,9,10,11,19,20,21,22-octahydro-18aH,24H-18 ,15-(metheno)pyrido[2,1-l]pyrimido[6,1-h][1,4,7,9,10,13]benzoxapentaazacyclohexadecine-7 ,24(6H)-dione;
(18aS)-2-chloro-13-(3-hydroxyazetidin-1-yl)-11-methyl-8,9,10,11,19,20,21,22-octahydro-18 aH,24H-18,15-(metheno)pyrido[2,1-l]pyrimido[6,1-h][1,4,7,9,10,13]benzoxapentaazacycloh exadecine-7,24(6H)-dione;
(18aS)-13-(3-aminoazetidin-1-yl)-2-chloro-11-methyl-8,9,10,11,19,20,21,22-octahydro-18aH ,24H-18,15-(metheno)pyrido[2,1-l]pyrimido[6,1-h][1,4,7,9,10,13]benzoxapentaazacyclohexa decine-7,24(6H)-dione;
(18aS)-13-(azetidin-1-yl)-2-chloro-8,11-dimethyl-8,9,10,11,19,20,21,22-octahydro-18aH,24 H-18,15-(metheno)pyrido[2,1-l]pyrimido[6,1-h][1,4,7,9,10,13]benzoxapentaazacyclohexadec ine-7,24(6H)-dione;
(18aS)-13-(3-hydroxyazetidin-1-yl)-2,11-dimethyl-8,9,10,11,19,20,21,22-octahydro-18aH,24 H-18,15-(metheno)pyrido[2,1-1]pyrimido[6,1-h][1,4,7,9,10,13]benzoxapentaazacyclohexadec ine-7,24(6H)-dione;
(18aS)-13-(3-aminoazetidin-1-yl)-2,11-dimethyl-8,9,10,11,19,20,21,22-octahydro-18aH,24H -18,15-(metheno)pyrido[2,1-l]pyrimido[6,1-h][1,4,7,9,10,13]benzoxapentaazacyclohexadecin e-7,24(6H)-dione;
(18aS)-13-(azetidin-1-yl)-2,11-dimethyl-8,9,10,11,19,20,21,22-octahydro-18aH,24H-18,15-( metheno)pyrido[2,1-l]pyrimido[6,1-h][1,4,7,9,10,13]benzoxapentaazacyclohexadecine-7,24( 6H)-dione;
(18aS)-13-(azetidin-1-yl)-2-ethyl-11-methyl-8,9,10,11,19,20,21,22-octahydro-18aH,24H-18, 15-(metheno)pyrido[2,1-l]pyrimido[6,1-h][1,4,7,9,10,13]benzoxapentaazacyclohexadecine-7, 24(6H)-dione;
(18aS)-13-(2-aminoethoxy)-2-ethyl-11-methyl-8,9,10,11,19,20,21,22-octahydro-18aH,24H-1 8,15-(metheno)pyrido[2,1-l]pyrimido[6,1-h][1,4,7,9,10,13]benzoxapentaazacyclohexadecine-7,24(6H)-dione;
(18aS)-13-(azetidin-1-yl)-2-methoxy-11-methyl-8,9,10,11,19,20,21,22-octahydro-18aH,24H-18,15-(metheno)pyrido[2,1-l]pyrimido[6,1-h][1,4,7,9,10,13]benzoxapentaazacyclohexadecin e-7,24(6H)-dione;
(18aS)-13-(3-hydroxyazetidin-1-yl)-11-methyl-2-trideuteriomethyl-8,9,10,11,19,20,21,22-oct ahydro-18aH,24H-18,15-(metheno)pyrido[2,1-l]pyrimido[6,1-h][1,4,7,9,10,13]benzoxapenta azacyclohexadecine-7,24(6H)-dione;
(19aS)-14-(3-aminoazetidin-1-yl)-2-fluoro-12-methyl-9,10,11,12,20,21,22,23-octahydro-6H, 19aH,25H-19,16-(metheno)pyrido[2,1-m]pyrimido[6,1-i][1,4,8,10,11,14]benzoxapentaazacy cloheptadecine-7,25(8H)-dione;
(18aS)-13-(azetidin-1-yl)-2-fluoro-11,12-dimethyl-8,9,10,11,19,20,21,22-octahydro-18aH,24 H-18,15-(metheno)pyrido[2,1-1]pyrimido[6,1-h][1,4,7,9,10,13]benzoxapentaazacyclohexadec ine-7,24(6H)-dione;
(18aS)-2-fluoro-13-(3-hydroxyazetidin-1-yl)-11,12-dimethyl-8,9,10,11,19,20,21,22-octahydr o-18aH,24H-18,15-(metheno)pyrido[2,1-l]pyrimido[6,1-h][1,4,7,9,10,13]benzoxapentaazacy clohexadecine-7,24(6H)-dione;
(18aS)-13-(3-aminoazetidin-1-yl)-2-fluoro-11,12-dimethyl-8,9,10,11,19,20,21,22-octahydro-18aH,24H-18,15-(metheno)pyrido[2,1-l]pyrimido[6,1-h][1,4,7,9,10,13]benzoxapentaazacycl ohexadecine-7,24(6H)-dione;
(18aS)-13-(2-aminoethoxy)-2-fluoro-11,12-dimethyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione;
(18aS)-13-(azetidin-1-yl)-2-fluoro-8,11,12-trimethyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione;
(18aS)-13-(3-aminoazetidin-1-yl)-2-fluoro-12-methyl-8,9,10,11,19,20,21,22-octahydro-18aH ,24H-18,15-(metheno)pyrido[2,1-l]pyrimido[6,1-h][1,4,7,9,10,13]benzoxapentaazacyclohexa decine-7,24(6H)-dione;
(18aS)-13-(3-aminoazetidin-1-yl)-2-chloro-11,12-dimethyl-8,9,10,11,19,20,21,22-octahydro-18aH,24H-18,15-(metheno)pyrido[2,1-l]pyrimido[6,1-h][1,4,7,9,10,13]benzoxapentaazacycl ohexadecine-7,24(6H)-dione;
(18aS)-2-chloro-13-(3-hydroxyazetidin-1-yl)-11,12-dimethyl-8,9,10,11,19,20,21,22-octahydr o-18aH,24H-18,15-(metheno)pyrido[2,1-l]pyrimido[6,1-h][1,4,7,9,10,13]benzoxapentaazacy clohexadecine-7,24(6H)-dione;
(18aS)-13-[(2-aminoethyl)amino]-2-chloro-11,12-dimethyl-8,9,10,11,19,20,21,22-octahydro-18aH,24H-18,15-(metheno)pyrido[2,1-l]pyrimido[6,1-h][1,4,7,9,10,13]benzoxapentaazacycl ohexadecine-7,24(6H)-dione;
(18aS)-13-[(2-aminoethyl)sulfanyl]-2-chloro-11,12-dimethyl-8,9,10,11,19,20,21,22-octahydr o-18aH,24H-18,15-(metheno)pyrido[2,1-l]pyrimido[6,1-h][1,4,7,9,10,13]benzoxapentaazacy clohexadecine-7,24(6H)-dione;
(18aS)-13-(2-aminoethoxy)-2-chloro-11,12-dimethyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione;
(18aS)-2-chloro-13-(3-hydroxypropyl)-11,12-dimethyl-8,9,10,11,19,20,21,22-octahydro-18a H,24H-18,15-(metheno)pyrido[2,1-l]pyrimido[6,1-h][1,4,7,9,10,13]benzoxapentaazacyclohe xadecine-7,24(6H)-dione;
(18aS)-13-(3-aminopropyl)-2-chloro-11,12-dimethyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione;
(18aS)-13-(2-aminoethyl)-2-chloro-11,12-dimethyl-8,9,10,11,19,20,21,22-octahydro-18aH,2 4H-18,15-(metheno)pyrido[2,1-l]pyrimido[6,1-h][1,4,7,9,10,13]benzoxapentaazacyclohexade cine-7,24(6H)-dione;
(18aS)-13-(4-aminobutyl)-2-chloro-11,12-dimethyl-8,9,10,11,19,20,21,22-octahydro-18aH,2 4H-18,15-(metheno)pyrido[2,1-l]pyrimido[6,1-h][1,4,7,9,10,13]benzoxapentaazacyclohexade cine-7,24(6H)-dione;
(18aS)-13-(3-aminoazetidin-1-yl)-2,11,12-trimethyl-8,9,10,11,19,20,21,22-octahydro-18aH,2 4H-18,15-(metheno)pyrido[2,1-l]pyrimido[6,1-h][1,4,7,9,10,13]benzoxapentaazacyclohexade cine-7,24(6H)-dione;
(18aS)-13-(2-aminoethoxy)-2,11,12-trimethyl-8,9,10,11,19,20,21,22-octahydro-18aH,24H-1 8,15-(metheno)pyrido[2,1-l]pyrimido[6,1-h][1,4,7,9,10,13]benzoxapentaazacyclohexadecine-7,24(6H)-dione;
(18aS)-13-(3-aminoazetidin-1-yl)-2,12-difluoro-11-methyl-8,9,10,11,19,20,21,22-octahydro-18aH,24H-18,15-(metheno)pyrido[2,1-l]pyrimido[6,1-h][1,4,7,9,10,13]benzoxapentaazacycl ohexadecine-7,24(6H)-dione;
(18aS)-2,12-difluoro-13-(3-hydroxyazetidin-1-yl)-11-methyl-8,9,10,11,19,20,21,22-octahydr o-18aH,24H-18,15-(metheno)pyrido[2,1-l]pyrimido[6,1-h][1,4,7,9,10,13]benzoxapentaazacy clohexadecine-7,24(6H)-dione;
(18aS)-13-(3-aminoazetidin-1-yl)-2-chloro-12-fluoro-11-methyl-8,9,10,11,19,20,21,22-octah ydro-18aH,24H-18,15-(metheno)pyrido[2,1-l]pyrimido[6,1-h][1,4,7,9,10,13]benzoxapentaaz acyclohexadecine-7,24(6H)-dione;
(18aS)-13-(3-aminoazetidin-1-yl)-12-chloro-2-fluoro-11-methyl-8,9,10,11,19,20,21,22-octah ydro-18aH,24H-18,15-(metheno)pyrido[2,1-l]pyrimido[6,1-h][1,4,7,9,10,13]benzoxapentaaz acyclohexadecine-7,24(6H)-dione;
(18aS)-13-cyclopropyl-2-fluoro-11-methyl-8,9,10,11,19,20,21,22-octahydro-18aH,24H-18,1 5-(metheno)pyrido[2,1-l]pyrimido[6,1-h][1,4,7,9,10,13]benzoxapentaazacyclohexadecine-7,2 4(6H)-dione;
(18aS)-13-cyclopropyl-11-ethyl-2-fluoro-8,9,10,11,19,20,21,22-octahydro-18aH,24H-18,15-( metheno)pyrido[2,1-l]pyrimido[6,1-h][1,4,7,9,10,13]benzoxapentaazacyclohexadecine-7,24( 6H)-dione;
(18aS)-13-cyclopropyl-2-fluoro-11-trideuteriomethyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione;
(18aS)-13-cyclopropyl-2-ethenyl-11-methyl-8,9,10,11,19,20,21,22-octahydro-18aH,24H-18, 15-(metheno)pyrido[2,1-l]pyrimido[6,1-h][1,4,7,9,10,13]benzoxapentaazacyclohexadecine-7, 24(6H)-dione;
(18aS)-13-cyclopropyl-2-iodo-11-methyl-8,9,10,11,19,20,21,22-octahydro-18aH,24H-18,15-(metheno)pyrido[2,1-l]pyrimido[6,1-h][1,4,7,9,10,13]benzoxapentaazacyclohexadecine-7,24( 6H)-dione;
(18aS)-13-cyclopropyl-11-methyl-7,24-dioxo-6,7,8,9,10,11,19,20,21,22-decahydro-18aH,24 H-18,15-(metheno)pyrido[2,1-l]pyrimido[6,1-h][1,4,7,9,10,13]benzoxapentaazacyclohexadec ine-2-carbonitrile;
(18aS)-2,13-dicyclopropyl-11-methyl-8,9,10,11,19,20,21,22-octahydro-18aH,24H-18,15-(me theno)pyrido[2,1-l]pyrimido[6,1-h][1,4,7,9,10,13]benzoxapentaazacyclohexadecine-7,24(6H) -dione;
(18aS)-13-cyclopropyl-11-methyl-2-trideuteriomethyl-8,9,10,11,19,20,21,22-octahydro-18a H,24H-18,15-(metheno)pyrido[2,1-l]pyrimido[6,1-h][1,4,7,9,10,13]benzoxapentaazacyclohe xadecine-7,24(6H)-dione; (18aS)-13-cyclopropyl-2,11-dimethyl-8,9,10,11,19,20,21,22-octahydro-18aH,24H-18,15-(me theno)pyrido[2,1-l]pyrimido[6,1-h][1,4,7,9,10,13]benzoxapentaazacyclohexadecine-7,24(6H) -dione;
(18aS)-2-chloro-13 -cyclopropyl-11-methyl-8,9,10,11,19,20,21,22-octahydro-18aH,24H-18,1 5-(metheno)pyrido[2,1-l]pyrimido[6,1-h][1,4,7,9,10,13]benzoxapentaazacyclohexadecine-7,2 4(6H)-dione;
(18S)-3-cyclopropyl-18-ethyl-14-fluoro-5,17-dimethyl-5,6,7,8,17,18-hexahydro-16H-19,1-( metheno)pyrimido[6,1-h][1,4,7,9,10,13]benzoxapentaazacyclohexadecine-9,16(10H)-dione; (18aS)-13-(trans-3-aminocyclobutyl)-2-fluoro-11-methyl-8,9,10,11,19,20,21,22-octahydro-1 8aH,24H-18,15-(metheno)pyrido[2,1-l]pyrimido[6,1-h][1,4,7,9,10,13]benzoxapentaazacyclo hexadecine-7,24(6H)-dione;
(18aS)-13-(cis-3-aminocyclobutyl)-2-fluoro-11-methyl-8,9,10,11,19,20,21,22-octahydro-18a H,24H-18,15-(metheno)pyrido[2,1-l]pyrimido[6,1-h][1,4,7,9,10,13]benzoxapentaazacyclohe xadecine-7,24(6H)-dione;
(18aS)-2-fluoro-13-(cis-3-hydroxycyclobutyl)-11-methyl-8,9,10,11,19,20,21,22-octahydro-1 8aH,24H-18,15-(metheno)pyrido[2,1-l]pyrimido[6,1-h][1,4,7,9,10,13]benzoxapentaazacyclo hexadecine-7,24(6H)-dione;
(18aS)-2-fluoro-13-(trans-3-hydroxycyclobutyl)-11-methyl-8,9,10,11,19,20,21,22-octahydro-18aH,24H-18,15-(metheno)pyrido[2,1-l]pyrimido[6,1-h][1,4,7,9,10,13]benzoxapentaazacycl ohexadecine-7,24(6H)-dione;
(18aS)-2-chloro-13-(cis-3-hydroxycyclobutyl)-11-methyl-8,9,10,11,19,20,21,22-octahydro-1 8aH,24H-18,15-(metheno)pyrido[2,1-l]pyrimido[6,1-h][1,4,7,9,10,13]benzoxapentaazacyclo hexadecine-7,24(6H)-dione;
(18aS)-2-chloro-13-(trans-3-hydroxycyclobutyl)-11-methyl-8,9,10,11,19,20,21,22-octahydro -18aH,24H-18,15-(metheno)pyrido[2,1-l]pyrimido[6,1-h][1,4,7,9,10,13]benzoxapentaazacycl ohexadecine-7,24(6H)-dione;
(18aS)-2-chloro-13-(cis-3-hydroxycyclobutyl)-11-trideuteriomethyl-8,9,10,11,19,20,21,22-oc tahydro-18aH,24H-18,15-(metheno)pyrido[2,1-l]pyrimido[6,1-h][1,4,7,9,10,13]benzoxapenta azacyclohexadecine-7,24(6H)-dione;
(18aS)-2-chloro-13-(trans-3-hydroxycyclobutyl)-11-trideuteriomethyl-8,9,10,11,19,20,21,22-octahydro-18aH,24H-18,15-(metheno)pyrido[2,1-l]pyrimido[6,1-h][1,4,7,9,10,13]benzoxape ntaazacyclohexadecine-7,24(6H)-dione;
(18aS)-13-(cis-3-hydroxycyclobutyl)-2,11-dimethyl-8,9,10,11,19,20,21,22-octahydro-18aH,2 4H-18,15-(metheno)pyrido[2,1-l]pyrimido[6,1-h][1,4,7,9,10,13]benzoxapentaazacyclohexade cine-7,24(6H)-dione;
(18aS)-13-(trans-3-hydroxycyclobutyl)-2,11-dimethyl-8,9,10,11,19,20,21,22-octahydro-18aH ,24H-18,15-(metheno)pyrido[2,1-l]pyrimido[6,1-h][1,4,7,9,10,13]benzoxapentaazacyclohexa decine-7,24(6H)-dione;
(18aS)-13-(cis-3-hydroxycyclobutyl)-11-methyl-2-trideuteriomethyl-8,9,10,11,19,20,21,22-o ctahydro-18aH,24H-18,15-(metheno)pyrido[2,1-l]pyrimido[6,1-h][1,4,7,9,10,13]benzoxapent aazacyclohexadecine-7,24(6H)-dione;
(18aS)-13-(trans-3-hydroxycyclobutyl)-11-methyl-2-trideuteriomethyl-8,9,10,11,19,20,21,22 -octahydro-18aH,24H-18,15-(metheno)pyrido[2,1-l]pyrimido[6,1-h] [1,4,7,9,10,13]benzoxap entaazacyclohexadecine-7,24(6H)-dione;
(18aS)-13-(cis-3-hydroxycyclobutyl)-2,11-bis[trideuteriomethyl]-8,9,10,11,19,20,21,22-octa hydro-18aH,24H-18,15-(metheno)pyrido[2,1-1]pyrimido[6,1-h][1,4,7,9,10,13]benzoxapentaa zacyclohexadecine-7,24(6H)-dione;
(18aS)-13-(trans-3-hydroxycyclobutyl)-2,11-bis[trideuteriomethyl]-8,9,10,11,19,20,21,22-oct ahydro-18aH,24H-18,15-(metheno)pyrido[2,1-1]pyrimido[6,1-h][1,4,7,9,10,13]benzoxapenta azacyclohexadecine-7,24(6H)-dione;
(18aS)-13-cyclopropyl-2-fluoro-11-methyl-8,9,10,11,19,20,21,22-octahydro-18aH,24H-18,1 5-(metheno)pyrido[2,1-1]pyrimido[6,1-h][1,4,9,10,13]benzoxatetraazacyclohexadecine-7,24( 6H)-dione;
(18aS)-2-chloro-13-cyclopropyl-11-methyl-8,9,10,11,19,20,21,22-octahydro-18aH,24H-18,1 5-(metheno)pyrido[2,1-1]pyrimido[6,1-h][1,4,9,10,13]benzoxatetraazacyclohexadecine-7,24( 6H)-dione;
(18aS)-2-chloro-13-(3-hydroxyazetidin-1-yl)-11-methyl-8,9,10,11,19,20,21,22-octahydro-18 aH,24H-18,15-(metheno)pyrido[2,1-l]pyrimido[6,1-h][1,4,9,10,13]benzoxatetraazacyclohexa decine-7,24(6H)-dione;
(18aS)-13-cyclopropyl-2,11-dimethyl-8,9,10,11,19,20,21,22-octahydro-18aH,24H-18,15-(me theno)pyrido[2,1-1]pyrimido[6,1-h][1,4,9,10,13]benzoxatetraazacyclohexadecine-7,24(6H)-di one;
(18aS)-13-(3-hydroxyazetidin-1-yl)-2,11-dimethyl-8,9,10,11,19,20,21,22-octahydro-18aH,24 H-18,15-(metheno)pyrido[2,1-l]pyrimido[6,1-h][1,4,9,10,13]benzoxatetraazacyclohexadecin e-7,24(6H)-dione;
(18aS)-13-(3-hydroxyazetidin-1-yl)-11-methyl-2-trideuteriomethyl-8,9,10,11,19,20,21,22-oct ahydro-18aH,24H-18,15-(metheno)pyrido[2,1-1]pyrimido[6,1-h][1,4,9,10,13]benzoxatetraaza cyclohexadecine-7,24(6H)-dione;
(18aS)-13-(azetidin-1-yl)-2-fluoro-11-methyl-6,7,8,9,10,11,19,20,21,22-decahydro-18aH,24 H-18,15-(metheno)pyrido[2,1-l]pyrimido[6,1-h][1,4,7,9,10,13]benzoxapentaazacyclohexadec in-24-one;
(18aS)-13-(azetidin-1-yl)-2-chloro-8,11-dimethyl-6,7,8,9,10,11,19,20,21,22-decahydro-18aH ,24H-18,15-(metheno)pyrido[2,1-l]pyrimido[6,1-h][1,4,7,9,10,13]benzoxapentaazacyclohexa decin-24-one;
(18aS)-13-(azetidin-1-yl)-2,11-dimethyl-6,7,8,9,10,11,19,20,21,22-decahydro-18aH,24H-18, 15-(metheno)pyrido[2,1-l]pyrimido[6,1-h][1,4,7,9,10,13]benzoxapentaazacyclohexadecin-24 -one;
(18aS)-2,11-dimethyl-13-(pyrrolidin-1-yl)-6,7,8,9,10,11,19,20,21,22-decahydro-18aH,24H-1 8,15-(metheno)pyrido[2,1-l]pyrimido[6,1-h][1,4,7,9,10,13]benzoxapentaazacyclohexadecin-2 4-one;
(18aS)-13-(azetidin-1-yl)-2,8,11-trimethyl-6,7,8,9,10,11,19,20,21,22-decahydro-18aH,24H-1 8,15-(metheno)pyrido[2,1-l]pyrimido[6,1-h][1,4,7,9,10,13]benzoxapentaazacyclohexadecin-2 4-one;
(18aS)-13-(azetidin-1-yl)-8-ethyl-2,11-dimethyl-6,7,8,9,10,11,19,20,21,22-decahydro-18aH, 24H-18,15-(metheno)pyrido[2,1-l]pyrimido[6,1-h][1,4,7,9,10,13]benzoxapentaazacyclohexa decin-24-one;
(18aS)-13-(azetidin-1-yl)-8-(2-hydroxyethyl)-2,11-dimethyl-6, 7,8,9,10,11,19,20,21,22-decah ydro-18aH,24H-18,15-(metheno)pyrido[2,1-l]pyrimido[6,1-h][1,4,7,9,10,13]benzoxapentaaz acyclohexadecin-24-one;
(18aS)-13-cyclopropyl-2,11-dimethyl-6H,8H,9H,10H,11H,18aH,19H,20H,21H,22H,24H-spi ro[18,15-(metheno)pyrido[2,1-l]pyrimido[6,1-h][1,4,7,9,10,13]benzoxapentaazacyclohexade cine-7,3'-oxetan]-24-one;
(18aS)-13-(azetidin-1-yl)-2,12-difluoro-11-methyl-6,7,8,9,10,11,19,20,21,22-decahydro-18a H,24H-18,15-(metheno)pyrido[2,1-l]pyrimido[6,1-h][1,4,7,9,10,13]benzoxapentaazacyclohe xadecin-24-one;
(17S)-3-[(3S)-3-aminopyrrolidin-1-yl]-13-chloro-17-ethyl-5,16-dimethyl-6,7,8,9,16,17-hexah ydro-5H,15H-18,1-(metheno)pyrimido[6,1-g][1,6,8,9,12]benzoxatetraazacyclopentadecin-15 -one;
(17S)-3-[(3S)-3-aminopyrrolidin-1-yl]-13-chloro-5,17-diethyl-16-methyl-6,7,8,9,16,17-hexah ydro-5H, 15H-18,1-(metheno)pyrimido[6,1-g][1,6,8,9,12]benzoxatetraazacyclopentadecin-15 -one;
(17S)-3-[(3S)-3-aminopyrrolidin-1-yl]-17-ethyl-5,13,16-trimethyl-6,7,8,9,16,17-hexahydro-5 H,15H-18,1-(metheno)pyrimido[6,1-g][1,6,8,9,12]benzoxatetraazacyclopentadecin-15-one;
(17S)-3-[(2-aminoethyl)amino]-17-ethyl-5,13,16-trimethyl-6,7,8,9,16,17-hexahydro-5H,15H-18,1-(metheno)pyrimido[6,1-g][1,6,8,9,12]benzoxatetraazacyclopentadecin-15-one;
(17S)-3-(3-aminopropyl)-17-ethyl-5,13,16-trimethyl-6,7,8,9,16,17-hexahydro-5H,15H-18,1-( metheno)pyrimido[6,1-g][1,6,8,9,12]benzoxatetraazacyclopentadecin-15-one;
(17S)-3-[(3S)-3-aminopyrrolidin-1-yl]-17-ethyl-5,13,16-trimethyl-5,6,7,8,9,10,16,17-octahyd ro-15H-18,1-(metheno)pyrimido[6,1-g][1,6,8,9,12]benzopentaazacyclopentadecin-15-one;
(17S)-3-[(3S)-3-aminopyrrolidin-1-yl]-17-ethyl-4,13,16-trimethyl-6,7,8,9,16,17-hexahydro-5 H, 15H-18,1-(metheno)pyrimido[6,1-g][1,6,8,9,12]benzoxatetraazacyclopentadecin-15-one;
(17S)-3-[(3S)-3-aminopyrrolidin-1-yl]-17-ethyl-4,5,13,16-tetramethyl-5,6,7,8,9,10,16,17-oct ahydro-15H-18,1-(metheno)pyrimido[6,1-g][1,6,8,9,12]benzopentaazacyclopentadecin-15-on e;
(23aS)-18-(3-aminoazetidin-1-yl)-8,16-dimethyl-1,3,4,13,14,15,16,23a-octahydro-2H,6H,12 H-23,20-(metheno)pyrido[2,1-k]pyrimido[6,1-g][1,6,8,9,12]benzoxatetraazacyclopentadecin-6-one;
(23aS)-18-(3-hydroxyazetidin-1-yl)-8,16-dimethyl-1,3,4,13,14,15,16,23a-octahydro-2H,6H,1 2H-23,20-(metheno)pyrido[2,1-k]pyrimido[6,1-g][1,6,8,9,12]benzoxatetraazacyclopentadeci n-6-one;
(23aS)-18-[(3S)-3-aminopyrrolidin-1-yl]-7-chloro-16-methyl-1,3,4,13,14,15,16,23a-octahydr o-2H,6H,12H-23,20-(metheno)pyrido[2,1-k]pyrimido[6,1-g][1,6,8,9,12]benzoxatetraazacycl opentadecin-6-one;
(23aS)-18-[(3S)-3-aminopyrrolidin-1-yl]-7-fluoro-16-methyl-1,3,4,13,14,15,16,23a-octahydr o-2H,6H,12H-23,20-(metheno)pyrido[2,1-k]pyrimido[6,1-g][1,6,8,9,12]benzoxatetraazacycl opentadecin-6-one;
(23aS)-18-(3-aminopropyl)-8-chloro-16-methyl-1,3,4,13,14,15,16,23a-octahydro-2H,6H,12H -23,20-(metheno)pyrido[2,1-k]pyrimido[6,1-g][1,6,8,9,12]benzoxatetraazacyclopentadecin-6 -one;
(23aS)-18-[(3S)-3-aminopyrrolidin-1-yl]-8-fluoro-16-methyl-1,3,4,13,14,15,16,23a-octahydr o-2H,6H,12H-23,20-(metheno)pyrido[2,1-k]pyrimido[6,1-g][1,6,8,9,12]benzoxatetraazacycl opentadecin-6-one;
(23aS)-18-(3-aminoazetidin-1-yl)-8-fluoro-16-methyl-1,3,4,13,14,15,16,23a-octahydro-2H,6 H,12H-23,20-(metheno)pyrido[2,1-k]pyrimido[6,1-g][1,6,8,9,12]benzoxatetraazacyclopentad ecin-6-one;
(23aS)-18-[(3S)-3-aminopyrrolidin-1-yl]-9-fluoro-16-methyl-1,3,4,13,14,15,16,23a-octahydr o-2H,6H,12H-23,20-(metheno)pyrido[2,1-k]pyrimido[6,1-g][1,6,8,9,12]benzoxatetraazacycl opentadecin-6-one;
(23aS)-18-[(3S)-3-aminopyrrolidin-1-yl]-16-methyl-1,3,4,13,14,15,16,23a-octahydro-2H,6H, 12H-23,20-(metheno)pyrido[2,1-k]pyrimido[6,1-g][1,6,8,9,12]benzoxatetraazacyclopentadec in-6-one;
(23aS)-18-(azetidin-1-yl)-8-chloro-15-methyl-1,3,4,13,14,15,16,23a-octahydro-2H,6H,12H-2 3,20-(metheno)pyrido[2,1-k]pyrimido[6,1-g][1,5,8,9,12]benzoxatetraazacyclopentadecin-6-o ne;
(17S)-3-[(3S)-3-aminopyrrolidin-1-yl]-17-ethyl-4,13,16-trimethyl-7,8,16,17-tetrahydro-5H-1 8,1-(metheno)-9λ⁶-pyrimido[6,1-g][2,1,6,8,9,12]benzothiapentaazacyclopentadecine-9,9,15( 6H,10H)-trione;
(17S)-3-[(2-aminoethyl)amino]-17-ethyl-4,13,16-trimethyl-7,8,16,17-tetrahydro-5H-18,1-(m etheno)-9λ⁶-pyrimido[6,1-g][2,1,6,8,9,12]benzothiapentaazacyclopentadecine-9,9,15(6H,10 H)-trione;
(23aS)-18-[(2-aminoethyl)amino]-8-chloro-16,17-dimethyl-l,3,4,13,14,15,16,23a-octahydro-2H,6H-23,20-(metheno)-12λ⁶-pyrido[2,1-k]pyrimido[6,1-g][2,1,6,8,9,12]benzothiapentaazac yclopentadecine-6,12,12(11H)-trione;
(20aS)-15-(azetidin-1-yl)-2-chloro-9,13-dimethyl-12,13,21,22,23,24-hexahydro-6H,11H,20a H,26H-20,17-(metheno)pyrido[2,1-l]pyrimido[6,1-h][1,2,4]triazolo[3,4-c][1,4,7,9,10,13]benz oxapentaazacyclohexadecin-26-one;
(20aS)-15-(azetidin-1-yl)-2-chloro-13-methyl-12,13,21,22,23,24-hexahydro-6H, 11H,20aH,2 6H-20,17-(metheno)imidazo[2,1-c]pyrido[2,1-l]pyrimido[6,1-h][1,4,7,9,10,13]benzoxapenta azacyclohexadecin-26-one;
(18aS)-13-(3-aminoazetidin-1-yl)-2-chloro-11-methyl-7-(trifluoromethyl)-6,7,8,9,10,11,19,2 0,21,22-decahydro-18aH,24H-18,15-(metheno)pyrido[2,1-l]pyrimido[6,1-h][1,4,7,9,10,13]be nzoxapentaazacyclohexadecin-24-one;
(18aS)-2-chloro-13-(3-hydroxyazetidin-1-yl)-11-methyl-7-(trifluoromethyl)-6,7,8,9,10,11,19, 20,21,22-decahydro-18aH,24H-18,15-(metheno)pyrido[2,1-l]pyrimido[6,1-h][1,4,7,9,10,13]b enzoxapentaazacyclohexadecin-24-one;
(18aS)-13-(3-hydroxyazetidin-1-yl)-2,11-dimethyl-7-(trifluoromethyl)-6,7,8,9,10,11,19,20,21 ,22-decahydro-18aH,24H-18,15-(metheno)pyrido[2,1-l]pyrimido[6,1-h][1,4,7,9,10,13]benzo xapentaazacyclohexadecin-24-one;
(18aS)-13-[(3 S)-3-aminopyrrolidin-1-yl]-2,11-dimethyl-7-(trifluoromethyl)-6,7,8,9,10,11,19, 20,21,22-decahydro-18aH,24H-18,15-(metheno)pyrido[2,1-l]pyrimido[6,1-h][1,4,7,9,10,13]b enzoxapentaazacyclohexadecin-24-one;
(18aS)-13-(3-hydroxyazetidin-1-yl)-2,11-dimethyl-9-(trifluoromethyl)-6,7,8,9,10,11,19,20,21 ,22-decahydro-18aH,24H-18,15-(metheno)pyrido[2,1-l]pyrimido[6,1-h][1,4,7,9,10,13]benzo xapentaazacyclohexadecin-24-one;
(18aS)-3-(azetidin-1-yl)-5,12-dimethyl-12-phenyl-6,7,8,9,11,12,16,17,18,18a-decahydro-5H, 15H-19,1-(metheno)-10λ⁶-pyrido[1,2-e]pyrimido[1,6-i][1,2,5,8,9,11]thiapentaazacyclopentad ecine-10,10,13 -trione;
(18aS)-3-[(3 S)-3-aminopyrrolidin-1-yl]-5,12-dimethyl-12-phenyl-6,7,8,9,11,12,16,17,18,18a-decahydro-5H,15H-19,1-(metheno)-10λ⁶-pyrido[1,2-e]pyrimido[1,6-i][1,2,5,8,9,11]thiapenta azacyclopentadecine-10,10,13-trione; and
(18a'S)-3'-[(3S)-3-aminopyrrolidin-1-yl]-5'-methyl-2H,3H,5'H,6'H,7'H,8'H,9'H,10'H,11'H,13' H, 15'H, 16'H, 17'H, 18'H, 18a'H-spiro[indene-1,12'-[19,1](metheno)[10λ⁶]pyrido[1,2-e]pyrimid o[1,6-i][1,2,5,8,9,11]thiapentaazacyclopentadecine]-10',10',13'-trione,
or pharmaceutically acceptable salts thereof.

In the present inventive compound (I), the steric configuration at the carbon atom to which R⁴ and R⁵ are bonded is fixed. However, stereoisomers such as optical isomers and rotational isomers may exist in the present inventive compound (I) due to moieties other than that carbon atom, and the scope of the present inventive compound includes all of such isomers. Also, single isomers among them, as well as mixtures thereof, are included in the scope of the compound of the present invention. In addition, when the present inventive compound (I) or salts thereof form hydrates or solvates, these are also included within the scope of the present invention. Similarly, pharmaceutically acceptable salts of the hydrates or solvates of the present inventive compound are also included within the scope of the present invention. Furthermore, the present inventive compound (I) may be or may not be labeled with isotopes (for example, D, ³H, ¹³C, ¹⁴C, ¹⁵N, ³⁵S, ¹²⁵I, and the like), unless otherwise noted.

By formulating the present inventive compound (I) or a pharmaceutically acceptable salt as it is or together with a pharmacologically acceptable carrier according to a means known per se, a medicament is produced. Examples of the pharmacologically acceptable carrier include a variety of organic or inorganic carrier substances commonly used as formulation materials, such as excipients (for example, milk sugar, white sugar, D-mannitol, starch, cornstarch, crystal cellulose, light anhydrous silica acid, and the like), lubricants (for example, magnesium stearate, calcium stearate, talc, colloidal silica, and the like), binders (for example, crystal cellulose, white sugar, D-mannitol, dextrin, hydroxypropyl cellulose, hydroxypropyl methylcellulose, polyvinylpyrrolidone, starch, sucrose, gelatin, methylcellulose, sodium carboxymethylcellulose, and the like), and disintegrating agents (for example, starch, carboxymethylcellulose, calcium carboxymethylcellulose, sodium croscarmellose, sodium carboxymethyl starch, low substituted hydroxypropyl cellulose, and the like) in solid formulations; and solvents (for example, water for injection, alcohols, propylene glycol, macrogol, sesame oil, corn oil, and the like), dissolving aids (for example, polyethylene glycol, propylene glycol, D-mannitol, benzyl benzoate, ethanol, trisaminomethane, cholesterol, triethanolamine, sodium carbonate, sodium citrate, and the like), suspending agents (for example, surfactants such as stearyltriethanolamine, sodium lauryl sulfate, lauryl aminopropionate, lecithin, benzalkonium chloride, benzethonium chloride, and glyceryl monostearate, or hydrophilic polymers such as polyvinyl alcohol, polyvinylpyrrolidone, sodium carboxymethylcellulose, methylcellulose, hydroxymethylcellulose, hydroxypropylcellulose, and the like), isotonizing agents (for example, dextrose, D-sorbitol, sodium chloride, glycerin, D-mannitol, and the like), buffering agents (for example, phosphate, acetate, carbonate, citrate, and the like), and analgesic agents (for example, benzyl alcohol and the like) in liquid formulations. In addition, upon the formulation, it is also possible to use, if required, preservatives (for example, paraoxybenzoate esters, chlorobutanol, benzyl alcohol, phenethyl alcohol, dehydroacetic acid, sorbic acid, and the like), antioxidants (for example, sulfites, ascorbic acid, and the like), coloring agents, sweetening agents, adsorbents, wetting agents, and the like.

The present inventive compound (I) or a pharmaceutically acceptable salt can be administered orally or parenterally (for example, intravenously, topically, rectally, or the like). Its dosage form is, for example, a tablet (including a sugar coated tablet and a film coated tablet), a pulvis, a granule, a powder, a troche, a capsule (including a soft capsule), a liquid, an injection (for example, a subcutaneous injection, an intravenous injection, an intramuscular injection, an intraperitoneal injection, or the like), an external preparation (for example, a formulation for transnasal administration, a transdermal formulation, an ointment, a cream, or the like), a suppository (for example, a rectal suppository, a vaginal suppository, or the like), a sustained release preparation (for example, a sustained release microcapsule or the like), a pellet, a drip, or the like, and all of them can be produced by commonly used production technologies (for example, methods described in the Japanese Pharmacopoeia, 16th edition and the like).

The dosage of the present inventive compound (I) or a pharmaceutically acceptable salt is selected as appropriate according to the target of administration, route of administration, disease, age, weight, and symptoms of the patient. For example, when treating adult patients, the dosage is 1 to 5,000 mg per day, and this amount is administered once a day or in several divided doses.

The present inventive compound (I) and a pharmaceutically acceptable salt thereof can be synthesized using a combination of various organic synthesis approaches that are publicly known to those skilled in the art. For example, production methods are shown below, but the production methods for the compound of the present invention are not limited to the following synthesis methods. Also, in the following reaction formulas, R¹, R², R³, R⁴, R⁵, R⁶, R^{X12}, X¹, X², Y, and L have the same meanings as described above.

Although all of the compounds of the present invention are novel compounds that have not yet been described in publications, they can be produced by publicly known methods described in publications or by methods similar to them. Examples of such publications include, for example, Organic Functional Group Preparations, 1968, authored by S. R. Sandler et al., Academic Press INC.; Synthetic Organic Chemistry, 1961, authored by S. R. Wagner et al., John Wiley INC.; Comprehensive Organic Transformations, 1989, authored by R. C. Larock, VCH Publishers, INC.; Encyclopedia of Reagents for Organic Synthesis, 1995, authored by L. A. Paquette et al., Wiley INC.; and Compendium of Organic Synthetic Methods, 12th edition, 2009, authored by Michael B. Smith, Wiley INC.

The term "inert solvent" refers to, for example, an aromatic solvent such as benzene, toluene, xylene, and pyridine; a hydrocarbon solvent such as hexane, pentane, and cyclohexane; a halogenated hydrocarbon solvent such as dichloromethane, chloroform, 1,2-dichloroethane, and carbon tetrachloride; an ether solvent such as tetrahydrofuran, diethyl ether, 1,2-dimethoxyethane, and 1,4-dioxane; an ester solvent such as ethyl acetate and ethyl formate; an alcohol solvent such as methanol, ethanol, isopropyl alcohol, tert-butyl alcohol, and ethylene glycol; a ketone solvent such as acetone and methyl ethyl ketone; an amide solvent such as N,N-dimethylformamide, N-methylpyrrolidone, and N,N-dimethylacetamide; a sulfoxide solvent such as dimethyl sulfoxide; a nitrile solvent such as acetonitrile and propionitrile; and water, as well as their homogeneous and heterogeneous mixed solvent, but is not limited to them. These inert solvents are selected as appropriate according to the various reaction conditions that are publicly known to those skilled in the art.

The term "base" refers to, for example, an alkali metal or alkaline earth metal hydride such as lithium hydride, sodium hydride, potassium hydride, and calcium hydride; an alkali metal or alkaline earth metal amide such as lithium amide, sodium amide, lithium diisopropylamide, lithium dicyclohexylamide, lithium hexamethyldisilazide, sodium hexamethyldisilazide, and potassium hexamethyldisilazide; a lower alkoxide of an alkali metal or alkaline earth metal such as sodium methoxide, sodium ethoxide, and potassium tert-butoxide; an alkyllithium such as butyllithium, sec-butyllithium, tert-butyllithium, and methyllithium; a hydroxide of an alkali metal or alkaline earth metal such as sodium hydroxide, potassium hydroxide, lithium hydroxide, and barium hydroxide; a carbonate of an alkali metal or alkaline earth metal such as sodium carbonate, potassium carbonate, and cesium carbonate; a hydrogen carbonate of an alkali metal or alkaline earth metal such as sodium hydrogen carbonate and potassium hydrogen carbonate; an amine such as triethylamine, N-methylmorpholine, N,N-diisopropylethylamine,
1,8-diazabicyclo[5.4.0]undec-7-ene (DBU), 1,5-diazabicyclo[4.3.0]non-5-ene (DBN), and N,N-dimethylaniline; a basic heterocyclic compound such as pyridine, imidazole, and 2,6-lutidine; and the like, but is not limited to them. These bases are selected as appropriate according to the various reaction conditions that are publicly known to those skilled in the art.

The term "acid" refers to, for example, an inorganic acid such as hydrochloric acid, hydrobromic acid, sulfuric acid, nitric acid, and phosphoric acid; and an organic acid such as p-toluenesulfonic acid, methanesulfonic acid, trifluoroacetic acid, formic acid, and acetic acid, but is not limited to them. These acids are selected as appropriate according to the various reaction conditions that are publicly known to those skilled in the art.

### [Production Method 1]

The present inventive compound (I) can be produced by the method shown in Scheme 1.

When X¹ of the inventive compound (I) is NR^{X11}, the inventive compound (I) can be produced by performing Step I-1 to Step 1-7.

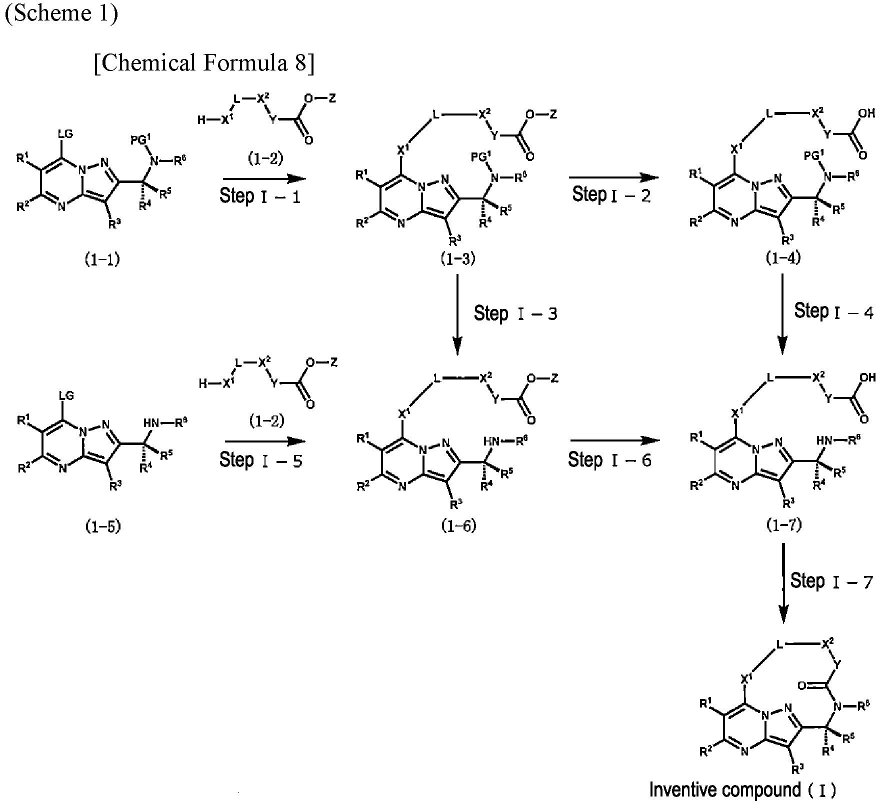

In the above formulas:
LG represents a leaving group such as a halogen atom or a trifluoromethanesulfonyloxy group;
PG¹ represents a protecting group for the amino group; and
Z represents a protecting group for the carboxy group such as a C₁₋₄ alkyl group and a benzyl group.

Step 1-1: In an inert solvent, Compound (1-2) is allowed to act on Compound (1-1) in the presence or absence of a base such as triethylamine, thereby affording Compound (1-3). Alternatively, in an inert solvent, Compound (1-1) is subjected to a coupling reaction with Organometallic Compound (1-2) in the presence or absence of a base, in the presence of a transition metal catalyst, and using a ligand if required, thereby affording Compound (1-3). Here, as for the coupling reaction, mention may be made of the coupling reaction conditions that are publicly known to those skilled in the art, and it can be performed by, for example, methods described in Comprehensive Organic Transformations, 1989, authored by R. C. Larock, VCH Publishers, INC. and the like, methods conforming thereto, or a combination of these methods. Here, examples of the transition metal catalyst include, for example, palladium(II) acetate, palladium(II) chloride, palladium(II) bis(triphenylphosphine)acetate, palladium(II) bis(triphenylphosphine)chloride, tris(dibenzylideneacetone)dipalladium(0), bis(dibenzylideneacetone)palladium(0), tetrakistriphenylphosphine palladium(0), [1,1'-bis(diphenylphosphino)ferrocene]palladium(II) chloride, allylpalladium(II) chloride, bis(acetonitrile)palladium(II) chloride,
[1,3-bis(2,6-diisopropylphenyl)imidazol-2-ylidene](3-chloropyridyl)palladium(II) dichloride or [1,3-bis(2,6-diisopropylphenyl)imidazolidene](3-chloropyridyl)palladium(II) dichloride, [1,1'-bis(diphenylphosphino)ferrocene]palladium(II) dichloride dichloromethane complex (1:1), copper powder, copper(I) chloride, copper(I) bromide, copper(I) iodide, and copper(I) acetate. Examples of the ligand include, for example, triphenylphosphine, tributylphosphine, tri(2-furyl)phosphine, 2,2-bis(diphenylphosphino)-1,1-binaphthyl (BINAP), 2-(di-tert-butylphosphino)biphenyl, 1,1-bis(diphenylphosphino)ferrocene, 1,10-phenanthroline, N,N'-dimethylethylenediamine, or tetramethylethylenediamine.

Step 1-2: Compound (1-3) can be converted into Compound (1-4) by various hydrolysis reactions that are publicly known to those skilled in the art [see Protective Groups in Organic Synthesis, 4th Edition, 2006, authored by T. W. Green et al., Wiley INC.].

Step 1-3: In an inert solvent, the protecting group PG¹ for the amino group of Compound (1-3) can be removed by using various organic synthesis methods that are publicly known to those skilled in the art [see Protective Groups in Organic Synthesis, 4th Edition, 2006, authored by T. W. Green et al., Wiley INC.], thereby affording Compound (1-6).

Step 1-4: Compound (1-4) can be converted into Compound (1-7) by the same approach as in Step 1-3 described above.

Step 1-5: Compound (1-5) can be converted into Compound (1-6) by the same approach as in Step I-1 described above.

Step 1-6: Compound (1-6) can be converted into Compound (1-7) by the same approach as in Step 1-2 described above.

Step 1-7: In an inert solvent, Compound (1-7) is subjected to an amidation reaction in the presence or absence of a base, thereby affording the present inventive compound (I).

Here, the amidation reaction refers to: an amidation reaction using a condensing agent such as HATU, N,N'-dicyclohexylcarbodiimide, 1-ethyl-3-(3-dimethylaminopropyl)carbodiimide hydrochloride, diphenylphosphoryl azide, or carbonyldiimidazole in the presence or absence of a base in an inert solvent; an amidation reaction via a mixed acid anhydride using ethyl chlorocarbonate, isobutyl chlorocarbonate, pivaloyl chloride, or the like [see Fundamentals and Experiments of Peptide Synthesis (1995, authored by Michinori Waki et al., Maruzen Co., Ltd.)]; or the like. Here, during the amidation reaction using a condensing agent, an additive agent such as 1-hydroxybenzotriazole can be used, if required.

### [Production Method 2]

The present inventive compound (I) can be produced by the method shown in Scheme 2.

When X¹ of the inventive compound (I) is NR^{X11}, the present inventive compound (I) can be produced by performing Step II-1 to Step II-3.

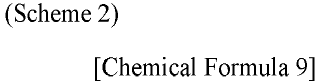

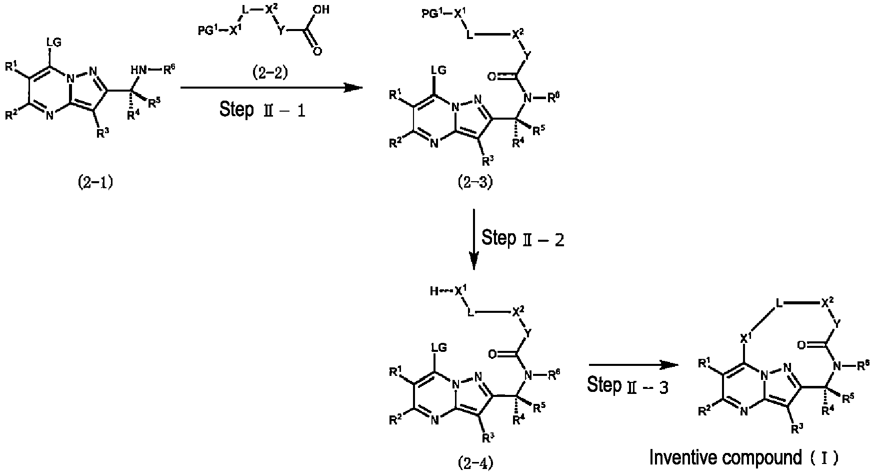

In the above formulas:
LG represents a leaving group such as a halogen atom or a trifluoromethanesulfonyloxy group; and
PG¹ represents a protecting group for the amino group.

Step II-1: In an inert solvent, Compound (2-1) and Carboxylic Acid Compound (2-2) are subjected to an amidation reaction in the presence or absence of a base, thereby affording Compound (2-3). Here, the amidation reaction refers to: an amidation reaction using a condensing agent such as HATU, N,N'-dicyclohexylcarbodiimide, 1-ethyl-3-(3-dimethylaminopropyl)carbodiimide hydrochloride, diphenylphosphoryl azide, or carbonyldiimidazole in the presence or absence of a base in an inert solvent; an amidation reaction via a mixed acid anhydride using ethyl chlorocarbonate, isobutyl chlorocarbonate, pivaloyl chloride, or the like [see Fundamentals and Experiments of Peptide Synthesis (1995, authored by Michinori Waki et al., Maruzen Co., Ltd.)]; or the like. Here, during the amidation reaction using a condensing agent, an additive agent such as 1-hydroxybenzotriazole can be used, if required.

Step II-2: Compound (2-3) can be converted into Compound (2-4) by the same approach as in Step 1-3 described above.

Step II-3: By the same approach as in Step I-1 described above, the present inventive compound (I) can be obtained from Compound (2-4).

### [Production Method 3]

The present inventive compound (I) can also be produced by the method shown in Scheme 3.

When X¹ of the inventive compound (I) is NR^{X11}, X² is O, and Y is phenylene, then the present inventive compound (I) can be produced by performing Step III-1 to Step III-4.

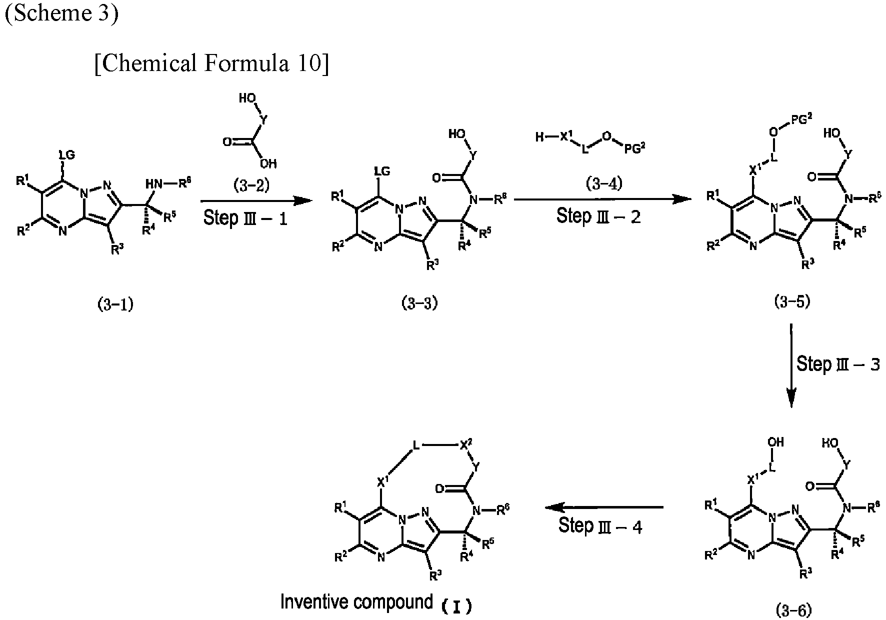

In the above formulas:
LG represents a leaving group such as a halogen atom or a trifluoromethanesulfonyloxy group; and
PG² represents a protecting group for the hydroxy group.

Step III-1: By the same approach as in Step II-1 described above, Compound (3-1) and Carboxylic Acid Compound (3-2) can be converted into Compound (3-3) by subjecting them to an amidation reaction.

Step III-2: By the same approach as in Step I-1 described above, Compound (3-3) can be converted into Compound (3-5) by allowing Compound (3-4) to act on Compound (3-3).

Step III-3: In an inert solvent, the protecting group PG² for the hydroxy group of Compound (3-5) can be removed by using various organic synthesis methods that are publicly known to those skilled in the art [see Protective Groups in Organic Synthesis, 4th Edition, 2006, authored by T. W. Green et al., Wiley INC.], thereby affording Compound (3-6).

Step III-4: In an inert solvent, by carrying out an intramolecular Mitsunobu reaction of Compound (3-6) in the presence of a dialkyl azodicarboxylate and a trialkyl- or triaryl-phosphine, the present inventive compound (I) can be obtained.

### [Production Method 4]

The present inventive compound (I) can also be produced by the method shown in Scheme 4.

When X¹-L-X² of the inventive compound (I) is N(R^{X11})-CH₂CH₂NHCO-CH₂, the inventive compound (I) can be produced by performing Step IV-1 to Step IV-5.

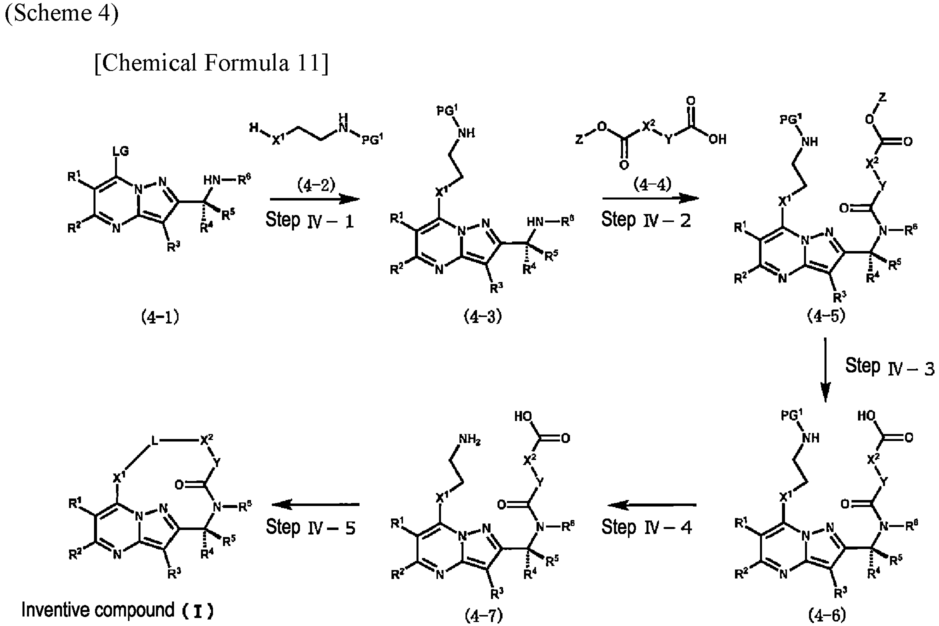

In the above formulas:
LG represents a leaving group such as a halogen atom or a methanesulfonyloxy group;
PG¹ represents a protecting group for the amino group; and
Z represents a protecting group for the carboxy group such as a C₁₋₄ alkyl group and a benzyl group.

Step IV-1: By the same approach as in Step I-1 described above, Compound (4-1) can be converted into Compound (4-3) by allowing Amine Compound (4-2) to act on Compound (4-1).

Step IV-2: By the same approach as in Step III-1 described above, Compound (4-3) and Carboxylic Acid Compound (4-4) can be converted into Compound (4-5) by subjecting them to an amidation reaction.

Step IV-3: Compound (4-5) can be converted into Compound (4-6) by the same approach as in Step 1-2 described above.

Step IV-4: Compound (4-6) can be converted into Compound (4-7) by the same approach as in Step 1-3 described above.

Step IV-5: By the same approach as in Step 1-7 described above, the present inventive compound (I) can be obtained from Compound (4-7).

### [Production Method 5]

The present inventive compound (I) can also be produced by the method shown in Scheme 5.

When X¹-L-X² of the inventive compound (I) is CH(R^{X12})-CH₂CH₂NHCOCH₂-O, the present inventive compound (I) can be produced by performing Step V-1 to Step V-13.

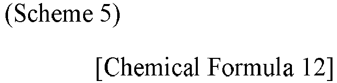

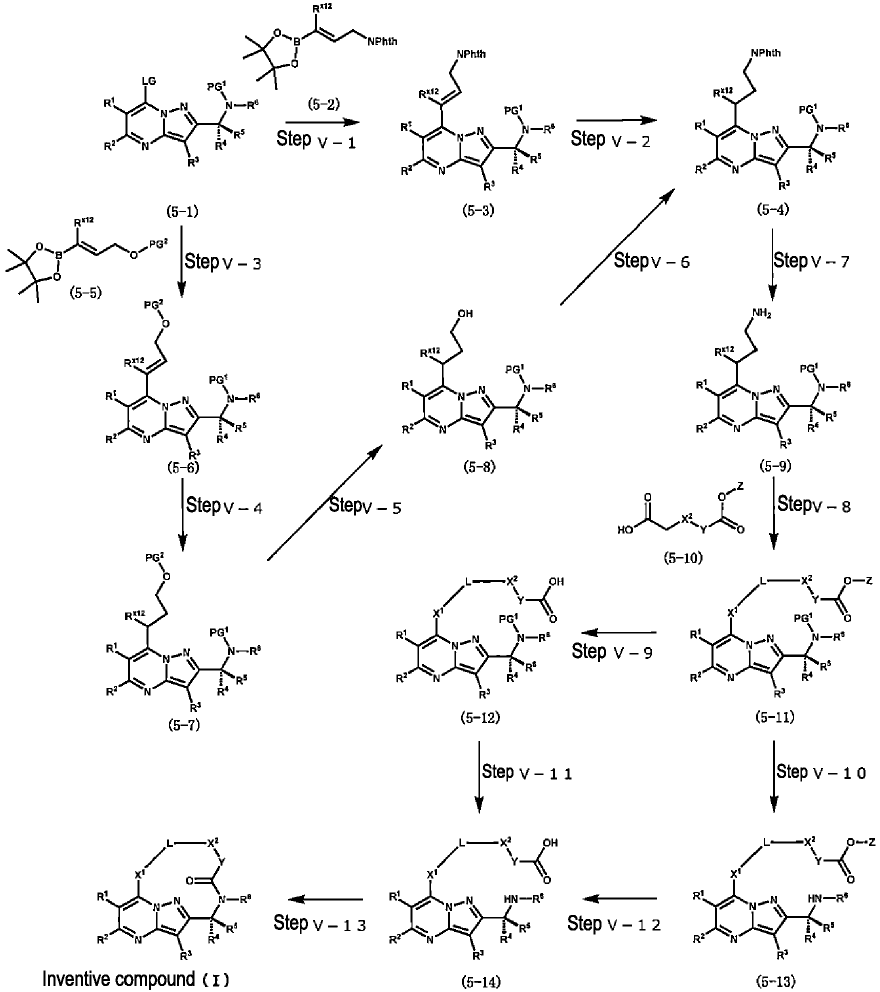

In the above formulas:
LG represents a leaving group such as a halogen atom or a trifluoromethanesulfonyloxy group;
PG¹ represents a protecting group for the amino group;
PG² represents a protecting group for the hydroxy group;
Phth represents a phthaloyl group; and
Z represents a protecting group for the carboxy group such as a C₁₋₄ alkyl group and a benzyl group.

Step V-1: In an inert solvent, Compound (5-1) can be converted into Compound (5-3) by subjecting it to a Suzuki-Miyaura coupling reaction with Organic Boron Compound (5-2) such as an organic boronate ester and an organic boronic acid in the presence or absence of a base, in the presence of a transition metal catalyst, and using a ligand if required. Here, examples of the transition metal catalyst include, for example, palladium(II) acetate, palladium(II) chloride, palladium(II) bis(triphenylphosphine)acetate, palladium(II) bis(triphenylphosphine)chloride, tris(dibenzylideneacetone)dipalladium(0), bis(dibenzylideneacetone)palladium(0), tetrakistriphenylphosphine palladium(0), [1,1'-bis(diphenylphosphino)ferrocene]palladium(II) chloride, allylpalladium(II) chloride, bis(acetonitrile)palladium(II) chloride, [1,3-bis(2,6-diisopropylphenyl)imidazol-2-ylidene](3-chloropyridyl)palladium(II) dichloride or [1,3-bis(2,6-diisopropylphenyl)imidazolidene](3-chloropyridyl)palladium(II) dichloride, [1,1'-bis(diphenylphosphino)ferrocene]palladium(II) dichloride dichloromethane complex (1:1), copper powder, copper(I) chloride, copper(I) bromide, copper(I) iodide, and copper(I) acetate. Examples of the ligand include, for example, triphenylphosphine, tributylphosphine, tri(2-furyl)phosphine, 2,2-bis(diphenylphosphino)-1,1-binaphthyl (BINAP), 2-(di-tert-butylphosphino)biphenyl, 1,1-bis(diphenylphosphino)ferrocene, 1,10-phenanthroline, N,N'-dimethylethylenediamine, and tetramethylethylenediamine.

Step V-2: Compound (5-3) can be converted into Compound (5-4) by performing various hydrogenation reactions that are publicly known to those skilled in the art [see Comprehensive Organic Transformations, 1989, authored by R. C. Larock, VCH Publishers, INC.].

Step V-3: By the same approach as in Step V-1 described above, Compound (5-1) can be converted into Compound (5-6) by subjecting it to a Suzuki coupling reaction with Boronate Ester (5-5).

Step V-4: Compound (5-6) can be converted into Compound (5-7) by the same approach as in Step V-2 described above.

Step V-5: By the same approach as in Step IV-3 described above, Compound (5-7) can be converted into Compound (5-8) by deprotecting its protecting group for the hydroxy group.

Step V-6: In an inert solvent, Compound (5-8) can be converted into Compound (5-4) by carrying out a Mitsunobu reaction with phthalimide in the presence of a dialkyl azodicarboxylate and a trialkyl- or triaryl-phosphine.

Step V-7: In an inert solvent, the phthaloyl group of Compound (5-4) can be removed by using various organic synthesis methods that are publicly known to those skilled in the art [see Protective Groups in Organic Synthesis, 4th Edition, 2006, authored by T. W. Green et al., Wiley INC.], thereby affording Compound (5-9).

Step V-8: By the same approach as in Step III-1 described above, Compound (5-9) and Carboxylic Acid Compound (5-10) can be converted into Compound (5-11) by subjecting them to an amidation reaction.

Step V-9: Compound (5-11) can be converted into Compound (5-12) by the same approach as in Step 1-2 described above.

Step V-10: Compound (5-11) can be converted into Compound (5-13) by the same approach as in Step 1-3 described above.

Step V-11: Compound (5-12) can be converted into Compound (5-14) by the same approach as in Step 1-3 described above.

Step V-12: Compound (5-13) can be converted into Compound (5-14) by the same approach as in Step 1-2 described above.

Step V-13: By the same approach as in Step 1-7 described above, the present inventive compound (I) can be obtained from Compound (5-14).

### [Production Method 6]

The present inventive compound (I) can also be produced by the method shown in Scheme 6.

When X¹-L-X² of the inventive compound (I) is CH₂₋N(R^{X12})CH₂CH₂CH₂-O, the inventive compound (I) can be produced by performing Step VI-1 to Step VI-6.

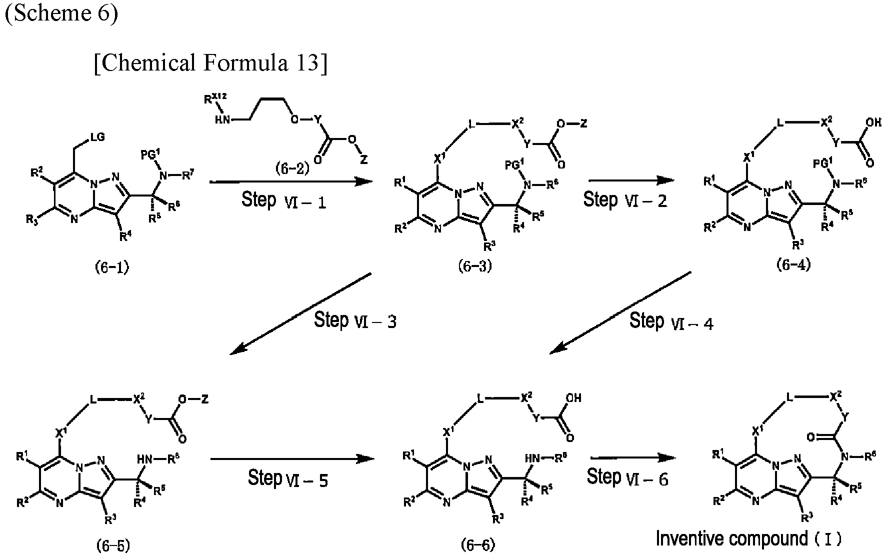

In the above formulas:
LG represents a leaving group such as a halogen atom or a methanesulfonyloxy group;
PG¹ represents a protecting group for the amino group; and
Z represents a protecting group for the carboxy group such as a C₁₋₄ alkyl group and a benzyl group.

Step VI-1: In an inert solvent, Amine Compound (6-2) is allowed to act on Compound (6-1) in the presence or absence of a base such as potassium carbonate, thereby affording Compound (6-3).

Step VI-2: Compound (6-3) can be converted into Compound (6-4) by the same approach as in Step 1-2 described above.

Step VI-3: Compound (6-3) can be converted into Compound (6-5) by the same approach as in Step 1-3 described above.

Step VI-4: Compound (6-4) can be converted into Compound (6-6) by the same approach as in Step 1-3 described above.

Step VI-5: Compound (6-5) can be converted into Compound (6-6) by the same approach as in Step 1-2 described above.

Step VI-6: By the same approach as in Step 1-7 described above, the present inventive compound (I) can be obtained from Compound (6-6).

### [Production Method 7]

The present inventive compound (I) can also be produced by the method shown in Scheme 7.

When R² of the inventive compound (I) obtained in Schemes 1 to 6 is LG (LG represents a leaving group such as a halogen atom or a trifluoromethanesulfonyloxy group), the inventive compound (I) can be produced by performing Step VII-1.

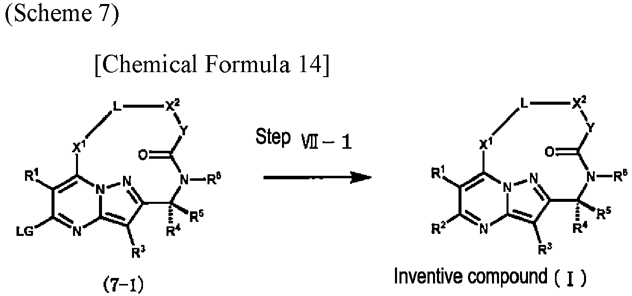

In the above formulas:
LG represents a leaving group such as a halogen atom or a trifluoromethanesulfonyloxy group.

Step VII-1: In an inert solvent, an amine compound such as pyrrolidine or an alcohol compound such as methanol and ethanol is allowed to act on Compound (7-1) in the presence or absence of a base, thereby affording the present inventive compound (I). Alternatively, in an inert solvent, Compound (7-1) is subjected to a coupling reaction with an amine compound, an alcohol compound, an organometallic compound, an olefin compound, or a terminal alkyne compound in the presence or absence of a base, in the presence of a transition metal catalyst, and using a ligand if required, thereby affording the present inventive compound (I).

Here, as for the coupling reaction, mention may be made of the coupling reaction conditions that are publicly known to those skilled in the art, and it can be performed by, for example, methods described in Comprehensive Organic Transformations, 1989, authored by R. C. Larock, VCH Publishers, INC. and the like, methods conforming thereto, or a combination of these methods. Here, examples of the organometallic compound include, for example, a magnesium reactive agent, a zinc reactive agent, a boron reactive agent, and a tin reactive agent, and commercially available compounds, publicly known compounds, or compounds synthesized from commercially available compounds or publicly known compounds using various organic synthesis methods that are publicly known to those skilled in the art can be used. Here, as the olefin compound and the terminal alkyne compound, commercially available compounds, publicly known compounds, or compounds synthesized from commercially available compounds or publicly known compounds using various organic synthesis methods that are publicly known to those skilled in the art can be used. Here, examples of the transition metal catalyst include, for example, palladium(II) acetate, palladium(II) chloride, palladium(II) bis(triphenylphosphine)acetate, palladium(II) bis(triphenylphosphine)chloride, tris(dibenzylideneacetone)dipalladium(0), bis(dibenzylideneacetone)palladium(0), tetrakistriphenylphosphine palladium(0), [1,1'-bis(diphenylphosphino)ferrocene]palladium(II) chloride, allylpalladium(II) chloride, bis(acetonitrile)palladium(II) chloride, [1,3-bis(2,6-diisopropylphenyl)imidazol-2-ylidene](3-chloropyridyl)palladium(II) dichloride or [1,3-bis(2,6-diisopropylphenyl)imidazolidene](3-chloropyridyl)palladium(II) dichloride, [1,1'-bis(diphenylphosphino)ferrocene]palladium(II) dichloride dichloromethane complex (1:1), copper powder, copper(I) chloride, copper(I) bromide, copper(I) iodide, and copper(I) acetate. Examples of the ligand include, for example, triphenylphosphine, tributylphosphine, tri(2-furyl)phosphine, 2,2-bis(diphenylphosphino)-1,1-binaphthyl (BINAP), 2-(di-tert-butylphosphino)biphenyl, 1,1-bis(diphenylphosphino)ferrocene, 1,10-phenanthroline, N,N'-dimethylethylenediamine, and tetramethylethylenediamine.

Furthermore, the present inventive compound (I) can also be produced by changing the order in which the steps are performed, by performing a reaction while providing a protecting group for the hydroxy group or the amino group and performing deprotection in a later step, or by adding a new step in the middle of each step and converting the functional groups of R¹, R², R³, R⁴, R⁵, R⁶, X¹, X², Y, and L using various organic synthesis methods that are publicly known to those skilled in the art within the range not departing from the present invention.

The reaction temperature for the general production methods of the present inventive compound is -78°C to 250°C, and is preferably -78°C to 120°C. The reaction time is 5 minutes to 3 days, and is preferably 30 minutes to 18 hours. These production methods can be performed under ordinary pressure, under pressurization, or under microwave irradiation.

Examples of the protecting group for the amino group include, for example, a C₁₋₆ acyl group (a formyl group, an acetyl group, a propionyl group, and the like), a C₂₋₁₂ alkoxycarbonyl group (a methoxycarbonyl group, an ethoxycarbonyl group, a tert-butoxycarbonyl group, a benzyloxycarbonyl group, a 9-fluorenylmethylenoxycarbonyl group, and the like), an arylcarbonyl group (a benzoyl group and the like), a trityl group, a phthaloyl group, a N,N-dimethylaminomethylene group, a substituted silyl group (a trimethylsilyl group, a triethylsilyl group, a dimethylphenylsilyl group, a tert-butyldimethylsilyl group, a tert-butyldiethylsilyl group, and the like), and a C₂₋₆ alkenyl group (a 1-allyl group and the like). These groups are optionally substituted with one or more substituents selected from a halogen atom, a C₁₋₆ alkoxy group (a methoxy group, an ethoxy group, a propoxy group, and the like), and a nitro group.

Examples of the protecting group for the hydroxy group include, for example, a C₁₋₆ alkyl group (a methyl group, an ethyl group, a tert-butyl group, and the like), a C₇₋₂₀ aralkyl group (a benzyl group, a trityl group, and the like), a phenyl group, a substituted silyl group (a trimethylsilyl group, a triethylsilyl group, a dimethylphenylsilyl group, a tert-butyldimethylsilyl group, a tert-butyldiethylsilyl group, and the like), a C₂₋₆ alkenyl group (1-allyl and the like), a C₁₋₆ acyl group (a formyl group, an acetyl group, a propionyl group, and the like), a C₂₋₁₂ alkoxycarbonyl group (a methoxycarbonyl group, an ethoxycarbonyl group, a tert-butoxycarbonyl group, a benzyloxycarbonyl group, a 9-fluorenylmethylenoxycarbonyl group, and the like), an arylcarbonyl group (a benzoyl group and the like), a 2-tetrahydropyranyl group, and a 2-tetrahydrofuranyl group. These groups are optionally substituted with one or more substituents selected from a halogen atom, a C₁₋₆ alkoxy group (a methoxy group, an ethoxy group, a propoxy group, and the like), and a nitro group.

### EXAMPLES

Hereinafter, the present invention will be further described in detail with reference to Reference Examples, Examples, and Test Example. However, they do not limit the present invention, and may be varied in the range without departing the scope of the present invention.

In Reference Examples and Examples, the term "Phase Separator" for post treatment refers to ISOLUTE (R) Phase Separator from Biotage AB. For purification using silica gel column chromatography, "Silica Gel 60" from Merck KGaA, "Silica Gel PSQ60" from Fuji Silysia Chemical, Ltd., "Silica Gel 60" and "Silica Gel 60N" from Kanto Chemical Co., Inc., "Chromatrex NH" from Fuji Silysia Chemical, Ltd., or packed columns such as SNAP Cartridge KP-NH from Biotage AB, SNAP Cartridge KP-Sil from Biotage AB, SNAP Cartridge HP-Sil from Biotage AB, REVELERISTM (Amino, 40 µM) from W. R. Grace & Co., and REVELERISTM (Silica, 40 µM) from W. R. Grace & Co. were used. Unless otherwise noted, "Silica Gel PSQ60" from Fuji Silysia Chemical, Ltd., "Silica Gel 60N" from Kanto Chemical Co., Inc., or a packed column was used. For purification using preparative thin layer chromatography (PTLC), Silica Gel 60 F₂₅₄, 20 cm × 20 cm from Merck KGaA was used. For preparative purification by the supercritical fluid chromatography (SFC) method, SFC 30 from Waters Corporation was used. For "reversed phase column chromatography" (hereinafter, this may also be referred to as reversed phase preparative HPLC) in purification, Waters SunFire prep C18 OBD, 5.0 µm, ϕ30 × 50 mm, YMC-Actus Triart C18, 5.0 µm, ϕ30 × 50 mm, Xbridge Prep C18 5.0 µm OBD, ϕ30 × 50 mm, or Waters XSelect CSH C18, 5.0 µm, ϕ30 × 50 mm was used. The preparative condition was selected as appropriate from the following four conditions to carry out the purification.

Preparative apparatus: Agilent 1260 and Agilent 6130 (preparative conditions 1 to 3) or the GILSON preparative HPLC system (preparative condition 4)

Mobile phase A: water containing 0.1% formic acid, B: acetonitrile containing 0.1% formic acid
Flow rate: 50 mL/min
Preparative condition 1:
   0.0 to 0.5 min (solution A/solution B = 90/10)
   0.5 to 7.5 min (solution A/solution B = 90/10 to 20/80)
   7.5 to 7.95 min (solution A/solution B = 20/80)
   7.95 to 8.0 min (solution A/solution B = 20/80 to 5/95)
   8.0 to 9.0 min (solution A/solution B = 5/95)
Preparative condition 2:
   0.0 to 0.5 min (solution A/solution B = 95/5)
   0.5 to 7.5 min (solution A/solution B = 95/5 to 50/50)
   7.5 to 7.95 min (solution A/solution B = 50/50)
   7.95 to 8.0 min (solution A/solution B = 50/50 to 5/95)
   8.0 to 9.0 min (solution A/solution B = 5/95)
Preparative condition 3:
   0.0 to 0.5 min (solution A/solution B = 80/20)
   0.5 to 7.0 min (solution A/solution B = 80/20 to 5/95)
   7.0 to 7.45 min (solution A/solution B = 5/95)
   7.45 to 7.5 min (solution A/solution B = 5/95 to 1/99)
   7.5 to 9.0 min (solution A/solution B = 1/99)
Preparative condition 4:
   0.0 to 2,0 min (solution A/solution B = 90/10)
   2.0 to 11 min (solution A/solution B = 90/10 to 20/80)
   11 to 13.5 min (solution A/solution B = 20/80 to 5/95)

The instrumental data described in the following Reference Examples and Examples were measured with the following measurement instruments.
NMR spectrum: JNM-ECA 600 (600 MHz) from JEOL Ltd., JNM-ECA 500 (500 MHz) from JEOL Ltd., or AVANCE III HD 400 (400 MHz, BRUKER) from Bruker Corporation.
MS spectrum: LCMS-2010EV from Shimadzu Corporation or Platform LC from MicroMass

In the following Reference Examples and Examples, the high performance liquid chromatography mass spectrum (LCMS) was measured under the following conditions.

### Analysis condition 1

Measurement instrument: Platform LC from MicroMass and Agilent 1100 from Agilent Technologies
Column: SunFire C18, 2.5 µm, ϕ 4.6 × 50 mm from Waters Corporation
Ionization method: electron spray ionization (ESI) method
Solvent: solution A, water containing 0.1% trifluoroacetic acid; solution B, acetonitrile containing 0.1% trifluoroacetic acid
Detection method: 254 nm, Flow rate: 1.0 mL/min
Gradient: 0 min (solution A/solution B = 90/10), 0.5 min (solution A/solution B = 90/10), 5.5 min (solution A/solution B = 20/80), 6.0 min (solution A/solution B = 1/99), 6.3 min (solution A/solution B = 1/99)
Analysis condition 2 (hereinafter, referred to as mode H)
Measurement machine: Agilent 1290 and Agilent 6130
Column: Waters Acquity CSH C18, 1.7 µm, ϕ 2.1 × 50 mm
Ionization method: electron spray ionization (ESI) method
Solvent: solution A, water containing 0.1% formic acid; solution B, acetonitrile containing 0.1% formic acid
Detection method: 254 nm
Flow rate and gradient: 0.8 mL/min, 0 min (solution A/solution B = 80/20) → 1.2 min (solution A/solution B = 50/50), 1.0 mL/min → 1.38 min (solution A/solution B = 3/97).
Analysis condition 3 (hereinafter, referred to as mode N)

The measurement instrument, column, solvent, ionization method, and detection method are the same as in analysis condition 2.
Flow rate and gradient: 0.8 mL/min, 0 min (solution A/solution B = 80/20) → 1.2 to 1.4 min (solution A/solution B = 1/99).
Analysis condition 4 (hereinafter, referred to as mode L)
Flow rate and gradient: 0 min (solution A/solution B = 70/30) → 0.8 min (solution A/solution B = 5/95), 1.5 mL/min → 1.0 to 1.4 min (solution A/solution B = 1/99)

In the following Reference Examples and Examples, analysis by the supercritical fluid chromatography (SFC) method was performed using UPC2 from Waters Corporation.

In the following Reference Examples and Examples, the compounds were denominated by ACD/Name (ACD/Labs 12.01, Advanced Chemistry Development Inc.).

The following terms and reagents are denoted as follows in the present Reference Examples and Examples.
AcCl (acetyl chloride), AcOH (acetic acid), APCI (atmospheric pressure chemical ionization method), aq. (aqueous solution), Bn (benzyl), Boc (tert-butoxycarbonyl), BoC₂O (di-tert-butyl dicarbonate), Brine (saturated saline solution), br s (broad singlet), Bu (butyl), Cbz (benzyloxycarbonyl), CbzCl (benzyloxycarbonyl chloride), CDCl₃ (deuterated chloroform), CD₃I (deuterated methyl iodide), CD₃OD (deuterated methanol), CHCl₃ (chloroform), CMBP (cyanomethylenetributylphosphorane), CO₂ (carbon dioxide), cPr (cyclopropyl),
Cs₂CO₃ (cesium carbonate), CuI (copper(I) iodide), d (doublet), dd (double doublet), DEAD (diethyl azodicarboxylate), DIBAL-H (diisobutylaluminium hydride), DeoxoFluor (bis(2-methoxyethyl)aminosulfur trifluoride), Dess-Martin reagent
(1,1,1-triacetoxy-1,1-dihydro-1,2-benziodoxol-3(1H)-one), DIPEA
(N,N-diisopropylethylamine), DMA (dimethylacetamide), DMAP
(N,N-dimethyl-4-aminopyridine), DMF (N,N-dimethylformamide), DMSO (dimethyl sulfoxide), DMSO-d₆ (deuterated dimethyl sulfoxide), dt (double triplet), ee (enantiomeric excess), ESI (electrospray ionization method), Et (ethyl), EtI (ethyl iodide), Et₃N (triethylamine), Et₂O (diethyl ether), EtOAc (ethyl acetate), EtOH (ethanol), H (proton), HATU (O-(7-azabenzotriazol-1-yl)-N,N,N',N'-tetramethyluronium hexafluorophosphate), HCl (hydrogen chloride), HMDS (1,1,1,3,3,3-hexamethyldisilazane), HPLC (high performance liquid chromatography), Hz (hertz), IPA (isopropyl alcohol), IPE (isopropyl ether), iPr (isopropyl), J (coupling constant), K₂CO₃ (potassium carbonate), KOH (potassium hydroxide), K₃PO₄ (potassium phosphate), LAWESSON reagent
(2,4-bis(4-methoxyphenyl)-1,3,2,4-dithiadiphosphetane-2,4-disulfide), LCMS (high performance liquid chromatography mass spectrum), LiBH₄ (lithium borohydride), m (multiplet), M (mol/L), Me (methyl), MeCN (acetonitrile), MeI (methyl iodide), MeOH (methanol), MHz (megahertz), MgSO₄ (anhydrous magnesium sulfate), min (minute), MS (mass spectrometry), MsCl (methanesulfonyl chloride), m/z (mass to charge ratio),
NaBH₄ (sodium borohydride), NaBH₃CN (sodium cyanoborohydride), NaBH(OAc)₃ (sodium triacetoxyborohydride), Na₂CO₃ (sodium carbonate), NaH (sodium hydride),
NaHCO₃ (sodium hydrogen carbonate), NaHMDS (sodium hexamethyldisilazide), NaI (sodium iodide), NaOH (sodium hydroxide), NaOMe (sodium methoxide), NaOEt (sodium ethoxide), Na₂S₂O₃ (sodium thiosulfate), Na₂SO₄ (anhydrous sodium sulfate), NaO^{t}Bu (sodium tert-butoxide), NCS (N-chlorosuccinimide), NH₄Cl (ammonium chloride), NH₄OH (25 to 28% aqueous ammonia), NIS (N-iodosuccinimide), NMP (N-methyl-2-pyrrolidone), NMR (nuclear magnetic resonance spectroscopy),
PdCl₂(PPh₃)₂ [bis(triphenylphosphine)palladium(II) dichloride],
PdCl₂(dppf)·CH₂Cl₂ {[1,1'-bis(diphenylphosphino)ferrocene]palladium(II) dichloride dichloromethane complex (1:1)}, Pd₂(dba)₃ [tris(dibenzylidenacetone)dipalladium(0)], PdG3-Ruphos
((2-dicyclohexylphosphino-2',6'-diisopropoxy-1,1'-biphenyl)[2-(2'-amino-1,1'-biphenyl)]palla dium(II) methanesulfonate), Pd(OAc)₂ (palladium(II) acetate),
Pd(PPh₃)₄ (tetrakis(triphenylphosphine)palladium(0)), Ph (phenyl), POCl₃ (phosphorus oxychloride), P(o-tol)₃ (tris(2-methylphenyl)phosphine), PPh₃ (triphenylphosphine), Pr (propyl), PTLC (preparative thin layer chromatography), q (quartet), Ruphos (2-dicyclohexylphosphino-2',6'-diisopropoxybiphenyl), s (singlet), Sat. (saturated), Selectfluor (1-chloromethyl-4-fluoro-1,4-diazoniabicyclo[2.2.2]octane bis(tetrafluoroborate)), SFC (supercritical fluid chromatography), SUPERSTABLE Pd(0) CATALYST (tris{tris[3,5-bis(trifluoromethyl)phenyl]phosphine}palladium(0)), t (triplet), TBAF (tetrabutylammonium fluoride), TBDPSCl (tert-butyldiphenylchlorosilane), tBuOH (tert-butyl alcohol), td (triple doublet), TFA (trifluoroacetic acid), Tf₂O (trifluoromethanesulfonic anhydride), TFP [tri(2-furyl)phosphine], THF (tetrahydrofuran), TMSCl (trimethylsilyl chloride), t_{R} (retention time), TsCl (p-toluenesulfonyl chloride), v/v (volume/volume), δ [the degree of downfield from tetramethylsilane (parts per million)].

### Reference Example 1-1: Synthesis of 5,7-dichloro-2-[(2S)-piperidin-2-yl]pyrazolo[1,5-a]pyrimidinehydrochloride

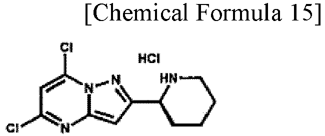

(1) To a solution of tert-butyl (2S)-2-(5-amino-1H-pyrazol-3-yl)piperidine-1-carboxylate (15 g) (described in International Publication No. WO 2011/163518, page 154) in EtOH (0.28 L), diethyl propanedioate (14 g) and a 20% NaOEt solution in EtOH (0.12 L) were added, and the mixture was stirred at 90°C for 18 hours. The reaction solution was made acidic with a 1 M HCl aq. and then extracted with CHCl₃. The organic layer was washed with Brine, dried over MgSO₄, filtered, and then the solvent was concentrated to afford tert-butyl
   (2S)-2-(7-hydroxy-5-oxo-4,5-dihydropyrazolo[1,5-a]pyrimidin-2-yl)piperidine-1-carboxylate (20 g, yellow amorphous).
   LCMS (ESI/APCI dual) m/z 333 [M-H]⁻, t_{R} = 0.712 min, mode N.
(2) A solution of tert-butyl (2S)-2-(7-hydroxy-5-oxo-4,5-dihydropyrazolo[1,5-a]pyrimidin-2-yl)piperidine-1-carboxylate (20 g) in 4 M hydrochloric acid 1,4-dioxane (0.15 L) was added and the mixture was stirred at room temperature for 3 hours. The reaction solution was concentrated, suspended in Et₂O, and filtered to afford 7-hydroxy-2-[(2S)-2-piperidyl]-4H-pyrazolo[1,5-a]pyrimidin-5-one hydrochloride (17 g, yellow powder).
   LCMS (ESI/APCI dual) m/z 235 [M+H]⁺, t_{R} = 0.227 min, mode H.
(3) A solution of 7-hydroxy-2-[(2S)-2-piperidyl]-4H-pyrazolo[1,5-a]pyrimidin-5-one hydrochloride (8.5 g) in POCl₃ (83 mL) was stirred at 90°C overnight. The reaction solution was concentrated and the residue was washed with a mixed solution of IPA/IPE (1/4, v/v) to afford 5,7-dichloro-2-[(2S)-piperidin-2-yl]pyrazolo[1,5-a]pyrimidine hydrochloride (8.5 g, brown powder).
   ¹H NMR (400 MHz, DMSO-d₆) δ ppm 1.58 - 1.95 (m, 5H), 2.13 - 2.25 (m, 1H), 2.99 - 3.17 (m, 1H), 3.28 - 3.41 (m, 1H), 4.57 - 4.70 (m, 1H), 7.11 (s, 1H), 7.77 (s, 1H), 9.37 - 9.59 (m, 2H).
   LCMS (ESI) m/z 271 [M+H]⁺, t_{R} = 0.655 min, mode H.

### Reference Example 1-2: Synthesis of tert-butyl (2S)-2-(5,7-dichloropyrazolo[1,5-a]pyrimidin-2-yl)piperidine-1-carboxylate

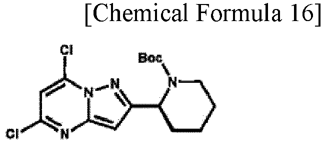

To a solution of 5,7-dichloro-2-[(2S)-piperidin-2-yl]pyrazolo[1,5-a]pyrimidine hydrochloride (1.6 g) in CHCl₃ (52 mL), BoC₂O (2.3 g) and Et₃N (2.2 mL) were added, and the mixture was stirred at room temperature for 1.5 hours. To the reaction liquid, a Sat. NaHCO₃ aq. was added, and the aqueous layer was extracted with CHCl₃. The organic layer was collected by using Phase Separator and concentrated. The residue was purified by OH type silica gel column chromatography (mobile phase: hexane/EtOAc = 95/5 to 30/70; v/v) to afford tert-butyl

(2S)-2-(5,7-dichloropyrazolo[1,5-a]pyrimidin-2-yl)piperidine-1-carboxylate (0.86 g, colorless gum).
¹H NMR (400 MHz, CDCl₃) δ ppm 1.34 - 1.73 (m, 13H), 1.83 - 1.97 (m, 1H), 2.39 - 2.53 (m, 1H), 2.73 - 2.97 (m, 1H), 3.98 - 4.19 (m, 1H), 5.53 - 5.72 (m, 1H), 6.50 (s, 1H), 6.91 (s, 1H). LCMS (ESI/APCI dual) m/z 371 [M+H]⁺, t_{R} = 1.247 min, mode N.

### Reference Example 1-3: Synthesis of 5,7-dichloro-6-methyl-2-[(2S)-piperidin-2-yl]pyrazolo[1,5-a]pyrimidine hydrochloride

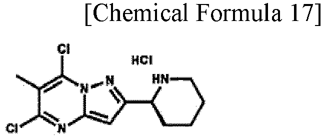

By using the same approaches as in Reference Example 1-1 (1) to (3), the title compound (12 g, brown powder) was obtained from diethyl methylpropanedioate (10 g) and tert-butyl (2S)-2-(5-amino-1H-pyrazol-3-yl)piperidine-1-carboxylate (10 g).
¹H NMR (400 MHz, DMSO-d₆) δ ppm 1.59 - 1.94 (m, 5H), 2.13 - 2.27 (m, 1H), 2.48 (s, 3H), 3.01 - 3.19 (m, 1H), 3.27 - 3.44 (m, 1H), 4.51 - 4.73 (m, 1H), 6.99 (s, 1H), 9.09 (br s, 1H), 9.31 (br s, 1H).
LCMS (ESI) m/z 285 [M+H]⁺, t_{R} = 0.734 min, mode H.

### Reference Example 1-4: Synthesis of 5,7-dichloro-6-fluoro-2-[(2S)-piperidin-2-yl]pyrazolo[1,5-a]pyrimidine hydrochloride

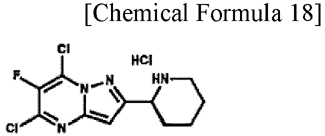

By using the same approaches as in Reference Example 1-1 (1) to (3), the title compound (4.3 g, brown gum) was obtained as a crude product from diethyl fluoropropanedioate (2.0 g) and tert-butyl
(2 S)-2-(5 -amino-1H-pyrazol -3 -yl)piperidine- 1-carboxylate (2.0 g).
LCMS (ESI) m/z 289 [M+H]⁺, t_{R} = 0.727 min, mode H.

### Reference Example 1-5: Synthesis of 5,6,7-trichloro-2-[(2S)-piperidin-2-yl]pyrazolo[1,5-a]pyrimidine hydrochloride

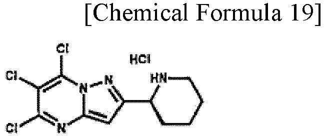

A solution of
5,7-dichloro-6-fluoro-2-[(2S)-piperidin-2-yl]pyrazolo[1,5-a]pyrimidine hydrochloride (1.8 g) obtained in Reference Example 1-4 in POCl₃ (2.0 mL) was stirred at 95°C for 19 hours. The reaction solution was concentrated to afford the title compound (5.6 g, brown oily matter) as a crude product.
LCMS (ESI) m/z 305 [M+H]⁺, t_{R} = 0.818 min, mode H.

### Reference Example 1-6: Synthesis of tert-butyl (2S)-2-(7-chloro-5-cyclopropylpyrazolo[1,5-a]pyrimidin-2-yl)piperidine-1-carboxylate

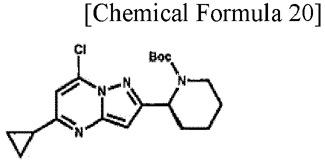

### (1) A solution of tert-butyl

(2S)-2-(5-amino-1H-pyrazol-3-yl)piperidine-1-carboxylate (5.0 g) and ethyl 3-cyclopropyl-3-oxo-propanoate (4.4 g) in AcOH (47 mL) was stirred at 100°C for 45 minutes. The reaction solution was concentrated and then purified by OH type silica gel column chromatography (mobile phase: CHCl₃/MeOH = 100/0 to 90/10; v/v) to afford tert-butyl
(2S)-2-(5-cyclopropyl-7-oxo-4,7-dihydropyrazolo[1,5-a]pyrimidin-2-yl)piperidine-1-carboxy late (4.2 g, colorless powder).
¹H NMR (400 MHz, DMSO-d₆) δ ppm 0.91 - 1.11 (m, 4H), 1.27 - 1.58 (m, 13H), 1.63 - 1.78 (m, 1H), 1.82 - 1.93 (m, 1H), 2.20 - 2.35 (m, 1H), 2.68 - 2.95 (m, 1H), 3.78 - 4.01 (m,
1H), 5.20 - 5.51 (m, 2H), 5.76 (s, 1H), 11.95 (br s, 1H).
LCMS (ESI/APCI dual) m/z 359 [M+H]⁺, t_{R} = 0.771 min, mode N.

### (2) To a solution of DMAP (76 mg) in pyridine (2.8 mL), POCl₃ (0.37 mL) and tert-butyl

(2S)-2-(5-cyclopropyl-7-oxo-4,7-dihydropyrazolo[1,5-a]pyrimidin-2-yl)piperidine-1-carboxy late (0.21 g) were sequentially added under ice cooling, and the mixture was stirred at 65°C for 1 hour. POCl₃ was removed by concentration. Purification by OH type silica gel column chromatography (mobile phase: hexane/EtOAc = 80/20 to 50/50; v/v) afforded tert-butyl
(2S)-2-(7-chloro-5-cyclopropylpyrazolo[1,5-a]pyrimidin-2-yl)piperidine-1-carboxylate (0.17 g, colorless oily matter).
¹H NMR (400 MHz, CDCl₃) δ ppm 1.05 - 1.21 (m, 4H), 1.36 - 1.70 (m, 20H), 1.79 - 1.93 (m, 1H), 1.96 - 2.09 (m, 1H), 2.41 - 2.57 (m, 1H), 2.80 - 3.01 (m, 1H), 3.94 - 4.18 (m, 1H), 5.47 - 5.74 (m, 1H), 6.36 (s, 1H), 6.74 (s, 1H).
LCMS (ESI) m/z 377 [M+H]⁺, t_{R} = 0.954 min, mode L.

### Reference Example 1-7: Synthesis of tert-butyl (2S)-2-(7-chloro-5-cyclopropyl-3-fluoropyrazolo[1,5-a]pyrimidin-2-yl)piperidine-1-carboxylate

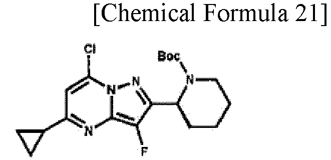

To a solution of tert-butyl
(2S)-2-(7-chloro-5-cyclopropylpyrazolo[1,5-a]pyrimidin-2-yl)piperidine-1-carboxylate (0.51 g) in MeCN (10 mL), Selectfluor (R) (0.53 g) was added, and the mixture was stirred at 0°C for 2 hours. The reaction solution was poured into a Sat. Na₂S₂O₃ aq./a Sat.
NaHCO₃ aq. (1/1; v/v) and extracted with CHCl₃. The organic layer was washed with Brine, dried over MgSO₄, filtered, and then the solvent was concentrated. The residue was purified by OH type silica gel column chromatography (mobile phase: hexane/EtOAc = 80/20 to 50/50; v/v) to afford tert-butyl
(2S)-2-(7-chloro-5-cyclopropyl-3-fluoropyrazolo[1,5-a]pyrimidin-2-yl)piperidine-1-carboxyl ate (0.18 g, brown gum).
¹H NMR (400 MHz, CDCl₃) δ ppm 1.04 - 1.24 (m, 4H), 1.36 - 1.74 (m, 13H), 1.79 - 1.94 (m, 1H), 1.98 - 2.13 (m, 1H), 2.31 - 2.59 (m, 1H), 2.83 - 3.07 (m, 1H), 3.96 - 4.18 (m, 1H), 5.56 - 5.79 (m, 1H), 6.72 (s, 1H).
LCMS (ESI/APCI dual) m/z 395 [M+H]⁺, t_{R} = 1.327 min, mode N.

### Reference Example 1-8: Synthesis of tert-butyl (3R)-3-(7-chloro-5-cyclopropylpyrazolo[1,5-a]pyrimidin-2-yl)morpholine-4-carboxylate

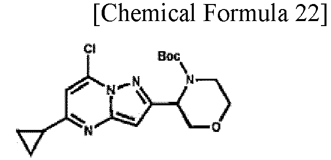

By using the same approaches as in Reference Example 1-6 (1) and (2), the title compound (0.20 g, colorless gum) was obtained by using tert-butyl
(3R)-3-(5-amino-1H-pyrazol-3-yl)morpholine-4-carboxylate (1.8 g) instead of tert-butyl (2S)-2-(5-amino-1H-pyrazol-3-yl)piperidine-1-carboxylate as the starting raw material. ¹H NMR (400 MHz, CDCl₃) δ ppm 1.06 - 1.21 (m, 4H), 1.48 (s, 9H), 1.93 - 2.09 (m, 1H), 3.22 - 3.37 (m, 1H), 3.47 - 3.68 (m, 1H), 3.86 (m, J = 3.0 Hz, 3H), 4.51 - 4.71 (m, 1H), 5.19 - 5.41 (m, 1H), 6.44 (s, 1H), 6.77 (s, 1H).
LCMS (ESI/APCI dual) m/z 379 [M+H]⁺, t_{R} = 1.057 min, mode N.

### Reference Example 1-9: Synthesis of tert-butyl 2-(7-chloro-5-cyclopropylpyrazolo[1,5-a]pyrimidin-2-yl)-4,4-difluoropiperidine-1-carboxylate

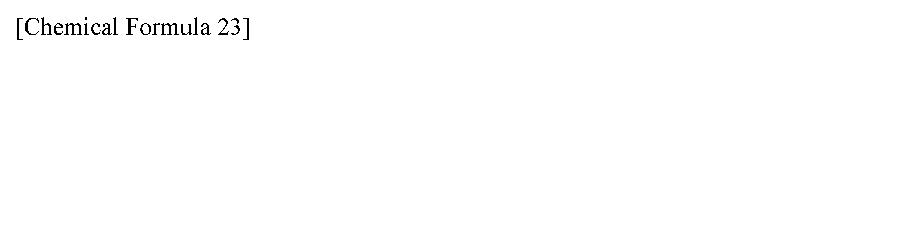

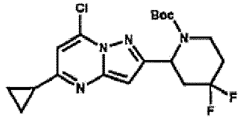

By using the same approaches as in Reference Example 1-6 (1) and (2), the title compound (0.32 g, colorless gum) was obtained by using tert-butyl
2-(5-amino-1H-pyrazol-3-yl)-4,4-difluoropiperidine-1-carboxylate (1.3 g) instead of tert-butyl (2S)-2-(5-amino-1H-pyrazol-3-yl)piperidine-1-carboxylate as the starting raw material.
¹H NMR (400 MHz, CDCl₃) δ ppm 1.08 - 1.21 (m, 4H), 1.47 (s, 9H), 1.85 - 2.09 (m, 3H), 2.20 - 2.46 (m, 1H), 2.91 - 3.11 (m, 1H), 3.17 - 3.35 (m, 1H), 4.18 - 4.39 (m, 1H), 5.72 - 5.89 (m, 1H), 6.34 (s, 1H), 6.77 (s, 1H).
LCMS (ESI/APCI dual) m/z 413 [M+H]⁺, t_{R} = 1.193 min, mode N.

### Reference Example 1-10: Synthesis of tert-butyl (2S)-2-(7-chloro-5,6-dimethyl-pyrazolo[1,5-a]pyrimidin-2-yl)piperidine-1-carboxylate

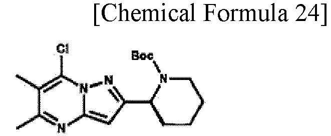

By using the same approaches as in Reference Example 1-6 (1) and (2), the title compound (0.26 g, colorless oily matter) was obtained from tert-butyl (2S)-2-(5-amino-1H-pyrazol-3-yl)piperidine-1-carboxylate (1.0 g) and ethyl 2-methyl-3-oxobutanoate (0.81 g).
¹H NMR (400 MHz, CDCl₃) δ ppm 1.47 (s, 9H), 1.52 - 1.70 (m, 4H), 1.82 - 1.93 (m, 1H), 2.41 (s, 3H), 2.50 (d, J = 13.2 Hz, 1H), 2.59 (s, 3H), 2.85 - 2.96 (m, 1H), 3.97 - 4.20 (m, 1H), 5.62 (br s, 1H), 6.39 (s, 1H).
LCMS (ESI) m/z 365 [M+H]⁺, t_{R} = 1.266 min, mode N.

### Reference Example 1-11: Synthesis of tert-butyl (2S)-2-(7-chloro-5-cyclobutylpyrazolo[1,5-a]pyrimidin-2-yl)piperidine-1-carboxylate

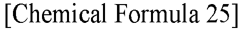

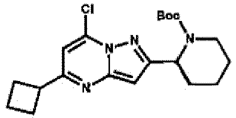

By using the same approaches as in Reference Example 1-6 (1) and (2), the title compound (52 mg, colorless oily matter) was obtained from tert-butyl (2S)-2-(5-amino-1H-pyrazol-3-yl)piperidine-1-carboxylate (1.0 g) and ethyl 3-cyclobutyl-3-oxopropanoate (0.96 g).
¹H NMR (400 MHz, CDCl₃) δ ppm 1.48 (s, 9H), 1.57 - 1.72 (m, 2H), 1.85 - 1.98 (m, 2H), 2.03 - 2.17 (m, 1H), 2.36 - 2.42 (m, 6H), 2.47 - 2.54 (m, 1H), 2.88 - 2.95 (m, 1H), 3.63 - 3.69 (m, 1H), 4.05 - 4.12 (m, 1H), 5.61 - 5.65 (m, 1H), 6.49 (s, 1H), 6.76 (s, 1H).
LCMS (ESI) m/z 391 [M+H]⁺, t_{R} = 1.127 min, mode L.

### Reference Example 1-12: Synthesis of tert-butyl (2S)-2-{5-[cis-3-(benzyloxy)cyclobutyl]-7-chloropyrazolo[1,5-a]pyrimidin-2-yl}piperidine-1 -carboxylate

### Reference Example 1-13: Synthesis of tert-butyl (2S)-2-{5-[trans-3-(benzyloxy)cyclobutyl]-7-chloropyrazolo[1,5-a]pyrimidin-2-yl}piperidine -1-carboxylate

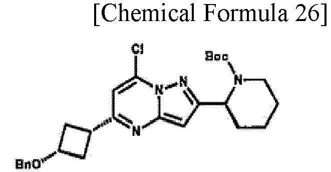

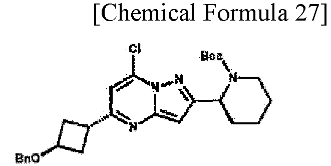

By sequentially performing the same approaches as in Reference Example 1-6 (1), Reference Example 1-2, and Reference Example 1-6 (2), the title compound (Reference Example 1-12) (9.0 g, colorless oily matter, cis form) and the title compound (Reference Example 1-13) (2.5 g, colorless oily matter, trans form) were obtained from tert-butyl (2S)-2-(5-amino-1H-pyrazol-3-yl)piperidine-1-carboxylate (12 g) and ethyl 3-[3-(benzyloxy)cyclobutyl]-3-oxopropanoate (15 g).

### Reference Example 1-12:

¹H NMR (400 MHz, CDCl₃) δ ppm 1.45 - 1.69 (m, 13H), 1.76 - 1.97 (m, 1H), 2.34 (q, J = 8.8 Hz, 2H), 2.43 - 2.58 (m, 1H), 2.62 - 2.81 (m, 2H), 2.81 - 3.00 (m, 1H), 3.13 (quin, J = 8.9 Hz, 1H), 3.99 - 4.17 (m, 2H), 4.48 (s, 2H), 5.63 (br s, 1H), 6.48 (s, 1H), 6.80 (s, 1H), 7.23 - 7.39 (m, 5H).
LCMS (ESI) m/z 497 [M+H]⁺, t_{R} = 1.156 min, mode L.

### Reference Example 1-13:

¹H NMR (400 MHz, CDCl₃) δ ppm 1.45 - 1.69 (m, 13H), 1.77 - 2.05 (m, 1H), 2.43 - 2.60 (m, 3H), 2.60 - 2.76 (m, 2H), 2.92 (t, J = 11.4 Hz, 1H), 3.56 - 3.74 (m, 1H), 3.98 - 4.23 (m, 1H), 4.28 - 4.43 (m, 1H), 4.47 (s, 2H), 5.64 (br s, 1H), 6.49 (s, 1H), 6.74 (s, 1H), 7.28 - 7.43 (m, 5H).
LCMS (ESI) m/z 497 [M+H]⁺, t_{R} = 1.176 min, mode L.

### Reference Example 1-14: Synthesis of tert-butyl (2S)-2-[5-(1-{[(benzyloxy)carbonyl]amino}cyclopropyl)-7-chloropyrazolo[1,5-a]pyrimidin-2 -yl]piperidine-1-carboxylate

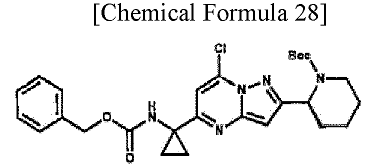

By sequentially performing the same approaches as in Reference Example 1-6 (1), Reference Example 1-2, and Reference Example 1-6 (2), the title compound (0.24 g, colorless oily matter) was obtained from tert-butyl
(2S)-2-(5-amino-1H-pyrazol-3-yl)piperidine-1-carboxylate (2.3 g) and ethyl 3-(1-{[(benzyloxy)carbonyl]amino}cyclopropyl)-3-oxopropanoate (4.0 g).
¹H NMR (400 MHz, CDCl₃) δ ppm 1.38 - 1.42 (m, 2H), 1.47 (s, 9H), 1.55 - 1.79 (m, 6H), 1.81 - 1.95 (m, 1H), 2.44 - 2.56 (m, 1H), 2.89 (t, J = 11.6 Hz, 1H), 4.00 - 4.19 (m, 1H),
5.17 (s, 2H), 5.56 (br s, 1H), 5.61 (br s, 1H), 6.38 (s, 1H), 6.94 - 7.02 (m, 1H), 7.31 - 7.56 (m, 5H).
LCMS (ESI) m/z 526 [M+H]⁺, t_{R} = 1.34 min, mode N.

### Reference Example 1-15: Synthesis of (1S)-1-(5,7-dichloropyrazolo[1,5-a]pyrimidin-2-yl)-N-methylpropan-1-amine hydrochloride

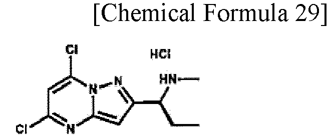

By using the same approaches as in Reference Example 1-1 (1) and (3), the title compound (1.2 g, brown gum) was obtained as a crude product by using tert-butyl [(1S)-1-(5-amino-1H-pyrazol-3-yl)propyl]methylcarbamate (2.0 g) (described in International Publication No. WO 2016/1481452) instead of tert-butyl (2S)-2-(5-amino-1H-pyrazol-3-yl)piperidine-1-carboxylate as the starting raw material. LCMS (ESI) m/z 259 [M+H]⁺, t_{R} = 0.650 min, mode H.

### Reference Example 1-16: Synthesis of (1S)-1-(5,7-dichloro-6-methylpyrazolo[1,5-a]pyrimidin-2-yl)-N-methylpropan-1-amine hydrochloride

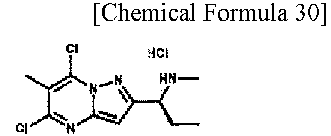

By using the same approaches as in Reference Example 1-1 (1) and (3), the title compound (0.58 g, colorless powder) was obtained as a crudely purified product from tert-butyl [(1S)-1-(5-amino-1H-pyrazol-3-yl)propyl]methylcarbamate (3.0 g) and diethyl methylpropanedioate (3.0 mL).
¹H NMR (400 MHz, DMSO-d₆) δ ppm 0.81 (t, J = 7.4 Hz, 3H), 1.94 - 2.22 (m, 2H), 2.43 - 2.53 (m, 9H), 4.35 - 4.47 (m, 1H), 7.05 (s, 1H), 9.42 (br s, 2H).
LCMS (ESI) m/z 273 [M+H]⁺, t_{R} = 0.775 min, mode H.

### Reference Example 1-17: Synthesis of (1S)-1-(5,7-dichloro-6-fluoropyrazolo[1,5-a]pyrimidin-2-yl)-N-methylpropan-1-amine hydrochloride

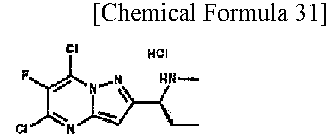

By using the same approaches as in Reference Example 1-1 (1) and (3), the title compound (0.61 g, brown gum) was obtained as a crude product from tert-butyl [(1S)-1-(5-amino-1H-pyrazol-3-yl)propyl]methylcarbamate (0.30 g) and diethyl fluoropropanedioate (0.30 g).
LCMS (ESI) m/z 277 [M+H]⁺, t_{R} = 0.758 min, mode H.

### Reference Example 1-18: Synthesis of tert-butyl [(1S)-1-(7-chloro-5-cyclopropylpyrazolo[1,5-a]pyrimidin-2-yl)propyl]methylcarbamate

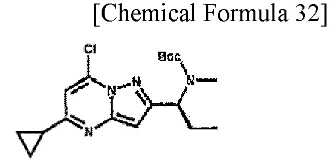

By using the same approaches as in Reference Example 1-6 (1) and (2), the title compound (0.35 g, colorless gum) was obtained by using tert-butyl [(1S)-1-(5-amino-1H-pyrazol-3-yl)propyl]methylcarbamate (3.4 g) instead of tert-butyl (2S)-2-(5-amino-1H-pyrazol-3-yl)piperidine-1-carboxylate as the starting raw material. ¹H NMR (400 MHz, CDCl₃) δ ppm 0.95 - 1.20 (m, 7H), 1.49 (s, 9H), 1.87 - 2.08 (m, 2H), 2.14 - 2.33 (m, 1H), 2.64 - 2.82 (m, 3H), 5.23 - 5.56 (m, 1H), 6.41 (s, 1H), 6.75 (s, 1H). LCMS (ESI/APCI dual) m/z 365 [M+H]⁺, t_{R} = 1.222 min, mode N.

### Reference Example 1-19: Synthesis of 1-(5,7-dichloropyrazolo[1,5-a]pyrimidin-2-yl)-N-methylmethanamine hydrochloride

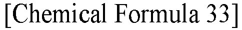

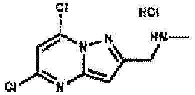

By using the same approaches as in Reference Example 1-1 (1) and (3), the title compound (brown gum) was obtained as a crude product by using tert-butyl [(5-amino-1H-pyrazol-3-yl)methyl]methylcarbamate (2.0 g) instead of tert-butyl (2S)-2-(5-amino-1H-pyrazol-3-yl)piperidine-1-carboxylate as the starting raw material. LCMS (ESI) m/z 231 [M+H]⁺, t_{R} = 0.419 min, mode N.

### Reference Example 1-20: Synthesis of 2-(5,7-dichloropyrazolo[1,5-a]pyrimidin-2-yl)-N-methylpropan-2-amine hydrochloride

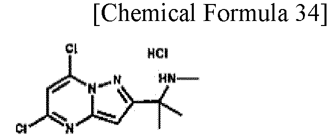

By using the same approaches as in Reference Example 1-1 (1) and (3), the title compound (brown gum) was obtained as a crude product by using tert-butyl [2-(5-amino-1H-pyrazol-3-yl)propan-2-yl]methylcarbamate (1.4 g) instead of tert-butyl (2S)-2-(5-amino-1H-pyrazol-3-yl)piperidine-1-carboxylate as the starting raw material. LCMS (ESI) m/z 259 [M+H]⁺, t_{R} = 0.236 min, mode N.

### Reference Example 1-21: Synthesis of tert-butyl (2S)-2-[5-(azetidin-1-yl)-7-(hydroxymethyl)pyrazolo[1,5-a]pyrimidin-2-yl]piperidine-1-carb oxylate

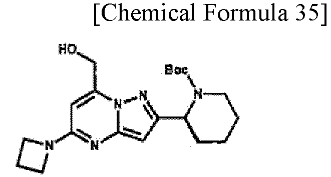

### (1) To a solution of tert-butyl

(2S)-2-(5-amino-1H-pyrazol-3-yl)piperidine-1-carboxylate (1.1 g) in MeOH (40 mL), methyl 4-[tert-butyl(dimethyl)silyl]oxybut-2-ynoate (1.0 g) was added, and the mixture was stirred at 80°C for 21 hours. The reaction liquid was poured into water and extracted with EtOAc. The organic layer was washed with Brine, dried over MgSO₄, filtered, and then the solvent was concentrated. The residue was purified by OH type silica gel column chromatography (mobile phase: hexane/EtOAc) to afford tert-butyl
(2S)-2-[7-[[tert-butyl(dimethyl)silyl]oxymethyl]-5-oxo-4H-pyrazolo[1,5-a]pyrimidin-2-yl]pi peridine-1-carboxylate (0.45 g, yellow powder).

### (2) To a solution of tert-butyl

(2S)-2-[7-[[tert-butyl(dimethyl)silyl]oxymethyl]-5-oxo-4H-pyrazolo[1,5-a]pyrimidin-2-yl]pi peridine-1-carboxylate (0.44 g) in CHCl₃ (15 mL), 2,6-lutidine (0.22 mL) and Tf₂O (0.19 mL) were added at -78°C, and the mixture was stirred at room temperature for 1 hour. The reaction liquid was poured into water and extracted with CHCl₃. The organic layer was washed with Brine, dried over MgSO₄, filtered, and then the solvent was concentrated to afford tert-butyl
(2S)-2-[7-[[tert-butyl(dimethyl)silyl]oxymethyl]-5-(trifluoromethylsulfonyloxy)pyrazolo[1,5-a]pyrimidin-2-yl]piperidine-1-carboxylate (1.0 g, brown oily matter).

### (3) To a solution of tert-butyl

(2S)-2-[7-[[tert-butyl(dimethyl)silyl]oxymethyl]-5-(trifluoromethylsulfonyloxy)pyrazolo[1,5-a]pyrimidin-2-yl]piperidine-1-carboxylate (crude product, ca. 0.95 mmol) in THF (6.0 mL), azetidine hydrochloride (0.37 g) and Et₃N (1.1 mL) were added, and the mixture was stirred at 70°C for 1.5 hours. The reaction liquid was poured into water and extracted with EtOAc. The organic layer was washed with Brine, dried over MgSO₄, filtered, and then the solvent was concentrated. The residue was purified by OH type silica gel column chromatography (mobile phase: hexane/EtOAc) to afford tert-butyl
(2S)-2-[5-(azetidin-1-yl)-7-[[tert-butyl(dimethyl)silyl]oxymethyl]pyrazolo[1,5-a]pyrimidin-2 -yl]piperidine-1-carboxylate (0.42 g, yellow oily matter).

### (4) To a solution of tert-butyl

(2S)-2-[5-(azetidin-1-yl)-7-[[tert-butyl(dimethyl)silyl]oxymethyl]pyrazolo[1,5-a]pyrimidin-2 -yl]piperidine-1-carboxylate (0.41 g) in THF (5.0 mL), TBAF (1.6 mL, 1 M THF solution) was added at 0°C, and the mixture was stirred at 0°C for 4 hours. The reaction liquid was poured into water and extracted with EtOAc. The organic layer was washed with Brine, dried over MgSO₄, filtered, and then the solvent was concentrated. The residue was purified by OH type silica gel column chromatography (mobile phase: hexane/EtOAc) to afford tert-butyl
(2S)-2-[5-(azetidin-1-yl)-7-(hydroxymethyl)pyrazolo[1,5-a]pyrimidin-2-yl]piperidine-1-carb oxylate (0.31 g, colorless amorphous).
¹H NMR (400 MHz, CDCl₃) δ ppm 1.39 - 1.67 (m, 13H), 1.75 - 1.90 (m, 1H), 2.28 - 2.50 (m, 3H), 2.78 - 2.97 (m, 1H), 3.95 - 4.08 (m, 1H), 4.09 - 4.24 (m, 4H), 4.47 (br s, 1H), 4.80 - 4.91 (m, 2H), 5.39 - 5.58 (m, 1H), 5.82 (s, 1H), 5.96 (s, 1H).
LCMS (ESI) m/z 388 [M+H]⁺, t_{R} = 0.64 min, mode L.

### Reference Example 1-22: Synthesis of tert-butyl (2S)-2-[5-(3-{[tert-butyl(diphenyl)silyl]oxy}azetidin-1-yl)-7-(hydroxymethyl)pyrazolo[1,5-a] pyrimidin-2-yl]piperidine-1-carboxylate

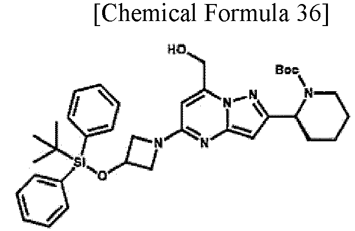

(1) To a solution of the compound (0.59 g) obtained in Reference Example 1-20 (2) in THF (6.0 mL), Et₃N (1.1 mL) and 3-((tert-butyldiphenylsilyl)oxy)azetidine (1.2 g) were added, and the mixture was stirred at 70°C for 2 hours and at room temperature overnight. To the reaction liquid, in addition to a Sat. NaHCO₃ aq., EtOAc was added for extraction, and the extract was dried over MgSO₄, filtered, and concentrated. The residue was purified by OH type silica gel column chromatography (mobile phase: hexane/EtOAc = 97/3 to 70/30; v/v) to afford tert-butyl
(2S)-2-[7-[[tert-butyl(dimethyl)silyl]oxymethyl]-5-[3-[tert-butyl(diphenyl)silyl]oxyazetidin-1 -yl]pyrazolo[1,5-a]pyrimidin-2-yl]piperidine-1-carboxylate (0.70 g, yellow oily matter).

(2) To a solution of tert-butyl (2S)-2-[7-[[tert-butyl(dimethyl)silyl]oxymethyl]-5-[3-[tert-butyl(diphenyl)silyl]oxyazetidin-1 -yl]pyrazolo[1,5-a]pyrimidin-2-yl]piperidine-1-carboxylate (0.65 g) in THF/MeOH (6.8 mL/2.9 mL; v/v), a 1 M HCl aq. (17 mL) was added, and the mixture was stirred at room temperature for 3 hours. To the reaction liquid, a 1 M NaOH aq. was added, and EtOAc and Brine were added for the separating operation. The filtrate dried over MgSO₄ was concentrated under reduced pressure. The residue was purified by OH type silica gel column chromatography (mobile phase: hexane/EtOAc = 80/20 to 50/50; v/v) to afford tert-butyl
(2S)-2-[5-[3-[tert-butyl(diphenyl)silyl]oxyazetidin-1-yl]-7-(hydroxymethyl)pyrazolo[1,5-a]p yrimidin-2-yl]piperidine-1-carboxylate (0.41 g, colorless oily matter).
¹H NMR (400 MHz, CDCl₃) δ ppm 1.06 (s, 9H), 1.39 - 1.67 (m, 15H), 1.74 - 1.88 (m, 1H), 2.29 - 2.37 (m, 1H), 2.80 - 2.91 (m, 1H), 3.96 - 4.23 (m, 4H), 4.69 - 4.76 (m, 1H), 4.84 (s, 2H), 5.49 (br s, 1H), 5.80 (s, 1H), 5.96 (s, 1H), 7.37 - 7.48 (m, 6H), 7.59 - 7.66 (m, 4H).

### Reference Example 1-23: Synthesis of tert-butyl (2S)-2-{5-[3-(benzyloxy)azetidin-1-yl]-7-chloropyrazolo[1,5-a]pyrimidin-2-yl}piperidine-1-carboxylate

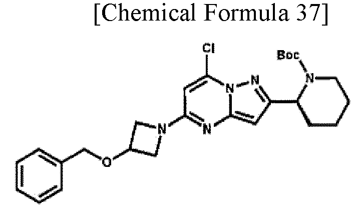

(1) To a solution of tert-butyl
(2S)-2-(5,7-dichloropyrazolo[1,5-a]pyrimidin-2-yl)piperidine-1-carboxylate (3.6 g) in MeCN (20 mL), a 1 M NaOH aq. (20 mL) was added, and the mixture was stirred at 65°C for 3 hours. After adding a 1 M HCl aq. (50 mL) to the reaction liquid for neutralization, the aqueous layer was extracted with EtOAc. The obtained organic layer was washed with Brine, dried over MgSO₄, filtered, and concentrated to afford the residue. Purification by OH type silica gel column chromatography (mobile phase: CHCl₃/MeOH = 100/0 to 80/20; v/v) afforded tert-butyl
(2S)-2-(5-chloro-7-hydroxy-pyrazolo[1,5-a]pyrimidin-2-yl)piperidine-1-carboxylate (3.7 g, blue amorphous).

(2) A suspension of tert-butyl
(2S)-2-(5-chloro-7-hydroxy-pyrazolo[1,5-a]pyrimidin-2-yl)piperidine-1-carboxylate (3.0 g), 3-(benzyloxy)azetidine hydrochloride, RuPhos (R) (0.39 g), PdG3-Ruphos (R) (3.9 g), and C_{S2}CO₃ (14 g) in THF (42 mL) was stirred at 75°C for 37 hours. The reaction liquid was filtered through Celite (R) to remove insolubles. The mother liquor was divided with EtOAc/water, and the obtained organic layer was dried over MgSO₄, filtered, washed, and concentrated to afford the residue. The obtained residue was purified by OH type silica gel column chromatography (mobile phase: CHCl₃/MeOH = 100/0 to 80/20; v/v) to afford tert-butyl
(2S)-2-[5-(3-benzyloxyazetidin-1-yl)-7-hydroxy-pyrazolo[1,5-a]pyrimidin-2-yl]piperidine-1-carboxylate (1.7 g, blue amorphous).

(3) To a solution of tert-butyl
(2S)-2-[5-(3-benzyloxyazetidin-1-yl)-7-hydroxy-pyrazolo[1,5-a]pyrimidin-2-yl]piperidine-1-carboxylate (1.7 g) in CHCl₃ (35 mL), Et₃N (1.5 mL) and Tf₂O (0.90 mL) were added under ice cooling, and the mixture was stirred at 0°C for 1 hour. Water was added to the reaction liquid. The aqueous layer was extracted with CHCl₃. The obtained organic layer was dried over MgSO₄, filtered, and concentrated to afford the residue. To the residue, a 4 M hydrochloric acid 1,4-dioxane solution (30 mL) was added, and the mixture was stirred at room temperature for 5 hours. The reaction solution was concentrated under reduced pressure to afford the residue. To a solution of the residue in CHCl₃ (35 mL), BoC₂O (2.3 g) and Et₃N (5.0 mL) were sequentially added under ice cooling, and the mixture was stirred at room temperature for 15 hours. To the reaction solution, a Sat. NaHCO₃ aq. and CHCl₃ were added, and the separating operation was carried out. The obtained organic layer was dried over MgSO₄, filtered, and concentrated to afford the residue. To the obtained residue, EtOAc and NH silica (16 g) were added, and the mixture was stirred at room temperature for 10 minutes. Thereafter, the NH silica was filtered off and washed, and the mother liquor was concentrated to afford the residue. Purification by OH type silica gel column chromatography (mobile phase: hexane/EtOAc = 88/12 to 0/100; v/v) afforded the title compound (0.35 g, light yellow amorphous).
¹H NMR (400 MHz, CDCl₃) δ ppm 1.35 - 1.65 (m, 4H), 1.46 (s, 9H), 1.76-1.88 (m, 1H), 2.44 (d, J = 12.0 Hz, 1H), 2.90 (t, J = 12.0 Hz, 1H), 3.97-4.11 (m, 3H), 4.22-4.31 (m, 2H), 4.49-4.56 (m, 1H), 4.53 (s, 2H), 5.53 (brs, 1H), 6.00(s, 1H), 6.02 (s, 1H), 7.30-7.43 (m, 5H). LCMS (ESI) m/z 498 [M+H]⁺, t_{R} = 1.402 min, mode N.

### Reference Example 1-24: Synthesis of tert-butyl (2S)-2-(7-chloro-5-{cis-3-[(prop-2-en-1-yl)oxy]cyclobutyl}pyrazolo[1,5-a]pyrimidin-2-yl)pi peridine-1-carboxylate

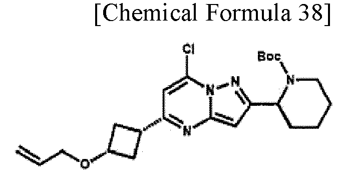

By using the same approaches as in Reference Example 1-6 (1), (2), and Reference Example 1-2, the title compound (0.24 g, colorless oily matter) was obtained from tert-butyl (2S)-2-(5-amino-1H-pyrazol-3-yl)piperidine-1-carboxylate (0.65 g) and ethyl 3-(3-(allylcyclobutyl)-3-oxopropanoate (0.66 g).
¹H NMR (400 MHz, CDCl₃) δ ppm 1.17 - 1.73 (m, 13H), 1.76 - 1.96 (m, 1H), 2.26 - 2.40 (m, 2H), 2.43 - 2.58 (m, 1H), 2.62 - 2.80 (m, 2H), 2.80 - 3.00 (m, 1H), 3.14 (quin, J = 8.9 Hz, 1H), 3.95 (d, J = 5.6 Hz, 2H), 3.99 - 4.15 (m, 2H), 5.19 (d, J = 10.3 Hz, 1H), 5.30 (d, J = 17.2 Hz, 1H), 5.63 (br s, 1H), 5.85 - 6.03 (m, 1H), 6.48 (s, 1H), 6.81 (s, 1H).
LCMS (ESI) m/z 447 [M+H]⁺, t_{R} = 0.412 min, mode N.

### Reference Example 2-1: Synthesis of methyl 5-fluoro-2-(2-{ [2-(methylamino)ethyl]amino}-2-oxoethoxy)benzoate hydrochloride

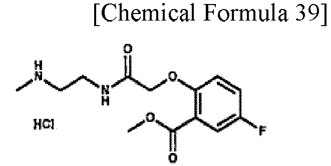

(1) To a solution of tert-butyl N-(2-aminoethyl)-N-methyl-carbamate (5.0 g) in CHCl₃ (0.10 L), 2-chloroacetyl chloride (2.7 mL) and Et₃N (8.0 mL) were added, and the mixture was stirred at room temperature for 1 hour. The reaction solution was poured into water and extracted with CHCl₃. The organic layer was washed with Brine, dried over MgSO₄, filtered, and then the solvent was concentrated. The residue was purified by OH type silica gel column chromatography (mobile phase: hexane/EtOAc = 50/50 to 0/100; v/v) to afford tert-butyl [2-(2-chloroacetamido)ethyl]methylcarbamate (6.2 g, yellow powder). ¹H NMR (400 MHz, CDCl₃) δ ppm 1.47 (s, 9H), 2.69 - 3.06 (m, 3H), 3.45 (br s, 4H), 3.83 - 4.20 (m, 2H), 6.76 (br s, 0.3H), 7.36 (br s, 0.7H).
(2) To a solution of tert-butyl [2-(2-chloroacetamido)ethyl]methylcarbamate (6.8 g) in DMF (40 mL), methyl 5-fluoro-2-hydroxy-benzoate (5.5 g) and K₂CO₃ (7.5 g) were added, and the mixture was stirred at 80°C for 3 hours. The reaction solution was poured into water and extracted with EtOAc. The organic layer was washed with Brine, dried over MgSO₄, filtered, and then the solvent was concentrated. The residue was purified by OH type silica gel column chromatography (mobile phase: hexane/EtOAc = 50/50 to 0/100; v/v) to afford methyl
   2-[2-({2-[(tert-butoxycarbonyl)(methyl)amino]ethyl}amino)-2-oxoethoxy]-5-fluorobenzoate (10 g, light yellow oily matter).
   ¹H NMR (400 MHz, CDCl₃) δ ppm 1.41 (s, 9H), 2.91 (s, 3H), 3.36 - 3.47 (m, 2H), 3.49 - 3.61 (m, 2H), 3.92 (s, 3H), 4.54 (s, 2H), 6.89 (dd, J = 9.0, 4.0 Hz, 1H), 7.18 - 7.25 (m, 1H), 7.63 (dd, J = 8.7, 3.0 Hz, 1H), 8.06 - 8.52 (m, 1H).
   LCMS (ESI/APCI dual) m/z 385 [M+H]⁺, t_{R} = 0.888 min, mode N.
(3) A solution of methyl 2-[2-({2-[(tert-butoxycarbonyl)(methyl)amino]ethyl}amino)-2-oxoethoxy]-5-fluorobenzoate (10 g) in 4 M hydrochloric acid 1,4-dioxane (80 mL) was stirred at room temperature overnight. The reaction solution was concentrated to afford the title compound (5.4 g, colorless powder).
   ¹H NMR (400 MHz, CDCl₃) δ ppm 2.77 (br s, 3H), 3.16 - 3.34 (m, 2H), 3.76 - 3.87 (m, 2H), 3.90 (s, 3H), 4.66 (br s, 2H), 6.85 - 6.98 (m, 1H), 7.15 - 7.25 (m, 1H), 7.51 - 7.72 (m, 1H), 8.70 - 8.95 (m, 1H), 9.51 - 9.85 (m, 2H).
   LCMS (ESI/APCI dual) m/z 285 [M+H]⁺, t_{R} = 0.205 min, mode N.

### Reference Example 2-2: Synthesis of methyl 2-{2-[(2-aminoethyl)amino]-2-oxoethoxy}-5-fluorobenzoate hydrochloride

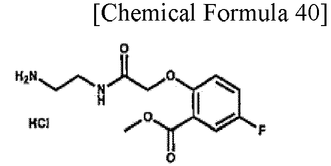

By using the same approaches as in Reference Example 2-1 (1) to (3), the title compound (3.8 g, colorless powder) was obtained by using tert-butyl (2-aminoethyl)carbamate (3.0 g) instead of tert-butyl N-(2-aminoethyl)-N-methyl-carbamate. LCMS (ESI/APCI dual) m/z 271 [M+H]⁺, t_{R} = 0.220 min, mode N.

### Reference Example 2-3: Synthesis of methyl 2-(2-{[2-(ethylamino)ethyl]amino}-2-oxoethoxy)-5-fluorobenzoate

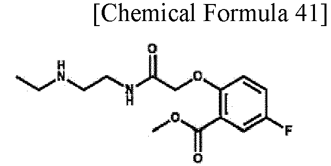

To a solution of methyl 2-{2-[(2-aminoethyl)amino]-2-oxoethoxy}-5-fluorobenzoate (0.50 g) obtained in Reference Example 2-2 (3) in CHCl₃ (8.2 mL), acetaldehyde (0.65 mL) and AcOH (0.28 mL) were added, and the mixture was stirred at room temperature for 0.5 hours. After that, NaBH(OAc)₃ (1.0 g) was added and the mixture was stirred at room temperature for 7 hours. To the reaction liquid, a Sat. NaHCO₃ aq. was added, and the mixture was extracted with CHCl₃. The organic layer was collected by using Phase Separator and concentrated. The residue was purified by NH type silica gel column chromatography (mobile phase: hexane/EtOAc = 50/50 to 0/100; v/v) to afford the title compound (0.22 g, yellow oily matter).
¹H NMR (400 MHz, CDCl₃) δ ppm 1.13 (t, J = 7.0 Hz, 3H), 2.71 (q, J = 7.0 Hz, 2H), 2.82 - 2.91 (m, 2H), 3.45 - 3.57 (m, 2H), 3.91 (s, 3H), 4.55 (s, 2H), 6.83 - 6.94 (m, 1H), 7.17 - 7.29 (m, 2H), 7.55 - 7.70 (m, 1H), 8.20 (br s, 1H).
LCMS (ESI) m/z 299 [M+H]⁺, t_{R} = 0.698 min, mode H.

### Reference Example 2-4: Synthesis of methyl 5-fluoro-2-[2-oxo-2-({2-[(propan-2-yl)amino]ethyl}amino)ethoxy]benzoate

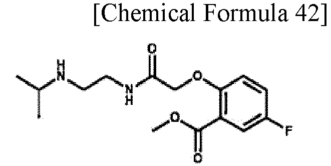

By using the same approach as in Reference Example 2-3, the title compound (0.41 g, yellow oily matter) was obtained from methyl
2-{2-[(2-aminoethyl)amino]-2-oxoethoxy}-5-fluorobenzoate (0.49 g) obtained in Reference Example 2-2 (3), using acetone instead of acetaldehyde.
¹H NMR (400 MHz, CDCl₃) δ ppm 1.07 (d, J = 6.2 Hz, 6H), 2.80 - 2.92 (m, 3H), 3.50 (q, J = 5.8 Hz, 2H), 3.91 (s, 3H), 4.55 (s, 2H), 6.89 (dd, J = 9.0, 3.9 Hz, 1H), 7.20 - 7.25 (m, 1H), 7.64 (dd, J = 8.6, 2.6 Hz, 1H), 8.19 (br s, 1H).
LCMS (ESI) m/z 313 [M+H]⁺, t_{R} = 0.746 min, mode H.

### Reference Example 2-5: Synthesis of methyl 2-(2-{[2-(cyclopropylamino)ethyl]amino}-2-oxoethoxy)-5-fluorobenzoate

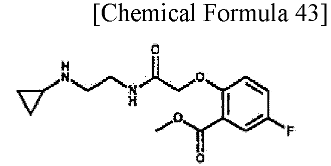

(1) To a solution of 2-(4-fluoro-2-methoxycarbonyl-phenoxy)acetic acid (4.2 g) in DMF (62 mL), Et₃N (18 mL), 2-aminoethanol (1.4 g) and HATU (8.4 g) were added, and the mixture was stirred at room temperature for 21 hours. To the reaction liquid, a Sat. NaHCO₃ aq. was added, and the aqueous layer was extracted with EtOAc. The organic layer was dried over MgSO₄ and then concentrated under reduced pressure. The residue was purified by NH type silica gel column chromatography (mobile phase: CHCl₃/MeOH = 100/0 to 95/5; v/v). The residue was suspended in Et₂O, which was filtered to afford methyl 5-fluoro-2-[2-(2-hydroxyethylamino)-2-oxo-ethoxy]benzoate (1.7 g, colorless powder).
(2) To a solution of methyl
   5-fluoro-2-[2-(2-hydroxyethylamino)-2-oxo-ethoxy]benzoate (0.41 g) in CHCl₃ (5.0 mL), the Dess-Martin reagent (0.70 g) was added, and the mixture was stirred at room temperature for 1 hour. To the reaction liquid, a Sat. NaHCO₃ aq. was added, and the mixture was extracted with CHCl₃. The organic layer was collected by using Phase Separator and concentrated.
   Methyl 5-fluoro-2-[2-oxo-2-(2-oxoethylamino)ethoxy]benzoate was obtained as a crude product.
(3) To a solution of methyl 5-fluoro-2-[2-oxo-2-(2-oxoethylamino)ethoxy]benzoate (0.40 g) in MeOH (5.0 mL), cyclopropanamine (0.17 g) and AcOH (0.26 mL) were added, and the mixture was stirred at room temperature for 0.5 hours. To this, 2-methylpyridine borane complex (0.32 g) was added, and the mixture was stirred at room temperature for 16 hours. To the reaction liquid, a Sat. NaHCO₃ aq. was added, and the mixture was extracted with CHCl₃. The organic layer was collected by using Phase Separator and concentrated. The obtained residue was purified by NH type silica gel column chromatography (mobile phase: hexane/EtOAc = 80/20 to 0/100 → CHCl₃/MeOH = 100/0 to 95/5; v/v) to afford the title compound (0.39 g, yellow oily matter).
   LCMS (ESI) m/z 311 [M+H]⁺, t_{R} = 0.729 min, mode H.

### Reference Example 2-6: Synthesis of methyl 2-{2-[(2-{[2-(benzyloxy)ethyl]amino}ethyl)amino]-2-oxoethoxy}-5-fluorobenzoate

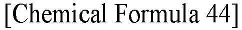

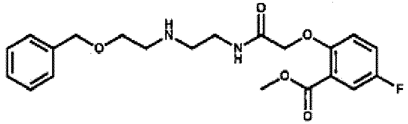

By using the same approach as in Reference Example 2-3, the title compound (0.15 g, colorless oily matter) was obtained from methyl
2-{2-[(2-aminoethyl)amino]-2-oxoethoxy}-5-fluorobenzoate (0.50 g) obtained in Reference Example 2-2 (3), using benzyloxyacetaldehyde instead of acetaldehyde.
¹H NMR (400 MHz, CDCl₃) δ ppm 2.85 - 2.91 (m, 4H), 3.47 - 3.54 (m, 2H), 3.58 - 3.64 (m, 2H), 3.87 (s, 3H), 4.51 (s, 2H), 4.53 (s, 2H), 6.83 - 6.92 (m, 1H), 7.16 - 7.34 (m, 7H), 7.57 - 7.65 (m, 1H), 8.20 (br s, 1H).
LCMS (ESI) m/z 405 [M+H]⁺, t_{R} = 1.006 min, mode H.

### Reference Example 2-7: Synthesis of methyl 5-fluoro-2-[2-({2-[(trideuteriomethyl)amino]ethyl}amino)-2-oxoethoxy]benzoate hydrochloride

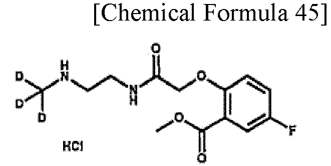

(1) To a solution of methyl
2-{2-[(2-aminoethyl)amino]-2-oxoethoxy}-5-fluorobenzoate (0.50 g) obtained in Reference Example 2-2 (3) in MeOH (10 mL), ethyl trifluoroacetate (0.39 mL) and Et₃N (0.68 mL) were added, and the mixture was stirred at room temperature for 1 hour. The reaction solution was concentrated to afford methyl
   5 -fluoro-2- [2-oxo-2-[2- [(2,2,2-trifluoroacetyl)amino] ethyl amino]ethoxy]benzoate (0.62 g, yellow oily matter).

(2) To a solution of methyl
5-fluoro-2-[2-oxo-2-[2-[(2,2,2-trifluoroacetyl)amino]ethylamino]ethoxy]benzoate (0.62 g) in DMF (6.0 mL), CD₃I (0.22 mL) and Cs₂CO₃ (1.6 g) were added, and the mixture was stirred at 65°C for 1 hour. The reaction solution was poured into water and extracted with EtOAc. The organic layer was washed with Brine, dried over MgSO₄, filtered, and then the solvent was concentrated. The residue was purified by OH type silica gel column chromatography (mobile phase: CHCl₃/MeOH = 95/5 to 90/10; v/v) to afford methyl
5-fluoro-2-[2-oxo-2-[2-[trideuteriomethyl-(2,2,2-trifluoroacetyl)amino]ethylamino]ethoxy]be nzoate (0.55 g, colorless powder).

(3) To a solution of methyl
5-fluoro-2-[2-oxo-2-[2-[trideuteriomethyl-(2,2,2-trifluoroacetyl)amino]ethylamino]ethoxy]be nzoate (0.55 g) in MeOH (5.0 mL), water (5.0 mL) and K₂CO₃ (0.40 g) were added, and the mixture was stirred at room temperature for 2 hours. The reaction solution was poured into water and extracted with CHCl₃. The organic layer was washed with Brine, dried over MgSO₄, filtered, and then the solvent was concentrated. The residue was purified by NH type silica gel column chromatography (mobile phase: EtOAc/MeOH = 100/0 to 90/10; v/v) to afford the title compound (0.28 g, colorless powder).
¹H NMR (400 MHz, CDCl₃) δ ppm 2.75 - 2.94 (m, 2H), 3.39 - 3.62 (m, 2H), 3.92 (s, 3H), 4.55 (s, 2H), 6.79 - 6.98 (m, 1H), 7.16 - 7.30 (m, 2H), 7.53 - 7.76 (m, 1H), 8.05 - 8.34 (m, 1H).
LCMS (ESI) m/z 288 [M+H]⁺, t_{R} = 0.243 min, mode H.

### Reference Example 2-8: Synthesis of methyl 5-fluoro-2-[(2-methyl-1-{[2-(methylamino)ethyl]amino}-1-oxopropan-2-yl)oxy]benzoate hydrochloride

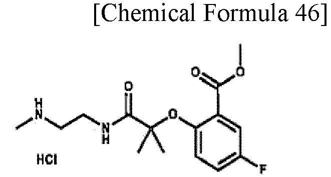

(1) To a solution of 2-(4-fluoro-2-methoxycarbonyl-phenoxy)-2-methyl-propanoic acid (1.5 g) in DMF (11 mL), tert-butyl N-(2-aminoethyl)-N-methyl-carbamate (1.1 mL), HATU (2.5 g), and Et₃N (5.6 mL) were added, and the mixture was stirred at room temperature for 30 minutes. To the reaction liquid, a Sat. NaHCO₃ aq. was added, and the mixture was extracted with hexane/EtOAc (1/1; v/v), which was washed with Brine, dried over MgSO₄, filtered, and concentrated. Purification by OH type silica gel column chromatography (mobile phase: hexane/EtOAc = 90/10 to 50/50; v/v) afforded methyl 2-[2-[2-[tert-butoxycarbonyl(methyl)amino]ethylamino]-1,1-dimethyl-2-oxo-ethoxy]-5-fluor o-benzoate (2.2 g, yellow oily matter).

(2) To a solution of methyl
2-[2-[2-[tert-butoxycarbonyl(methyl)amino]ethylamino]-1,1-dimethyl-2-oxo-ethoxy]-5-fluor o-benzoate (0.62 g) in 1,4-dioxane (3.0 mL), a 4 M hydrochloric acid 1,4-dioxane solution (3.0 mL) was added, and the mixture was stirred at room temperature for 1.5 hours. The reaction liquid was concentrated to afford the title compound (0.43 g, light yellow oily matter).
LCMS (ESI) m/z 313 [M+H]⁺, t_{R} = 0.797 min, mode H.

### Reference Example 2-9: Synthesis of methyl 5-fluoro-2-(2-{[2-methyl-1-(methylamino)propan-2-yl]amino}-2-oxoethoxy)benzoate hydrochloride

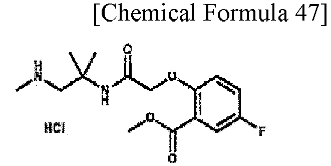

By using the same approaches as in Reference Example 2-1 (1), (2), and (3), the title compound (0.11 g, colorless powder) was obtained by using tert-butyl N-(2-amino-2-methyl-propyl)-N-methyl-carbamate (91 mg) instead of tert-butyl N-(2-aminoethyl)-N-methyl-carbamate.
¹H NMR (400 MHz, CDCl₃) δ ppm 1.61 (s, 6H), 2.71 (br s, 3H), 3.14 - 3.29 (m, 2H), 3.89 (s, 3H), 4.57 (s, 2H), 6.90 (dd, J = 9.0, 4.2 Hz, 1H), 7.64 (dd, J = 8.6, 3.1 Hz, 1H), 8.62 (br s, 1H), 10.00 - 10.25 (m, 2H).
LCMS (ESI) m/z 313 [M+H]⁺, t_{R} = 0.435 min, mode N.

### Reference Example 2-10: Synthesis of methyl 5-fluoro-2-[(1-{[2-(methylamino)ethyl]amino}-1-oxopropan-2-yl)oxy]benzoate hydrochloride

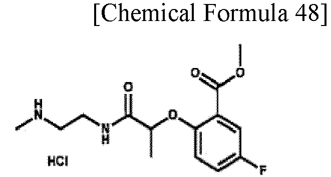

By using the same approaches as in Reference Example 2-1 (2) and (3), the title compound (1.2 g, colorless powder) was obtained by using 2-chloropropanoyl chloride (0.80 g) instead of 2-chloroacetyl chloride.
LCMS (ESI) m/z 299 [M+H]⁺, t_{R} = 0.741 min, mode H.

### Reference Example 2-11: Synthesis of tert-butyl 5-fluoro-2-[2-oxo-2-[[2,2,2-trifluoro-1-(methylaminomethyl)ethyl]amino]ethoxy]benzoate

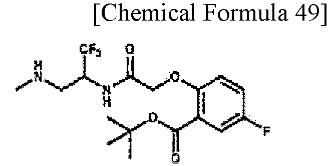

(1) By using the same approach as in Reference Example 2-8 (1), tert-butyl 2-[2-[[1-[[benzyl(methyl)amino]methyl]-2,2,2-trifluoro-ethyl]amino]-2-oxo-ethoxy]-5-fluoro -benzoate (0.78 g, colorless powder) was obtained from 2-(2-tert-butoxycarbonyl-4-fluoro-phenoxy)acetic acid (0.53 g) and N1-benzyl-3,3,3-trifluoro-N1-methyl-propane-1,2-diamine dihydrochloride (0.54 g).

(2) To a solution of tert-butyl 2-[2-[[1-[[benzyl(methyl)amino]methyl]-2,2,2-trifluoro-ethyl]amino]-2-oxo-ethoxy]-5-fluoro -benzoate (0.78 g) in MeOH (16 mL), 10% palladium-carbon (0.39 g) was added, and the mixture was stirred at room temperature for 3 hours under a hydrogen gas atmosphere. Celite (R) filtration was carried out. The filtrate was concentrated to afford the title compound (0.62 g, colorless powder). ¹H NMR (400 MHz, CDCl₃) δ ppm 1.59 (s, 9H), 2.45 (s, 3H), 3.02 (d, J = 6.6 Hz, 2H), 4.64 (s, 2H), 4.77 - 4.90 (m, 1H), 6.88 (dd, J =9.0, 4.2 Hz, 1H), 7.16 - 7.24 (m, 1H), 7.54 (dd, J =8.8, 2.9 Hz, 1H), 8.91 (d, J = 9.2 Hz, 1H).
LCMS (ESI) m/z 395 [M+H]⁺, t_{R} = 0.679 min, mode N.

### Reference Example 2-12: Synthesis of methyl 5-chloro-2-(2-{[2-(methylamino)ethyl]amino}-2-oxoethoxy)benzoate hydrochloride

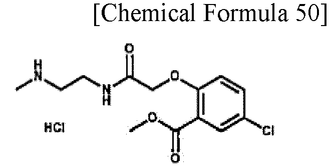

By using the same approaches as in Reference Example 2-1 (2) and (3), the title compound (7.2 g, colorless powder) was obtained by using methyl 5-chloro-2-hydroxy-benzoate (6.1 g) instead of methyl 5-fluoro-2-hydroxy-benzoate. ¹H NMR (400 MHz, CDCl₃) δ ppm 2.68 - 2.85 (m, 3H), 3.15 - 3.37 (m, 2H), 3.73 - 3.84 (m, 2H), 3.90 (s, 3H), 4.56 - 4.76 (m, 2H), 6.81 - 6.96 (m, 1H), 7.45 (br s, 1H), 7.76 - 7.95 (m, 1H), 8.51 - 8.99 (m, 1H), 9.59 (br s, 2H).
LCMS (ESI/APCI dual) m/z 301 [M+H]⁺, t_{R} = 0.210 min, mode N.

### Reference Example 2-13: Synthesis of methyl 5-chloro-2-[2-({2-[(trideuteriomethyl)amino]ethyl}amino)-2-oxoethoxy]benzoate hydrochloride

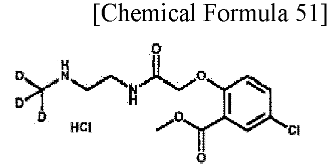

By using the same approaches as in Reference Example 2-8 (1) and (2), the title compound (2.4 g, colorless powder) was obtained from [4-chloro-2-(methoxycarbonyl)phenoxy]acetic acid (2.0 g) and tert-butyl (2-aminoethyl)(trideuteriomethyl)carbamate (1.5 g).
¹H NMR (400 MHz, CDCl₃) δ ppm 3.15 - 3.32 (m, 2H), 3.69 - 3.85 (m, 2H), 3.89 (s, 3H), 4.58 - 4.74 (m, 2H), 6.81 - 6.95 (m, 1H), 7.38 - 7.53 (m, 1H), 7.77 - 7.97 (m, 1H), 8.51 - 8.78 (m, 1H), 9.55 (br s, 2H).
LCMS (ESI/APCI dual) m/z 304 [M+H]⁺, t_{R} = 0.365 min, mode N.

### Reference Example 2-14: Synthesis of methyl 5-methyl-2-(2-{[2-(methylamino)ethyl]amino}-2-oxoethoxy)benzoate hydrochloride

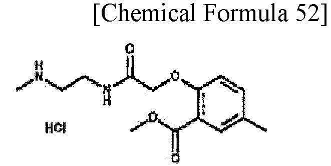

By using the same approaches as in Reference Example 2-1 (2) and (3), the title compound (0.52 g, colorless powder) was obtained by using methyl 2-hydroxy-5-methyl-benzoate (0.64 g) instead of methyl 5-fluoro-2-hydroxy-benzoate. ¹H NMR (400 MHz, DMSO-d₆) δ ppm 2.28 (s, 3H), 2.53 - 2.58 (m, 3H), 3.01 (quin, J =6.0 Hz, 2H), 3.49 (q, J = 6.0 Hz, 2H), 4.39 (br s, 3H), 4.58 (s, 2H), 7.06 (d, J = 8.4 Hz, 1H), 7.37 (dd, J = 8.4, 2.0 Hz, 1H), 7.57 (d, J = 2.0 Hz, 1H), 8.25 (t, J = 5.7 Hz, 1H), 8.85 (br s, 2H).
LCMS (ESI) m/z 281 [M+H]⁺, t_{R} = 0.315 min, mode N.

### Reference Example 2-15: Synthesis of methyl 5-(trideuteriomethyl)-2-(2-{[2-(methylamino)ethyl]amino}-2-oxoethoxy)benzoate hydrochloride

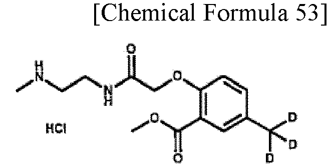

By using the same approaches as in Reference Example 2-8 (1) and (2), the title compound (5.6 g, colorless powder) was obtained from [2-(methoxycarbonyl)-4-(trideuteriomethyl)phenoxy]acetic acid (8.2 g) and tert-butyl (2-aminoethyl)methylcarbamate (6.9 g).
¹H NMR (400 MHz, DMSO-d₆) δ ppm 2.55 (s, 3H), 2.93 - 3.08 (m, 2H), 3.45 - 3.54 (m, 2H), 3.83 (s, 3H), 4.58 (s, 2H), 7.06 (d, J = 8.3 Hz, 1H), 7.37 (d, J = 8.3 Hz, 1H), 7.57 (s, 1H), 8.27 (brs, 1H), 9.06 (br s, 2H).
LCMS (ESI) m/z 284 [M+H]⁺, t_{R} = 0.755 min, mode H.

### Reference Example 2-16: Synthesis of methyl 5-(trideuteriomethyl)-2-[2-({2-[(trideuteriomethyl)amino]ethyl}amino)-2-oxoethoxy]benzoate hydrochloride

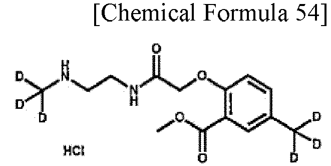

By using the same approaches as in Reference Example 2-8 (1) and (2), the title compound (0.56 g, colorless powder) was obtained from [2-(methoxycarbonyl)-4-(trideuteriomethyl)phenoxy]acetic acid (0.37 g) and tert-butyl (2-aminoethyl)(trideuteriomethyl)carbamate (0.32 g).
¹H NMR (400 MHz, DMSO-d₆) δ ppm 2.96 - 3.08 (m, 2H), 3.45 - 3.53 (m, 2H), 3.83 (s, 3H), 4.58 (s, 2H), 7.03 - 7.10 (m, 1H), 7.34 - 7.41 (m, 1H), 7.58 (s, 1H), 8.23 (br s, 1H), 8.66 (br s, 2H).
LCMS (ESI) m/z 287 [M+H]⁺, t_{R} = 0.292 min, mode N.

### Reference Example 2-17: Synthesis of methyl 5-ethyl-2-(2-{[2-(methylamino)ethyl]amino}-2-oxoethoxy)benzoate hydrochloride

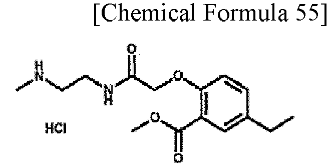

By using the same approaches as in Reference Example 2-1 (2) and (3), the title compound (0.57 g, colorless powder) was obtained by using methyl 5-ethyl-2-hydroxy-benzoate (0.71 g) instead of methyl 5-fluoro-2-hydroxy-benzoate. ¹H NMR (400 MHz, CDCl₃) δ ppm 1.23 (t, J = 7.6 Hz, 3H), 1.51 (br s, 2H), 2.47 (s, 3H), 2.63 (q, J = 7.6 Hz, 2H), 2.81 - 2.86 (m, 2H), 3.48 - 3.54 (m, 2H), 3.91 (s, 3H), 4.56 (s, 2H), 6.85 (d, J = 8.6 Hz, 1H), 7.34 (dd, J = 8.5, 2.4 Hz, 1H), 7.74 (d, J = 2.3 Hz, 1H), 8.29 (br s, 1H).
LCMS (ESI) m/z 295 [M+H]⁺, t_{R} = 0.470 min, mode N.

### Reference Example 2-18: Synthesis of methyl 5-methoxy-2-(2-{[2-(methylamino)ethyl]amino}-2-oxoethoxy)benzoate hydrochloride

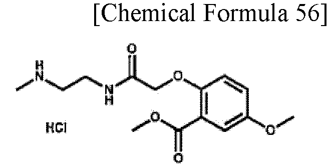

By using the same approaches as in Reference Example 2-1 (2) and (3), the title compound (0.41 g, colorless powder) was obtained by using methyl 2-hydroxy-5-methoxy-benzoate (0.70 g) instead of methyl 5-fluoro-2-hydroxy-benzoate. ¹H NMR (400 MHz, CD₃OD) δ ppm 2.75 (s, 3H), 3.20 - 3.27 (m, 2H), 3.61 - 3.76 (m, 2H), 3.79 (s, 3H), 3.91 (s, 3H), 4.62 (s, 2H), 7.07 - 7.14 (m, 1H), 7.14 - 7.20 (m, 1H), 7.42 (d, J =3.1 Hz, 1H), 8.86 (br s, 1H).
LCMS (ESI) m/z 297 [M+H]⁺, t_{R} = 0.674 min, mode H.

### Reference Example 2-19: Synthesis of methyl 5-iodo-2-(2-{[2-(methylamino)ethyl]amino}-2-oxoethoxy)benzoate hydrochloride

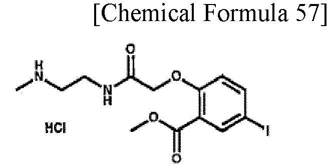

By using the same approaches as in Reference Example 2-1 (2) and (3), the title compound (5.4 g, colorless powder) was obtained by using methyl 2-hydroxy-5-iodo-benzoate (5.5 g) instead of methyl 5-fluoro-2-hydroxy-benzoate. ¹H NMR (400 MHz, CDCl₃) δ ppm 2.76 (s, 3H), 3.17 - 3.28 (m, 2H), 3.75 - 3.84 (m, 2H), 3.89 (s, 3H), 4.65 (s, 2H), 6.56 - 6.88 (m, 1H), 7.68 - 7.85 (m, 1H), 8.17 (s, 1H), 8.53 - 8.80 (m, 1H), 9.58 (brs, 2H).
LCMS (ESI/APCI dual) m/z 393 [M+H]⁺, t_{R} = 0.377 min, mode N.

### Reference Example 2-20: Synthesis of methyl 5-(1,1-difluoroethyl)-2-(2-{[2-(methylamino)ethyl]amino}-2-oxoethoxy)benzoate hydrochloride

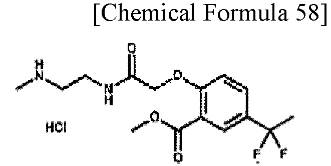

By using the same approaches as in Reference Example 2-8 (1) and (2), the title compound (0.19 g, yellow oily matter) was obtained from [4-(1,1-difluoroethyl)-2-(methoxycarbonyl)phenoxy]acetic acid (0.15 g) and tert-butyl (2-aminoethyl)methylcarbamate (0.11 g).
LCMS (ESI) m/z 331 [M+H]⁺, t_{R} = 0.435 min, mode N.

### Reference Example 2-21: Synthesis of methyl 5-(1-hydroxyethyl)-2-[2-[2-(methylamino)ethylamino]-2-oxo-ethoxy]benzoate hydrochloride

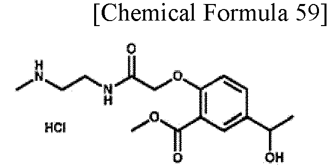

(1) By using the same approach as in Reference Example 2-1 (2), methyl 5-acetyl-2-[2-[2-[tert-butoxycarbonyl(methyl)amino]ethylamino]-2-oxo-ethoxy]benzoate (0.63 g, colorless powder) was obtained by using methyl 5-acetyl-2-hydroxy-benzoate (1.6 g) instead of methyl 5-fluoro-2-hydroxy-benzoate.

(2) To a solution of methyl 5-acetyl-2-[2-[2-[tert-butoxycarbonyl(methyl)amino]ethylamino]-2-oxo-ethoxy]benzoate (0.63 g) in MeOH (5.0 mL), NaBH₄ (0.23 g) was added, and the mixture was stirred at room temperature for 2 hours. The reaction solution was poured into water and extracted with EtOAc. The organic layer was washed with Brine, dried over MgSO₄, filtered, and then the solvent was concentrated. The residue was purified by OH type silica gel column chromatography (mobile phase: hexane/EtOAc = 50/50 to 0/100; v/v) to afford methyl 2-[2-[2-[tert-butoxycarbonyl(methyl)amino]ethylamino]-2-oxo-ethoxy]-5-(1-hydroxyethyl)be nzoate (0.63 g, yellow powder).

(3) By using the same approach as in Reference Example 2-1 (3), the title compound (0.53 g, colorless gum) was obtained.
¹H NMR (400 MHz, CDCl₃) δ ppm 1.83 (d, J = 6.8 Hz, 4H), 2.76 (s, 3H), 3.17 - 3.32 (m, 2H), 3.76 - 3.86 (m, 2H), 3.91 (s, 3H), 4.69 (s, 2H), 4.97 - 5.16 (m, 1H), 6.92 (d, J = 8.6 Hz, 1H), 7.56 (dd, J = 8.7, 2.3 Hz, 1H), 7.95 (d, J = 2.3 Hz, 1H), 8.75 (br s, 1H).

### Reference Example 2-22: Synthesis of methyl 5-fluoro-2-(2-{[3-(methylamino)propyl]amino}-2-oxoethoxy)benzoate hydrochloride

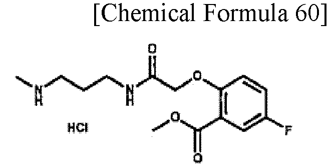

By using the same approaches as in Reference Example 2-1 (1) to (3), the title compound (1.1 g, colorless powder) was obtained by using tert-butyl
N-(3-aminopropyl)-N-methyl-carbamate (1.0 g) instead of tert-butyl
N-(2-aminoethyl)-N-methyl-carbamate.
LCMS (ESI) m/z 299 [M+H]⁺, t_{R} = 0.699 min, mode H.

### Reference Example 2-23: Synthesis of methyl 5-fluoro-2-{2-[(N-methylglycyl)amino]ethoxy}benzoate hydrochloride

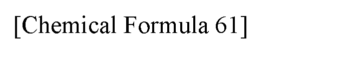

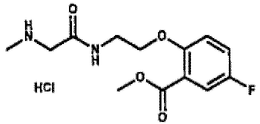

By using the same approaches as in Reference Example 2-8 (1) and (2), the title compound (1.0 g, colorless powder) was obtained from
N-(tert-butoxycarbonyl)-N-methylglycine (0.61 g) and methyl
2-(2-aminoethoxy)-5-fluorobenzoate (0.69 g).
LCMS (ESI) m/z 285 [M+H]⁺, t_{R} = 0.667 min, mode H.

### Reference Example 2-24: Synthesis of methyl 5-fluoro-2-({[2-(methylamino)ethyl]sulfamoyl}methoxy)benzoate hydrochloride

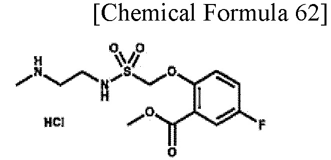

By using the same approaches as in Reference Example 2-1 (1) to (3), the title compound (0.25 g, colorless powder) was obtained by using chloromethanesulfonyl chloride (1.2 g) instead of 2-chloroacetyl chloride.
LCMS (ESI) m/z 321 [M+H]⁺, t_{R} = 0.236 min, mode N.

### Reference Example 2-25: Synthesis of methyl 5-fluoro-2-[4-(methylamino)butoxy]benzoate hydrochloride

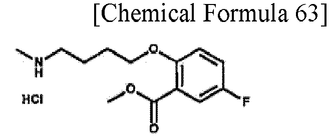

(1) To a solution of tert-butyl N-(4-hydroxybutyl)-N-methyl-carbamate (3.3 g) in CHCl₃ (16 mL), Et₃N (6.8 mL) and MsCl (1.9 mL) were added, and the mixture was stirred for 1 hour. The reaction solution was poured into water and extracted with CHCl₃. The organic layer was washed with Brine, dried over MgSO₄, filtered, and then the solvent was concentrated. The residue was purified by OH type silica gel column chromatography (mobile phase: hexane/EtOAc = 80/20 to 50/50; v/v) to afford tert-butyl 4-[tert-butoxycarbonyl(methyl)amino]butyl methanesulfonate (4.9 g, colorless oily matter).

(2) To a solution of tert-butyl 4-[tert-butoxycarbonyl(methyl)amino]butyl methanesulfonate (4.6 g) in DMF (60 mL), K₂CO₃ (6.8 g) and methyl 5-fluoro-2-hydroxy-benzoate (4.2 g) were added, and the mixture was stirred at 90°C for 2 hours. The reaction solution was poured into water and extracted with EtOAc. The organic layer was washed with Brine, dried over MgSO₄, filtered, and then the solvent was concentrated. The residue was purified by OH type silica gel column chromatography (mobile phase: hexane/EtOAc = 80/20 to 50/50; v/v) to afford methyl 2-[4-[tert-butoxycarbonyl(methyl)amino]butoxy]-5-fluoro-benzoate (4.6 g, colorless oily matter).

(3) To methyl 2-[4-[tert-butoxycarbonyl(methyl)amino]butoxy]-5-fluoro-benzoate (4.6 g), a 4 M hydrochloric acid 1,4-dioxane solution (25 mL) was added, and the mixture was stirred at room temperature for 2 hours. The reaction solution was concentrated to afford the title compound (3.6 g, colorless powder).
LCMS (ESI) m/z 256 [M+H]⁺, t_{R} = 0.213 min, mode N.

### Reference Example 2-26: Synthesis of methyl 5-chloro-2-[4-(methylamino)butoxy]benzoate hydrochloride

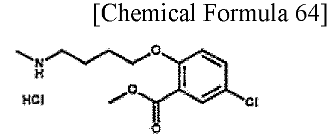

By using the same approaches as in Reference Example 2-25 (2) and (3), the title compound (0.46 g, colorless powder) was obtained from tert-butyl 4-[tert-butoxycarbonyl(methyl)amino]butyl methanesulfonate (0.46 g) obtained in Reference Example 2-25 (1) and methyl 5-chloro-2-hydroxybenzoate (0.61 g).
¹H NMR (400 MHz, CDCl₃) δ ppm 1.99 - 2.10 (m, 2H), 2.15 - 2.25 (m, 2H), 2.75 (s, 3H), 3.14 (t, J = 6.8 Hz, 2H), 3.89 (s, 3H), 4.13 (t, J = 5.2 Hz, 2H), 6.93 (d, J = 8.9 Hz, 1H), 7.42 - 7.47 (m, 1H), 7.83 (d, J = 1.7 Hz, 1H).
LCMS (ESI) m/z 272 [M+H]⁺, t_{R} = 0.486 min, mode N.

### Reference Example 2-27: Synthesis of methyl 5-methyl-2-[4-(methylamino)butoxy]benzoate hydrochloride

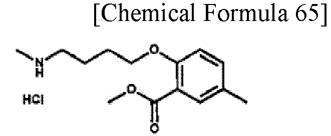

By using the same approaches as in Reference Example 2-25 (2) and (3), the title compound (4.7 g, colorless oily matter) was obtained from tert-butyl 4-[tert-butoxycarbonyl(methyl)amino]butyl methanesulfonate (4.6 g) obtained in Reference Example 2-25 (1) and methyl 2-hydroxy-5-methylbenzoate (4.1 g).
LCMS (ESI/APCI dual) m/z 252 [M+H]⁺, t_{R} = 0.359 min, mode N.

### Reference Example 2-28: Synthesis of methyl 5-ethyl-2-[4-(methylamino)butoxy]benzoate hydrochloride

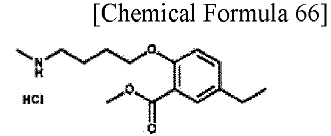

By using the same approaches as in Reference Example 2-25 (2) and (3), the title compound (0.38 g, colorless powder) was obtained from tert-butyl 4-[tert-butoxycarbonyl(methyl)amino]butyl methanesulfonate (0.46 g) obtained in Reference Example 2-25 (1) and methyl 5-ethyl-2-hydroxybenzoate (0.59 g).
¹H NMR (400 MHz, CDCl₃) δ ppm 1.22 (t, J = 7.6 Hz, 3H), 1.99 - 2.10 (m, 2H), 2.21 (quin, J = 6.6 Hz, 2H), 2.61 (q, J = 7.6 Hz, 2H), 2.74 (s, 3H), 3.13 (t, J = 6.6 Hz, 2H), 3.87 (s, 3H), 4.12 (t, J = 5.2 Hz, 2H), 6.90 (d, J = 8.6 Hz, 1H), 7.26 - 7.34 (m, 1H), 7.68 (s, 1H), 9.44 (br s, 1H).
LCMS (ESI) m/z 266 [M+H]⁺, t_{R} = 0526 min, mode N.

### Reference Example 2-29: Synthesis of methyl 5-methoxy-2-[4-(methylamino)butoxy]benzoate hydrochloride

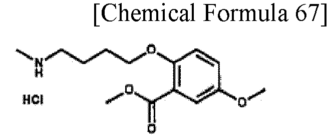

By using the same approaches as in Reference Example 2-25 (2) and (3), the title compound (0.36 g, colorless powder) was obtained from tert-butyl 4-[tert-butoxycarbonyl(methyl)amino]butyl methanesulfonate (0.46 g) obtained in Reference Example 2-25 (1) and methyl 2-hydroxy-5-methoxybenzoate (0.38 g).
¹H NMR (400 MHz, DMSO-d₆) δ ppm 1.71 - 1.82 (m, 4H), 2.53 (s, 3H), 2.94 (br s, 2H), 3.73 (s, 3H), 3.81 (s, 3H), 3.99 (t, J = 5.3 Hz, 2H), 7.07 - 7.12 (m, 2H), 7.17 (dd, J = 2.4, 1.0 Hz, 1H), 8.82 (br s, 2H).
LCMS (ESI) m/z 268 [M+H]⁺, t_{R} = 0.748 min, mode H.

### Reference Example 2-30: Synthesis of methyl 2-[4-(methylamino)butoxy]-5-(trifluoromethyl)benzoate hydrochloride

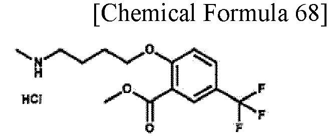

By using the same approaches as in Reference Example 2-25 (2) and (3), the title compound (0.25 g, colorless powder) was obtained from tert-butyl 4-[tert-butoxycarbonyl(methyl)amino]butyl methanesulfonate (0.46 g) obtained in Reference Example 2-25 (1) and methyl 2-hydroxy-5-(trifluoromethyl)benzoate (0.72 g).
¹H NMR (400 MHz, CD₃OD) δ ppm 1.91 - 2.02 (m, 4H), 2.74 (s, 3H), 3.17 (t, J = 7.3 Hz, 2H), 3.87 - 3.95 (m, 3H), 4.23 (t, J = 5.3 Hz, 2H), 7.31 (d, J = 8.8 Hz, 1H), 7.82 (dd, J = 8.8, 2.2 Hz, 1H), 8.05 (d, J = 2.2 Hz, 1H).
LCMS (ESI) m/z 306 [M+H]⁺, t_{R} = 0.575 min, mode N.

### Reference Example 2-31: Synthesis of methyl 2-chloro-6-[4-(methylamino)butoxy]benzoate hydrochloride

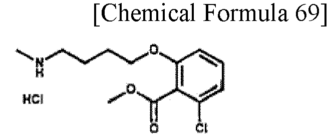

By using the same approaches as in Reference Example 2-25 (2) and (3), the title compound (0.15 g, colorless oily matter) was obtained from tert-butyl 4-[tert-butoxycarbonyl(methyl)amino]butyl methanesulfonate (0.10 g) obtained in Reference Example 2-25 (1) and methyl 2-chloro-6-hydroxybenzoate (86 mg).
¹H NMR (400 MHz, CDCl₃) δ ppm 1.85 - 1.96 (m, 2H), 1.97 - 2.12 (m, 2H), 2.71 (s, 3H), 3.02 (t, J = 7.5 Hz, 2H), 3.96 (s, 3H), 4.04 (t, J = 5.7 Hz, 2H), 6.77 - 6.82 (m, 1H), 6.97 - 7.02 (m, 1H), 7.23 - 7.30 (m, 1H), 9.54 (br s, 2H).
LCMS (ESI) m/z 272 [M+H]⁺, t_{R} = 0.834 min, mode H.

### Reference Example 2-32: Synthesis of methyl 2-fluoro-6-[4-(methylamino)butoxy]benzoate hydrochloride

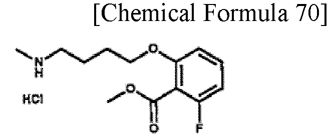

By using the same approaches as in Reference Example 2-25 (2) and (3), the title compound (0.36 g, colorless powder) was obtained from tert-butyl 4-[tert-butoxycarbonyl(methyl)amino]butyl methanesulfonate (0.50 g) obtained in Reference Example 2-25 (1) and methyl 2-fluoro-6-hydroxybenzoate (0.30 g).
¹H NMR (400 MHz, CDCl₃) δ ppm 1.87 - 2.02 (m, 2H), 2.02 - 2.18 (m, 2H), 2.73 (br s, 3H), 2.97 - 3.15 (m, 2H), 3.94 (s, 3H), 4.08 (t, J = 5.6 Hz, 2H), 6.65 - 6.79 (m, 2H), 7.28 - 7.39 (m, 1H), 9.48 (br s, 2H).
LCMS (ESI) m/z 256 [M+H]⁺, t_{R} = 0.744 min, mode H.

### Reference Example 2-33: Synthesis of methyl 3,5-dichloro-2-[4-(methylamino)butoxy]benzoate hydrochloride

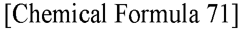

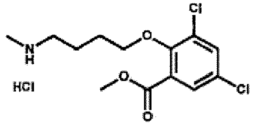

By using the same approaches as in Reference Example 2-25 (2) and (3), the title compound (1.2 g, colorless powder) was obtained from tert-butyl 4-[tert-butoxycarbonyl(methyl)amino]butyl methanesulfonate (1.0 g) obtained in Reference Example 2-25 (1) and methyl 3,5-dichloro-2-hydroxybenzoate (1.5 g).
¹H NMR (400 MHz, CDCl₃) δ ppm 1.88 - 2.06 (m, 2H), 2.10 - 2.20 (m, 2H), 3.07 - 3.19 (m, 2H), 3.70 (s, 3H), 3.92 (s, 3H), 4.05 (t, J = 5.7 Hz, 2H), 7.53 (d, J = 2.7 Hz, 1H), 7.69 (d, J = 2.7 Hz, 1H), 9.57 (brs, 2H).
LCMS (ESI) m/z 306 [M+H]⁺, t_{R} = 0.567 min, mode N.

### Reference Example 2-34: Synthesis of methyl 3,5-difluoro-2-[4-(methylamino)butoxy]benzoate hydrochloride

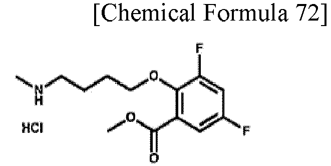

By using the same approaches as in Reference Example 2-25 (2) and (3), the title compound (0.70 g, colorless powder) was obtained from tert-butyl 4-[tert-butoxycarbonyl(methyl)amino]butyl methanesulfonate (1.0 g) obtained in Reference Example 2-25 (1) and methyl 3,5-difluoro-2-hydroxybenzoate (0.57 g).
LCMS (ESI) m/z 274 [M+H]⁺, t_{R} = 0.367 min, mode N.

### Reference Example 2-35: Synthesis of methyl 5-(hydroxymethyl)-2-[4-(methylamino)butoxy]benzoate hydrochloride

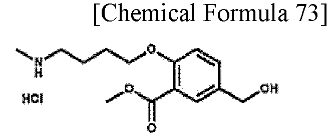

(1) By using the same approach as in Reference Example 2-25 (2), methyl 2-[4-[tert-butoxycarbonyl(methyl)amino]butoxy]-5-formyl-benzoate (0.75 g, colorless oily matter) was obtained from tert-butyl 4-[tert-butoxycarbonyl(methyl)amino]butyl methanesulfonate (1.5 g) obtained in Reference Example 2-25 (1) and methyl 5-formyl-2-hydroxybenzoate (0.90 g).

(2) To a solution of methyl 2-[4-[tert-butoxycarbonyl(methyl)amino]butoxy]-5-formyl-benzoate (0.75 g) in MeOH (10 mL), NaBH₄ (0.24 g) was added, and the mixture was stirred at room temperature for 2 hours. The reaction solution was poured into a Sat. NH₄Cl aq. and extracted with EtOAc.

The organic layer was washed with Brine, dried over MgSO₄, filtered, and then the solvent was concentrated. The residue was purified by OH type silica gel column chromatography (mobile phase: hexane/EtOAc = 50/50 to 0/100; v/v) to afford methyl 2-[4-[tert-butoxycarbonyl(methyl)amino]butoxy]-5-(hydroxymethyl)benzoate (0.70 g, colorless oily matter).

(3) By the same approach as in Reference Example 2-25 (3), the title compound (0.60 g, colorless powder) was obtained from methyl 2-[4-[tert-butoxycarbonyl(methyl)amino]butoxy]-5-(hydroxymethyl)benzoate (0.70 g).
LCMS (ESI) m/z 268 [M+H]⁺, t_{R} = 0.537 min, mode N.

### Reference Example 2-36: Synthesis of methyl 6-methyl-3-[4-(methylamino)butoxy]pyridine-2-carboxylate hydrochloride

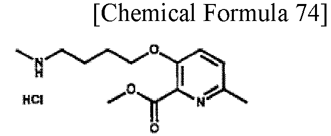

By using the same approaches as in Reference Example 2-25 (2) and (3), the title compound (0.37 g, colorless powder) was obtained from tert-butyl 4-[tert-butoxycarbonyl(methyl)amino]butyl methanesulfonate (0.40 g) obtained in Reference Example 2-25 (1) and methyl 3-hydroxy-6-methylpyridine-2-carboxylate (0.47 g). ¹H NMR (400 MHz, CD₃OD) δ ppm 1.90 - 2.04 (m, 4H), 2.73 (s, 3H), 2.77 (s, 3H), 3.07 - 3.20 (m, 2H), 4.06 - 4.12 (m, 3H), 4.37 (t, J = 5.5 Hz, 2H), 8.02 (d, J =9.2 Hz, 1H), 8.38 (d, J = 9.2 Hz, 1H).
LCMS (ESI) m/z 253 [M+H]⁺, t_{R} = 0.525 min, mode H.

### Reference Example 2-37: Synthesis of methyl 4-fluoro-5-methyl-2-[4-(methylamino)butoxy]benzoate hydrochloride

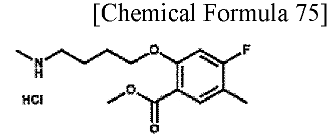

(1) By using the same approach as in Reference Example 2-25 (2), methyl 5-bromo-2-[4-[tert-butoxycarbonyl(methyl)amino]butoxy]-4-fluoro-benzoate (0.71 g, colorless oily matter) was obtained from tert-butyl 4-[tert-butoxycarbonyl(methyl)amino]butyl methanesulfonate (0.85 g) obtained in Reference Example 2-25 (1) and methyl 5-bromo-4-fluoro-2-hydroxy-benzoate (0.68 g).

(2) To a solution of methyl

5-bromo-2-[4-[tert-butoxycarbonyl(methyl)amino]butoxy]-4-fluoro-benzoate (0.66 g) in DMF (5.0 mL), trimethylboroxine (0.57 g), Cs₂CO₃ (2.4 g), and PdCl₂(dppf)-CH₂Cl₂ (0.20 g) were added, and the mixture was stirred at 110°C for 3 hours. The reaction solution was poured into water and extracted with EtOAc. The organic layer was washed with Brine, dried over MgSO₄, filtered, and then the solvent was concentrated. The residue was purified by OH type silica gel column chromatography (mobile phase: hexane/EtOAc = 80/20 to 0/100; v/v) to afford methyl 2-[4-[tert-butoxycarbonyl(methyl)amino]butoxy]-4-fluoro-5-methyl-benzoate (0.48 g, oily matter).

(3) By using the same approach as in Reference Example 2-25 (3), the title compound (0.37 g, colorless powder) was obtained from methyl 2-[4-[tert-butoxycarbonyl(methyl)amino]butoxy]-4-fluoro-5-methyl-benzoate (0.48 g). LCMS (ESI) m/z 270 [M+H]⁺, t_{R} = 0.485 min, mode N.

### Reference Example 2-38: Synthesis of methyl 4-chloro-2-[4-(methylamino)butoxy]benzoate hydrochloride

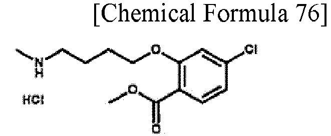

By using the same approaches as in Reference Example 2-25 (2) and (3), the title compound (0.33 g, colorless powder) was obtained from tert-butyl 4-[tert-butoxycarbonyl(methyl)amino]butyl methanesulfonate (0.35 g) obtained in Reference Example 2-25 (1) and methyl 4-chloro-2-hydroxybenzoate (0.44 g).
¹H NMR (400 MHz, CDCl₃) δ ppm 1.87 - 2.02 (m, 2H), 2.02 - 2.18 (m, 2H), 2.73 (br s, 3H), 2.97 - 3.15 (m, 2H), 3.94 (s, 3H), 4.00 - 4.15 (m, 2H), 6.65 - 6.79 (m, 2H), 7.29 - 7.39 (m, 1H), 9.46 (br s, 2H).
LCMS (ESI) m/z 272 [M+H]⁺, t_{R} = 0.858 min, mode H.

### Reference Example 2-39: Synthesis of methyl 4-fluoro-2-[4-(methylamino)butoxy]benzoate hydrochloride

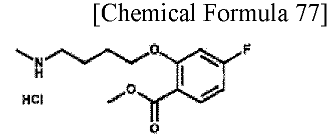

By using the same approaches as in Reference Example 2-25 (2) and (3), the title compound (0.15 g, colorless powder) was obtained from tert-butyl

4-[tert-butoxycarbonyl(methyl)amino]butyl methanesulfonate (0.53 g) obtained in Reference Example 2-25 (1) and methyl 4-fluoro-2-hydroxybenzoate (0.64 g). ¹H NMR (400 MHz, DMSO-d₆) δ ppm 1.71 - 1.85 (m, 4H), 2.95 (t, J = 7.1 Hz, 2H), 3.32 (s, 3H), 3.79 (s, 3H), 4.08 (t, J = 5.3 Hz, 2H), 6.82 - 6.90 (m, 1H), 7.07 (dd, J = 11.6, 2.3 Hz, 1H), 7.77 (dd, J = 8.7, 7.1 Hz, 1H), 8.62 (br s, 2H).
LCMS (ESI) m/z 256 [M+H]⁺, t_{R} = 0.381 min, mode N.

### Reference Example 2-40: Synthesis of methyl 5-[(methanesulfonyl)amino]-2-[4-(methylamino)butoxy]benzoate hydrochloride

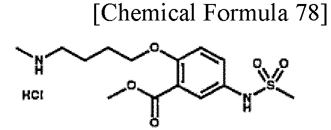

By using the same approaches as in Reference Example 2-25 (2) and (3), the title compound (0.42 g, brown oily matter) was obtained from tert-butyl 4-[tert-butoxycarbonyl(methyl)amino]butyl methanesulfonate (0.41 g) obtained in Reference Example 2-25 (1) and methyl 5-[(tert-butoxycarbonyl)(methanesulfonyl)amino]-2-hydroxybenzoate (1.0 g).
¹H NMR (400 MHz, DMSO-d₆) δ ppm 1.71 - 1.83 (m, 4H), 2.87 - 2.99 (m, 5H), 3.40 (br s, 3H), 3.81 (s, 2H), 3.97 - 4.11 (m, 2H), 7.16 (d, J = 9.0 Hz, 1H), 7.39 (dd, J = 9.0, 2.8 Hz, 1H), 7.53 (d, J = 2.8 Hz, 1H), 8.80 (br s, 2H), 9.61 (s, 1H).
LCMS (ESI) m/z 331 [M+H]⁺, t_{R} = 0.581 min, mode H.

### Reference Example 2-41: Synthesis of methyl 3-[4-(methylamino)butoxy]naphthalene-2-carboxylate hydrochloride

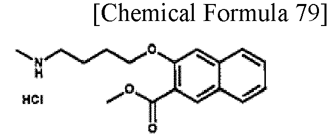

By using the same approaches as in Reference Example 2-25 (2) and (3), the title compound (0.46 g, yellow powder) was obtained from tert-butyl 4-[tert-butoxycarbonyl(methyl)amino]butyl methanesulfonate (0.47 g) obtained in Reference Example 2-25 (1) and methyl 3-hydroxynaphthalene-2-carboxylate (0.67 g).
¹H NMR (400 MHz, CD₃OD) δ ppm 1.95 - 2.07 (m, 4H), 2.76 (s, 3H), 3.20 (t, J = 7.2 Hz, 2H), 3.94 (s, 3H), 4.24 (t, J = 5.3 Hz, 2H), 7.37 - 7.43 (m, 2H), 7.49 - 7.59 (m, 1H), 7.80 (d, J = 8.6 Hz, 1H), 7.86 (d, J = 8.4 Hz, 1H), 8.33 (s, 1H).
LCMS (ESI) m/z 288 [M+H]⁺, t_{R} = 0.573 min, mode N.

Reference Example 2-42: Synthesis of methyl 2-[4-(methylamino)butoxy]benzoate

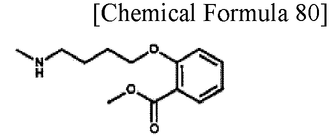

To a solution of methyl 5-chloro-2-[4-(methylamino)butoxy]benzoate (0.35 g) obtained in Reference Example 2-26 in MeOH (5.0 mL), 10% palladium-carbon (0.30 g) was added, and the mixture was stirred at room temperature overnight in the presence of hydrogen gas. The reaction solution was filtered through Celite (R), and then concentrated to afford the title compound (0.30 g, colorless oily matter).
LCMS (ESI) m/z 238 [M+H]⁺, t_{R} = 0.694 min, mode H.

### Reference Example 2-43: Synthesis of methyl 2-(4-aminobutoxy)-5-chlorobenzoate hydrochloride

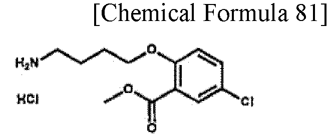

By using the same approaches as in Reference Example 2-25 (1) to (3), the title compound (1.9 g, colorless powder) was obtained from tert-butyl (4-hydroxybutyl)carbamate (1.5 g) and methyl 5-chloro-2-hydroxybenzoate (2.0 g).
¹H NMR (400 MHz, CDCl₃) δ ppm 1.98 - 2.14 (m, 4H), 3.20 (t, J = 6.2 Hz, 2H), 3.90 (s, 3H), 4.11 (t, J = 5.1 Hz, 2H), 6.90 (d, J = 8.9 Hz, 1H), 7.42 (dd, J = 8.9, 2.7 Hz, 1H), 7.82 (d, J = 2.7 Hz, 1H).
LCMS (ESI) m/z 258 [M+H]⁺, t_{R} = 0.851 min, mode H.

### Reference Example 2-44: Synthesis of methyl 2-(4-aminobutoxy)-5-methylbenzoate hydrochloride

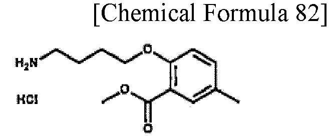

By using the same approaches as in Reference Example 2-25 (1) to (3), the title compound (0.70 g, colorless powder) was obtained from tert-butyl (4-hydroxybutyl)carbamate and methyl 2-hydroxy-5-methylbenzoate.
¹H NMR (400 MHz, CDCl₃) δ ppm 2.00 - 2.14 (m, 4H), 2.29 (s, 3H), 3.20 (t, J = 6.3 Hz, 2H), 3.88 (s, 3H), 4.09 (t, J = 5.1 Hz, 2H), 6.83 - 6.87 (m, 1H), 7.24 - 7.29 (m, 1H), 7.61 - 7.68 (m, 1H).
LCMS (ESI) m/z 238 [M+H]⁺, t_{R} = 0.386 min, mode N.

### Reference Example 2-45: Synthesis of methyl 5-methyl-2-[3-(methylamino)propoxy]benzoate hydrochloride

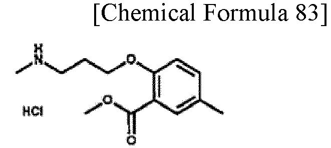

By using the same approaches as in Reference Example 2-25 (1) to (3), the title compound (0.70 g, colorless oily matter) was obtained from tert-butyl (3-hydroxypropyl)(methyl)carbamate (2.1 g) and methyl 2-hydroxy-5-methylbenzoate (1.2 g).
¹H NMR (400 MHz, CDCl₃) δ ppm 2.33 (s, 3H), 2.41 - 2.50 (m, 2H), 2.76 - 2.85 (m, 3H), 3.25 (br s, 2H), 3.70 (s, 3H), 3.87 (s, 3H), 4.16 - 4.23 (m, 2H), 6.86 (d, J = 8.5 Hz, 1H), 7.34 (dd, J = 8.5, 1.9 Hz, 1H), 7.77 (d, J = 1.9 Hz, 1H), 9.84 (br s, 2H).
LCMS (ESI) m/z 238 [M+H]⁺, t_{R} = 0.384 min, mode N.

### Reference Example 2-46: Synthesis of methyl 5-methyl-2-{[5-(methylamino)pentyl]oxy}benzoate hydrochloride

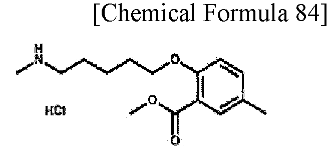

By using the same approaches as in Reference Example 2-25 (1) to (3), the title compound (0.24 g, colorless powder) was obtained from tert-butyl (5-hydroxypentyl)methylcarbamate (0.77 g) and methyl 2-hydroxy-5-methylbenzoate (0.29 mL).
¹H NMR (400 MHz, DMSO-d₆) δ ppm 1.43 - 1.53 (m, 2H), 1.58 - 1.78 (m, 4H), 2.25 (s, 3H), 2.53 (s, 3H), 2.82 - 2.92 (m, 2H), 3.77 (s, 3H), 3.95 - 4.04 (m, 2H), 6.99 - 7.07 (m, 1H), 7.28 - 7.35 (m, 1H), 7.44 (s, 1H), 8.48 (br s, 2H).
LCMS (ESI) m/z 266 [M+H]⁺, t_{R} = 0.494 min, mode N.

### Reference Example 2-47: Synthesis of butyl 2-{[5-(methylamino)pentyl]oxy}benzoate hydrochloride

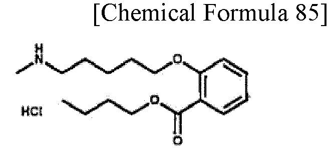

By using the same approaches as in Reference Example 2-25 (1) to (3), the title compound (0.27 g, colorless oily matter) was obtained from tert-butyl (5-hydroxypentyl)methylcarbamate (0.77 g) and butyl 2-hydroxybenzoate (0.37 µL). ¹H NMR (400 MHz, CDCl₃) δ ppm 0.97 (t, J = 7.3 Hz, 3H), 1.41 - 1.55 (m, 2H), 1.63 - 1.76 (m, 2H), 1.82 - 1.93 (m, 2H), 1.93 - 2.06 (m, 2H), 2.66 - 2.73 (m, 2H), 2.96 - 3.08 (m, 2H), 3.70 (s, 3H), 4.06 (t, J = 5.8 Hz, 2H), 4.27 (t, J = 6.6 Hz, 2H), 6.92 - 7.01 (m, 2H), 7.38 - 7.49 (m, 1H), 7.77 (d, J = 7.8 Hz, 1H), 9.46 (br s, 2H).
LCMS (ESI) m/z 294 [M+H]⁺, t_{R} = 0.631 min, mode N.

### Reference Example 2-48: Synthesis of methyl 5-methyl-2-{[6-(methylamino)hexyl]oxy}benzoate hydrochloride

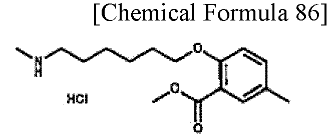

By using the same approaches as in Reference Example 2-25 (1) to (3), the title compound (0.79 g, colorless powder) was obtained from tert-butyl (6-hydroxyhexyl)methylcarbamate (1.8 g) and methyl 2-hydroxy-5-methylbenzoate (1.0 g). ¹H NMR (400 MHz, DMSO-d₆) δ ppm 1.31 - 1.50 (m, 4H), 1.55 - 1.73 (m, 4H), 2.25 (s, 3H), 2.84 (t, J = 7.6 Hz, 2H), 3.31 (s, 3H), 3.77 (s, 3H), 3.99 (t, J = 6.2 Hz, 2H), 7.02 (d, J = 8.5 Hz, 1H), 7.31 (dd, J = 8.5, 1.6 Hz, 1H), 7.43 (d, J = 1.6 Hz, 1H), 8.69 (br s, 2H). LCMS (ESI) m/z 280 [M+H]⁺, t_{R} = 0.549 min, mode N.

### Reference Example 2-49: Synthesis of ethyl 3-[4-(methylamino)butoxy]benzoate hydrochloride

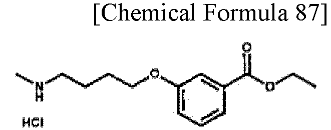

By using the same approaches as in Reference Example 2-25 (2) and (3), the title compound (0.22 g, colorless powder) was obtained from tert-butyl 4-[tert-butoxycarbonyl(methyl)amino]butyl methanesulfonate (0.29 g) obtained in Reference Example 2-25 (1) and ethyl 3-hydroxybenzoate (0.34 g). ¹H NMR (400 MHz, CDCl₃) δ ppm 1.39 (t, J = 7.1 Hz, 3H), 1.88 - 1.98 (m, 2H), 2.05 - 2.14 (m, 2H), 2.71 (s, 3H), 3.02 - 3.08 (m, 2H), 4.03 (t, J = 5.9 Hz, 2H), 4.37 (q, J = 7.1 Hz, 2H), 7.04 - 7.08 (m, 1H), 7.30 - 7.35 (m, 1H), 7.50 - 7.52 (m, 1H), 7.62 - 7.65 (m, 1H).
LCMS (ESI) m/z 252 [M+H]⁺, t_{R} = 0.468 min, mode N.

### Reference Example 2-50: Synthesis of methyl 2-[4-(ethylamino)butoxy]-5-fluorobenzoate

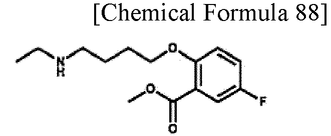

(1) By using the same approaches as in Reference Example 2-25 (2) and (3), methyl 2-(4-aminobutoxy)-5-fluoro-benzoate hydrochloride (1.6 g, colorless powder) was obtained from tert-butyl 4-[tert-butoxycarbonyl(methyl)amino]butyl methanesulfonate (1.9 g) and methyl 5-fluoro-2-hydroxy-benzoate (2.4 g).

(2) To a solution of methyl 2-(4-aminobutoxy)-5-fluoro-benzoate hydrochloride (0.31 g) in DMF (11 mL), K₂CO₃ (0.47 g) and EtI (0.14 mL) were added, and the mixture was stirred at 90°C for 1.5 hours. After allowing it to be cooled to room temperature, the reaction liquid was diluted with EtOAc and washed with water. The organic layer was dried over MgSO₄, filtered, and the filtrate was concentrated to afford the title compound (0.25 g, yellow oily matter) as a crude product.
LCMS (ESI) m/z 270 [M+H]⁺, t_{R} = 0.812 min, mode H.

### Reference Example 2-51: Synthesis of methyl 5-chloro-2-[4-(ethylamino)butoxy]benzoate hydrochloride

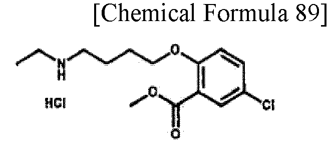

By using the same approaches as in Reference Example 2-25 (1) to (3), the title compound (1.3 g, colorless powder) was obtained from tert-butyl ethyl(4-hydroxybutyl)carbamate (1.0 g) and methyl 5-chloro-2-hydroxybenzoate (1.1 g). ¹H NMR (400 MHz, DMSO-d₆) δ ppm 1.15 - 1.23 (m, 3H), 1.71 - 1.83 (m, 4H), 2.88 - 2.99 (m, 4H), 3.81 (s, 3H), 4.04 - 4.11 (m, 2H), 7.20 (d, J = 9.0 Hz, 1H), 7.59 (dd, J = 9.0, 2.8 Hz, 1H), 7.66 (d, J = 2.8 Hz, 1H), 8.48 (br s, 2H).
LCMS (ESI) m/z 286 [M+H]⁺, t_{R} = 0.512 min, mode N.

### Reference Example 2-52: Synthesis of methyl 5-chloro-2-[4-(cyclopropylamino)butoxy]benzoate hydrochloride

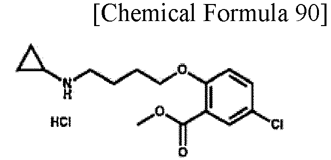

By using the same approaches as in Reference Example 2-25 (1) to (3), the title compound (67 mg, colorless powder) was obtained from tert-butyl cyclopropyl(4-hydroxybutyl)carbamate (51 mg) and methyl 5-chloro-2-hydroxybenzoate (46 mg).
LCMS (ESI) m/z 298 [M+H]⁺, t_{R} = 0.940 min, mode H.

### Reference Example 2-53: Synthesis of methyl 5-chloro-2-{4-[(2-methoxyethyl)amino]butoxy}benzoate

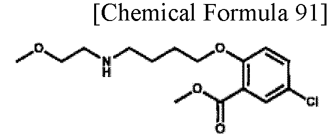

By using the same approach as in Reference Example 2-50 (2), the title compound was obtained as a crude product from methyl 2-(4-aminobutoxy)-5-chlorobenzoate hydrochloride (0.10 g) obtained in Reference Example 2-43 and 1-bromo-2-methoxyethane (32 µL).
LCMS (ESI) m/z 316 [M+H]⁺, t_{R} = 0.926 min, mode H.

### Reference Example 2-54: Synthesis of methyl 2-{4-[(2-{[tert-butyl(dimethyl)silyl]oxy}ethyl)amino]butoxy}-5-chlorobenzoate

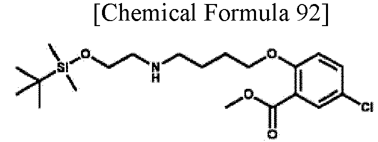

By using the same approach as in Reference Example 2-50 (2), the title compound was obtained as a crude product from methyl 2-(4-aminobutoxy)-5-chlorobenzoate hydrochloride (0.15 g) obtained in Reference Example 2-43 and (2-bromoethoxy)(tert-butyl)dimethylsilane (0.11 mL).
LCMS (ESI) m/z 416 [M+H]⁺, t_{R} = 0.870 min, mode N.

### Reference Example 2-55: Synthesis of methyl 2-[4-({2-[(tert-butoxycarbonyl)amino]ethyl}amino)butoxy]-5-fluorobenzoate

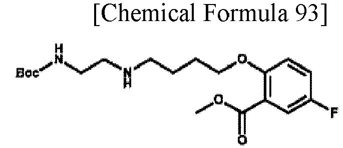

By using the same approach as in Reference Example 2-50 (2), the title compound (0.70 g, colorless powder) was obtained as a crude product from methyl 2-(4-aminobutoxy)-5-fluoro-benzoate hydrochloride (0.54 g) obtained in Reference Example 2-50 (1) and tert-butyl (2-bromoethyl)carbamate (0.43 g). LCMS (ESI) m/z 385 [M+H]⁺, t_{R} = 0.608 min, mode N.

### Reference Example 2-56: Synthesis of methyl 5-fluoro-2-{2-[2-(methylamino)ethoxy]ethoxy}benzoate hydrochloride

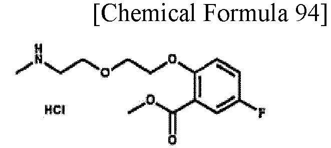

(1) By using the same approaches as in Reference Example 2-25 (1) to (2), methyl 2-[2-[2-(tert-butoxycarbonylamino)ethoxy]ethoxy]-5-fluoro-benzoate (1.9 g, colorless oily matter) was obtained from tert-butyl [2-(2-hydroxyethoxy)ethyl]methylcarbamate (2.0 g) and methyl 5-fluoro-2-hydroxy-benzoate (1.2 g).

(2) To a solution of methyl 2-[2-[2-(tert-butoxycarbonylamino)ethoxy]ethoxy]-5-fluoro-benzoate (0.79 g) in DMF (4.4 mL), silver oxide (2.6 g) and MeI (0.69 mL) were added, and the mixture was stirred at 90°C for 2 hours. The reaction solution was filtered through Celite (R), and then the organic layer was concentrated under reduced pressure. The obtained residue was purified by OH type silica gel column chromatography (mobile phase: hexane/EtOAc = 70/30 to 40/60; v/v) to afford methyl 2-[2-[2-[tert-butoxycarbonyl(methyl)amino]ethoxy]ethoxy]-5-fluoro-benzoate (0.82 g, colorless oily matter).

(3) By using the same approach as in Reference Example 2-25-(3), the title compound (0.67 g, colorless powder) was obtained from methyl 2-[2-[2-[tert-butoxycarbonyl(methyl)amino]ethoxy]ethoxy]-5-fluoro-benzoate (0.82 g). ¹H NMR (400 MHz, DMSO-d₆) δ ppm 2.55 (s, 3H), 3.05 - 3.13 (m, 2H), 3.73 - 3.86 (m, 7H), 4.14 - 4.22 (m, 2H), 7.17 - 7.25 (m, 1H), 7.36 - 7.50 (m, 2H), 8.67 (br s, 2H).

### Reference Example 2-57: Synthesis of methyl 5-chloro-2-{2-[2-(methylamino)ethoxy]ethoxy}benzoate hydrochloride

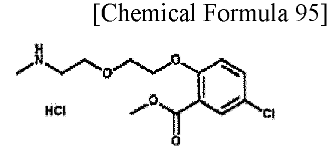

By using the same approaches as in Reference Example 2-25 (1) to (3), the title compound (1.2 g, colorless powder) was obtained from tert-butyl [2-(2-hydroxyethoxy)ethyl]methylcarbamate (1.0 g) and methyl 5-chloro-2-hydroxybenzoate (1.1 g).
¹H NMR (400 MHz, DMSO-d₆) δ ppm 2.55 (s, 3H), 3.06 - 3.13 (m, 2H), 3.74 - 3.84 (m, 7H), 4.17 - 4.25 (m, 2H), 7.22 (d, J = 9.0 Hz, 1H), 7.60 (dd, J = 9.0, 2.8 Hz, 1H), 7.66 (d, J = 2.8 Hz, 1H), 8.65 (brs, 2H).
LCMS (ESI) m/z 288 [M+H]⁺, t_{R} = 0.429 min, mode N.

### Reference Example 2-58: Synthesis of methyl 5-methyl-2-{2-[2-(methylamino)ethoxy]ethoxy}benzoate hydrochloride

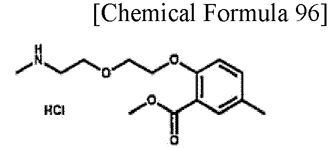

By using the same approaches as in Reference Example 2-25 (1) and (2), Reference Example 2-56 (2), and Reference Example 2-25 (3), the title compound (88 mg, colorless oily matter) was obtained from tert-butyl N-[2-(2-hydroxyethoxy)ethyl]carbamate (0.10 g) and methyl 2-hydroxy-5-methylbenzoate (0.14 mL).
¹H NMR (400 MHz, DMSO-d₆) δ ppm 2.26 (s, 3H), 2.56 (s, 3H), 3.06 - 3.14 (m, 2H), 3.75 - 3.84 (m, 7H), 4.12 - 4.18 (m, 2H), 7.06 (d, J = 8.5 Hz, 1H), 7.33 (dd, J = 8.5, 1.9 Hz, 1H), 7.47 (d, J = 1.9 Hz, 1H), 8.58 (br s, 2H).
LCMS (ESI) m/z 268 [M+H]⁺, t_{R} = 0.358 min, mode N.

### Reference Example 2-59: Synthesis of methyl 5-methyl-2-({2-[2-(methylamino)ethoxy]ethyl}amino)benzoate hydrochloride

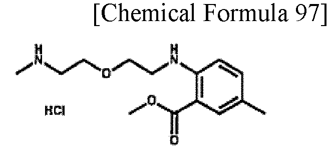

(1) Methyl 5-methyl-2-[(2,2,2-trifluoroacetyl)amino]benzoate (0.50 g) and tert-butyl N-[2-(2-hydroxyethoxy)ethyl]carbamate (0.79 g) were dissolved in THF (9.6 mL), PPh₃ (1.3 g) and DEAD (2.2 mL, 2.2 M toluene solution) were added thereto, and the mixture was stirred at room temperature for 1 hour. After adding water to the reaction liquid and stirring the mixture, it was extracted with CHCl₃. The aqueous layer was separated using Phase Separator, the organic layer was concentrated under reduced pressure and dried, and the resulting residue was purified by OH type silica gel chromatography (mobile phase: hexane:AcOEt = 95/5 to 80/20; v/v) to afford methyl 2-[2-[2-(tert-butoxycarbonylamino)ethoxy]ethyl-(2,2,2-trifluoroacetyl)amino]-5-methyl-benz oate (0.30 g, colorless oily matter).

(2) Methyl 2-[2-[2-(tert-butoxycarbonylamino)ethoxy]ethyl-(2,2,2-trifluoroacetyl)amino]-5-methyl-benz oate (0.30 g) was dissolved in DMF (6.6 mL), silver oxide (0.76 g) and MeI (0.20 mL) were added thereto, and the mixture was stirred at 100°C for 16 hours. The reaction liquid was filtered through Celite (R) and washed with EtOAc, the resulting filtrate was concentrated under reduced pressure and azeotroped with toluene, and the resulting residue was purified by OH type silica gel column chromatography (mobile phase: hexane/EtOAc = 95/5 to 90/10; v/v) to afford methyl 2-[2-[2-[tert-butoxycarbonyl(methyl)amino]ethoxy]ethylamino]-5-methyl-benzoate (69 mg, colorless amorphous).

(3) By using the same approach as in Reference Example 2-25 (3), the title compound (65 mg, colorless amorphous) was obtained from methyl 2-[2-[2-[tert-butoxycarbonyl(methyl)amino]ethoxy]ethylamino]-5-methyl-benzoate (65 mg). ¹H NMR (400 MHz, DMSO-d₆) δ ppm 2.18 (s, 3H), 2.58 (t, J = 5.5 Hz, 3H), 3.11 (quin, J = 5.5 Hz, 2H), 3.37 (t, J =5.5 Hz, 2H), 3.65 - 3.73 (m, 4H), 3.78 (s, 3H), 6.73 (d, J = 8.6 Hz, 1H), 7.24 (dd, J = 8.6, 2.0 Hz, 1H), 7.62 (d, J = 2.0 Hz, 1H), 8.56 (br s, 2H).
LCMS (ESI) m/z 267 [M+H]⁺, t_{R} = 0.512 min, mode N.

### Reference Example 2-60: Synthesis of methyl 5-methyl-2-[4-(methylamino)butyl-(2,2,2-trifluoroacetyl)amino]benzoate hydrochloride

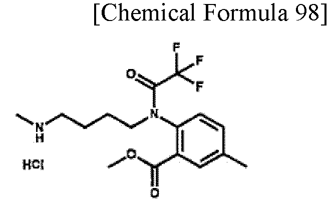

(1) By using the same approach as in Reference Example 2-59 (1), methyl 2-[4-[tert-butoxycarbonyl(methyl)amino]butyl-(2,2,2-trifluoroacetyl)amino]-5-methyl-benzo ate (0.94 g, colorless oily matter) was obtained from methyl 5-methyl-2-[(2,2,2-trifluoroacetyl)amino]benzoate (1.1 g) and tert-butyl N-(4-hydroxybutyl)-N-methyl-carbamate (1.5 g).

(2) By using the same approach as in Reference Example 2-25 (3), the title compound (0.28 g, colorless oily matter) was obtained from methyl 2-[4-[tert-butoxycarbonyl(methyl)amino]butyl-(2,2,2-trifluoroacetyl)amino]-5-methyl-benzo ate (0.26 g).
¹H NMR (400 MHz, DMSO-d₆) δ ppm 1.47 - 1.66 (m, 4H), 2.42 (s, 3H), 2.49 - 2.53 (m, 3H), 2.85 (t, J = 7.3 Hz, 2H), 3.11 - 3.21 (m, 1H), 3.82 (s, 3H), 3.98 - 4.10 (m, 1H), 7.40 (d, J = 8.1 Hz, 1H), 7.57 (dd, J = 8.1, 1.6 Hz, 1H), 7.88 (d, J = 1.6 Hz, 1H), 8.62 (br s, 2H).
LCMS (ESI) m/z 347 [M+H]⁺, t_{R} = 0.555 min, mode N.

### Reference Example 2-61: Synthesis of methyl 5-methyl-2-{methyl[4-(methylamino)butyl]amino}benzoate hydrochloride

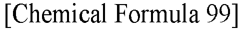

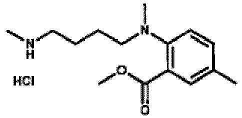

(1) The compound (0.30 g) obtained in Reference Example 2-60 (1) was dissolved in MeOH (6.7 mL), K₂CO₃ (0.14 g) was added thereto, and the mixture was stirred at 70°C for 6 hours and then stirred at room temperature for 16 hours. K₂CO₃ (0.14 g) was further added and the mixture was stirred at 80°C for 5 hours. The reaction solution was concentrated under reduced pressure. The resulting residue was dissolved in DMF (6.7 mL), MeI (0.11 mL) was added thereto, and the mixture was stirred at 80°C for 1 hour. After adding water to the reaction liquid and stirring the mixture, it was extracted with EtOAc/toluene (1/4; v/v). The organic layer was concentrated under reduced pressure, and the resulting residue was purified by OH type silica gel column chromatography (mobile phase: hexane/EtOAc = 90/10 to 0/100; v/v) to afford methyl 2-[4-[tert-butoxycarbonyl(methyl)amino]butylamino]-5-methyl-benzoate (0.17 g, light yellow oily matter).

(2) Methyl 2-[4-[tert-butoxycarbonyl(methyl)amino]butylamino]-5-methyl-benzoate (0.17 g) was dissolved in AcOH (4.9 mL), paraformaldehyde (0.15 g) was added thereto, and the mixture was stirred for 10 minutes. Then, NaBH₃CN (0.15 g) was added thereto and the mixture was stirred for 20 hours. After adding a NaHCO₃ aq. to the reaction liquid, a 1 M NaOH aq. was added to make it basic, and the mixture was extracted with CHCl₃. The aqueous layer was separated using Phase Separator, and the organic layer was concentrated under reduced pressure and dried to afford methyl 2-[4-[tert-butoxycarbonyl(methyl)amino]butyl-methyl-amino]-5-methyl-benzoate (0.17 g, colorless amorphous).

(3) By using the same approach as in Reference Example 2-25 (3), the title compound (0.18 g, colorless amorphous) was obtained from methyl 2-[4-[tert-butoxycarbonyl(methyl)amino]butyl-methyl-amino]-5-methyl-benzoate (0.17 g). ¹H NMR (400 MHz, DMSO-d₆) δ ppm 1.41 - 1.63 (m, 4H), 2.33 (br s, 3H), 2.47 (t, J =5.5 Hz, 3H), 2.76 - 2.86 (m, 2H), 2.91 - 3.83 (m, 5H), 3.90 (br s, 3H), 7.18 - 7.85 (m, 3H), 8.83 (br s, 2H).
LCMS (ESI) m/z 265 [M+H]⁺, t_{R} = 0.235 min, mode H.

### Reference Example 2-62: Synthesis of methyl 5-chloro-2-{[4-(methylamino)butyl]amino}pyridine-3-carboxylatehydrochloride

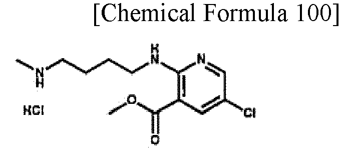

(1) Methyl 2,5-dichloropyridine-3-carboxylate (0.50 g) and tert-butyl N-(4-aminobutyl)-N-methyl-carbamate (0.59 g) were dissolved in MeOH (12 mL), Et₃N (0.68 mL) was added thereto, and the mixture was stirred at 80°C for 20 hours. After adding water to the reaction liquid and stirring the mixture, it was extracted with CHCl₃. The aqueous layer was separated using Phase Separator, the organic layer was concentrated under reduced pressure and dried, and the resulting residue was purified by OH type silica gel column chromatography (mobile phase: hexane/EtOAc = 95/5 to 80/20; v/v) to afford methyl 2-[4-[tert-butoxycarbonyl(methyl)amino]butylamino]-5-chloro-pyridine-3-carboxylate (0.17 g, colorless oily matter).

(2) By using the same approach as in Reference Example 2-25 (3), the title compound (0.20 g, colorless amorphous) was obtained from methyl 2-[4-[tert-butoxycarbonyl(methyl)amino]butylamino]-5-chloro-pyridine-3-carboxylate (0.17 g).
¹H NMR (400 MHz, DMSO-d₆) δ ppm 1.55 - 1.67 (m, 4H), 2.49 - 2.55 (m, 3H), 2.82 - 2.96 (m, 2H), 3.41 - 3.57 (m, 2H), 3.84 (s, 3H), 7.95 - 8.03 (m, 1H), 8.06 (d, J = 2.6 Hz, 1H), 8.33 (d, J = 2.6 Hz, 1H), 8.56 (br s, 2H).
LCMS (ESI) m/z 272 [M+H]⁺, t_{R} = 0.460 min, mode N.

### Reference Example 2-63: Synthesis of methyl 1-{2-[2-(methylamino)ethoxy] ethyl}-6-oxo-1,6-dihydropyridine-2-carboxylate hydrochloride

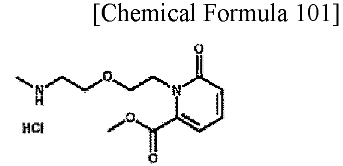

By using the same approaches as in Reference Example 2-25 (1) to (3), the title compound (62 mg, colorless amorphous) was obtained from tert-butyl N-[2-(2-hydroxyethoxy)ethyl]-N-methyl-carbamate (1.0 g) and methyl 6-oxo-1,6-dihydropyridine-2-carboxylate (0.95 g).
LCMS (ESI) m/z 255 [M+H]⁺, t_{R} = 0.370 min, mode H.

### Reference Example 2-64: Synthesis of methyl 2-(2-{[(benzyloxy)carbonyl][2-(methylamino)ethyl]amino}ethoxy)-5-fluorobenzoate hydrochloride

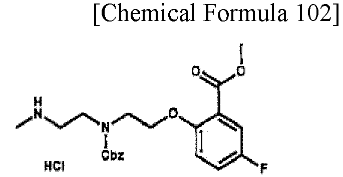

(1) By using the same approaches as in Reference Example 2-25 (1) and (2), methyl 2-[2-[benzyloxycarbonyl-[2-[tert-butoxycarbonyl(methyl)amino]ethyl]amino]ethoxy]-5-fluor o-benzoate (0.50 g, colorless oily matter) was obtained from tert-butyl N-[2-[benzyloxycarbonyl(2-hydroxyethyl)amino]ethyl]-N-methyl-carbamate (0.86 g) and methyl 5-fluoro-2-hydroxy-benzoate (0.83 g).

(2) By using the same approach as in Reference Example 2-25 (3), the title compound (0.25 g, colorless oily matter) was obtained from methyl 2-[2-[benzyloxycarbonyl-[2-[tert-butoxycarbonyl(methyl)amino]ethyl]amino]ethoxy]-5-fluor o-benzoate (0.25 g).
LCMS (ESI) m/z 405 [M+H]⁺, t_{R} = 0.631 min, mode N.

### Reference Example 2-65: Synthesis of methyl 5-fluoro-2-(2-{methyl[2-(methylamino)ethyl]amino}ethoxy)benzoate hydrochloride

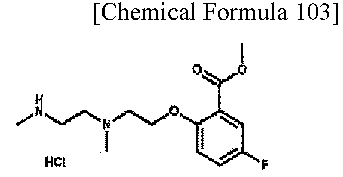

(1) The compound (0.25 g) obtained in Reference Example 2-64 (1) was dissolved in MeOH (2.5 mL), 10% palladium-carbon (0.25 g) was added thereto, and the mixture was stirred for 2 hours under a hydrogen gas atmosphere. The reaction liquid was filtered through Celite (R) and washed with MeOH, and the resulting filtrate was concentrated under reduced pressure and dried to afford methyl 2-[2-[2-[tert-butoxycarbonyl(methyl)amino]ethylamino]ethoxy]-5-fluoro-benzoate (0.18 g, colorless oily matter).

(2) Methyl 2-[2-[2-[tert-butoxycarbonyl(methyl)amino]ethylamino]ethoxy]-5-fluoro-benzoate (0.18 g) was dissolved in CHCl₃ (5.0 mL), a 37% aqueous formaldehyde solution (0.19 mL) and NaBH(OAc)₃ (0.21 g) were added thereto under ice cooling, and the mixture was stirred at room temperature for 2 hours. After adding a Sat. NaHCO₃ aq. to the reaction liquid and stirring the mixture, it was extracted with CHCl₃. The aqueous layer was separated using Phase Separator, the organic layer was concentrated under reduced pressure and dried, and the resulting residue was purified by NH type silica gel column chromatography (mobile phase: hexane/EtOAc = 90/10 to 0/100; v/v) to afford methyl 2-[2-[2-[tert-butoxycarbonyl(methyl)amino]ethyl-methyl-amino]ethoxy]-5-fluoro-benzoate (0.14 g, colorless oily matter).

(3) By using the same approach as in Reference Example 2-25 (3), the title compound (0.19 g, colorless amorphous) was obtained from methyl 2-[2-[2-[tert-butoxycarbonyl(methyl)amino]ethyl-methyl-amino]ethoxy]-5-fluoro-benzoate (0.14 g).
LCMS (ESI) m/z 285 [M+H]⁺, t_{R} = 0.178 min, mode N.

### Reference Example 2-66: Synthesis of methyl 2-(2-{[(benzyloxy)carbonyl][2-(methylamino)ethyl]amino}ethoxy)-5-methylbenzoate hydrochloride

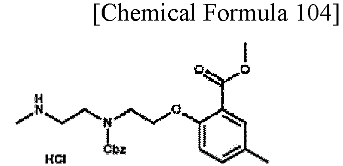

By using the same approaches as in Reference Example 2-25 (1) to (3), the title compound (0.62 g, colorless powder) was obtained from tert-butyl N-[2-[benzyloxycarbonyl(2-hydroxyethyl)amino]ethyl]-N-methyl-carbamate (1.1 g) and methyl 2-hydroxy-5-methyl-benzoate (0.96 g).
¹H NMR (400 MHz, CDCl₃) δ ppm 2.30 (s, 3H), 2.58 (br s, 1.2H), 2.71 (br s, 1.8H), 3.20 - 3.48 (m, 2H), 3.71 - 3.79 (m, 2H), 3.82 (s, 3H), 3.88 (t, J = 4.8 Hz, 2H), 4.34 - 4.55 (m, 2H), 5.01 (s, 1.2H), 5.18 (br s, 0.8H), 6.94 (d, J = 8.4 Hz, 0.6H), 7.01 (d, J = 7.9 Hz, 0.4H), 7.09 - 7.45 (m, 6H), 7.52 - 7.71 (m, 1H), 9.52 (br s, 2H).
LCMS (ESI) m/z 401 [M+H]⁺, t_{R} = 0.662 min, mode N.

### Reference Example 2-67: Synthesis of methyl 2-(2-{[(benzyloxy)carbonyl][2-(ethylamino)ethyl]amino}ethoxy)-5-fluorobenzoate hydrochloride

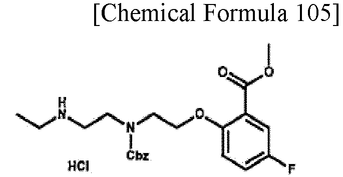

By using the same approaches as in Reference Example 2-25 (1) to (3), the title compound (0.28 g, colorless oily matter) was obtained from tert-butyl N-[2-[benzyloxycarbonyl(2-hydroxyethyl)amino]ethyl]-N-ethyl-carbamate (0.70 g) and methyl 5-fluoro-2-hydroxy-benzoate (0.31 g).
LCMS (ESI) m/z 419 [M+H]⁺, t_{R} = 0.494 min, mode L.

### Reference Example 2-68: Synthesis of methyl 2-(2-{[(benzyloxy)carbonyl][2-(methylamino)ethyl]amino}propoxy)-5-fluorobenzoate hydrochloride

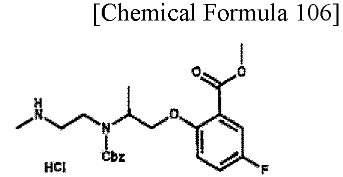

(1) By using the same approaches as in Reference Example 2-73 (1) to (4), methyl 2-[2-[2-[tert-butoxycarbonyl(methyl)amino]ethylamino]propoxy]-5-fluoro-benzoate (0.22 g, light yellow oily matter) was obtained from tert-butyl N-(2-hydroxy-1-methyl-ethyl)carbamate (0.60 g).

(2) To a solution of methyl 2-[2-[2-[tert-butoxycarbonyl(methyl)amino]ethylamino]propoxy]-5-fluoro-benzoate (0.22 g) in CHCl₃ (2.9 mL), Et₃N (0.24 mL) and benzyl chloroformate (0.25 mL) were added under ice cooling, and the mixture was stirred at 60°C overnight. To the reaction solution, a Sat. NaHCO₃ aq. and CHCl₃ were added, and the separating operation was carried out. The aqueous layer was separated using Phase Separator, and then the organic layer was concentrated under reduced pressure. Purification by silica gel column chromatography afforded methyl 2-[2-[benzyloxycarbonyl-[2-[tert-butoxycarbonyl(methyl)amino]ethyl]amino]propoxy]-5-flu oro-benzoate (0.13 g, colorless oily matter).

(3) By using the same approach as in Reference Example 2-25 (3), the title compound (0.12 g, colorless oily matter) was obtained from methyl 2-[2-[benzyloxycarbonyl-[2-[tert-butoxycarbonyl(methyl)amino]ethyl]amino]propoxy]-5-flu oro-benzoate (0.13 g).
LCMS (ESI) m/z 419 [M+H]⁺, t_{R} = 0.670 min, mode N.

### Reference Example 2-69: Synthesis of methyl 2-[3-(benzyloxy)-2-{[(benzyloxy)carbonyl][2-(methylamino)ethyl]amino}propoxy]-5-fluoro benzoate hydrochloride

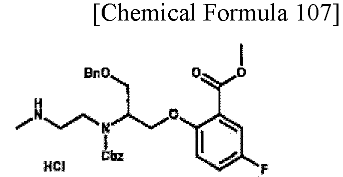

By using the same approaches as in Reference Example 2-73 (1) to (4), Reference Example 2-68 (2), and Reference Example 2-25 (3), the title compound (0.26 g, light yellow oily matter) was obtained from tert-butyl N-[1-(benzyloxymethyl)-2-hydroxy-ethyl]carbamate (0.60 g) and methyl 5-fluoro-2-hydroxy-benzoate (0.40 g).
LCMS (ESI) m/z 525 [M+H]⁺, t_{R} = 0.812 min, mode N.

### Reference Example 2-70: Synthesis of methyl 2-(2-{(methanesulfonyl)[2-(methylamino)ethyl]amino}ethoxy)-5-methylbenzoate hydrochloride

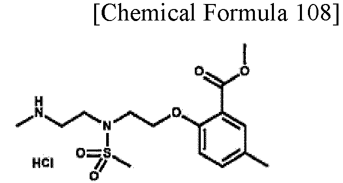

By using the same approaches as in Reference Example 2-25 (1) to (3), the title compound (0.75 g, colorless powder) was obtained from tert-butyl N-[2-(2-hydroxyethylamino)ethyl]-N-methyl-carbamate (0.50 g) and methyl 2-hydroxy-5-methyl-benzoate (0.75 g).
¹H NMR (400 MHz, CDCl₃) δ ppm 2.32 (s, 3H), 2.94 (s, 3H), 3.27 - 3.45 (m, 2H), 3.49 - 3.59 (m, 2H), 3.70 (s, 3H), 3.80 - 3.93 (m, 5H), 4.48 - 4.59 (m, 2H), 7.00 (d, J = 8.4 Hz, 1H), 7.32 (dd, J = 8.4, 1.8 Hz, 1H), 7.64 (d, J = 1.8 Hz, 1H), 9.55 (br s, 2H).
LCMS (ESI) m/z 345 [M+H]⁺, t_{R} = 0.398 min, mode N.

### Reference Example 2-71: Synthesis of methyl 2-[(2-{[(benzyloxy)carbonyl][2-(methylamino)ethyl]amino}ethyl)(methanesulfonyl)amino]-5 -methylbenzoate hydrochloride

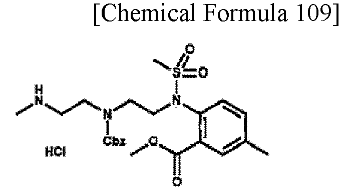

By using the same approaches as in Reference Example 2-59 (1) and Reference Example 2-25 (3), the title compound (0.44 g, colorless amorphous) was obtained from tert-butyl N-[2-[benzyloxycarbonyl(2-hydroxyethyl)amino]ethyl]-N-methyl-carbamate (1.3 g) and methyl 5-methyl-2-(methylsulfonamido)benzoate (0.80 g).
LCMS (ESI) m/z 478 [M+H]⁺, t_{R} = 0.597 min, mode N.

### Reference Example 2-72: Synthesis of methyl 5-chloro-2-(2-{methyl[2-(methylamino)ethyl]amino}ethoxy)benzoate hydrochloride

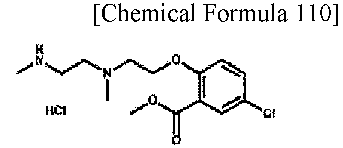

By using the same approaches as in Reference Example 2-25 (1) to (3), the title compound (0.54 g, colorless oily matter) was obtained from tert-butyl N-[2-[2-hydroxyethyl(methyl)amino]ethyl]-N-methyl-carbamate (0.41 g) and methyl 5-chloro-2-hydroxybenzoate (0.23 g).
LCMS (ESI) m/z 301 [M+H]⁺, t_{R} = 0.206 min, mode N.

### Reference Example 2-73: Synthesis of methyl 5-fluoro-2-(3,3,3-trifluoro-2-{[2-(methylamino)ethyl]amino}propoxy)benzoate hydrochloride

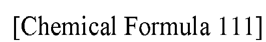

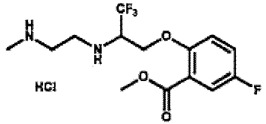

(1) A solution of tert-butyl N-[2,2,2-trifluoro-1-(hydroxymethyl)ethyl]carbamate (0.74 g) in CHCl₃ (6.5 mL) was added and cooled to 0°C. To this, Et₃N (1.4 mL) and MsCl (0.38 mL) were added, and the mixture was stirred at 0°C for 20 minutes. To the reaction solution, a Sat. NaHCO₃ aq. and CHCl₃ were added, and the separating operation was carried out. The aqueous layer was separated using Phase Separator, and then the organic layer was concentrated under reduced pressure to afford [2-(tert-butoxycarbonylamino)-3,3,3-trifluoro-propyl] methanesulfonate (yellow oily matter) as a crude product.

(2) To a solution of [2-(tert-butoxycarbonylamino)-3,3,3-trifluoro-propyl] methanesulfonate (0.99 g) and methyl 5-fluoro-2-hydroxy-benzoate (0.61 g) in DMF (16 mL), K₂CO₃ (1.8 g) was added, and the mixture was stirred at 90°C for 1 hour. To the reaction solution, EtOAc and Brine were added, and the separating operation was carried out. The aqueous layer was separated using Phase Separator, and then the organic layer was concentrated under reduced pressure. Purification by silica gel column chromatography afforded methyl 2-[2-(tert-butoxycarbonylamino)-3,3,3-trifluoro-propoxy]-5-fluoro-benzoate (0.26 g, yellow powder).

(3) By using the same approach as in Reference Example 2-25 (3), methyl 2-(2-amino-3,3,3-trifluoro-propoxy)-5-fluoro-benzoate hydrochloride (0.22 g, light yellow powder) was obtained from methyl 2-[2-(tert-butoxycarbonylamino)-3,3,3-trifluoro-propoxy]-5-fluoro-benzoate (0.26 g).

(4) To a solution of methyl 2-(2-amino-3,3,3-trifluoro-propoxy)-5-fluoro-benzoate hydrochloride (0.22 g) and tert-butyl N-methyl-N-(2-oxoethyl)carbamate (0.12 g) in CHCl₃ (3.5 mL), NaBH(OAc)₃ (0.44 g) was added, and the mixture was stirred at room temperature for 30 minutes. To the reaction solution, water and CHCl₃ were added, and the separating operation was carried out. The aqueous layer was separated using Phase Separator, and then the organic layer was concentrated under reduced pressure. Purification by OH type silica gel column chromatography (mobile phase: hexane/EtOAc = 90/10 to 50/50; v/v) afforded methyl 2-[2-[2-[tert-butoxycarbonyl(methyl)amino]ethylamino]-3,3,3-trifluoro-propoxy]-5-fluoro-be nzoate (0.26 g, light yellow oily matter).

(5) By using the same approach as in Reference Example 2-25 (3), the title compound (0.24 g, yellow oily matter) was obtained from methyl 2-[2-[2-[tert-butoxycarbonyl(methyl)amino]ethylamino]-3,3,3-trifluoro-propoxy]-5-fluoro-be nzoate (0.22 g).
LCMS (ESI) m/z 339 [M+H]⁺, t_{R} = 0.519 min, mode N.

### Reference Example 2-74: Synthesis of methyl 5-chloro-2-(3,3,3-trifluoro-2-{[2-(methylamino)ethyl]amino}propoxy)benzoate

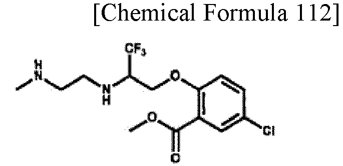

By using the same approaches as in Reference Example 2-73 (2) to (4) and Reference Example 2-77 (5), the title compound (0.37 g, colorless oily matter) was obtained from the compound (1.2 g) obtained in Reference Example 2-73 (1) and methyl 5-chloro-2-hydroxybenzoate (0.93 g).
LCMS (ESI) m/z 355 [M+H]⁺, t_{R} = 0.590 min, mode N.

### Reference Example 2-75: Synthesis of methyl 5-methyl-2-(3,3,3-trifluoro-2-{[2-(methylamino)ethyl]amino}propoxy)benzoate

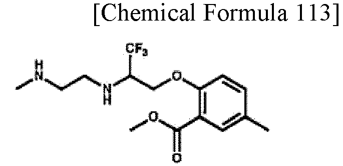

By using the same approaches as in Reference Example 2-73 (2) to (4) and Reference Example 2-77 (5), the title compound (0.75 g, colorless oily matter) was obtained from the compound (1.2 g) obtained in Reference Example 2-73 (1) and methyl 2-hydroxy-5-methylbenzoate (0.84 g).
LCMS (ESI) m/z 335 [M+H]⁺, t_{R} = 0.554 min, mode N.

### Reference Example 2-76: Synthesis of methyl 5-chloro-2-[(1-{[2-(methylamino)ethyl]amino}cyclopropyl)methoxy]benzoate hydrochloride

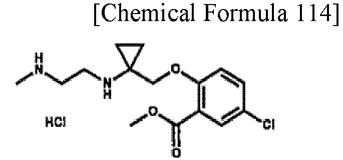

By using the same approaches as in Reference Example 2-73 (1) to (3), Reference Example 2-77 (4), and Reference Example 2-25 (3), the title compound (0.15 g, colorless gum) was obtained from methyl 5-chloro-2-hydroxybenzoate (0.64 g) and tert-butyl [1-(hydroxymethyl)cyclopropyl]carbamate (4.5 g).
LCMS (ESI) m/z 313 [M+H]⁺, t_{R} = 0.680 min, mode H.

### Reference Example 2-77: Synthesis of methyl 5-methyl-2-[(3-{[2-(methylamino)ethyl]amino}oxetan-3-yl)methoxy]benzoate

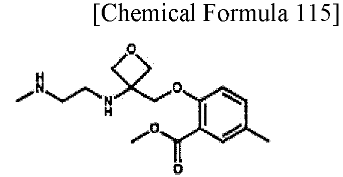

(1) To a solution of benzyl N-[3-(hydroxymethyl)oxetan-3-yl]carbamate (0.55 g) in CHCl₃ (8.0 mL), Et₃N (0.97 mL) and TsCl (0.53 g) were added, and the mixture was stirred for 1 hour. The reaction solution was poured into water and extracted with CHCl₃. The organic layer was washed with Brine, dried over MgSO₄, filtered, and the solvent was concentrated to afford [3-(benzyloxycarbonylamino)oxetan-3-yl]methyl 4-methylbenzenesulfonate (0.90 g, colorless oily matter).

(2) To a solution of [3-(benzyloxycarbonylamino)oxetan-3-yl]methyl 4-methylbenzenesulfonate (0.90 g) in DMF (10 mL), methyl 2-hydroxy-5-methyl-benzoate (0.76 g) and K₂CO₃ (0.95 g) were added, and the mixture was stirred at 80°C for 2 hours. The reaction solution was poured into water and extracted with EtOAc. The organic layer was washed with Brine, dried over MgSO₄, filtered, and then the solvent was concentrated. The residue was purified by OH type silica gel column chromatography (mobile phase: hexane/EtOAc = 50/50 to 0/100; v/v) to afford methyl
2-[[3-(benzyloxycarbonylamino)oxetan-3-yl]methoxy]-5-methyl-benzoate (0.64 g, yellow oily matter).

(3) To a solution of methyl 2-[[3-(benzyloxycarbonylamino)oxetan-3-yl]methoxy]-5-methyl-benzoate (0.64 g) in MeOH (10 mL), 5% palladium-carbon (0.20 g/g) was added, and the mixture was stirred at room temperature for 1 hour in the presence of hydrogen gas. The reaction solution was filtered through Celite (R), and then the solvent was concentrated. The residue was purified by NH type silica gel column chromatography (mobile phase: MeOH/EtOAc = 0/100 to 10/90; v/v) to afford methyl 2-[(3-aminooxetan-3-yl)methoxy]-5-methyl-benzoate (0.38 g, colorless oily matter).

(4) To a solution of methyl 2-[(3-aminooxetan-3-yl)methoxy]-5-methyl-benzoate (0.38 g) in MeOH (6.0 mL)/AcOH (0.10 mL), tert-butyl N-methyl-N-(2-oxoethyl)carbamate (0.31 g) and 2-methylpyridine borane complex (0.32 g) were added, and the mixture was stirred at room temperature for 1 hour. The reaction solution was poured into a Sat. NaHCO₃ aq. and extracted with CHCl₃. The organic layer was washed with Brine, dried over MgSO₄, filtered, and then the solvent was concentrated. The residue was purified by NH type silica gel column chromatography (mobile phase: hexane/EtOAc = 50/50 to 0/100; v/v) to afford methyl
2-[[3-[2-[tert-butoxycarbonyl(methyl)amino]ethylamino]oxetan-3-yl]methoxy]-5-methyl-ben zoate (0.79 g, colorless oily matter) as a crude product.

(5) To a solution of methyl 2-[[3-[2-[tert-butoxycarbonyl(methyl)amino]ethylamino]oxetan-3-yl]methoxy]-5-methyl-ben zoate (0.79 g) in CHCl₃ (2.0 mL), TFA (2.0 mL) was added, and the mixture was stirred at room temperature for 2 hours. The reaction solution was poured into a 20% K₂CO₃ aq. and extracted with CHCl₃. The organic layer was washed with Brine, dried over MgSO₄, filtered, and then the solvent was concentrated. The residue was purified by NH type silica gel column chromatography (mobile phase: MeOH/EtOAc = 0/100 to 10/90; v/v) to afford the title compound (0.36 g, colorless oily matter).
¹H NMR (400 MHz, CDCl₃) δ ppm 2.31 (s, 3H), 2.45 (s, 3H), 2.68 - 2.78 (m, 2H), 2.81 - 2.89 (m, 2H), 3.86 (s, 3H), 4.29 (s, 2H), 4.51 (d, J = 6.6 Hz, 2H), 4.73 (d, J = 6.5 Hz, 2H), 6.89 - 6.97 (m, 1H), 7.27 - 7.33 (m, 1H), 7.59 - 7.66 (m, 1H).
LCMS (ESI/APCI dual) m/z 309 [M+H]⁺, t_{R} = 0.226 min, mode H.

### Reference Example 2-78: Synthesis of methyl 2-[2-[2-[tert-butoxycarbonyl(methyl)amino]ethyl-cyclopropyl-amino]ethoxy]-5-methyl-benz oate hydrochloride

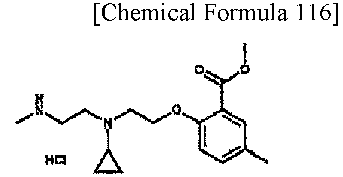

(1) By using the same approach as in Reference Example 2-5 (2), methyl 5-methyl-2-(2-oxoethoxy)benzoate (0.14 g, colorless oily matter) was obtained as a crude product from methyl 2-(2-hydroxyethoxy)-5-methylbenzoate (0.18 g).

(2) By using the same approach as in Reference Example 2-73 (4), methyl 2-[2-[2-[tert-butoxycarbonyl(methyl)amino]ethyl-cyclopropyl-amino]ethoxy]-5-methyl-benz oate (0.12 g, brown oily matter) was obtained from methyl 5-methyl-2-(2-oxoethoxy)benzoate (0.15 g) and tert-butyl N-[2-(cyclopropylamino)ethyl]-N-methyl-carbamate (0.12 g).

(3) By using the same approach as in Reference Example 2-25 (3), the title compound (0.10 g, brown oily matter) was obtained from methyl 2-[2-[2-[tert-butoxycarbonyl(methyl)amino]ethyl-cyclopropyl-amino]ethoxy]-5-methyl-benz oate (0.12 g).
LCMS (ESI) m/z 307 [M+H]⁺, t_{R} = 0.464 min, mode N.

### Reference Example 2-79: Synthesis of tert-butyl 5-methyl-2-(2-{[1,1,1-trifluoro-3-(methylamino)propan-2-yl]amino}ethoxy)benzoate

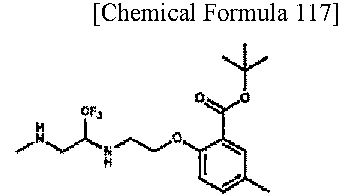

(1) By using the same approach as in Reference Example 2-5 (2), tert-butyl 5-methyl-2-(2-oxoethoxy)benzoate (0.39 g, colorless oily matter) was obtained as a crude product from tert-butyl 2-(2-hydroxyethoxy)-5-methyl-benzoate (0.56 g).

(2) By using the same approach as in Reference Example 2-73 (4), tert-butyl 2-[2-[[1-[[benzyl(methyl)amino]methyl]-2,2,2-trifluoro-ethyl]amino]ethoxy]-5-methyl-benzo ate (0.53 g, colorless oily matter) was obtained from tert-butyl 5-methyl-2-(2-oxoethoxy)benzoate (0.39 g) and N1-benzyl-3,3,3-trifluoro-N1-methyl-propane-1,2-diamine dihydrochloride (0.47 g).

(3) tert-Butyl 2-[2-[[1-[[benzyl(methyl)amino]methyl]-2,2,2-trifluoro-ethyl]amino]ethoxy]-5-methyl-benzo ate (0.53 g) was dissolved in MeOH (15 mL), 10% palladium-carbon (0.27 g) was added thereto, and the mixture was stirred for 2 hours under a hydrogen gas atmosphere. The reaction liquid was filtered through Celite (R) and washed with MeOH, and the resulting filtrate was concentrated under reduced pressure and dried to afford the title compound (0.38 g, colorless oily matter).
¹H NMR (400 MHz, CDCl₃) δ ppm 1.53 - 1.61 (m, 9H), 2.24 - 2.36 (m, 3H), 2.42 (s, 3H), 2.62 - 2.74 (m, 1H), 2.84 (dd, J = 12.4, 3.6 Hz, 1H), 3.00 - 3.09 (m, 1H), 3.15 - 3.34 (m, 2H), 3.49 (s, 1H), 3.64 (s, 1H), 4.05 - 4.15 (m, 2H), 6.81 - 6.86 (m, 1H), 7.17 - 7.23 (m, 1H), 7.45 - 7.52 (m, 1H).
LCMS (ESI) m/z 377 [M+H]⁺, t_{R} = 0.758 min, mode N.

### Reference Example 2-80: Synthesis of methyl 5-chloro-2-({1-[2-(methylamino)ethyl]-1H-imidazol-2-yl}methoxy)benzoate hydrochloride

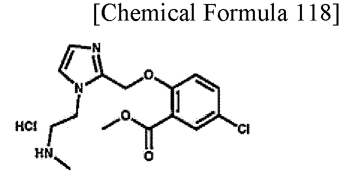

(1) To ethyl 1H-imidazole-2-carboxylate (1.3 g) and tert-butyl N-(2-hydroxyethyl)-N-methyl-carbamate (1.5 g) in toluene (10 mL), cyanomethylenetributylphosphorane (4.5 mL) was added, and the mixture was stirred at 90°C for 1 hour. The reaction solution was poured into water and extracted with EtOAc. The organic layer was washed with Brine, dried over MgSO₄, filtered, and then the solvent was concentrated. The residue was purified by NH type and OH type silica gel column chromatography (mobile phase: hexane/EtOAc = 50/50 to 0/100; v/v) to afford ethyl 1-[2-[tert-butoxycarbonyl(methyl)amino]ethyl]imidazole-2-carboxylate (2.3 g, colorless amorphous).

(2) To a solution of ethyl 1-[2-[tert-butoxycarbonyl(methyl)amino]ethyl]imidazole-2-carboxylate (2.3 g) in THF (20 mL), LiBH₄ (9.0 mL, 3 M THF solution) was added, and the mixture was stirred at 65°C for 3 hours. The reaction solution was poured into water and extracted with EtOAc. The organic layer was washed with Brine, dried over MgSO₄, filtered, and then the solvent was concentrated. The residue was purified by NH type silica gel column chromatography (mobile phase: MeOH/EtOAc = 0/100 to 10/90; v/v) to afford tert-butyl N-[2-[2-(hydroxymethyl)imidazol-1-yl]ethyl]-N-methyl-carbamate (1.8 g, colorless powder).

(3) By using the same approach as in Reference Example 2-77 (1), [1-[2-[tert-butoxycarbonyl(methyl)amino]ethyl]imidazol-2-yl]methyl 4-methylbenzenesulfonate (0.18 g, colorless powder) was obtained from tert-butyl N-[2-[2-(hydroxymethyl)imidazol-1-yl]ethyl]-N-methyl-carbamate (0.10 g).

(4) To a solution of [1-[2-[tert-butoxycarbonyl(methyl)amino]ethyl]imidazol-2-yl]methyl 4-methylbenzenesulfonate (0.16 g) in DMSO (3.0 mL), methyl 5-chloro-2-hydroxy-benzoate (0.13 g) and Cs₂CO₃ (0.25 g) were added, and the mixture was stirred at 80°C for 1 hour. The reaction solution was poured into water and extracted with EtOAc. The organic layer was washed with Brine, dried over MgSO₄, filtered, and then the solvent was concentrated. The residue was purified by OH type silica gel column chromatography (mobile phase: hexane/EtOAc) to afford methyl 2-[[1-[2-[tert-butoxycarbonyl(methyl)amino]ethyl]imidazol-2-yl]methoxy]-5-chloro-benzoat e (95 mg, colorless amorphous).

(5) By using the same approach as in Reference Example 2-25 (3), the title compound (0.77 g, colorless amorphous) was obtained from methyl 2-[[1-[2-[tert-butoxycarbonyl(methyl)amino]ethyl]imidazol-2-yl]methoxy]-5-chloro-benzoat e (0.74 g).
LCMS (ESI) m/z 324 [M+H]⁺, t_{R} = 0.206 min, mode N.

### Reference Example 2-81: Synthesis of methyl 5-chloro-2-({5-methyl-4-[2-(methylamino)ethyl]-4H-1,2,4-triazol-3-yl}methoxy)benzoate hydrochloride

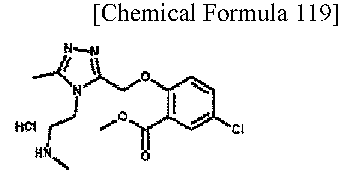

(1) To a solution of methyl 2-[2-[2-[tert-butoxycarbonyl(methyl)amino]ethylamino]-2-oxo-ethoxy]-5-chloro-benzoate (0.10 g) in THF (1.0 mL), the LAWESSON reagent (R) (0.10 g) was added, and the mixture was stirred at 50°C for 1 hour. The reaction solution was purified by OH type silica gel column chromatography (mobile phase: hexane/EtOAc = 75/25; v/v) to afford methyl 2-[2-[2-[tert-butoxycarbonyl(methyl)amino]ethylamino]-2-thioxo-ethoxy]-5-chloro-benzoate (0.10 g, colorless oily matter).

(2) To a solution of methyl 2-[2-[2-[tert-butoxycarbonyl(methyl)amino]ethylamino]-2-thioxo-ethoxy]-5-chloro-benzoate (0.10 g) in DMF (1.0 mL), Mel (30 mg) and K₂CO₃ (62 mg) were added, and the mixture was stirred at room temperature overnight. The reaction solution was poured into water and extracted with CHCl₃. The organic layer was washed with Brine, dried over MgSO₄, filtered, and the solvent was concentrated to afford methyl 2-[(2Z)-2-[2-[tert-butoxycarbonyl(methyl)amino]ethylimino]-2-methylsulfanyl-ethoxy]-5-chl oro-benzoate as a crude product.

(3) To a solution of methyl 2-[(2Z)-2-[2-[tert-butoxycarbonyl(methyl)amino]ethylimino]-2-methylsulfanyl-ethoxy]-5-chl oro-benzoate in DMF (1.0 mL), acetohydrazine (0.10 g) was added, and the mixture was stirred at 120°C for 4 hours. The reaction solution was poured into water and extracted with EtOAc. The organic layer was washed with Brine, dried over MgSO₄, filtered, and then the solvent was concentrated. The residue was purified by OH type silica gel column chromatography (mobile phase: hexane/EtOAc = 100/0 to 85/15; v/v) to afford methyl 2-[[4-[2-[tert-butoxycarbonyl(methyl)amino]ethyl]-5-methyl-1,2,4-triazol-3-yl]methoxy]-5-c hloro-benzoate (31 mg, colorless oily matter).

(4) By using the same approach as in Reference Example 2-1 (3), the title compound (27 mg, colorless powder) was obtained from methyl 2-[[4-[2-[tert-butoxycarbonyl(methyl)amino]ethyl]-5-methyl-1,2,4-triazol-3-yl]methoxy]-5-c hloro-benzoate (31 mg). LCMS (ESI) m/z 339 [M+H]⁺, t_{R} = 0.290 min, mode N.

### Reference Example 2-82: Synthesis of methyl 5-chloro-2-({4-[2-(methylamino)ethyl]-1H-1,2,3-triazol-1-yl}methyl)benzoate hydrochloride

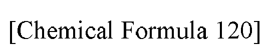

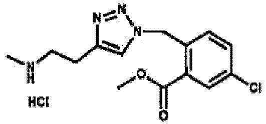

(1) To a solution of methyl 2-(azidomethyl)-5-chloro-benzoate (1.6 g) in DMF (20 mL), tert-butyl N-but-3-ynylcarbamate (1.0 g), copper(I) sulfate pentahydrate (0.30 g), sodium ascorbate (0.24 g), and water (10 mL) were added, and the mixture was stirred at room temperature overnight. The reaction solution was poured into water, which was poured into a K₂CO₃-aqueous ammonia solution and extracted with EtOAc. The organic layer was concentrated and purified by OH type silica gel column chromatography (mobile phase: hexane/EtOAc = 50/50 to 0/100; v/v) to afford methyl 2-[[4-[2-(tert-butoxycarbonylamino)ethyl]triazol-1-yl]methyl]-5-chloro-benzoate (1.6 g, colorless powder) as a crude product.

(2) To a solution of methyl 2-[[4-[2-(tert-butoxycarbonylamino)ethyl]triazol-1-yl]methyl]-5-chloro-benzoate (1.6 g) in DMF (10 mL), NaH (0.23 g, 60% in oil) and Mel (0.63 g) were sequentially added, and the mixture was stirred at room temperature for 2 hours. The reaction solution was poured into water and extracted with EtOAc. The organic layer was washed with Brine, dried over MgSO₄, filtered, and then the solvent was concentrated. The residue was purified by NH type silica gel column chromatography to afford methyl 2-[[4-[2-[tert-butoxycarbonyl(methyl)amino]ethyl]triazol-1-yl]methyl]-5-chloro-benzoate (0.28 g, colorless powder).

(3) By using the same approach as in Reference Example 2-1 (3), the title compound (0.24 g, light yellow amorphous) was obtained from methyl 2-[[4-[2-[tert-butoxycarbonyl(methyl)amino]ethyl]triazol-1-yl]methyl]-5-chloro-benzoate (0.28 g).
LCMS (ESI) m/z 309 [M+H]⁺, t_{R} = 0.415 min, mode N.

### Reference Example 2-83: Synthesis of methyl 5-fluoro-2-[5-(methylamino)pentyl]benzoate hydrochloride

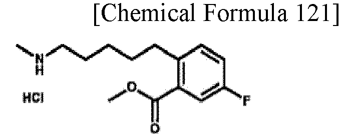

(1) To a solution of methyl 2-bromo-5-fluoro-benzoate (3.0 g) in toluene (43 mL)/MeOH (7.0 mL), 2-[(E)-5-chloropent-1-enyl]-4,4,5,5-tetramethyl-1,3,2-dioxaborolane (3.2 g), Pd(PPh₃)₄ (1.5 g), and a 2 M Na₂CO₃ aq. (22 mL) were added, and the mixture was stirred at 80°C for 3 hours. The reaction solution was poured into water and extracted with EtOAc. The organic layer was washed with Brine, dried over MgSO₄, filtered, and then the solvent was concentrated. The residue was purified by OH type silica gel column chromatography (mobile phase: hexane/EtOAc = 97/3; v/v) to afford methyl 2-[(E)-5-chloropent-1-enyl]-5-fluoro-benzoate (1.6 g, colorless oily matter).

(2) By the same approaches as in Reference Example 2-86 (2) and (3), the title compound (0.56 g, yellow oily matter) was obtained from the compound (1.6 g) obtained in Reference Example 2-83 (1).
¹H NMR (400 MHz, CDCl₃) 1.34 - 1.63 (m, 8H), 2.43 (s, 3H), 2.57 (t, J = 7.2 Hz, 2H), 2.85 - 2.95 (m, 2H), 3.88 - 3.93 (m, 3H), 7.09 - 7.15 (m, 1H), 7.18 - 7.25 (m, 1H), 7.56 (dd, J = 9.5, 2.8 Hz, 1H).
LCMS (ESI) m/z 254 [M+H]⁺, t_{R} = 0.514 min, mode N.

### Reference Example 2-84: Synthesis of methyl 2-(2-{[(benzyloxy)carbonyl][2-(methylamino)ethyl]amino}ethyl)-5-fluorobenzoate hydrochloride

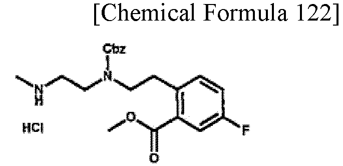

(1) (5-Fluoro-2-vinyl-phenyl)methanol (0.85 g) was dissolved in DMF (28 mL), imidazole (1.1 g) and TBDPSCl (1.7 g) were added thereto, and the mixture was stirred for 1 hour. After adding water to the reaction liquid and stirring the mixture, it was extracted with CHCl₃. The aqueous layer was separated using Phase Separator, the organic layer was concentrated under reduced pressure and dried, and the resulting residue was purified by OH type silica gel column chromatography (mobile phase: hexane/EtOAc = 95/5; v/v) to afford tert-butyl-[(5-fluoro-2-vinyl-phenyl)methoxy]-diphenyl-silane (2.0 g, colorless oily matter).

(2) To a solution of tert-butyl-[(5-fluoro-2-vinyl-phenyl)methoxy]-diphenyl-silane (2.0 g) in THF (50 mL), a solution of 0.9 M borane-THF complex (6.6 mL) was added at -78°C, and the mixture was stirred for 3 hours while raising the temperature to ice-cold. To the reaction solution, a 10% NaOH aq. (10 mL) and a 30% aqueous hydrogen peroxide solution (1.0 mL) were added, and the mixture was stirred from ice-cold to room temperature for 3 hours. After the reaction was completed, the reaction mixture was extracted by adding CHCl₃ and a Sat. NH₄Cl aq. The aqueous layer was separated using Phase Separator, the organic layer was concentrated under reduced pressure and dried, and the resulting residue was purified by OH type silica gel column chromatography (mobile phase: hexane/EtOAc = 90/10 to 80/20; v/v) to afford 2-[2-[[tert-butyl(diphenyl)silyl]oxymethyl]-4-fluoro-phenyl]ethanol (1.4 g, colorless oily matter).

(3) 2-[2-[[tert-Butyl(diphenyl)silyl]oxymethyl]-4-fluoro-phenyl]ethanol (1.2 g) was dissolved in CHCl₃ (30 mL), the DESS-MARTIN reagent (R) (1.9 g) was added thereto, and the mixture was stirred at room temperature for 2 hours. After adding a Sat. NaHCO₃ aq. to the reaction liquid and stirring the mixture, it was extracted with CHCl₃. The aqueous layer was separated using Phase Separator, the organic layer was concentrated under reduced pressure and dried, and the resulting residue was purified by OH type silica gel column chromatography (mobile phase: hexane/EtOAc = 95/5 to 90/10; v/v) to afford 2-[2-[[tert-butyl(diphenyl)silyl]oxymethyl]-4-fluoro-phenyl]acetaldehyde (1.1 g, colorless oily matter).

(4) 2-[2-[[tert-Butyl(diphenyl)silyl]oxymethyl]-4-fluoro-phenyl]acetaldehyde (1.1 g) was dissolved in CHCl₃ (28 mL), and tert-butyl N-(2-aminoethyl)-N-methyl-carbamate (0.98 g) was added thereto at room temperature. Then, AcOH (0.32 mL) and NaBH(OAc)₃ (1.2 g) were added thereto under ice cooling, and the mixture was stirred for 2 hours. After adding a Sat. NaHCO₃ aq. to the reaction liquid and stirring the mixture, it was extracted with CHCl₃. The aqueous layer was separated using Phase Separator, the organic layer was concentrated under reduced pressure and dried, and the resulting residue was purified by NH type silica gel column chromatography (mobile phase: hexane/EtOAc = 90/10 to 70/30; v/v) to afford tert-butyl N-[2-[2-[2-[[tert-butyl(diphenyl)silyl]oxymethyl]-4-fluoro-phenyl]ethylamino]ethyl]-N-meth yl-carbamate (1.4 g, colorless oily matter).

(5) To a solution of tert-butyl N-[2-[2-[2-[[tert-butyl(diphenyl)silyl]oxymethyl]-4-fluoro-phenyl]ethylamino]ethyl]-N-meth yl-carbamate (1.4 g) in CHCl₃ (25 mL), Et₃N (1.1 mL) and CbzCl (0.40 mL) were added at 0°C, and the mixture was stirred at room temperature for 1 hour. To the reaction solution, water and CHCl₃ were added, and the separating operation was carried out. The aqueous layer was separated using Phase Separator, and then the organic layer was concentrated under reduced pressure. The residue was purified by OH type silica gel column chromatography (mobile phase: hexane/EtOAc = 90/10 to 50/50; v/v) to afford tert-butyl N-[2-[benzyloxycarbonyl-[2-[2-[[tert-butyl(diphenyl)silyl]oxymethyl]-4-fluoro-phenyl]ethyl] amino]ethyl]-N-methyl-carbamate (1.3 g, colorless oily matter).

(6) tert-Butyl N-[2-[benzyloxycarbonyl-[2-[2-[[tert-butyl(diphenyl)silyl]oxymethyl]-4-fluoro-phenyl]ethyl] amino]ethyl]-N-methyl-carbamate (0.50 g) was dissolved in THF (7.2 mL), and after adding TBAF (1.4 mL, 1 M THF solution) thereto under ice cooling, the temperature was raised to room temperature and the mixture was stirred for 1 hour. After adding a Sat. NH₄Cl aq. to the reaction liquid and stirring the mixture, it was extracted with CHCl₃. The aqueous layer was separated using Phase Separator, the organic layer was concentrated under reduced pressure and dried, and the resulting residue was purified by OH type silica gel column chromatography (mobile phase: hexane/EtOAc = 90/10 to 50/50; v/v) to afford tert-butyl N-[2-[benzyloxycarbonyl-[2-[4-fluoro-2-(hydroxymethyl)phenyl]ethyl]amino]ethyl]-N-meth yl-carbamate (0.32 g, colorless oily matter).

(7) tert-Butyl N-[2-[benzyloxycarbonyl-[2-[4-fluoro-2-(hydroxymethyl)phenyl]ethyl]amino]ethyl]-N-meth yl-carbamate (0.32 g) was dissolved in CHCl₃ (4.8 mL), manganese dioxide (0.51 g) was added thereto, and the mixture was stirred at 75 °C for 4 hours. The reaction liquid was filtered through Celite (R), washed with CHCl₃, and the resulting filtrate was concentrated under reduced pressure and dried to afford tert-butyl N-[2-[benzyloxycarbonyl-[2-(4-fluoro-2-formyl-phenyl)ethyl]amino]ethyl]-N-methyl-carbam ate (0.35 g, light yellow amorphous). tert-Butyl N-[2-[benzyloxycarbonyl-[2-(4-fluoro-2-formyl-phenyl)ethyl]amino]ethyl]-N-methyl-carbam ate (0.32 g) was dissolved in tBuOH (2.8 mL), and a solution of 2-methyl-2-butene (0.39 g) in THF (2.8 mL) was added thereto. Then, an aqueous solution (0.92 mL) of sodium chlorite (0.25 g) and sodium dihydrogen phosphate (0.33 g) was added thereto, and the mixture was stirred at room temperature for 2 hours. The reaction liquid was concentrated under reduced pressure and the resulting residue was made acidic by adding CHCl₃ and a 1 M HCl aq. and then extracted. The aqueous layer was separated using Phase Separator, and the organic layer was concentrated under reduced pressure and dried to afford 2-[2-[benzyloxycarbonyl-[2-[tert-butoxycarbonyl(methyl)amino]ethyl]amino]ethyl]-5-fluorobenzoic acid (0.36 g, light yellow oily matter).

(8) To a solution of 2-[2-[benzyloxycarbonyl-[2-[tert-butoxycarbonyl(methyl)amino]ethyl]amino]ethyl]-5-fluorobenzoic acid (0.33 g) in DMF (3.5 mL), Mel (86 µL) and K₂CO₃ (0.29 g) were added, and the mixture was stirred at 80°C for 1 hour. To the reaction solution, EtOAc/toluene (1/4; v/v) and water were added, and the separating operation was carried out. The organic layer was concentrated under reduced pressure, and the resulting residue was purified by OH type silica gel column chromatography (mobile phase: hexane/EtOAc = 100/0 to 90/10; v/v) to afford methyl 2-[2-[benzyloxycarbonyl-[2-[tert-butoxycarbonyl(methyl)amino]ethyl]amino]ethyl]-5-fluorobenzoate (0.29 g, colorless oily matter).

(9) By using the same approach as in Reference Example 2-25 (3), methyl 2-[2-[benzyloxycarbonyl-[2-[tert-butoxycarbonyl(methyl)amino]ethyl]amino]ethyl]-5-fluorobenzoate (0.29 g) was dissolved in a 1,4-dioxane solution (2.9 mL), a 4 M hydrochloric acid 1,4-dioxane solution (2.9 mL) was added thereto, and the mixture was stirred at room temperature for 2 hours. The reaction solution was concentrated under reduced pressure and dried to afford the title compound (0.25 g, colorless amorphous).
LCMS (ESI) m/z 389 [M+H]⁺, t_{R} = 0.654 min, mode N.

### Reference Example 2-85: Synthesis of methyl 5-chloro-2-{[3-(methylamino)propane-1-sulfonyl]amino}benzoate

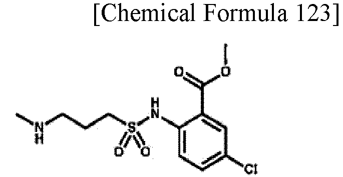

(1) Methyl 2-amino-5-chloro-benzoate (4.0 g) was dissolved in CHCl₃ (0.10 L), Et₃N (9.0 mL) and 3-chloropropane-1-sulfonyl chloride (2.7 mL) were added thereto under ice cooling, and the mixture was stirred at room temperature for 1 hour. After adding water to the reaction liquid and stirring the mixture, it was extracted with CHCl₃. The aqueous layer was separated using Phase Separator, the organic layer was concentrated under reduced pressure and dried, and the resulting residue was purified by OH type silica gel column chromatography (mobile phase: hexane/EtOAc = 90/10 to 50/50; v/v) to afford methyl 5-chloro-2-(3-chloropropylsulfonylamino)benzoate (3.8 g, colorless powder).

(2) To a solution of methyl 5-chloro-2-(3-chloropropylsulfonylamino)benzoate (3.8 g) in DMF (50 mL), Boc₂O (4.5 g), pyridine (2.8 mL), and DMAP (0.14 g) were added, and the mixture was stirred at room temperature for 3 hours. The reaction liquid was poured into water and extracted with EtOAc/toluene (1/4; v/v). The organic layer was washed with Brine, dried over MgSO₄, filtered, and then the solvent was concentrated. The residue was purified by OH type silica gel column chromatography (mobile phase: hexane/EtOAc = 90/10 to 80/20; v/v) to afford methyl 2-[tert-butoxycarbonyl(3-chloropropylsulfonyl)amino]-5-chloro-benzoate (4.7 g, yellow oily matter).

(3) Methyl 2-[tert-butoxycarbonyl(3-chloropropylsulfonyl)amino]-5-chloro-benzoate (3.0 g) was dissolved in MeCN (70 mL), Na₂CO₃ (1.5 g), NaI (1.3 g), and 1-(4-methoxyphenyl)-N-methylmethanamine (2.1 g) were added, and the mixture was stirred at 90°C for 6 hours. After adding water to the reaction liquid and stirring the mixture, it was extracted with CHCl₃. The aqueous layer was separated using Phase Separator, the organic layer was concentrated under reduced pressure and dried, and the resulting residue was purified by OH type silica gel column chromatography (mobile phase: hexane/EtOAc = 90/10 to 80/20; v/v) to afford methyl 2-[tert-butoxycarbonyl-[3-[(4-methoxyphenyl)methyl-methyl-amino]propylsulfonyl]amino]-5-chloro-benzoate (4.8 g, yellow oily matter).

(4) By using the same approach as in Reference Example 2-25 (3), methyl 5-chloro-2-[3-[(4-methoxyphenyl)m ethyl-methyl-amino]propylsulfonylamino]benzoate (1.6 g, light yellow oily matter) was obtained from methyl 2-[tert-butoxycarbonyl-[3-[(4-methoxyphenyl)methyl-methyl-amino]propylsulfonyl]amino]-5-chloro-benzoate (4.8 g).

(5) To a solution of methyl 5-chloro-2-[3-[(4-methoxyphenyl)methyl-methyl-amino]propylsulfonylamino]benzoate (0.21 g) in CHCl₃ (5.0 mL), 1-chloroethyl chloroformate (63 µL) was added at 0°C, and the mixture was stirred at room temperature for 1 hour. The reaction liquid was concentrated, MeOH (5.0 mL) was added to the residue, and the mixture was stirred at 80°C for 1 hour. The reaction liquid was concentrated, suspended in EtOAc, and filtered to afford the title compound (0.12 g, light yellow amorphous).
LCMS (ESI) m/z 321 [M+H]⁺, t_{R} = 0.498 min, mode N.

### Reference Example 2-86: Synthesis of methyl 5-ethyl-2-{[3-(methylamino)propane-1-sulfonyl]amino}benzoate

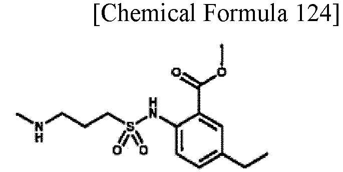

(1) By using the same approach as in Reference Example 2-85 (1), methyl 2-(3-chloropropylsulfonylamino)-5-ethyl-benzoate (0.74 g, colorless powder) was obtained from methyl 2-amino-5-ethyl-benzoate (0.50 g).

(2) Methyl 2-(3-chloropropylsulfonylamino)-5-ethyl-benzoate (0.73 g) was dissolved in MeCN (23 mL), Na₂CO₃ (0.49 g), NaI (0.41 g), and N-methyl-1-phenylmethanamine (0.83 g) were added thereto, and the mixture was stirred at 90°C for 30 hours. After adding water to the reaction liquid and stirring the mixture, it was extracted with CHCl₃. The aqueous layer was separated using Phase Separator, the organic layer was concentrated under reduced pressure and dried, and the resulting residue was purified by OH type silica gel column chromatography (mobile phase: hexane/EtOAc = 90/10 to 80/20; v/v) to afford methyl 2-[3-[benzyl(methyl)amino]propylsulfonylamino]-5-ethyl-benzoate (0.49 g, colorless oily matter).

(3) Methyl 2-[3-[benzyl(methyl)amino]propylsulfonylamino]-5-ethyl-benzoate (0.49 g) was dissolved in MeOH (12 mL), 10% palladium-carbon (0.24 g) was added thereto, and the mixture was stirred for 2 hours under a hydrogen gas atmosphere. The reaction liquid was filtered through Celite (R) and washed with MeOH, and the resulting filtrate was concentrated under reduced pressure and dried to afford the title compound (0.35 g, colorless powder).
¹H NMR (400 MHz, CDCl₃) δ ppm 1.19 - 1.27 (m, 3H), 1.91 - 2.00 (m, 2H), 2.36 (s, 3H), 2.59 - 2.69 (m, 4H), 3.19 - 3.26 (m, 2H), 3.93 (s, 3H), 7.37 (dd, J =8.6, 2.1 Hz, 1H), 7.67 (d, J = 8.6 Hz, 1H), 7.86 (d, J = 2.1 Hz, 1H).
LCMS (ESI) m/z 315 [M+H]⁺, t_{R} = 0.532 min, mode N.

### Reference Example 2-87: Synthesis of methyl 5-methyl-2-{[3-(methylamino)propane-1-sulfonyl]amino}benzoate

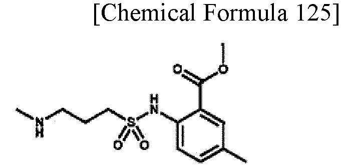

By using the same approaches as in Reference Example 2-86 (1) to (3), the title compound (0.56 g, colorless powder) was obtained from methyl 2-amino-5-methylbenzoate (3.0 g).
¹H NMR (400 MHz, CDCl₃) δ ppm 1.91 - 2.00 (m, 2H), 2.33 (s, 3H), 2.36 (s, 3H), 2.66 (t, J = 6.8 Hz, 2H), 3.16 - 3.27 (m, 2H), 3.93 (s, 3H), 7.34 (dd, J = 8.6, 1.8 Hz, 1H), 7.65 (d, J = 8.6 Hz, 1H), 7.84 (d, J = 1.8 Hz, 1H).
LCMS (ESI) m/z 301 [M+H]⁺, t_{R} = 0.436 min, mode N.

### Reference Example 2-88: Synthesis of methyl 4-fluoro-5-methyl-2-{[3-(methylamino)propane-1-sulfonyl]amino}benzoate hydrochloride

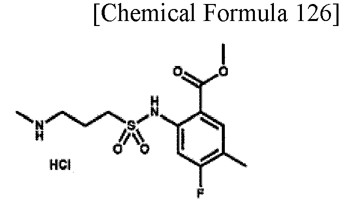

By using the same approaches as in Reference Example 2-85 (1) to (5), the title compound (0.22 g, colorless powder) was obtained from methyl 2-amino-4-fluoro-5-methylbenzoate (0.50 g).
¹H NMR (400 MHz, CDCl₃) δ ppm 2.23 (s, 3H), 2.34 - 2.45 (m, 2H), 2.70 (s, 3H), 3.18 (t, J = 7.3 Hz, 2H), 3.36 - 3.46 (m, 2H), 3.93 (s, 3H), 7.40 (d, J = 11.4 Hz, 1H), 7.90 (d, J = 8.3 Hz, 1H).
LCMS (ESI) m/z 319 [M+H]⁺, t_{R} = 0.533 min, mode N.

### Reference Example 2-89: Synthesis of methyl 1-methyl-4-{[3-(methylamino)propane-1-sulfonyl]amino}-1H-pyrazole-3-carboxylate

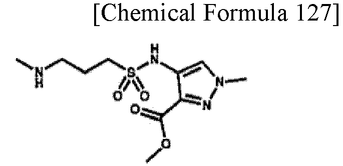

By using the same approaches as in Reference Example 2-85 (1) to (5), the title compound (colorless powder) was obtained as a crude product from methyl 4-amino-1-methyl-1H-pyrazole-3-carboxylate (1.0 g).
LCMS (ESI) m/z 291 [M+H]⁺, t_{R} = 0.309 min, mode H.

### Reference Example 2-90: Synthesis of methyl 3-{[3-(methylamino)propane-1-sulfonyl]amino}thiophene-2-carboxylate hydrochloride

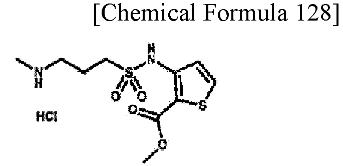

By using the same approaches as in Reference Example 2-85 (1) to (5), the title compound (0.75 g, colorless powder) was obtained from methyl 3-aminothiophene-2-carboxylate (1.5 g).
¹H NMR (400 MHz, DMSO-d₆) δ ppm 1.99 - 2.12 (m, 2H), 2.50 (s, 3H), 2.90 - 3.03 (m, 2H), 3.46 - 3.53 (m, 2H), 3.85 (s, 3H), 7.29 (d, J = 5.4 Hz, 1H), 7.96 (d, J = 5.4 Hz, 1H), 8.95 (br s, 2H), 9.49 (br s, 1H).
LCMS (ESI) m/z 293 [M+H]⁺, t_{R} = 0.297 min, mode N.

### Reference Example 2-91: Synthesis of methyl 2-{[3-(ethylamino)propane-1-sulfonyl]amino}-5-fluorobenzoate

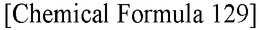

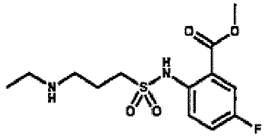

By using the same approaches as in Reference Example 2-85 (1) to (4) and Reference Example 2-86 (3), the title compound (0.68 g, colorless amorphous) was obtained from methyl 2-amino-5-fluoro-benzoate (4.0 g) and N-benzylethanamine (1.4 g).
LCMS (ESI) m/z 319 [M+H]⁺, t_{R} = 0.374 min, mode N.

### Reference Example 2-92: Synthesis of methyl 5-methyl-2-{[4-(methylamino)butane-1-sulfonyl]amino}benzoate

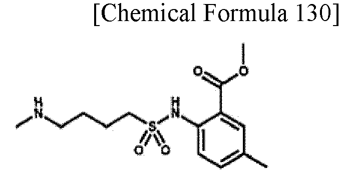

By using the same approaches as in Reference Example 2-86 (1) to (3), the title compound (0.14 g, colorless powder) was obtained from methyl 2-amino-5-methylbenzoate (3.0 g) and 4-chlorobutane-1-sulfonyl chloride (4.2 g).
¹H NMR (400 MHz, CDCl₃) δ ppm 1.44 - 1.60 (m, 2H), 1.76 - 1.89 (m, 2H), 2.34 (s, 3H), 2.38 (s, 3H), 2.53 (t, J = 7.1 Hz, 2H), 3.10 - 3.17 (m, 2H), 3.93 (s, 3H), 7.34 (dd, J = 8.4, 1.7 Hz, 1H), 7.65 (d, J = 8.4 Hz, 1H), 7.85 (d, J = 1.7 Hz, 1H).
LCMS (ESI) m/z 315 [M+H]⁺, t_{R} = 0.487 min, mode N.

### Reference Example 2-93: Synthesis of methyl 5-methyl-2-{[2-(methylamino)ethanesulfonyl]amino}benzoate

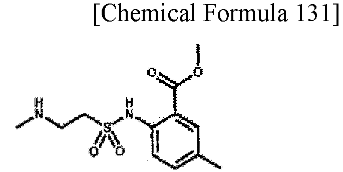

By using the same approaches as in Reference Example 2-86 (1) to (3), the title compound (0.53 g, yellow powder) was obtained from methyl 2-amino-5-methylbenzoate (2.0 g) and 2-chloroethane-1-sulfonyl chloride (2.4 g).
¹H NMR (400 MHz, DMSO-d₆) δ ppm 2.27 (s, 3H), 2.38 (s, 3H), 3.00 - 3.07 (m, 2H), 3.30 - 3.37 (m, 2H), 3.84 (s, 3H), 7.31 - 7.38 (m, 1H), 7.40 - 7.48 (m, 1H), 7.65 - 7.71 (m, 1H), 7.73 - 8.07 (m, 2H).
LCMS (ESI) m/z 287 [M+H]⁺, t_{R} = 0.371 min, mode N.

### Reference Example 2-94: Synthesis of methyl 2-[(3-aminopropane-1-sulfonyl)amino]-5-methylbenzoate

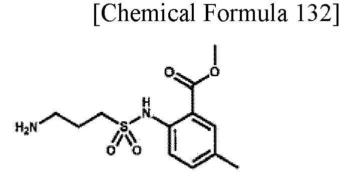

(1) By using the same approach as in Reference Example 2-85 (1), methyl 2-[(3-chloropropane-1-sulfonyl)amino]-5-methylbenzoate (5.8 g, colorless powder) was obtained from methyl 2-amino-5-methylbenzoate (3.0 g) and 3-chloropropane-1-sulfonyl chloride (3.9 g).

(2) To a solution of methyl 2-[(3-chloropropane-1-sulfonyl)amino]-5-methylbenzoate (5.6 g) in DMSO (91 mL), sodium azide (1.5 g) was added, and the mixture was stirred at 80°C for 3 hours. The reaction liquid was poured into water and extracted with EtOAc. The organic layer was concentrated. The residue was purified by OH type silica gel column chromatography (mobile phase: hexane/EtOAc = 90/10 to 0/100; v/v) to afford methyl 2-[(3-azidopropane-1-sulfonyl)amino]-5-methylbenzoate (4.4 g, colorless powder).

(3) By using the same approach as in Reference Example 2-86 (3), the title compound (0.93 g, colorless powder) was obtained from methyl 2-[(3-azidopropane-1-sulfonyl)amino]-5-methylbenzoate (1.0 g).
¹H NMR (400 MHz, CDCl₃) δ ppm 1.87 - 1.98 (m, 2H), 2.33 (s, 3H), 2.80 (t, J = 6.5 Hz, 2H), 3.18 - 3.27 (m, 2H), 3.93 (s, 3H), 7.34 (dd, J = 8.5, 1.7 Hz, 1H), 7.65 (d, J = 8.5 Hz, 1H), 7.84 (d, J = 1.7 Hz, 1H).
LCMS (ESI) m/z 287 [M+H]⁺, t_{R} = 0.415 min, mode N.

### Reference Example 2-95: Synthesis of methyl 2-{(methanesulfonyl)[4-(methylamino)butyl]amino}-5-methylbenzoate hydrochloride

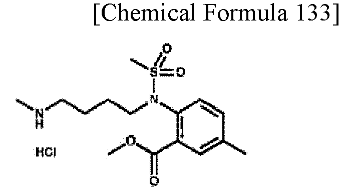

By using the same approaches as in Reference Example 2-59 (1) and Reference Example 2-25 (3), the title compound (0.34 g, colorless oily matter) was obtained from tert-butyl N-(4-hydroxybutyl)-N-methyl-carbamate (0.25 g) and methyl 2-[(methanesulfonyl)amino]-5-methylbenzoate (0.20 g).
LCMS (ESI) m/z 329 [M+H]⁺, t_{R} = 0.700 min, mode H.

### Reference Example 2-96: Synthesis of methyl 5-methyl-2-{[4-(methylamino)butyl](trifluoromethanesulfonyl)amino}benzoate

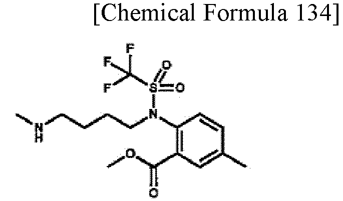

By using the same approaches as in Reference Example 2-59 (1) and Reference Example 2-25 (3), the title compound (0.13 g, colorless powder) was obtained from tert-butyl N-(4-hydroxybutyl)-N-methyl-carbamate (0.12 g) and methyl 5-methyl-2-[(trifluoromethanesulfonyl)amino]benzoate (0.30 g).
¹H NMR (400 MHz, DMSO-d₆) δ ppm 1.48 - 1.67 (m, 4H), 2.40 (s, 3H), 2.45 - 2.50 (m, 3H), 2.76 - 2.89 (m, 2H), 3.69 - 3.81 (m, 1H), 3.84 (s, 3H), 3.86 - 3.97 (m, 1H), 7.44 - 7.49 (m, 1H), 7.55 (dd, J = 8.1, 1.6 Hz, 1H), 7.80 (d, J = 1.6 Hz, 1H), 8.65 (br s, 2H).
LCMS (ESI) m/z 383 [M+H]⁺, t_{R} = 0.599 min, mode N.

### Reference Example 2-97: Synthesis of methyl 2-{(methanesulfonyl)[5-(methylamino)pentyl]amino}-5-methylbenzoate

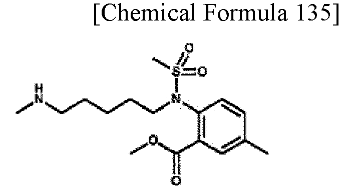

By using the same approaches as in Reference Example 2-59 (1) and Reference Example 2-25 (3), the title compound (0.50 g, colorless oily matter) was obtained from tert-butyl (5-hydroxypentyl)methylcarbamate (0.35 g) and methyl 2-[(methanesulfonyl)amino]-5-methylbenzoate (0.30 g).
LCMS (ESI/APCI dual) m/z 343 [M+H]⁺, t_{R} = 0.773 min, mode H.

### Reference Example 2-98: Synthesis of methyl 2-{[4-(methylamino)butane-1-sulfonyl]amino}-2-phenylpropanoate

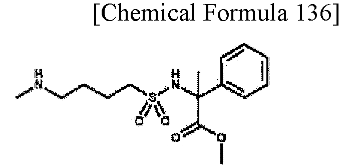

By using the same approaches as in Reference Example 2-86 (1) to (3), the title compound (0.24 g, light yellow oily matter) was obtained from methyl 2-amino-2-phenylpropanoate (0.23 g), 4-chlorobutane-1-sulfonyl chloride (0.30 g), and 1-(4-methoxyphenyl)-N-methylmethanamine (0.38 g).
¹H NMR (400 MHz, DMSO-d₆) δ ppm 1.58 - 1.80 (m, 4H), 1.86 (s, 3H), 2.50 (s, 3H), 2.79 - 2.88 (m, 2H), 2.95 - 3.08 (m, 2H), 3.66 (s, 3H), 7.30 - 7.41 (m, 3H), 7.43 - 7.49 (m, 2H), 8.04 (br s, 2H).

### Reference Example 2-99: Synthesis of methyl 2-(4-fluorophenyl)-2-{[4-(methylamino)butane-1-sulfonyl]amino}propanoate

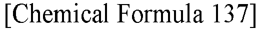

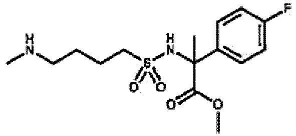

By using the same approaches as in Reference Example 2-86 (1) to (3), the title compound (0.63 g, colorless amorphous) was obtained from methyl 2-amino-2-(4-fluorophenyl)propanoate (0.59 g), 4-chlorobutane-1-sulfonyl chloride (0.69 g), and N-methyl-1-phenylmethanamine (0.76 g).
¹H NMR (400 MHz, CDCl₃) δ ppm 1.75 - 1.99 (m, 4H), 1.99 - 2.10 (m, 3H), 2.68 (s, 3H), 2.73 - 2.89 (m, 2H), 2.89 - 3.01 (m, 2H), 3.49 (s, 3H), 3.75 (s, 3H), 7.06 - 7.12 (m, 2H), 7.42 - 7.52 (m, 2H).
LCMS (ESI) m/z 347 [M+H]⁺, t_{R} = 0.747 min, mode H.

### Reference Example 2-100: Synthesis of methyl 2-(4-chlorophenyl)-2-{[4-(methylamino)butane-1-sulfonyl]amino}propanoate

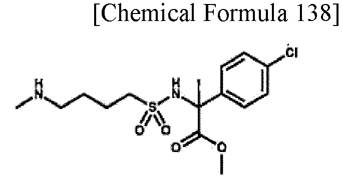

By using the same approaches as in Reference Example 2-86 (1) to (2) and Reference Example 2-85 (5), the title compound (0.43 g, colorless oily matter) was obtained from methyl 2-amino-2-(4-chlorophenyl)propanoate (0.52 g), 4-chlorobutane-1-sulfonyl chloride (0.56 g), and 1-(4-methoxyphenyl)-N-methylmethanamine (0.60 g).
¹H NMR (400 MHz, CDCl₃) δ ppm 1.39 - 1.64 (m, 2H), 1.66 - 1.94 (m, 2H), 2.05 (s, 3H), 2.41 (s, 3H), 2.54 (t, J = 7.1 Hz, 2H), 2.61 - 2.84 (m, 2H), 3.76 (s, 3H), 7.33 - 7.41 (m, 4H). LCMS (ESI) m/z 363 [M+H]⁺, t_{R} = 0.471 min, mode N.

### Reference Example 2-101: Synthesis of methyl 1-{[4-(methylamino)butane-1-sulfonyl]amino}-2,3-dihydro-1H-indene-1-carboxylate

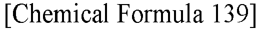

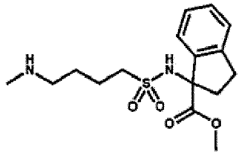

By using the same approaches as in Reference Example 2-86 (1) to (3), the title compound (0.45 g, light yellow oily matter) was obtained from methyl 1-amino-2,3-dihydro-1H-indene-1-carboxylate (0.43 g), 4-chlorobutane-1-sulfonyl chloride (0.43 g), and 1-(4-methoxyphenyl)-N-methylmethanamine (0.71 g).
¹H NMR (400 MHz, DMSO-d₆) δ ppm 1.56 - 1.73 (m, 4H), 2.31 - 2.43 (m, 1H), 2.48 (s, 3H), 2.70 - 2.81 (m, 3H), 2.81 - 3.07 (m, 4H), 3.64 (s, 3H), 7.21 - 7.28 (m, 1H), 7.29 - 7.34 (m, 2H), 7.41 - 7.47 (m, 1H), 8.09 (br s, 2H).

### Reference Example 2-102: Synthesis of methyl N-[4-(methylamino)butane-1-sulfonyl]phenylalaninate

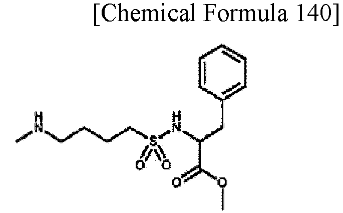

By using the same approaches as in Reference Example 2-86 (1) to (3), the title compound (0.77 g, colorless powder) was obtained from methyl 2-amino-3-phenyl-propanoate hydrochloride (0.60 g), 4-chlorobutane-1-sulfonyl chloride (0.64 g), and 1-(4-methoxyphenyl)-N-methylmethanamine (1.2 g).
¹H NMR (400 MHz, DMSO-d₆) δ ppm 1.25 - 1.54 (m, 4H), 2.49 (s, 3H), 2.60 - 2.76 (m, 4H), 2.77 - 2.86 (m, 1H), 2.99 - 3.07 (m, 1H), 3.64 (s, 3H), 4.07 - 4.18 (m, 1H), 7.21 - 7.36 (m, 5H), 7.90 (br s, 1H), 8.64 (br s, 2H).
LCMS (ESI) m/z 329 [M+H]⁺, t_{R} = 0.712 min, mode H.

### Reference Example 2-103: Synthesis of ethyl 2-methyl-3-[4-(methylamino)butoxy]-2-phenylpropanoate hydrochloride

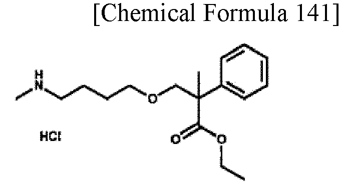

(1) A solution of ethyl 3-hydroxy-2-methyl-2-phenylpropanoate (0.50 g) in DMF (12 mL) was cooled to 0°C. To this, NaH (0.15 g, 60% in oil) was added, and the mixture was stirred at 0°C for 20 minutes. Then, tert-butyl N-(4-bromobutyl)-N-methyl-carbamate (0.89 g) was added thereto, and the mixture was stirred at room temperature for 3 hours. To the reaction solution, a Sat. NH₄Cl aq. was added under ice cooling, and the separating operation was carried out with CHCl₃. The aqueous layer was separated using Phase Separator, and then the organic layer was concentrated under reduced pressure. Purification by OH type silica gel column chromatography (mobile phase: hexane/EtOAc = 90/10 to 65/35; v/v) afforded ethyl 3-[4-[tert-butoxycarbonyl(methyl)amino]butoxy]-2-methyl-2-phenyl-propanoate (0.27 g, colorless oily matter).

(2) To a solution of ethyl 3-[4-[tert-butoxycarbonyl(methyl)amino]butoxy]-2-methyl-2-phenyl-propanoate (0.27 g) in 1,4-dioxane (2.5 mL), 4 M hydrochloric acid 1,4-dioxane (2.5 mL) and MeOH (2.0 mL) were added, and the mixture was stirred at room temperature for 1.5 hours. The reaction solution was concentrated under reduced pressure to afford the title compound (0.25 g, yellow oily matter).
¹H NMR (400 MHz, CDCl₃) δ ppm 1.18 - 1.23 (m, 3H), 1.65 - 1.74 (m, 2H), 1.86 - 1.97 (m, 2H), 2.52 - 2.58 (m, 3H), 2.61 - 2.73 (m, 2H), 2.84 - 2.96 (m, 3H), 3.44 - 3.56 (m, 2H), 3.63 (d, J = 9.2 Hz, 1H), 3.96 (d, J = 9.2 Hz, 1H), 4.17 (q, J = 7.1 Hz, 2H), 7.27 - 7.37 (m, 5H), 9.18 - 9.55 (m, 2H).
LCMS (ESI) m/z 294 [M+H]⁺, t_{R} = 0.334 min, mode L.

### Reference Example 2-104: Synthesis of ethyl 2-(4-fluorophenyl)-2-methyl-3-[4-(methylamino)butoxy]propanoate hydrochloride

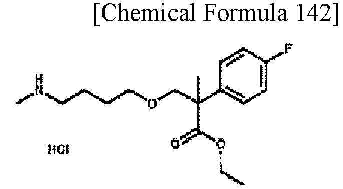

By using the same approaches as in Reference Example 2-103 (1) to (2), the title compound (0.29 g, yellow oily matter) was obtained from ethyl 2-(4-fluorophenyl)-3-hydroxy-2-methylpropanoate (0.50 g).
LCMS (ESI) m/z 312 [M+H]⁺, t_{R} = 0.374 min, mode L.

### Reference Example 2-105: Synthesis of ethyl 2-methyl-3-[4-(methylamino)butoxy]-2-(pyridin-3-yl)propanoate hydrochloride

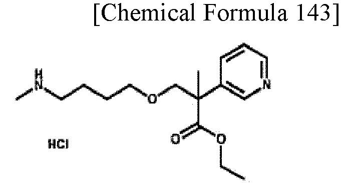

By using the same approaches as in Reference Example 2-103 (1) to (2), the title compound (1.0 g, yellow oily matter) was obtained from ethyl 3-hydroxy-2-methyl-2-(pyridin-3-yl)propanoate (0.99 g).
¹H NMR (400 MHz, CDCl₃) δ ppm 1.16 - 1.30 (m, 3H), 1.33 - 1.51 (m, 2H), 1.72 (d, J = 5.3 Hz, 3H), 1.83 - 2.24 (m, 2H), 2.58 - 2.74 (m, 2H), 2.85 - 3.09 (m, 1H), 3.32 - 3.55 (m, 2H), 3.65 - 3.76 (m, 7H), 4.13 - 4.32 (m, 1H), 7.93 - 8.02 (m, 1H), 8.35 (d, J = 8.3 Hz, 0.35H), 8.46 (d, J = 8.3 Hz, 0.65H), 8.63 - 8.73 (m, 0.35H), 8.77 - 8.81 (m, 0.65H), 8.94 (s, 0.65H), 9.02 (br s, 0.35H), 9.56 (br s, 0.65H), 10.06 (br s, 0.35H).
LCMS (ESI) m/z 295 [M+H]⁺, t_{R} = 0.245 min, mode H.

### Reference Example 2-106: Synthesis of ethyl 2-methyl-3-(2-{[2-(methylamino)ethyl]amino}-2-oxoethoxy)-2-phenylpropanoate hydrochloride

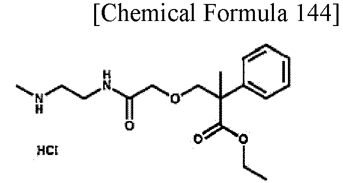

(1) By using the same approach as in Reference Example 2-103 (1), ethyl 3-(2-tert-butoxy-2-oxo-ethoxy)-2-methyl-2-phenyl-propanoate (0.33 g, colorless oily matter) was obtained from ethyl 3-hydroxy-2-methyl-2-phenylpropanoate (0.50 g) and tert-butyl bromoacetate (0.47 g).

(2) By using the same approach as in Reference Example 2-77 (5), 2-(3-ethoxy-2-methyl-3-oxo-2-phenyl-propoxy)acetic acid (0.22 g) was obtained from ethyl 3-(2-tert-butoxy-2-oxo-ethoxy)-2-methyl-2-phenyl-propanoate (0.27 g).

(3) By using the same approaches as in Reference Example 2-8 (1) and (2), the title compound (0.29 g, light yellow oily matter) was obtained from 2-(3-ethoxy-2-methyl-3-oxo-2-phenyl-propoxy)acetic acid (0.22 g).
LCMS (ESI) m/z 323 [M+H]⁺, t_{R} = 0.482 min, mode N.

### Reference Example 2-107: Synthesis of ethyl 2-(4-fluorophenyl)-2-methyl-3-[4-(methylamino)butanamido]propanoate

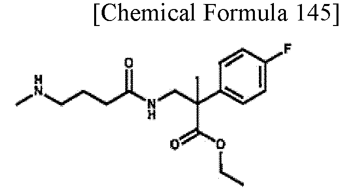

(1) By using the same approach as in Reference Example 2-5 (2), ethyl 2-(4-fluorophenyl)-2-methyl-3-oxo-propanoate (0.22 g, colorless oily matter) was obtained from ethyl 2-(4-fluorophenyl)-3-hydroxy-2-methylpropanoate (0.25 g).

(2) By using the same approach as in Reference Example 2-73 (4), ethyl 2-(4-fluorophenyl)-3-[(4-methoxyphenyl)methylamino]-2-methyl-propanoate (0.27 g, colorless oily matter) was obtained from ethyl 2-(4-fluorophenyl)-2-methyl-3-oxo-propanoate (0.21 g).

(3) By using the same approach as in Reference Example 2-86 (3), ethyl 3-amino-2-(4-fluorophenyl)-2-methyl-propanoate (0.19 g, colorless oily matter) was obtained from ethyl 2-(4-fluorophenyl)-3-[(4-methoxyphenyl)methylamino]-2-methyl-propanoate (0.27 g).

(4) By using the same approach as in Reference Example 2-8 (1), ethyl 2-(4-fluorophenyl)-3-[4-[(4-methoxyphenyl)methyl-methyl-amino]butanoylamino]-2-methylpropanoate (0.25 g, colorless oily matter) was obtained from ethyl 3-amino-2-(4-fluorophenyl)-2-methyl-propanoate (0.17 g) and 4-[(4-methoxyphenyl)methyl-methyl-amino]butanoic acid (0.18 g).

(5) By using the same approach as in Reference Example 2-86 (3), the title compound (0.17 g, colorless oily matter) was obtained from ethyl 2-(4-fluorophenyl)-3-[4-[(4-methoxyphenyl)methyl-methyl -amino]butanoylamino]-2-methylpropanoate (0.25 g).
¹H NMR (400 MHz, CDCl₃) δ ppm 1.22 (t, J = 7.2 Hz, 3H), 1.59 (s, 3H), 1.84 - 2.03 (m, 2H), 2.29 - 2.42 (m, 2H), 2.53 (s, 3H), 2.61 - 2.86 (m, 3H), 3.62 - 3.73 (m, 2H), 4.13 - 4.25 (m, 2H), 6.51 - 6.63 (m, 1H), 6.99 - 7.10 (m, 2H), 7.22 - 7.28 (m, 2H).
LCMS (ESI) m/z 325 [M+H]⁺, t_{R} = 0.449 min, mode N.

### Reference Example 2-108: Synthesis of ethyl 2-(4-fluorophenyl)-2-methyl-3-[methyl(2-{[2-(methylamino)ethyl]amino}-2-oxoethyl)amino ]propanoate hydrochloride

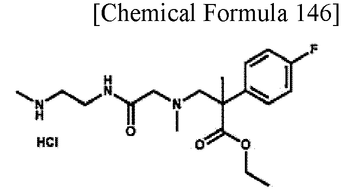

(1) By using the same approach as in Reference Example 2-73 (4), ethyl 3-[(2-tert-butoxy-2-oxo-ethyl)-methyl-amino]-2-(4-fluorophenyl)-2-methyl-propanoate (97 mg, colorless oily matter) was obtained from the compound (0.16 g) obtained in Reference Example 2-107 (1) and tert-butyl 2-(methylamino)acetate (0.39 g).

(2) By using the same approach as in Reference Example 2-77 (5), 2-[[3-ethoxy-2-(4-fluorophenyl)-2-methyl-3-oxo-propyl]-methyl-amino]acetic acid trifluoroacetate salt (0.11 g) was obtained from ethyl 3-[(2-tert-butoxy-2-oxo-ethyl)-methyl-amino]-2-(4-fluorophenyl)-2-methyl-propanoate (97 mg).

(3) By using the same approaches as in Reference Example 2-8 (1) and (2), the title compound (0.13 g, colorless oily matter) was obtained from 2-[[3-ethoxy-2-(4-fluorophenyl)-2-methyl-3-oxo-propyl]-methyl-amino]acetic acid trifluoroacetate salt (0.11 g).
LCMS (ESI) m/z 354 [M+H]⁺, t_{R} = 0.228 min, mode N.

### Reference Example 2-109: Synthesis of ethyl 2-(4-fluorophenyl)-3-{[(4-methoxyphenyl)methyl](2-{ [2-(methylamino)ethyl]amino}-2-oxoe thyl)amino}-2-methylpropanoate hydrochloride

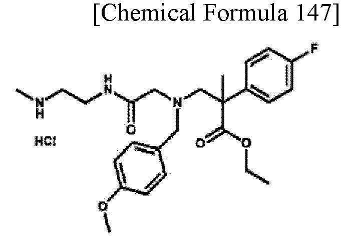

(1) By using the same approach as in Reference Example 2-25 (2), ethyl 3-[(2-tert-butoxy-2-oxo-ethyl)-[(4-methoxyphenyl)methyl]amino]-2-(4-fluorophenyl)-2-meth yl-propanoate (0.44 g, colorless oily matter) was obtained from the compound (0.32 g) obtained in Reference Example 2-107 (2) and tert-butyl bromoacetate (0.36 g).

(2) By using the same approach as in Reference Example 2-77 (5), 2-[[3-ethoxy-2-(4-fluorophenyl)-2-methyl-3-oxo-propyl]-[(4-methoxyphenyl)methyl]amino]a cetic acid trifluoroacetate salt (0.49 g, light yellow oily matter) was obtained from ethyl 3-[(2-tert-butoxy-2-oxo-ethyl)-[(4-methoxyphenyl)methyl]amino]-2-(4-fluorophenyl)-2-meth yl-propanoate (0.44 g).

(3) By using the same approaches as in Reference Example 2-8 (1) and (2), the title compound (0.48 g, colorless amorphous) was obtained from 2-[[3-ethoxy-2-(4-fluorophenyl)-2-methyl-3-oxo-propyl]-[(4-methoxyphenyl)methyl]amino]a cetic acid trifluoroacetate salt (0.49 g).
LCMS (ESI) m/z 460 [M+H]⁺, t_{R} = 0.646 min, mode N.

### Reference Example 2-110: Synthesis of methyl {2- [4-(methylamino)butoxy]phenyl} acetate hydrochloride

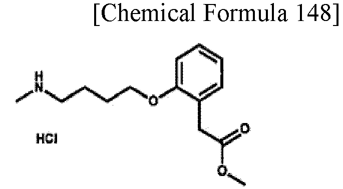

By using the same approaches as in Reference Example 2-25 (2) and (3), the title compound (0.19 g, yellow amorphous) was obtained from tert-butyl 4-[tert-butoxycarbonyl(methyl)amino]butyl methanesulfonate (0.69 g) obtained in Reference Example 2-25 (1) and methyl (2-hydroxyphenyl)acetate (0.41 g).
LCMS (ESI) m/z 252 [M+H]⁺, t_{R} = 0.743 min, mode H.

### Reference Example 2-111: Synthesis of methyl 3-[3-(methylamino)propylsulfonylamino]-3-phenyl-propanoate

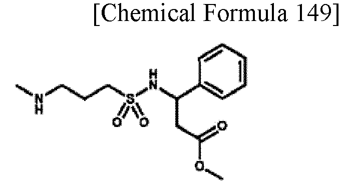

By using the same approaches as in Reference Example 2-85 (1) to (4) and Reference Example 2-86 (3), the title compound (45 mg, light yellow amorphous) was obtained from ethyl 3-amino-3-phenylpropanoate hydrochloride (0.40 g), 3-chloropropane-1-sulfonyl chloride (0.37 g), and 1-(4-methoxyphenyl)-N-methylmethanamine (0.42 g).
¹H NMR (400 MHz, DMSO-d₆) δ ppm 1.47 - 1.64 (m, 2H), 2.14 (s, 3H), 2.24 - 2.31 (m, 2H), 2.64 - 2.88 (m, 4H), 3.56 (s, 3H), 4.63 - 4.74 (m, 1H), 7.32 - 7.42 (m, 5H), 7.92 (br s, 1H).

### Reference Example 2-112: Synthesis of methyl 2-[2-[tert-butyl-[2-(methylamino)ethyl]amino]ethoxy]-5-methyl-benzoate hydrochloride

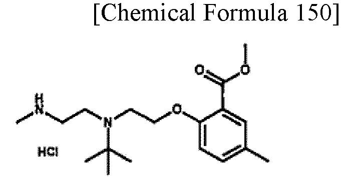

By using the same approaches as in Reference Example 2-73 (4) and Reference Example 2-25 (3), the title compound (60 mg, yellow oily matter) was obtained from methyl 5-methyl-2-(2-oxoethoxy)benzoate (0.12 g) obtained in Reference Example 2-78 (1) and tert-butyl N-[2-(tert-butylamino)ethyl]-N-methyl-carbamate (0.11 g).
¹H NMR (400 MHz, DMSO-d₆) δ ppm 1.39 - 1.51 (m, 9H), 2.22 - 2.36 (m, 4H), 2.60 - 2.70 (m, 3H), 2.70 - 3.02 (m, 1H), 3.58 - 3.84 (m, 5H), 3.87 - 4.05 (m, 2H), 4.31 - 4.42 (m, 1H), 4.46 - 4.61 (m, 1H), 7.03 - 7.18 (m, 1H), 7.32 - 7.49 (m, 1H), 7.49 - 7.60 (m, 1H), 9.10 (br s, 2H), 10.22 (br s, 1H).
LCMS (ESI) m/z 323 [M+H]⁺, t_{R} = 0.217 min, mode N.

### Reference Example 2-113: Synthesis of methyl 5-fluoro-2-(2-{methyl[2-(methylamino)ethyl]amino}-2-oxoethoxy)benzoate hydrochloride

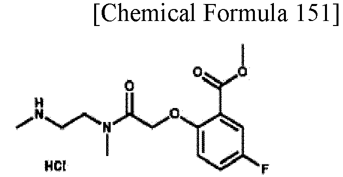

By using the same approaches as in Reference Example 2-1 (1) to (3), the title compound (1.2 g, light yellow oily matter) was obtained from tert-butyl N-methyl-N-[2-(methylamino)ethyl]carbamate (1.0 g) and methyl 5-fluoro-2-hydroxy-benzoate (0.94 g).
LCMS (ESI) m/z 299 [M+H]⁺, t_{R} = 0.628 min, mode H.

### Reference Example 2-114: Synthesis of ethyl 3-[3-(methylamino)propoxy]benzoate hydrochloride

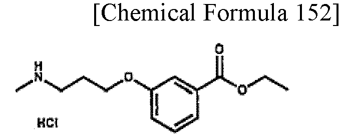

By using the same approaches as in Reference Example 2-25 (1) to (3), the title compound (0.56 g, colorless powder) was obtained from tert-butyl (3-hydroxypropyl)(methyl)carbamate (1.1 g) and ethyl 3-hydroxybenzoate (1.1 g).
¹H NMR (400 MHz, CDCl₃) δ ppm 1.39 (t, J = 7.1 Hz, 3H), 2.36 - 2.45 (m, 2H), 2.75 (s, 3H), 3.18 - 3.25 (m, 2H), 4.15 (t, J = 5.7 Hz, 2H), 4.37 (q, J = 7.1 Hz, 2H), 7.07 - 7.13 (m, 1H), 7.31 - 7.36 (m, 1H), 7.51 - 7.56 (m, 1H), 7.63 - 7.67 (m, 1H).
LCMS (ESI) m/z 238 [M+H]⁺, t_{R} = 0.777 min, mode H.

### Reference Example 2-115: Synthesis of methyl 5-fluoro-2-[3-(methylamino)propoxy]benzoate hydrochloride

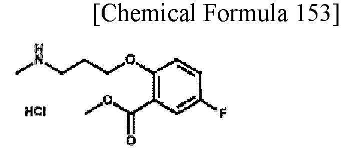

By using the same approaches as in Reference Example 2-25 (1) to (3), the title compound (0.39 g, colorless oily matter) was obtained from tert-butyl (3-hydroxypropyl)(methyl)carbamate (1.0 g) and methyl 3-hydroxybenzoate (0.45 g). ¹H NMR (400 MHz, CDCl₃) δ ppm 2.43 - 2.53 (m, 2H), 2.78 - 2.84 (m, 3H), 3.22 - 3.32 (m, 2H), 3.90 (s, 3H), 4.18 - 4.25 (m, 2H), 6.91 - 6.98 (m, 1H), 7.15 - 7.33 (m, 1H), 7.65 - 7.70 (m, 1H), 9.83 (br s, 2H).
LCMS (ESI) m/z 242 [M+H]⁺, t_{R} = 0.713 min, mode H.

### Reference Example 2-116: Synthesis of methyl 5-chloro-2-[3-(methylamino)propoxy]benzoate hydrochloride

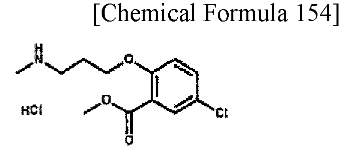

By using the same approaches as in Reference Example 2-25 (1) to (3), the title compound (1.1 g, colorless powder) was obtained from tert-butyl (3-hydroxypropyl)(methyl)carbamate (1.0 g) and methyl ethyl 3-hydroxybenzoate (1.1 g).
¹H NMR (400 MHz, DMSO-d₆) δ ppm 2.05 - 2.15 (m, 2H), 2.57 (s, 3H), 3.03 - 3.13 (m, 2H), 3.83 (s, 3H), 4.13 - 4.20 (m, 2H), 7.21 (d, J = 9.0 Hz, 1H), 7.63 (dd, J = 9.0, 2.8 Hz, 1H), 7.71 (d, J = 2.8 Hz, 1H), 8.83 (br s, 2H).
LCMS (ESI) m/z 258 [M+H]⁺, t_{R} = 0.446 min, mode N.

### Reference Example 2-117: Synthesis of methyl 2-[2-(2-aminoethoxy)ethoxy]-5-chlorobenzoate hydrochloride

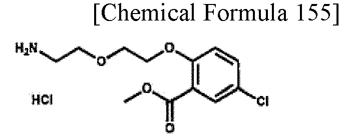

By using the same approaches as in Reference Example 2-25 (1) to (3), the title compound (1.9 g, light yellow powder) was obtained from tert-butyl N-[2-(2-hydroxyethoxy)ethyl]carbamate (1.5 g) and methyl 5-chloro-2-hydroxybenzoate (1.7 g).
¹H NMR (400 MHz, DMSO-d₆) 2.92 - 3.03 (m, 2H), 3.72 (t, J = 5.3 Hz, 2H), 3.76 - 3.85 (m, 5H), 4.16 - 4.25 (m, 2H), 7.23 (d, J = 9.0 Hz, 1H), 7.59 (dd, J = 9.0, 2.8 Hz, 1H), 7.66 (d, J = 2.8 Hz, 1H), 8.03 (br s, 3H).
LCMS (ESI) m/z 274 [M+H]⁺, t_{R} = 0.453 min, mode N.

### Reference Example 2-118: Synthesis of ethyl 1-{[4-(methylamino)butanamido]methyl}cyclohexane-1-carboxylate hydrochloride

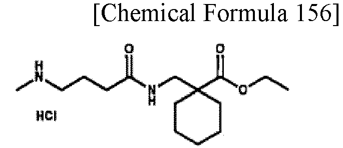

By using the same approaches as in Reference Example 2-8 (1) and (2), the title compound (0.41 g, yellow oily matter) was obtained from 4-[tert-butoxycarbonyl(methyl)amino]butanoic acid (0.30 g) and ethyl 1-(aminomethyl)cyclohexanecarboxylate (0.27 g).
¹H NMR (400 MHz, DMSO-d₆) δ ppm 1.14 - 1.22 (m, 6H), 1.45 - 1.60 (m, 3H), 1.72 - 1.83 (m, 2H), 1.88 - 1.98 (m, 2H), 2.14 - 2.23 (m, 2H), 2.47 - 2.55 (m, 3H), 2.79 - 2.88 (m, 2H), 3.14 - 3.21 (m, 2H), 3.99 - 4.08 (m, 2H), 7.86 - 7.94 (m, 1H), 8.59 - 8.73 (m, 2H).

### Reference Example 2-119: Synthesis of butyl 2-[4-(methylamino)butoxy]benzoate

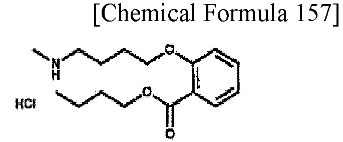

By using the same approaches as in Reference Example 2-25 (2) and (3), the title compound (0.46 g, colorless oily matter) was obtained from tert-butyl 4-[tert-butoxycarbonyl(methyl)amino]butyl methanesulfonate (0.46 g) obtained in Reference Example 2-25 (1) and butyl 2-hydroxybenzoate (0.64 g).
¹H NMR (400 MHz, CDCl₃) δ ppm 0.98 (t, J = 7.4 Hz, 3H), 1.47 (sxt, J = 7.4 Hz, 2H), 1.69 - 1.77 (m, 2H), 2.02 - 2.13 (m, 2H), 2.17 - 2.26 (m, 2H), 2.74 (t, J = 5.4 Hz, 3H), 3.13 (quin, J = 6.4 Hz, 2H), 4.12 - 4.20 (m, 2H), 4.26 (t, J = 6.6 Hz, 2H), 6.96 - 7.04 (m, 2H), 7.46 - 7.53 (m, 1H), 7.84 - 7.89 (m, 1H), 9.44 (br s, 2H).
LCMS (ESI) m/z 280 [M+H]⁺, t_{R} = 0.599 min, mode N.

### Reference Example 3-1: Synthesis of [4-iodo-2-(methoxycarbonyl)phenoxy]acetic acid

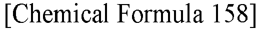

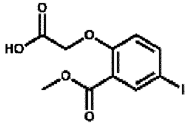

(1) To a solution of methyl 2-hydroxy-5-iodo-benzoate (5.7 g) in MeCN (40 mL), K₂CO₃ (5.7 g) and tert-butyl 2-bromoacetate (6.0 g) were added, and the mixture was stirred at 80°C for 2 hours. The reaction solution was poured into water and extracted with EtOAc. The organic layer was washed with Brine, dried over MgSO₄, filtered, and then the solvent was concentrated. The residue was purified by OH type silica gel column chromatography (mobile phase: hexane/EtOAc = 80/20 to 50/50; v/v) to afford methyl 2-(2-tert-butoxy-2-oxo-ethoxy)-5-iodo-benzoate (8.0 g, colorless oily matter).

(2) A solution of methyl 2-(2-tert-butoxy-2-oxo-ethoxy)-5-iodo-benzoate (8.0 g) in 4 M hydrochloric acid 1,4-dioxane (50 mL) was stirred at room temperature overnight. The reaction solution was concentrated. The residue was powderized with a mixed solution of hexane/EtOAc and filtered to afford the title compound (5.7 g, colorless powder).
¹H NMR (400 MHz, CDCl₃) δ ppm 3.96 (s, 3H), 4.73 (s, 2H), 6.77 (d, J = 8.8 Hz, 1H), 7.85 (d, J = 8.7 Hz, 1H), 8.24 (s, 1H).
LCMS (ESI) m/z 337 [M+H]⁺, t_{R} = 0.770 min, mode N.

### Reference Example 3-2: Synthesis of [4-fluoro-2-(methoxycarbonyl)phenoxy]acetic acid

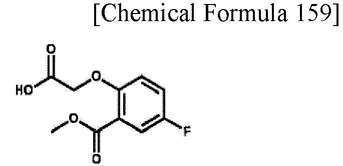

By using the same approaches as in Reference Example 3-1 (1) and (2), the title compound (4.8 g, colorless powder) was obtained from methyl 5-fluoro-2-hydroxy-benzoate (3.0 g).
¹H NMR (400 MHz, CDCl₃) δ ppm 3.97 (s, 3H), 4.74 (s, 2H), 6.99 (dd, J = 9.0, 4.2 Hz, 1H), 7.27 - 7.36 (m, 1H), 7.61 - 7.69 (m, 1H).
LCMS (ESI/APCI dual) m/z 229 [M+H]⁺, t_{R} = 0.623 min, mode N.

### Reference Example 3-3: Synthesis of [2-(methoxycarbonyl)-4-(trideuteriomethyl)methylphenoxy]acetic acid

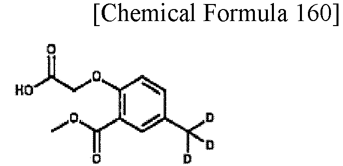

(1) To a suspension of zinc powder (2.3 g) in DMF (26 mL), TMSCl (83 mg) and CD₃I (5.2 g) were sequentially added at room temperature, and the mixture was stirred to prepare an organozinc reagent solution.

To a solution of the compound (1.0 g) obtained in Reference Example 3-1 (1) in DMF (13 mL), SUPERSTABLE Pd(0) CATALYST (R) (1.1 g) and the pre-prepared organozinc reagent solution were sequentially added at room temperature, and the mixture was stirred at 70°C for 30 minutes. The reaction liquid was concentrated and extracted by adding water and EtOAc/toluene (5/1; v/v). The obtained organic layer was washed with water and Brine, dried over MgSO₄, and concentrated. The obtained residue was purified by OH type silica gel column chromatography (mobile phase: hexane/EtOAc = 95/5 to 60/40; v/v) to afford methyl 2-(2-tert-butoxy-2-oxo-ethoxy)-5-(trideuteriomethyl)benzoate (0.64 g, colorless oily matter).

(2) By using the same approach as in Reference Example 3-1 (2), the title compound (0.47 g, colorless powder) was obtained from methyl 2-(2-tert-butoxy-2-oxo-ethoxy)-5-(trideuteriomethyl)benzoate (0.63 g).
¹H NMR (400 MHz, CDCl₃) δ ppm 3.95 (s, 3H), 4.74 (s, 2H), 6.90 (d, J = 8.4 Hz, 1H), 7.36 (d, J = 8.4 Hz, 1H), 7.74 (s, 1H).
LCMS (ESI) m/z 228 [M+H]⁺, t_{R} = 0.758 min, mode N.

### Reference Example 3-4: Synthesis of [4-(1,1-difluoroethyl)-2-(methoxycarbonyl)phenoxy]acetic acid

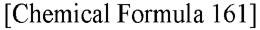

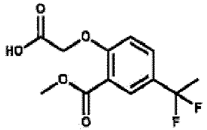

(1) By using the same approach as in Reference Example 3-1 (1), methyl 5-acetyl-2-(2-tert-butoxy-2-oxo-ethoxy)benzoate (4.0 g, colorless oily matter) was obtained from methyl 5-acetyl-2-hydroxybenzoate (2.0 g).

(2) To a solution of methyl 5-acetyl-2-(2-tert-butoxy-2-oxo-ethoxy)benzoate (0.50 g) in CHCl₃ (3.2 mL), DeoxoFluor (R) (1.5 mL) was added, and the mixture was stirred at 65°C for 24 hours. The reaction solution was poured into a Sat. NaHCO₃ aq. and extracted with CHCl₃. The organic layer was washed with Brine, dried over MgSO₄, filtered, and then the solvent was concentrated. The residue was purified by OH type silica gel column chromatography (mobile phase: hexane/EtOAc = 90/10 to 60/40; v/v) to afford methyl 2-(2-tert-butoxy-2-oxo-ethoxy)-5-(1,1-difluoroethyl)benzoate (0.21 g, colorless oily matter).

(3) By using the same approach as in Reference Example 3-1 (2), the title compound (0.15 g, colorless oily matter) was obtained from methyl 2-(2-tert-butoxy-2-oxo-ethoxy)-5-(1,1-difluoroethyl)benzoate (0.18 g).
LCMS (ESI) m/z 275 [M+H]⁺, t_{R} = 0.826 min, mode N.

### Reference Example 3-5: Synthesis of methyl 2-(2-hydroxyethoxy)-5-methylbenzoate

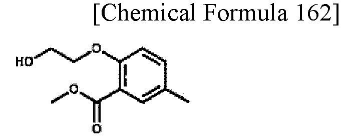

(1) By using the same approach as in Reference Example 3-1 (1), methyl 2-(2-benzyloxyethoxy)-5-methyl-benzoate (1.9 g, colorless oily matter) was obtained from methyl 2-hydroxy-5-methyl-benzoate (1.0 g) and 2-bromoethoxymethylbenzene (1.9 g).

(2) To a solution of methyl 2-(2-benzyloxyethoxy)-5-methyl-benzoate (1.0 g) in MeOH (44 mL), 10% palladium-carbon (0.50 g) was added, and the mixture was stirred for 19 hours in a hydrogen gas atmosphere. The reaction liquid was filtered through Celite (R), then concentrated under reduced pressure, and dried to afford the title compound (0.62 g, colorless oily matter).
¹H NMR (400 MHz, CDCl₃) δ ppm 2.32 (s, 3H), 3.70 - 3.82 (m, 1H), 3.86 - 3.92 (m, 5H), 4.17 - 4.23 (m, 2H), 6.92 (d, J = 8.4 Hz, 1H), 7.27 - 7.30 (m, 1H), 7.62 (d, J = 1.7 Hz, 1H). LCMS (ESI) m/z 211 [M+H]⁺, t_{R} = 0.799 min, mode N.

### Reference Example 3-6: Synthesis of methyl 2-(2-aminoethoxy)-5-fluorobenzoate

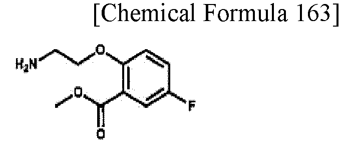

By using the same approaches as in Reference Example 3-1 (1) and Reference Example 3-5 (2), the title compound (1.1 g, colorless powder) was obtained from methyl 5-fluoro-2-hydroxy-benzoate (1.5 g) and benzyl N-(2-bromoethyl)carbamate (2.5 g). LCMS (ESI) m/z 214 [M+H]⁺, t_{R} = 0.229 min, mode N.

### Reference Example 3-7: Synthesis of benzyl {2-[(tert-butoxycarbonyl)(methyl)amino]ethyl}(2-hydroxyethyl)carbamate

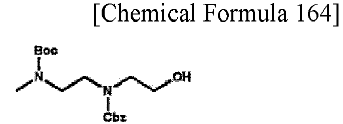

A solution of tert-butyl N-[2-(2-hydroxyethylamino)ethyl]-N-methyl-carbamate (1.8 g) in CHCl₃ (84 mL) was cooled to 0°C, then Et₃N (3.5 mL) and CbzCl (1.3 mL) were added, and the mixture was stirred at room temperature for 30 minutes. To the reaction solution, a Sat. NH₄Cl aq. and CHCl₃ were added, and the separating operation was carried out. The aqueous layer was separated using Phase Separator, and then the organic layer was concentrated under reduced pressure. The residue was purified by OH type silica gel column chromatography (mobile phase: hexane/EtOAc = 50/50 to 25/75; v/v) to afford the title compound (1.5 g, colorless oily matter).
¹H NMR (400 MHz, CDCl₃) δ ppm 1.43 (s, 9H), 2.66 - 2.95 (m, 3H), 3.28 - 3.53 (m, 6H), 3.68 - 3.85 (m, 2H), 5.07 - 5.17 (m, 2H), 7.28 - 7.41 (m, 5H).

### Reference Example 3-8: Synthesis of benzyl {2-[(tert-butoxycarbonyl)(ethyl)amino]ethyl}(2-hydroxyethyl)carbamate

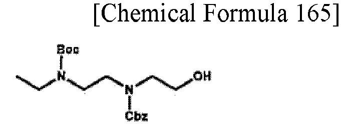

By using the same approach as in Reference Example 3-7, the title compound (0.70 g, colorless oily matter) was obtained from tert-butyl N-ethyl-N-[2-(2-hydroxyethylamino)ethyl]carbamate (0.85 g).
¹H NMR (400 MHz, CDCl₃) 0.97 - 1.14 (m, 3H), 1.43 (s, 9H), 3.21 - 3.81 (m, 11H), 5.13 (br s, 2H), 7.30 - 7.38 (m, 5H).
LCMS (ESI) m/z 389 [M+Na]⁺, t_{R} = 1.016 min, mode N.

### Reference Example 3-9: Synthesis of ethyl 3-hydroxy-2-methyl-2-(pyridin-3-yl)propanoate

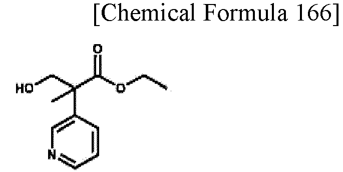

To a solution of diethyl 2-methyl-2-(3-pyridyl)propanedioate (1.7 g) in THF (34 mL), lithium tri-tert-butoxyaluminum hydride (34 mL, 1 M THF solution) was added at 0°C, and the mixture was stirred at room temperature for 10 minutes and stirred at 75°C for 1.5 hours. To the reaction solution, a saturated aqueous Rochelle salt solution was added dropwise under ice cooling, Et₂O and EtOAc were added thereto, and the mixture was stirred overnight while allowing it to be gradually cooled to room temperature. The separating operation was carried out. After washing with Brine, the filtrate was dried over MgSO₄ and concentrated under reduced pressure. Purification by OH type silica gel column chromatography (mobile phase: hexane/EtOAc = 60/40 to 0/100 to EtOAc/MeOH = 99/1 to 93/7; v/v) afforded the title compound (0.99 g, colorless oily matter).
LCMS (ESI) m/z 210 [M+H]⁺, t_{R} = 0.204 min, mode N.

### Example 1: Synthesis of (18aS)-13-[(3S)-3-aminopyrrolidin-1-yl]-2-fluoro-11-methyl-8,9,10,11,19,20,21,22-octahydr o-18aH,24H-18,15-(metheno)pyrido[2,1-l]pyrimido[6,1-h][1,4,7,9,10,13]benzoxapentaazacy clohexadecine-7,24(6H)-dione

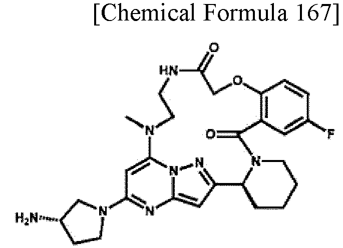

Step 1-1: To a solution of the compound (0.43 g) obtained in Reference Example 1-1 (3) and the compound (0.44 g) obtained in Reference Example 2-1 (3) in EtOH (12 mL), Et₃N (1.7 mL) was added, and the mixture was stirred at 70°C for 30 minutes. After cooling the reaction liquid to room temperature, the reaction solution was divided by adding a Sat. NaHCO₃ aq., and the aqueous layer was extracted with CHCl₃. The organic layer was filtered through Phase Separator, and the filtrate was concentrated under reduced pressure. The obtained residue was purified by NH type silica gel column chromatography (mobile phase: hexane/EtOAc = 30/70; v/v) to afford methyl 2-[2-[2-[[5-chloro-2-[(2S)-2-piperidyl]pyrazolo[1,5-a]pyrimidin-7-yl]-methyl-amino]ethylam ino]-2-oxo-ethoxy]-5-fluoro-benzoate (0.36 g, colorless amorphous).
LCMS (ESI) m/z 519 [M+H]⁺, t_{R} = 0.601 min, mode N.

Step 1-2: To a solution of the compound (0.36 g) obtained in Step 1-1 in THF (7.0 mL) and IPA (7.0 mL), a 1 M NaOH aq. (14 mL) was added, and the mixture was stirred at 80°C for 30 minutes. After cooling the reaction liquid to room temperature, it was divided by adding a 1 M HCl aq., and the aqueous layer was extracted with CHCl₃. The organic layer was filtered through Phase Separator, and then the filtrate was concentrated under reduced pressure to afford 2-[2-[2-[[5-chloro-2-[(2S)-2-piperidyl]pyrazolo[1,5-a]pyrimidin-7-yl]-methyl-amino]ethylam ino]-2-oxo-ethoxy]-5-fluoro-benzoic acid (0.40 g, colorless amorphous).
LCMS (ESI) m/z 505 [M+H]⁺, t_{R} = 0.521 min, mode N.

Step 1-3: To a solution of the compound (0.40 g) obtained in Step 1-2 in DMF (79 mL), HATU (0.45 g) and Et₃N (0.55 mL) were added, and the mixture was stirred at room temperature for 15 hours. The reaction solution was divided by adding a Sat. NaHCO₃ aq., and the aqueous layer was extracted with EtOAc/toluene (1/1, v/v). The organic layer was washed with Brine, dried over MgSO₄, filtered, and then the filtrate was concentrated under reduced pressure. The obtained residue was purified by OH type silica gel column chromatography (mobile phase: hexane/EtOAc = 15/85 → CHCl₃/MeOH = 93/7; v/v) to afford (18aS)-13-chloro-2-fluoro-11-methyl-8,9,10,11,19,20,21,22-octahydro-18aH,24H-18,15-(me theno)pyrido[2,1-l]pyrimido[6,1-h][1,4,7,9,10,13]benzoxapentaazacyclohexadecine-7,24(6H) -dione (0.25 g, yellow amorphous).
LCMS (ESI) m/z 487 [M+H]⁺, t_{R} = 0.915 min, mode N.

Step 1-4: To a solution of the compound (0.15 g) obtained in Step 1-3 in NMP (1.5 mL), Et₃N (0.43 mL) and (3S)-pyrrolidin-3-amine (0.14 mL) were added, and the mixture was stirred at 150°C for 30 minutes under microwave irradiation. The obtained residue was purified by reversed phase preparative HPLC to afford the title compound (82 mg, colorless powder).
¹H NMR (400 MHz, CDCl₃) δ ppm 1.16 - 2.41 (m, 9H), 2.76 - 3.82 (m, 12H), 4.04 - 4.96 (m, 6H), 5.29 (s, 0.7H), 5.36 (s, 0.3H), 5.87 (s, 0.7H), 6.17 (s, 0.3H), 6.82 - 6.90 (m, 1H), 6.98 - 7.07 (m, 2H), 7.81 (d, J = 6.8 Hz, 0.3H), 9.16 (d, J = 6.8 Hz, 0.7H).
LCMS (ESI) m/z 537 [M+H]⁺, t_{R} = 0.233 min, mode N.

### Example 2: Synthesis of (18aS)-13-(azetidin-1-yl)-2-fluoro-11-methyl-8,9,10,11,19,20,21,22-octahydro-18aH,24H-18 ,15-(metheno)pyrido[2,1-l]pyrimido[6,1-h][1,4,7,9,10,13]benzoxapentaazacyclohexadecine-7 ,24(6H)-dione

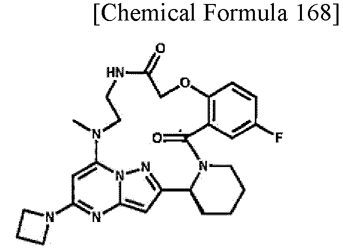

By using the same approach as in Step 1-4, the title compound (53 mg, colorless powder) was obtained from the compound (0.10 g) obtained in Step 1-3 and azetidine (0.14 mL).
¹H NMR (400 MHz, CDCl₃) δ ppm 1.59 - 2.54 (m, 8H), 2.77 - 3.37 (m, 6H), 4.01 - 4.96 (m, 10H), 5.10 (s, 0.7H), 5.17 (s, 0.3H), 5.88 (s, 0.7H), 6.18 (s, 0.3H), 6.82 - 6.91 (m, 1H), 6.98 - 7.07 (m, 2H), 7.79 (d, J = 7.5 Hz, 0.3H), 9.12 (d, J =7.5 Hz, 0.7H).
LCMS (ESI) m/z 508 [M+H]⁺, t_{R} = 0.517 min, mode N.

### Example 3: Synthesis of (18aS)-2-fluoro-13-(3-hydroxyazetidin-1-yl)-11-methyl-8,9,10,11,19,20,21,22-octahydro-18a H,24H-18,15-(metheno)pyrido[2,1-1]pyrimido[6,1-h][1,4,7,9,10,13]benzoxapentaazacyclohe xadecine-7,24(6H)-dione

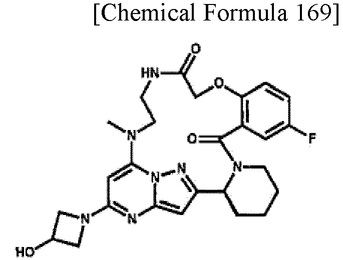

Step 3-1: To a solution of the compound (23 mg) obtained in Step 1-3 in IPA (0.47 mL), a 1 M NaOH aq. (0.47 mL) and 3-hydroxyazetidine hydrochloride (52 mg) were added, and the mixture was stirred at 130° C for 1 hour under microwave irradiation. Furthermore, 3-hydroxyazetidine hydrochloride (52 mg) was added thereto, and the mixture was stirred at 130°C for 30 minutes under microwave irradiation. After cooling the reaction liquid to room temperature, the aqueous layer was extracted with CHCl₃. The organic layer was filtered through Phase Separator, and the filtrate was concentrated under reduced pressure. The obtained residue was purified by NH type silica gel column chromatography (mobile phase: CHCl₃/MeOH = 100/0 to 94/6; v/v) to afford the title compound (11 mg, colorless powder).
LCMS (ESI) m/z 524 [M+H]⁺, t_{R} = 0.447 min, mode N.

### Example 4: Synthesis of (18aS)-13-(3-aminoazetidin-1-yl)-2-fluoro-11-methyl-8,9, 10,11, 19,20,21,22-octahydro-18aH ,24H-18,15-(metheno)pyrido[2,1-1]pyrimido[6,1-h][1,4,7,9,10, 13]benzoxapentaazacyclohexa decine-7,24(6H)-dione

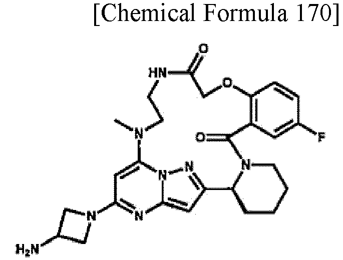

Step 4-1: By using the same approach as in Step 1-4, benzyl {1-[(18aS)-2-fluoro-11-methyl-7,24-dioxo-6,7,8,9,10,11,19,20,21,22-decahydro-18aH,24H-1 8,15-(metheno)pyrido[2,1-l]pyrimido[6,1-h][1,4,7,9,10,13]benzoxapentaazacyclohexadecin-1 3-yl]azetidin-3-yl}carbamate (35 mg, colorless oily matter) was obtained from the compound (31 mg) obtained in Step 1-3 and benzyl N-(azetidin-3-yl)carbamate (0.20 g).
LCMS (ESI) m/z 657 [M+H]⁺, t_{R} = 0.719 min, mode N.

Step 4-2: To a solution of the compound (35 mg) obtained in Step 4-1 in MeOH (0.27 mL), 10% palladium-carbon (35 mg) was added, and the mixture was stirred for 16.5 hours under a hydrogen gas atmosphere. The reaction solution was filtered through Celite (R) and washed with MeOH, and the resulting filtrate was concentrated under reduced pressure to afford the title compound (16 mg, colorless powder).
LCMS (ESI) m/z 523 [M+H]⁺, t_{R} = 0.731 min, mode H.

### Example 5: Synthesis of (18aS)-2-fluoro-11-methyl-13-[3-(methylamino)azetidin-1-yl]-8,9,10,11,19,20,21,22-octahyd ro-18aH,24H-18,15-(metheno)pyrido[2,1-l]pyrimido[6,1-h][1,4,7,9,10,13]benzoxapentaazac yclohexadecine-7,24(6H)-dione

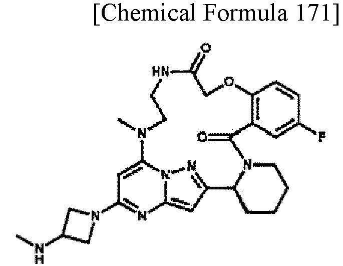

Step 5-1: By using the same approach as in Step 3-1, tert-butyl {1-[(18aS)-2-fluoro-11-methyl-7,24-dioxo-6,7,8,9,10,11,19,20,21,22-decahydro-18aH,24H-1 8,15-(metheno)pyrido[2,1-l]pyrimido[6,1-h][1,4,7,9,10,13]benzoxapentaazacyclohexadecin-1 3-yl]azetidin-3-yl}methylcarbamate (44 mg, colorless oily matter) was obtained from the compound (29 mg) obtained in Step 1-3 and tert-butyl N-(azetidin-3-yl)-N-methyl-carbamate hydrochloride (0.13 g).
LCMS (ESI) m/z 637 [M+H]⁺, t_{R} = 0.770 min, mode N.

Step 5-2: To a solution of the compound (44 mg) obtained in Step 5-1 in CHCl₃ (1.0 mL), TFA (0.16 mL) was added, and the mixture was stirred at room temperature for 5 hours. The reaction solution was divided by adding a Sat. NaHCO₃ aq., and the aqueous layer was extracted with CHCl₃. The organic layer was filtered through Phase Separator, and the filtrate was concentrated under reduced pressure. The obtained residue was purified by reversed phase preparative HPLC to afford the title compound (9.3 mg, colorless powder).
LCMS (ESI) m/z 537 [M+H]⁺, t_{R} = 0.746 min, mode H.

### Example 6: Synthesis of (18aS)-13-[3-(dimethylamino)azetidin-1-yl]-2-fluoro-11-methyl-8,9,10,11,19,20,21,22-octah ydro-18aH,24H-18,15-(metheno)pyrido[2,1-1]pyrimido[6,1-h][1,4,7,9,10,13]benzoxapentaaz acyclohexadecine-7,24(6H)-dione

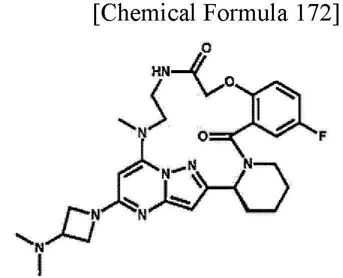

By using the same approach as in Step 3-1, the title compound (3.4 mg, colorless powder) was obtained from the compound (30 mg) obtained in Step 1-3 and N,N-dimethylazetidin-3-amine hydrochloride (84 mg).
LCMS (ESI) m/z 551 [M+H]⁺, t_{R} = 0.765 min, mode H.

### Example 7: Synthesis of (18aS)-13-(azetidin-1-yl)-2-fluoro-8,11-dimethyl-8,9,10,11,19,20,21,22-octahydro-18aH,24 H-18,15-(metheno)pyrido[2,1-1]pyrimido[6,1-h][1,4,7,9,10, 13]benzoxapentaazacyclohexadec ine-7,24(6H)-dione

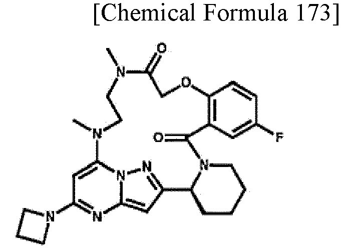

Step 7-1: To a solution of the compound (37 mg) obtained in Step 1-3 in DMF (0.76 mL), NaH (4.6 mg, 60% in oil) was added under ice cooling, and the mixture was stirred for 30 minutes as it was. Then, MeI (16 mg) was added thereto, and the mixture was stirred at room temperature for 2 hours. The reaction solution was divided by adding water, and the aqueous layer was extracted with EtOAc. The organic layer was washed with Brine, dried over MgSO₄, filtered, and then the filtrate was concentrated under reduced pressure. The obtained residue was purified by NH type silica gel column chromatography (mobile phase: hexane/EtOAc = 50/50 to 0/100; v/v) to afford (18aS)-13-chloro-2-fluoro-8,11-dimethyl-8,9,10,11,19,20,21,22-octahydro-18aH,24H-18,15-(metheno)pyrido[2,1-1]pyrimido[6,1-h][1,4,7,9,10,13]benzoxapentaazacyclohexadecine-7,24( 6H)-dione (38 mg, yellow oily matter).
LCMS (ESI) m/z 501 [M+H]⁺, t_{R} = 0.937 min, mode N.

Step 7-2: By using the same approach as in Step 1-4, the title compound (11 mg, colorless amorphous) was obtained from the compound (30 mg) obtained in Step 7-1 and azetidine (40 µL).
LCMS (ESI) m/z 522 [M+H]⁺, t_{R} = 0.553 min, mode N.

### Example 8: Synthesis of (18aS)-13-(3-aminoazetidin-1-yl)-2-fluoro-8,11-dimethyl-8,9,10,11,19,20,21,22-octahydro-1 8aH,24H-18,15-(metheno)pyrido[2,1-1]pyrimido[6,1-h][1,4,7,9,10, 13]benzoxapentaazacyclo hexadecine-7,24(6H)-dione

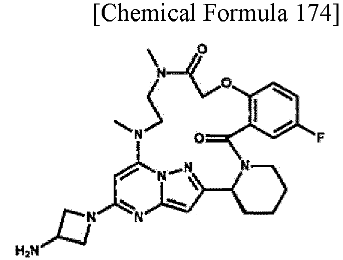

By using the same approaches as in Step 1-4 and Step 5-2, the title compound (10 mg, colorless amorphous) was obtained from the compound (25 mg) obtained in Step 7-1 and 3-N-BOC-amino-azetidine (83 mg).
LCMS (ESI) m/z 537 [M+H]⁺, t_{R} = 0.754 min, mode H.

### Example 9: Synthesis of (18aS)-13-(3-aminoazetidin-1-yl)-8-ethyl-2-fluoro-11-methyl-8,9,10,11,19,20,21,22-octahyd ro-18aH,24H-18,15-(metheno)pyrido[2,1-1]pyrimido[6,1-h][1,4,7,9,10,13]benzoxapentaazac yclohexadecine-7,24(6H)-dione

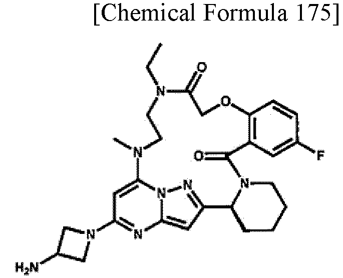

By using the same approaches as in Step 7-1, Step 1-4, and Step 5-2, the title compound (11 mg, colorless powder) was obtained from the compound (57 mg) obtained in Step 1-3, EtI (47 µL), and 3-N-BOC-amino-azetidine (39 mg).
LCMS (ESI) m/z 551 [M+H]⁺, t_{R} = 0.809 min, mode H.

### Example 10: Synthesis of (18aS)-13-(3-aminoazetidin-1-yl)-2-fluoro-6,6,11-trimethyl-8,9,10,11,19,20,21,22-octahydro -18aH,24H-18,15-(metheno)pyrido[2,1-1]pyrimido[6,1-h][1,4,7,9,10, 13]benzoxapentaazacycl ohexadecine-7,24(6H)-dione

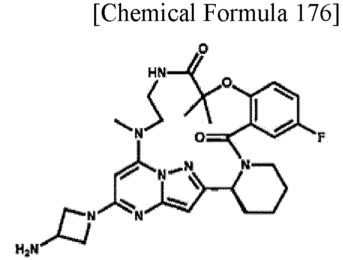

By using the same approaches as in Step 1-1 to Step 1-4 and Step 5-2, the title compound (97 mg, colorless powder) was obtained from the compound (0.30 g) obtained in Reference Example 1-1 (3), the compound (0.34 g) obtained in Reference Example 2-8, and 3-N-BOC-amino-azetidine (0.20 g).
LCMS (ESI) m/z 551 [M+H]⁺, t_{R} = 0.763 min, mode H.

### Example 11: Synthesis of (18aS)-13-(3-aminoazetidin-1-yl)-2-fluoro-9,9,11-trimethyl-8,9,10,11,19,20,21,22-octahydro -18aH,24H-18,15-(metheno)pyrido[2,1-1]pyrimido[6,1-h][1,4,7,9,10,13]benzoxapentaazacycl ohexadecine-7,24(6H)-dione

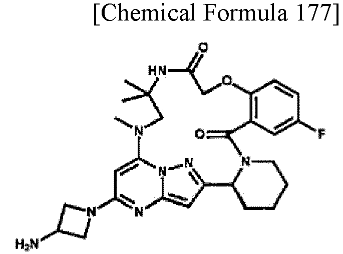

By using the same approaches as in Step 1-1 to Step 1-4 and Step 5-2, the title compound (10 mg, colorless amorphous) was obtained from the compound (0.13 g) obtained in Reference Example 1-1 (3), the compound (0.11 g) obtained in Reference Example 2-9, and 3-N-BOC-amino-azetidine (40 mg).
LCMS (ESI) m/z 551 [M+H]⁺, t_{R} = 0.819 min, mode H.

### Example 12: Synthesis of (18aS)-13-(3-aminoazetidin-1-yl)-2-fluoro-6,11-dimethyl-8,9,10,11,19,20,21,22-octahydro-1 8aH,24H-18,15-(metheno)pyrido[2,1-1]pyrimido[6,1-h][1,4,7,9,10, 13]benzoxapentaazacyclo hexadecine-7,24(6H)-dione

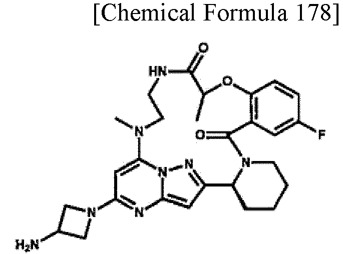

By using the same approaches as in Step 1-1 to Step 1-4 and Step 5-2, the title compound (55 mg, colorless amorphous) was obtained from the compound (0.10 g) obtained in Reference Example 1-1 (3), the compound (0.11 g) obtained in Reference Example 2-10, and 3-N-BOC-amino-azetidine (96 mg).
LCMS (ESI) m/z 537 [M+H]⁺, t_{R} = 0.764 min, mode H.

### Example 13: Synthesis of (18aS)-13-(3-aminoazetidin-1-yl)-2-fluoro-11-(propan-2-yl)-8,9,10,11,19,20,21,22-octahydro -18aH,24H-18, 15-(metheno)pyrido[2,1-1]pyrimido[6,1-h][1,4,7,9,10, 13]benzoxapentaazacycl ohexadecine-7,24(6H)-dione

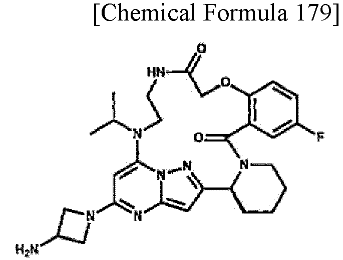

Step 13-1: By using the same approaches as in Step 1-1 and Step 1-2, 2-[2-({2-[{2-[(2S)-1-(tert-butoxycarbonyl)piperidin-2-yl]-5-chloropyrazolo[1,5-a]pyrimidin-7-yl}(propan-2-yl)amino]ethyl}amino)-2-oxoethoxy]-5-fluorobenzoic acid (0.13 g, colorless oily matter) was obtained from the compound (78 mg) obtained in Reference Example 1-2 and the compound (72 mg) obtained in Reference Example 2-4.
LCMS (ESI) m/z 633 [M+H]⁺, t_{R} = 1.303 min, mode N.

Step 13-2: To the compound (0.13 g) obtained in Step 13-1, a 4 M hydrochloric acid 1,4-dioxane solution (1.0 mL) was added, and the mixture was stirred at room temperature for 1 hour. The reaction liquid was concentrated under reduced pressure, and then dried under reduced pressure to afford 2-[2-({2-[{5-chloro-2-[(2S)-piperidin-2-yl]pyrazolo[1,5-a]pyrimidin-7-yl}(propan-2-yl)amin o]ethyl}amino)-2-oxoethoxy]-5-fluorobenzoic acid hydrochloride (0.14 g, colorless powder). LCMS (ESI) m/z 533 [M+H]⁺, t_{R} = 0.622 min, mode N.

Step 13-3: By using the same approaches as in Step 1-3, Step 1-4, and Step 5-2, the title compound (18 mg, colorless amorphous) was obtained from the compound (0.13 g) obtained in Step 13-2 and 3-N-BOC-amino-azetidine (50 mg).
LCMS (ESI) m/z 551 [M+H]⁺, t_{R} = 0.508 min, mode N.

### Example 14: Synthesis of (18aS)-13-(3-aminoazetidin-1-yl)-11-cyclopropyl-2-fluoro-8,9,10,11,19,20,21,22-octahydro-18aH,24H-18,15-(metheno)pyrido[2,1-l]pyrimido[6,1-h][1,4,7,9,10,13]benzoxapentaazacycl ohexadecine-7,24(6H)-dione

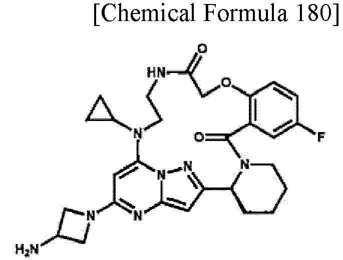

By using the same approaches as in Step 1-1, Step 1-2, Step 13-2, Step 1-3, Step 1-4, and Step 5-2, the title compound (20 mg, colorless amorphous) was obtained from the compound (78 mg) obtained in Reference Example 1-2, the compound (72 mg) obtained in Reference Example 2-5, and 3-N-BOC-amino-azetidine (54 mg).
LCMS (ESI) m/z 549 [M+H]⁺, t_{R} = 0.818 min, mode H.

### Example 15: Synthesis of (18aS)-13-(3-aminoazetidin-1-yl)-2-fluoro-11-methyl-9-(trifluoromethyl)-8,9,10,11,19,20,21, 22-octahydro-18aH,24H-18,15-(metheno)pyrido[2,1-1]pyrimido[6,1-h][1,4,7,9,10,13]benzox apentaazacyclohexadecine-7,24(6H)-dione

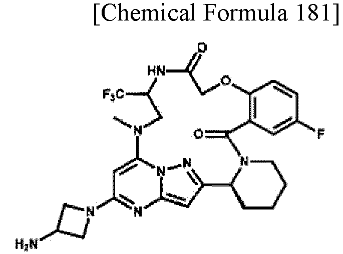

Step 15-1: By using the same approaches as in Step 1-1 and Step 1-4, tert-butyl 2-[2-({3-[{5-(3-{[(benzyloxy)carbonyl]amino}azetidin-1-yl)-2-[(2S)-piperidin-2-yl]pyrazolo [1,5-a]pyrimidin-7-yl}(methyl)amino]-1,1,1-trifluoropropan-2-yl}amino)-2-oxoethoxy]-5-flu orobenzoate (0.14 g, brown oily matter) was obtained from the compound (0.12 g) obtained in Reference Example 1-1 (3), the compound (0.17 g) obtained in Reference Example 2-11, and benzyl N-(azetidin-3-yl)carbamate (1.2 g).
LCMS (ESI) m/z 799 [M+H]⁺, t_{R} = 0.849 min, mode N.

Step 15-2: To a solution of the compound (0.12 g) obtained in Step 15-1 in CHCl₃ (1.6 mL), TFA (1.6 mL) was added, and the mixture was stirred at room temperature for 3 hours. The reaction solution was concentrated under reduced pressure to afford 2-[2-({3-[{5-(3-{[(benzyloxy)carbonyl]amino}azetidin-1-yl)-2-[(2S)-piperidin-2-yl]pyrazolo [1,5-a]pyrimidin-7-yl}(methyl)amino]-1,1,1-trifluoropropan-2-yl}amino)-2-oxoethoxy]-5-flu orobenzoic acid trifluoroacetate salt (0.14 g, brown oily matter).
LCMS (ESI) m/z 743 [M+H]⁺, t_{R} = 0.648 min, mode N.

Step 15-3: By using the same approach as in Step 1-3, Diastereomer A (32 mg, colorless powder) and Diastereomer B (15 mg, colorless powder) of benzyl {1-[(18aS)-2-fluoro-11-methyl-7,24-dioxo-9-(trifluoromethyl)-6,7,8,9,10,11,19,20,21,22-dec ahydro-18aH,24H-18,15-(metheno)pyrido[2,1-l]pyrimido[6,1-h][1,4,7,9,10,13]benzoxapenta azacyclohexadecin-13-yl]azetidin-3-yl}carbamate were obtained from the compound (0.15 g) obtained in Step 15-2.
Diastereomer A: LCMS (ESI) m/z 725 [M+H]⁺, t_{R} = 0.920, 0.944 min, mode N.
Diastereomer B: LCMS (ESI) m/z 725 [M+H]⁺, t_{R} = 0.946, 0.996 min, mode N.

Step 15-4: By using the same approach as in Step 4-2, the title compound (6.0 mg, colorless powder) was obtained from Diastereomer B (15 mg) obtained in Step 15-3. LCMS (ESI) m/z 591 [M+H]⁺, t_{R} = 0.526, 0.582 min, mode N.

### Example 16: Synthesis of (18aS)-13-(3-aminoazetidin-1-yl)-2-fluoro-11-methyl-9-(trifluoromethyl)-8,9,10,11,19,20,21, 22-octahydro-18aH,24H-18,15-(metheno)pyrido[2,1-1]pyrimido[6,1-h][1,4,7,9,10,13]benzox apentaazacyclohexadecine-7,24(6H)-dione

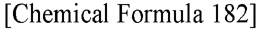

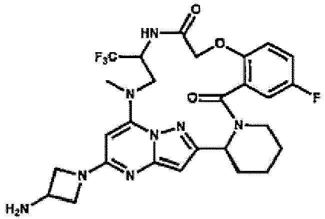

By using the same approach as in Step 4-2, the title compound (20 mg, colorless powder) was obtained from Diastereomer A (32 mg) obtained in Step 15-3.
LCMS (ESI) m/z 591 [M+H]⁺, t_{R} = 0.523 min, mode N.

### Example 17: Synthesis of (18aS)-13-(cyclopropylamino)-2-fluoro-11-methyl-8,9,10,11,19,20,21,22-octahydro-18aH,24 H-18,15-(metheno)pyrido[2,1-1]pyrimido[6,1-h][1,4,7,9,10, 13]benzoxapentaazacyclohexadec ine-7,24(6H)-dione

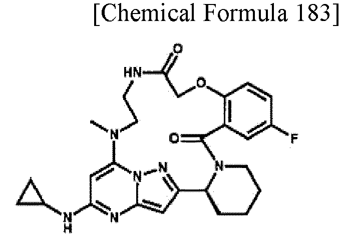

By using the same approach as in Step 1-4, the title compound (21 mg, colorless amorphous) was obtained from the compound (53 mg) obtained in Step 1-3 and cyclopropylamine (76 µL).
LCMS (ESI) m/z 508 [M+H]⁺, t_{R} = 0.517 min, mode N.

### Example 18: Synthesis of (18aS)-13-{[(1-aminocyclopropyl)methyl]amino}-2-fluoro-11-methyl-8,9,10,11,19,20,21,22-octahydro-18aH,24H-18,15-(metheno)pyrido[2,1-1]pyrimido[6,1-h][1,4,7,9,10,13]benzoxape ntaazacyclohexadecine-7,24(6H)-dione

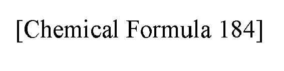

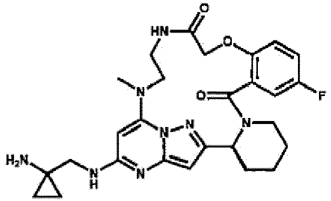

By using the same approaches as in Step 1-4 and Step 5-2, the title compound (32 mg, colorless powder) was obtained from the compound (45 mg) obtained in Step 1-3 and tert-butyl N-[1-(aminomethyl)cyclopropyl]carbamate (0.17 g).
LCMS (ESI) m/z 537 [M+H]⁺, t_{R} = 0.802 min, mode H.

### Example 19: Synthesis of (18aS)-2-fluoro-11-methyl-13-[(oxetan-3-yl)amino]-8,9,10,11,19,20,21,22-octahydro-18aH,2 4H-18, 15-(metheno)pyrido[2,1-1]pyrimido[6,1-h][1,4,7,9,10, 13]benzoxapentaazacyclohexade cine-7,24(6H)-dione

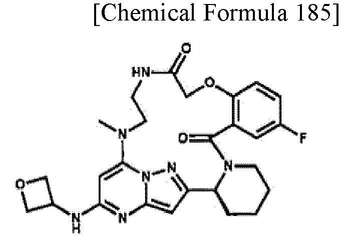

By using the same approach as in Step 1-4, the title compound (9.0 mg, colorless powder) was obtained from the compound (48 mg) obtained in Step 1-3 and oxetan-3-amine (72 mg).
LCMS (ESI) m/z 524 [M+H]⁺, t_{R} = 0.611 min, mode N.

### Example 20: Synthesis of (18aS)-2-fluoro-13-methoxy-11-methyl-8,9,10,11,19,20,21,22-octahydro-18aH,24H-18,15-( metheno)pyrido[2,1-1]pyrimido[6,1-h][1,4,7,9,10, 13]benzoxapentaazacyclohexadecine-7,24( 6H)-dione

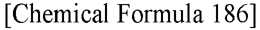

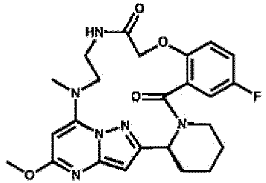

To a solution of the compound (45 mg) obtained in Step 1-3 in MeOH (0.93 mL), a 28% solution of NaOMe in MeOH (0.23 mL) was added, and the mixture was stirred at 70°C for 2.5 hours. The reaction solution was divided by adding water, and the aqueous layer was extracted with CHCl₃. The organic layer was filtered through Phase Separator, and the filtrate was concentrated under reduced pressure. The obtained residue was purified by NH type silica gel column chromatography (mobile phase: hexane/EtOAc = 50/50 to 0/100; v/v) to afford the title compound (29 mg, colorless amorphous).
LCMS (ESI) m/z 483 [M+H]⁺, t_{R} = 0.936 min, mode N.

### Example 21: Synthesis of (18aS)-13-(azetidin-3-yl)-2-fluoro-11-methyl-8,9,10,11,19,20,21,22-octahydro-18aH,24H-18 ,15-(metheno)pyrido[2,1-l]pyrimido[6,1-h][1,4,7,9,10, 13]benzoxapentaazacyclohexadecine-7 ,24(6H)-dione

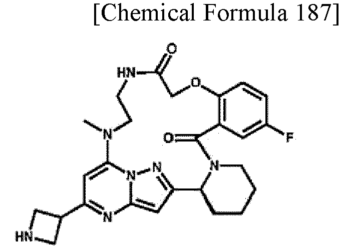

Step 21-1: To a DMA solution (2.0 mL) of the compound (86 mg) obtained in Step 1-3, CuI (6.7 mg) and PdCl₂(dppf)·CH₂Cl₂ (78 mg) were added, and the mixture was heated to 85°C. To this, a prepared suspension of a zinc reagent (a DMA solution (2.0 mL) of 1-BOC-3-iodoazetidine (0.25 g) was heated to 65°C. To this, zinc powder (59 mg) was added, and the mixture was stirred at 65°C for 20 minutes) was added dropwise, and then the mixture was stirred at 85°C for 30 minutes. The reaction solution was divided by adding a Sat. NH₄Cl aq., and the aqueous layer was extracted with EtOAc. The organic layer was filtered through Phase Separator, and the filtrate was concentrated under reduced pressure. The obtained residue was purified by NH type silica gel column chromatography (mobile phase: hexane/EtOAc = 50/50 to 0/100; v/v) to afford

(18aS)-13-(azetidin-3-yl)-2-fluoro-11-methyl-8,9,10,11,19,20,21,22-octahydro-18aH,24H-18 ,15-(metheno)pyrido[2,1-l]pyrimido[6,1-h][1,4,7,9,10, 13]benzoxapentaazacyclohexadecine-7 ,24(6H)-dione (79 mg, light brown amorphous).
LCMS (ESI) m/z 608 [M+H]⁺, t_{R} = 0.948 min, mode N.

Step 21-2: By using the same approach as in Step 5-2, the title compound (27 mg, colorless powder) was obtained from the compound (78 mg) obtained in Step 21-1.
LCMS (ESI) m/z 508 [M+H]⁺, t_{R} = 0.799 min, mode H.

### Example 22: Synthesis of (18aS)-2-fluoro-11-methyl-13-(1-methylazetidin-3-yl)-8,9,10,11,19,20,21,22-octahydro-18a H,24H-18,15-(metheno)pyrido[2,1-1]pyrimido[6,1-h][1,4,7,9,10,13]benzoxapentaazacyclohe xadecine-7,24(6H)-dione

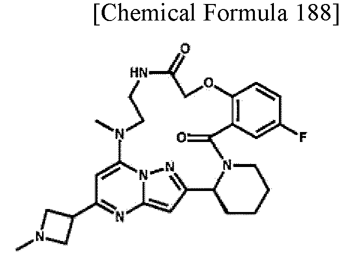

The compound (14 mg) obtained in Step 21-2 was dissolved in CHCl₃ (2.0 mL), formaldehyde (8.0 µL, 37% aqueous solution) and NaBH(OAc)₃ (11 mg) were added thereto under ice cooling, and the mixture was stirred at room temperature for 1 hour. The reaction solution was divided by adding a Sat. NaHCO₃ aq., and the aqueous layer was extracted with CHCl₃. The organic layer was filtered through Phase Separator, and the filtrate was concentrated under reduced pressure. The obtained residue was purified by NH type silica gel column chromatography (mobile phase: hexane/EtOAc = 50/50 to 0/100 to CHCl₃/MeOH = 97/3; v/v) to afford the title compound (11 mg, colorless powder).
LCMS (ESI) m/z 522 [M+H]⁺, t_{R} = 0.830 min, mode H.

### Example 23: Synthesis of (18aS)-13-cyclobutyl-2-fluoro-11-methyl-8,9,10,11,19,20,21,22-octahydro-18aH,24H-18,15-(metheno)pyrido[2,1-l]pyrimido[6,1-h][1,4,7,9,10,13]benzoxapentaazacyclohexadecine-7,24( 6H)-dione

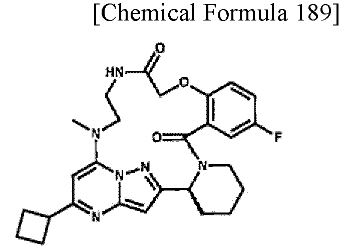

Step 23-1: To a solution of the compound (52 mg) obtained in Reference Example 1-11 and the compound (52 mg) obtained in Reference Example 2-1 (3) in NMP (0.45 mL), Et₃N (0.19 mL) was added, and the mixture was stirred at 150°C for 1 hour under microwave irradiation. The reaction solution was divided by adding a Sat. NaHCO₃ aq., and the aqueous layer was extracted with EtOAc. After drying over MgSO₄ and filtration, the filtrate was concentrated under reduced pressure. The obtained residue was purified by OH type silica gel column chromatography (mobile phase: hexane/EtOAc = 40/60 to 0/100 → EtOAc/MeOH = 98/2 to 90/10; v/v) to afford tert-butyl

(2S)-2-[5-cyclobutyl-7-[2-[[2-(4-fluoro-2-methoxycarbonyl-phenoxy)acetyl]amino]ethyl-met hyl-amino]pyrazolo[1,5-a]pyrimidin-2-yl]piperidine-1-carboxylate (43 mg, colorless amorphous).
LCMS (ESI) m/z 639 [M+H]⁺, t_{R} = 0.682 min, mode L.

Step 23-2: By using the same approaches as in Step 1-2, Step 13-2, and Step 1-3, the title compound (12 mg, yellow amorphous) was obtained from the compound (43 mg) obtained in Step 23-1.
LCMS (ESI) m/z 507 [M+H]⁺, t_{R} = 0.681 min, mode N.

### Example 24: Synthesis of (18aS)-2-fluoro-11-methyl-7,24-dioxo-6,7,8,9,10,11,19,20,21,22-decahydro-18aH,24H-18,15 -(metheno)pyrido[2,1-l]pyrimido[6,1-h][1,4,7,9,10,13]benzoxapentaazacyclohexadecine-13-c arbonitrile

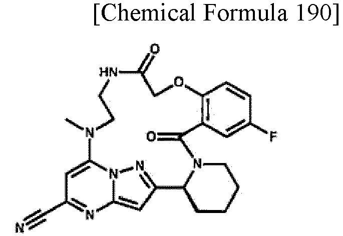

Step 24: To a solution of the compound (90 mg) obtained in Step 1-3 in DMF (1.8 mL), Pd(PPh₃)₄ (64 mg) and zinc cyanide (33 mg) were added, and the mixture was stirred at 110°C for 2 hours. The reaction solution was poured into Brine and extracted with CHCl₃. The organic layer was concentrated, and the resulting residue was purified by NH type silica gel column chromatography (mobile phase: EtOAc/MeOH = 100/0 to 90/10; v/v) to afford the title compound (46 mg, colorless amorphous).
LCMS (ESI/APCI dual) m/z 478 [M+H]⁺, t_{R} = 0.803 min, mode N.

### Example 25: Synthesis of (18aS)-13-(aminomethyl)-2-fluoro-11-methyl-8,9,10,11,19,20,21,22-octahydro-18aH,24H-18 ,15-(metheno)pyrido[2,1-l]pyrimido[6,1-h][1,4,7,9,10, 13]benzoxapentaazacyclohexadecine-7 ,24(6H)-dione

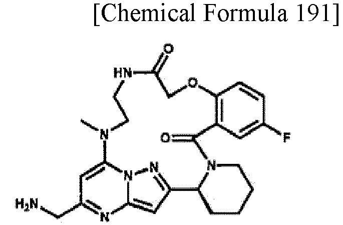

By using the same approach as in Step 4-2, the title compound (9.8 mg, yellow powder) was obtained from the compound (30 mg) obtained in Step 24.
LCMS (ESI) m/z 482 [M+H]⁺, t_{R} = 0.781 min, mode H.

### Example 26: Synthesis of (18aS)-13-(1-aminocyclopropyl)-2-fluoro-11-methyl-8,9,10,11,19,20,21,22-octahydro-18aH, 24H-18,15-(metheno)pyrido[2,1-1]pyrimido[6,1-h][1,4,7,9,10,13]benzoxapentaazacyclohexa decine-7,24(6H)-dione

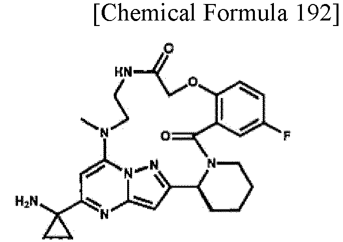

By using the same approaches as in Step 23-1, Step 1-2, Step 13-2, Step 1-3, and Step 4-2, the title compound (35 mg, colorless powder) was obtained from the compound (0.10 g) obtained in Reference Example 1-14 and the compound (86 mg) obtained in Reference Example 2-1 (3).
LCMS (ESI) m/z 508 [M+H]⁺, t_{R} = 0.441 min, mode N.

### Example 27: Synthesis of (2E)-3-[(18aS)-2-fluoro-11-methyl-7,24-dioxo-6,7,8,9,10,11,19,20,21,22-decahydro-18aH,24 H-18,15-(metheno)pyrido[2,1-1]pyrimido[6,1-h][1,4,7,9,10,13]benzoxapentaazacyclohexadec in-13-yl]prop-2-enoic acid

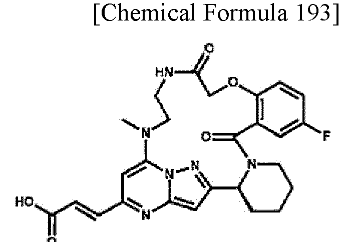

Step 27-1: To a solution of the compound (31 mg) obtained in Step 1-3 in DMF (0.64 mL), Et₃N (27 µL), ethyl acrylate (69 µL), P(o-tol)₃ (3.9 mg), and Pd(OAc)₂ (1.4 mg) were added, and the mixture was stirred at 100°C for 3 hours. Furthermore, ethyl acrylate (69 µL) was added thereto, and the mixture was stirred at 150°C for 3 hours under microwave irradiation. Water was added to the reaction liquid, and the aqueous layer was extracted with EtOAc. The organic layer was washed with Brine. The organic layer was dried over MgSO₄ and then concentrated under reduced pressure. The obtained residue was purified by NH type silica gel column chromatography (mobile phase: hexane/EtOAc = 95/5 to 0/100; v/v) to afford ethyl (2E)-3-[(18aS)-2-fluoro-11-methyl-7,24-dioxo-6,7,8,9,10,11,19,20,21,22-decahydro-18aH,24 H-18,15-(metheno)pyrido[2,1-1]pyrimido[6,1-h][1,4,7,9,10,13]benzoxapentaazacyclohexadec in-13-yl]prop-2-enoate (25 mg, yellow powder).
LCMS (ESI) m/z 551 [M+H]⁺, t_{R} = 0.983 min, mode N.

Step 27-2: By using the same approach as in Step 1-2, the title compound (1.5 mg, yellow powder) was obtained from the compound (25 mg) obtained in Step 27-1.
LCMS (ESI) m/z 523 [M+H]⁺, t_{R} = 0.753 min, mode N.

### Example 28: Synthesis of (18aS)-2-fluoro-13-(3-hydroxyprop-1-yn-1-yl)-11-methyl-8,9,10,11,19,20,21,22-octahydro-1 8aH,24H-18,15-(metheno)pyrido[2,1-1]pyrimido[6,1-h][1,4,7,9,10,13]benzoxapentaazacyclo hexadecine-7,24(6H)-dione

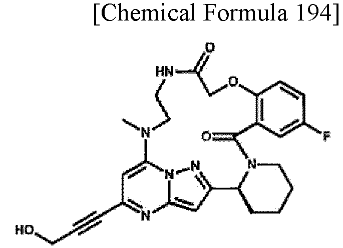

Step 28: To a solution of the compound (40 mg) obtained in Step 1-3 in DMF (0.82 mL), Et₃N (0.52 mL), propargyl alcohol (24 µL), PPh₃ (4.3 mg), HMDS (26 µL), CuI (1.6 mg), and PdCl₂(PPh₃)₂ (5.8 mg) were added, and the mixture was stirred at 110°C for 0.5 hours. Water was added to the reaction liquid. The aqueous layer was extracted with EtOAc. The organic layer was washed with Brine. The organic layer was dried over MgSO₄ and then concentrated under reduced pressure. The obtained residue was purified by NH type silica gel column chromatography (mobile phase: CHCl₃/MeOH = 100/0 to 95/5; v/v) to afford the title compound (39 mg, brown powder).
LCMS (ESI) m/z 507 [M+H]⁺, t_{R} = 0.703 min, mode N.

### Example 29: Synthesis of (18aS)-2-fluoro-13-(3-hydroxypropyl)-11-methyl-8,9,10,11,19,20,21,22-octahydro-18aH,24 H-18,15-(metheno)pyrido[2,1-l]pyrimido[6,1-h][1,4,7,9,10,13]benzoxapentaazacyclohexadec ine-7,24(6H)-dione

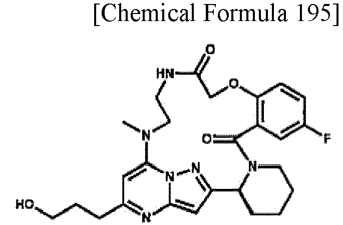

By using the same approach as in Step 4-2, the title compound (7.7 mg, colorless powder) was obtained from the compound (31 mg) obtained in Step 28.
LCMS (ESI) m/z 511 [M+H]⁺, t_{R} = 0.441 min, mode N.

### Example 30: Synthesis of (18aS)-2-fluoro-13-[2-(hydroxymethyl)cyclopropyl]-11-methyl-8,9,10,11,19,20,21,22-octahy dro-18aH,24H-18,15-(metheno)pyrido[2,1-l]pyrimido[6,1-h][1,4,7,9,10,13]benzoxapentaaza cyclohexadecine-7,24(6H)-dione

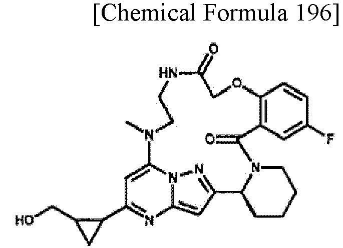

Step 30-1: To a solution of the compound (0.45 g) obtained in Step 1-3 in MeCN (9.2 mL), NaI (0.69 g) and TMSCl (0.59 mL) were added, and the mixture was stirred at 65°C for 1.5 hours. Furthermore, NaI (0.69 g) and TMSCl (0.59 mL) were added thereto, and the mixture was stirred at 65°C for 1.5 hours. NaI (0.69 g) and TMSCl (0.59 mL) were added thereto, and the mixture was stirred at 65°C for 1.5 hours. After allowing it to be cooled to room temperature, a Sat. NaHCO₃ aq. and a Na₂S₂O₃ aq. were added to the reaction liquid. The aqueous layer was extracted with EtOAc. The organic layer was dried over MgSO₄ and then concentrated under reduced pressure. The obtained residue was purified by OH type silica gel column chromatography (mobile phase: hexane/EtOAc = 50/50 to 0/100; v/v) to afford (18aS)-2-fluoro-13-iodo-11-methyl-8,9,10,11,19,20,21,22-octahydro-18aH,24H-18,15-(meth eno)pyrido[2,1-l]pyrimido[6,1-h][1,4,7,9,10,13]benzoxapentaazacyclohexadecine-7,24(6H)-d ione (0.43 g, colorless amorphous).
LCMS (ESI) m/z 579 [M+H]⁺, t_{R} = 0.975 min, mode N.

Step 30-2: To a solution of the compound (0.33 g) obtained in Step 30-1 in toluene (5.6 mL)/water (0.56 mL), ethyl 2-(4,4,5,5-tetramethyl-1,3,2-dioxaborolan-2-yl)cyclopropanecarboxylate (0.40 g), K₃PO₄ (0.36 g), Pd(OAc)₂ (0.13 g), and tricyclohexylphosphine (0.90 mL) were added, and the mixture was stirred at 90°C for 1.5 hours. After allowing it to be cooled to room temperature, the reaction solution was filtered through Celite (R) and the filtrate was concentrated. The obtained residue was purified by OH type silica gel column chromatography (mobile phase: hexane/EtOAc = 70/30 to 0/100; v/v) to afford ethyl 2-[(18aS)-2-fluoro-11-methyl-7,24-dioxo-6,7,8,9,10,11,19,20,21,22-decahydro-18aH,24H-18 ,15-(metheno)pyrido[2,1-l]pyrimido[6,1-h][1,4,7,9,10,13]benzoxapentaazacyclohexadecin-13 -yl]cyclopropane-1-carboxylate (0.21 g, colorless amorphous).
LCMS (ESI) m/z 565 [M+H]⁺, t_{R} = 0.700 min, mode N.

Step 30-3: By using the same approach as in Step 1-2, 2-[(18aS)-2-fluoro-11-methyl-7,24-dioxo-6,7,8,9,10,11,19,20,21,22-decahydro-18aH,24H-18 ,15-(metheno)pyrido[2,1-l]pyrimido[6,1-h][1,4,7,9,10,13]benzoxapentaazacyclohexadecin-13 -yl]cyclopropane-1-carboxylic acid (0.12 g, colorless amorphous) was obtained from the compound (0.21 g) obtained in Step 30-2.
LCMS (ESI) m/z 537 [M+H]⁺, t_{R} = 0.549 min, mode N.

Step 30-4: To a solution of the compound (5.5 mg) obtained in Step 30-3 in THF (0.10 mL), borane-tetrahydrofuran complex (15 µL, 1.0 M) was added, and the mixture was stirred at room temperature for 1.5 days. Furthermore, borane-tetrahydrofuran complex (50 µL, 1.0 M) was added thereto, and the mixture was stirred at room temperature for 2 hours. Furthermore, borane-tetrahydrofuran complex (0.10 mL, 1.0 M) was added thereto, and the mixture was stirred at room temperature for 2 hours. To the reaction liquid, water was added at 0°C. The aqueous layer was extracted with EtOAc. The organic layer was dried over MgSO₄ and then concentrated under reduced pressure. The obtained residue was purified by NH type silica gel column chromatography (mobile phase: CHCl₃/MeOH = 100/0 to 95/5; v/v) to afford the title compound (3.1 mg, colorless amorphous).
LCMS (ESI) m/z 523 [M+H]⁺, t_{R} = 0.532 min, mode N.

### Example 31: Synthesis of (18aS)-2-fluoro-13-[(1E)-3-hydroxyprop-1-en-1-yl]-11-methyl-8,9,10,11,19,20,21,22-octahy dro-18aH,24H-18,15-(metheno)pyrido[2,1-l]pyrimido[6,1-h][1,4,7,9,10,13]benzoxapentaaza cyclohexadecine-7,24(6H)-dione

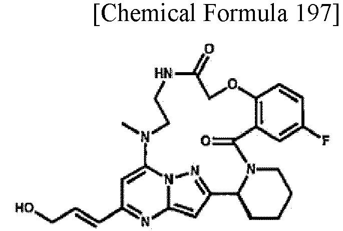

Step 31-1: To a solution of the compound (43 mg) obtained in Step 30-1 in 1,4-dioxane (0.37 mL)/water (0.97 mL), tert-butyl(dimethyl){[(2E)-3-(4,4,5,5-tetramethyl-1,3,2-dioxaborolan-2-yl)prop-2-en-1-yl]ox y} silane (48 mg), K₂CO₃ (31 mg), and Pd(PPh₃)₄ (8.5 mg) were added, and the mixture was stirred at 90°C for 4 hours. The reaction solution was poured into Brine, and the aqueous layer was extracted with EtOAc. The organic layer was concentrated, and the resulting residue was purified by NH type silica gel column chromatography (mobile phase: EtOAc/MeOH = 100/0 to 90/10; v/v) to afford (18aS)-13-[(1E)-3-{[tert-butyl(dimethyl)silyl]oxy}prop-1-en-1-yl]-2-fluoro-11-methyl-8,9,10 ,11,19,20,21,22-octahydro-18aH,24H-18,15-(metheno)pyrido[2,1-l]pyrimido[6,1-h][1,4,7,9,1 0,13]benzoxapentaazacyclohexadecine-7,24(6H)-dione (45 mg, colorless amorphous). LCMS (ESI/APCI dual) m/z 623 [M+H]⁺, t_{R} = 1.105 min, mode N.

Step 31-2: By using the same approach as in Step 5-2, the title compound (25 mg, colorless amorphous) was obtained from the compound (45 mg) obtained in Step 31-1. LCMS (ESI/APCI dual) m/z 509 [M+H]⁺, t_{R} = 0.215 min, mode N.

### Example 32: Synthesis of 2-[(18aS)-2-fluoro-11-methyl-7,24-dioxo-6,7,8,9,10,11,19,20,21,22-decahydro-18aH,24H-18 ,15-(metheno)pyrido[2,1-l]pyrimido[6,1-h][1,4,7,9,10,13]benzoxapentaazacyclohexadecin-13 -yl]cyclopropane-1-carboxamide

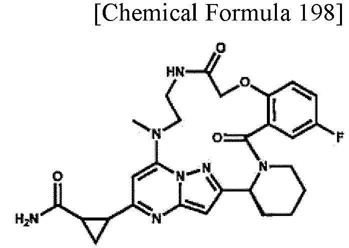

To a solution of the compound (20 mg) obtained in Step 30-3 in CHCl₃ (0.37 mL), oxalyl chloride (4.7 µL) and DMF (catalytic amount) were added, and the mixture was stirred at room temperature for 10 minutes. The reaction liquid was concentrated. To a solution of the obtained residue in THF (0.37 mL), a 28% aqueous ammonia solution (11 µL) was added, and the mixture was stirred at room temperature for 10 minutes. The reaction liquid was concentrated. The obtained residue was purified by NH type silica gel column chromatography (mobile phase: CHCl₃/MeOH = 100/0 to 95/5; v/v) to afford the title compound (16 mg, colorless powder).
LCMS (ESI) m/z 536 [M+H]⁺, t_{R} = 0.824 min, mode N.

### Example 33: Synthesis of (18aS)-13-cyclopropyl-2,25-difluoro-11-methyl-8,9,10,11,19,20,21,22-octahydro-18aH,24H-18,15-(metheno)pyrido[2,1-l]pyrimido[6,1-h][1,4,7,9,10,13]benzoxapentaazacyclohexadecin e-7,24(6H)-dione

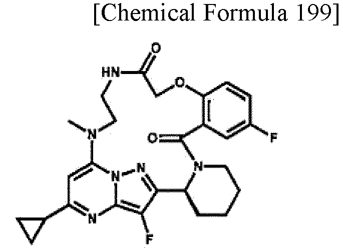

By using the same approaches as in Step 23-1, Step 1-2, Step 13-2, and Step 1-3, the title compound (0.15 g, colorless amorphous) was obtained from the compound (0.19 g) obtained in Reference Example 1-7 and the compound (0.23 g) obtained in Reference Example 2-1 (3).
LCMS (ESI/APCI dual) m/z 511 [M+H]⁺, t_{R} = 0.919 min, mode N.

### Example 34: Synthesis of (18aS)-13-(azetidin-1-yl)-2-fluoro-11-methyl-7,8,10,11,19,20,21,22-octahydro-18aH,24H-18 ,15-(metheno)pyrido[2,1-l]pyrimido[6,1-h][1,4,7,9,10,13]benzoxapentaazacyclohexadecine-9 ,24(6H)-dione

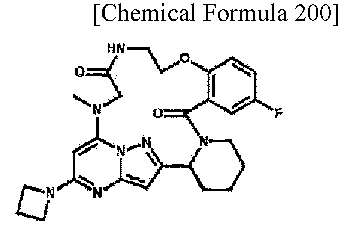

Step 34-1: By using the same approaches as in Step 1-1 to Step 1-3, (18aS)-13-chloro-2-fluoro-11 -methyl-7,8,10,11,19,20,21,22-octahydro-18aH,24H-18,15-(me theno)pyrido[2,1-l]pyrimido[6,1-h][1,4,7,9,10,13]benzoxapentaazacyclohexadecine-9,24(6H) -dione (0.21 g, orange oily matter) was obtained from the compound (0.45 g) obtained in Reference Example 1-1 (3) and the compound (0.46 g) obtained in Reference Example 2-23. LCMS (ESI) m/z 487 [M+H]⁺, t_{R} = 0.875 min, mode N.

Step 34-2: By using the same approach as in Step 1-4, the title compound (46 mg, colorless powder) was obtained from the compound (83 mg) obtained in Step 34-1 and azetidine (0.12 mL).
¹H NMR (400 MHz, CDCl₃) 1.24 - 1.73 (m, 4H), 1.95 - 2.11 (m, 1H), 2.17 - 2.25 (m, 1H), 2.35 - 2.46 (m, 2H), 2.97 (s, 3H), 3.26 - 3.42 (m, 2H), 3.44 - 3.65 (m, 2H), 3.88 - 3.98 (m, 1H), 4.07 - 4.18 (m, 6H), 4.90 (d, J = 12.3 Hz, 1H), 5.08 - 5.16 (m, 1H), 6.06 (s, 1H), 6.28 (d, J = 4.0 Hz, 1H), 6.85 (dd, J = 8.5, 4.0 Hz, 1H), 6.99 - 7.06 (m, 2H), 8.00 - 8.06 (m, 1H). LCMS (ESI) m/z 508 [M+H]⁺, t_{R} = 0.516 min, mode N.

### Example 35: Synthesis of (18aS)-13-[(3S)-3-aminopyrrolidin-1-yl]-2-fluoro-11-methyl-7,8,10,11,19,20,21,22-octahydr o-18aH,24H-18,15-(metheno)pyrido[2,1-l]pyrimido[6,1-h][1,4,7,9,10,13]benzoxapentaazacy clohexadecine-9,24(6H)-dione

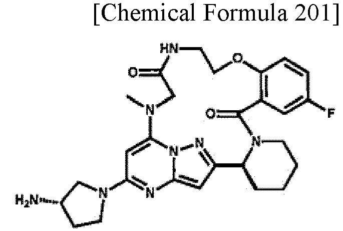

By using the same approach as in Step 1-4, the title compound (35 mg, colorless powder) was obtained from the compound (70 mg) obtained in Step 34-1 and (3S)-pyrrolidin-3-amine (62 mg).
LCMS (ESI) m/z 537 [M+H]⁺, t_{R} = 0.234 min, mode N.

### Example 36: Synthesis of (18aS)-2-fluoro-13-(3-hydroxyazetidin-1-yl)-11-methyl-7,8,10,11,19,20,21,22-octahydro-18a H,24H-18,15-(metheno)pyrido[2,1-l]pyrimido[6,1-h][1,4,7,9,10,13]benzoxapentaazacyclohe xadecine-9,24(6H)-dione

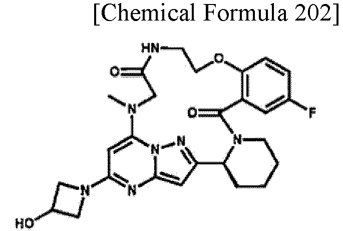

By using the same approach as in Step 3-1, the title compound (11 mg, pink powder) was obtained from the compound (23 mg) obtained in Step 34-1 and 3-hydroxyazetidine hydrochloride (52 mg).
LCMS (ESI) m/z 524 [M+H]⁺, t_{R} = 0.480 min, mode N.

### Example 37: Synthesis of (18aS)-13-(3-aminoazetidin-1-yl)-2-fluoro-11-methyl-7,8,10,11,19,20,21,22-octahydro-18aH ,24H-18,15-(metheno)pyrido[2,1-l]pyrimido[6,1-h][1,4,7,9,10,13]benzoxapentaazacyclohexa decine-9,24(6H)-dione

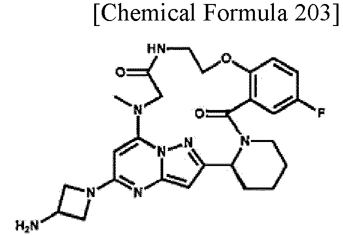

By using the same approaches as in Step 1-4 and Step 5-2, the title compound (14 mg, colorless powder) was obtained from the compound (51 mg) obtained in Step 34-1 and 3-N-BOC-amino-azetidine (90 mg).
LCMS (ESI) m/z 523 [M+H]⁺, t_{R} = 0.739 min, mode H.

### Example 38: Synthesis of (18aS)-13-(azetidin-1-yl)-2-chloro-11-methyl-8,9,10,11,19,20,21,22-octahydro-18aH,24H-18 ,15-(metheno)pyrido[2,1-l]pyrimido[6,1-h][1,4,7,9,10,13]benzoxapentaazacyclohexadecine-7 ,24(6H)-dione

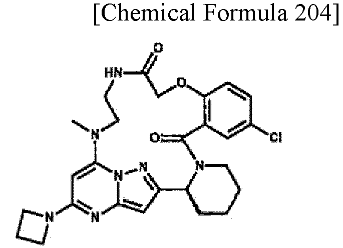

Step 38-1: By using the same approaches as in Step 1-1 to Step 1-3, (18aS)-2,13-dichloro-11-methyl-8,9,10,11,19,20,21,22-octahydro-18aH,24H-18,15-(metheno )pyrido[2,1-l]pyrimido[6,1-h][1,4,7,9,10,13]benzoxapentaazacyclohexadecine-7,24(6H)-dion e (0.55 g, colorless amorphous) was obtained from the compound (0.49 g) obtained in Reference Example 1-1 (3) and the compound (0.45 g) obtained in Reference Example 2-12. LCMS (ESI) m/z 503 [M+H]⁺, t_{R} = 0.985 min, mode N.

Step 38-2: By using the same approach as in Step 1-4, the title compound (0.17 g, colorless amorphous) was obtained from the compound (0.17 g) obtained in Step 38-1 and azetidine (0.21 mL).
LCMS (ESI) m/z 524 [M+H]⁺, t_{R} = 0.577 min, mode N.

### Example 39: Synthesis of (18aS)-2-chloro-13-(3-hydroxyazetidin-1-yl)-11-methyl-8,9,10,11,19,20,21,22-octahydro-18 aH,24H-18,15-(metheno)pyrido[2,1-l]pyrimido[6,1-h][1,4,7,9,10,13]benzoxapentaazacycloh exadecine-7,24(6H)-dione

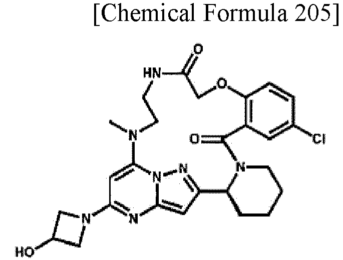

By using the same approach as in Step 3-1, the title compound (38 mg, colorless powder) was obtained from the compound (80 mg) obtained in Step 38-1 and 3-hydroxyazetidine hydrochloride (0.16 g).
¹H NMR (400 MHz, CDCl₃) δ ppm 1.18 - 1.90 (m, 3H), 1.95 - 2.15 (m, 1.6H), 2.31 (d, J = 10.9 Hz, 0.4H), 2.52 - 2.71 (m, 1H), 2.91 (s, 1.2H), 2.95 (s, 1.8H), 2.97 - 3.06 (m, 1H) 3.08 - 3.28 (m, 2H), 3.89 - 4.02 (m, 2H), 4.08 - 4.24 (m, 1H), 4.26 - 4.40 (m, 3.2H), 4.44 - 4.52 (m, 1H), 4.53 - 4.62 (m, 0.4H), 4.66 - 4.75 (m, 1H), 4.75 - 4.91 (m, 2.4H), 5.11 (s, 0.6H), 5.19 (s, 0.4H), 5.91 (s, 0.6H), 6.20 (s, 0.4H), 6.82 (d, J = 8.6 Hz, 1H), 7.27 - 7.34 (m, 2H), 7.73 - 7.81 (m, 0.4H), 8.93 - 9.01 (m, 0.6H).
LCMS (ESI) m/z 540 [M+H]⁺, t_{R} = 0.525 min, mode N.

### Example 40: Synthesis of (18aS)-13-(3-aminoazetidin-1-yl)-2-chloro-11-methyl-8,9,10,11,19,20,21,22-octahydro-18aH ,24H-18,15-(metheno)pyrido[2,1-l]pyrimido[6,1-h][1,4,7,9,10,13]benzoxapentaazacyclohexa decine-7,24(6H)-dione hydrochloride

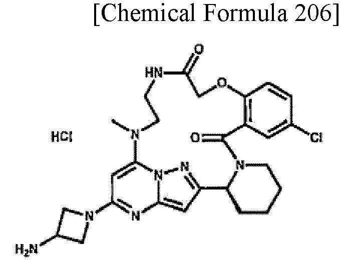

By using the same approaches as in Step 1-4 and Step 13-2, the title compound (62 mg, colorless powder) was obtained from the compound (0.30 g) obtained in Step 38-1 and 3-N-BOC-amino-azetidine (0.92 g).
LCMS (ESI) m/z 539 [M+H]⁺, t_{R} = 0.368 min, mode N.

### Example 41: Synthesis of (18aS)-13-(azetidin-1-yl)-2-chloro-8,11-dimethyl-8,9,10,11,19,20,21,22-octahydro-18aH,24 H-18,15-(metheno)pyrido[2,1-l]pyrimido[6,1-h][1,4,7,9,10,13]benzoxapentaazacyclohexadec ine-7,24(6H)-dione

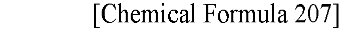

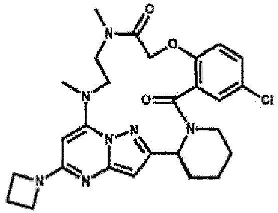

By using the same approach as in Step 7-1, the title compound (45 mg, colorless amorphous) was obtained from the compound (60 mg) obtained in Step 38-2.
LCMS (ESI) m/z 538 [M+H]⁺, t_{R} = 0.601 min, mode N.

### Example 42: Synthesis of (18aS)-13-(3-aminoazetidin-1-yl)-2-chloro-8,11-dimethyl-8,9,10,11,19,20,21,22-octahydro-1 8aH,24H-18,15-(metheno)pyrido[2,1-l]pyrimido[6,1-h] [1,4,7,9,10,13]benzoxapentaazacyclo hexadecine-7,24(6H)-dione

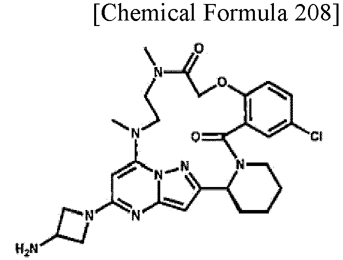

Step 42-1: By using the same approach as in Step 7-1, (18aS)-2,13-dichloro-8,11-dimethyl-8,9,10,11,19,20,21,22-octahydro-18aH,24H-18,15-(meth eno)pyrido[2,1-l]pyrimido[6,1-h][1,4,7,9,10,13]benzoxapentaazacyclohexadecine-7,24(6H)-d ione (0.20 g, colorless amorphous) was obtained from the compound (0.22 g) obtained in Step 38-1.
LCMS (ESI) m/z 517 [M+H]⁺, t_{R} = 0.998 min, mode N.

Step 42-2: By using the same approaches as in Step 1-4 and Step 5-2, the title compound (70 mg, colorless powder) was obtained from the compound (0.10 g) obtained in Step 42-1 and 3-N-BOC-amino-azetidine (0.33 g).
LCMS (ESI) m/z 553 [M+H]⁺, t_{R} = 0.833 min, mode H.

### Example 43: Synthesis of (18aS)-13-(3-aminoazetidin-1-yl)-2-chloro-8-ethyl-11-methyl-8,9,10,11,19,20,21,22-octahyd ro-18aH,24H-18,15-(metheno)pyrido[2,1-l]pyrimido[6,1-h][1,4,7,9,10,13]benzoxapentaazac yclohexadecine-7,24(6H)-dione

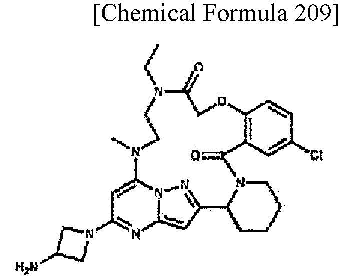

Step 43-1: By using the same approach as in Step 7-1, (18aS)-2,13-dichloro-8-ethyl-11-methyl-8,9,10,11,19,20,21,22-octahydro-18aH,24H-18,15-( metheno)pyrido[2,1-l]pyrimido[6,1-h][1,4,7,9,10,13]benzoxapentaazacyclohexadecine-7,24( 6H)-dione (0.12 g, colorless powder) was obtained by using the compound (0.22 g) obtained in Step 38-1 and EtI instead of MeI.
LCMS (ESI) m/z 531 [M+H]⁺, t_{R} = 1.052 min, mode N.

Step 43-2: By using the same approaches as in Step 1-4 and Step 5-2, the title compound (83 mg, colorless powder) was obtained from the compound (90 mg) obtained in Step 43-1 and 3-N-BOC-amino-azetidine (0.29 g).
LCMS (ESI) m/z 567 [M+H]⁺, t_{R} = 0.490 min, mode N.

### Example 44: Synthesis of (18aS)-13-(3-hydroxyazetidin-1-yl)-2,11-dimethyl-8,9,10,11,19,20,21,22-octahydro-18aH,24 H-18,15-(metheno)pyrido[2,1-l]pyrimido[6,1-h][1,4,7,9,10,13]benzoxapentaazacyclohexadec ine-7,24(6H)-dione

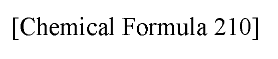

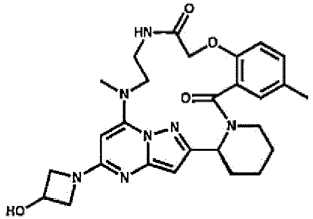

Step 44-1: By using the same approaches as in Step 1-1 to Step 1-3, (18aS)-13-chloro-2,11 -dimethyl-8,9,10,11,19,20,21,22-octahydro-18aH,24H-18,15-(metheno )pyrido[2,1-l]pyrimido[6,1-h][1,4,7,9,10,13]benzoxapentaazacyclohexadecine-7,24(6H)-dion e (0.38 g, pink powder) was obtained from the compound (0.33 g) obtained in Reference Example 1-1 (3) and the compound (0.30 g) obtained in Reference Example 2-14.
LCMS (ESI) m/z 483 [M+H]⁺, t_{R} = 0.956 min, mode N.

Step 44-2: By using the same approach as in Step 3-1, the title compound (32 mg, colorless powder) was obtained from the compound (60 mg) obtained in Step 44-1 and 3-hydroxyazetidine hydrochloride (0.14 g).
LCMS (ESI) m/z 520 [M+H]⁺, t_{R} = 0.484 min, mode N.

### Example 45: Synthesis of (18aS)-13-(3-aminoazetidin-1-yl)-2,11-dimethyl-8,9,10,11,19,20,21,22-octahydro-18aH,24H -18,15-(metheno)pyrido[2,1-l]pyrimido[6,1-h][1,4,7,9,10,13]benzoxapentaazacyclohexadecin e-7,24(6H)-dione

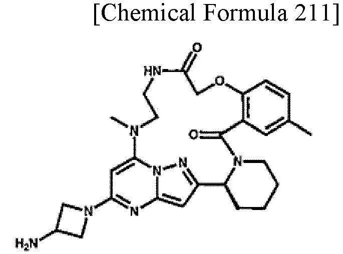

By using the same approaches as in Step 1-4 and Step 5-2, the title compound (40 mg, colorless powder) was obtained from the compound (60 mg) obtained in Step 44-1 and 3-N-BOC-amino-azetidine (0.21 g).
LCMS (ESI) m/z 519 [M+H]⁺, t_{R} = 0.235 min, mode N.

### Example 46: Synthesis of (18aS)-13-(azetidin-1-yl)-2,11-dimethyl-8,9,10,11,19,20,21,22-octahydro-18aH,24H-18,15-( metheno)pyrido[2,1-l]pyrimido[6,1-h][1,4,7,9,10,13]benzoxapentaazacyclohexadecine-7,24( 6H)-dione

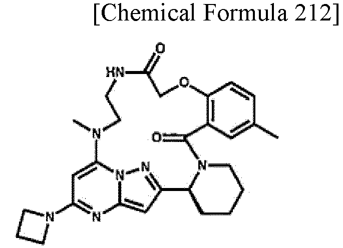

By using the same approach as in Step 1-4, the title compound (40 mg, colorless powder) was obtained from the compound (50 mg) obtained in Step 44-1 and azetidine (70 µL).
LCMS (ESI) m/z 504 [M+H]⁺, t_{R} = 0.577 min, mode N.

### Example 47: Synthesis of (18aS)-13-[(2-aminoethyl)amino]-2,11-dimethyl-8,9,10,11,19,20,21,22-octahydro-18aH,24H -18,15-(metheno)pyrido[2,1-l]pyrimido[6,1-h][1,4,7,9,10,13]benzoxapentaazacyclohexadecin e-7,24(6H)-dione hydrochloride

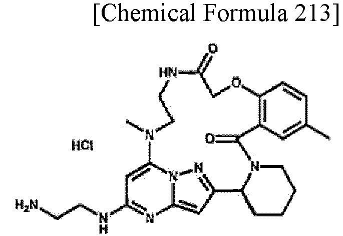

By using the same approaches as in Step 1-4 and Step 13-2, the title compound (26 mg, colorless amorphous) was obtained from the compound (50 mg) obtained in Step 44-1 and tert-butyl N-(2-aminoethyl)carbamate (0.17 g).
LCMS (ESI) m/z 507 [M+H]⁺, t_{R} = 0.343 min, mode N.

### Example 48: Synthesis of (18aS)-13-(azetidin-1-yl)-2-ethyl-11-methyl-8,9,10,11,19,20,21,22-octahydro-18aH,24H-18, 15-(metheno)pyrido[2,1-l]pyrimido[6,1-h][1,4,7,9,10,13]benzoxapentaazacyclohexadecine-7, 24(6H)-dione

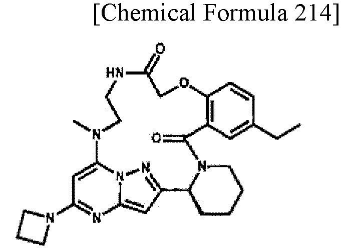

Step 48-1: By using the same approaches as in Step 1-1 to Step 1-3, (18aS)-13-chloro-2-ethyl-11-methyl-8,9,10,11,19,20,21,22-octahydro-18aH,24H-18,15-(met heno)pyrido[2,1-l]pyrimido[6,1-h][1,4,7,9,10,13]benzoxapentaazacyclohexadecine-7,24(6H)-dione (0.31 g, colorless amorphous) was obtained from the compound (0.30 g) obtained in Reference Example 1-1 (3) and the compound (0.30 g) obtained in Reference Example 2-17. LCMS (ESI) m/z 497 [M+H]⁺, t_{R} = 1.034 min, mode N.

Step 48-2: By using the same approach as in Step 1-4, the title compound (11 mg, colorless amorphous) was obtained from the compound (33 mg) obtained in Step 48-1 and azetidine (45 µL).
LCMS (ESI) m/z 518 [M+H]⁺, t_{R} = 0.618 min, mode N.

### Example 49: Synthesis of (18aS)-13-(3-aminoazetidin-1-yl)-2-ethyl-11-methyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione

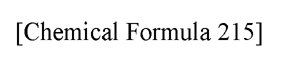

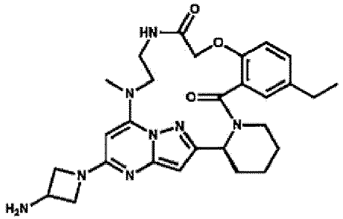

By using the same approaches as in Step 1-4 and Step 5-2, the title compound (18 mg, colorless powder) was obtained from the compound (33 mg) obtained in Step 48-1 and 3-N-BOC-amino-azetidine (57 mg).
LCMS (ESI) m/z 533 [M+H]⁺, t_{R} = 0.839 min, mode H.

### Example 50: Synthesis of (18aS)-13-(2-aminoethoxy)-2-ethyl-11-methyl-8,9,10,11,19,20,21,22-octahydro-18aH,24H-1 8,15-(metheno)pyrido[2,1-l]pyrimido[6,1-h][1,4,7,9,10,13]benzoxapentaazacyclohexadecine-7,24(6H)-dione

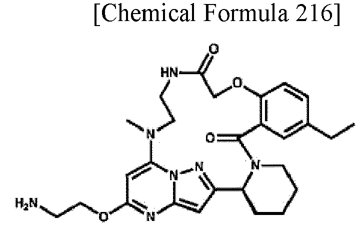

Step 50-1: To a solution of N,N-dibenzylethanolamine (73 mg) in DMF (4.0 mL), NaH (12 mg, 60% in oil) and the compound (60 mg) obtained in Step 48-1 were sequentially added, and the mixture was stirred at 90°C for 3 hours. The reaction solution was poured into water/Brine (1/1, v/v) and extracted with EtOAc. The organic layer was washed with Brine, dried over MgSO₄, filtered, and then the solvent was concentrated. The residue was purified by NH type silica gel column chromatography (mobile phase: hexane/EtOAc = 50/50 to 100/0; v/v) to afford

(18aS)-13-[2-(dibenzylamino)ethoxy]-2-ethyl-11-methyl-8,9,10,11,19,20,21,22-octahydro-18 aH,24H-18,15-(metheno)pyrido[2,1-l]pyrimido[6,1-h][1,4,7,9,10,13]benzoxapentaazacycloh exadecine-7,24(6H)-dione (40 mg, colorless amorphous). LCMS (ESI/APCI dual) m/z 702 [M+H]⁺, t_{R} = 0.720 min, mode N.

Step 50-2: To a solution of the compound (40 mg) obtained in Step 50-1 in MeOH (6.0 mL), 20% palladium hydroxide-carbon (20 mg) was added, and the mixture was stirred at room temperature overnight in the presence of hydrogen gas. The reaction solution was filtered through Celite (R), and then the solvent was concentrated. The residue was purified by NH type silica gel column chromatography (mobile phase: EtOAc/MeOH = 100/0 to 90/10; v/v) and was confirmed to be the title compound (24 mg, colorless amorphous). LCMS (ESI/APCI dual) m/z 522 [M+H]⁺, t_{R} = 0.208 min, mode N.

### Example 51: Synthesis of (18aS)-13-(azetidin-1-yl)-2-methoxy-11-methyl-8,9,10,11,19,20,21,22-octahydro-18aH,24H-18,15-(metheno)pyrido[2,1-l]pyrimido[6,1-h][1,4,7,9,10,13]benzoxapentaazacyclohexadecin e-7,24(6H)-dione

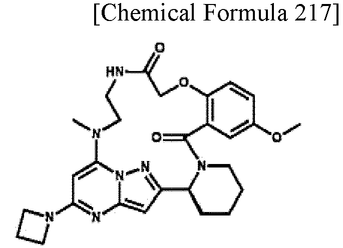

Step 51-1: By using the same approaches as in Step 1-1 to Step 1-3, (18aS)-13-chloro-2-methoxy-11-methyl-8,9,10,11,19,20,21,22-octahydro-18aH,24H-18,15-( metheno)pyrido[2,1-l]pyrimido[6,1-h][1,4,7,9,10,13]benzoxapentaazacyclohexadecine-7,24( 6H)-dione (0.31 g, colorless amorphous) was obtained from the compound (0.30 g) obtained in Reference Example 1-1 (3) and the compound (0.25 g) obtained in Reference Example 2-18.
LCMS (ESI) m/z 499 [M+H]⁺, t_{R} = 0.911 min, mode N.

Step 51-2: By using the same approach as in Step 1-4, the title compound (15 mg, colorless amorphous) was obtained from the compound (48 mg) obtained in Step 51-1 and azetidine (65 µL).
LCMS (ESI) m/z 520 [M+H]⁺, t_{R} = 0.508 min, mode N.

### Example 52: Synthesis of (18aS)-13-(3-aminoazetidin-1-yl)-2-methoxy-11-methyl-8,9,10,11,19,20,21,22-octahydro-18 aH,24H-18,15-(metheno)pyrido[2,1-l]pyrimido[6,1-h][1,4,7,9,10,13]benzoxapentaazacycloh exadecine-7,24(6H)-dione

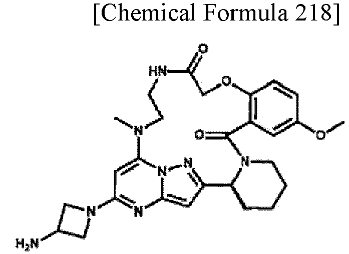

By using the same approaches as in Step 1-4 and Step 5-2, the title compound (21 mg, colorless amorphous) was obtained from the compound (48 mg) obtained in Step 51-1 and 3-N-BOC-amino-azetidine (83 mg).
LCMS (ESI) m/z 535 [M+H]⁺, t_{R} = 0.222 min, mode N.

### Example 53: Synthesis of (18aS)-13-(3-hydroxyazetidin-1-yl)-11-methyl-2-trideuteriomethyl-8,9,10,11,19,20,21,22-oct ahydro-18aH,24H-18,15-(metheno)pyrido[2,1-l]pyrimido[6,1-h][1,4,7,9,10,13]benzoxapenta azacyclohexadecine-7,24(6H)-dione

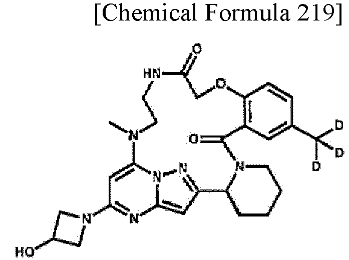

Step 53-1: By using the same approaches as in Step 1-1 to Step 1-3 and Step 3-1, (18aS)-13-(3-hydroxyazetidin-1-yl)-2-iodo-11-methyl-8,9,10,11,19,20,21,22-octahydro-18a H,24H-18,15-(metheno)pyrido[2,1-l]pyrimido[6,1-h][1,4,7,9,10,13]benzoxapentaazacyclohe xadecine-7,24(6H)-dione (0.30 g, colorless amorphous) was obtained from the compound (0.73 g) obtained in Reference Example 1-1 (3), the compound (1.2 g) obtained in Reference Example 2-19, and 3-hydroxyazetidine hydrochloride (0.58 g).
LCMS (ESI) m/z 632 [M+H]⁺, t_{R} = 0.579 min, mode N.

Step 53-2: To a suspension of zinc powder (54 mg) in DMF (0.55 mL), iodine (4.2 mg) and CD₃I (52 µL) were added, and the mixture was stirred at 70°C for 0.5 hours. In addition, to a solution of the compound (35 mg) obtained in Step 53-1 in DMF (0.50 mL), SUPERSTABLE Pd(0) CATALYST (R) (23 mg) and the above-described zinc reagent suspension were added, and after stirring the mixture at 90°C for 2.5 hours, it was left at rest at room temperature overnight. The reaction solution was filtered through Celite (R) and washed with EtOAc. Water was added to the filtrate, and the aqueous layer was extracted with EtOAc. The organic layer was dried over MgSO₄ and then concentrated under reduced pressure. The obtained residue was purified by NH type silica gel column chromatography (mobile phase: CHCl₃/MeOH = 100/0 to 95/5; v/v) and further purified by NH type silica gel column chromatography (mobile phase: CHCl₃/MeOH = 98/2; v/v) to afford the title compound (16 mg, colorless amorphous).
¹H NMR (400 MHz, CDCl₃) δ ppm 1.34 - 2.38 (m, 6.4H), 2.48 - 2.58 (m, 1H), 2.82 - 3.34 (m, 6.6H), 3.89 - 4.00 (m, 2H), 4.09 - 4.61 (m, 5.4H), 4.66 - 4.98 (m, 3.2H), 5.11 (s, 0.6H), 5.18 (s, 0.4H), 5.88 (s, 0.6H), 6.19 (s, 0.4H), 6.27 (s, 0.4H), 6.75 - 6.83 (m, 1H), 7.08 - 7.24 (m, 2H), 7.82 (m 0.4H), 9.10 (m 0.6H).
LCMS (ESI) m/z 523 [M+H]⁺, t_{R} = 0.498 min, mode N.

### Example 54: Synthesis of (18aS)-13-(azetidin-1-yl)-2-(1,1-difluoroethyl)-11-methyl-8,9,10,11,19,20,21,22-octahydro-1 8aH,24H-18,15-(metheno)pyrido[2,1-l]pyrimido[6,1-h][1,4,7,9,10,13]benzoxapentaazacyclo hexadecine-7,24(6H)-dione

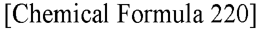

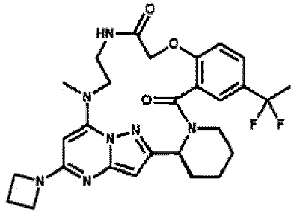

By using the same approaches as in Step 1-1 to Step 1-4, the title compound (13 mg, colorless powder) was obtained from the compound (0.19 g) obtained in Reference Example 1-1 (3), the compound (0.18 g) obtained in Reference Example 2-20, and azetidine (38 µL). LCMS (ESI) m/z 554 [M+H]⁺, t_{R} = 0.606 min, mode N.

### Example 55: Synthesis of (23aS)-18-[(3S)-3-aminopyrrolidin-1-yl]-16-methyl-1,3,4,13,14,15,16,23a-octahydro-2H,6H-23,20-(metheno)pyrido[1,2-b]pyrimido[1,6-f][2,5,6,8,11]benzopentaazacyclopentadecine-6,1 2(11H)-dione

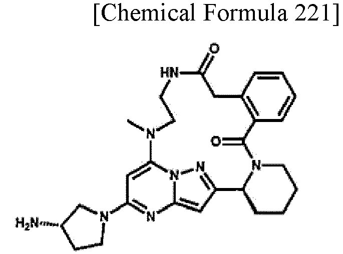

Step 55-1: By using the same approach as in Step 1-1, tert-butyl N-[2-[[5-chloro-2-[(2S)-2-piperidyl]pyrazolo[1,5-a]pyrimidin-7-yl]-methyl-amino]ethyl]carb amate (0.32 g, light yellow amorphous) was obtained from the compound (0.33 g) obtained in Reference Example 1-1 (3) and tert-butyl N-[2-(methylamino)ethyl]carbamate (0.15 g). LCMS (ESI) m/z 409 [M+H]⁺, t_{R} = 0.572 min, mode N.

Step 55-2: The compound (0.32 g) obtained in Step 55-1 and 2-(2-methoxy-2-oxo-ethyl)benzoic acid (0.16 g) were dissolved in DMF (3.9 mL), Et₃N (0.64 mL) and HATU (0.44 g) were added thereto, and the mixture was stirred at room temperature for 3 hours. After adding water to the reaction liquid and stirring the mixture, it was extracted with EtOAc/toluene (1/4, v/v). The organic layer was concentrated under reduced pressure, and the resulting residue was purified by OH type silica gel column chromatography (mobile phase: hexane/EtOAc = 90/10 to 30/70; v/v) to afford methyl 2-[2-[(2S)-2-[7-[2-(tert-butoxycarbonylamino)ethyl-methyl-amino]-5-chloro-pyrazolo[1,5-a] pyrimidin-2-yl]piperidine-1-carbonyl]phenyl]acetate (0.46 g, colorless amorphous).
LCMS (ESI) m/z 585 [M+H]⁺, t_{R} = 1.168 min, mode N.

Step 55-3: By using the same approaches as in Step 1-2, Step 13-2, and Step 1-3, (23aS)-18-chloro-16-methyl-1,3,4,13,14,15,16,23a-octahydro-2H,6H-23,20-(metheno)pyrido [1,2-b]pyrimido[1,6-f][2,5,6,8,11]benzopentaazacyclopentadecine-6,12(11H)-dione (74 mg, colorless powder) was obtained from the compound (0.25 g) obtained in Step 55-2.
LCMS (ESI) m/z 453 [M+H]⁺, t_{R} = 0.858 min, mode N.

Step 55-4: By using the same approach as in Step 1-4, the title compound (30 mg, colorless amorphous) was obtained from the compound (30 mg) obtained in Step 55-3 and (3S)-pyrrolidin-3-amine (57 mg).
¹H NMR (400 MHz, CDCl₃) δ ppm 1.70 - 1.78 (m, 1H), 1.79 - 1.89 (m, 1H), 1.90 - 2.16 (m, 2H), 2.17 - 2.44 (m, 3H), 2.52 - 2.88 (m, 2H), 2.91 (s, 3H), 3.07 - 3.18 (m, 1H), 3.27 - 3.36 (m, 1H), 3.37 - 3.92 (m, 10H), 4.39 (br s, 1H), 5.38 (s, 1H), 6.00 (s, 1H), 6.35 (d, J = 4.0 Hz, 1H), 7.21 - 7.32 (m, 2H), 7.37 (td, J = 7.6, 1.5 Hz, 1H), 7.71 (d, J = 7.6 Hz, 1H), 8.97 (br s, 1H).
LCMS (ESI) m/z 503 [M+H]⁺, t_{R} = 0.721 min, mode H.

### Example 56: Synthesis of (23aS)-18-(azetidin-1-yl)-16-methyl-1,3,4,13,14,15,16,23a-octahydro-2H,6H-23,20-(methen o)pyrido[1,2-b]pyrimido[1,6-f][2,5,6,8,11]benzopentaazacyclopentadecine-6,12(11H)-dione

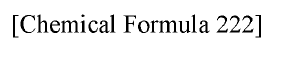

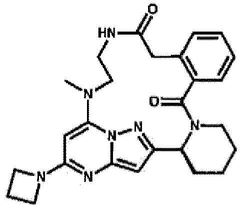

By using the same approach as in Step 1-4, the title compound (41 mg, colorless amorphous) was obtained from the compound (38 mg) obtained in Step 55-3 and azetidine (57 µL).
LCMS (ESI) m/z 474 [M+H]⁺, t_{R} = 0.501 min, mode N.

### Example 57: Synthesis of (19aS)-14-(3-aminoazetidin-1-yl)-2-fluoro-12-methyl-9,10,11,12,20,21,22,23-octahydro-6H, 19aH,25H-19,16-(metheno)pyrido[2,1-m]pyrimido[6,1-i] [1,4,8,10,11,14]benzoxapentaazacy cloheptadecine-7,25(8H)-dione

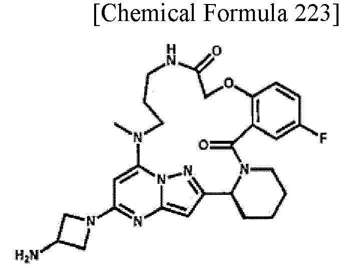

Step 57-1: By using the same approaches as in Step 1-1 to Step 1-3, (19aS)-14-chloro-2-fluoro-12-methyl-9,10,11,12,20,21,22,23-octahydro-6H,19aH,25H-19,16 -(metheno)pyrido[2,1-m]pyrimido[6,1-i][1,4,8,10,11,14]benzoxapentaazacycloheptadecine-7, 25(8H)-dione (0.17 g, colorless amorphous) was obtained from the compound (0.33 g) obtained in Reference Example 1-1 (3) and the compound (0.25 g) obtained in Reference Example 2-22.
LCMS (ESI) m/z 501 [M+H]⁺, t_{R} = 0.953 min, mode N.

Step 57-2: By using the same approaches as in Step 1-4 and Step 5-2, the title compound (67 mg, colorless powder) was obtained from the compound (80 mg) obtained in

Step 57-1 and 3-N-BOC-amino-azetidine (0.27 g).
¹H NMR (400 MHz, CDCl₃) δ ppm 1.43 - 2.49 (m, 8H), 2.98 (s, 1.2H), 3.01 (s, 1.8H), 3.03 - 3.48 (m, 2.8H), 3.61 - 4.02 (m, 4.8H), 4.16 - 4.52 (m, 4.4H), 4.70 - 4.80 (m, 0.6H), 4.94 - 5.06 (m, 2.4H), 5.69 (s, 0.6H), 6.24 (s, 0.4H), 6.74 - 6.86 (m, 1.6H), 7.00 - 7.08 (m, 2H), 7.36 (d, J = 6.4 Hz, 0.4H).
LCMS (ESI) m/z 537 [M+H]⁺, t_{R} = 0.738 min, mode H.

### Example 58: Synthesis of (19aS)-14-(azetidin-1-yl)-2-fluoro-12-methyl-9,10,11,12,20,21,22,23-octahydro-6H,19aH,25 H-19,16-(metheno)pyrido[2,1-m]pyrimido[6,1-i][1,4,8,10,11,14]benzoxapentaazacyclohepta decine-7,25(8H)-dione

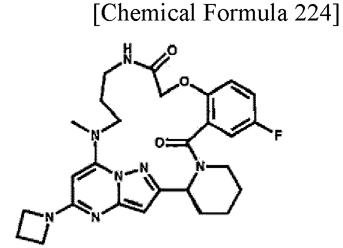

By using the same approach as in Step 1-4, the title compound (18 mg, colorless powder) was obtained from the compound (93 mg) obtained in Step 57-1 and azetidine (0.12 mL).
LCMS (ESI) m/z 522 [M+H]⁺, t_{R} = 0.952 min, mode H.

### Example 59: Synthesis of (18aS)-13-(azetidin-1-yl)-2-fluoro-11,12-dimethyl-8,9,10,11,19,20,21,22-octahydro-18aH,24 H-18,15-(metheno)pyrido[2,1-l]pyrimido[6,1-h][1,4,7,9,10,13]benzoxapentaazacyclohexadec ine-7,24(6H)-dione

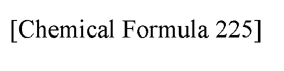

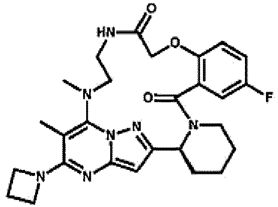

Step 59-1: By using the same approaches as in Step 1-1 to Step 1-3, (18aS)-13-chloro-2-fluoro-11,12-dimethyl-8,9,10,11,19,20,21,22-octahydro-18aH,24H-18,15 -(metheno)pyrido[2,1-l]pyrimido[6,1-h][1,4,7,9,10,13]benzoxapentaazacyclohexadecine-7,24 (6H)-dione (0.42 g, colorless powder) was obtained from the compound (0.40 g) obtained in Reference Example 1-3 and the compound (0.42 g) obtained in Reference Example 2-1 (3). LCMS (ESI) m/z 501 [M+H]⁺, t_{R} = 1.038 min, mode N.

Step 59-2: By using the same approach as in Step 1-4, the title compound (67 mg, colorless powder) was obtained from the compound (60 mg) obtained in Step 59-1 and azetidine (81 µL).
LCMS (ESI) m/z 522 [M+H]⁺, t_{R} = 0.755 min, mode N.

### Example 60: Synthesis of (18aS)-2-fluoro-13-(3-hydroxyazetidin-1-yl)-11,12-dimethyl-8,9,10,11,19,20,21,22-octahydr o-18aH,24H-18,15-(metheno)pyrido[2,1-l]pyrimido[6,1-h][1,4,7,9,10,13]benzoxapentaazacy clohexadecine-7,24(6H)-dione

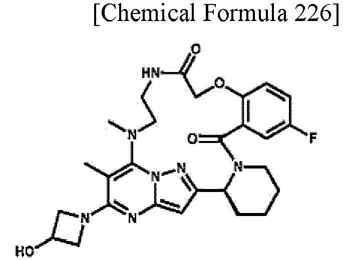

By using the same approach as in Step 3-1, the title compound (37 mg, colorless powder) was obtained from the compound (60 mg) obtained in Step 59-1 and 3-hydroxyazetidine hydrochloride (0.13 g).
LCMS (ESI) m/z 538 [M+H]⁺, t_{R} = 0.678 min, mode N.

### Example 61: Synthesis of (18aS)-13-(3-aminoazetidin-1-yl)-2-fluoro-11,12-dimethyl-8,9,10,11,19,20,21,22-octahydro-18aH,24H-18,15-(metheno)pyrido[2,1-l]pyrimido[6,1-h] [1,4,7,9,10,13]benzoxapentaazacycl ohexadecine-7,24(6H)-dione

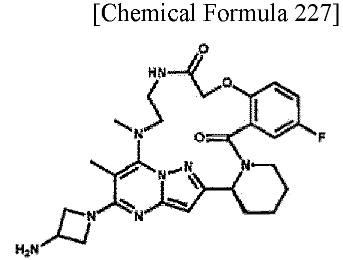

By using the same approaches as in Step 1-4 and Step 5-2, the title compound (51 mg, colorless powder) was obtained from the compound (60 mg) obtained in Step 59-1 and 3-N-BOC-amino-azetidine (0.21 g).
LCMS (ESI) m/z 537 [M+H]⁺, t_{R} = 0.934 min, mode H.

### Example 62: Synthesis of (18aS)-13-(2-aminoethoxy)-2-fluoro-11,12-dimethyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione

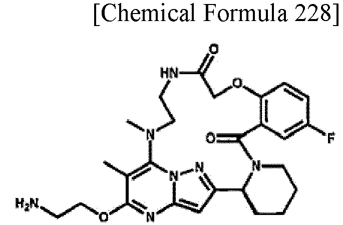

Step 62-1: To a solution of 2-(diallylamino)ethanol (41 mg) in DMF (1.2 mL), NaH (12 mg, 60% in oil) was added, and the mixture was stirred at room temperature for 5 minutes. To this, the compound (58 mg) obtained in Step 59-1 was added, and the mixture was stirred at 90°C for 1 hour. Furthermore, NaH (12 mg, 60% in oil) was added thereto, and the mixture was stirred at 90°C for 0.5 hours. After allowing the reaction liquid to be cooled to room temperature, water was added thereto. The aqueous layer was extracted with EtOAc. The organic layer was dried over MgSO₄ and then concentrated under reduced pressure. The obtained residue was purified by NH type silica gel column chromatography (mobile phase: hexane/EtOAc = 50/50 to 0/100; v/v) to afford (18aS)-13-{2-[di(prop-2-en-1-yl)amino]ethoxy}-2-fluoro-11,12-dimethyl-8,9,10,11,19,20,21, 22-octahydro-18aH,24H-18,15-(metheno)pyrido[2,1-l]pyrimido[6,1-h][1,4,7,9,10,13]benzox apentaazacyclohexadecine-7,24(6H)-dione (31 mg, colorless amorphous).
LCMS (ESI) m/z 602 [M+H]⁺, t_{R} = 0.681 min, mode N.

Step 62-2: To a solution of the compound (31 mg) obtained in Step 62-1 in CHCl₃ (1.0 mL), 1,3-dimethylbarbituric acid (48 mg) and Pd(PPh₃)₄ (12 mg) were added, and the mixture was stirred at room temperature for 0.5 hours. The reaction liquid was concentrated, and the resulting residue was purified by NH type silica gel column chromatography (mobile phase: hexane/EtOEt = 30/70 to CHCl₃; v/v) to afford the title compound (19 mg, yellow amorphous).
LCMS (ESI) m/z 526 [M+H]⁺, t_{R} = 0.554 min, mode N.

### Example 63: Synthesis of (18aS)-13-(azetidin-1-yl)-2-fluoro-8,11,12-trimethyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione

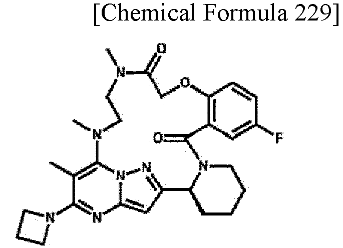

Step 63-1: By using the same approach as in Step 7-1, (18aS)-13-chloro-2-fluoro-8,11,12-trimethyl-8,9,10,11,19,20,21,22-octahydro-18aH,24H-18, 15-(metheno)pyrido[2,1-l]pyrimido[6,1-h][1,4,7,9,10,13]benzoxapentaazacyclohexadecine-7, 24(6H)-dione (0.23 g, colorless powder) was obtained from the compound (0.20 g) obtained in Step 59-1.
LCMS (ESI) m/z 515 [M+H]⁺, t_{R} = 1.008 min, mode N.

Step 63-2: By using the same approach as in Step 1-4, the title compound (25 mg, colorless powder) was obtained from the compound (68 mg) obtained in Step 63-1 and azetidine (80 µL).
LCMS (ESI) m/z 536 [M+H]⁺, t_{R} = 0.666 min, mode N.

### Example 64: Synthesis of (18aS)-2-fluoro-13-(3-hydroxyazetidin-1-yl)-8,11,12-trimethyl-8,9,10,11,19,20,21,22-octahy dro-18aH,24H-18,15-(metheno)pyrido[2,1-l]pyrimido[6,1-h][1,4,7,9,10,13]benzoxapentaaza cyclohexadecine-7,24(6H)-dione

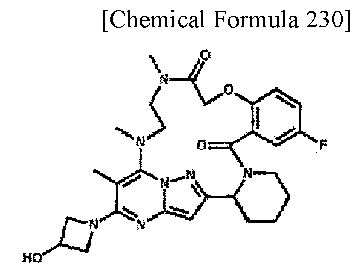

By using the same approach as in Step 3-1, the title compound (59 mg, colorless powder) was obtained from the compound (85 mg) obtained in Step 63-1 and 3-hydroxyazetidine hydrochloride (0.18 g).
LCMS (ESI) m/z 552 [M+H]⁺, t_{R} = 0.621 min, mode N.

### Example 65: Synthesis of (18aS)-13-(3-aminoazetidin-1-yl)-2-fluoro-8,11,12-trimethyl-8,9,10,11,19,20,21,22-octahydr o-18aH,24H-18,15-(metheno)pyrido[2,1-l]pyrimido[6,1-h][1,4,7,9,10,13]benzoxapentaazacy clohexadecine-7,24(6H)-dione

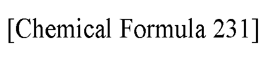

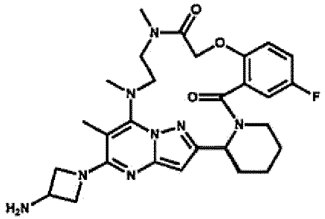

By using the same approaches as in Step 1-4 and Step 5-2, the title compound (20 mg, colorless powder) was obtained from the compound (71 mg) obtained in Step 63-1 and 3-N-BOC-amino-azetidine (0.21 g).
LCMS (ESI) m/z 551 [M+H]⁺, t_{R} = 0.524 min, mode N.

### Example 66: Synthesis of (18aS)-2-fluoro-13-(3-hydroxyazetidin-1-yl)-12-methyl-8,9,10,11,19,20,21,22-octahydro-18a H,24H-18,15-(metheno)pyrido[2,1-l]pyrimido[6,1-h][1,4,7,9,10,13]benzoxapentaazacyclohe xadecine-7,24(6H)-dione

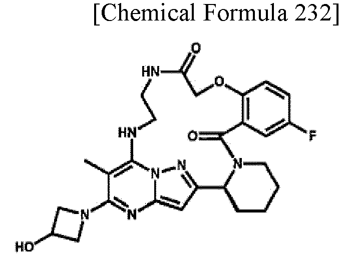

Step 66-1: By using the same approaches as in Step 1-1 to Step 1-3, (18aS)-13-chloro-2-fluoro-12-methyl-8,9,10,11,19,20,21,22-octahydro-18aH,24H-18,15-(me theno)pyrido[2,1-l]pyrimido[6,1-h][1,4,7,9,10,13]benzoxapentaazacyclohexadecine-7,24(6H) -dione (0.47 g, colorless powder) was obtained from the compound (0.55 g) obtained in Reference Example 1-3 and the compound (0.63 g) obtained in Reference Example 2-2. LCMS (ESI) m/z 487 [M+H]⁺, t_{R} = 0.899 min, mode N.

Step 66-2: By using the same approach as in Step 3-1, the title compound (65 mg, colorless powder) was obtained from the compound (0.10 g) obtained in Step 66-1 and 3-hydroxyazetidine hydrochloride (0.23 g).
LCMS (ESI/APCI dual) m/z 524 [M+H]⁺, t_{R} = 0.928 min, mode H.

### Example 67: Synthesis of (18aS)-13-(3-aminoazetidin-1-yl)-2-fluoro-12-methyl-8,9,10,11,19,20,21,22-octahydro-18aH ,24H-18,15-(metheno)pyrido[2,1-l]pyrimido[6,1-h][1,4,7,9,10,13]benzoxapentaazacyclohexa decine-7,24(6H)-dione

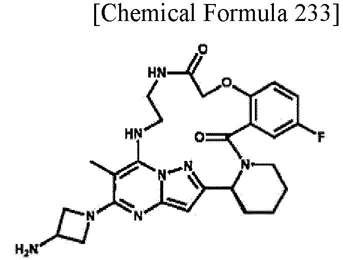

By using the same approaches as in Step 1-4 and Step 5-2, the title compound (71 mg, colorless powder) was obtained from the compound (0.11 g) obtained in Step 66-1 and 3-N-BOC-amino-azetidine (0.35 g).
LCMS (ESI/APCI dual) m/z 523 [M+H]⁺, t_{R} = 0.224 min, mode N.

### Example 68: Synthesis of (18aS)-13-(3-aminoazetidin-1-yl)-2-chloro-11,12-dimethyl-8,9,10,11,19,20,21,22-octahydro-18aH,24H-18,15-(metheno)pyrido[2,1-l]pyrimido[6,1-h][1,4,7,9,10,13]benzoxapentaazacycl ohexadecine-7,24(6H)-dione

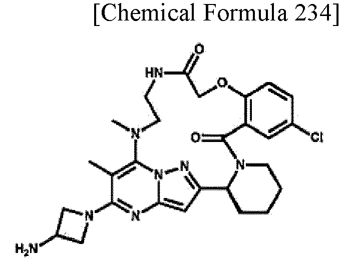

Step 68-1: By using the same approaches as in Step 1-1 to Step 1-3, (18aS)-2,13-dichloro-11,12-dimethyl-8,9,10,11,19,20,21,22-octahydro-18aH,24H-18,15-(met heno)pyrido[2,1-l]pyrimido[6,1-h][1,4,7,9,10,13]benzoxapentaazacyclohexadecine-7,24(6H)-dione (0.34 g, colorless powder) was obtained from the compound (0.41 g) obtained in Reference Example 1-3 and the compound (0.50 g) obtained in Reference Example 2-12. LCMS (ESI) m/z 517 [M+H]⁺, t_{R} = 1.103 min, mode N.

Step 68-2: By using the same approaches as in Step 1-4 and Step 5-2, the title compound (39 mg, colorless powder) was obtained from the compound (0.10 g) obtained in Step 68-1 and 3-N-BOC-amino-azetidine (0.33 g).
LCMS (ESI) m/z 553 [M+H]⁺, t_{R} = 0.586 min, mode N.

### Example 69: Synthesis of (18aS)-2-chloro-13-(3-hydroxyazetidin-1-yl)-11,12-dimethyl-8,9,10,11,19,20,21,22-octahydr o-18aH,24H-18,15-(metheno)pyrido[2,1-l]pyrimido[6,1-h][1,4,7,9,10,13]benzoxapentaazacy clohexadecine-7,24(6H)-dione

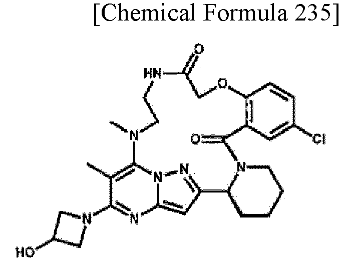

By using the same approach as in Step 3-1, the title compound (77 mg, colorless powder) was obtained from the compound (72 mg) obtained in Step 68-1 and 3-hydroxyazetidine hydrochloride (0.15 g).
LCMS (ESI) m/z 554 [M+H]⁺, t_{R} = 0.738 min, mode N.

### Example 70: Synthesis of (18aS)-13-[(2-aminoethyl)amino]-2-chloro-11,12-dimethyl-8,9,10,11,19,20,21,22-octahydro-18aH,24H-18,15-(metheno)pyrido[2,1-l]pyrimido[6,1-h][1,4,7,9,10,13]benzoxapentaazacycl ohexadecine-7,24(6H)-dione

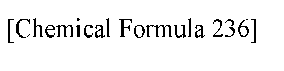

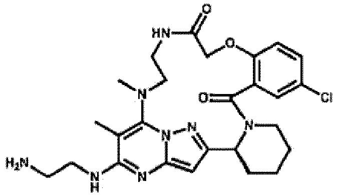

By using the same approaches as in Step 1-4 and Step 13-2, the title compound (38 mg, colorless amorphous) was obtained from the compound (48 mg) obtained in Step 68-1 and tert-butyl N-(2-aminoethyl)carbamate (0.15 mL).
LCMS (ESI) m/z 541 [M+H]⁺, t_{R} = 0.596 min, mode N.

### Example 71: Synthesis of (18aS)-13-[(2-aminoethyl)sulfanyl]-2-chloro-11,12-dimethyl-8,9,10,11,19,20,21,22-octahydr o-18aH,24H-18,15-(metheno)pyrido[2,1-l]pyrimido[6,1-h][1,4,7,9,10,13]benzoxapentaazacy clohexadecine-7,24(6H)-dione

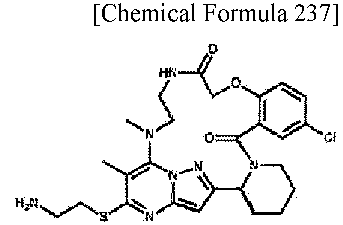

By using the same approaches as in Step 1-4 and Step 13-2, the title compound (40 mg, colorless amorphous) was obtained from the compound (41 mg) obtained in Step 68-1 and tert-butyl N-(2-sulfanylethyl)carbamate (0.14 mL).
LCMS (ESI) m/z 558 [M+H]⁺, t_{R} = 0.631 min, mode N.

### Example 72: Synthesis of (18aS)-13-(2-aminoethoxy)-2-chloro-11,12-dimethyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione

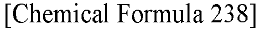

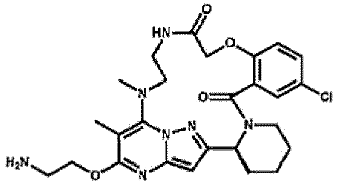

By using the same approaches as in Step 62-1 and Step 62-2, the title compound (13 mg, colorless amorphous) was obtained from the compound (43 mg) obtained in Step 68-1.
LCMS (ESI) m/z 542 [M+H]⁺, t_{R} = 0.605 min, mode N.

### Example 73: Synthesis of (18aS)-2-chloro-13-(3-hydroxypropyl)-11,12-dimethyl-8,9,10,11,19,20,21,22-octahydro-18a H,24H-18,15-(metheno)pyrido[2,1-l]pyrimido[6,1-h][1,4,7,9,10,13]benzoxapentaazacyclohe xadecine-7,24(6H)-dione

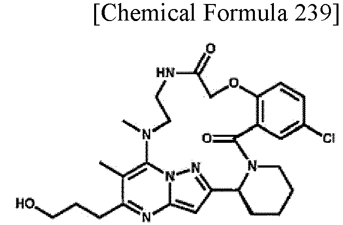

By using the same approaches as in Step 28 and Step 4-2, the title compound (15 mg, colorless powder) was obtained from the compound (31 mg) obtained in Step 68-1.
LCMS (ESI) m/z 541 [M+H]⁺, t_{R} = 0.801 min, mode N.

### Example 74: Synthesis of (18aS)-13-(3-aminoprop-1-yn-1-yl)-2-chloro-11,12-dimethyl-8,9,10,11,19,20,21,22-octahydr o-18aH,24H-18,15-(metheno)pyrido[2,1-l]pyrimido[6,1-h][1,4,7,9,10,13]benzoxapentaazacy clohexadecine-7,24(6H)-dione

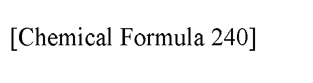

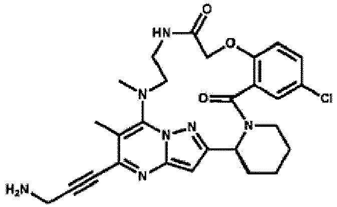

Step 74-1: By using the same approach as in Step 28, tert-butyl {3-[(18aS)-2-chloro-11,12-dimethyl-7,24-dioxo-6,7,8,9,10,11,19,20,21,22-decahydro-18aH,2 4H-18,15-(metheno)pyrido[2,1-l]pyrimido[6,1-h][1,4,7,9,10,13]benzoxapentaazacyclohexade cin-13-yl]prop-2-yn-1-yl}carbamate (85 mg, yellow powder) was obtained from the compound (58 mg) obtained in Step 68-1 and N-BOC-propargylamine (87 mg).
LCMS (ESI) m/z 636 [M+H]⁺, t_{R} = 1.122 min, mode N.

Step 74-2: By using the same approach as in Step 5-2, the title compound (15 mg, brown powder) was obtained from the compound (33 mg) obtained in Step 74-1.
LCMS (ESI) m/z 536 [M+H]⁺, t_{R} = 0.578 min, mode N.

### Example 75: Synthesis of (18aS)-13 -(3 -aminopropyl)-2-chloro-11,12-dimethyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione

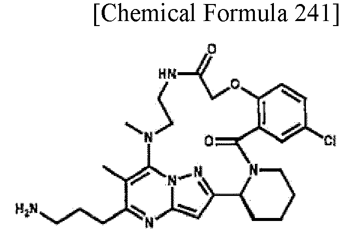

By using the same approaches as in Step 4-2 and Step 13-2, the title compound (30 mg, colorless powder) was obtained from the compound (61 mg) obtained in Step 74-1. LCMS (ESI) m/z 540 [M+H]⁺, t_{R} = 0.581 min, mode N.

### Example 76: Synthesis of (18aS)-2-chloro-13-[3-(dimethylamino)propyl]-11,12-dimethyl-8,9,10,11,19,20,21,22-octahy dro-18aH,24H-18,15-(metheno)pyrido[2,1-l]pyrimido[6,1-h][1,4,7,9,10,13]benzoxapentaaza cyclohexadecine-7,24(6H)-dione

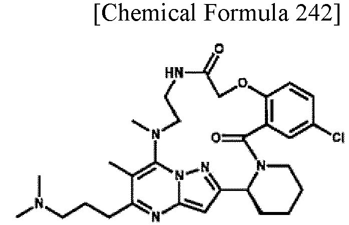

By using the same approaches as in Step 28 and Step 4-2, the title compound (20 mg, colorless amorphous) was obtained from the compound (52 mg) obtained in Step 68-1 and 1-dimethylamino-2-propyne (85 µL).
LCMS (ESI) m/z 568 [M+H]⁺, t_{R} = 0.595 min, mode N.

### Example 77: Synthesis of (18aS)-13-(2-aminoethyl)-2-chloro-11,12-dimethyl-8,9,10,11,19,20,21,22-octahydro-18aH,2 4H-18,15-(metheno)pyrido[2,1-l]pyrimido[6,1-h][1,4,7,9,10,13]benzoxapentaazacyclohexade cine-7,24(6H)-dione

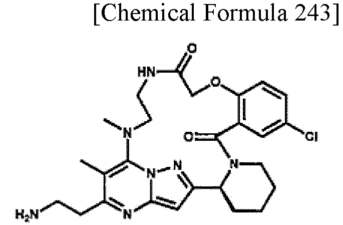

By using the same approaches as in Step 30-1, Step 30-2, and Step 5-2, the title compound (8.1 mg, colorless amorphous) was obtained from the compound (0.11 g) obtained in Step 68-1 and potassium {2-[(tert-butoxycarbonyl)amino]ethyl}(trifluoro)borate (0.14 g). LCMS (ESI/APCI dual) m/z 526 [M+H]⁺, t_{R} = 0.862 min, mode H.

### Example 78: Synthesis of (18aS)-13-(4-aminobutyl)-2-chloro-11,12-dimethyl-8,9,10,11,19,20,21,22-octahydro-18aH,2 4H-18,15-(metheno)pyrido[2,1-l]pyrimido[6,1-h][1,4,7,9,10,13]benzoxapentaazacyclohexade cine-7,24(6H)-dione

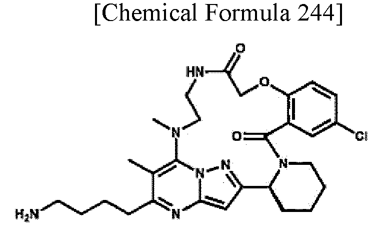

By using the same approaches as in Step 28, Step 4-2, and Step 13-2, the title compound (45 mg, colorless amorphous) was obtained from the compound (68 mg) obtained in Step 68-1 and tert-butyl N-but-3-ynylcarbamate (0.11 g).
LCMS (ESI) m/z 554 [M+H]⁺, t_{R} = 0.588 min, mode N.

### Example 79: Synthesis of (18aS)-13-(3-aminoazetidin-1-yl)-2-chloro-8,11,12-trimethyl-8,9,10,11,19,20,21,22-octahydr o-18aH,24H-18,15-(metheno)pyrido[2,1-l]pyrimido[6,1-h][1,4,7,9,10,13]benzoxapentaazacy clohexadecine-7,24(6H)-dione

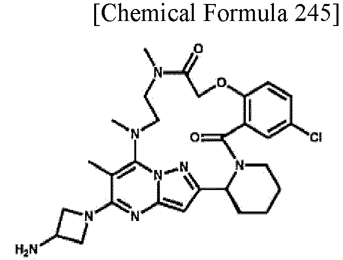

By using the same approaches as in Step 7-1, Step 1-4, and Step 5-2, the title compound (22 mg, colorless powder) was obtained from the compound (0.10 g) obtained in Step 68-1 and 3-N-BOC-amino-azetidine (0.18 g).
LCMS (ESI) m/z 567 [M+H]⁺, t_{R} = 0.577 min, mode N.

### Example 80: Synthesis of (18aS)-13-(3-aminoazetidin-1-yl)-2,11,12-trimethyl-8,9,10,11,19,20,21,22-octahydro-18aH,2 4H-18,15-(metheno)pyrido[2,1-l]pyrimido[6,1-h][1,4,7,9,10,13]benzoxapentaazacyclohexade cine-7,24(6H)-dione

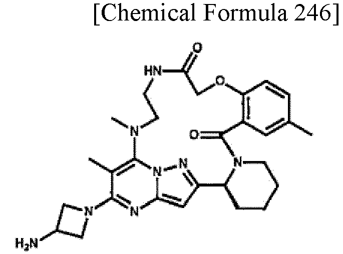

Step 80-1: By using the same approaches as in Step 1-1 to Step 1-3, (18aS)-13-chloro-2,11,12-trimethyl-8,9,10,11,19,20,21,22-octahydro-18aH,24H-18,15-(meth eno)pyrido[2,1-l]pyrimido[6,1-h][1,4,7,9,10,13]benzoxapentaazacyclohexadecine-7,24(6H)-d ione (0.62 g, colorless amorphous) was obtained from the compound (1.0 g) obtained in Reference Example 1-3 and the compound (1.2 g) obtained in Reference Example 2-14. LCMS (ESI) m/z 497 [M+H]⁺, t_{R} = 1.089 min, mode N.

Step 80-2: By using the same approaches as in Step 1-4 and Step 5-2, the title compound (64 mg, colorless amorphous) was obtained from the compound (65 mg) obtained in Step 80-1 and 3-N-BOC-amino-azetidine (0.23 g).
LCMS (ESI/APCI dual) m/z 533 [M+H]⁺, t_{R} = 0.221 min, mode N.

### Example 81: Synthesis of (18aS)-13-(2-aminoethoxy)-2,11,12-trimethyl-8,9,10,11,19,20,21,22-octahydro-18aH,24H-1 8,15-(metheno)pyrido[2,1-l]pyrimido[6,1-h][1,4,7,9,10,13]benzoxapentaazacyclohexadecine-7,24(6H)-dione

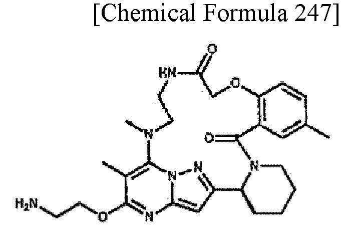

By using the same approaches as in Step 62-1 and Step 62-2, the title compound (23 mg, yellow amorphous) was obtained from the compound (51 mg) obtained in Step 80-1. LCMS (ESI) m/z 522 [M+H]⁺, t_{R} = 0.570 min, mode N.

### Example 82: Synthesis of (18aS)-13-(aminomethyl)-2,11,12-trimethyl-8,9,10,11,19,20,21,22-octahydro-18aH,24H-18, 15-(metheno)pyrido[2,1-l]pyrimido[6,1-h][1,4,7,9,10,13]benzoxapentaazacyclohexadecine-7, 24(6H)-dione

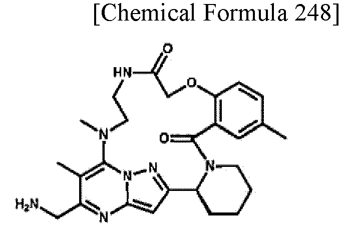

Step 82-1: To a solution of the compound (0.10 g) obtained in Step 80-1 in DMF (2.0 mL), Pd(PPh₃)₄ (0.23 g) and zinc cyanide (71 mg) were added, and the mixture was stirred at 110°C overnight. The reaction solution was poured into Brine and extracted with EtOAc. The organic layer was concentrated, and the resulting residue was purified by NH type silica gel column chromatography (mobile phase: EtOAc/MeOH = 100/0 to 90/10; v/v) to afford (18aS)-2,11,12-trimethyl-7,24-dioxo-6,7,8,9,10,11,19,20,21,22-decahydro-18aH,24H-18,15-( metheno)pyrido[2,1-l]pyrimido[6,1-h][1,4,7,9,10,13]benzoxapentaazacyclohexadecine-13-ca rbonitrile (0.18 g, colorless amorphous).
LCMS (ESI/APCI dual) m/z 488 [M+H]⁺, t_{R} = 0.899 min, mode N.

Step 82-2: By using the same approach as in Step 50-2, the title compound (40 mg, colorless amorphous) was obtained from the compound (0.18 g) obtained in Step 82-1. LCMS (ESI/APCI dual) m/z 492 [M+H]⁺, t_{R} = 0.206 min, mode N.

### Example 83: Synthesis of (18aS)-13-(3-aminoazetidin-1-yl)-2,12-difluoro-11-methyl-8,9,10,11,19,20,21,22-octahydro-18aH,24H-18,15-(metheno)pyrido[2,1-l]pyrimido[6,1-h][1,4,7,9,10,13]benzoxapentaazacycl ohexadecine-7,24(6H)-dione

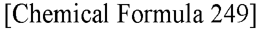

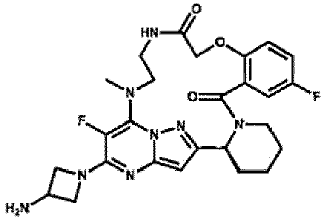

Step 83-1: By using the same approach as in Step 1-1, methyl 2-[2-[2-[[5-chloro-6-fluoro-2-[(2S)-2-piperidyl]pyrazolo[1,5-a]pyrimidin-7-yl]-methyl-amino ]ethylamino]-2-oxo-ethoxy]-5-fluoro-benzoate (0.23 g, yellow powder) was obtained from the compound (1.0 g) obtained in Reference Example 1-4 and the compound (0.22 g) obtained in Reference Example 2-1 (3).
LCMS (ESI) m/z 537 [M+H]⁺, t_{R} = 0.650 min, mode N.

Step 83-2: By using the same approach as in Step 1-4, methyl 2-[2-({2-[(5-{3-[(tert-butoxycarbonyl)amino]azetidin-1-yl}-6-fluoro-2-[(2S)-piperidin-2-yl]p yrazolo[1,5-a]pyrimidin-7-yl)(methyl)amino]ethyl}amino)-2-oxoethoxy]-5-fluorobenzoate (0.11 g, colorless oily matter) was obtained from the compound (0.23 g) obtained in Step 83-1 and 3-N-BOC-amino-azetidine (0.74 g).
LCMS (ESI) m/z 673 [M+H]⁺, t_{R} = 0.690 min, mode N.

Step 83-3: By using the same approaches as in Step 1-2, Step 1-3, and Step 5-2, the title compound (37 mg, colorless amorphous) was obtained from the compound (0.11 g) obtained in Step 83-2.
LCMS (ESI) m/z 541 [M+H]⁺, t_{R} = 0.481 min, mode N.

### Example 84: Synthesis of (18aS)-2,12-difluoro-13-(3-hydroxyazetidin-1-yl)-11-methyl-8,9,10,11,19,20,21,22-octahydr o-18aH,24H-18,15-(metheno)pyrido[2,1-l]pyrimido[6,1-h][1,4,7,9,10,13]benzoxapentaazacy clohexadecine-7,24(6H)-dione

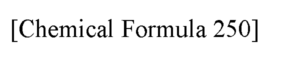

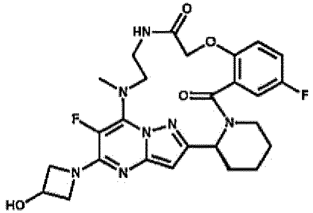

By using the same approaches as in Step 1-4, Step 1-2, and Step 1-3, the title compound (70 mg, colorless amorphous) was obtained from the compound (0.12 g) obtained in Step 83-1 and 3-hydroxyazetidine hydrochloride (94 mg).
LCMS (ESI) m/z 542 [M+H]⁺, t_{R} = 0.804 min, mode N.

### Example 85: Synthesis of (18aS)-13-(3-aminoazetidin-1-yl)-2,12-difluoro-8,11-dimethyl-8,9,10,11,19,20,21,22-octahy dro-18aH,24H-18,15-(metheno)pyrido[2,1-l]pyrimido[6,1-h][1,4,7,9,10,13]benzoxapentaaza cyclohexadecine-7,24(6H)-dione

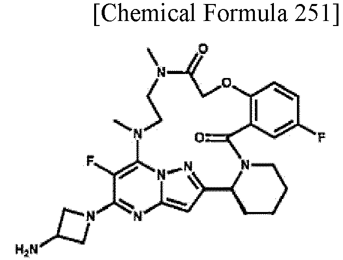

By using the same approaches as in Step 1-1, Step 1-4, Step 1-2, Step 1-3, and Step 5-2, the title compound (31 mg, colorless amorphous) was obtained from the compound (0.50 g) obtained in Reference Example 1-4, the compound (0.27 g) obtained in Reference Example 2-113, and 3-N-BOC-amino-azetidine (0.37 g).
LCMS (ESI) m/z 555 [M+H]⁺, t_{R} = 0.532 min, mode N.

### Example 86: Synthesis of (18aS)-13-(3-aminoazetidin-1-yl)-2-chloro-12-fluoro-11-methyl-8,9,10,11,19,20,21,22-octah ydro-18aH,24H-18,15-(metheno)pyrido[2,1-l]pyrimido[6,1-h][1,4,7,9,10,13]benzoxapentaaz acyclohexadecine-7,24(6H)-dione

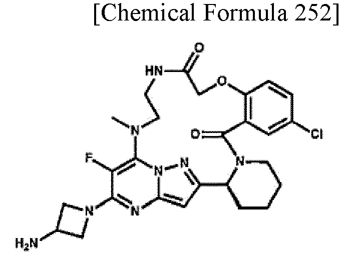

By using the same approaches as in Step 1-1, Step 1-4, Step 1-2, Step 1-3, and Step 5-2, the title compound (22 mg, colorless amorphous) was obtained from the compound (0.50 g) obtained in Reference Example 1-4, the compound (0.11 g) obtained in Reference Example 2-12, and 3-N-BOC-amino-azetidine (0.14 g).
LCMS (ESI) m/z 557 [M+H]⁺, t_{R} = 0.544 min, mode N.

### Example 87: Synthesis of (18aS)-13-[(3S)-3-aminopyrrolidin-1-yl]-2-chloro-12-fluoro-11-methyl-8,9,10,11,19,20,21,2 2-octahydro-18aH,24H-18,15-(metheno)pyrido[2,1-l]pyrimido[6,1-h][1,4,7,9,10,13]benzoxa pentaazacyclohexadecine-7,24(6H)-dione hydrochloride

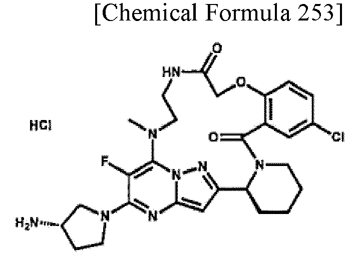

By using the same approaches as in Step 1-1, Step 1-4, Step 1-2, Step 1-3, and Step 13-2, the title compound (60 mg, yellow amorphous) was obtained from the compound (3.0 g) obtained in Reference Example 1-4, the compound (2.0 g) obtained in Reference Example 2-12, and tert-butyl (3S)-pyrrolidin-3-ylcarbamate (0.24 g).
LCMS (ESI) m/z 571 [M+H]⁺, t_{R} = 0.574 min, mode N.

### Example 88: Synthesis of (18aS)-13-(3-aminoazetidin-1-yl)-12-fluoro-2,11-dimethyl-8,9,10,11,19,20,21,22-octahydro-18aH,24H-18,15-(metheno)pyrido[2,1-l]pyrimido[6,1-h][1,4,7,9,10,13]benzoxapentaazacycl ohexadecine-7,24(6H)-dione

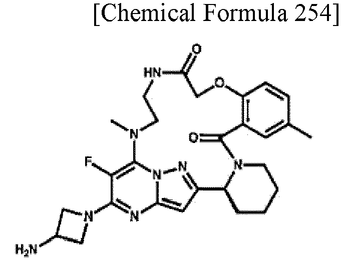

By using the same approaches as in Step 1-1, Step 1-4, Step 1-2, Step 1-3, and Step 5-2, the title compound (15 mg, colorless amorphous) was obtained from the compound (0.35 g) obtained in Reference Example 1-4, the compound (0.18 g) obtained in Reference Example 2-14, and 3-N-BOC-amino-azetidine (0.14 g).
LCMS (ESI) m/z 537 [M+H]⁺, t_{R} = 0.516 min, mode N.

### Example 89: Synthesis of (18aS)-13-(3-aminoazetidin-1-yl)-12-chloro-2-fluoro-11-methyl-8,9,10,11,19,20,21,22-octah ydro-18aH,24H-18,15-(metheno)pyrido[2,1-l]pyrimido[6,1-h][1,4,7,9,10,13]benzoxapentaaz acyclohexadecine-7,24(6H)-dione

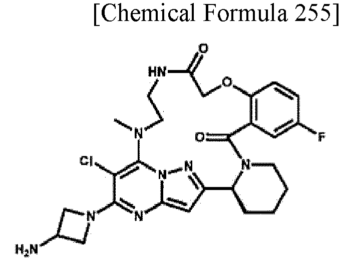

By using the same approaches as in Step 1-1, Step 1-4, Step 1-2, Step 1-3, and Step 5-2, the title compound (23 mg, colorless amorphous) was obtained from the compound (1.5 g) obtained in Reference Example 1-5, the compound (0.76 g) obtained in Reference Example 2-1 (3), and 3-N-BOC-amino-azetidine (0.14 g).
LCMS (ESI) m/z 557 [M+H]⁺, t_{R} = 0.549 min, mode N.

### Example 90: Synthesis of (18aS)-13-cyclopropyl-2-fluoro-11-methyl-8,9,10,11,19,20,21,22-octahydro-18aH,24H-18,1 5-(metheno)pyrido[2,1-l]pyrimido[6,1-h][1,4,7,9,10,13]benzoxapentaazacyclohexadecine-7,2 4(6H)-dione

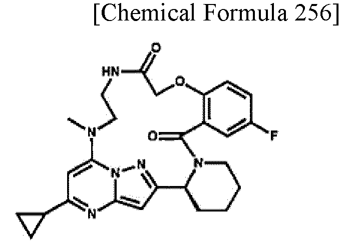

By using the same approaches as in Step 23-1, Step 1-2, Step 13-2, and Step 1-3, the title compound (0.12 g, colorless amorphous) was obtained from the compound (0.20 g) obtained in Reference Example 1-6 (2) and the compound (0.22 g) obtained in Reference Example 2-1 (3).
¹H NMR (400 MHz, CDCl₃) δ ppm 0.96 - 1.17 (m, 4H), 1.37 - 2.23 (m, 6.4H), 2.31 - 2.44 (m, 0.4H), 2.85 - 3.30 (m, 6.6H), 4.15 - 4.61 (m, 3H), 4.69 - 4.78 (m, 0.6H), 4.83 - 5.09 (m, 1.6H), 5.95 - 6.07 (m, 1H), 6.14 (s, 0.6H), 6.30-6.35 (m, 0.4H), 6.47 (s, 0.4H), 6.80 - 6.91 (m, 1H), 6.97 - 7.12 (m, 2H), 7.73 - 7.84 (m, 0.4H), 8.98 - 9.12 (m, 0.6H).
LCMS (ESI/APCI dual) m/z 493 [M+H]⁺, t_{R} = 0.535 min, mode N.

### Example 91: Synthesis of (18aS)-13-cyclopropyl-2-fluoro-8,9,10,11,19,20,21,22-octahydro-18aH,24H-18,15-(metheno )pyrido[2,1-l]pyrimido[6,1-h][1,4,7,9,10,13]benzoxapentaazacyclohexadecine-7,24(6H)-dion

e

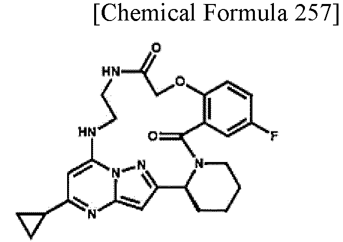

By using the same approaches as in Step 23-1, Step 1-2, Step 13-2, and Step 1-3, the title compound (0.22 g, colorless powder) was obtained from the compound (0.36 g) obtained in Reference Example 1-6 (2) and the compound (0.41 g) obtained in Reference Example 2-2.
LCMS (ESI/APCI dual) m/z 479 [M+H]⁺, t_{R} = 0.858 min, mode H.

### Example 92: Synthesis of (18aS)-13-cyclopropyl-11-ethyl-2-fluoro-8,9,10,11,19,20,21,22-octahydro-18aH,24H-18,15-( metheno)pyrido[2,1-l]pyrimido[6,1-h][1,4,7,9,10,13]benzoxapentaazacyclohexadecine-7,24( 6H)-dione

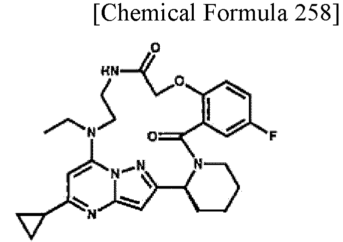

By using the same approaches as in Step 23-1, Step 1-2, Step 13-2, and Step 1-3, the title compound (40 mg, colorless powder) was obtained from the compound (92 mg) obtained in Reference Example 1-6 (2) and the compound (0.10 g) obtained in Reference Example 2-3.
LCMS (ESI) m/z 507 [M+H]⁺, t_{R} = 0.681 min, mode N.

### Example 93: Synthesis of (18aS)-13 -cyclopropyl-2-fluoro-11-(2-hydroxyethyl)-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione

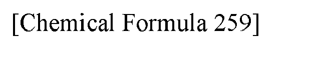

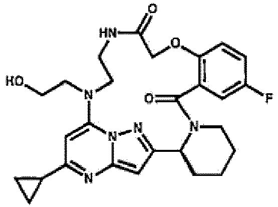

By using the same approaches as in Step 23-1, Step 1-2, Step 13-2, Step 1-3, and Step 4-2, the title compound (16 mg, colorless powder) was obtained from the compound (0.10 g) obtained in Reference Example 1-6 (2) and the compound (0.15 g) obtained in Reference Example 2-6.
LCMS (ESI) m/z 523 [M+H]⁺, t_{R} = 0.608 min, mode N.

### Example 94: Synthesis of (18aS)-13-cyclopropyl-2-fluoro-11-trideuteriomethyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione

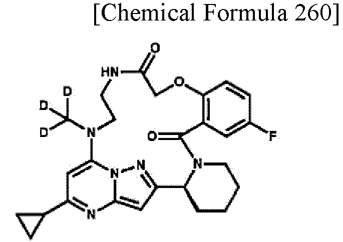

By using the same approaches as in Step 23-1, Step 1-2, Step 13-2, and Step 1-3, the title compound (0.25 g, colorless amorphous) was obtained from the compound (0.24 g) obtained in Reference Example 1-6 (2) and the compound (0.27 g) obtained in Reference Example 2-7.
LCMS (ESI/APCI dual) m/z 496 [M+H]⁺, t_{R} = 0.581 min, mode N.

### Example 95 and Example 96: Synthesis of (18aS)-13 -cyclopropyl-2-ethenyl-11-methyl-8,9,10,11,19,20,21,22-octahydro-18aH,24H-18, 15-(metheno)pyrido[2,1-l]pyrimido[6,1-h][1,4,7,9,10,13]benzoxapentaazacyclohexadecine-7, 24(6H)-dione (Compound A) and (18aS)-2-acetyl-13 -cyclopropyl-11-methyl-8,9,10,11,19,20,21,22-octahydro-18aH,24H-18,1 5-(metheno)pyrido[2,1-l]pyrimido[6,1-h][1,4,7,9,10,13]benzoxapentaazacyclohexadecine-7,2 4(6H)-dione (Compound B)

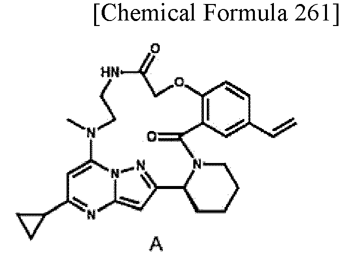

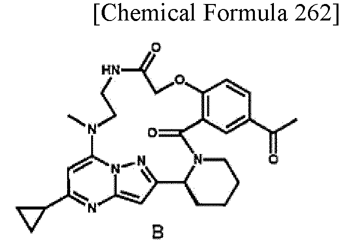

Step 95-1: By using the same approaches as in Step 23-1, Step 1-2, Step 13-2, and Step 1-3, (18aS)-13-cyclopropyl-2-(1-hydroxy ethyl)-11-methyl-8,9,10,11,19,20,21,22-octahydro-18aH ,24H-18,15-(metheno)pyrido[2,1-l]pyrimido[6,1-h][1,4,7,9,10,13]benzoxapentaazacyclohexa decine-7,24(6H)-dione (90 mg, colorless amorphous) was obtained from the compound (0.20 g) obtained in Reference Example 1-6 (2) and the compound (0.28 g) obtained in Reference Example 2-21.

Step 95-2: To a solution of the compound (90 mg) obtained in Step 95-1 in CHCl₃ (4.0 mL), manganese dioxide (0.15 g) was added, and the mixture was stirred at 65°C for 5 hours. The reaction solution was filtered through Celite (R), and then concentrated. The obtained residue was purified by OH type silica gel column chromatography (mobile phase: CHCl₃/MeOH = 100/0 to 90/10; v/v) to afford Example 95 title compound A (13 mg, colorless amorphous) and Example 96 title compound B (20 mg, colorless amorphous). Title compound A: LCMS (ESI/APCI dual) m/z 501 [M+H]⁺, t_{R} = 0.637 min, mode N. Title compound B: LCMS (ESI/APCI dual) m/z 517 [M+H]⁺, t_{R} = 0.499 min, mode N.

### Example 97: Synthesis of (18aS)-13-cyclopropyl-2-iodo-11-methyl-8,9,10,11,19,20,21,22-octahydro-18aH,24H-18,15-(metheno)pyrido[2,1-l]pyrimido[6,1-h][1,4,7,9,10,13]benzoxapentaazacyclohexadecine-7,24( 6H)-dione

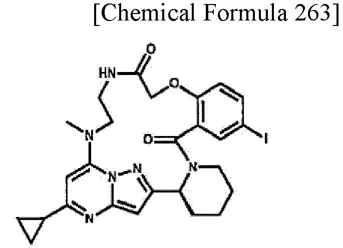

Step 97: By using the same approaches as in Step 23-1, Step 1-2, Step 13-2, and Step 1-3, the title compound (0.68 g, colorless amorphous) was obtained from the compound (0.54 g) obtained in Reference Example 1-6 (2) and the compound (0.91 g) obtained in Reference Example 2-19.
LCMS (ESI/APCI dual) m/z 601 [M+H]⁺, t_{R} = 0.683 min, mode N.

### Example 98: Synthesis of (18aS)-13 -cyclopropyl-11-methyl-7,24-dioxo-6,7,8,9,10,11,19,20,21,22-decahydro-18aH,24 H-18,15-(metheno)pyrido[2,1-l]pyrimido[6,1-h][1,4,7,9,10,13]benzoxapentaazacyclohexadec ine-2-carbonitrile

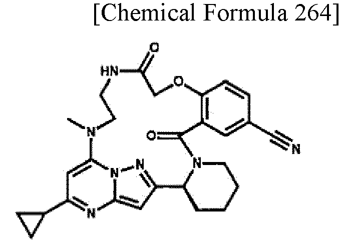

Step 98: By using the same approach as in Step 82-1, the title compound (25 mg, colorless amorphous) was obtained from the compound (0.10 g) obtained in Step 97. LCMS (ESI/APCI dual) m/z 500 [M+H]⁺, t_{R} = 0.525 min, mode N.

### Example 99: Synthesis of (18aS)-2,13-dicyclopropyl-11-methyl-8,9,10,11,19,20,21,22-octahydro-18aH,24H-18,15-(me theno)pyrido[2,1-1]pyrimido[6,1-h][1,4,7,9,10,13]benzoxapentaazacyclohexadecine-7,24(6H) -dione

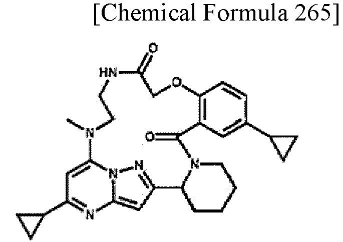

By using the same approach as in Step 30-2, the title compound (55 mg, colorless amorphous) was obtained from the compound (0.12 g) obtained in Step 97 and cyclopropylboronic acid (52 mg).
LCMS (ESI/APCI dual) m/z 515 [M+H]⁺, t_{R} = 0.688 min, mode N.

### Example 100: Synthesis of (18aS)-13-cyclopropyl-11-methyl-2-trideuteriomethyl-8,9,10,11,19,20,21,22-octahydro-18a H,24H-18,15-(metheno)pyrido[2,1-1]pyrimido[6,1-h][1,4,7,9,10,13]benzoxapentaazacyclohe xadecine-7,24(6H)-dione

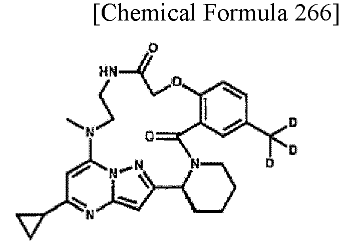

By using the same approach as in Step 53-2, the title compound (52 mg, colorless amorphous) was obtained from the compound (65 mg) obtained in Step 97.
¹H NMR (400 MHz, CDCl₃) δ ppm 0.92 - 1.19 (m, 4H), 1.40 - 2.18 (m, 6.4H), 2.29 - 2.41 (m, 0.4H), 2.85 - 3.35 (m, 6.6H), 4.13 - 4.61 (m, 3H), 4.72 - 4.81 (m, 0.6H), 4.84 - 5.07 (m, 1.6H), 5.91 - 6.06 (m, 1H), 6.09 - 6.19 (m, 0.6H), 6.28 - 6.39 (m, 0.4H), 6.41 - 6.50 (m, 0.4H), 6.70 - 6.83 (m, 1H), 7.05 - 7.19 (m, 2H), 7.76 - 7.88 (m, 0.4H), 8.98 - 9.12 (m, 0.6H). LCMS (ESI/APCI dual) m/z 492 [M+H]⁺, t_{R} = 0.595 min, mode N.

### Example 101: Synthesis of (18aS)-2-(aminom ethyl)-11-methyl-13-propyl-8,9,10,11,19,20,21,22-octahydro-18aH,24H-1 8, 15-(metheno)pyrido[2,1-1]pyrimido[6,1-h][1,4,7,9,10,13]benzoxapentaazacyclohexadecine-7,24(6H)-dione

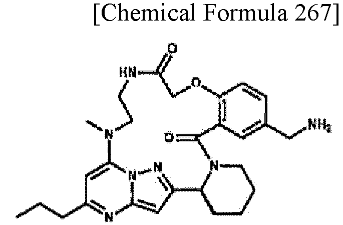

By using the same approach as in Step 50-2, the title compound (20 mg, colorless amorphous) was obtained from the compound (25 mg) obtained in Step 98.
LCMS (ESI/APCI dual) m/z 506 [M+H]⁺, t_{R} = 0.221 min, mode H.

### Example 102: Synthesis of (18aS)-13-cyclopropyl-2,11-dimethyl-8,9,10,11,19,20,21,22-octahydro-18aH,24H-18,15-(me theno)pyrido[2,1-1]pyrimido[6,1-h][1,4,7,9,10,13]benzoxapentaazacyclohexadecine-7,24(6H) -dione

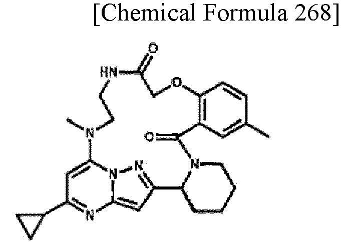

By using the same approaches as in Step 23-1, Step 1-2, Step 13-2, and Step 1-3, the title compound (0.16 g, colorless amorphous) was obtained from the compound (0.17 g) obtained in Reference Example 1-6 (2) and the compound (0.17 g) obtained in Reference Example 2-14.
LCMS (ESI/APCI dual) m/z 489 [M+H]⁺, t_{R} = 0.595 min, mode N.

### Example 103: Synthesis of (18aS)-2-chloro-13-cyclopropyl-11-methyl-8,9,10,11,19,20,21,22-octahydro-18aH,24H-18,1 5-(metheno)pyrido[2,1-l]pyrimido[6,1-h][1,4,7,9,10,13]benzoxapentaazacyclohexadecine-7,2 4(6H)-dione

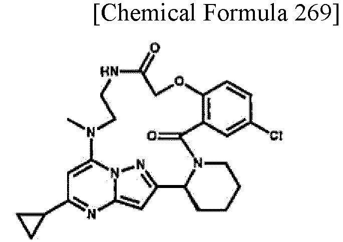

By using the same approaches as in Step 23-1, Step 1-2, Step 13-2, and Step 1-3, the title compound (0.14 g, colorless amorphous) was obtained from the compound (0.15 g) obtained in Reference Example 1-6 (2) and the compound (0.16 g) obtained in Reference Example 2-12.
LCMS (ESI/APCI dual) m/z 509 [M+H]⁺, t_{R} = 0.613 min, mode N.

### Example 104: Synthesis of (18aR)-13-cyclopropyl-2-fluoro-11-methyl-8,9,10,11,18a,19,21,22-octahydro-24H-18,15-(m etheno)[1,4]oxazino[3,4-l]pyrimido[6,1-h][1,4,7,9,10,13]benzoxapentaazacyclohexadecine-7, 24(6H)-dione

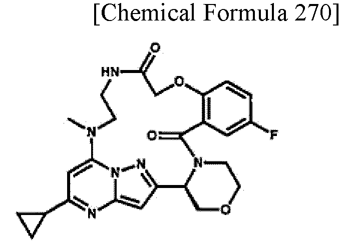

By using the same approaches as in Step 23-1, Step 1-2, Step 13-2, and Step 1-3, the title compound (90 mg, colorless amorphous) was obtained from the compound (0.19 g) obtained in Reference Example 1-8 and the compound (0.24 g) obtained in Reference

### Example 2-1 (3).

LCMS (ESI/APCI dual) m/z 495 [M+H]⁺, t_{R} = 0.801 min, mode H.

### Example 105: Synthesis of 13-cyclopropyl-2,20,20-trifluoro-11-methyl-8,9,10,11,19,20,21,22-octahydro-18aH,24H-18,1 5-(metheno)pyrido[2,1-1]pyrimido[6,1-h][1,4,7,9,10,13]benzoxapentaazacyclohexadecine-7,2 4(6H)-dione

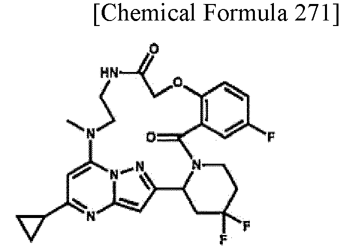

By using the same approaches as in Step 23-1, Step 1-2, Step 13-2, and Step 1-3, the title compound (0.33 g, colorless amorphous) was obtained from the compound (0.32 g) obtained in Reference Example 1-9 and the compound (0.40 g) obtained in Reference Example 2-1 (3).
LCMS (ESI/APCI dual) m/z 529 [M+H]⁺, t_{R} = 0.612 min, mode N.

### Example 106: Synthesis of (18S)-3-cyclopropyl-18-ethyl-14-fluoro-5,17-dimethyl-5,6,7,8,17,18-hexahydro-16H-19,1-( metheno)pyrimido[6,1-h][1,4,7,9,10,13]benzoxapentaazacyclohexadecine-9,16(10H)-dione

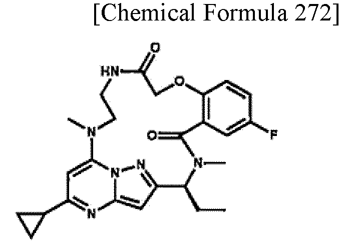

By using the same approaches as in Step 23-1, Step 1-2, Step 13-2, and Step 1-3, the title compound (0.25 g, colorless amorphous) was obtained from the compound (0.36 g) obtained in Reference Example 1-18 and the compound (0.44 g) obtained in Reference

### Example 2-1 (3).

LCMS (ESI/APCI dual) m/z 481 [M+H]⁺, t_{R} = 1.019 min, mode H.

### Example 107: Synthesis of (18S)-3-cyclopropyl-18-ethyl-14-fluoro-17-methyl-5,6,7,8,17,18-hexahydro-16H-19,1-(meth eno)pyrimido[6,1-h][1,4,7,9,10,13]benzoxapentaazacyclohexadecine-9,16(10H)-dione

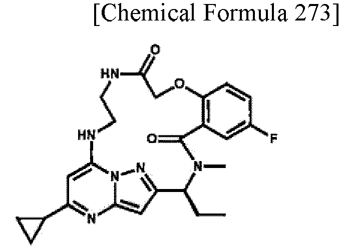

By using the same approaches as in Step 23-1, Step 1-2, Step 13-2, and Step 1-3, the title compound (0.22 g, colorless powder) was obtained from the compound (0.40 g) obtained in Reference Example 1-18 and the compound (0.44 g) obtained in Reference Example 2-2. LCMS (ESI/APCI dual) m/z 467 [M+H]⁺, t_{R} = 0.804 min, mode H.

### Example 108: Synthesis of (18aS)-13-(trans-3-aminocyclobutyl)-2-fluoro-11-methyl-8,9, 10,11,19,20,21,22-octahydro-1 8aH,24H-18,15-(metheno)pyrido[2,1-1]pyrimido[6,1-h][1,4,7,9,10,13]benzoxapentaazacyclo hexadecine-7,24(6H)-dione and (18aS)-13-(cis-3-aminocyclobutyl)-2-fluoro-11-methyl-8,9,10,11,19,20,21,22-octahydro-18a H,24H-18,15-(metheno)pyrido[2,1-1]pyrimido[6,1-h][1,4,7,9,10,13]benzoxapentaazacyclohe xadecine-7,24(6H)-dione (Example 108t and Example 108c)

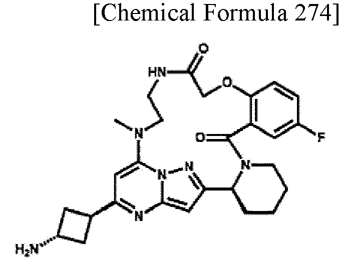

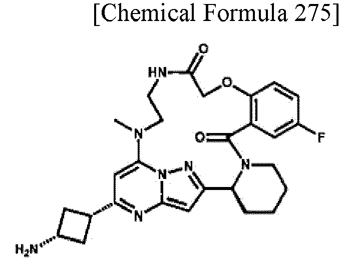

Step 108-1: By using the same approaches as in Step 21-1 and Step 4-2, (18aS)-13-(3-aminocyclobutyl)-2-fluoro-11-methyl-8,9,10,11,19,20,21,22-octahydro-18aH,2 4H-18,15-(metheno)pyrido[2,1-l]pyrimido[6,1-h][1,4,7,9,10,13]benzoxapentaazacyclohexade cine-7,24(6H)-dione (48 mg, brown amorphous) was obtained from the compound (0.10 g) obtained in Step 1-3 and tert-butyl N-(3-iodocyclobutyl)carbamate (0.31 g).

Step 108-2: The compound (cis/trans = 3/2, 32 mg) obtained in Step 108-1 was preparatively purified by chiral column chromatography (preparative conditions: column: CHIRALPAK OD (Daicel trademark), column size: 20 mm × 250 mm, mobile phase: hexane/EtOH = 50/50 (v/v), flow rate: 10 mL/min, column temperature: 25°C, detection wavelength: 210 nm) to afford the title compound (Example 108t) (trans form: 10 mg, colorless powder) and the title compound (Example 108c) (cis form: 15 mg, colorless powder). trans form: LCMS (ESI) m/z 522 [M+H]⁺, t_{R} = 0.243 min, mode N. cis form: LCMS (ESI) m/z 522 [M+H]⁺, t_{R} = 0.233 min, mode N.

### Example 109: Synthesis of (18aS)-2-fluoro-13-(cis-3-hydroxycyclobutyl)-11-methyl-8,9,10,11,19,20,21,22-octahydro-1 8aH,24H-18,15-(metheno)pyrido[2,1-l]pyrimido[6,1-h][1,4,7,9,10,13]benzoxapentaazacyclo hexadecine-7,24(6H)-dione

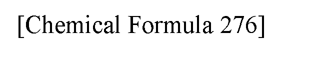

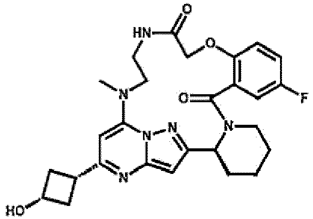

Step 109: By using the same approaches as in Step 23-1, Step 1-2, Step 13-2, Step 1-3, and Step 50-2, the title compound (33 mg, colorless amorphous) was obtained from the compound (80 mg) obtained in Reference Example 1-12 and the compound (72 mg) obtained in Reference Example 2-1 (3).
LCMS (ESI) m/z 523 [M+H]⁺, t_{R} = 0.488 min, mode N.

### Example 110: Synthesis of (18aS)-2-fluoro-13-(trans-3-hydroxycyclobutyl)-11-methyl-8,9,10,11,19,20,21,22-octahydro-18aH,24H-18,15-(metheno)pyrido[2,1-l]pyrimido[6,1-h][1,4,7,9,10,13]benzoxapentaazacycl ohexadecine-7,24(6H)-dione

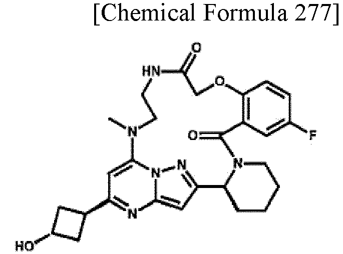

Step 110-1: To a solution of the compound (55 mg) obtained in Step 109 in THF (1.1 mL), 4-nitrobenzoic acid (26 mg), DEAD (0.29 mL, 2.2 M toluene solution), and PPh₃ (0.17 g) were added, and the mixture was stirred at 65°C for 0.5 hours. After allowing it to be cooled to room temperature, the reaction liquid was concentrated. The obtained residue was purified by NH type silica gel chromatography (mobile phase: hexane/EtOAc = 50/50 to 0/100; v/v) to afford trans-3-[(18aS)-2-fluoro-11-methyl-7,24-dioxo-6,7,8,9,10,11,19,20,21,22-decahydro-18aH,2 4H-18,15-(metheno)pyrido[2,1-1]pyrimido[6,1-h][1,4,7,9,10,13]benzoxapentaazacyclohexade cin-13-yl]cyclobutyl 4-nitrobenzoate (74 mg, colorless amorphous).
LCMS (ESI) m/z 672 [M+H]⁺, t_{R} = 1.030 min, mode N.

Step 110-2: By using the same approach as in Step 1-2, the title compound (41 mg, colorless amorphous) was obtained from the compound (73 mg) obtained in Step 110-1. LCMS (ESI) m/z 523 [M+H]⁺, t_{R} = 0.498 min, mode N.

### Example 111: Synthesis of (18aS)-2-chloro-13-(cis-3-hydroxycyclobutyl)-11-methyl-8,9,10,11,19,20,21,22-octahydro-1 8aH,24H-18,15-(metheno)pyrido[2,1-1]pyrimido[6,1-h][1,4,7,9,10,13]benzoxapentaazacyclo hexadecine-7,24(6H)-dione

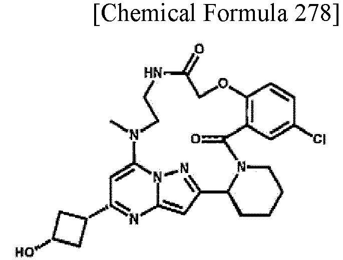

Step 111: By using the same approaches as in Step 23-1, Step 1-2, Step 13-2, Step 1-3, and Step 62-2, the title compound (12 mg, colorless amorphous) was obtained from the compound (0.12 g) obtained in Reference Example 1-24 and the compound (0.13 g) obtained in Reference Example 2-12.
LCMS (ESI) m/z 539 [M+H]⁺, t_{R} = 0.567 min, mode N.

### Example 112: Synthesis of (18aS)-2-chloro-13-(trans-3-hydroxycyclobutyl)-11-methyl-8,9,10,11,19,20,21,22-octahydro -18aH,24H-18,15-(metheno)pyrido[2,1-1]pyrimido[6,1-h][1,4,7,9,10,13]benzoxapentaazacycl ohexadecine-7,24(6H)-dione

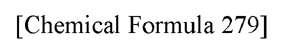

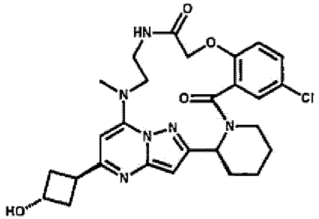

By using the same approaches as in Step 110-1 and Step 1-2, the title compound (16 mg, colorless amorphous) was obtained from the compound (31 mg) obtained in Step 111.
¹H NMR (400 MHz, CDCl₃) δ ppm 0.78 - 2.25 (m, 6.4H), 2.31 - 2.44 (m, 2H), 2.63 - 2.76 (m, 2H), 2.87 - 3.35 (m, 6.6H), 3.53 - 3.64 (m, 1H), 4.15 - 5.07 (m, 6.6H), 5.90 - 6.03 (m, 1H), 6.27 (s, 0.6H), 6.34 (s, 0.4H), 6.58 (s, 0.4H), 6.78 - 6.86 (m, 1H), 7.31 (d, J = 9.2 Hz, 3H), 7.72 - 7.85 (m, 0.4H), 8.83 - 8.90 (m, 0.6H).
LCMS (ESI) m/z 539 [M+H]⁺, t_{R} = 0.535 min, mode N.

### Example 113: Synthesis of (18aS)-2-chloro-13-(cis-3-hydroxycyclobutyl)-8-methyl-8,9,10,11,19,20,21,22-octahydro-18 aH,24H-18,15-(metheno)pyrido[2,1-1]pyrimido[6,1-h][1,4,7,9,10,13]benzoxapentaazacycloh exadecine-7,24(6H)-dione

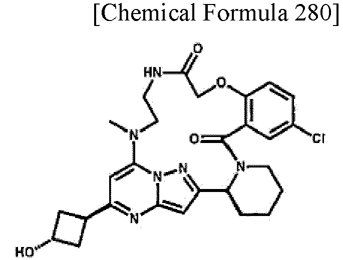

By using the same approaches as in Step 23-1, Step 50-2, Step 1-3, Step 1-2, Step 13-2, and Step 1-3, the title compound (68 mg, colorless amorphous) was obtained from the compound (0.51 g) obtained in Reference Example 1-12, benzyl
N-[2-(methylamino)ethyl]carboxylate hydrochloride (0.35 mg), and
2-(4-chloro-2-methoxycarbonyl-phenoxy)acetic acid (0.17 g).
LCMS (ESI) m/z 539 [M+H]⁺, t_{R} = 0.553 min, mode N.

### Example 114: Synthesis of (18aS)-2-chloro-13-(cis-3-hydroxycyclobutyl)-11-trideuteriomethyl-8,9,10,11,19,20,21,22-oc tahydro-18aH,24H-18, 15-(metheno)pyrido[2,1-1]pyrimido[6,1-h][1,4,7,9,10,13]benzoxapenta azacyclohexadecine-7,24(6H)-dione

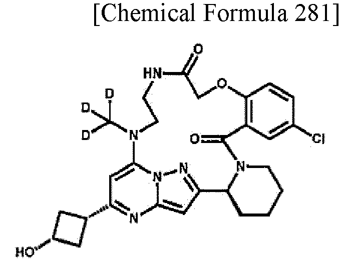

Step 114-1: By using the same approaches as in Step 1-1, Step 1-2, Step 13-2, and Step 1-3, (18aS)-13-[3-(benzyloxy)cyclobutyl]-2-chloro-11-trideuteriomethyl-8,9,10,11,19,20,21,22-o ctahydro-18aH,24H-18,15-(metheno)pyrido[2,1-1]pyrimido[6,1-h][1,4,7,9,10,13]benzoxapent aazacyclohexadecine-7,24(6H)-dione (0.45 g, colorless amorphous) was obtained from the compound (0.50 g) obtained in Reference Example 1-12 and the compound (0.51 g) obtained in Reference Example 2-13.
LCMS (ESI/APCI dual) m/z 632 [M+H]⁺, t_{R} = 0.817 min, mode N.

Step 114-2: To a solution of the compound (0.45 g) obtained in Step 114-1 in MeCN (7.1 mL), TMSCl (0.45 mL) and NaI (0.53 g) were added, and the mixture was stirred at 65°C for 1 hour. After allowing the reaction liquid to be cooled to room temperature, a Na₂S₂O₃ aq. was added thereto. The aqueous layer was extracted with CHCl₃. The organic layer was dried over MgSO₄ and then concentrated under reduced pressure. The obtained residue was purified by NH type silica gel chromatography (mobile phase: EtOAc/MeOH = 100/0 to 90/10; v/v). (18aS)-2-Chloro-13-(3-hydroxycyclobutyl)-11-trideuteriomethyl-8,9,10,11,19,20,21,22-octah ydro-18aH,24H-18,15-(metheno)pyrido[2,1-1]pyrimido[6,1-h][1,4,7,9,10,13]benzoxapentaaz acyclohexadecine-7,24(6H)-dione (0.40 g, colorless amorphous) was obtained.
LCMS (ESI/APCI dual) m/z 542 [M+H]⁺, t_{R} = 0.837 min, mode H.

Step 114-3: To a solution of the compound (0.15 g) obtained in Step 114-2 in CHCl₃ (1.0 mL), the Dess-Martin reagent (R) (0.15 g) was added, and the mixture was stirred at room temperature for 0.5 hours. To the reaction liquid, a Sat. NaHCO₃ aq. was added. The aqueous layer was extracted with CHCl₃. The organic layer was collected by using Phase Separator and concentrated. The obtained residue was purified by OH type silica gel chromatography (mobile phase: CHCl₃/MeOH = 100/0 to 90/10; v/v) to afford (18aS)-2-chloro-11-trideuteriomethyl-13-(3-oxocyclobutyl)-8,9,10,11,19,20,21,22-octahydro -18aH,24H-18,15-(metheno)pyrido[2,1-1]pyrimido[6,1-h][1,4,7,9,10,13]benzoxapentaazacycl ohexadecine-7,24(6H)-dione (0.15 g, colorless amorphous).
LCMS (ESI/APCI dual) m/z 540 [M+H]⁺, t_{R} = 0.668 min, mode N.

Step 114-4: To a solution of the compound (0.15 g) obtained in Step 114-3 in THF (2.8 mL), L-Selectride (R) (0.83 mL, 1.0 M in THF) was added at -78°C, and the mixture was stirred for 1 hour. After raising the temperature to 0°C, the mixture was stirred for 0.5 hours. To the reaction liquid, a Sat. NaHCO₃ aq. was added. The aqueous layer was extracted with CHCl₃. The organic layer was collected by using Phase Separator and concentrated. The obtained residue was purified by OH type silica gel chromatography (mobile phase: CHCl₃/MeOH = 100/0 to 90/10; v/v) to afford the title compound (0.13 g, colorless amorphous).
LCMS (ESI/APCI dual) m/z 542 [M+H]⁺, t_{R} = 0.829 min, mode H.

### Example 115: Synthesis of (18aS)-2-chloro-13-(trans-3-hydroxycyclobutyl)-11-trideuteriomethyl-8,9,10,11,19,20,21,22-octahydro-18aH,24H-18,15-(metheno)pyrido[2,1-l]pyrimido[6,1-h][1,4,7,9,10,13]benzoxape ntaazacyclohexadecine-7,24(6H)-dione

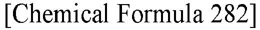

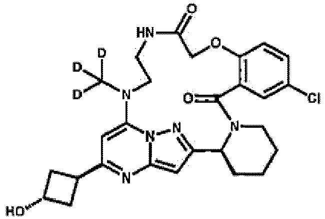

By using the same approaches as in Step 110-1 and Step 1-2, the title compound (58 mg, colorless amorphous) was obtained from the compound (0.11 g) obtained in Step 114-4.
¹H NMR (400 MHz, CDCl₃) δ ppm 1.41 - 2.24 (m, 5.4H), 2.31 - 2.48 (m, 2.4H), 2.62 - 2.79 (m, 2H), 2.89 - 3.33 (m, 3.6H), 3.52 - 3.68 (m, 1H), 4.15 - 4.80 (m, 4.6H), 4.86 - 5.07 (m, 1.6H), 5.89 - 6.01 (m, 1H), 6.27 (s, 0.6H), 6.31 - 6.39 (m, 0.4H), 6.58 (s, 0.4H), 6.77 - 6.87 (m, 1H), 7.21 - 7.36 (m, 2H), 7.72 - 7.83 (m, 0.4H), 8.80 - 8.93 (m, 0.6H). LCMS (ESI/APCI dual) m/z 542 [M+H]⁺, t_{R} = 0.239 min, mode N.

### Example 116: Synthesis of (18aS)-13-(cis-3-hydroxycyclobutyl)-2,11-dimethyl-8,9,10,11,19,20,21,22-octahydro-18aH,2 4H-18,15-(metheno)pyrido[2,1-1]pyrimido[6,1-h][1,4,7,9,10,13]benzoxapentaazacyclohexade cine-7,24(6H)-dione

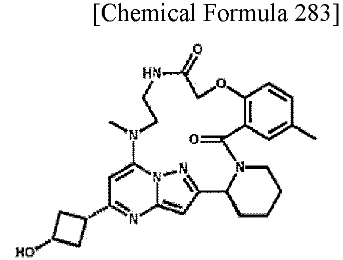

Step 116: By using the same approaches as in Step 23-1, Step 1-2, Step 13-2, Step 1-3, and Step 50-2, the title compound (29 mg, colorless amorphous) was obtained from the compound (71 mg) obtained in Reference Example 1-12 and the compound (63 mg) obtained in Reference Example 2-14.
LCMS (ESI) m/z 519 [M+H]⁺, t_{R} = 0.517 min, mode N.

### Example 117: Synthesis of (18aS)-13-(trans-3-hydroxycyclobutyl)-2,11-dimethyl-8,9,10,11,19,20,21,22-octahydro-18aH ,24H-18,15-(metheno)pyrido[2,1-1]pyrimido[6,1-h][1,4,7,9,10,13]benzoxapentaazacyclohexa decine-7,24(6H)-dione

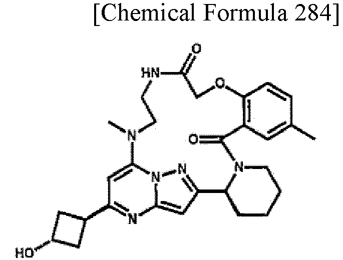

By using the same approaches as in Step 110-1 and Step 1-2, the title compound (94 mg, colorless amorphous) was obtained from the compound (99 mg) obtained in Step 116.
¹H NMR (400 MHz, CDCl₃) δ ppm 1.18 - 2.19 (m, 6.4H), 2.27 - 2.43 (m, 5H), 2.57 - 2.80 (m, 2H), 2.85 - 3.39 (m, 6.6H), 3.52 - 3.64 (m, 1H), 4.04 - 5.20 (m, 6.6H), 5.89 - 6.01 (m, 1H), 6.25 (s, 0.6H), 6.38 (s, 0.4H), 6.57 (s, 0.4H), 6.72 - 6.82 (m, 1H), 7.07 - 7.18 (m, 2H), 7.78 - 7.89 (m, 0.4H), 8.94 - 9.05 (m, 0.6H).
LCMS (ESI) m/z 519 [M+H]⁺, t_{R} = 0.526 min, mode N.

### Example 118: Synthesis of (18aS)-13-(cis-3-hydroxycyclobutyl)-11-methyl-2-trideuteriomethyl-8,9,10,11,19,20,21,22-o ctahydro-18aH,24H-18,15-(metheno)pyrido[2,1-1]pyrimido[6,1-h][1,4,7,9,10,13]benzoxapent aazacyclohexadecine-7,24(6H)-dione and (18aS)-13-(trans-3-hydroxycyclobutyl)-11-methyl-2-trideuteriomethyl-8,9,10,11,19,20,21,22 -octahydro-18aH,24H-18,15-(metheno)pyrido[2,1-l]pyrimido[6,1-h][1,4,7,9,10,13]benzoxap entaazacyclohexadecine-7,24(6H)-dione (Example 118c and Example 118t)

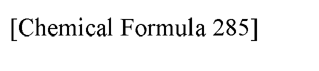

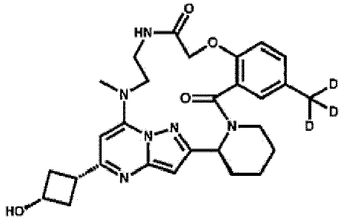

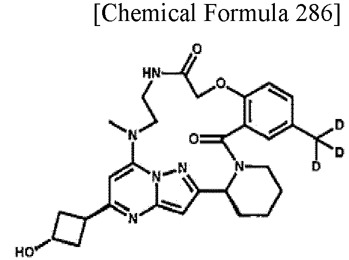

Step 118-1: By using the same approaches as in Step 23-1, Step 1-2, Step 13-2, Step 1-3, Step 53-2, and Step 50-2, (18aS)-13-(3-hydroxycyclobutyl)-11-methyl-2-trideuteriomethyl-8,9,10,11,19,20,21,22-octah ydro-18aH,24H-18,15-(metheno)pyrido[2,1-l]pyrimido[6,1-h][1,4,7,9,10,13]benzoxapentaaz acyclohexadecine-7,24(6H)-dione (0.13 g, colorless amorphous) was obtained from the compound (0.82 g) obtained in Reference Example 1-12 and the compound (0.99 g) obtained in Reference Example 2-19.

Step 118-2: The compound (87 mg) obtained in Step 118-1 was preparatively purified by chiral column chromatography (preparative conditions: SFC method; column: CHIRALCEL OD (Daicel trademark), column size: 20 mm × 250 mm, mobile phase: MeOH/CO₂ = 25/75 (v/v), flow rate: 30 mL/min, column temperature: 40°C, detection wavelength: 210 nm) to afford the title compound (Example 118c) (cis form: 49 mg, colorless powder) and the title compound (Example 118t) (trans form: 15 mg, colorless powder). cis form: LCMS (ESI) m/z 522 [M+H]⁺, t_{R} = 0.520 min, mode N. trans form:
¹H NMR (400 MHz, CDCl₃) δ ppm 1.39 - 2.23 (m, 6.4H), 2.31 - 2.46 (m, 2H), 2.63 - 2.79 (m, 2H), 2.86 - 3.36 (m, 6.6H), 3.48 - 3.65 (m, 1H), 4.15 - 5.11 (m, 6.6H), 5.90 - 6.00 (m, 1H), 6.25 (s, 0.6H), 6.37 (s, 0.4H), 6.57 (s, 0.4H), 6.75-6.81 (m, 1H), 7.08 - 7.17 (m, 2H), 7.68 - 7.89 (m, 0.4H), 8.96 - 9.04 (m, 0.6H).
LCMS (ESI) m/z 522 [M+H]⁺, t_{R} = 0.526 min, mode N.

### Example 119: Synthesis of (18aS)-13-(cis-3-hydroxycyclobutyl)-2,11-bis[trideuteriomethyl]-8,9,10,11,19,20,21,22-octa hydro-18aH,24H-18,15-(metheno)pyrido[2,1-l]pyrimido[6,1-h][1,4,7,9,10,13]benzoxapentaa zacyclohexadecine-7,24(6H)-dione

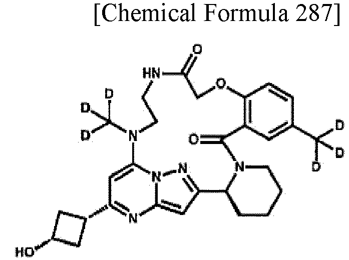

Step 119: By using the same approaches as in Step 23-1, Step 1-2, Step 13-2, Step 1-3, Step 50-2, Step 114-3, and Step 114-4, the title compound (0.18 g, colorless powder) was obtained from the compound (0.40 g) obtained in Reference Example 1-12 and the compound (0.36 g) obtained in Reference Example 2-16.
LCMS (ESI) m/z 525 [M+H]⁺, t_{R} = 0.490 min, mode N.

### Example 120: Synthesis of (18aS)-13-(trans-3-hydroxycyclobutyl)-2,11-bis[trideuteriomethyl]-8,9,10,11,19,20,21,22-oct ahydro-18aH,24H-18,15-(metheno)pyrido[2,1-1]pyrimido[6,1-h][1,4,7,9,10,13]benzoxapenta azacyclohexadecine-7,24(6H)-dione

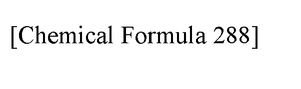

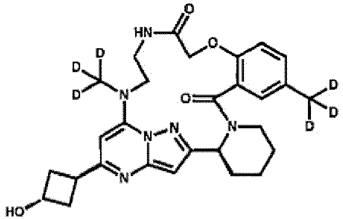

By using the same approaches as in Step 110-1 and Step 1-2, the title compound (94 mg, colorless powder) was obtained from the compound (0.15 g) obtained in Step 119. ¹H NMR (400 MHz, CDCl₃) δ ppm 1.61 - 2.21 (m, 6H), 2.30 - 2.46 (m, 2.4H), 2.61 - 2.78 (m, 2H), 2.84 - 3.36 (m, 3.6H), 3.51 - 3.67 (m, 1H), 4.16 - 4.31 (m, 1.4H), 4.31 - 4.71 (m, 3H), 4.71 - 4.85 (m, 0.6H), 4.85 - 5.13 (m, 1.6H), 5.92 (s, 0.6H), 5.97 (s, 0.4H), 6.24 (s, 0.6H), 6.38 (br s, 0.4H), 6.57 (s, 0.4H), 6.73 - 6.81 (m, 1H), 7.06 - 7.18 (m, 2H), 7.78 - 7.87 (m, 0.4H), 8.94 - 9.04 (m, 0.6H).
LCMS (ESI) m/z 525 [M+H]⁺, t_{R} = 0.487 min, mode N.

### Example 121: Synthesis of (18aS)-13-cyclopropyl-2-fluoro-8,9,10,11,19,20,21,22-octahydro-18aH,24H-18,15-(metheno )pyrido[2,1-l]pyrimido[6,1-h][1,4,9,10,13]benzoxatetraazacyclohexadecine-7,24(6H)-dione

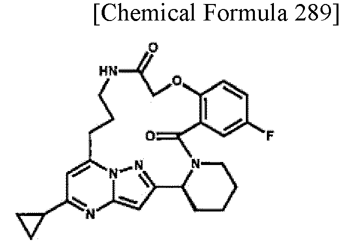

Step 121-1: By using the same approach as in Step 31-1, tert-butyl (2S)-2-[7-[(E)-3-[tert-butyl(dimethyl)silyl]oxyprop-1-enyl]-5-cyclopropyl-pyrazolo[1,5-a]pyr imidin-2-yl]piperidine-1-carboxylate (0.73 g, yellow oily matter) was obtained from the compound (0.71 g) obtained in Reference Example 1-6 (2) and tert-butyl(dimethyl){[(2E)-3-(4,4,5,5-tetramethyl-1,3,2-dioxaborolan-2-yl)prop-2-en-1-yl]ox y} silane (0.78 g).
¹H NMR (400 MHz, CDCl₃) δ ppm 0.14 (s, 6H), 0.97 (s, 9H), 1.02 - 1.09 (m, 2H), 1.11 - 1.16 (m, 2H), 1.43 - 1.67 (m, 13H), 1.78 - 1.93 (m, 1H), 1.99 - 2.10 (m, 1H), 2.40 - 2.52 (m, 1H), 2.83 - 2.98 (m, 1H), 3.98 - 4.09 (m, 1H), 4.50 (br s, 2H), 5.58 (br s, 1H), 6.29 (s, 1H), 6.61 (s, 1H), 7.15 (d, J = 16.0 Hz, 1H), 7.45 (d, J = 16.0 Hz, 1H).

Step 121-2: The compound (0.57 g) obtained in Step 121-1 was dissolved in MeOH (11 mL), palladium-activated carbon ethylenediamine complex (57 mg) was added thereto, and the mixture was stirred at room temperature for 45 minutes under a hydrogen gas atmosphere. After nitrogen purge, the reaction liquid was filtered through Celite (R) and washed with CHCl₃, and the resulting filtrate was concentrated under reduced pressure. The obtained residue was purified by OH type silica gel column chromatography (mobile phase: hexane/EtOAc = 100/0 to 80/20; v/v) to afford tert-butyl (2S)-2-[7-[3-[tert-butyl(dimethyl)silyl]oxypropyl]-5-cyclopropyl-pyrazolo[1,5-a]pyrimidin-2 -yl]piperidine-1-carboxylate (0.42 g, colorless oily matter).
¹H NMR (400 MHz, CDCl₃) δ ppm 0.02 - 0.10 (m, 6H), 0.86 - 0.96 (m, 9H), 0.96 - 1.05 (m, 4H), 1.38 - 1.68 (m, 10H), 1.71 - 1.93 (m, 3H), 1.99 - 2.13 (m, 2H), 2.40 - 2.52 (m, 1H), 2.69 - 2.78 (m, 2H), 2.85 - 3.01 (m, 1H), 3.11 - 3.22 (m, 2H), 3.67 - 3.76 (m, 2H), 3.98 - 4.12 (m, 1H), 5.59 (br s, 1H), 6.34 (s, 1H), 6.48 (s, 1H).

Step 121-3: To a solution of the compound (0.43 g) obtained in Step 121-2 in THF (4.2 mL), TBAF (1.7 mL, 1 M THF solution) was added under ice cooling, and the mixture was stirred at room temperature for 1 hour. To the reaction solution, a Sat. NaHCO₃ aq. and CHCl₃ were added, and the separating operation was carried out. The aqueous layer was separated using Phase Separator, and then the organic layer was concentrated under reduced pressure. The obtained residue was purified by OH type silica gel column chromatography (mobile phase: hexane/EtOAc = 80/20 to 30/70; v/v) to afford tert-butyl (2S)-2-[5-cyclopropyl-7-(3-hydroxypropyl)pyrazolo[1,5-a]pyrimidin-2-yl]piperidine-1-carbo xylate (0.32 g, colorless oily matter).
LCMS (ESI) m/z 401 [M+H]⁺, t_{R} = 1.145 min, mode N.

Step 121-4: By using the same approach as in Step 110-1, tert-butyl (2S)-2-[5-cyclopropyl-7-[3-(1,3-dioxoisoindolin-2-yl)propyl]pyrazolo[1,5-a]pyrimidin-2-yl]p iperidine-1-carboxylate (0.40 g, colorless oily matter) was obtained from the compound (0.32 g) obtained in Step 121-3 and phthalimide (0.18 g).
LCMS (ESI) m/z 530 [M+H]⁺, t_{R} = 1.399 min, mode N.

Step 121-5: To a solution of the compound (0.40 g) obtained in Step 121-4 in EtOH (2.0 mL), hydrazine monohydrate (0.11 mL) was added, and the mixture was stirred at 65°C for 2 hours. After cooling it to room temperature, insolubles were filtered off and the filtrate was concentrated under reduced pressure. The obtained residue was purified by NH type silica gel chromatography (mobile phase: EtOAc → CHCl₃/MeOH = 97/3; v/v) to afford tert-butyl
(2S)-2-[7-(3-aminopropyl)-5-cyclopropyl-pyrazolo[1,5-a]pyrimidin-2-yl]piperidine-1-carbox ylate (0.27 g, colorless oily matter).
LCMS (ESI) m/z 400 [M+H]⁺, t_{R} = 0.682 min, mode N.

Step 121-6: To a solution of the compound (0.17 g) obtained in Step 121-5 and 2-(2-tert-butoxycarbonyl-4-fluoro-phenoxy)acetic acid (0.12 g) in DMF (2.1 mL), Et₃N (0.58 mL) and HATU (0.24 g) were added, and the mixture was stirred at room temperature for 15 hours. The reaction liquid was diluted with EtOAc, and washed with water and a Sat. NaHCO₃ aq. The organic layer was dried over MgSO₄ and then concentrated under reduced pressure. The obtained residue was purified by OH type silica gel chromatography (mobile phase: hexane/EtOAc = 50/50 to 0/100; v/v) to afford tert-butyl (2S)-2-[7-[3-[[2-(2-tert-butoxycarbonyl-4-fluoro-phenoxy)acetyl]amino]propyl]-5-cycloprop yl-pyrazolo[1,5-a]pyrimidin-2-yl]piperidine-1-carboxylate (0.17 g, colorless powder). LCMS (ESI) m/z 652 [M+H]⁺, t_{R} = 1.494 min, mode N.

Step 121-7: By using the same approaches as in Step 13-2 and Step 1-3, the title compound (52 mg, colorless powder) was obtained from the compound (90 mg) obtained in Step 121-6.
LCMS (ESI) m/z 478 [M+H]⁺, t_{R} = 1.036 min, mode N.

### Example 122: Synthesis of (18aS)-13-cyclopropyl-2-fluoro-11-methyl-8,9,10,11,19,20,21,22-octahydro-18aH,24H-18,1 5-(metheno)pyrido[2,1-l]pyrimido[6,1-h][1,4,9,10,13]benzoxatetraazacyclohexadecine-7,24( 6H)-dione (Example 122a and Example 122b)

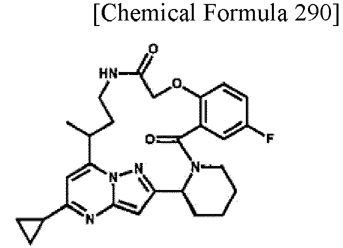

Step 122-1: By using the same approaches as in Step 121-1, Step 4-2, and Step 121-5, tert-butyl (2S)-2-[7-(3-amino-1-methyl-propyl)-5-cyclopropyl-pyrazolo[1,5-a]pyrimidin-2-yl]piperidin e-1-carboxylate (23 mg, light yellow oily matter) was obtained from the compound (92 mg) obtained in Reference Example 1-6 (2) and 2-[(Z)-3-(4,4,5,5-tetramethyl-1,3,2-dioxaborolan-2-yl)but-2-enyl]isoindoline-1,3-dione (0.11 g).
LCMS (ESI) m/z 414 [M+H]⁺, t_{R} = 0.539 min, mode L.

Step 122-2: By using the same approaches as in Step 121-6, Step 13-2, and Step 1-3, (18aS)-13-cyclopropyl-2-fluoro-11-methyl-8,9,10,11,19,20,21,22-octahydro-18aH,24H-18,1 5-(metheno)pyrido[2,1-1]pyrimido[6,1-h][1,4,9,10,13]benzoxatetraazacyclohexadecine-7,24( 6H)-dione (11 mg, light yellow amorphous) was obtained from the compound (21 mg) obtained in Step 122-1.

Step 122-3: The compound (3.0 mg) obtained in Step 122-2 was preparatively purified by chiral column chromatography (preparative conditions: column: CHIRALPAK AD-H (Daicel trademark), column size: 20 mm × 250 mm, mobile phase: hexane/IPA = 20/80 (v/v), flow rate: 10 mL/min) to afford Diastereomer A of the title compound (Example 122a) (0.58 mg, colorless oily matter) and Diastereomer B of the title compound (Example 122b) (1.3 mg, colorless oily matter).
Diastereomer A: LCMS (ESI) m/z 492 [M+H]⁺, t_{R} = 1.052 min, mode N.
Diastereomer B: LCMS (ESI) m/z 492 [M+H]⁺, t_{R} = 1.113 min, mode N.

### Example 123: Synthesis of (10E,18aS)-2-chloro-13-cyclopropyl-11-methyl-8,9,19,20,21,22-hexahydro-18aH,24H-18,15 -(metheno)pyrido[2,1-l]pyrimido[6,1-h][1,4,9,10,13]benzoxatetraazacyclohexadecine-7,24(6 H)-dione

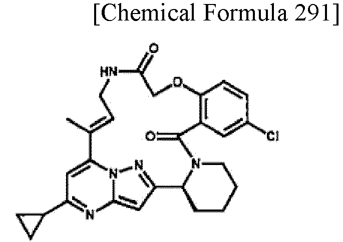

By using the same approaches as in Step 121-1, Step 121-5, Step 121-6, Step 1-2, Step 13-2, and Step 1-3, the title compound (7.4 mg, light yellow powder) was obtained from the compound (0.44 g) obtained in Reference Example 1-6 (2), 2-[(Z)-3-(4,4,5,5-tetramethyl-1,3,2-dioxaborolan-2-yl)but-2-enyl]isoindoline-1,3-dione (0.53 g), and 2-(4-chloro-2-methoxycarbonyl-phenoxy)acetic acid (35 mg).
LCMS (ESI) m/z 506 [M+H]⁺, t_{R} = 1.122 min, mode N.

### Example 124: Synthesis of (18aS)-2-chloro-13-cyclopropyl-11-methyl-8,9,10,11,19,20,21,22-octahydro-18aH,24H-18,1 5-(metheno)pyrido[2,1-l]pyrimido[6,1-h][1,4,9,10,13]benzoxatetraazacyclohexadecine-7,24( 6H)-dione (Example 124a and Example 124b)

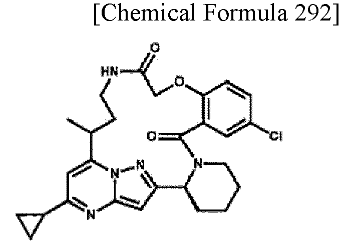

By using the same approaches as in Step 121-6, Step 1-2, Step 13-2, and Step 1-3,
Diastereomer A of the title compound (Example 124a) (17 mg, colorless powder) and
Diastereomer B of the title compound (Example 124b) (12 mg, colorless powder) were obtained from the compound (62 mg) obtained in Step 122-1 and 2-(4-chloro-2-methoxycarbonyl-phenoxy)acetic acid (40 mg).
Diastereomer A:
   ¹H NMR (400 MHz, CDCl₃) δ ppm 1.07 - 1.24 (m, 4H), 1.40 (d, J = 7.0 Hz, 3H), 1.62 - 1.89 (m, 4.4H), 1.91 - 2.18 (m, 4H), 2.38 - 2.51 (m, 1H), 2.74 - 2.84 (m, 0.6H), 3.03 - 3.19 (m, 1H), 3.39 - 3.50 (m, 1H), 3.86 - 3.98 (m, 1H), 4.07 - 4.20 (m, 1H), 4.44 - 4.53 (m, 1H), 4.56 - 4.64 (m, 1H), 6.29 - 6.37 (m, 1H), 6.46 - 6.61 (m, 2H), 6.84 (d, J = 8.6 Hz, 1H), 7.37 (d, J = 8.6 Hz, 1H), 7.47 (s, 1H), 7.96 - 8.09 (m, 1H).
   LCMS (ESI) m/z 508 [M+H]⁺, t_{R} = 1.120 min, mode N.
Diastereomer B:
   ¹H NMR (400 MHz, CDCl₃) δ ppm 1.07 - 1.13 (m, 2H), 1.14 - 1.22 (m, 2H), 1.39 - 1.59 (m, 5H), 1.68 - 1.89 (m, 2.4H), 1.97 - 2.14 (m, 3.6H), 2.24 - 2.32 (m, 1H), 2.34 - 2.45 (m, 1H), 3.38 - 3.48 (m, 1H), 3.61 - 3.74 (m, 1H), 3.82 - 4.02 (m, 2H), 4.38 (d, J = 13.6 Hz, 1H), 4.65 (d, J = 13.6 Hz, 1H), 6.40 (s, 2H), 6.56 (s, 1H), 6.88 (d, J = 8.8 Hz, 1H), 7.32 (s, 1H), 7.36 (d, J = 8.8 Hz, 1H), 7.73 (br s, 1H).
   LCMS (ESI) m/z 508 [M+H]⁺, t_{R} = 1.185 min, mode N.

### Example 125: Synthesis of (18aS)-2-chloro-13-(3-hydroxyazetidin-1-yl)-11-methyl-8,9,10,11,19,20,21,22-octahydro-18 aH,24H-18,15-(metheno)pyrido[2,1-l]pyrimido[6,1-h][1,4,9,10,13]benzoxatetraazacyclohexa decine-7,24(6H)-dione (Example 125a and Example 125b)

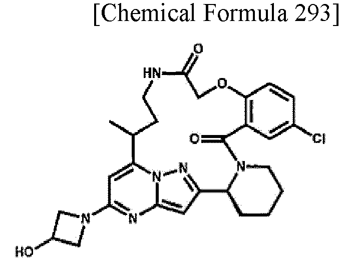

By using the same approaches as in Step 121-1, Step 4-2, Step 121-5, Step 121-6, Step 1-2, Step 13-2, and Step 1-3, Diastereomer A of the title compound (Example 125a) (14 mg, colorless amorphous) and Diastereomer B of the title compound (Example 125b) (17 mg, colorless amorphous) were obtained from the compound (0.35 g) obtained in Reference Example 1-23 (3), 2-[(Z)-3-(4,4,5,5-tetramethyl-1,3,2-dioxaborolan-2-yl)but-2-enyl]isoindoline-1,3-dione (0.38 g), and 2-(4-chloro-2-methoxycarbonyl-phenoxy)acetic acid (28 mg).
Diastereomer A: LCMS (ESI/APCI dual) m/z 539 [M+H]⁺, t_{R} = 0.658 min, mode N.
Diastereomer B: LCMS (ESI/APCI dual) m/z 539 [M+H]⁺, t_{R} = 0.705 min, mode N.

### Example 126: Synthesis of (18aS)-13-cyclopropyl-2,11-dimethyl-8,9,10,11,19,20,21,22-octahydro-18aH,24H-18,15-(me theno)pyrido[2,1-l]pyrimido[6,1-h][1,4,9,10,13]benzoxatetraazacyclohexadecine-7,24(6H)-di one (Example 126a and Example 126b)

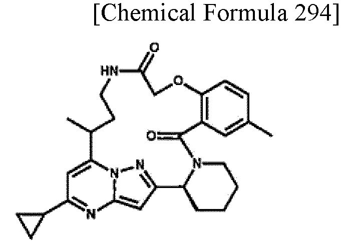

By using the same approaches as in Step 121-6, Step 1-2, Step 13-2, and Step 1-3, Diastereomer A of the title compound (Example 126a) (52 mg, colorless amorphous) and Diastereomer B of the title compound (Example 126b) (45 mg, colorless amorphous) were obtained from the compound (0.15 g) obtained in Step 122-1 and [2-(methoxycarbonyl)-4-methylphenoxy]acetic acid (89 mg).
Diastereomer A: LCMS (ESI) m/z 488 [M+H]⁺, t_{R} = 1.106 min, mode N.
Diastereomer B: LCMS (ESI) m/z 488 [M+H]⁺, t_{R} = 1.178 min, mode N.

### Example 127: Synthesis of (18aS)-13-(3-hydroxyazetidin-1-yl)-2,11-dimethyl-8,9,10,11,19,20,21,22-octahydro-18aH,24 H-18,15-(metheno)pyrido[2,1-l]pyrimido[6,1-h][1,4,9,10,13]benzoxatetraazacyclohexadecin e-7,24(6H)-dione (Example 127a and Example 127b)

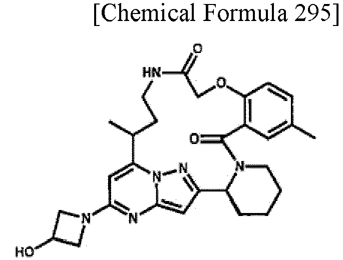

Step 127-1: By using the same approaches as in Step 121-1, Step 4-2, and Step 121-5, tert-butyl (2S)-2-{7-(4-aminobutan-2-yl)-5-[3-(benzyloxy)azetidin-1-yl]pyrazolo[1,5-a]pyrimidin-2-yl} piperidine-1-carboxylate (0.15 g, light yellow oily matter) was obtained from the compound (0.31 g) obtained in Reference Example 1-23 (3) and 2-[(Z)-3-(4,4,5,5-tetramethyl-1,3,2-dioxaborolan-2-yl)but-2-enyl]isoindoline-1,3-dione (0.31 g).
LCMS (ESI) m/z 535 [M+H]⁺, t_{R} = 0.608, 0.616 min, mode L.

Step 127-2: By using the same approaches as in Step 121-6, Step 1-2, Step 13-2, and Step 1-3, Diastereomer A (40 mg, colorless powder) and Diastereomer B (47 mg, colorless powder) of (18aS)-13-[3-(benzyloxy)azetidin-1-yl]-2,1-dimethyl-8,9,10,11,19,20,21,22-octahydro-18a H,24H-18,15-(metheno)pyrido[2,1-l]pyrimido[6,1-h][1,4,9,10,13]benzoxatetraazacyclohexad ecine-7,24(6H)-dione were obtained from the compound (0.15 g) obtained in Step 127-1 and [2-(methoxycarbonyl)-4-methylphenoxy]acetic acid (70 mg).
Diastereomer A: LCMS (ESI) m/z 609 [M+H]⁺, t_{R} = 0.923 min, mode L.
Diastereomer B: LCMS (ESI) m/z 609 [M+H]⁺, t_{R} = 0.870 min, mode L.

Step 127-3: By using the same approach as in Step 50-2, Diastereomer A of the title compound (Example 127a) (29 mg, colorless powder) was obtained from Diastereomer A (39 mg) of Step 127-2.
LCMS (ESI) m/z 519 [M+H]⁺, t_{R} = 0.602 min, mode L.

Step 127-4: By using the same approach as in Step 50-2, Diastereomer B of the title compound (Example 127b) (35 mg, colorless powder) was obtained from Diastereomer B (47 mg) of Step 127-2.
LCMS (ESI) m/z 519 [M+H]⁺, t_{R} = 0.549 min, mode L.

### Example 128: Synthesis of (18aS)-13-(3-aminoazetidin-1-yl)-2,11-dimethyl-8,9,10,11,19,20,21,22-octahydro-18aH,24H -18,15-(metheno)pyrido[2,1-l]pyrimido[6,1-h][1,4,9,10,13]benzoxatetraazacyclohexadecine-7,24(6H)-dione

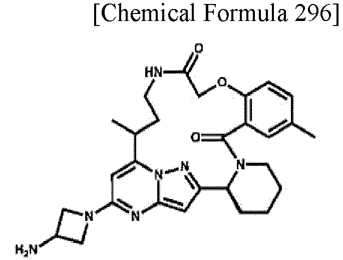

By using the same approaches as in Step 121-4 and Step 121-5, the title compound (10 mg, colorless powder) was obtained from the compound (23 mg) obtained in Step 127-4. LCMS (ESI) m/z 518 [M+H]⁺, t_{R} = 0.539 min, mode N.

### Example 129: Synthesis of (18aS)-13-(3-hydroxyazetidin-1-yl)-11-methyl-2-trideuteriomethyl-8,9,10,11,19,20,21,22-oct ahydro-18aH,24H-18,15-(metheno)pyrido[2,1-1]pyrimido[6,1-h][1,4,9,10,13]benzoxatetraaza cyclohexadecine-7,24(6H)-dione (Example 129a and Example 129b)

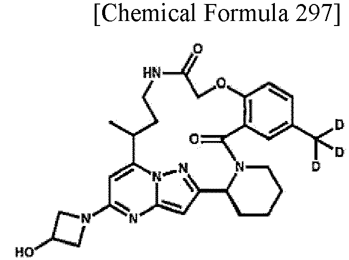

Step 129-1: By using the same approaches as in Step 121-6, Step 1-2, Step 13-2, and Step 1-3, Diastereomer A (60 mg, colorless amorphous) and Diastereomer B (65 mg, colorless amorphous) of (18aS)-13-[3-(benzyloxy)azetidin-1-yl]-11-methyl-2-trideuteriomethyl-8,9,10,11,19,20,21,22 -octahydro-18aH,24H-18,15-(metheno)pyrido[2,1-l]pyrimido[6,1-h][1,4,9,10,13]benzoxatetr aazacyclohexadecine-7,24(6H)-dione were obtained from the compound (0.16 g) obtained in Step 127-1 and 2-[2-methoxycarbonyl-4-(trideuteriomethyl)phenoxy]acetic acid (82 mg).
Diastereomer A: LCMS (ESI) m/z 612 [M+H]⁺, t_{R} = 1.130 min, mode N.
Diastereomer B: LCMS (ESI) m/z 612 [M+H]⁺, t_{R} = 1.198 min, mode N.

Step 129-2: By using the same approach as in Step 50-2, Diastereomer A of the title compound (Example 129a) (32 mg, colorless powder) was obtained from Diastereomer A (60 mg) of Step 129-1.
LCMS (ESI) m/z 522 [M+H]⁺, t_{R} = 0.739 min, mode N.

Step 129-3: By using the same approach as in Step 50-2, Diastereomer B of the title compound (Example 129b) (51 mg, colorless powder) was obtained from Diastereomer B (65 mg) of Step 129-1.
LCMS (ESI) m/z 522 [M+H]⁺, t_{R} = 0.799 min, mode N.

### Example 130: Synthesis of (18aS)-13-(3-aminoazetidin-1-yl)-11-methyl-2-trideuteriomethyl-8,9,10,11,19,20,21,22-octa hydro-18aH,24H-18,15-(metheno)pyrido[2,1-l]pyrimido[6,1-h][1,4,9,10,13]benzoxatetraazac yclohexadecine-7,24(6H)-dione

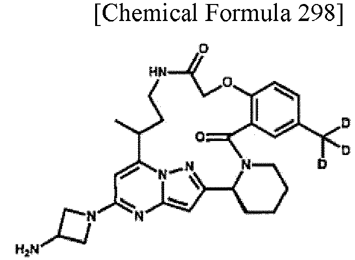

By using the same approaches as in Step 121-4 and Step 121-5, the title compound (14 mg, light pink powder) was obtained from the compound (23 mg) obtained in Step 129-2. LCMS (ESI) m/z 521 [M+H]⁺, t_{R} = 0.538 min, mode N.

### Example 131: Synthesis of (18aS)-13-(cis-3-hydroxycyclobutyl)-11-methyl-2-trideuteriomethyl-8,9,10,11,19,20,21,22-o ctahydro-18aH,24H-18,15-(metheno)pyrido[2,1-1]pyrimido[6,1-h][1,4,9,10,13]benzoxatetraa zacyclohexadecine-7,24(6H)-dione

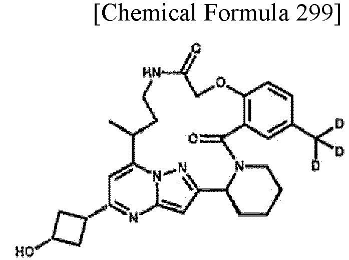

Step 131: By using the same approaches as in Step 121-1, Step 4-2, Step 121-5, Step 121-6, Step 1-2, Step 13-2, and Step 1-3, the title compound (0.17 g, brown amorphous) was obtained from the compound (0.52 g) obtained in Reference Example 1-12, 2-[(Z)-3-(4,4,5,5-tetramethyl-1,3,2-dioxaborolan-2-yl)but-2-enyl]isoindoline-1,3-dione (0.68 g), and 2-[2-methoxycarbonyl-4-(trideuteriomethyl)phenoxy]acetic acid (0.10 g). LCMS (ESI) m/z 521 [M+H]⁺, t_{R} = 0.845, 0.922 min, mode N.

### Example 132: Synthesis of (18aS)-13-(trans-3-aminocyclobutyl)-11-methyl-2-trideuteriomethyl-8,9,10,11,19,20,21,22-o ctahydro-18aH,24H-18,15-(metheno)pyrido[2,1-1]pyrimido[6,1-h][1,4,9,10,13]benzoxatetraa zacyclohexadecine-7,24(6H)-dione (Example 132a and Example 132b)

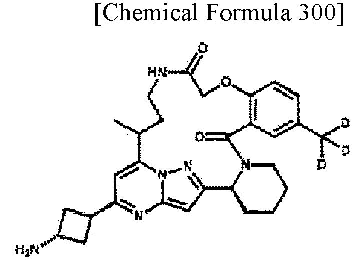

Step 132-1: By using the same approach as in Step 121-4, Diastereomer A (63 mg, colorless amorphous) and Diastereomer B (55 mg, colorless amorphous) of (18aS)-13-[trans-3-(1,3-dioxo-l,3-dihydro-2H-isoindol-2-yl)cyclobutyl]-II-methyl-2-trideut eriomethyl-8,9,10,11,19,20,21,22-octahydro-18aH,24H-18,15-(metheno)pyrido[2,1-l]pyrimid o[6,1-h][1,4,9,10,13]benzoxatetraazacyclohexadecine-7,24(6H)-dione were obtained from the compound (0.10 g) obtained in Step 131.
Diastereomer A: LCMS (ESI) m/z 650 [M+H]⁺, t_{R} = 1.211 min, mode N.
Diastereomer B: LCMS (ESI) m/z 650 [M+H]⁺, t_{R} = 1.289 min, mode N.

Step 132-2: By using the same approach as in Step 121-5, Diastereomer B of the title compound (Example 132b) (27 mg, colorless amorphous) was obtained from Diastereomer B (63 mg) of Step 132-1.
LCMS (ESI) m/z 520 [M+H]⁺, t_{R} = 0.614 min, mode N.

Step 132-3: By using the same approach as in Step 121-5, Diastereomer A of the title compound (Example 132a) (25 mg, colorless amorphous) was obtained from Diastereomer A (77 mg) of Step 132-1.
LCMS (ESI) m/z 520 [M+H]⁺, t_{R} = 0.560 min, mode N.

### Example 133: Synthesis of (18aS)-13-(azetidin-1-yl)-2-fluoro-11-methyl-6,7,8,9,10,11,19,20,21,22-decahydro-18aH,24 H-18,15-(metheno)pyrido[2,1-l]pyrimido[6,1-h][1,4,7,9,10,13]benzoxapentaazacyclohexadec in-24-one

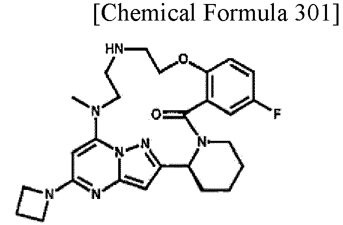

Step 133-1: By using the same approaches as in Step 1-1 to Step 1-3, benzyl (18aS)-13-chloro-2-fluoro-11-methyl-24-oxo-6,7,10,11,19,20,21,22-octahydro-18aH,24H-18 ,15-(metheno)pyrido[2,1-l]pyrimido[6,1-h][1,4,7,9,10,13]benzoxapentaazacyclohexadecine-8 (9H)-carboxylate (0.24 g, colorless amorphous) was obtained from the compound (0.26 g) obtained in Reference Example 1-1 (3) and the compound (0.26 g) obtained in Reference Example 2-64.
LCMS (ESI) m/z 607 [M+H]⁺, t_{R} = 1.275 min, mode N.

Step 133-2: By using the same approaches as in Step 1-4 and Step 4-2, the title compound (47 mg, colorless amorphous) was obtained from the compound (0.23 g) obtained in Step 133-1 and azetidine hydrochloride (0.36 g).
¹H NMR (400 MHz, CDCl₃) δ ppm 1.31 - 1.91 (m, 4H), 1.92 - 2.05 (m, 1H), 2.08 - 2.16 (m, 0.3H), 2.16 - 2.26 (m, 0.7H), 2.31 - 2.43 (m, 2H), 2.81 - 2.94 (m, 2H), 2.98 (s, 2.1H), 3.03 (s, 0.9H), 3.04 - 3.18 (m, 2.6H), 3.24 - 3.33 (m, 0.7H), 3.36 - 3.46 (m, 0.7H), 3.90 - 4.02 (m, 1.3H), 4.03 - 4.19 (m, 6.4H), 4.62 - 4.70 (m, 0.3H), 4.89 - 4.94 (m, 0.3H), 4.97 (s, 0.3H), 5.01 (s, 0.7H), 5.82 (s, 0.3H), 6.04 (s, 0.7H), 6.23 (d, J = 4.3 Hz, 0.7H), 6.77 (dd, J = 8.7, 3.9 Hz, 0.3H), 6.85 (dd, J = 8.7, 3.9 Hz, 0.7H), 6.93 - 7.06 (m, 2H).
LCMS (ESI) m/z 494 [M+H]⁺, t_{R} = 0.740 min, mode H.

### Example 134: Synthesis of (18aS)-13-[(3S)-3 -aminopyrrolidin-1-yl]-2-fluoro-11-methyl-6,7,8,9,10,11,19,20,21,22-deca hydro-18aH,24H-18,15-(metheno)pyrido[2,1-l]pyrimido[6,1-h][1,4,7,9,10,13]benzoxapentaa zacyclohexadecin-24-one

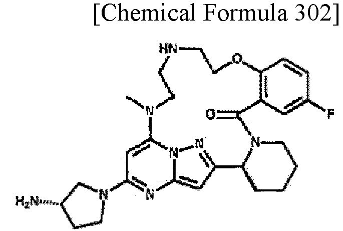

By using the same approaches as in Step 1-4 and Step 4-2, the title compound (4.6 mg, colorless powder) was obtained from the compound (15 mg) obtained in Step 133-1 and (3S)-pyrrolidin-3-amine (10 mg).
LCMS (ESI) m/z 523 [M+H]⁺, t_{R} = 0.596 min, mode H.

### Example 135: Synthesis of (18aS)-2-fluoro-13-(3-hydroxyazetidin-1-yl)-11-methyl-6,7,8,9,10,11,19,20,21,22-decahydro -18aH,24H-18,15-(metheno)pyrido[2,1-1]pyrimido[6,1-h][1,4,7,9,10,13]benzoxapentaazacycl ohexadecin-24-one

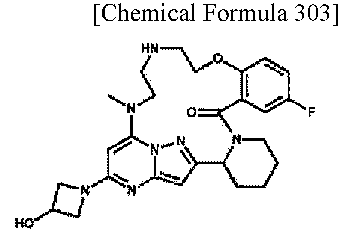

Step 135: By using the same approaches as in Step 3-1 and Step 4-2, the title compound (48 mg, colorless powder) was obtained from the compound (0.17 g) obtained in Step 133-1 and 3-hydroxyazetidine hydrochloride (0.16 g).
LCMS (ESI) m/z 510 [M+H]⁺, t_{R} = 0.688 min, mode H.

### Example 136: Synthesis of (18aS)-13-(3-aminoazetidin-1-yl)-2-fluoro-11-methyl-6,7,8,9,10,11,19,20,21,22-decahydro-1 8aH,24H-18,15-(metheno)pyrido[2,1-1]pyrimido[6,1-h][1,4,7,9,10,13]benzoxapentaazacyclo hexadecin-24-one hydrochloride

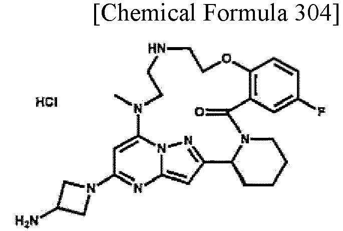

Step 136-1: By using the same approaches as in Step 1-4 and Step 4-2, tert-butyl {1-[(18aS)-2-fluoro-11-methyl-24-oxo-6,7,8,9,10,11,19,20,21,22-decahydro-18aH,24H-18,1 5-(metheno)pyrido[2,1-l]pyrimido[6,1-h][1,4,7,9,10,13]benzoxapentaazacyclohexadecin-13-yl]azetidin-3-yl}carbamate (0.22 g, colorless powder) was obtained from the compound (0.33 g) obtained in Step 133-1 and 3-N-BOC-amino-azetidine (0.76 g).
LCMS (ESI) m/z 609 [M+H]⁺, t_{R} = 0.577 min, mode N.

Step 136-2: By using the same approach as in Step 13-2, the title compound (47 mg, colorless powder) was obtained from the compound (54 mg) obtained in Step 136-1. LCMS (ESI) m/z 509 [M+H]⁺, t_{R} = 0.588 min, mode H.

### Example 137: Synthesis of (18aS)-13-(azetidin-1-yl)-2-fluoro-8,11-dimethyl-6,7,8,9,10,11,19,20,2 1,22-decahydro-18aH, 24H-18,15-(metheno)pyrido[2,1-1]pyrimido[6,1-h][1,4,7,9,10,13]benzoxapentaazacyclohexa decin-24-one (Compound A) and (18aS)-13-(azetidin-1-yl)-2-fluoro-8,11,25-trimethyl-6,7,8,9,10,11,19,20,21,22-decahydro-18 aH,24H-18,15-(metheno)pyrido[2,1-1]pyrimido[6,1-h][1,4,7,9,10,13]benzoxapentaazacycloh exadecin-24-one (Compound B) (Example 137a and Example 137b)

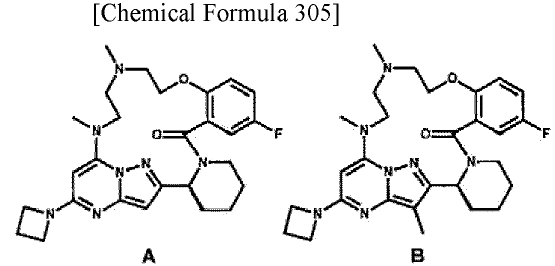

The compound (28 mg) obtained in Step 133-2 was dissolved in acetic acid (1.1 mL), formaldehyde (22 µL, 37% aqueous solution) was added thereto, and the mixture was stirred for 10 minutes. Then, NaBH₃CN (29 mg) was added thereto, and the mixture was stirred for 20 hours. To the reaction liquid, a 1 M NaOH aq. was added to make it basic, and the mixture was stirred, which was then extracted with CHCl₃. The aqueous layer was separated using Phase Separator, and the organic layer was concentrated under reduced pressure and dried. The obtained residue was purified by NH type silica gel chromatography (mobile phase: hexane/EtOAc = 80/20 to 0/100; v/v) and further purified by OH type silica gel chromatography (mobile phase: CHCl₃/MeOH = 100/0 to 70/30; v/v) to afford title compound A (Example 137a) (2.3 mg, colorless amorphous) and title compound B (Example 137b) (21 mg, colorless amorphous).
Title compound A: LCMS (ESI) m/z 508 [M+H]⁺, t_{R} = 0.769 min, mode H.
Title compound B: LCMS (ESI) m/z 522 [M+H]⁺, t_{R} = 0.841 min, mode H.

### Example 138: Synthesis of (18aS)-13-(azetidin-1-yl)-2-fluoro-8-(methanesulfonyl)-11-methyl-6,7,8,9,10,11,19,20,21,22 -decahydro-18aH,24H-18,15-(metheno)pyrido[2,1-l]pyrimido[6,1-h][1,4,7,9,10,13]benzoxap entaazacyclohexadecin-24-one

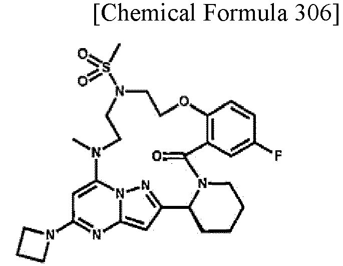

To a solution of the compound (7.1 mg) obtained in Step 133-2 in CHCl₃ (0.48 mL), Et₃N (12 µL) and MsCl (3.4 µL) were added at 0°C, and the mixture was stirred at room temperature for 30 minutes. To the reaction solution, a Sat. NH₄Cl aq. and CHCl₃ were added, and the separating operation was carried out. After concentration under reduced pressure, the solvent was removed. The obtained residue was purified by OH type silica gel column chromatography (mobile phase: hexane/EtOAc = 75/25 to 0/100; v/v) to afford the title compound (7.5 mg, light yellow powder).
LCMS (ESI) m/z 572 [M+H]⁺, t_{R} = 0.630 min, mode N.

### Example 139: Synthesis of (18aS)-2-fluoro-13-(3-hydroxyazetidin-1-yl)-8-(methanesulfonyl)-11-methyl-6,7,8,9,10,11,1 9,20,21,22-decahydro-18aH,24H-18,15-(metheno)pyrido[2,1-l]pyrimido[6,1-h][1,4,7,9,10,13 ]benzoxapentaazacyclohexadecin-24-one (Example 139a) and 1-[(18aS)-2-fluoro-8-(methanesulfonyl)-11-methyl-24-oxo-6,7,8,9,10,11,19,20,21,22-decahy dro-18aH,24H-18,15-(metheno)pyrido[2,1-1]pyrimido[6,1-h][1,4,7,9,10,13]benzoxapentaaza cyclohexadecin-13-yl]azetidin-3-yl methanesulfonate (Example 139b)

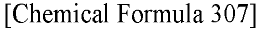

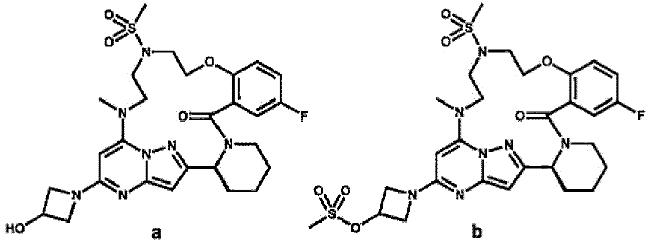

Step 139: By using the same approach as in Step 138, title compound a (24 mg, colorless powder) and title compound b (79 mg, yellow oily matter) were obtained from the compound (78 mg) obtained in Step 135.
Title compound a: LCMS (ESI) m/z 588 [M+H]⁺, t_{R} = 0.904 min, mode H.
Title compound b: LCMS (ESI) m/z 666 [M+H]⁺, t_{R} = 1.179 min, mode H.

### Example 140: Synthesis of (18aS)-13-(3-aminoazetidin-1-yl)-2-fluoro-8-(methanesulfonyl)-11-methyl-6,7,8,9,10,11,19, 20,21,22-decahydro-18aH,24H-18,15-(metheno)pyrido[2,1-1]pyrimido[6,1-h][1,4,7,9,10,13]b enzoxapentaazacyclohexadecin-24-one

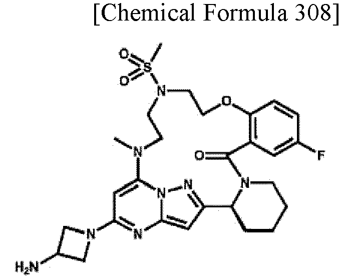

To a solution of Compound B (76 mg) obtained in Step 139 in DMF (0.23 mL), sodium azide (22 mg) was added, and the mixture was stirred at 100°C for 55 hours. To the reaction solution, EtOAc and Brine were added, and the separating operation was carried out. The aqueous layer was separated using Phase Separator, and then the organic layer was concentrated under reduced pressure. To a solution of the obtained crudely purified product in MeOH (1.0 mL), 10% palladium-carbon (0.14 g) was added, and the mixture was stirred for 1.5 hours under a hydrogen gas atmosphere. The reaction liquid was filtered through Celite (R) and washed with MeOH, and the resulting filtrate was concentrated under reduced pressure. After purification by reversed phase preparative HPLC, purification by NH type silica gel column chromatography (mobile phase: EtOAc to CHCl₃/MeOH = 100/0 to 95/5; v/v) afforded the title compound (16 mg, colorless powder).
LCMS (ESI) m/z 587 [M+H]⁺, t_{R} = 0.464 min, mode N.

### Example 141: Synthesis of (18aS)-2-fluoro-13-(3-hydroxyazetidin-1-yl)-8,11-dimethyl-6,7,8,9,10,11,19,20,21,22-decah ydro-18aH,24H-18,15-(metheno)pyrido[2,1-l]pyrimido[6,1-h][1,4,7,9,10,13]benzoxapentaaz acyclohexadecin-24-one

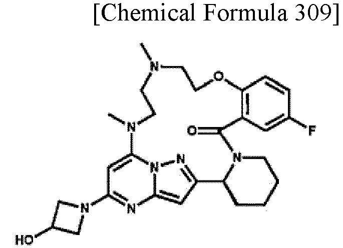

Step 141-1: By using the same approaches as in Step 1-1 to Step 1-3, (18aS)-13-chloro-2-fluoro-8,11-dimethyl-6,7,8,9,10,11,19,20,21,22-decahydro-18aH,24H-18 ,15-(metheno)pyrido[2,1-l]pyrimido[6,1-h][1,4,7,9,10,13]benzoxapentaazacyclohexadecin-24 -one (0.15 g, colorless amorphous) was obtained from the compound (0.16 g) obtained in Reference Example 1-1 (3) and the compound (0.12 g) obtained in Reference Example 2-65. LCMS (ESI) m/z 487 [M+H]⁺, t_{R} = 0.617 min, mode N.

Step 141-2: By using the same approach as in Step 3-1, the title compound (60 mg, colorless amorphous) was obtained from the compound (60 mg) obtained in Step 141-1 and 3-hydroxyazetidine hydrochloride (0.14 g).
LCMS (ESI) m/z 524 [M+H]⁺, t_{R} = 0.717 min, mode H.

### Example 142: Synthesis of (18aS)-13-(3-aminoazetidin-1-yl)-2-fluoro-8,11-dimethyl-6,7,8,9,10,11,19,20,21,22-decahyd ro-18aH,24H-18,15-(metheno)pyrido[2,1-l]pyrimido[6,1-h][1,4,7,9,10,13]benzoxapentaazac yclohexadecin-24-one hydrochloride

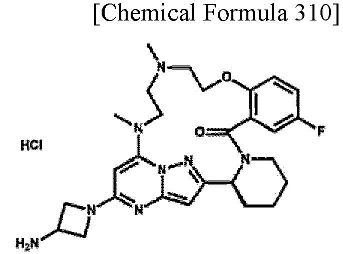

By using the same approaches as in Step 22-1 and Step 13-2, the title compound (41 mg, colorless powder) was obtained from the compound (85 mg) obtained in Step 136-1. LCMS (ESI) m/z 523 [M+H]⁺, t_{R} = 0.612 min, mode H.

### Example 143: Synthesis of (18aS)-13-(azetidin-1-yl)-11-ethyl-2-fluoro-6,7,8,9,10,11,19,20,21,22-decahydro-18aH,24H-18,15-(metheno)pyrido[2,1-l]pyrimido[6,1-h][1,4,7,9,10,13]benzoxapentaazacyclohexadecin-24-one

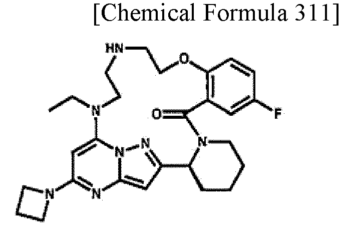

By using the same approaches as in Step 1-1 to Step 1-4 and Step 4-2, the title compound (92 mg, colorless amorphous) was obtained from the compound (0.25 g) obtained in Reference Example 1-1 (3), the compound (0.29 g) obtained in Reference Example 2-67, and azetidine (0.21 g).
LCMS (ESI) m/z 508 [M+H]⁺, t_{R} = 0.792 min, mode H.

### Example 144: Synthesis of (18aS)-13-(azetidin-1-yl)-2-fluoro-7,11-dimethyl-6,7,8,9,10,11,19,20,21,22-decahydro-18aH, 24H-18,15-(metheno)pyrido[2,1-1]pyrimido[6,1-h][1,4,7,9,10,13]benzoxapentaazacyclohexa decin-24-one

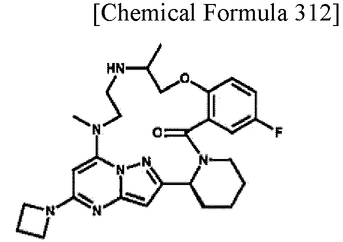

Step 144-1: By using the same approaches as in Step 1-1 to Step 1-3, benzyl (18aS)-13-chloro-2-fluoro-7,11-dimethyl-24-oxo-6,7,10,11,19,20,21,22-octahydro-18aH,24H -18,15-(metheno)pyrido[2,1-l]pyrimido[6,1-h][1,4,7,9,10,13]benzoxapentaazacyclohexadecin e-8(9H)-carboxylate (99 mg, colorless amorphous) was obtained from the compound (80 mg) obtained in Reference Example 1-1 (3) and the compound (0.12 g) obtained in Reference Example 2-68.
LCMS (ESI) m/z 621 [M+H]⁺, t_{R} = 1.312 min, mode N.

Step 144-2: By using the same approaches as in Step 1-4 and Step 4-2, the title compound (36 mg, colorless powder) was obtained from the compound (49 mg) obtained in Step 144-1 and azetidine (53 µL).
LCMS (ESI) m/z 508 [M+H]⁺, t_{R} = 0.753 min, mode H.

### Example 145: Synthesis of (18aS)-13-(3-aminoazetidin-1-yl)-2-fluoro-7,11-dimethyl-6,7,8,9,10,11,19,20,21,22-decahyd ro-18aH,24H-18,15-(metheno)pyrido[2,1-l]pyrimido[6,1-h][1,4,7,9,10,13]benzoxapentaazac yclohexadecin-24-one

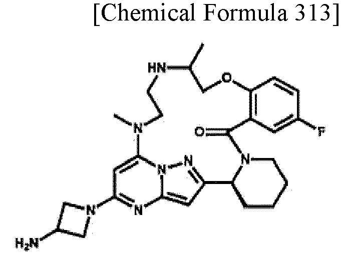

By using the same approaches as in Step 1-4, Step 4-2, and Step 13-2, the title compound (23 mg, light yellow amorphous) was obtained from the compound (49 mg) obtained in Step 144-1 and 3-N-BOC-amino-azetidine (0.14 g).
LCMS (ESI) m/z 523 [M+H]⁺, t_{R} = 0.602 min, mode H.

### Example 146: Synthesis of (18aS)-13-(azetidin-1-yl)-2-fluoro-7-(hydroxymethyl)-11-methyl-6,7,8,9,10,11,19,20,21,22-d ecahydro-18aH,24H-18,15-(metheno)pyrido[2,1-1]pyrimido[6,1-h][1,4,7,9,10,13]benzoxapen taazacyclohexadecin-24-one

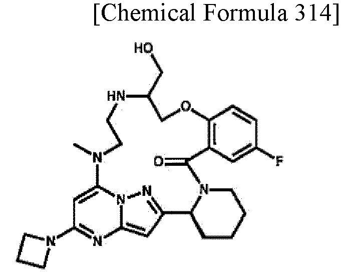

Step 146: By using the same approaches as in Step 1-1 to Step 1-4 and Step 4-2, the title compound (28 mg, light yellow powder) was obtained from the compound (0.13 g) obtained in Reference Example 1-1 (3), the compound (0.25 g) obtained in Reference Example 2-69, and azetidine (74 µL).
LCMS (ESI) m/z 524 [M+H]⁺, t_{R} = 0.559, 0.600 min, mode N.

### Example 147: Synthesis of (20aS)-15-(azetidin-1-yl)-2-fluoro-13-methyl-6a,7,12,13,21,22,23,24-octahydro-6H,9H,11H, 20aH,26H-20,17-(metheno)[1,3]oxazolo[4,3-c]pyrido[2,1-l]pyrimido[6,1-h][1,4,7,9,10,13]be nzoxapentaazacyclohexadecine-9,26-dione

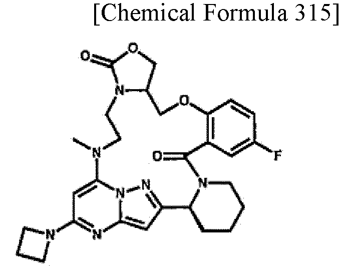

A solution of the compound (23 mg) obtained in Step 146 in CHCl₃ (0.44 mL) was cooled to 0°C, Et₃N (31 µL) and triphosgene (6.5 mg) were added thereto, and the mixture was stirred at 0°C for 1 hour. To the reaction solution, water and CHCl₃ were added, and the separating operation was carried out. The aqueous layer was separated using Phase Separator, and then the organic layer was concentrated under reduced pressure. The obtained residue was purified by NH type silica gel column chromatography (mobile phase: hexane/EtOAc = 50/50 to 0/100; v/v) and purified by reversed phase preparative HPLC. Then, to the collected fraction, a Sat. NaHCO₃ aq. and CHCl₃ were added, and the extracting operation was carried out. The aqueous layer was separated using Phase Separator, and then the organic layer was concentrated under reduced pressure. To the obtained residue, MeCN (1.0 mL) and water (1.0 mL) were added, K₂CO₃ (30 mg) was added thereto, and the mixture was stirred at 60°C for 30 minutes. To the reaction solution, water and CHCl₃ were added, and the separating operation was carried out. The aqueous layer was separated using Phase Separator, and then the organic layer was concentrated under reduced pressure. The obtained residue was purified by NH type silica gel column chromatography (mobile phase: hexane/EtOAc = 50/50 to 0/100; v/v) to afford the title compound (7.0 mg, colorless amorphous).
LCMS (ESI) m/z 550 [M+H]⁺, t_{R} = 0.555, 0.625 min, mode N.

### Example 148: Synthesis of (23aS)-18-(azetidin-1-yl)-8-fluoro-16-methyl-1,3,4,11,12,13,14,15,16,23a-decahydro-2H,6H-23,20-(metheno)pyrido[1,2-b]pyrimido[1,6-f][2,5,6,8,11]benzopentaazacyclopentadecin-6-one

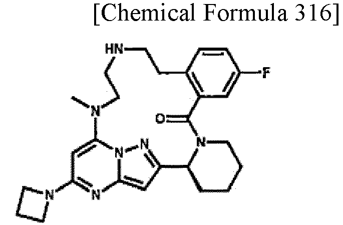

Step 148: By using the same approaches as in Step 1-1 to Step 1-4 and Step 4-2, the title compound (86 mg, colorless amorphous) was obtained from the compound (0.23 g) obtained in Reference Example 1-1 (3), the compound (0.25 g) obtained in Reference Example 2-84, and azetidine (0.14 mL).
¹H NMR (400 MHz, CDCl₃) δ ppm 1.42 - 1.57 (m, 2H), 1.58 - 1.85 (m, 2H), 1.94 - 2.08 (m, 1H), 2.26 - 2.34 (m, 1H), 2.39 (quin, J = 7.4 Hz, 2H), 2.62 - 2.76 (m, 1H), 2.81 - 3.00 (m, 5H), 3.04 - 3.23 (m, 2H), 3.24 - 3.47 (m, 3H), 4.04 - 4.17 (m, 5H), 4.20 - 4.34 (m, 1H), 5.05 (s, 1H), 6.03 (s, 1H), 6.19 (d, J = 4.2 Hz, 1H), 6.94 - 7.06 (m, 2H), 7.24 (dd, J = 8.4, 5.4 Hz, 1H).
LCMS (ESI) m/z 478 [M+H]⁺, t_{R} = 0.743 min, mode H.

### Example 149: Synthesis of (23aS)-18-(azetidin-1-yl)-8-fluoro-13,16-dimethyl-1,3,4,11,12,13,14,15,16,23a-decahydro-2 H,6H-23,20-(metheno)pyrido[1,2-b]pyrimido[1,6-f][2,5,6,8,11]benzopentaazacyclopentadeci n-6-one

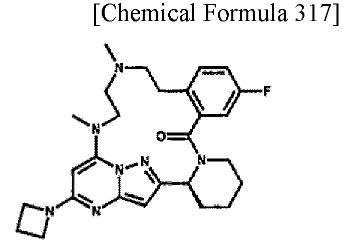

By using the same approach as in Step 137, the title compound (14 mg, colorless amorphous) was obtained from the compound (18 mg) obtained in Step 148.
LCMS (ESI) m/z 492 [M+H]⁺, t_{R} = 0.745 min, mode H.

### Example 150: Synthesis of (18aS)-13-(azetidin-1-yl)-2-chloro-8,11-dimethyl-6,7,8,9,10,11,19,20,21,22-decahydro-18aH ,24H-18,15-(metheno)pyrido[2,1-1]pyrimido[6,1-h][1,4,7,9,10,13]benzoxapentaazacyclohexa decin-24-one

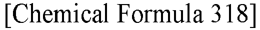

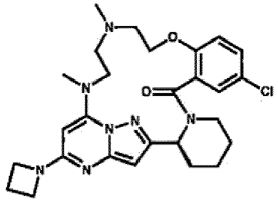

Step 150-1: By using the same approaches as in Step 1-1 to Step 1-3, (18aS)-2,13-dichloro-8,11-dimethyl-6,7,8,9,10,11,19,20,21,22-decahydro-18aH,24H-18,15-( metheno)pyrido[2,1-l]pyrimido[6,1-h][1,4,7,9,10,13]benzoxapentaazacyclohexadecin-24-one (0.32 g, light yellow amorphous) was obtained from the compound (0.36 g) obtained in Reference Example 1-1 (3) and the compound (0.35 g) obtained in Reference Example 2-72. LCMS (ESI) m/z 503 [M+H]⁺, t_{R} = 0.667 min, mode N.

Step 150-2: By using the same approach as in Step 1-4, the title compound (96 mg, colorless amorphous) was obtained from the compound (0.10 g) obtained in Step 150-1 and azetidine (0.13 mL).
LCMS (ESI) m/z 524 [M+H]⁺, t_{R} = 0.450 min, mode N.

### Example 151: Synthesis of (18aS)-13-(3-aminoazetidin-1-yl)-2-chloro-8,11-dimethyl-6,7,8,9,10,11,19,20,21,22-decahyd ro-18aH,24H-18,15-(metheno)pyrido[2,1-l]pyrimido[6,1-h][1,4,7,9,10,13]benzoxapentaazac yclohexadecin-24-one

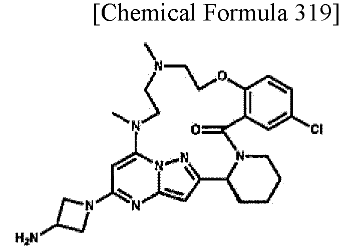

Step 151: By using the same approaches as in Step 3-1 and Step 5-2, the title compound (71 mg, colorless amorphous) was obtained from the compound (0.13 g) obtained in Step 150-1 and 3-N-BOC-amino-azetidine (0.34 g).
LCMS (ESI) m/z 539 [M+H]⁺, t_{R} = 0.216 min, mode N.

### Example 152: Synthesis of (18aS)-2-chloro-13-(3-hydroxyazetidin-1-yl)-8,11-dimethyl-6,7,8,9,10,11,19,20,21,22-decah ydro-18aH,24H-18,15-(metheno)pyrido[2,1-l]pyrimido[6,1-h][1,4,7,9,10,13]benzoxapentaaz acyclohexadecin-24-one

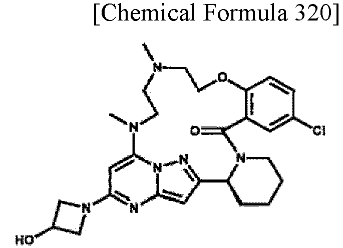

By using the same approach as in Step 3-1, the title compound (57 mg, colorless amorphous) was obtained from the compound (0.12 g) obtained in Step 150-1 and 3-hydroxyazetidine hydrochloride (0.17 g).
LCMS (ESI) m/z 540 [M+H]⁺, t_{R} = 0.784 min, mode H.

### Example 153: Synthesis of (18aS)-2-chloro-8,11-dimethyl-13-(piperidin-1-yl)-6,7,8,9,10,11,19,20,21,22-decahydro-18a H,24H-18,15-(metheno)pyrido[2,1-l]pyrimido[6,1-h][1,4,7,9,10,13]benzoxapentaazacyclohe xadecin-24-one

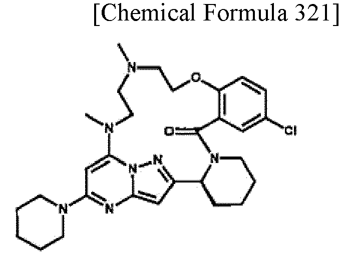

By using the same approach as in Step 1-4, the title compound (38 mg, colorless amorphous) was obtained from the compound (50 mg) obtained in Step 150-1 and piperidine (50 µL).
LCMS (ESI) m/z 552 [M+H]⁺, t_{R} = 0.673 min, mode N.

### Example 154: Synthesis of (18aS)-2-chloro-8,11-dimethyl-13-(morpholin-4-yl)-6,7,8,9,10,11,19,20,21,22-decahydro-18 aH,24H-18,15-(metheno)pyrido[2,1-l]pyrimido[6,1-h][1,4,7,9,10,13]benzoxapentaazacycloh exadecin-24-one

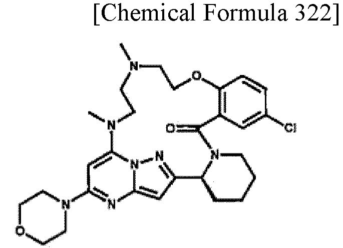

By using the same approach as in Step 1-4, the title compound (24 mg, colorless amorphous) was obtained from the compound (50 mg) obtained in Step 150-1 and morpholine (44 µL).
LCMS (ESI) m/z 554 [M+H]⁺, t_{R} = 0.622 min, mode N.

### Example 155: Synthesis of (18aS)-2-chloro-13-[(2-hydroxyethyl)amino]-8,11-dimethyl-6,7,8,9,10,11,19,20,21,22-decah ydro-18aH,24H-18,15-(metheno)pyrido[2,1-l]pyrimido[6,1-h][1,4,7,9,10,13]benzoxapentaaz acyclohexadecin-24-one

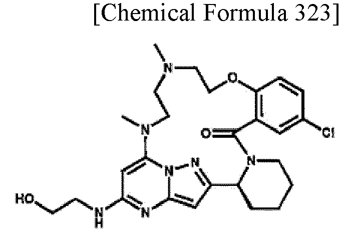

By using the same approach as in Step 1-4, the title compound (29 mg, light yellow amorphous) was obtained from the compound (50 mg) obtained in Step 150-1 and 2-aminoethanol (48 µL).
LCMS (ESI) m/z 528 [M+H]⁺, t_{R} = 0.787 min, mode H.

### Example 156: Synthesis of (18aS)-13-[(2-aminoethyl)amino]-2-chloro-8,11-dimethyl-6,7,8,9,10,11,19,20,21,22-decahyd ro-18aH,24H-18,15-(metheno)pyrido[2,1-l]pyrimido[6,1-h][1,4,7,9,10,13]benzoxapentaazac yclohexadecin-24-one hydrochloride

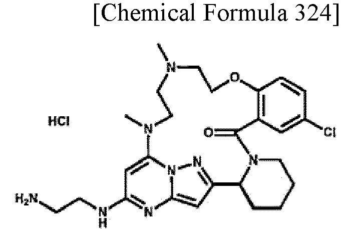

By using the same approaches as in Step 1-4 and Step 13-2, the title compound (13 mg, colorless amorphous) was obtained from the compound (50 mg) obtained in Step 150-1 and tert-butyl N-(2-aminoethyl)carbamate (0.13 mL).
LCMS (ESI) m/z 527 [M+H]⁺, t_{R} = 0.698 min, mode H.

### Example 157: Synthesis of (18aS)-13-[(2-aminoethyl)amino]-2-chloro-8,11-dimethyl-6,7,8,9,10,11,19,20,21,22-decahyd ro-18aH,24H-18,15-(metheno)pyrido[2,1-l]pyrimido[6,1-h][1,4,7,9,10,13]benzoxapentaazac yclohexadecin-24-one hydrochloride

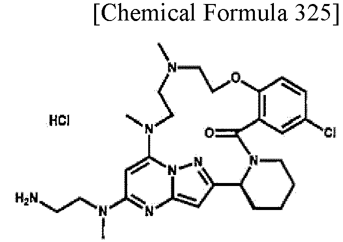

By using the same approaches as in Step 1-4 and Step 13-2, the title compound (19 mg, colorless amorphous) was obtained from the compound (50 mg) obtained in Step 150-1 and tert-butyl N-[2-(methylamino)ethyl]carbamate (0.14 g).
LCMS (ESI) m/z 541 [M+H]⁺, t_{R} = 0.735 min, mode H.

### Example 158: Synthesis of (18aS)-2-chloro-13-(3-hydroxy-3-methylazetidin-1-yl)-8,11-dimethyl-6,7,8,9,10,11,19,20,21, 22-decahydro-18aH,24H-18,15-(metheno)pyrido[2,1-l]pyrimido[6,1-h][1,4,7,9,10,13]benzox apentaazacyclohexadecin-24-one

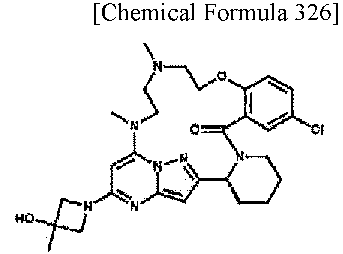

By using the same approach as in Step 3-1, the title compound (7.0 mg, pink amorphous) was obtained from the compound (54 mg) obtained in Step 150-1 and 3-methylazetidin-3-ol hydrochloride (66 mg).
LCMS (ESI) m/z 554 [M+H]⁺, t_{R} = 0.835 min, mode N.

### Example 159: Synthesis of (18aS)-2-chloro-8,11-dimethyl-13-(3-methylazetidin-1-yl)-6,7,8,9,10,11,19,20,21,22-decahy dro-18aH,24H-18,15-(metheno)pyrido[2,1-l]pyrimido[6,1-h][1,4,7,9,10,13]benzoxapentaaza cyclohexadecin-24-one

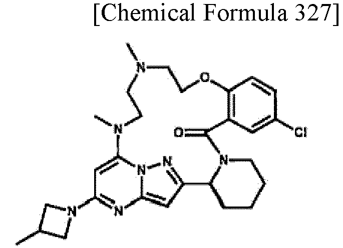

By using the same approach as in Step 3-1, the title compound (23 mg, colorless powder) was obtained from the compound (50 mg) obtained in Step 150-1 and 3-methylazetidine hydrochloride (53 mg).
LCMS (ESI) m/z 538 [M+H]⁺, t_{R} = 0.508 min, mode N.

### Example 160: Synthesis of (18aS)-2-chloro-13-(3-methoxyazetidin-1-yl)-8,1-dimethyl-6,7,8,9,10,11,19,20,21,22-decah ydro-18aH,24H-18,15-(metheno)pyrido[2,1-l]pyrimido[6,1-h][1,4,7,9,10,13]benzoxapentaaz acyclohexadecin-24-one

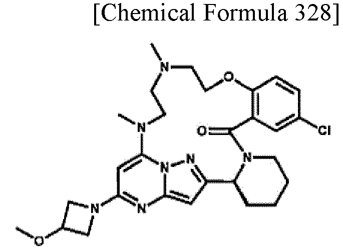

By using the same approach as in Step 3-1, the title compound (18 mg, colorless powder) was obtained from the compound (50 mg) obtained in Step 150-1 and 3-methoxyazetidine hydrochloride (61 mg).
LCMS (ESI) m/z 554 [M+H]⁺, t_{R} = 0.506 min, mode N.

### Example 161: Synthesis of (18aS)-13-[(azetidin-3-yl)amino]-2-chloro-8,11-dimethyl-6,7,8,9,10,11,19,20,21,22-decahydr o-18aH,24H-18,15-(metheno)pyrido[2,1-l]pyrimido[6,1-h][1,4,7,9,10,13]benzoxapentaazacy clohexadecin-24-one

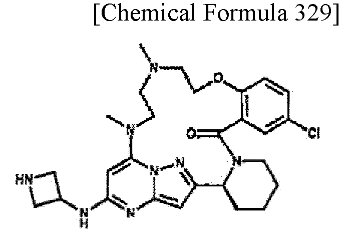

By using the same approaches as in Step 1-4 and Step 5-2, the title compound (6.5 mg, colorless amorphous) was obtained from the compound (48 mg) obtained in Step 150-1 and tert-butyl 3-aminoazetidine-1-carboxylate (0.16 g).
LCMS (ESI) m/z 539 [M+H]⁺, t_{R} = 0.735 min, mode N.

### Example 162: Synthesis of (18aS)-2-chloro-13-(3-fluoroazetidin-1-yl)-8,11-dimethyl-6,7,8,9,10,11,19,20,21,22-decahyd ro-18aH,24H-18,15-(metheno)pyrido[2,1-l]pyrimido[6,1-h][1,4,7,9,10,13]benzoxapentaazac yclohexadecin-24-one

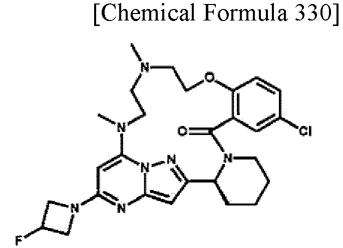

By using the same approach as in Step 3-1, the title compound (4.5 mg, colorless amorphous) was obtained from the compound (67 mg) obtained in Step 150-1 and 3-fluoroazetidine hydrochloride (0.30 g).
LCMS (ESI) m/z 542 [M+H]⁺, t_{R} = 0.564 min, mode N.

### Example 163: Synthesis of (18aS)-13-[3-(aminomethyl)azetidin-1-yl]-2-chloro-8,11-dimethyl-6,7,8,9,10,11,19,20,21,22-decahydro-18aH,24H-18,15-(metheno)pyrido[2,1-l]pyrimido[6,1-h][1,4,7,9,10,13]benzoxape ntaazacyclohexadecin-24-one

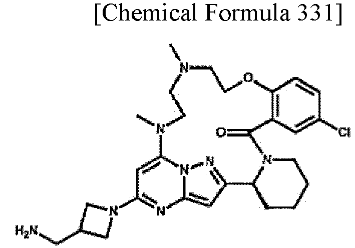

By using the same approach as in Step 3-1, the title compound (14 mg, colorless amorphous) was obtained from the compound (54 mg) obtained in Step 150-1 and azetidin-3-ylmethanamine dihydrochloride (0.17 g).
LCMS (ESI) m/z 553 [M+H]⁺, t_{R} = 0.666 min, mode N.

### Example 164: Synthesis of N-{1-[(18aS)-2-chloro-8,11-dimethyl-24-oxo-6,7,8,9,10,11,19,20,21,22-decahydro-18aH,24 H-18,15-(metheno)pyrido[2, 1-l]pyrimido[6,1-h][1,4,7,9,10,13]benzoxapentaazacyclohexadec in-13-yl]azetidin-3-yl}acetamide

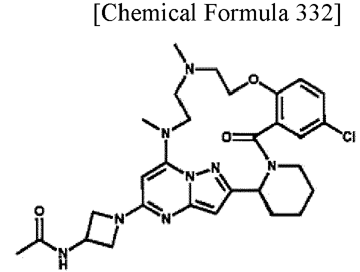

To a solution of the compound (15 mg) obtained in Step 151 in CHCl₃ (1.0 mL), Et₃N (12 µL) and acetyl chloride (3.0 µL) were added at 0°C, and the mixture was stirred at room temperature for 2 hours. Water was added to the reaction liquid, and the aqueous layer was extracted with CHCl₃. The organic layer was collected by using Phase Separator and concentrated under reduced pressure. The obtained residue was purified by reversed phase preparative HPLC. The title compound (7.0 mg, colorless amorphous) was obtained. LCMS (ESI) m/z 581 [M+H]⁺, t_{R} = 0.819 min, mode H.

### Example 165: Synthesis of (18a'S)-13'-(azetidin-1-yl)-2'-chloro-11'-methyl-6'H,8'H,9'H,10'H,11'H,18a'H,19'H,20'H,21'H ,22'H,24'H-spiro[cyclopropane-1,7'-[5]oxa[8,11,14,16,17,23]hexaaza[18,15](metheno)pyrido [2,1-1]pyrimido[6,1-h][1,4,7,9,10,13]benzoxapentaazacyclohexadecin-24'-one

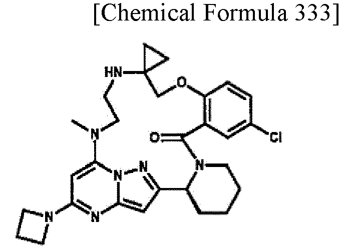

Step 165-1: By using the same approaches as in Step 1-1 to Step 1-3, (18a'S)-2',13'-dichloro-11'-methyl-6'H,8'H,9'H,10'H,11'H,18a'H,19'H,20'H,21'H,22'H,24'H-sp iro[cyclopropane-1,7'-[5]oxa[8,11,14,16,17,23]hexaaza[18,15](metheno)pyrido[2,1-l]pyrimid o[6,1-h][1,4,7,9,10,13]benzoxapentaazacyclohexadecin]-24'-one (51 mg, colorless amorphous) was obtained from the compound (0.15 g) obtained in Reference Example 1-1 (3) and the compound (0.13 g) obtained in Reference Example 2-76.
LCMS (ESI) m/z 515 [M+H]⁺, t_{R} = 0.669 min, mode N.

Step 165-2: By using the same approach as in Step 1-4, the title compound (25 mg, colorless amorphous) was obtained from the compound (51 mg) obtained in Step 150-1 and azetidine (67 µL).
LCMS (ESI) m/z 536 [M+H]⁺, t_{R} = 0.823 min, mode H.

### Example 166: Synthesis of (18a'S)-13'-(3-aminoazetidin-1-yl)-2'-chloro-11'-methyl-6'H,8'H,9'H,10'H,11H,18aH,19H,2 0'H,21'H,22'H,24'H-spiro[cyclopropane-1,7'-[5]oxa[8,11,14,16,17,23]hexaaza[18,15](methen o)pyrido[2,1-1]pyrimido[6,1-h][1,4,7,9,10,13]benzoxapentaazacyclohexadecin]-24'-one

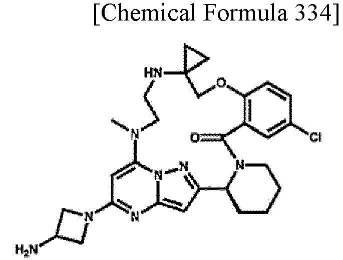

By using the same approaches as in Step 1-4 and Step 5-2, the title compound (61 mg, colorless amorphous) was obtained from the compound (83 mg) obtained in Step 165-1 and 3-N-BOC-amino-azetidine (0.27 g).
LCMS (ESI) m/z 551 [M+H]⁺, t_{R} = 0.706 min, mode H.

### Example 167: Synthesis of (18a'S)-2'-chloro-13'-(3-hydroxyazetidin-1-yl)-11'-methyl-6'H,8'H,9'H,10'H,11'H,18a'H,19'H, 20'H,21'H,22'H,24'H-spiro[cyclopropane-1,7'-[5]oxa[8,11,14,16,17,23]hexaaza[18,15](methe no)pyrido[2,1-l]pyrimido[6,1-h][1,4,7,9,10,13]benzoxapentaazacyclohexadecin]-24'-one

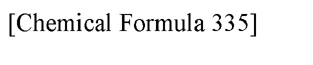

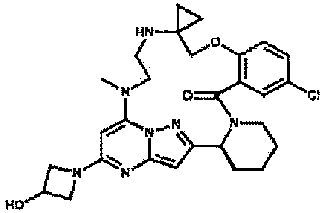

By using the same approach as in Step 3-1, the title compound (46 mg, colorless amorphous) was obtained from the compound (80 mg) obtained in Step 165-1 and 3-hydroxyazetidine hydrochloride (0.17 g).
LCMS (ESI) m/z 552 [M+H]⁺, t_{R} = 0.798 min, mode H.

### Example 168: Synthesis of (18aS)-13-(azetidin-1-yl)-2,11-dimethyl-6,7,8,9,10,11,19,20,21,22-decahydro-18aH,24H-18, 15-(metheno)pyrido[2,1-1]pyrimido[6,1-h][1,4,7,9,10,13]benzoxapentaazacyclohexadecin-24 -one

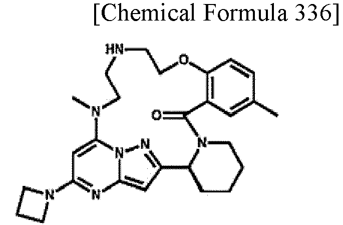

Step 168-1: By using the same approaches as in Step 1-1 to Step 1-3, benzyl (18aS)-13-chloro-2,11-dimethyl-24-oxo-6,7,10,11,19,20,21,22-octahydro-18aH,24H-18,15-( metheno)pyrido[2,1-l]pyrimido[6,1-h][1,4,7,9,10,13]benzoxapentaazacyclohexadecine-8(9H) -carboxylate (0.11 g, colorless amorphous) was obtained from the compound (0.25 g) obtained in Reference Example 1-1 (3) and the compound (0.25 g) obtained in Reference Example 2-66.
LCMS (ESI) m/z 603 [M+H]⁺, t_{R} = 1.318 min, mode N.

Step 168-2: By using the same approaches as in Step 3-1 and Step 4-2, the title compound (43 mg, colorless amorphous) was obtained from the compound (0.11 g) obtained in Step 168-1 and azetidine hydrochloride (0.16 g).
LCMS (ESI) m/z 490 [M+H]⁺, t_{R} = 0.336 min, mode N.

### Example 169: Synthesis of (18aS)-13-[3-(hydroxymethyl)azetidin-1-yl]-2,11-dimethyl-6,7,8,9,10,11,19,20,21,22-decahy dro-18aH,24H-18,15-(metheno)pyrido[2,1-l]pyrimido[6,1-h][1,4,7,9,10,13]benzoxapentaaza cyclohexadecin-24-one

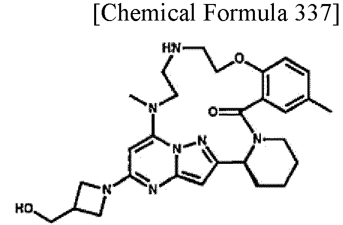

By using the same approaches as in Step 3-1 and Step 4-2, the title compound (30 mg, colorless amorphous) was obtained from the compound (50 mg) obtained in Step 168-1 and azetidin-3-ylmethanol hydrochloride (0.10 g).
LCMS (ESI) m/z 520 [M+H]⁺, t_{R} = 0.725 min, mode H.

### Example 170: Synthesis of (18aS)-2,11-dimethyl-13-(pyrrolidin-1-yl)-6,7,8,9,10,11,19,20,21,22-decahydro-18aH,24H-1 8,15-(metheno)pyrido[2,1-l]pyrimido[6,1-h][1,4,7,9,10,13]benzoxapentaazacyclohexadecin-2 4-one

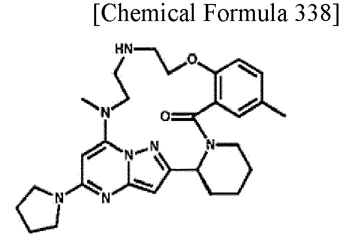

By using the same approaches as in Step 1-4 and Step 4-2, the title compound (28 mg, colorless amorphous) was obtained from the compound (60 mg) obtained in Step 168-1 and pyrrolidine (83 µL).
LCMS (ESI) m/z 504 [M+H]⁺, t_{R} = 0.433 min, mode N.

### Example 171: Synthesis of (18aS)-13-(3-hydroxyazetidin-1-yl)-2,11-dimethyl-6,7,8,9,10,11,19,20,21,22-decahydro-18a H,24H-18,15-(metheno)pyrido[2,1-l]pyrimido[6,1-h][1,4,7,9,10,13]benzoxapentaazacyclohe xadecin-24-one

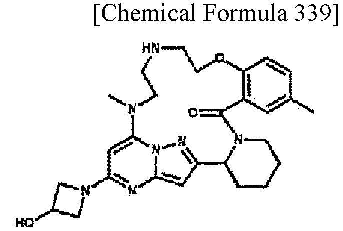

By using the same approaches as in Step 3-1 and Step 4-2, the title compound (88 mg, colorless powder) was obtained from the compound (0.14 g) obtained in Step 168-1 and 3-hydroxyazetidine hydrochloride (0.26 g).
LCMS (ESI) m/z 506 [M+H]⁺, t_{R} = 0.756 min, mode H.

### Example 172: Synthesis of (18aS)-13-(3-aminoazetidin-1-yl)-2,11-dimethyl-6,7,8,9,10,11,19,20,21,22-decahydro-18aH, 24H-18,15-(metheno)pyrido[2,1-l]pyrimido[6,1-h][1,4,7,9,10,13]benzoxapentaazacyclohexa decin-24-one trifluoroacetate salt

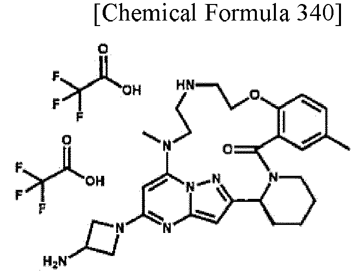

Step 172-1: By using the same approaches as in Step 3-1 and Step 4-2, tert-butyl {1-[(18aS)-2,11-dimethyl-24-oxo-6,7,8,9,10,11,19,20,21,22-decahydro-18aH,24H-18,15-(me theno)pyrido[2,1-l]pyrimido[6,1-h][1,4,7,9,10,13]benzoxapentaazacyclohexadecin-13-yl]azet idin-3-yl}carbamate (83 mg, colorless amorphous) was obtained from the compound (0.16 g) obtained in Step 168-1 and 3-N-BOC-amino-azetidine mesylate salt (0.35 g).
LCMS (ESI) m/z 605 [M+H]⁺, t_{R} = 0.623 min, mode N.

Step 172-2: By using the same approach as in Step 5-2, the title compound (13 mg, colorless amorphous) was obtained from the compound (11 mg) obtained in Step 172-1. LCMS (ESI) m/z 505 [M+H]⁺, t_{R} = 0.669 min, mode H.

### Example 173: Synthesis of (18aS)-13-(azetidin-1-yl)-2,8,11-trimethyl-6,7,8,9,10,11,19,20,21,22-decahydro-18aH,24H-1 8,15-(metheno)pyrido[2,1-l]pyrimido[6,1-h][1,4,7,9,10,13]benzoxapentaazacyclohexadecin-2 4-one

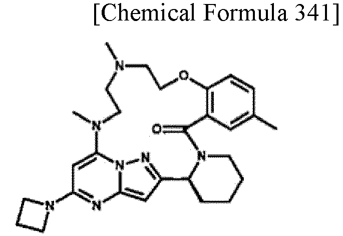

By using the same approach as in Step 22-1, the title compound (34 mg, colorless amorphous) was obtained from the compound (35 mg) obtained in Step 168-2.
LCMS (ESI) m/z 504 [M+H]⁺, t_{R} = 0.823 min, mode H.

### Example 174: Synthesis of (18aS)-13-(azetidin-1-yl)-8-ethyl-2,11-dimethyl-6,7,8,9,10,11,19,20,21,22-decahydro-18aH, 24H-18,15-(metheno)pyrido[2,1-l]pyrimido[6,1-h][1,4,7,9,10,13]benzoxapentaazacyclohexa decin-24-one

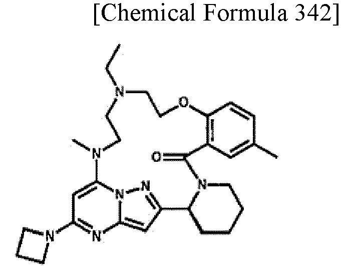

By using the same approach as in Step 22-1, the title compound (26 mg, colorless amorphous) was obtained from the compound (30 mg) obtained in Step 168-2, using acetaldehyde (25 µL) instead of formaldehyde.
LCMS (ESI) m/z 518 [M+H]⁺, t_{R} = 0.452 min, mode N.

### Example 175: Synthesis of (18aS)-13-(azetidin-1-yl)-2,1 I -dimethyl -8 -(prop an-2-yl)- 6,7,8,9,10,11,19,20,21,22-decahydr o-18aH,24H-18,15-(metheno)pyrido[2,1-l]pyrimido[6,1-h][1,4,7,9,10,13]benzoxapentaazacy clohexadecin-24-one

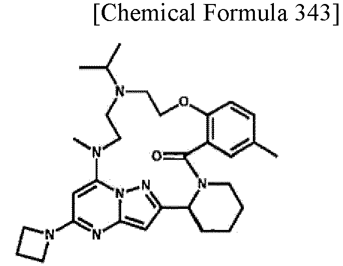

By using the same approach as in Step 22-1, the title compound (6.0 mg, colorless amorphous) was obtained from the compound (30 mg) obtained in Step 168-2, using acetone (14 µL) instead of formaldehyde.
LCMS (ESI) m/z 532 [M+H]⁺, t_{R} = 0.498 min, mode N.

### Example 176: Synthesis of (18aS)-8-acetyl-13-(azetidin-1-yl)-2,11-dimethyl-6,7,8,9,10,11,19,20,21,22-decahydro-18aH, 24H-18,15-(metheno)pyrido[2,1-l]pyrimido[6,1-h][1,4,7,9,10,13]benzoxapentaazacyclohexa decin-24-one

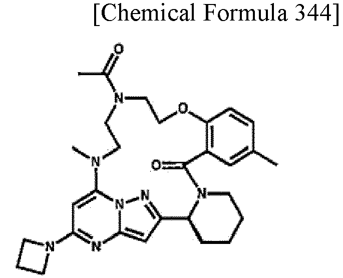

By using the same approach as in Step 164, the title compound (35 mg, colorless amorphous) was obtained from the compound (50 mg) obtained in Step 168-2.
LCMS (ESI) m/z 532 [M+H]⁺, t_{R} = 0.602 min, mode N.

### Example 177: Synthesis of (18aS)-8-(aminoacetyl)-13-(azetidin-1-yl)-2,11-dimethyl-6,7,8,9,10,11,19,20,21,22-decahydr o-18aH,24H-18,15-(metheno)pyrido[2,1-l]pyrimido[6,1-h][1,4,7,9,10,13]benzoxapentaazacy clohexadecin-24-one

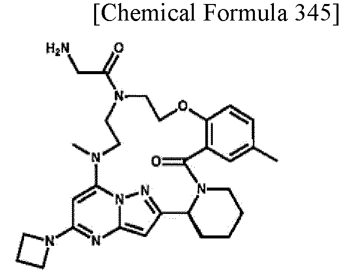

By using the same approaches as in Step 121-6 and Step 5-2, the title compound (10 mg, colorless powder) was obtained from the compound (32 mg) obtained in Step 168-2 and N-(tert-butoxycarbonyl)glycine (17 mg).
LCMS (ESI) m/z 547 [M+H]⁺, t_{R} = 0.802 min, mode H.

### Example 178: Synthesis of (18aS)-13-(azetidin-1-yl)-2,11-dimethyl-24-oxo-6,7,10,11,19,20,21,22-octahydro-18aH,24H-18,15-(metheno)pyrido[2,1-l]pyrimido[6,1-h][1,4,7,9,10,13]benzoxapentaazacyclohexadecin e-8(9H)-sulfonamide

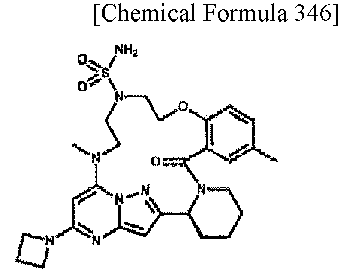

Step 178-1: To CHCl₃ (1.0 mL), chlorosulfonyl isocyanate (8.9 µL) and tBuOH (13 µL) were added under ice cooling, and the mixture was stirred for 10 minutes. Then, Et₃N (17 µL) and the compound (50 mg) obtained in Step 168-2 were added thereto, and the mixture was stirred at room temperature for 30 minutes. A solution prepared by the same method from chlorosulfonyl isocyanate (8.9 µL) and tBuOH (13 µL) in CHCl₃ (1.0 mL) under ice cooling was further added thereto, and the mixture was stirred for 30 minutes. After adding water to the reaction liquid and stirring the mixture, it was extracted with CHCl₃. The aqueous layer was separated using Phase Separator, and the organic layer was concentrated under reduced pressure and dried. The obtained residue was purified by NH type silica gel chromatography (mobile phase: hexane/EtOAc = 90/10 to 0/100; v/v) to afford tert-butyl [(18aS)-13-(azetidin-1-yl)-2,11-dimethyl-24-oxo-6,7,10,11,19,20,21,22-octahydro-18aH,24H -18,15-(metheno)pyrido[2,1-l]pyrimido[6,1-h][1,4,7,9,10,13]benzoxapentaazacyclohexadecin e-8(9H)-sulfonyl]carbamate (40 mg, colorless amorphous).
LCMS (ESI) m/z 669 [M+H]⁺, t_{R} = 0.784 min, mode N.

Step 178-2: By using the same approach as in Step 5-2, the title compound (25 mg, colorless powder) was obtained from the compound (40 mg) obtained in Step 178-1.
LCMS (ESI) m/z 569 [M+H]⁺, t_{R} = 0.625 min, mode N.

### Example 179: Synthesis of (18aS)-13-(3-hydroxyazetidin-1-yl)-8-(methanesulfonyl)-2,11-dimethyl-6,7,8,9,10,11,19,20, 21,22-decahydro-18aH,24H-18,15-(metheno)pyrido[2,1-l]pyrimido[6,1-h][1,4,7,9,10,13]ben zoxapentaazacyclohexadecin-24-one

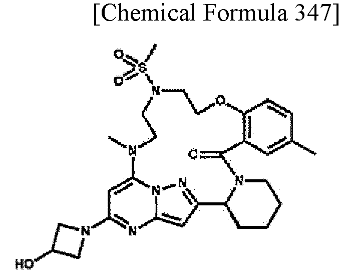

Step 179-1: By using the same approaches as in Step 1-1 to Step 1-3, (18aS)-13-chloro-8-(methanesulfonyl)-2,11-dimethyl-6,7,8,9,10,11,19,20,21,22-decahydro-1 8aH,24H-18,15-(metheno)pyrido[2,1-l]pyrimido[6,1-h][1,4,7,9,10,13]benzoxapentaazacyclo hexadecin-24-one (0.29 g, colorless amorphous) was obtained from the compound (0.34 g) obtained in Reference Example 1-1 (3) and the compound (0.37 g) obtained in Reference Example 2-70.
LCMS (ESI) m/z 547 [M+H]⁺, t_{R} = 1.118 min, mode N.

Step 179-2: By using the same approach as in Step 3-1, the title compound (46 mg, pink amorphous) was obtained from the compound (97 mg) obtained in Step 179-1 and azetidine hydrochloride (0.19 g).
LCMS (ESI) m/z 584 [M+H]⁺, t_{R} = 0.634 min, mode N.

### Example 180: Synthesis of (18aS)-13-(3-aminoazetidin-1-yl)-8-(methanesulfonyl)-2,11-dimethyl-6,7,8,9,10,11,19,20,21, 22-decahydro-18aH,24H-18,15-(metheno)pyrido[2,1-l]pyrimido[6,1-h][1,4,7,9,10,13]benzox apentaazacyclohexadecin-24-one

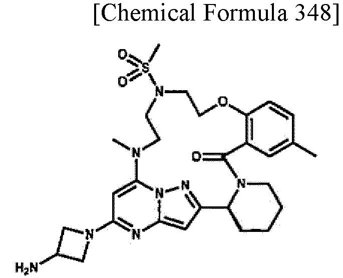

By using the same approaches as in Step 1-4 and Step 4-2, the title compound (38 mg, colorless amorphous) was obtained from the compound (97 mg) obtained in Step 179-1 and benzyl N-(azetidin-3-yl)carbamate (0.18 g).
LCMS (ESI) m/z 583 [M+H]⁺, t_{R} = 0.532 min, mode N.

### Example 181: Synthesis of (18aS)-13-(azetidin-1-yl)-8-(methanesulfonyl)-2,11-dimethyl-6,7,8,9,10,11,19,20,21,22-deca hydro-18aH,24H-18,15-(metheno)pyrido[2,1-l]pyrimido[6,1-h][1,4,7,9,10,13]benzoxapentaa zacyclohexadecin-24-one

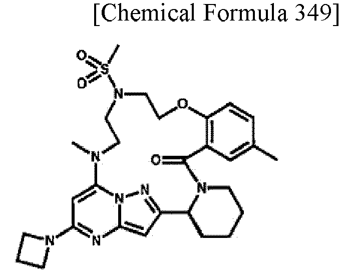

By using the same approach as in Step 1-4, the title compound (33 mg, colorless powder) was obtained from the compound (48 mg) obtained in Step 179-1 and azetidine (30 µL).
LCMS (ESI) m/z 568 [M+H]⁺, t_{R} = 0.678 min, mode N.

### Example 182: Synthesis of (18aS)-13-[(2-aminoethyl)amino]-8-(methanesulfonyl)-2,11-dimethyl-6,7,8,9,10,11,19,20,21, 22-decahydro-18aH,24H-18,15-(metheno)pyrido[2,1-l]pyrimido[6,1-h][1,4,7,9,10,13]benzox apentaazacyclohexadecin-24-one hydrochloride

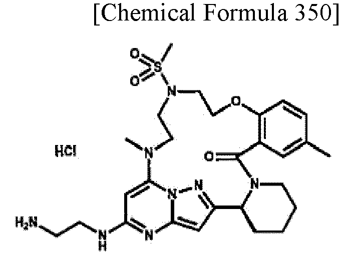

By using the same approaches as in Step 1-4 and Step 13-2, the title compound (37 mg, colorless powder) was obtained from the compound (48 mg) obtained in Step 179-1 and tert-butyl N-(2-aminoethyl)carbamate (69 µL).
LCMS (ESI) m/z 571 [M+H]⁺, t_{R} = 0.529 min, mode N.

### Example 183: Synthesis of (18aS)-13-(azetidin-1-yl)-8-benzyl-2,11-dimethyl-6,7,8,9,10,11,19,20,21,22-decahydro-18aH ,24H-18,15-(metheno)pyrido[2,1-l]pyrimido[6,1-h][1,4,7,9,10,13]benzoxapentaazacyclohexa decin-24-one

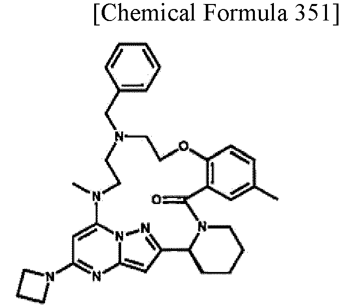

By using the same approach as in Step 22-1, the title compound (6.6 mg, colorless amorphous) was obtained from the compound (29 mg) obtained in Step 168-2, using benzaldehyde instead of formaldehyde.
LCMS (ESI) m/z 580 [M+H]⁺, t_{R} = 0.578 min, mode N.

### Example 184: Synthesis of (18aS)-13-(azetidin-1-yl)-2,11-dimethyl-8-(pyridin-2-yl)-6,7,8,9,10,11,19,20,21,22-decahydr o-18aH,24H-18,15-(metheno)pyrido[2,1-l]pyrimido[6,1-h][1,4,7,9,10,13]benzoxapentaazacy clohexadecin-24-one

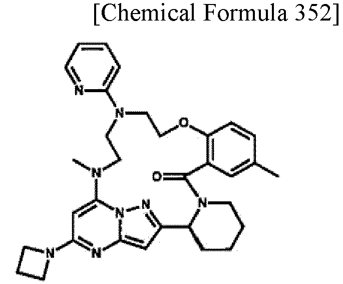

To a solution of the compound (20 mg) obtained in Step 168-2 in toluene (0.41 mL), 2-bromopyridine (3.9 µL), Pd(OAc)₂ (0.91 mg), PPh₃ (21 mg), and Na^{t}OBu (12 mg) were added, and the mixture was stirred at 80°C for 2 hours. Furthermore, Pd(PPh₃)₄ (4.7 mg) was added thereto and the mixture was stirred at 80°C for 1 hour, and 2-bromopyridine (3.9 µL) was added thereto and the mixture was further stirred at 110°C for 1 hour. The reaction system was purified as it was by PTLC (NH: 0.5 mm × 2 plates, mobile phase:
EtOAc/MeOH = 20/1) to afford the title compound (5.3 mg, light yellow powder).
LCMS (ESI) m/z 567 [M+H]⁺, t_{R} = 0.536 min, mode N.

### Example 185: Synthesis of (18aS)-13-(azetidin-1-yl)-8-(2-hydroxyethyl)-2,11-dimethyl-6,7,8,9,10,11,19,20,21,22-decah ydro-18aH,24H-18,15-(metheno)pyrido[2,1-l]pyrimido[6,1-h][1,4,7,9,10,13]benzoxapentaaz acyclohexadecin-24-one

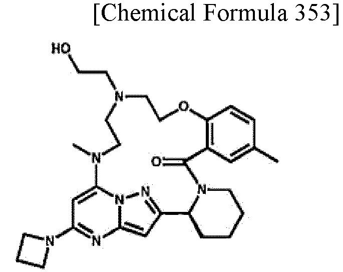

By using the same approaches as in Step 22-1 and Step 13-2, the title compound (12 mg, colorless powder) was obtained from the compound (39 mg) obtained in Step 168-2, using {[tert-butyl(dimethyl)silyl]oxy}acetaldehyde instead of formaldehyde.
LCMS (ESI) m/z 534 [M+H]⁺, t_{R} = 0.809 min, mode H.

### Example 186: Synthesis of (18aS)-13-(azetidin-1-yl)-8-(cyclopropylmethyl)-2,11-dimethyl-6,7,8,9,10,11,19,20,21,22-de cahydro-18aH,24H-18,15-(metheno)pyrido[2,1-l]pyrimido[6,1-h][1,4,7,9,10,13]benzoxapent aazacyclohexadecin-24-one

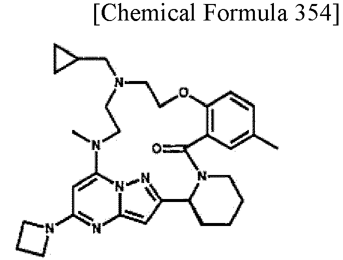

By using the same approach as in Step 22-1, the title compound (28 mg, orange amorphous) was obtained from the compound (20 mg) obtained in Step 168-2, using cyclopropanecarbaldehyde instead of formaldehyde.
LCMS (ESI) m/z 544 [M+H]⁺, t_{R} = 0.894 min, mode N.

### Example 187: Synthesis of (18aS)-13-(azetidin-1-yl)-8-tert-butyl-2,11-dimethyl-6,7,8,9,10,11,19,20,21,22-decahydro-18 aH,24H-18,15-(metheno)pyrido[2,1-l]pyrimido[6,1-h][1,4,7,9,10,13]benzoxapentaazacycloh exadecin-24-one

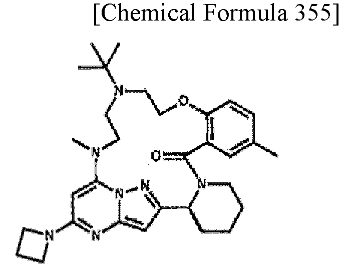

By using the same approaches as in Step 1-1 to Step 1-4, the title compound (17 mg, light yellow amorphous) was obtained from the compound (73 mg) obtained in Reference Example 1-1 (3) and the compound (60 mg) obtained in Reference Example 2-112, and azetidine (80 µL).
LCMS (ESI) m/z 546 [M+H]⁺, t_{R} = 0.534 min, mode N.

### Example 188: Synthesis of (18aS)-13-(azetidin-1-yl)-8-cyclopropyl-2,11-dimethyl-6,7,8,9,10,11,19,20,21,22-decahydro-18aH,24H-18,15-(metheno)pyrido[2,1-l]pyrimido[6,1-h][1,4,7,9,10,13]benzoxapentaazacycl ohexadecin-24-one

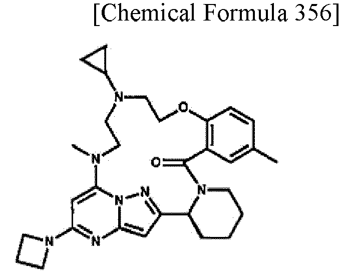

By using the same approaches as in Step 1-1 to Step 1-4, the title compound (9.5 mg, light yellow amorphous) was obtained from the compound (0.13 g) obtained in Reference Example 1-1 (3) and the compound (0.10 g) obtained in Reference Example 2-78, and azetidine (0.15 mL).
LCMS (ESI) m/z 530 [M+H]⁺, t_{R} = 0.882 min, mode H.

### Example 189: Synthesis of (18aS)-13-(azetidin-1-yl)-5-(methanesulfonyl)-2,11-dimethyl-6,7,8,9,10,11,19,20,21,22-deca hydro-18aH-18,15-(metheno)pyrido[2,1-l]pyrimido[6,1-h][1,4,7,9,10,13]benzohexaazacyclo hexadecin-24(5H)-one

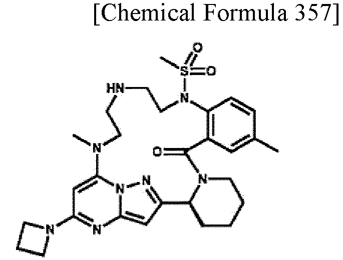

By using the same approaches as in Step 1-1 to Step 1-3, Step 3-1, and Step 4-2, the title compound (49 mg, colorless powder) was obtained from the compound (0.25 g) obtained in Reference Example 1-1 (3) and the compound (0.29 g) obtained in Reference Example 2-71, and azetidine hydrochloride (0.16 g).
LCMS (ESI) m/z 567 [M+H]⁺, t_{R} = 0.338 min, mode N.

### Example 190: Synthesis of (18aS)-13-cyclopropyl-2,11-dimethyl-6H,8H,9H,10H,11H,18aH,19H,20H,21H,22H,24H-spi ro[18,15-(metheno)pyrido[2,1-l]pyrimido[6,1-h][1,4,7,9,10,13]benzoxapentaazacyclohexade cine-7,3'-oxetan]-24-one

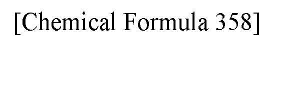

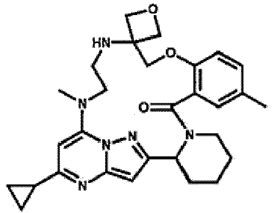

By using the same approaches as in Step 23-1, Step 5-2, Step 1-2, and Step 1-3, the title compound (90 mg, colorless amorphous) was obtained from the compound (0.38 g) obtained in Reference Example 1-6 (2) and the compound (0.34 g) obtained in Reference Example 2-77.
LCMS (ESI/APCI dual) m/z 517 [M+H]⁺, t_{R} = 0.821 min, mode H.

### Example 191: Synthesis of benzyl (18S)-3-[(3S)-3-aminopyrrolidin-1-yl]-18-ethyl-5,14,17-trimethyl-16-oxo-6,7,9,10,17,18-hex ahydro-16H-19,1-(metheno)pyrimido[6,1-h][1,4,7,9,10,13]benzoxapentaazacyclohexadecine-8(5H)-carboxylate hydrochloride

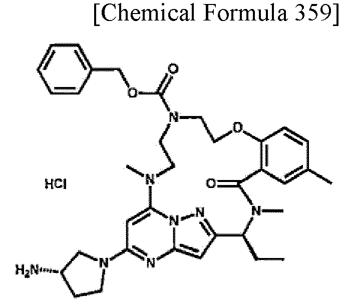

Step 191-1: By using the same approaches as in Step 1-1 to Step 1-4, benzyl (18S)-3-{(3S)-3-[(tert-butoxycarbonyl)amino]pyrrolidin-1-yl}-18-ethyl-5,14,17-trimethyl-16 -oxo-6,7,9,10,17,18-hexahydro-16H-19,1-(metheno)pyrimido[6,1-h][1,4,7,9,10,13]benzoxap entaazacyclohexadecine-8(5H)-carboxylate (0.17 g, colorless amorphous) was obtained from the compound (0.34 g) obtained in Reference Example 1-15 and the compound (0.59 g) obtained in Reference Example 2-66, and tert-butyl N-[(3S)-pyrrolidin-3-yl]carbamate (0.24 g).
LCMS (ESI) m/z 741 [M+H]⁺, t_{R} = 0.917 min, mode N.

Step 191-2: By using the same approach as in Step 13-2, the title compound (8.7 mg, colorless powder) was obtained from the compound (15 mg) obtained in Step 191-1.
LCMS (ESI) m/z 641 [M+H]⁺, t_{R} = 0.624 min, mode N.

### Example 192: Synthesis of (18S)-3-[(3S)-3-aminopyrrolidin-1-yl]-18-ethyl-5,14,17-trimethyl-5,6,7,8,9,10,17,18-octahyd ro-16H-19,1-(metheno)pyrimido[6,1-h][1,4,7,9,10,13]benzoxapentaazacyclohexadecin-16-on e hydrochloride

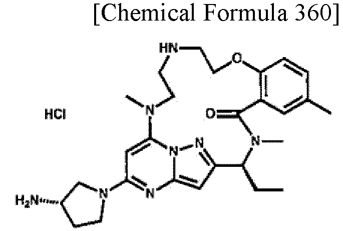

Step 192-1: By using the same approach as in Step 4-2, tert-butyl {(3S)-1-[(18S)-18-ethyl-5,14,17-trimethyl-16-oxo-5,6,7,8,9,10,17,18-octahydro-16H-19,1-( metheno)pyrimido[6,1-h][1,4,7,9,10,13]benzoxapentaazacyclohexadecin-3-yl]pyrrolidin-3-yl }carbamate (0.11 g, colorless powder) was obtained from the compound (0.15 g) obtained in Step 191-1.
LCMS (ESI) m/z 607 [M+H]⁺, t_{R} = 0.618 min, mode N.

Step 192-2: By using the same approach as in Step 13-2, the title compound (17 mg, colorless powder) was obtained from the compound (20 mg) obtained in Step 192-1.
LCMS (ESI) m/z 507 [M+H]⁺, t_{R} = 0.614 min, mode H.

### Example 193: Synthesis of (18S)-8-acetyl-3-[(3S)-3-aminopyrrolidin-1-yl]-18-ethyl-5,14,17-trimethyl-5,6,7,8,9, 10,17,18 -octahydro-16H-19,1-(metheno)pyrimido[6,1-h][1,4,7,9,10,13]benzoxapentaazacyclohexadec in-16-one hydrochloride

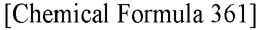

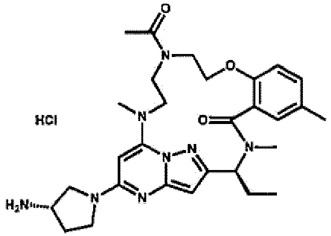

Step 193-1: To a solution of the compound (25 mg) obtained in Step 151 in CHCl₃ (0.41 mL), Et₃N (17 µL) and acetic anhydride (4.3 µL) were added at 0°C, and the mixture was stirred at 0°C for 1 hour. To the reaction liquid, a Sat. NH₄Cl aq. was added, and the mixture was extracted with CHCl₃. The organic layer was collected by using Phase Separator and concentrated under reduced pressure. The obtained residue was purified by NH type silica gel column chromatography (mobile phase: EtOAc/CHCl₃ = 100/0 to 0/100; v/v) to afford tert-butyl {(3S)-1-[(18S)-8-acetyl-18-ethyl-5,14,17-trimethyl-16-oxo-5,6,7,8,9,10,17,18-octahydro-16 H-19,1-(metheno)pyrimido[6,1-h][1,4,7,9,10,13]benzoxapentaazacyclohexadecin-3-yl]pyrrol idin-3-yl}carbamate (25 mg, colorless powder).
LCMS (ESI) m/z 649 [M+H]⁺, t_{R} = 0.698 min, mode N.

Step 193-2: By using the same approach as in Step 13-2, the title compound (23 mg, colorless powder) was obtained from the compound (25 mg) obtained in Step 193-1. LCMS (ESI) m/z 549 [M+H]⁺, t_{R} = 0.766 min, mode H.

### Example 194: Synthesis of (18S)-3-[(3S)-3-aminopyrrolidin-1-yl]-18-ethyl-5,8,14,17-tetramethyl-5,6,7,8,9,10,17,18-oct ahydro-16H-19,1-(metheno)pyrimido[6,1-h][1,4,7,9,10,13]benzoxapentaazacyclohexadecin-16-one hydrochloride

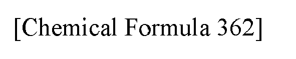

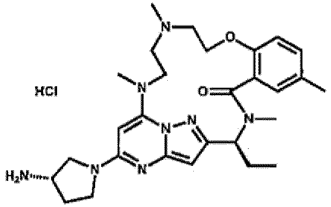

By using the same approaches as in Step 137 and Step 13-2, the title compound (19 mg, colorless powder) was obtained from the compound (25 mg) obtained in Step 192-1. LCMS (ESI) m/z 521 [M+H]⁺, t_{R} = 0.653 min, mode H.

### Example 195: Synthesis of (18S)-3-[(3S)-3-aminopyrrolidin-1-yl]-18-ethyl-8-(methanesulfonyl)-5,14,17-trimethyl-5,6,7, 8,9,10,17,18-octahydro-16H-19,1-(metheno)pyrimido[6,1-h][1,4,7,9,10,13]benzoxapentaazac yclohexadecin-16-one

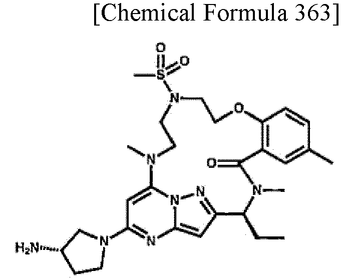

By using the same approaches as in Step 1-1 to Step 1-4, the title compound (0.11 g, colorless amorphous) was obtained from the compound (0.50 g) obtained in Reference Example 1-15 and the compound (0.39 g) obtained in Reference Example 2-70.
LCMS (ESI) m/z 585 [M+H]⁺, t_{R} = 0.827 min, mode H.

### Example 196: Synthesis of (18aS)-13-(azetidin-1-yl)-2-fluoro-11,12-dimethyl-6,7,8,9,10,11,19,20,21,22-decahydro-18a H,24H-18,15-(metheno)pyrido[2,1-l]pyrimido[6,1-h][1,4,7,9,10,13]benzoxapentaazacyclohe xadecin-24-one

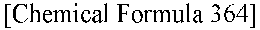

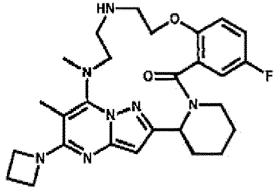

By using the same approaches as in Step 1-1 to Step 1-4 and Step 4-2, the title compound (45 mg, colorless powder) was obtained from the compound (0.17 g) obtained in Reference Example 1-3, the compound (0.26 g) obtained in Reference Example 2-64, and azetidine (0.11 mL).
LCMS (ESI) m/z 508 [M+H]⁺, t_{R} = 0.588 min, mode N.

### Example 197: Synthesis of (18aS)-13-(azetidin-1-yl)-5-(methanesulfonyl)-2,11,12-trimethyl-6,7,8,9,10,11,19,20,21,22-d ecahydro-18aH-18,15-(metheno)pyrido[2,1-l]pyrimido[6,1-h][1,4,7,9,10,13]benzohexaazacy clohexadecin-24(5H)-one

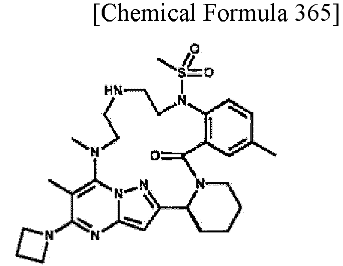

By using the same approaches as in Step 1-1 to Step 1-3, Step 3-1, and Step 4-2, the title compound (55 mg, colorless amorphous) was obtained from the compound (0.28 g) obtained in Reference Example 1-3, the compound (0.44 g) obtained in Reference Example 2-71, and azetidine hydrochloride (0.15 g).
LCMS (ESI) m/z 581 [M+H]⁺, t_{R} = 0.599 min, mode N.

### Example 198: Synthesis of (18aS)-13-(azetidin-1-yl)-2,12-difluoro-11-methyl-6,7,8,9,10,11,19,20,21,22-decahydro-18a H,24H-18,15-(metheno)pyrido[2,1-l]pyrimido[6,1-h][1,4,7,9,10,13]benzoxapentaazacyclohe xadecin-24-one

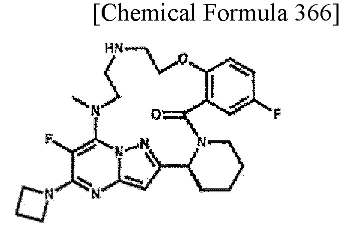

By using the same approaches as in Step 1-1, Step 1-4, Step 1-2, Step 1-3, and Step 4-2, the title compound (7.7 mg, colorless powder) was obtained from the compound (0.80 g) obtained in Reference Example 1-4, the compound (0.24 g) obtained in Reference Example 2-64, and azetidine (0.14 mL).
LCMS (ESI) m/z 512 [M+H]⁺, t_{R} = 0.633 min, mode N.

### Example 199: Synthesis of (18aS)-13-[(3S)-3-aminopyrrolidin-1-yl]-2,12-difluoro-11-methyl-6,7,8,9,10,11,19,20,21,22-decahydro-18aH,24H-18,15-(metheno)pyrido[2,1-l]pyrimido[6,1-h][1,4,7,9,10,13]benzoxape ntaazacyclohexadecin-24-one

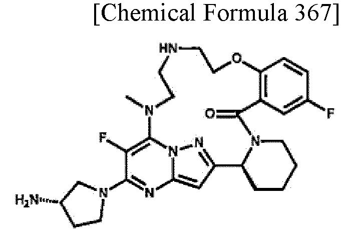

By using the same approaches as in Step 1-1, Step 1-4, Step 1-2, Step 1-3, Step 13-2, and Step 4-2, the title compound (5.5 mg, colorless powder) was obtained from the compound (0.54 g) obtained in Reference Example 1-4, the compound (80 mg) obtained in Reference Example 2-64, and tert-butyl N-[(3S)-pyrrolidin-3-yl]carbamate (62 mg).
LCMS (ESI) m/z 541 [M+H]⁺, t_{R} = 0.219 min, mode N.

### Example 200: Synthesis of (18aS)-13-cyclobutyl-2-fluoro-11-methyl-6,7,8,9, 10,11, 19,20,21,22-decahydro-18aH,24H-18 ,15-(metheno)pyrido[2,1-l]pyrimido[6,1-h][1,4,7,9,10,13]benzoxapentaazacyclohexadecin-24 -one

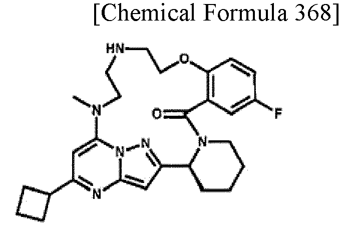

By using the same approaches as in Step 23-1, Step 13-2, Step 1-2, Step 1-3, and Step 4-2, the title compound (53 mg, colorless amorphous) was obtained from the compound (0.13 g) obtained in Reference Example 1-11 and the compound (0.16 g) obtained in Reference Example 2-64.
LCMS (ESI) m/z 493 [M+H]⁺, t_{R} = 0.928 min, mode H.

### Example 201: Synthesis of (18aS)-2-fluoro-11,12,13 -trimethyl-6,7,8,9,10,11,19,20,21,22-decahydro-18aH,24H-18,15-( metheno)pyrido[2,1-l]pyrimido[6,1-h][1,4,7,9,10,13]benzoxapentaazacyclohexadecin-24-one

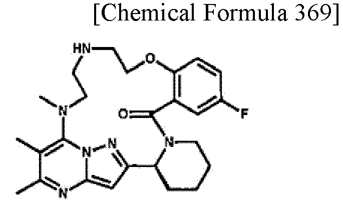

By using the same approaches as in Step 23-1, Step 13-2, Step 1-2, Step 1-3, and Step 4-2, the title compound (34 mg, colorless powder) was obtained from the compound (0.20 g) obtained in Reference Example 1-10 and the compound (0.24 g) obtained in Reference Example 2-64.
LCMS (ESI) m/z 467 [M+H]⁺, t_{R} = 0.560 min, mode N.

### Example 202: Synthesis of (17S)-3-[(3S)-3-aminopyrrolidin-1-yl]-13-chloro-17-ethyl-5,16-dimethyl-6,7,8,9,16,17-hexah ydro-5H,15H-18,1-(metheno)pyrimido[6,1-g][1,6,8,9,12]benzoxatetraazacyclopentadecin-15 -one

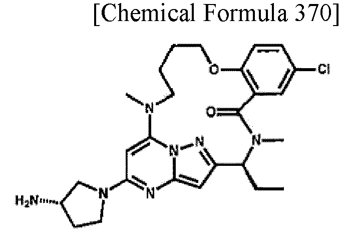

Step 202-1: By using the same approach as in Step 1-1, methyl 5-chloro-2-[4-[[5-chloro-2-[(1S)-1-(methylamino)propyl]pyrazolo[1,5-a]pyrimidin-7-yl]-met hyl-amino]butoxy]benzoate (0.26 g, yellow oily matter) was obtained from the compound (0.50 g) obtained in Reference Example 1-15 and the compound (0.36 g) obtained in Reference Example 2-26.
LCMS (ESI) m/z 494 [M+H]⁺, t_{R} = 0.766 min, mode N.

Step 202-2: To a solution of the compound (0.26 g) obtained in Step 202-1 in THF (1.5 mL), sodium trimethylsilanolate (0.66 mL) was added, and the mixture was stirred at room temperature for 10 minutes. To the reaction solution, a Sat. NH₄Cl aq. and CHCl₃ were added, and the separating operation was carried out. The aqueous layer was separated using Phase Separator, the organic layer was then concentrated under reduced pressure, and the resulting residue was purified by NH type silica gel chromatography (mobile phase: CHCl₃/MeOH = 60/40 to 35/65; v/v) to afford 5-chloro-2-[4-[[5-chloro-2-[(1S)-1-(methylamino)propyl]pyrazolo[1,5-a]pyrimidin-7-yl]-met hyl-amino]butoxy]benzoic acid (59 mg, colorless powder).
LCMS (ESI) m/z 480 [M+H]⁺, t_{R} = 0.671 min, mode N.

Step 202-3: By using the same approach as in Step 1-3, (17S)-3,13-dichloro-17-ethyl-5,16-dimethyl-6,7,8,9,16,17-hexahydro-5H,15H-18,1-(metheno )pyrimido[6,1-g][1,6,8,9,12]benzoxatetraazacyclopentadecin-15-one (39 mg, yellow oily matter) was obtained from the compound (59 mg) obtained in Step 202-2.
LCMS (ESI) m/z 462 [M+H]⁺, t_{R} = 1.231 min, mode N.

Step 202-4: By using the same approach as in Step 1-4, the title compound (11 mg, colorless amorphous) was obtained from the compound (19 mg) obtained in Step 202-3 and (3S)-pyrrolidin-3-amine (18 µL).
LCMS (ESI) m/z 512 [M+H]⁺, t_{R} = 0.499 min, mode N.

### Example 203: Synthesis of (17S)-3-[(2-aminoethyl)amino]-13-chloro-17-ethyl-5,16-dimethyl-6,7,8,9,16,17-hexahydro-5 H,15H-18,1-(metheno)pyrimido[6,1-g][1,6,8,9,12]benzoxatetraazacyclopentadecin-15-one hydrochloride

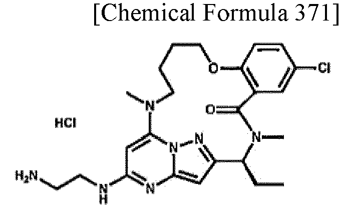

By using the same approaches as in Step 1-4 and Step 13-2, the title compound (1.4 mg, colorless powder) was obtained from the compound (19 mg) obtained in Step 202-3 and tert-butyl N-(2-aminoethyl)carbamate (66 µL).
LCMS (ESI) m/z 486 [M+H]⁺, t_{R} = 0.542 min, mode N.

### Example 204: Synthesis of (17S)-3-[(3S)-3-aminopyrrolidin-1-yl]-13-chloro-5,17-diethyl-16-methyl-6,7,8,9,16,17-hexah ydro-5H,15H-18,1-(metheno)pyrimido[6,1-g][1,6,8,9,12]benzoxatetraazacyclopentadecin-15 -one

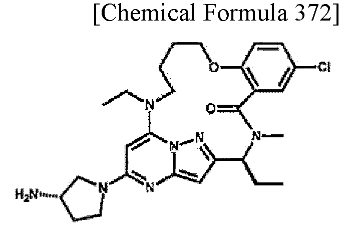

By using the same approaches as in Step 1-1 to Step 1-4, the title compound (48 mg, colorless powder) was obtained from the compound (0.40 g) obtained in Reference Example 1-15 and the compound (0.31 g) obtained in Reference Example 2-51.
LCMS (ESI) m/z 526 [M+H]⁺, t_{R} = 0.518 min, mode N.

### Example 205: Synthesis of (7S)-13-[(3S)-3-aminopyrrolidin-1-yl]-3-chloro-7-ethyl-6,15-dimethyl-6,7,15,16,17,18,19,20 -octahydro-5H-8,11-(metheno)pyrido[2,3-j]pyrimido[1,6-b][1,2,4,9,13]pentaazacyclopentade cin-5-one

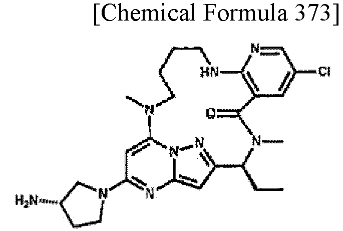

By using the same approaches as in Step 1-1 to Step 1-4, the title compound (52 mg, colorless amorphous) was obtained from the compound (0.20 g) obtained in Reference Example 1-15 and the compound (0.14 g) obtained in Reference Example 2-62.
LCMS (ESI) m/z 512 [M+H]⁺, t_{R} = 0.469 min, mode N.

### Example 206: Synthesis of (17S)-3-[(3S)-3-aminopyrrolidin-1-yl]-17-ethyl-5,13,16-trimethyl-6,7,8,9,16,17-hexahydro-5 H,15H-18,1-(metheno)pyrimido[6,1-g][1,6,8,9,12]benzoxatetraazacyclopentadecin-15-one hydrochloride

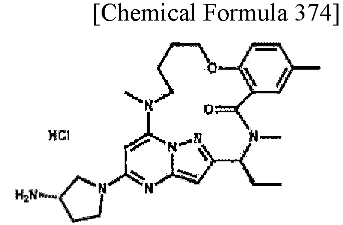

Step 206-1: To a solution of the compound (0.18 g) obtained in Reference Example 1-15 and the compound (0.22 g) obtained in Reference Example 2-27 in MeCN (4.0 mL) and water (2.0 mL), NaHCO₃ (0.51 g) was added, and the mixture was stirred at 65°C for 3 hours. To the reaction solution, water and EtOAc were added, and the separating operation was carried out. The organic layer was washed with Brine, dried over MgSO₄, filtered, and then the organic layer was concentrated under reduced pressure. The obtained residue was purified by NH type silica gel column chromatography (mobile phase: hexane/EtOAc = 50/50 to 0/100; v/v) to afford methyl 2-{4-[{5-chloro-2-[(1S)-1-(methylamino)propyl]pyrazolo[1,5-a]pyrimidin-7-yl}(methyl)ami no]butoxy}-5-methylbenzoate (90 mg, colorless amorphous).
LCMS (ESI/APCI dual) m/z 442 [M+H]⁺, t_{R} = 1.088, 1.124 min, mode N.

Step 206-2: By using the same approaches as in Step 1-2 to Step 1-4 and Step 13-2, the title compound (25 mg, colorless amorphous) was obtained from the compound (90 mg) obtained in Step 206-1 and tert-butyl N-[(3S)-pyrrolidin-3-yl]carbamate (0.15 g).
LCMS (ESI) m/z 492 [M+H]⁺, t_{R} = 0.807, 0.850 min, mode H.

### Example 207: Synthesis of (17S)-3-[(2-aminoethyl)amino]-17-ethyl-5,13,16-trimethyl-6,7,8,9,16,17-hexahydro-5H,15H-18,1-(metheno)pyrimido[6,1-g][1,6,8,9,12]benzoxatetraazacyclopentadecin-15-one hydrochloride

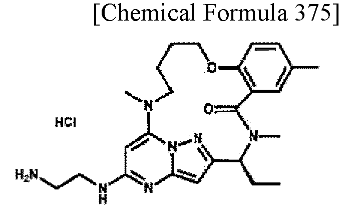

By using the same approaches as in Step 1-4 and Step 13-2, the title compound (30 mg, colorless powder) was obtained from the compound (60 mg) obtained in Step 206-1 and tert-butyl N-(2-aminoethyl)carbamate (0.22 g).
LCMS (ESI) m/z 466 [M+H]⁺, t_{R} = 0.461, 0.508 min, mode N.

### Example 208: Synthesis of (17S)-3-[(2-amino-2-methylpropyl)amino]-17-ethyl-5,13,16-trimethyl-6,7,8,9,16,17-hexahyd ro-5H,15H-18,1-(metheno)pyrimido[6,1-g][1,6,8,9,12]benzoxatetraazacyclopentadecin-15-o ne hydrochloride

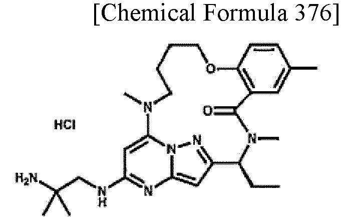

By using the same approach as in Step 1-4, the title compound (35 mg, colorless powder) was obtained from the compound (60 mg) obtained in Step 206-1 and 2-methylpropane-1,2-diamine (0.12 g).
LCMS (ESI) m/z 494 [M+H]⁺, t_{R} = 0.540, 0.579 min, mode N.

### Example 209: Synthesis of (17S)-3-(3-aminoazetidin-1-yl)-17-ethyl-5,13,16-trimethyl-6,7,8,9,16,17-hexahydro-5H,15H-18,1-(metheno)pyrimido[6,1-g][1,6,8,9,12]benzoxatetraazacyclopentadecin-15-one

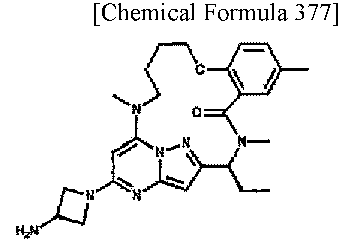

By using the same approaches as in Step 3-1 and Step 5-2, the title compound (68 mg, colorless powder) was obtained from the compound (70 mg) obtained in Step 206-1 and 3-N-BOC-amino-azetidine mesylate salt (0.21 g).
LCMS (ESI) m/z 478 [M+H]⁺, t_{R} = 0.888 min, mode H.

### Example 210: Synthesis of (17S)-3-(3-aminopropyl)-17-ethyl-5,13,16-trimethyl-6,7,8,9,16,17-hexahydro-5H,15H-18,1-( metheno)pyrimido[6,1-g][1,6,8,9,12]benzoxatetraazacyclopentadecin-15-one hydrochloride

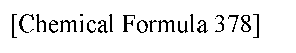

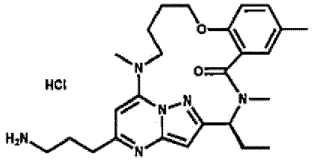

By using the same approaches as in Step 28-1, Step 4-2, and Step 13-2, the title compound (25 mg, colorless amorphous) was obtained from the compound (70 mg) obtained in Step 206-1, using tert-butyl N-prop-2-ynylcarbamate instead of propargyl alcohol. LCMS (ESI) m/z 465 [M+H]⁺, t_{R} = 0.787, 0.826 min, mode H.

### Example 211: Synthesis of (17S)-3-[(3S)-3-aminopyrrolidin-1-yl]-13,17-diethyl-5,16-dimethyl-6,7,8,9,16,17-hexahydro-5H,15H-18,1-(metheno)pyrimido[6,1-g][1,6,8,9,12]benzoxatetraazacyclopentadecin-15-one

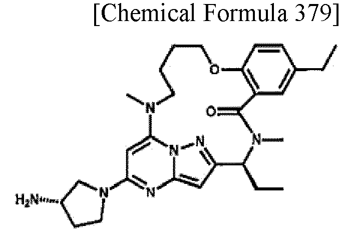

Step 211-1: By using the same approaches as in Step 1-1, Step 202-2, and Step 1-3, (17S)-3-chloro-13,17-diethyl-5,16-dimethyl-6,7,8,9,16,17-hexahydro-5H,15H-18,1-(metheno )pyrimido[6,1-g][1,6,8,9,12]benzoxatetraazacyclopentadecin-15-one (0.15 g, yellow oily matter) was obtained from the compound (0.50 g) obtained in Reference Example 1-15 and the compound (0.35 g) obtained in Reference Example 2-28.
LCMS (ESI) m/z 456 [M+H]⁺, t_{R} = 1.280 min, mode N.

Step 211-2: By using the same approach as in Step 1-4, the title compound (64 mg, colorless amorphous) was obtained from the compound (70 mg) obtained in Step 211-1. LCMS (ESI) m/z 506 [M+H]⁺, t_{R} = 0.528 min, mode N.

### Example 212: Synthesis of (17S)-3-[(2-aminoethyl)amino]-13,17-diethyl-5,16-dimethyl-6,7,8,9,16,17-hexahydro-5H,15 H-18,1-(metheno)pyrimido[6,1-g][1,6,8,9,12]benzoxatetraazacyclopentadecin-15-one hydrochloride

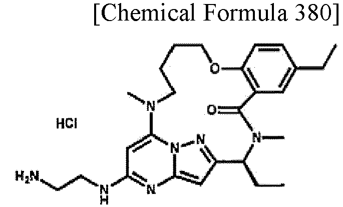

By using the same approaches as in Step 1-4 and Step 13-2, the title compound (26 mg, colorless powder) was obtained from the compound (70 mg) obtained in Step 211-1 and tert-butyl N-(2-aminoethyl)carbamate (0.25 g).
LCMS (ESI) m/z 480 [M+H]⁺, t_{R} = 0.572 min, mode N.

### Example 213: Synthesis of (17S)-3-[(3S)-3-aminopyrrolidin-1-yl]-17-ethyl-5,16-dimethyl-6,7,8,9,16,17-hexahydro-5H,1 5H-18,1-(metheno)pyrimido[6,1-g][1,6,8,9,12]benzoxatetraazacyclopentadecin-15-one

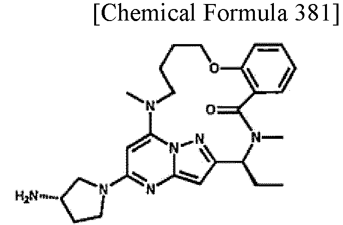

Step 213-1: By using the same approaches as in Step 1-1, Step 202-2, and Step 1-3, (17S)-3-chloro-17-ethyl-5,16-dimethyl-6,7,8,9,16,17-hexahydro-5H,15H-18,1-(metheno)pyri mido[6,1-g][1,6,8,9,12]benzoxatetraazacyclopentadecin-15-one (0.16 g, yellow powder) was obtained from the compound (0.50 g) obtained in Reference Example 1-15 and the compound (0.37 g) obtained in Reference Example 2-119.
LCMS (ESI) m/z 428 [M+H]⁺, t_{R} = 1.145 min, mode N.

Step 213-2: By using the same approach as in Step 1-4, the title compound (44 mg, colorless amorphous) was obtained from the compound (71 mg) obtained in Step 213-1. LCMS (ESI) m/z 478 [M+H]⁺, t_{R} = 0.788 min, mode H.

### Example 214: Synthesis of (17S)-3-[(2-aminoethyl)amino]-17-ethyl-5,16-dimethyl-6,7,8,9,16,17-hexahydro-5H,15H-18, 1-(metheno)pyrimido[6,1-g][1,6,8,9,12]benzoxatetraazacyclopentadecin-15-one hydrochloride

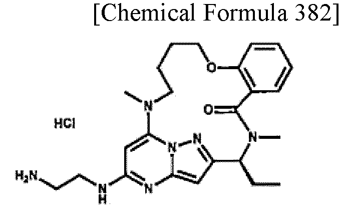

By using the same approaches as in Step 1-4 and Step 13-2, the title compound (12 mg, colorless powder) was obtained from the compound (71 mg) obtained in Step 213-1 and tert-butyl N-(2-aminoethyl)carbamate (0.27 g).
LCMS (ESI) m/z 452 [M+H]⁺, t_{R} = 0.834 min, mode H.

### Example 215: Synthesis of (17S)-3-[(3S)-3-aminopyrrolidin-1-yl]-17-ethyl-13,16-dimethyl-6,7,8,9,16,17-hexahydro-5H, 15H-18,1-(metheno)pyrimido[6,1-g][1,6,8,9,12]benzoxatetraazacyclopentadecin-15-one

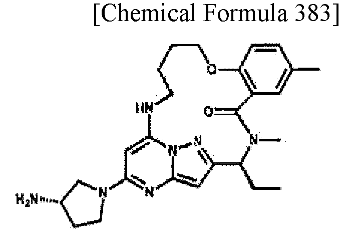

By using the same approaches as in Step 1-1 to Step 1-4, the title compound (4.3 mg, light pink amorphous) was obtained from the compound (0.15 g) obtained in Reference Example 1-15 and the compound (0.12 g) obtained in Reference Example 2-44.
LCMS (ESI) m/z 478 [M+H]⁺, t_{R} = 0.799, 0.838 min, mode H.

### Example 216: Synthesis of (17S)-3-[(3S)-3-aminopyrrolidin-1-yl]-17-ethyl-5,13,16-trimethyl-5,6,7,8,9,10,16,17-octahyd ro-15H-18,1-(metheno)pyrimido[6,1-g][1,6,8,9,12]benzopentaazacyclopentadecin-15-one

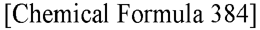

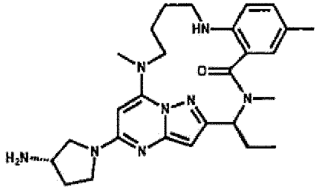

Step 216-1: By using the same approaches as in Step 1-1 to Step 1-3, (17S)-3-chloro-17-ethyl-5,13,16-trimethyl-5,6,7,8,9,10,16,17-octahydro-15H-18,1-(metheno) pyrimido[6,1-g][1,6,8,9,12]benzopentaazacyclopentadecin-15-one (66 mg, colorless amorphous) was obtained from the compound (0.30 g) obtained in Reference Example 1-15 and the compound (0.22 g) obtained in Reference Example 2-60.
LCMS (ESI) m/z 441 [M+H]⁺, t_{R} = 1.236 min, mode N.

Step 216-2: By using the same approach as in Step 1-4, the title compound (41 mg, colorless amorphous) was obtained from the compound (40 mg) obtained in Step 216-1. LCMS (ESI) m/z 491 [M+H]⁺, t_{R} = 0.489 min, mode N.

### Example 217: Synthesis of (17S)-3-(azetidin-1-yl)-17-ethyl-5,13,16-trimethyl-5,6,7,8,9,10,16,17-octahydro-15H-18,1-( metheno)pyrimido[6,1-g][1,6,8,9,12]benzopentaazacyclopentadecin-15-one

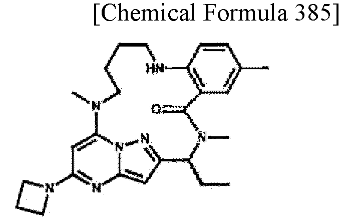

By using the same approach as in Step 3-1, the title compound (5.8 mg, colorless amorphous) was obtained from the compound (20 mg) obtained in Step 216-1 and azetidine hydrochloride (42 mg).
LCMS (ESI) m/z 462 [M+H]⁺, t_{R} = 0.712 min, mode N.

### Example 218: Synthesis of (17S)-3-[(3S)-3-aminopyrrolidin-1-yl]-17-ethyl-5,10,13,16-tetramethyl-5,6,7,8,9,10,16,17-oc tahydro-15H-18,l-(metheno)pyrimido[6,1-g][1,6,8,9,12]benzopentaazacyclopentadecin-15-o ne

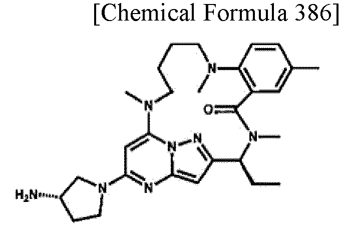

By using the same approaches as in Step 1-1 to Step 1-4, the title compound (22 mg, colorless amorphous) was obtained from the compound (0.19 g) obtained in Reference Example 1-15 and the compound (0.14 g) obtained in Reference Example 2-61.
LCMS (ESI) m/z 505 [M+H]⁺, t_{R} = 0.711 min, mode H.

### Example 219: Synthesis of (3S)-1-[(17S)-17-ethyl-5,13,16-trimethyl-6,7,8,9,16,17-hexahydro-5H,15H-18,1-(metheno)p yrimido[6,1-g][1,6,8,9,12]benzoxatetraazacyclopentadecin-3-yl]pyrrolidin-3-amine

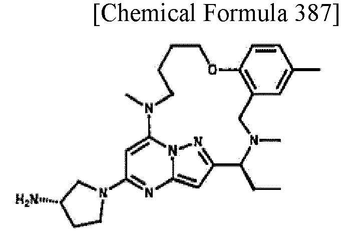

Step 219-1: By using the same approach as in Step 1-1, methyl 2-[3-[[5-chloro-2-[(1S)-1-(methylamino)propyl]pyrazolo[1,5-a]pyrimidin-7-yl]-methyl-amin o]propoxy]-5-methyl-benzoate (0.12 g, colorless oily matter) was obtained from the compound (0.32 g) obtained in Reference Example 1-15 and the compound (0.19 g) obtained in Reference Example 2-27.
LCMS (ESI) m/z 474 [M+H]⁺, t_{R} = 0.739 min, mode N.

Step 219-2: A solution of the compound (0.12 g) obtained in Step 219-1 in THF (2.4 mL) was cooled to 0°C, DIBAL-H (0.72 mL, 1.0 M in toluene) was then added thereto, and the mixture was stirred at 0°C for 30 minutes. Then, DIBAL-H (0.72 mL, 1.0 M in toluene) was added thereto, and the mixture was further stirred at 0°C for 30 minutes. To the reaction solution, Et₂O and a saturated aqueous Rochelle salt solution were added, and the mixture was stirred for 18 hours. The separating operation was carried out, the aqueous layer was separated using Phase Separator, and then the organic layer was concentrated under reduced pressure. The obtained residue was purified by NH type silica gel column chromatography (mobile phase: hexane/EtOAc = 90/10 to 0/100; v/v) to afford (17S)-3-chloro-17-ethyl-5,13,16-trimethyl-6,7,8,9,16,17-hexahydro-5H,15H-18,1-(metheno) pyrimido[6,1-g][1,6,8,9,12]benzoxatetraazacyclopentadecine (52 mg, colorless oily matter). LCMS (ESI) m/z 428 [M+H]⁺, t_{R} = 0.712 min, mode N.

Step 219-3: By using the same approach as in Step 1-4, the title compound (28 mg, colorless amorphous) was obtained from the compound (48 mg) obtained in Step 219-2. LCMS (ESI) m/z 478 [M+H]⁺, t_{R} = 0.700 min, mode H.

### Example 220: Synthesis of (17S)-3-[(3S)-3-aminopyrrolidin-1-yl]-17-ethyl-4,5,13,16-tetramethyl-6,7,8,9,16,17-hexahyd ro-5H,15H-18,1-(metheno)pyrimido[6,1-g][1,6,8,9,12]benzoxatetraazacyclopentadecin-15-o ne

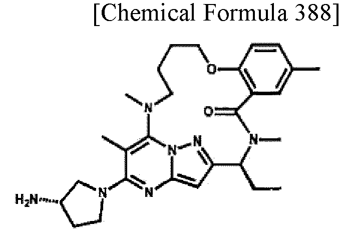

By using the same approaches as in Step 206-1 and Step 1-2 to Step 1-4, the title compound (6.6 mg, light yellow amorphous) was obtained from the compound (40 mg) obtained in Reference Example 1-16 and the compound (40 mg) obtained in Reference Example 2-27.
LCMS (ESI) m/z 506 [M+H]⁺, t_{R} = 0.562, 0.603 min, mode N.

### Example 221: Synthesis of (17S)-3-[(3S)-3-aminopyrrolidin-1-yl]-17-ethyl-4,13,16-trimethyl-6,7,8,9,16,17-hexahydro-5 H,15H-18,1-(metheno)pyrimido[6,1-g][1,6,8,9,12]benzoxatetraazacyclopentadecin-15-one

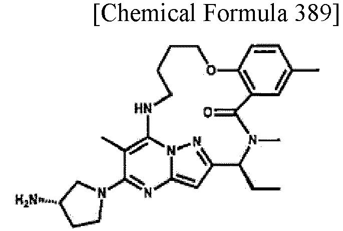

By using the same approaches as in Step 1-1 to Step 1-4, the title compound (21 mg, pink amorphous) was obtained from the compound (0.10 g) obtained in Reference Example 1-16 and the compound (92 mg) obtained in Reference Example 2-44.
LCMS (ESI) m/z 492 [M+H]⁺, t_{R} = 0.477, 0.504 min, mode N.

### Example 222: Synthesis of (17S)-17-ethyl-3-methoxy-4,5,13,16-tetramethyl-5,6,7,8,9,10,16,17-octahydro-15H-18,1-(me theno)pyrimido[6,1-g][1,6,8,9,12]benzopentaazacyclopentadecin-15-one (Example 222a) and (17S)-3-chloro-17-ethyl-4,5,13,16-tetramethyl-5,6,7,8,9,10,16,17-octahydro-15H-18,1-(meth eno)pyrimido[6,1-g][1,6,8,9,12]benzopentaazacyclopentadecin-15-one (Example 222b)

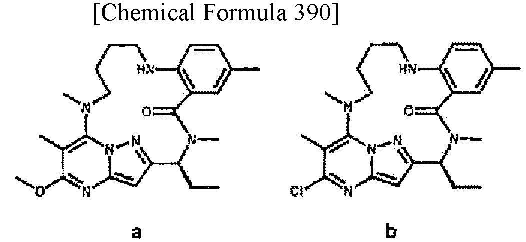

Step 222: By using the same approaches as in Step 1-1 to Step 1-3, title compound a (86 mg, light yellow amorphous) and title compound b (38 mg, colorless amorphous) were obtained from the compound (0.15 g) obtained in Reference Example 1-16 and the compound (0.22 g) obtained in Reference Example 2-60.
Title compound a: LCMS (ESI) m/z 451 [M+H]⁺, t_{R} = 1.396 min, mode N.
Title compound b: LCMS (ESI) m/z 455 [M+H]⁺, t_{R} = 1.354 min, mode N.

### Example 223: Synthesis of (17S)-3-[(3S)-3-aminopyrrolidin-1-yl]-17-ethyl-4,5,13,16-tetramethyl-5,6,7,8,9,10,16,17-oct ahydro-15H-18,1-(metheno)pyrimido[6,1-g][1,6,8,9,12]benzopentaazacyclopentadecin-15-on

e

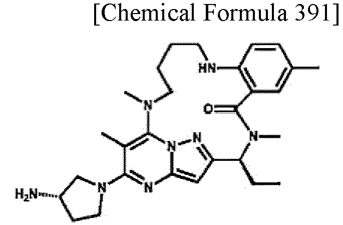

By using the same approach as in Step 1-4, the title compound (21 mg, colorless amorphous) was obtained from Compound B (20 mg) obtained in Step 222.
LCMS (ESI) m/z 505 [M+H]⁺, t_{R} = 0.636 min, mode N.

### Example 224: Synthesis of (17S)-3-[(3S)-3-aminopyrrolidin-1-yl]-17-ethyl-10-(methanesulfonyl)-4,5,13,16-tetramethyl-5,6,7,8,9,10,16,17-octahydro-15H-18,1-(metheno)pyrimido[6,1-g][1,6,8,9,12]benzopentaaza cyclopentadecin-15-one

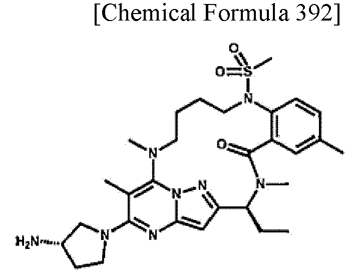

Step 224-1: By using the same approaches as in Step 206-1, Step 1-2, and Step 1-3, (17S)-3-chloro-17-ethyl-10-(methanesulfonyl)-4,5,13,16-tetramethyl-5,6,7,8,9,10,16,17-octa hydro-15H-18,1-(metheno)pyrimido[6,1-g][1,6,8,9,12]benzopentaazacyclopentadecin-15-one (88 mg, colorless amorphous) was obtained from the compound (0.20 g) obtained in Reference Example 1-16 and the compound (0.31 g) obtained in Reference Example 2-95.
LCMS (ESI) m/z 533 [M+H]⁺, t_{R} = 1.248 min, mode N.

Step 224-2: By using the same approach as in Step 1-4, the title compound (22 mg, colorless amorphous) was obtained from the compound (20 mg) obtained in Step 224-1. LCMS (ESI) m/z 583 [M+H]⁺, t_{R} = 0.599, 0.625 min, mode N.

### Example 225: Synthesis of (17S)-3-[(2-amino-2-methylpropyl)amino]-17-ethyl-10-(methanesulfonyl)-4,5,13,16-tetramet hyl-5,6,7,8,9,10,16,17-octahydro-15H-18,1-(metheno)pyrimido[6,1-g][1,6,8,9,12]benzopenta azacyclopentadecin-15-one

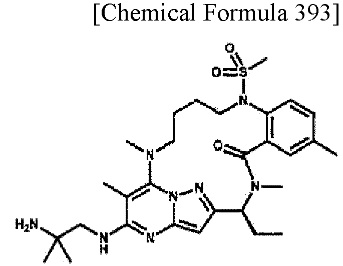

By using the same approach as in Step 1-4, the title compound (15 mg, colorless amorphous) was obtained from the compound (20 mg) obtained in Step 224-1 and 2-methylpropane-1,2-diamine (39 µL).
LCMS (ESI) m/z 585 [M+H]⁺, t_{R} = 0.676, 0.720 min, mode N.

### Example 226: Synthesis of (17S)-3-[(3S)-3-aminopyrrolidin-1-yl]-17-ethyl-4,5,13,16-tetramethyl-10-(trifluoromethanes ulfonyl)-5,6,7,8,9,10,16,17-octahydro-15H-18,1-(metheno)pyrimido[6,1-g][1,6,8,9,12]benzo pentaazacyclopentadecin-15-one

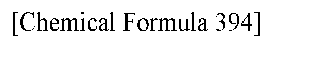

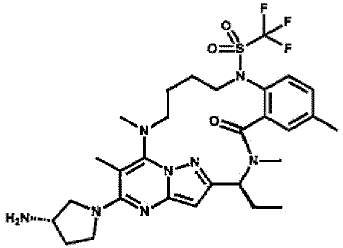

By using the same approaches as in Step 1-1 to Step 1-4, the title compound (41 mg, light yellow amorphous) was obtained from the compound (0.13 g) obtained in Reference Example 1-16 and the compound (0.13 g) obtained in Reference Example 2-96.
LCMS (ESI) m/z 637 [M+H]⁺, t_{R} = 0.733 min, mode N.

### Example 227: Synthesis of (17S)-3-[(3S)-3-aminopyrrolidin-1-yl]-17-ethyl-4-fluoro-5,13,16-trimethyl-6,7,8,9,16,17-hex ahydro-5H,15H-18,1-(metheno)pyrimido[6,1-g][1,6,8,9,12]benzoxatetraazacyclopentadecin-15-one hydrochloride

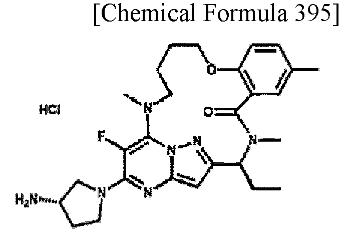

By using the same approaches as in Step 1-1, Step 1-4, Step 1-2, Step 1-3, and Step 13-2, the title compound (33 mg, colorless amorphous) was obtained from the compound (0.37 g) obtained in Reference Example 1-17, the compound (0.20 g) obtained in Reference Example 2-27, and tert-butyl N-[(3S)-pyrrolidin-3-yl]carbamate (91 mg).
LCMS (ESI) m/z 510 [M+H]⁺, t_{R} = 0.631, 0.674 min, mode N.

### Example 228: Synthesis of 3-[(3S)-3-aminopyrrolidin-1-yl]-5,13,16-trimethyl-6,7,8,9,16,17-hexahydro-5H,15H-18,1-(m etheno)pyrimido[6,1-g][1,6,8,9,12]benzoxatetraazacyclopentadecin-15-one

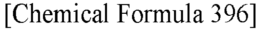

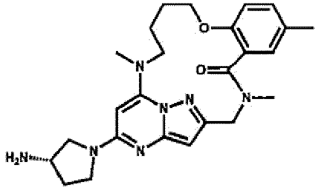

By using the same approaches as in Step 1-1 to Step 1-4, the title compound (5.2 mg, light yellow amorphous) was obtained from the compound (0.45 g) obtained in Reference Example 1-19 and the compound (0.64 g) obtained in Reference Example 2-27.
LCMS (ESI) m/z 464 [M+H]⁺, t_{R} = 0.783 min, mode H.

### Example 229: Synthesis of 3-[(3S)-3-aminopyrrolidin-1-yl]-5,13,16,17,17-pentamethyl-6,7,8,9,16,17-hexahydro-5H,15 H-18,1-(metheno)pyrimido[6,1-g][1,6,8,9,12]benzoxatetraazacyclopentadecin-15-one

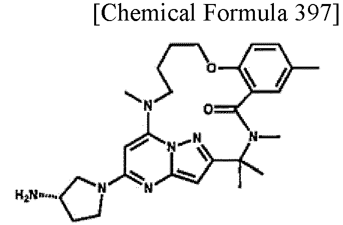

By using the same approaches as in Step 1-1 to Step 1-4, the title compound (20 mg, light brown amorphous) was obtained from the compound (92 mg) obtained in Reference Example 1-20 and the compound (0.11 g) obtained in Reference Example 2-27.
LCMS (ESI) m/z 492 [M+H]⁺, t_{R} = 0.501 min, mode N.

### Example 230: Synthesis of (18S)-3-[(3S)-3-aminopyrrolidin-1-yl]-18-ethyl-5,14,17-trimethyl-5,6,7,8,9,10,17,18-octahyd ro-16H-19,1-(metheno)pyrimido[6,1-h][1,7,9,10,13]benzoxatetraazacyclohexadecin-16-one

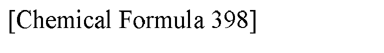

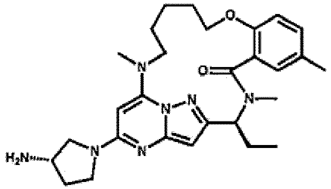

Step 230-1: By using the same approaches as in Step 1-1 to Step 1-3, (18S)-3-chloro-18-ethyl-5,14,17-trimethyl-5,6,7,8,9,10,17,18-octahydro-16H-19,1-(metheno) pyrimido[6,1-h][1,7,9,10,13]benzoxatetraazacyclohexadecin-16-one (0.20 g, colorless amorphous) was obtained from the compound (0.60 g) obtained in Reference Example 1-15 and the compound (0.22 g) obtained in Reference Example 2-46.
LCMS (ESI) m/z 456 [M+H]⁺, t_{R} = 1.249 min, mode N.

Step 230-2: By using the same approach as in Step 1-4, the title compound (45 mg, colorless powder) was obtained from the compound (60 mg) obtained in Step 230-1.
LCMS (ESI) m/z 506 [M+H]⁺, t_{R} = 0.517 min, mode N.

### Example 231: Synthesis of (18S)-3-[(2-aminoethyl)amino]-18-ethyl-5,14,17-trimethyl-5,6,7,8,9,10,17,18-octahydro-16H -19,1-(metheno)pyrimido[6,1-h][1,7,9,10,13]benzoxatetraazacyclohexadecin-16-one hydrochloride

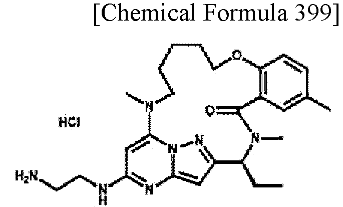

By using the same approaches as in Step 1-4 and Step 13-2, the title compound (30 mg, yellow powder) was obtained from the compound (60 mg) obtained in Step 230-1 and tert-butyl N-(2-aminoethyl)carbamate (0.21 g).
LCMS (ESI) m/z 480 [M+H]⁺, t_{R} = 0.546 min, mode N.

### Example 232: Synthesis of (18S)-3-[(3S)-3-aminopyrrolidin-1-yl]-18-ethyl-4,5,14,17-tetramethyl-5,6,7,8,9,10,17,18-oct ahydro-16H-19,1-(metheno)pyrimido[6,1-h][1,7,9,10,13]benzoxatetraazacyclohexadecin-16-one

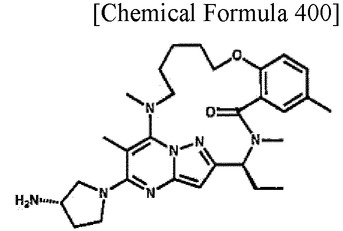

By using the same approaches as in Step 206-1 and Step 1-2 to Step 1-4, the title compound (35 mg, colorless powder) was obtained from the compound (0.15 g) obtained in Reference Example 1-16 and the compound (0.22 g) obtained in Reference Example 2-46. LCMS (ESI) m/z 520 [M+H]⁺, t_{R} = 0.679 min, mode H.

### Example 233: Synthesis of (18S)-3-[(3S)-3-aminopyrrolidin-1-yl]-18-ethyl-4,5,17-trimethyl-5,6,7,8,9,10,17,18-octahydr o-16H-19,1-(metheno)pyrimido[6,1-h][1,7,9,10,13]benzoxatetraazacyclohexadecin-16-one

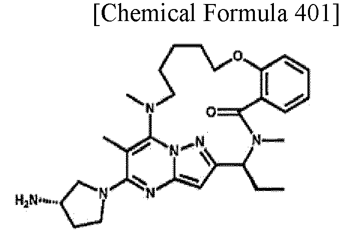

Step 233-1: By using the same approaches as in Step 206-1, Step 202-2, and Step 1-3, (18S)-3-chloro-18-ethyl-4,5,17-trimethyl-5,6,7,8,9,10,17,18-octahydro-16H-19,1-(metheno)p yrimido[6,1-h][1,7,9,10,13]benzoxatetraazacyclohexadecin-16-one (0.11 g, colorless amorphous) was obtained from the compound (0.15 g) obtained in Reference Example 1-16 and the compound (0.24 g) obtained in Reference Example 2-47.
LCMS (ESI) m/z 456 [M+H]⁺, t_{R} = 1.297 min, mode N.

Step 233-2: By using the same approach as in Step 1-4, the title compound (23 mg, yellow oily matter) was obtained from the compound (30 mg) obtained in Step 233-1. LCMS (ESI) m/z 506 [M+H]⁺, t_{R} = 0.617 min, mode N.

### Example 234: Synthesis of (18S)-3-[(2-amino-2-methylpropyl)amino]-18-ethyl-4,5,17-trimethyl-5,6,7,8,9,10,17,18-octa hydro-16H-19, 1-(metheno)pyrimido[6,1-h][1,7,9,10,13]benzoxatetraazacyclohexadecin-16-o ne

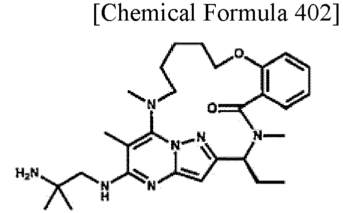

By using the same approach as in Step 1-4, the title compound (24 mg, colorless powder) was obtained from the compound (41 mg) obtained in Step 233-1 and 1,2-diamino-2-methylpropane (47 µL).
LCMS (ESI) m/z 508 [M+H]⁺, t_{R} = 0.703 min, mode N.

### Example 235: Synthesis of (18S)-3-[(3S)-3-aminopyrrolidin-1-yl]-18-ethyl-11-(methanesulfonyl)-4,5,14,17-tetramethyl-6,7,8,9,10,11,17,18-octahydro-19,1-(metheno)pyrimido[6,1-h][1,7,9,10,13]benzopentaazacyc lohexadecin-16(5H)-one

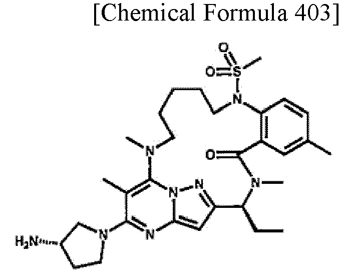

Step 235-1: By using the same approaches as in Step 206-1, Step 1-2, and Step 1-3, (18S)-3-chloro-18-ethyl-11-(methanesulfonyl)-4,5,14,17-tetramethyl-6,7,8,9,10,11,17,18-oct ahydro-19,1-(metheno)pyrimido[6,1-h][1,7,9,10,13]benzopentaazacyclohexadecin-16(5H)-on e (0.24 g, colorless powder) was obtained from the compound (0.15 g) obtained in Reference Example 1-16 and the compound (0.28 g) obtained in Reference Example 2-97.
LCMS (ESI/APCI dual) m/z 547 [M+H]⁺, t_{R} = 1.213 min, mode N.

Step 235-2: By using the same approach as in Step 1-4, the title compound (35 mg, colorless powder) was obtained from Step 235-1 (50 mg).
LCMS (ESI) m/z 597 [M+H]⁺, t_{R} = 0.658 min, mode H.

### Example 236: Synthesis of (18S)-3-[(2-aminoethyl)amino]-18-ethyl-11-(methanesulfonyl)-4,5,14,17-tetramethyl-6,7,8,9, 10,11,17,18-octahydro-19,1-(metheno)pyrimido[6,1-h][1,7,9,10,13]benzopentaazacyclohexa decin-16(5H)-one

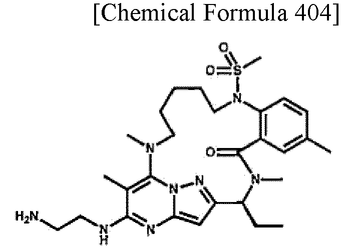

By using the same approaches as in Step 1-4 and Step 5-2, the title compound (43 mg, colorless powder) was obtained from the compound (50 mg) obtained in Step 235-1 and tert-butyl N-(2-aminoethyl)carbamate (0.12 mL).
LCMS (ESI) m/z 571 [M+H]⁺, t_{R} = 0.699 min, mode H.

### Example 237: Synthesis of (18S)-18-ethyl-11-(methanesulfonyl)-4,5,14,17-tetramethyl-6,7,8,9,10,11,17,18-octahydro-1 9,1-(metheno)pyrimido[6,1-h][1,7,9,10,13]benzopentaazacyclohexadecin-16(5H)-one

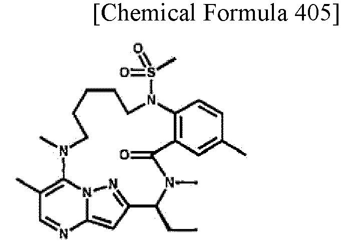

By using the same approach as in Step 4-2, the title compound (28 mg, light yellow powder) was obtained from the compound (29 mg) obtained in Step 235-1.
¹H NMR (400 MHz, DMSO-d₆) δ ppm 0.97 - 1.61 (m, 9H), 1.99 - 2.17 (m, 2H), 2.21 - 2.28 (m, 3H), 2.35 (s, 2.4H), 2.40 (s, 0.6H), 2.60 (s, 2.4H), 2.89 (s, 0.6H), 3.00 (s, 3H), 3.03 - 3.09 (m, 3H), 3.18 - 3.63 (m, 4H), 4.75 - 4.82 (m, 0.2H), 5.91 - 5.99 (m, 0.8H), 6.63 (s, 0.8H), 6.81 (s, 0.2H), 7.18 (s, 0.8H), 7.23 (s, 1.6H), 7.29 (s, 0.2H), 7.32 (s, 0.4H), 8.21 - 8.26 (m, 1H).

### Example 238: Synthesis of (16S)-3-[(3S)-3-aminopyrrolidin-1-yl]-16-ethyl-5,12,15-trimethyl-5,6,7,8,15,16-hexahydro-1 4H-17,1-(metheno)pyrimido[6,1-f][1,5,7,8,11]benzoxatetraazacyclotetradecin-14-one

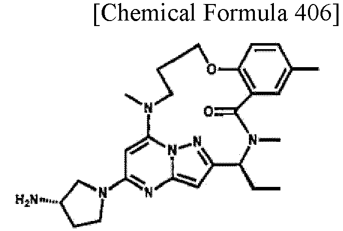

By using the same approaches as in Step 1-1 to Step 1-4, the title compound (12 mg, colorless amorphous) was obtained from the compound (0.41 g) obtained in Reference Example 1-15 and the compound (0.23 g) obtained in Reference Example 2-45.
LCMS (ESI) m/z 478 [M+H]⁺, t_{R} = 0.841 min, mode H.

### Example 239: Synthesis of (16S)-3-[(3S)-3-aminopyrrolidin-1-yl]-16-ethyl-4,5,12,15-tetramethyl-5,6,7,8,15,16-hexahyd ro-14H-17,1-(metheno)pyrimido[6,1-f][1,5,7,8,11]benzoxatetraazacyclotetradecin-14-one

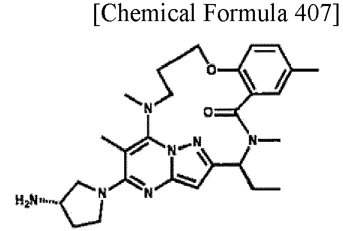

By using the same approaches as in Step 206-1, Step 202-2, Step 1-3, and Step 1-4, the title compound (16 mg, colorless powder) was obtained from the compound (0.10 g) obtained in Reference Example 1-16 and the compound (0.13 g) obtained in Reference Example 2-45.
LCMS (ESI) m/z 492 [M+H]⁺, t_{R} = 0.585 min, mode N.

### Example 240: Synthesis of (19S)-3-[(3S)-3-aminopyrrolidin-1-yl]-19-ethyl-5,15,18-trimethyl-6,7,8,9,10,11,18,19-octahy dro-5H,17H-20,1-(metheno)pyrimido[6,1-i][1,8,10,11,14]benzoxatetraazacycloheptadecin-17 -one

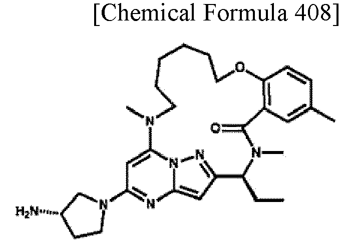

Step 240-1: By using the same approaches as in Step 1-1, Step 202-2, and Step 1-3, (19S)-3-chloro-19-ethyl-5,15,18-trimethyl-6,7,8,9,10,11,18,19-octahydro-5H,17H-20,1-(met heno)pyrimido[6,1-i][1,8,10,11,14]benzoxatetraazacycloheptadecin-17-one (96 mg, yellow oily matter) was obtained from the compound (0.33 g) obtained in Reference Example 1-15 and the compound (0.21 g) obtained in Reference Example 2-48.
LCMS (ESI) m/z 470 [M+H]⁺, t_{R} = 1.423 min, mode N.

Step 240-2: By using the same approach as in Step 1-4, the title compound (16 mg, yellow powder) was obtained from the compound (32 mg) obtained in Step 240-1.
LCMS (ESI) m/z 520 [M+H]⁺, t_{R} = 0.573 min, mode N.

### Example 241: Synthesis of (19S)-3-[(2-aminoethyl)amino]-19-ethyl-5,15,18-trimethyl-6,7,8,9,10,11,18,19-octahydro-5H ,17H-20,1-(metheno)pyrimido[6,1-i][1,8,10,11,14]benzoxatetraazacycloheptadecin-17-one hydrochloride

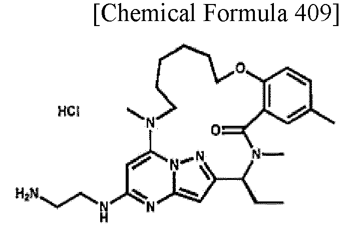

By using the same approaches as in Step 1-4 and Step 13-2, the title compound (9.6 mg, colorless powder) was obtained from the compound (32 mg) obtained in Step 240-1 and tert-butyl N-(2-aminoethyl)carbamate (55 mg).
LCMS (ESI) m/z 494 [M+H]⁺, t_{R} = 0.614 min, mode N.

### Example 242: Synthesis of (3S)-18-[(3S)-3-aminopyrrolidin-1-yl]-3-ethyl-4,16-dimethyl-3,4,13,14,15,16-hexahydro-5H, 12H-2,20:6,10-di(metheno)pyrimido[6,1-g][1,6,8,9,12]oxatetraazacyclooctadecin-5-one

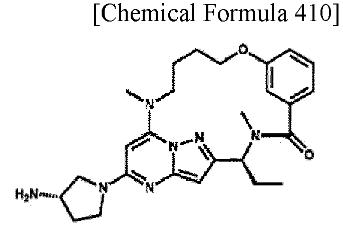

By using the same approaches as in Step 1-1 to Step 1-4, the title compound (30 mg, light pink amorphous) was obtained from the compound (0.30 g) obtained in Reference Example 1-15 and the compound (0.18 g) obtained in Reference Example 2-49.
LCMS (ESI) m/z 478 [M+H]⁺, t_{R} = 0.815 min, mode H.

### Example 243: Synthesis of (3S)-17-[(3S)-3-aminopyrrolidin-1-yl]-3-ethyl-4,15-dimethyl-3,4,12,13,14,15-hexahydro-5H-2,19:6,10-di(metheno)pyrimido[6,1-f][1,5,7,8,11]oxatetraazacycloheptadecin-5-one

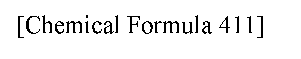

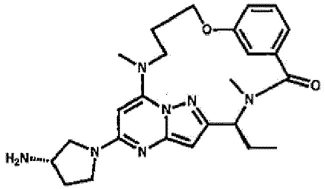

By using the same approaches as in Step 1-1 to Step 1-4, the title compound (42 mg, light pink amorphous) was obtained from the compound (0.30 g) obtained in Reference Example 1-15 and the compound (0.17 g) obtained in Reference Example 2-114.
LCMS (ESI) m/z 464 [M+H]⁺, t_{R} = 0.793 min, mode H.

### Example 244: Synthesis of (23aS)-18-[(3S)-3-aminopyrrolidin-1-yl]-8,16-dimethyl-1,3,4,13,14,15,16,23a-octahydro-2H, 6H,12H-23,20-(metheno)pyrido[2,1-k]pyrimido[6,1-g][1,6,8,9,12]benzoxatetraazacyclopenta decin-6-one hydrochloride

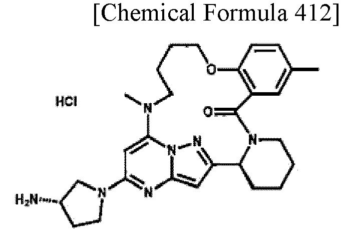

Step 244-1: By using the same approaches as in Step 1-1, Step 202-2, and Step 1-3, (23aS)-18-chloro-8,16-dimethyl-1,3,4,13,14,15,16,23a-octahydro-2H,6H,12H-23,20-(methen o)pyrido[2,l-k]pyrimido[6,1-g][1,6,8,9,12]benzoxatetraazacyclopentadecin-6-one (0.14 g, colorless amorphous) was obtained from the compound (0.19 g) obtained in Reference Example 1-1 (3) and the compound (0.22 g) obtained in Reference Example 2-27.
LCMS (ESI) m/z 454 [M+H]⁺, t_{R} = 0.910, 0.956 min, mode L.

Step 244-2: By using the same approaches as in Step 1-4 and Step 13-2, the title compound (43 mg, colorless amorphous) was obtained from the compound (50 mg) obtained in Step 244-1 and tert-butyl (3S)-pyrrolidin-3-ylcarbamate (0.10 g).
¹H NMR (400 MHz, DMSO-d₆) δ ppm 1.15 - 2.42 (m, 15H), 3.14 (s, 3H), 3.23 - 4.09 (m, 11H), 5.25 (s, 1H), 6.03 - 6.16 (m, 1H), 6.22 - 6.38 (m, 1H), 6.79 - 7.25 (m, 3H), 8.42 - 8.72 (m, 3H).
LCMS (ESI) m/z 504 [M+H]⁺, t_{R} = 0.506 min, mode N.

### Example 245: Synthesis of (23aS)-18-[(2-aminoethyl)amino]-8,16-dimethyl-1,3,4,13,14,15,16,23a-octahydro-2H,6H,12 H-23,20-(metheno)pyrido[2,1-k]pyrimido[6,1-g][1,6,8,9,12]benzoxatetraazacyclopentadecin-6-one hydrochloride

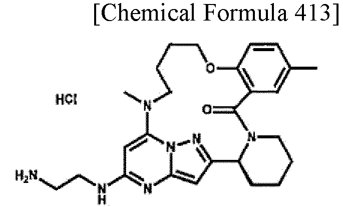

By using the same approaches as in Step 1-4 and Step 13-2, the title compound (23 mg, colorless amorphous) was obtained from the compound (50 mg) obtained in Step 244-1 and tert-butyl N-(2-aminoethyl)carbamate (0.14 g).
LCMS (ESI) m/z 478 [M+H]⁺, t_{R} = 0.538 min, mode N.

### Example 246: Synthesis of (23aS)-18-(3-aminoazetidin-1-yl)-8,16-dimethyl-1,3,4,13,14,15,16,23a-octahydro-2H,6H,12 H-23,20-(metheno)pyrido[2,1-k]pyrimido[6,1-g][1,6,8,9,12]benzoxatetraazacyclopentadecin-6-one

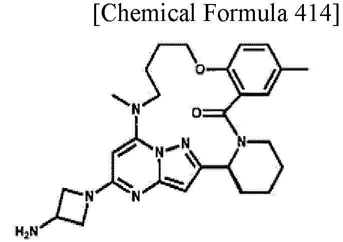

By using the same approaches as in Step 1-4 and Step 4-2, the title compound (41 mg, colorless amorphous) was obtained from the compound (60 mg) obtained in Step 244-1 and 3-N-BOC-amino-azetidine (0.23 g).
LCMS (ESI) m/z 490 [M+H]⁺, t_{R} = 0.937 min, mode H.

### Example 247: Synthesis of (23aS)-18-(3-hydroxyazetidin-1-yl)-8,16-dimethyl-1,3,4,13,14,15,16,23a-octahydro-2H,6H,1 2H-23,20-(metheno)pyrido[2,1-k]pyrimido[6,1-g][1,6,8,9,12]benzoxatetraazacyclopentadeci n-6-one

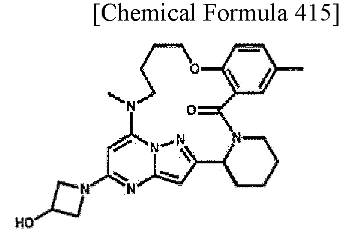

By using the same approach as in Step 3-1, the title compound (65 mg, colorless amorphous) was obtained from the compound (70 mg) obtained in Step 244-1 and 3-hydroxyazetidine hydrochloride (0.17 g).
LCMS (ESI) m/z 491 [M+H]⁺, t_{R} = 0.570, 0.675 min, mode N.

### Example 248: Synthesis of (23aS)-18-[(3S)-3-aminopyrrolidin-1-yl]-7-chloro-16-methyl-1,3,4,13,14,15,16,23a-octahydr o-2H,6H,12H-23,20-(metheno)pyrido[2,1-k]pyrimido[6,1-g][1,6,8,9,12]benzoxatetraazacycl opentadecin-6-one

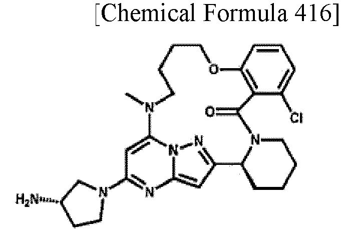

By using the same approaches as in Step 1-1 to Step 1-4, the title compound (9.4 mg, colorless amorphous) was obtained from the compound (90 mg) obtained in Reference Example 1-1 (3) and the compound (90 mg) obtained in Reference Example 2-31. LCMS (ESI) m/z 524 [M+H]⁺, t_{R} = 0.494 min, mode H.

### Example 249: Synthesis of (23aS)-18-[(3S)-3-aminopyrrolidin-1-yl]-7-fluoro-16-methyl-1,3,4,13,14,15,16,23a-octahydr o-2H,6H,12H-23,20-(metheno)pyrido[2,1-k]pyrimido[6,1-g][1,6,8,9,12]benzoxatetraazacycl opentadecin-6-one

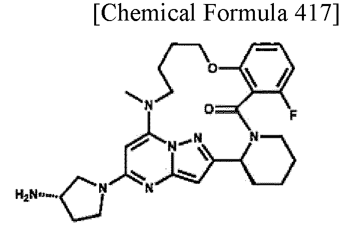

By using the same approaches as in Step 1-1 to Step 1-4, the title compound (62 mg, colorless powder) was obtained from the compound (0.20 g) obtained in Reference Example 1-1 (3) and the compound (0.20 g) obtained in Reference Example 2-32.
LCMS (ESI) m/z 508 [M+H]⁺, t_{R} = 0.732, 0.844 min, mode H.

### Example 250: Synthesis of (23aS)-18-[(3S)-3-aminopyrrolidin-1-yl]-8,10-dichloro-16-methyl-1,3,4,13,14,15,16,23a-octa hydro-2H,6H,12H-23,20-(metheno)pyrido[2,1-k]pyrimido[6,1-g][1,6,8,9,12]benzoxatetraaza cyclopentadecin-6-one

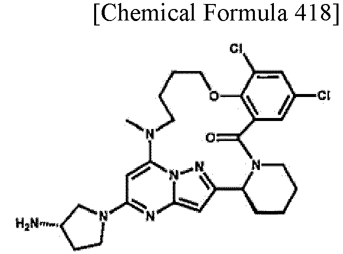

By using the same approaches as in Step 1-1 to Step 1-4, the title compound (95 mg, colorless amorphous) was obtained from the compound (0.20 g) obtained in Reference Example 1-1 (3) and the compound (0.23 g) obtained in Reference Example 2-33. LCMS (ESI) m/z 558 [M+H]⁺, t_{R} = 1.035 min, mode H.

### Example 251: Synthesis of (23aS)-18-[(3S)-3-aminopyrrolidin-1-yl]-8,10-difluoro-16-methyl-1,3,4,13,14,15,16,23a-octa hydro-2H,6H,12H-23,20-(metheno)pyrido[2,1-k]pyrimido[6,1-g][1,6,8,9,12]benzoxatetraaza cyclopentadecin-6-one

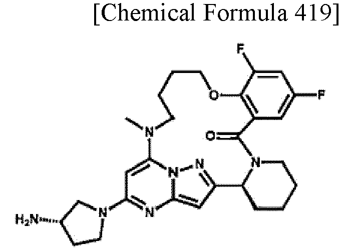

By using the same approaches as in Step 1-1 to Step 1-4, the title compound (57 mg, colorless powder) was obtained from the compound (0.20 g) obtained in Reference Example 1-1 (3) and the compound (0.21 g) obtained in Reference Example 2-34.
LCMS (ESI) m/z 526 [M+H]⁺, t_{R} = 0.485 min, mode N.

### Example 252: Synthesis of (23aS)-18-[(3S)-3-aminopyrrolidin-1-yl]-16-methyl-8-(trifluoromethyl)-1,3,4,13,14,15,16,23 a-octahydro-2H,6H,12H-23,20-(metheno)pyrido[2,1-k]pyrimido[6,1-g][1,6,8,9,12]benzoxate traazacyclopentadecin-6-one

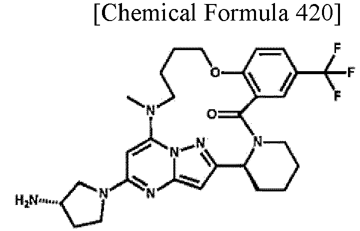

By using the same approaches as in Step 1-1 to Step 1-4, the title compound (56 mg, pink amorphous) was obtained from the compound (0.22 g) obtained in Reference Example 1-1 (3) and the compound (0.19 g) obtained in Reference Example 2-30.
LCMS (ESI) m/z 558 [M+H]⁺, t_{R} = 0.558 min, mode N.

### Example 253: Synthesis of (23aS)-18-[(3S)-3-aminopyrrolidin-1-yl]-8-(hydroxymethyl)-16-methyl-1,3,4,13,14,15,16,23 a-octahydro-2H,6H,12H-23,20-(metheno)pyrido[2,1-k]pyrimido[6,1-g][1,6,8,9,12]benzoxate traazacyclopentadecin-6-one

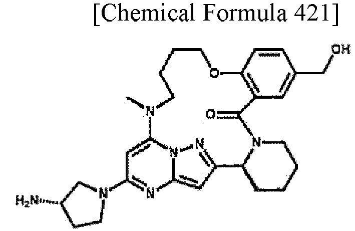

By using the same approaches as in Step 1-1 to Step 1-4, the title compound (14 mg, colorless amorphous) was obtained from the compound (0.20 g) obtained in Reference Example 1-1 (3) and the compound (0.31 g) obtained in Reference Example 2-35.
LCMS (ESI) m/z 520 [M+H]⁺, t_{R} = 0.706 min, mode H.

### Example 254: Synthesis of (23aS)-18-[(3S)-3-aminopyrrolidin-1-yl]-8-chloro-16-methyl-1,3,4,13,14,15,16,23a-octahydr o-2H,6H,12H-23,20-(metheno)pyrido[2,1-k]pyrimido[6,1-g][1,6,8,9,12]benzoxatetraazacycl opentadecin-6-one

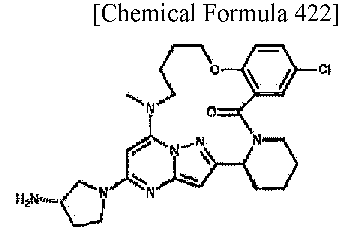

Step 254-1: By using the same approaches as in Step 1-1 to Step 1-3, (23aS)-8,18-dichloro-16-methyl-1,3,4,13,14,15,16,23a-octahydro-2H,6H,12H-23,20-(methen o)pyrido[2,1-k]pyrimido[6,1-g][1,6,8,9,12]benzoxatetraazacyclopentadecin-6-one (39 mg, colorless powder) was obtained from the compound (0.15 g) obtained in Reference Example 1-1 (3) and the compound (0.18 g) obtained in Reference Example 2-26.
LCMS (ESI) m/z 474 [M+H]⁺, t_{R} = 1.265 min, mode N.

Step 254-2: By using the same approach as in Step 1-4, the title compound (5.5 mg, colorless powder) was obtained from the compound (37 mg) obtained in Step 254-1. LCMS (ESI) m/z 524 [M+H]⁺, t_{R} = 0.926 min, mode H.

### Example 255: Synthesis of (23aS)-18-[(2-amino-2-methylpropyl)amino]-8-chloro-16-methyl-1,3,4,13,14,15,16,23a-octa hydro-2H,6H,12H-23,20-(metheno)pyrido[2,1-k]pyrimido[6,1-g][1,6,8,9,12]benzoxatetraaza cyclopentadecin-6-one

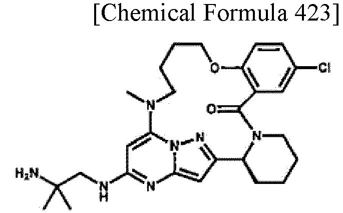

By using the same approach as in Step 1-4, the title compound (74 mg, colorless amorphous) was obtained from the compound (0.15 g) obtained in Step 254-1 and 1,2-diamino-2-methylpropane (0.16 mL).
LCMS (ESI) m/z 526 [M+H]⁺, t_{R} = 0.626 min, mode N.

### Example 256: Synthesis of (23aS)-18-[(2-aminoethyl)amino]-8-chloro-16-methyl-1,3,4,13,14,15,16,23a-octahydro-2H,6 H,12H-23,20-(metheno)pyrido[2,1-k]pyrimido[6,1-g][1,6,8,9,12]benzoxatetraazacyclopentad ecin-6-one hydrochloride

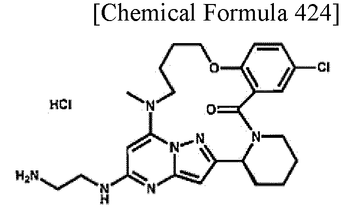

By using the same approaches as in Step 1-4 and Step 13-2, the title compound (7.0 mg, colorless powder) was obtained from the compound (0.15 g) obtained in Step 254-1 and tert-butyl N-(2-aminoethyl)carbamate (0.25 g).
LCMS (ESI) m/z 498 [M+H]⁺, t_{R} = 0.564 min, mode N.

### Example 257: Synthesis of (23aS)-18-(3-aminopropyl)-8-chloro-16-methyl-1,3,4,13,14,15,16,23a-octahydro-2H,6H,12H -23,20-(metheno)pyrido[2,1-k]pyrimido[6,1-g][1,6,8,9,12]benzoxatetraazacyclopentadecin-6 -one hydrochloride

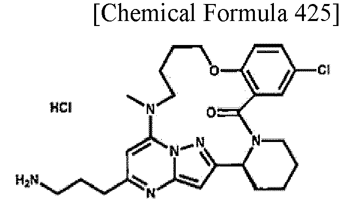

By using the same approaches as in Step 28-1, Step 4-2, and Step 13-2, the title compound (76 mg, colorless powder) was obtained from the compound (0.15 g) obtained in Step 254-1, using tert-butyl N-prop-2-ynylcarbamate instead of propargyl alcohol.
LCMS (ESI) m/z 497 [M+H]⁺, t_{R} = 0.500 min, mode N.

### Example 258: Synthesis of (23aS)-18-[2-(1-aminocyclopropyl)ethyl]-8-chloro-16-methyl-1,3,4,13,14,15, 16,23a-octahyd ro-2H,6H,12H-23,20-(metheno)pyrido[2,1-k]pyrimido[6,1-g][1,6,8,9,12]benzoxatetraazacycl opentadecin-6-one hydrochloride

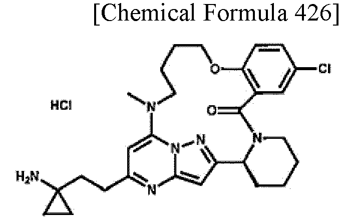

By using the same approaches as in Step 28-1, Step 4-2, and Step 13-2, the title compound (0.11 g, colorless powder) was obtained from the compound (0.15 g) obtained in Step 254-1, using tert-butyl N-(1-ethynylcyclopropyl)carbamate instead of propargyl alcohol. LCMS (ESI) m/z 523 [M+H]⁺, t_{R} = 0.538 min, mode N.

### Example 259: Synthesis of (23aS)-18-[(3S)-3-aminopyrrolidin-1-yl]-8,16-dimethyl-1,3,4,13,14,15,16,23a-octahydro-2H, 6H,12H-23,20-(metheno)dipyrido[3,2-b:1',2'-e]pyrimido[1,6-i][1,5,8,9,11]oxatetraazacyclope ntadecin-6-one

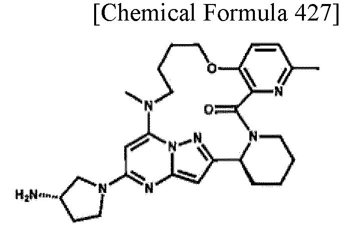

By using the same approaches as in Step 1-1 to Step 1-4, the title compound (28 mg, pink powder) was obtained from the compound (0.20 g) obtained in Reference Example 1-1 (3) and the compound (0.14 g) obtained in Reference Example 2-36.
LCMS (ESI) m/z 505 [M+H]⁺, t_{R} = 0.270, 0.360 min, mode N.

### Example 260: Synthesis of (23aS)-18-[(3S)-3-aminopyrrolidin-1-yl]-8-fluoro-16-methyl-1,3,4,13,14,15,16,23a-octahydr o-2H,6H,12H-23,20-(metheno)pyrido[2,1-k]pyrimido[6,1-g][1,6,8,9,12]benzoxatetraazacycl opentadecin-6-one

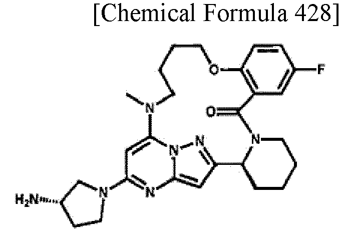

Step 260-1: By using the same approaches as in Step 1-1, Step 202-2, and Step 1-3, (23aS)-18-chloro-8-fluoro-16-methyl-1,3,4,13,14,15,16,23a-octahydro-2H,6H,12H-23,20-(m etheno)pyrido[2,1-k]pyrimido[6,1-g][1,6,8,9,12]benzoxatetraazacyclopentadecin-6-one (78 mg, colorless powder) was obtained from the compound (0.20 g) obtained in Reference Example 1-1 (3) and the compound (0.16 g) obtained in Reference Example 2-25.
LCMS (ESI) m/z 458 [M+H]⁺, t_{R} = 1.187 min, mode N.

Step 260-2: By using the same approach as in Step 1-4, the title compound (6.8 mg, colorless powder) was obtained from the compound (6.5 mg) obtained in Step 260-1. LCMS (ESI) m/z 508 [M+H]⁺, t_{R} = 0.850 min, mode H.

### Example 261: Synthesis of (23aS)-18-(azetidin-1-yl)-8-fluoro-16-methyl-1,3,4,13,14,15,16,23a-octahydro-2H,6H,12H-2 3,20-(metheno)pyrido[2,1-k]pyrimido[6,1-g][1,6,8,9,12]benzoxatetraazacyclopentadecin-6-o ne

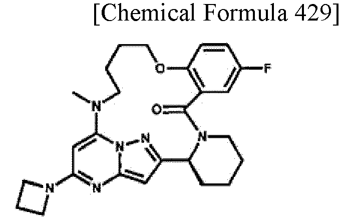

By using the same approach as in Step 1-4, the title compound (51 mg, colorless powder) was obtained from the compound (85 mg) obtained in Step 260-1 and azetidine (63 µL).
LCMS (ESI) m/z 479 [M+H]⁺, t_{R} = 0.689 min, mode N.

### Example 262: Synthesis of (23aS)-18-(3-aminoazetidin-1-yl)-8-fluoro-16-methyl-1,3,4,13,14,15,16,23a-octahydro-2H,6 H,12H-23,20-(metheno)pyrido[2,1-k]pyrimido[6,1-g][1,6,8,9,12]benzoxatetraazacyclopentad ecin-6-one

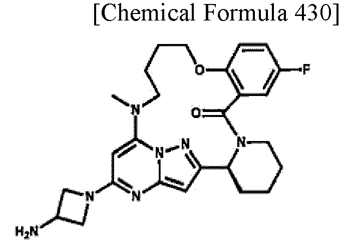

By using the same approaches as in Step 1-4 and Step 4-2, the title compound (70 mg, colorless amorphous) was obtained from the compound (60 mg) obtained in Step 260-1 and 3-N-BOC-amino-azetidine (0.23 g).
LCMS (ESI) m/z 494 [M+H]⁺, t_{R} = 0.877 min, mode H.

### Example 263: Synthesis of (23aS)-8-fluoro-18-(3-hydroxyazetidin-1-yl)-16-methyl-1,3,4,13,14,15,16,23a-octahydro-2H, 6H,12H-23,20-(metheno)pyrido[2,1-k]pyrimido[6,1-g][1,6,8,9,12]benzoxatetraazacyclopenta decin-6-one

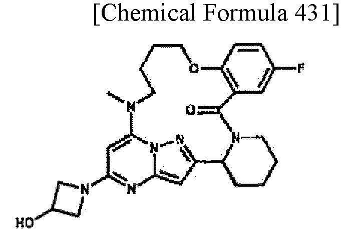

By using the same approach as in Step 3-1, the title compound (65 mg, colorless amorphous) was obtained from the compound (80 mg) obtained in Step 260-1 and 3-hydroxyazetidine hydrochloride (0.19 g).
LCMS (ESI) m/z 495 [M+H]⁺, t_{R} = 0.540, 0.638 min, mode N.

### Example 264: Synthesis of (23aS)-18-[(3S)-3-aminopyrrolidin-1-yl]-9-fluoro-8,16-dimethyl-1,3,4,13,14,15,16,23a-octah ydro-2H,6H,12H-23,20-(metheno)pyrido[2,1-k]pyrimido[6,1-g][1,6,8,9,12]benzoxatetraazac yclopentadecin-6-one

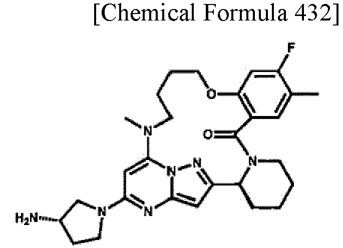

By using the same approaches as in Step 1-1 to Step 1-4, the title compound (53 mg, colorless powder) was obtained from the compound (0.20 g) obtained in Reference Example 1-1 (3) and the compound (0.21 g) obtained in Reference Example 2-37.
LCMS (ESI) m/z 522 [M+H]⁺, t_{R} = 0.527 min, mode N.

### Example 265: Synthesis of (23aS)-18-[(3S)-3-aminopyrrolidin-1-yl]-8-methoxy-16-methyl-1,3,4,13,14,15,16,23a-octahy dro-2H,6H,12H-23,20-(metheno)pyrido[2,1-k]pyrimido[6,1-g][1,6,8,9,12]benzoxatetraazacy clopentadecin-6-one

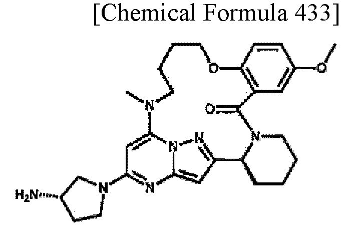

By using the same approaches as in Step 1-1 to Step 1-4, the title compound (67 mg, pink amorphous) was obtained from the compound (0.21 g) obtained in Reference Example 1-1 (3) and the compound (0.16 g) obtained in Reference Example 2-29.
LCMS (ESI) m/z 520 [M+H]⁺, t_{R} = 0.483 min, mode N.

### Example 266: Synthesis of N"-[(23aS)-18-[(3S)-3-aminopyrrolidin-1-yl]-16-methyl-6-oxo-1,3,4,13,14,15,16,23a-octahy dro-2H,6H,12H-23,20-(metheno)pyrido[2,1-k]pyrimido[6,1-g][1,6,8,9,12]benzoxatetraazacy clopentadecin-8-yl]-N,N,N',N'-tetramethylguanidine

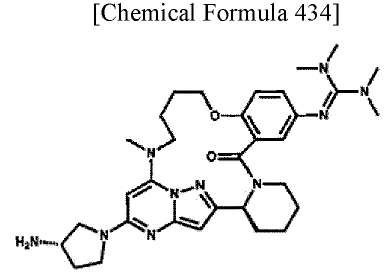

By using the same approaches as in Step 1-1 to Step 1-4, the title compound (12 mg, pink amorphous) was obtained from the compound (0.18 g) obtained in Reference Example 1-1 (3) and the compound (0.17 g) obtained in Reference Example 2-40.
LCMS (ESI) m/z 603 [M+H]⁺, t_{R} = 0.620 min, mode N.

### Example 267: Synthesis of (23aS)-18-[(3S)-3-aminopyrrolidin-1-yl]-9-chloro-16-methyl-1,3,4,13,14,15,16,23a-octahydr o-2H,6H,12H-23,20-(metheno)pyrido[2,1-k]pyrimido[6,1-g][1,6,8,9,12]benzoxatetraazacycl opentadecin-6-one

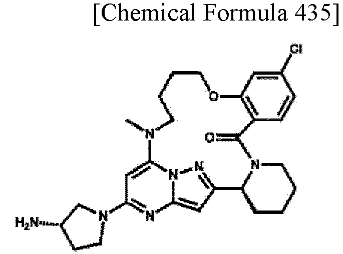

By using the same approaches as in Step 1-1 to Step 1-4, the title compound (12 mg, pink amorphous) was obtained from the compound (0.15 g) obtained in Reference Example 1-1 (3) and the compound (0.15 g) obtained in Reference Example 2-38.
LCMS (ESI) m/z 524 [M+H]⁺, t_{R} = 0.528 min, mode N.

### Example 268: Synthesis of (23aS)-18-[(3S)-3-aminopyrrolidin-1-yl]-9-fluoro-16-methyl-1,3,4,13,14,15,16,23a-octahydr o-2H,6H,12H-23,20-(metheno)pyrido[2,1-k]pyrimido[6,1-g][1,6,8,9,12]benzoxatetraazacycl opentadecin-6-one

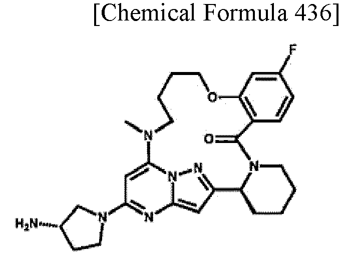

By using the same approaches as in Step 1-1 to Step 1-4, the title compound (98 mg, pink amorphous) was obtained from the compound (0.18 g) obtained in Reference Example 1-1 (3) and the compound (0.13 g) obtained in Reference Example 2-39.
LCMS (ESI) m/z 508 [M+H]⁺, t_{R} = 0.458 min, mode N.

### Example 269: Synthesis of (4aS)-10-[(3S)-3-aminopyrrolidin-1-yl]-12-methyl-2,3,4,4a,13,14,15,16-octahydro-1H,12H,2 4H-5,8-(metheno)naphtho[2,3-b]pyrido[1,2-e]pyrimido[1,6-i][1,5,8,9,11]oxatetraazacyclopen tadecin-24-one

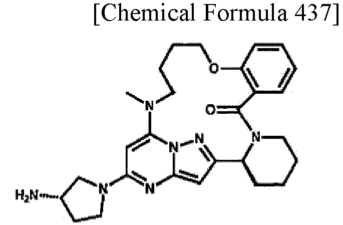

By using the same approaches as in Step 1-1 to Step 1-4, the title compound (50 mg, pink amorphous) was obtained from the compound (0.21 g) obtained in Reference Example 1-1 (3) and the compound (0.17 g) obtained in Reference Example 2-41.
LCMS (ESI) m/z 540 [M+H]⁺, t_{R} = 0.675 min, mode N.

### Example 270: Synthesis of (23aS)-18-[(3S)-3-aminopyrrolidin-1-yl]-16-methyl-1,3,4,13,14,15,16,23a-octahydro-2H,6H, 12H-23,20-(metheno)pyrido[2,1-k]pyrimido[6,1-g][1,6,8,9,12]benzoxatetraazacyclopentadec in-6-one

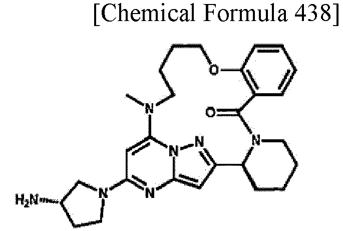

To a solution of the compound (41 mg) obtained in Step 254-2 in MeOH (1.0 mL), palladium-carbon (21 mg) and Et₃N (13 µL) were added, and the mixture was stirred at room temperature for 4 hours in the presence of hydrogen gas. Furthermore, palladium-carbon (21 mg) and Et₃N (1.0 mL) were added thereto, and the mixture was stirred at room temperature for 2 hours in the presence of hydrogen gas. The reaction solution was filtered through Celite (R), concentrated, and purified by NH type silica gel column chromatography (mobile phase: CHCl₃/MeOH = 100/0 to 95/5; v/v) to afford the title compound (23 mg, colorless powder).
LCMS (ESI) m/z 490 [M+H]⁺, t_{R} = 0.858 min, mode H.

### Example 271: Synthesis of (23aS)-18-(3-aminoazetidin-1-yl)-16-methyl-1,3,4,13,14,15,16,23a-octahydro-2H,6H,12H-2 3,20-(metheno)pyrido[2,1-k]pyrimido[6,1-g][1,6,8,9,12]benzoxatetraazacyclopentadecin-6-o ne

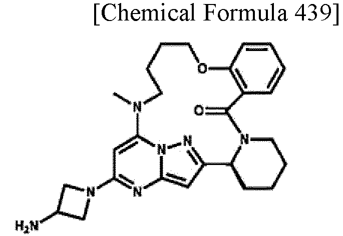

By using the same approaches as in Step 1-1 to Step 1-3 and Step 3-1, the title compound (16 mg, colorless amorphous) was obtained from the compound (0.22 g) obtained in Reference Example 1-1 (3), the compound (0.21 g) obtained in Reference Example 2-42, and 3-N-BOC-amino-azetidine mesylate salt (92 mg).
LCMS (ESI) m/z 476 [M+H]⁺, t_{R} = 0.500 min, mode N.

### Example 272: Synthesis of (23aS)-18-[(3S)-3-aminopyrrolidin-1-yl]-16-ethyl-8-fluoro-1,3,4,13,14,15,16,23a-octahydro-2H,6H,12H-23,20-(metheno)pyrido[2,1-k]pyrimido[6,1-g][1,6,8,9,12]benzoxatetraazacyclop entadecin-6-one

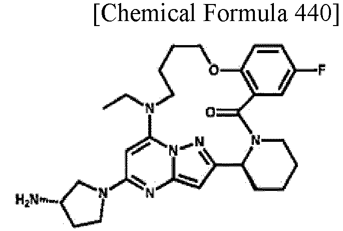

By using the same approaches as in Step 1-1 to Step 1-4, the title compound (18 mg, pink amorphous) was obtained from the compound (0.15 g) obtained in Reference Example 1-1 (3) and the compound (0.12 g) obtained in Reference Example 2-50.
LCMS (ESI) m/z 522 [M+H]⁺, t_{R} = 0.893 min, mode H.

### Example 273: Synthesis of (23aS)-16-(2-aminoethyl)-18-[(3S)-3-aminopyrrolidin-1-yl]-8-fluoro-1,3,4,13,14,15,16,23a-o ctahydro-2H,6H,12H-23,20-(metheno)pyrido[2,1-k]pyrimido[6,1-g][1,6,8,9,12]benzoxatetraa zacyclopentadecin-6-one hydrochloride

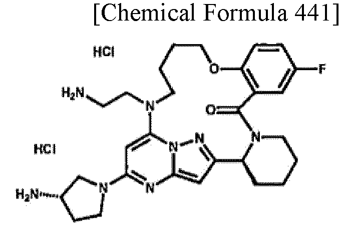

By using the same approaches as in Step 1-1 to Step 1-4 and Step 13-2, the title compound (39 mg, pink powder) was obtained from the compound (0.35 g) obtained in Reference Example 1-1 (3), the compound (0.78 g) obtained in Reference Example 2-55, and 3-N-BOC-amino-azetidine (0.12 g).
LCMS (ESI) m/z 537 [M+H]⁺, t_{R} = 0.771 min, mode H.

### Example 274: Synthesis of (23aS)-18-[(3S)-3-aminopyrrolidin-1-yl]-8-chloro-16-cyclopropyl-1,3,4,13,14,15,16,23a-octa hydro-2H,6H,12H-23,20-(metheno)pyrido[2,1-k]pyrimido[6,1-g][1,6,8,9,12]benzoxatetraaza cyclopentadecin-6-one

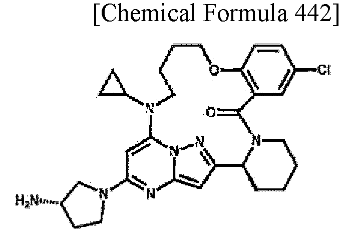

By using the same approaches as in Step 1-1 to Step 1-4, the title compound (51 mg, colorless powder) was obtained from the compound (85 mg) obtained in Reference Example 1-1 (3) and the compound (65 mg) obtained in Reference Example 2-52.
LCMS (ESI) m/z 550 [M+H]⁺, t_{R} = 0.601 min, mode N.

### Example 275: Synthesis of (23aS)-18-[(3S)-3-aminopyrrolidin-1-yl]-8-chloro-16-ethyl-1,3,4,13,14,15,16,23a-octahydro-2H,6H,12H-23,20-(metheno)pyrido[2,1-k]pyrimido[6,1-g][1,6,8,9,12]benzoxatetraazacyclop entadecin-6-one

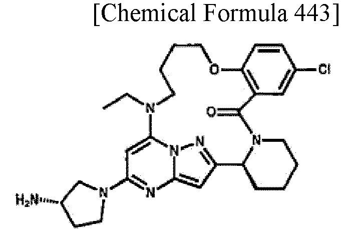

By using the same approaches as in Step 1-1 to Step 1-4, the title compound (72 mg, colorless powder) was obtained from the compound (0.20 g) obtained in Reference Example 1-1 (3) and the compound (0.16 g) obtained in Reference Example 2-51.
LCMS (ESI) m/z 538 [M+H]⁺, t_{R} = 0.545 min, mode N.

### Example 276: Synthesis of (23aS)-18-[(3S)-3-aminopyrrolidin-1-yl]-8-chloro-16-(2-hydroxyethyl)-1,3,4,13,14,15,16,23 a-octahydro-2H,6H,12H-23,20-(metheno)pyrido[2,1-k]pyrimido[6,1-g][1,6,8,9,12]benzoxate traazacyclopentadecin-6-one

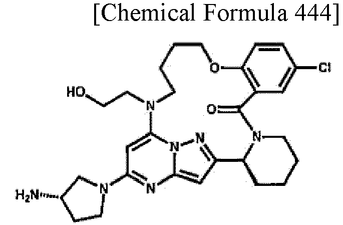

By using the same approaches as in Step 1-1 to Step 1-3, Step 121-3, and Step 1-4, the title compound (26 mg, colorless powder) was obtained from the compound (0.11 g) obtained in Reference Example 1-1 (3) and the compound (0.10 g) obtained in Reference Example 2-54.
LCMS (ESI) m/z 554 [M+H]⁺, t_{R} = 0.869 min, mode H.

### Example 277: Synthesis of (23aS)-18-[(3S)-3-aminopyrrolidin-1-yl]-8-chloro-16-(2-methoxyethyl)-1,3,4,13,14,15,16,23 a-octahydro-2H,6H,12H-23,20-(metheno)pyrido[2,1-k]pyrimido[6,1-g][1,6,8,9,12]benzoxate traazacyclopentadecin-6-one

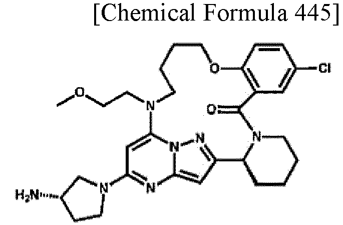

By using the same approaches as in Step 1-1 to Step 1-4, the title compound (13 mg, colorless powder) was obtained from the compound (35 mg) obtained in Reference Example 1-1 (3) and the compound (50 mg) obtained in Reference Example 2-53.
LCMS (ESI) m/z 568 [M+H]⁺, t_{R} = 0.547 min, mode N.

### Example 278: Synthesis of (23aS)-18-[(3S)-3-aminopyrrolidin-1-yl]-8-fluoro-16-methyl-1,3,4,11,12,13,14,15,16,23a-de cahydro-2H,6H-23,20-(metheno)pyrido[1,2-b]pyrimido[1,6-f][2,5,6,8]benzotetraazacyclopen tadecin-6-one

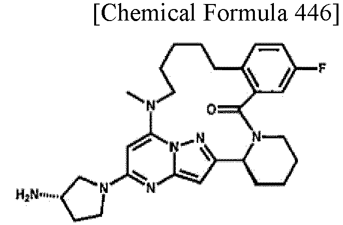

Step 278-1: By using the same approaches as in Step 1-1 to Step 1-3, (23aS)-18-chloro-8-fluoro-16-methyl-1,3,4,11,12,13,14,15,16,23a-decahydro-2H,6H-23,20-( metheno)pyrido[1,2-b]pyrimido[1,6-f][2,5,6,8]benzotetraazacyclopentadecin-6-one (0.52 g, colorless amorphous) was obtained from the compound (0.70 g) obtained in Reference Example 1-1 (3) and the compound (0.57 g) obtained in Reference Example 2-83.
LCMS (ESI) m/z 456 [M+H]⁺, t_{R} = 1.273 min, mode N.

Step 278-2: By using the same approach as in Step 1-4, the title compound (0.13 g, colorless amorphous) was obtained from the compound (0.15 g) obtained in Step 278-1. LCMS (ESI) m/z 506 [M+H]⁺, t_{R} = 0.541 min, mode N.

### Example 279: Synthesis of (23aS)-18-(azetidin-1-yl)-8-fluoro-16-methyl-1,3,4,11,12,13,14,15,16,23a-decahydro-2H,6H-23,20-(metheno)pyrido[1,2-b]pyrimido[1,6-f][2,5,6,8]benzotetraazacyclopentadecin-6-one

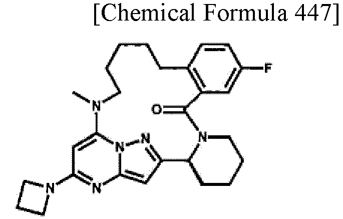

By using the same approach as in Step 1-4, the title compound (72 mg, colorless powder) was obtained from the compound (0.15 g) obtained in Step 278-1 and azetidine (0.22 mL).
LCMS (ESI) m/z 477 [M+H]⁺, t_{R} = 0.758 min, mode N.

### Example 280: Synthesis of (23aS)-18-(azetidin-1-yl)-8-fluoro-15-methyl-1,3,4,13,14,15,16,23a-octahydro-2H,6H,12H-2 3,20-(metheno)pyrido[2,1-k]pyrimido[6,1-g][1,5,8,9,12]benzoxatetraazacyclopentadecin-6-o ne

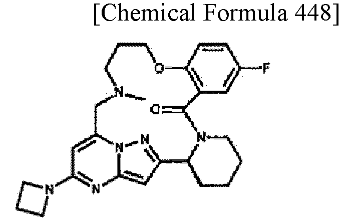

Step 280-1: By using the same approach as in Step 138, tert-butyl (2S)-2-[5-(azetidin-1-yl)-7-(methylsulfonyloxymethyl)pyrazolo[1,5-a]pyrimidin-2-yl]piperidi ne-1-carboxylate (72 mg, light yellow oily matter) was obtained from the compound (50 mg) obtained in Reference Example 1-21 (4).
LCMS (ESI) m/z 466 [M+H]⁺, t_{R} = 1.061 min, mode N.

Step 280-2: To the compound (37 mg) obtained in Step 280-1, a solution of K₂CO₃ (44 mg) and the compound (44 mg) obtained in Reference Example 2-115 in DMF (3.0 mL) was added, and the mixture was stirred at 80°C for 2 hours. To the reaction liquid, water was added, and the mixture was extracted with hexane/EtOAc (1/3; v/v) twice, which was washed with Brine, dried over MgSO₄, filtered, and concentrated. The obtained residue was purified by NH type silica gel chromatography (mobile phase: hexane/EtOAc = 100/0 to 60/40; v/v) and then purified by OH type silica gel chromatography (mobile phase: hexane/EtOAc = 50/50 to 0/100; v/v) to afford tert-butyl

(2S)-2-[5-(azetidin-1-yl)-7-[[3-(4-fluoro-2-methoxycarbonyl-phenoxy)propyl-methyl-amino] methyl]pyrazolo[1,5-a]pyrimidin-2-yl]piperidine-1-carboxylate (30 mg, colorless oily matter).
LCMS (ESI) m/z 611 [M+H]⁺, t_{R} = 0.806 min, mode N.

Step 280-3: By using the same approaches as in Step 13-2, Step 1-2, and Step 1-3, the title compound (6.0 mg, colorless amorphous) was obtained from the compound (30 mg) obtained in Step 280-2.
LCMS (ESI) m/z 479 [M+H]⁺, t_{R} = 0.944 min, mode H.

### Example 281: Synthesis of (23aS)-18-(azetidin-1-yl)-8-chloro-15-methyl-1,3,4,13,14,15,16,23a-octahydro-2H,6H,12H-2 3,20-(metheno)pyrido[2,1-k]pyrimido[6,1-g][1,5,8,9,12]benzoxatetraazacyclopentadecin-6-o ne

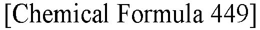

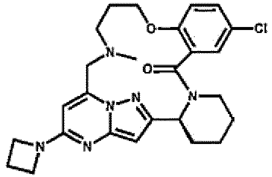

By using the same approaches as in Step 280-2, Step 13-2, Step 1-2, and Step 1-3, the title compound (42 mg, colorless amorphous) was obtained from the compound (74 mg) obtained in Step 280-2 and the compound (93 mg) obtained in Reference Example 2-116. LCMS (ESI) m/z 495 [M+H]⁺, t_{R} = 0.615 min, mode N.

### Example 282: Synthesis of

(23aS)-8-chloro-18-(3-hydroxyazetidin-1-yl)-15-methyl-1,3,4,13,14,15,16,23a-octahydro-2H ,6H,12H-23,20-(metheno)pyrido[2,1-k]pyrimido[6,1-g][1,5,8,9,12]benzoxatetraazacyclopent adecin-6-one

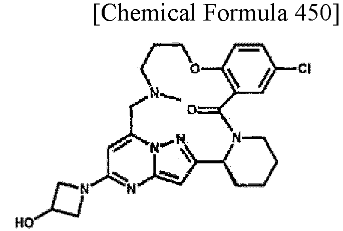

By using the same approaches as in Step 138, Step 280-2, Step 121-3, Step 13-2, Step 1-2, and Step 1-3, the title compound (69 mg, colorless amorphous) was obtained from the compound (0.31 g) obtained in Reference Example 1-22 (2) and the compound (0.25 g) obtained in Reference Example 2-116.
LCMS (ESI) m/z 511 [M+H]⁺, t_{R} = 0.550 min, mode N.

### Example 283: Synthesis of (23aS)-18-[(3S)-3-aminopyrrolidin-1-yl]-8-chloro-17-methyl-1,3,4,13,14,15,16,23a-octahydr o-2H,6H,12H-23,20-(metheno)pyrido[2,1-k]pyrimido[6,1-g][1,6,8,9,12]benzoxatetraazacycl opentadecin-6-one

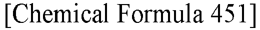

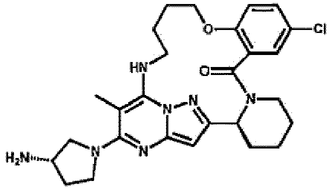

Step 283-1: By using the same approaches as in Step 1-1 to Step 1-3, (23aS)-8,18-dichloro-17-methyl-1,3,4,13,14,15,16,23a-octahydro-2H,6H,12H-23,20-(methen o)pyrido[2,1-k]pyrimido[6,1-g][1,6,8,9,12]benzoxatetraazacyclopentadecin-6-one (0.14 g, colorless powder) was obtained from the compound (0.15 g) obtained in Reference Example 1-3 and the compound (0.15 g) obtained in Reference Example 2-43.
LCMS (ESI) m/z 474 [M+H]⁺, t_{R} = 1.234 min, mode N.

Step 283-2: By using the same approach as in Step 1-4, the title compound (55 mg, colorless amorphous) was obtained from the compound (74 mg) obtained in Step 283-1. LCMS (ESI) m/z 524 [M+H]⁺, t_{R} = 0.547 min, mode N.

### Example 284: Synthesis of (23aS)-18-(azetidin-1-yl)-8-chloro-17-methyl-1,3,4,13,14,15,16,23a-octahydro-2H,6H,12H-2 3,20-(metheno)pyrido[2,1-k]pyrimido[6,1-g][1,6,8,9,12]benzoxatetraazacyclopentadecin-6-o ne

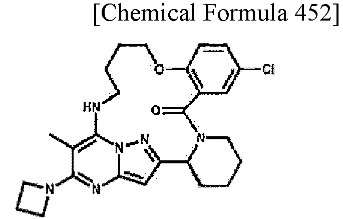

By using the same approach as in Step 3-1, the title compound (8.0 mg, colorless powder) was obtained from the compound (20 mg) obtained in Step 283-1 and azetidine hydrochloride (39 mg).
LCMS (ESI) m/z 495 [M+H]⁺, t_{R} = 0.746 min, mode N.

### Example 285: Synthesis of (23aS)-18-[(2-aminoethyl)amino]-8-chloro-17-methyl-1,3,4,13,14,15,16,23a-octahydro-2H,6 H,12H-23,20-(metheno)pyrido[2,1-k]pyrimido[6,1-g][1,6,8,9,12]benzoxatetraazacyclopentad ecin-6-one hydrochloride

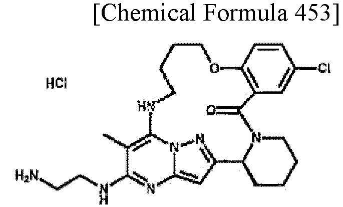

By using the same approaches as in Step 1-4 and Step 13-2, the title compound (2.2 mg, brown powder) was obtained from the compound (23 mg) obtained in Step 283-1 and tert-butyl N-(2-aminoethyl)carbamate (77 mg).
LCMS (ESI) m/z 498 [M+H]⁺, t_{R} = 0.583 min, mode N.

### Example 286: Synthesis of ] (23aS)-18-[(3S)-3-aminopyrrolidin-1-yl]-8-chloro-16,17-dimethyl-1,3,4,13,14,15,16,23a-octa hydro-2H,6H,12H-23,20-(metheno)pyrido[2,1-k]pyrimido[6,1-g][1,6,8,9,12]benzoxatetraaza cyclopentadecin-6-one

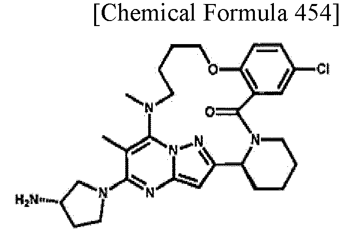

By using the same approaches as in Step 1-1 to Step 1-4, the title compound (64 mg, colorless powder) was obtained from the compound (0.16 g) obtained in Reference Example 1-3 and the compound (0.16 g) obtained in Reference Example 2-26.
LCMS (ESI) m/z 538 [M+H]⁺, t_{R} = 0.697 min, mode N.

### Example 287: Synthesis of (23aS)-18-[(3S)-3-aminopyrrolidin-1-yl]-8-chloro-17-fluoro-16-methyl-1,3,4,13,14,15,16,23 a-octahydro-2H,6H,12H-23,20-(metheno)pyrido[2,1-k]pyrimido[6,1-g][1,6,8,9,12]benzoxate traazacyclopentadecin-6-one hydrochloride

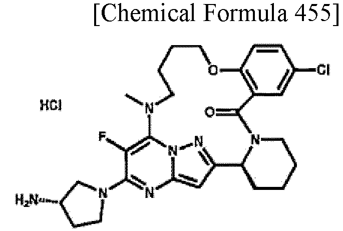

By using the same approaches as in Step 1-1, Step 1-4, Step 1-2, Step 1-3, and Step 13-2, the title compound (38 mg, light pink powder) was obtained from the compound (0.37 g) obtained in Reference Example 1-4, the compound (0.20 g) obtained in Reference Example 2-26, and tert-butyl N-[(3S)-pyrrolidin-3-yl]carbamate (0.20 g).
LCMS (ESI) m/z 542 [M+H]⁺, t_{R} = 0.725 min, mode N.

### Example 288: Synthesis of (23aS)-18-[(3S)-3-aminopyrrolidin-1-yl]-8-chloro-16-ethyl-17-fluoro-1,3,4,13,14,15,16,23a-octahydro-2H,6H,12H-23,20-(metheno)pyrido[2,1-k]pyrimido[6,1-g][1,6,8,9,12]benzoxatetr aazacyclopentadecin-6-one hydrochloride

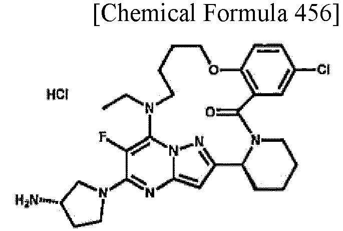

By using the same approaches as in Step 1-1, Step 1-4, Step 1-2, Step 1-3, and Step 13-2, the title compound (62 mg, light yellow powder) was obtained from the compound (0.49 g) obtained in Reference Example 1-4, the compound (0.48 g) obtained in Reference Example 2-51, and tert-butyl N-[(3S)-pyrrolidin-3-yl]carbamate (0.17 g).
LCMS (ESI) m/z 556 [M+H]⁺, t_{R} = 0.763 min, mode N.

### Example 289: Synthesis of (22aS)-17-[(3 S)-3-aminopyrrolidin-1-yl]-8-chloro-15,16-dimethyl-1,3,4,12,13,14,15,22a-octa hydro-2H,6H-22,19-(metheno)pyrido[2,1-j]pyrimido[6,1-f][1,5,7,8,11]benzoxatetraazacyclot etradecin-6-one

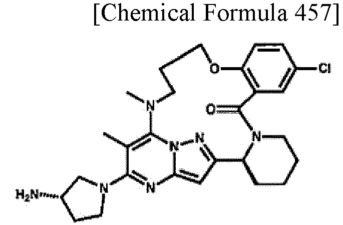

By using the same approaches as in Step 1-1 to Step 1-4, the title compound (21 mg, colorless powder) was obtained from the compound (56 mg) obtained in Reference Example 1-3 and the compound (51 mg) obtained in Reference Example 2-116.
LCMS (ESI) m/z 524 [M+H]⁺, t_{R} = 0.597 min, mode N.

### Example 290: Synthesis of (18S)-3-[(3S)-3-aminopyrrolidin-1-yl]-18-ethyl-5,14,17-trimethyl-6,7,9,10,17,18-hexahydro-5H,16H-19,1-(metheno)pyrimido[6,1-h][1,4,7,9,10,13]benzodioxatetraazacyclohexadecin-16 -one

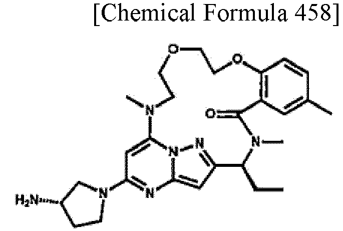

Step 290-1: By using the same approaches as in Step 1-1 to Step 1-3, (18S)-3-chloro-18-ethyl-5,14,17-trimethyl-6,7,9,10,17,18-hexahydro-5H,16H-19,1-(metheno )pyrimido[6,1-h][1,4,7,9,10,13]benzodioxatetraazacyclohexadecin-16-one (0.23 g, colorless amorphous) was obtained from the compound (1.0 g) obtained in Reference Example 1-15 and the compound (0.57 g) obtained in Reference Example 2-58.
LCMS (ESI) m/z 458 [M+H]⁺, t_{R} = 1.102 min, mode N.

Step 290-2: By using the same approach as in Step 1-4, the title compound (95 mg, colorless powder) was obtained from the compound (0.13 g) obtained in Step 290-1.
LCMS (ESI) m/z 508 [M+H]⁺, t_{R} = 0.810 min, mode H.

### Example 291: Synthesis of (18S)-3-[(2-aminoethyl)amino]-18-ethyl-5,14,17-trimethyl-6,7,9,10,17,18-hexahydro-5H,16 H-19,1-(metheno)pyrimido[6,1-h][1,4,7,9,10,13]benzodioxatetraazacyclohexadecin-16-one

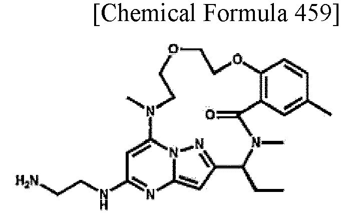

By using the same approaches as in Step 1-4 and Step 5-2, the title compound (40 mg, colorless powder) was obtained from the compound (0.12 g) obtained in Step 290-1 and tert-butyl N-(2-aminoethyl)carbamate (0.21 g).
LCMS (ESI) m/z 482 [M+H]⁺, t_{R} = 0.853 min, mode H.

### Example 292: Synthesis of (18S)-3-[(3S)-3-aminopyrrolidin-1-yl]-18-ethyl-5,14,17-trimethyl-6,7,10,11,17,18-hexahydro -5H-19,1-(metheno)pyrimido[6,1-h][4,1,7,9,10,13]benzoxapentaazacyclohexadecin-16(9H)-o ne

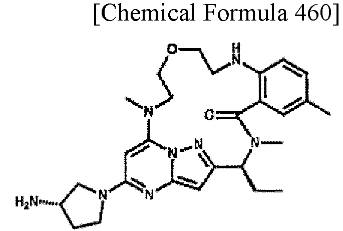

By using the same approaches as in Step 1-1 to Step 1-4, the title compound (18 mg, light yellow amorphous) was obtained from the compound (90 mg) obtained in Reference Example 1-15 and the compound (55 mg) obtained in Reference Example 2-59.
LCMS (ESI) m/z 507 [M+H]⁺, t_{R} = 0.449 min, mode N.

### Example 293: Synthesis of (18S)-3-[(3S)-3-aminopyrrolidin-1-yl]-18-ethyl-4,5,14,17-tetramethyl-6,7,9,10,17,18-hexahy dro-5H,16H-19,1-(metheno)pyrimido[6, 1-h][1,4,7,9,10,13]benzodioxatetraazacyclohexadeci n-16-one

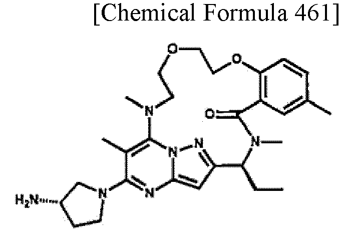

By using the same approaches as in Step 206-1 and Step 1-2 to Step 1-4, the title compound (15 mg, colorless amorphous) was obtained from the compound (77 mg) obtained in Reference Example 1-16 and the compound (76 mg) obtained in Reference Example 2-58. LCMS (ESI) m/z 522 [M+H]⁺, t_{R} = 0.574 min, mode N.

### Example 294: Synthesis of (18aS)-13-(azetidin-1-yl)-2-fluoro-11-methyl-6,7,10,11,19,20,21,22-octahydro-9H,18aH,24H -18,15-(metheno)pyrido[2,1-1]pyrimido[6,1-h][1,4,7,9,10,13]benzodioxatetraazacyclohexade cin-24-one

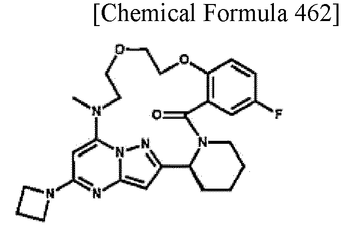

Step 294-1: By using the same approaches as in Step 1-1 to Step 1-3, (18aS)-13-chloro-2-fluoro-11-methyl-6,7,10,11,19,20,21,22-octahydro-9H,18aH,24H-18,15-( metheno)pyrido[2,1-1]pyrimido[6,1-h][1,4,7,9,10,13]benzodioxatetraazacyclohexadecin-24-o ne (0.13 g, colorless amorphous) was obtained from the compound (0.20 g) obtained in Reference Example 1-1 (3) and the compound (0.14 g) obtained in Reference Example 2-56. LCMS (ESI) m/z 474 [M+H]⁺, t_{R} = 1.075 min, mode N.

Step 294-2: By using the same approach as in Step 1-4, the title compound (55 mg, colorless powder) was obtained from the compound (62 mg) obtained in Step 294-1 and azetidine (88 µL).
¹H NMR (400 MHz, CDCl₃) δ ppm 1.38 - 1.51 (m, 2H), 1.64 - 1.76 (m, 2H), 1.90 - 2.03 (m, 1H), 2.07 - 2.15 (m, 0.1H), 2.20 - 2.28 (m, 0.9H), 2.34 - 2.43 (m, 2H), 3.02 (s, 0.3H), 3.06 (s, 2.7H), 3.35 - 3.51 (m, 2.7H), 3.78 - 3.84 (m, 0.3H), 3.86 - 4.00 (m, 2.1H), 4.05 - 4.20 (m, 6.8H), 4.21 - 4.36 (m, 1.8H), 4.36 - 4.48 (m, 0.2H), 4.63 - 4.72 (m, 0.1H), 4.94 (s, 0.9H), 4.96 (s, 0.1H), 4.99 - 5.04 (m, 0.1H), 5.76 (s, 0.1H), 5.99 (s, 0.9H), 6.14 - 6.19 (m, 0.9H), 6.74 - 6.79 (m, 0.1H), 6.80 - 6.87 (m, 0.9H), 6.93 - 7.06 (m, 2H).
LCMS (ESI) m/z 495 [M+H]⁺, t_{R} = 0.627 min, mode N.

### Example 295: Synthesis of (18aS)-2-fluoro-13-(3-hydroxyazetidin-1-yl)-11-methyl-6,7,10,11,19,20,21,22-octahydro-9H, 18aH,24H-18, 15-(metheno)pyrido[2,1-1]pyrimido[6,1-h][1,4,7,9,10,13]benzodioxatetraazacy clohexadecin-24-one

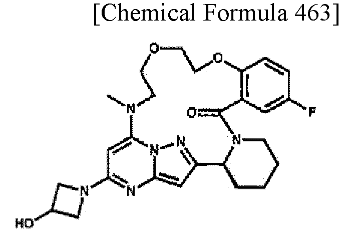

By using the same approach as in Step 3-1, the title compound (28 mg, colorless powder) was obtained from the compound (33 mg) obtained in Step 294-1 and 3-hydroxyazetidine hydrochloride (76 mg).
LCMS (ESI) m/z 511 [M+H]⁺, t_{R} = 0.997 min, mode H.

### Example 296: Synthesis of (18aS)-13-(3-aminoazetidin-1-yl)-2-fluoro-11-methyl-6,7,10,11,19,20,21,22-octahydro-9H, 1 8aH,24H-18,15-(metheno)pyrido[2,1-1]pyrimido[6,1-h][1,4,7,9,10,13]benzodioxatetraazacycl ohexadecin- 24-one

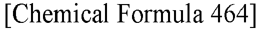

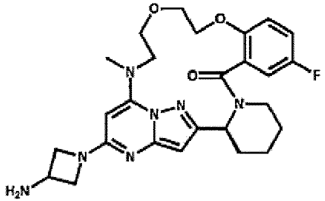

By using the same approach as in Step 3-1, the title compound (21 mg, colorless powder) was obtained from the compound (33 mg) obtained in Step 294-1 and 3-N-BOC-amino-azetidine mesylate salt (0.19 g).
LCMS (ESI) m/z 510 [M+H]⁺, t_{R} = 0.812 min, mode H.

### Example 297: Synthesis of (18aS)-13-[(3S)-3-aminopyrrolidin-1-yl]-2-chloro-11-methyl-6,7,10,11,19,20,21,22-octahydr o-9H,18aH,24H-18,15-(metheno)pyrido[2,1-1]pyrimido[6,1-h][1,4,7,9,10,13]benzodioxatetra azacyclohexadecin-24-one

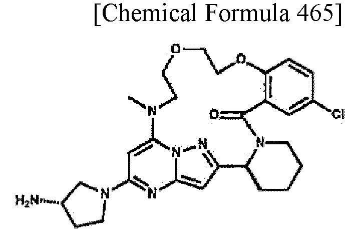

Step 297-1: By using the same approaches as in Step 1-1 to Step 1-3, (18aS)-2,13-dichloro-11-methyl-6,7,10,11,19,20,21,22-octahydro-9H,18aH,24H-18,15-(meth eno)pyrido[2,1-1]pyrimido[6,1-h][1,4,7,9,10,13]benzodioxatetraazacyclohexadecin-24-one (52 mg, colorless powder) was obtained from the compound (0.15 g) obtained in Reference Example 1-1 (3) and the compound (0.16 g) obtained in Reference Example 2-57.
LCMS (ESI) m/z 490 [M+H]⁺, t_{R} = 1.146 min, mode N.

Step 297-2: By using the same approach as in Step 1-4, the title compound (31 mg, colorless powder) was obtained from the compound (48 mg) obtained in Step 297-1.
LCMS (ESI) m/z 540 [M+H]⁺, t_{R} = 0.860 min, mode H.

### Example 298: Synthesis of (18aS)-13-(3-aminoazetidin-1-yl)-2-chloro-11-methyl-6,7,10,11,19,20,21,22-octahydro-9H, 1 8aH,24H-18,15-(metheno)pyrido[2,1-1]pyrimido[6,1-h][1,4,7,9,10,13]benzodioxatetraazacycl ohexadecin-24-one

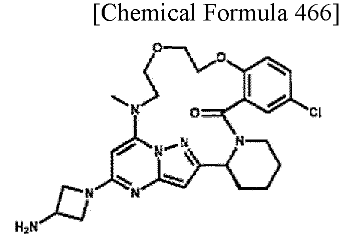

By using the same approaches as in Step 3-1 and Step 5-2, the title compound (62 mg, colorless powder) was obtained from the compound (70 mg) obtained in Step 297-1 and 3-N-BOC-amino-azetidine mesylate salt (0.19 g).
LCMS (ESI) m/z 526 [M+H]⁺, t_{R} = 0.504 min, mode N.

### Example 299: Synthesis of (18aS)-13-[(3S)-3-aminopyrrolidin-1-yl]-2-chloro-12-methyl-6,7,10,11,19,20,21,22-octahydr o-9H,18aH,24H-18,15-(metheno)pyrido[2,1-1]pyrimido[6,1-h][1,4,7,9,10,13]benzodioxatetra azacyclohexadecin- 24-one

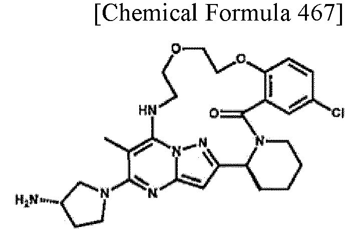

By using the same approaches as in Step 1-1 to Step 1-4, the title compound (0.22 g, colorless oily matter) was obtained from the compound (0.15 g) obtained in Reference Example 1-3 and the compound (0.16 g) obtained in Reference Example 2-117.
LCMS (ESI) m/z 540 [M+H]⁺, t_{R} = 0.640 min, mode N.

### Example 300: Synthesis of (24aS)-19-[(3S)-3-aminopyrrolidin-1-yl]-17-methyl-1,3,4,12,13,16,17,24a-octahydro-2H,15 H-24,21-(metheno)dipyrido[2,1-i:2',1'-1]pyrimido[6,1-e][1,4,6,7,10,13]oxapentaazacyclopent adecine-6, 10-dione

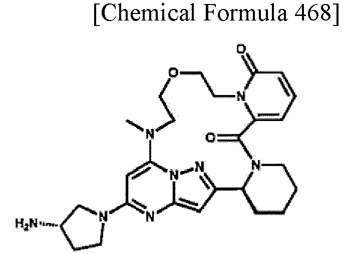

By using the same approaches as in Step 1-1 to Step 1-4, the title compound (32 mg, colorless amorphous) was obtained from the compound (80 mg) obtained in Reference Example 1-1 (3) and the compound (53 mg) obtained in Reference Example 2-63.
LCMS (ESI) m/z 507 [M+H]⁺, t_{R} = 0.601, 0.626 min, mode H.

### Example 301: Synthesis of (17S)-3-[(3S)-3-aminopyrrolidin-1-yl]-17-ethyl-5,13,16-trimethyl-7,8,16,17-tetrahydro-5H-1 8,1-(metheno)-gλ⁶-pyrimido[6,1-g][2,1,6,8,9,12]benzothiapentaazacyclopentadecine-9,9,15( 6H,10H)-trione

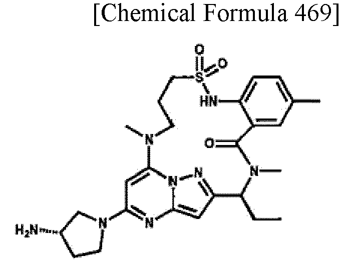

By using the same approaches as in Step 1-1, Step 202-2, Step 1-3, and Step 1-4, the title compound (6.6 mg, colorless amorphous) was obtained from the compound (0.32 g) obtained in Reference Example 1-15 and the compound (0.20 g) obtained in Reference Example 2-87.
LCMS (ESI) m/z 541 [M+H]⁺, t_{R} = 0.831 min, mode H.

### Example 302: Synthesis of (17S)-3-[(3S)-3-aminopyrrolidin-1-yl]-17-ethyl-13,16-dimethyl-7,8,16,17-tetrahydro-5H-18, 1-(metheno)-9λ⁶-pyrimido[6,1-g][2,1,6,8,9,12]benzothiapentaazacyclopentadecine-9,9,15(6H ,10H)-trione

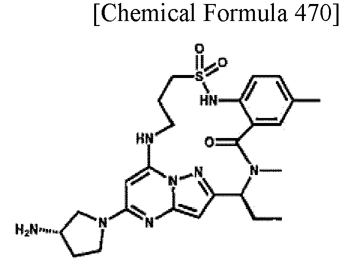

By using the same approaches as in Step 206-1 and Step 1-2 to Step 1-4, the title compound (6.7 mg, light orange powder) was obtained from the compound (0.21 g) obtained in Reference Example 1-15 and the compound (0.23 g) obtained in Reference Example 2-94. LCMS (ESI) m/z 527 [M+H]⁺, t_{R} = 0.759 min, mode H.

### Example 303: Synthesis of (17S)-3-[(3S)-3-aminopyrrolidin-1-yl]-13,17-diethyl-5,16-dimethyl-7,8,16,17-tetrahydro-5H-18,1-(metheno)-9λ⁶-pyrimido[6,1-g][2, 1,6,8,9,12]benzothiapentaazacyclopentadecine-9,9, 15 (6H,10H)-trione

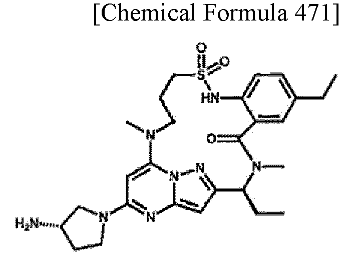

Step 303-1: By using the same approaches as in Step 1-1, Step 202-2, and Step 1-3, (17S)-3-chloro-13,17-diethyl-5,16-dimethyl-7,8,16,17-tetrahydro-5H-18,1-(metheno)-9λ⁶-py rimido[6,1-g][2,1,6,8,9,12]benzothiapentaazacyclopentadecine-9,9,15(6H,10H)-trione (0.11 g, colorless amorphous) was obtained from the compound (0.28 g) obtained in Reference Example 1-15 and the compound (0.29 g) obtained in Reference Example 2-86.
LCMS (ESI) m/z 505 [M+H]⁺, t_{R} = 1.176 min, mode N.

Step 303-2: By using the same approach as in Step 1-4, the title compound (37 mg, colorless amorphous) was obtained from the compound (40 mg) obtained in Step 303-1. LCMS (ESI) m/z 555 [M+H]⁺, t_{R} = 0.518 min, mode N.

### Example 304: Synthesis of (17S)-3-[(2-aminoethyl)amino]-13,17-diethyl-5,16-dimethyl-7,8,16,17-tetrahydro-5H-18,1-( metheno)-9λ⁶-pyrimido[6,1-g][2,1,6,8,9,12]benzothiapentaazacyclopentadecine-9,9,15(6H,1 0H)-trione

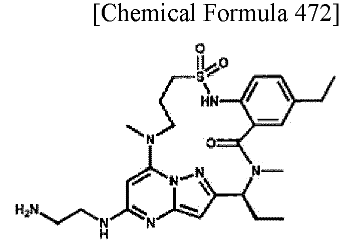

By using the same approaches as in Step 1-4 and Step 5-2, the title compound (26 mg, colorless amorphous) was obtained from the compound (40 mg) obtained in Step 303-1 and tert-butyl N-(2-aminoethyl)carbamate (0.13 g).
LCMS (ESI) m/z 529 [M+H]⁺, t_{R} = 0.562 min, mode N.

### Example 305: Synthesis of (17S)-3-[(3S)-3-aminopyrrolidin-1-yl]-13,17-diethyl-5,10,16-trimethyl-7,8,16,17-tetrahydro-5H-18,1-(metheno)-9λ⁶-pyrimido[6,1-g][2,1,6,8,9,12]benzothiapentaazacyclopentadecine-9,9 ,15(6H,10H)-trione

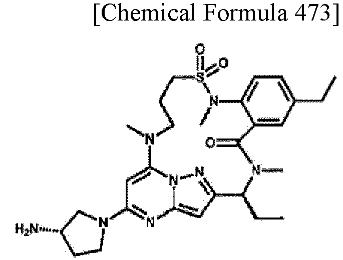

Step 305-1: The compound (15 mg) obtained in Step 303-1 was dissolved in DMF (0.30 mL), K₂CO₃ (25 mg) and MeI (5.5 µL) were added thereto, and the mixture was stirred at room temperature for 1 hour. After adding water to the reaction liquid and stirring the mixture, it was extracted with EtOAc/toluene (1/4, v/v). The organic layer was concentrated under reduced pressure, and the resulting residue was purified by OH type silica gel chromatography (mobile phase: hexane/EtOAc = 90/10 to 0/100; v/v) to afford (17S)-3-chloro-13,17-diethyl-5,10,16-trimethyl-7,8,16,17-tetrahydro-5H-18,1-(metheno)-9λ⁶ -pyrimido[6,1-g][2,1,6,8,9,12]benzothiapentaazacyclopentadecine-9,9, 15(6H,10H)-trione (13 mg, colorless powder).
LCMS (ESI) m/z 519 [M+H]⁺, t_{R} = 1.129 min, mode N.

Step 305-2: By using the same approach as in Step 1-4, the title compound (12 mg, colorless amorphous) was obtained from the compound (12 mg) obtained in Step 305-1. LCMS (ESI) m/z 569 [M+H]⁺, t_{R} = 0.463 min, mode N.

### Example 306: Synthesis of (17S)-3-[(2-amino-2-methylpropyl)amino]-13,17-diethyl-5,16-dimethyl-7,8,16,17-tetrahydro -5H-18,1-(metheno)-9λ⁶-pyrimido[6,1-g][2,1,6,8,9,12]benzothiapentaazacyclopentadecine-9, 9,15(6H,10H)-trione

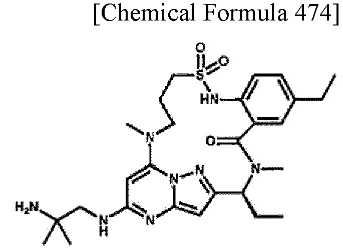

By using the same approach as in Step 1-4, the title compound (5.1 mg, colorless amorphous) was obtained from the compound (15 mg) obtained in Step 303-1 and 1,2-diamino-2-methylpropane (31 µL).
LCMS (ESI) m/z 557 [M+H]⁺, t_{R} = 0.636 min, mode N.

### Example 307: Synthesis of (17S)-3-[(3S)-3-aminopyrrolidin-1-yl]-17-ethyl-4,5,13,16-tetramethyl-7,8,16,17-tetrahydro-5 H-18,1-(metheno)-9λ⁶-pyrimido[6,1-g][2,1,6,8,9,12]benzothiapentaazacyclopentadecine-9,9, 15(6H,10H)-trione

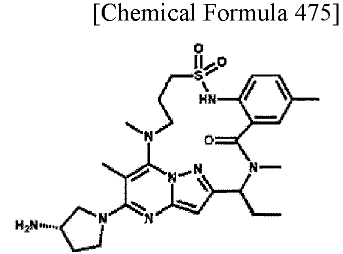

By using the same approaches as in Step 206-1 and Step 1-2 to Step 1-4, the title compound (24 mg, colorless amorphous) was obtained from the compound (0.20 g) obtained in Reference Example 1-16 and the compound (0.16 g) obtained in Reference Example 2-87. LCMS (ESI) m/z 555 [M+H]⁺, t_{R} = 0.610 min, mode N.

### Example 308: Synthesis of (17S)-3-[(3S)-3-aminopyrrolidin-1-yl]-17-ethyl-4,13,16-trimethyl-7,8,16,17-tetrahydro-5H-1 8,1-(metheno)-9λ⁶-pyrimido[6,1-g][2, 1,6,8,9,12]benzothiapentaazacyclopentadecine-9,9, 15( 6H,10H)-trione

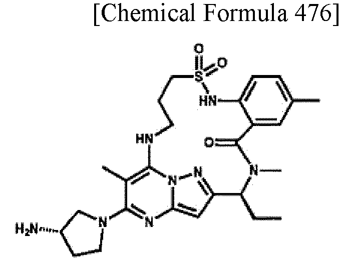

Step 308-1: By using the same approaches as in Step 206-1, Step 1-2, and Step 1-3, (17S)-3-chloro-17-ethyl-4,13,16-trimethyl-7,8,16,17-tetrahydro-5H-18,1-(metheno)-9λ⁶-pyri mido[6,1-g][2,1,6,8,9,12]benzothiapentaazacyclopentadecine-9,9,15(6H,10H)-trione(97 mg, colorless powder) was obtained from the compound (0.30 g) obtained in Reference Example 1-16 and the compound (0.38 g) obtained in Reference Example 2-94.
LCMS (ESI) m/z 491 [M+H]⁺, t_{R} = 1.078 min, mode N.

Step 308-2: By using the same approach as in Step 1-4, the title compound (13 mg, colorless powder) was obtained from Step 308-1 (20 mg).
LCMS (ESI) m/z 541 [M+H]⁺, t_{R} = 0.501 min, mode N.

### Example 309: Synthesis of (17S)-3-[(2-aminoethyl)amino]-17-ethyl-4,13,16-trimethyl-7,8,16,17-tetrahydro-5H-18,1-(m etheno)-9λ⁶-pyrimido[6,1-g][2,1,6,8,9,12]benzothiapentaazacyclopentadecine-9,9,15(6H,10 H)-trione

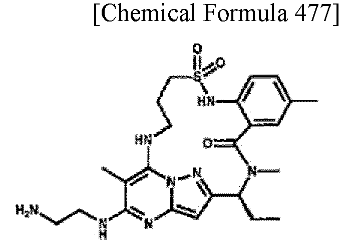

By using the same approaches as in Step 1-4 and Step 13-2, the title compound (1.7 mg, colorless powder) was obtained from the compound (20 mg) obtained in Step 308-1 and tert-butyl N-(2-aminoethyl)carbamate (65 mg).
LCMS (ESI) m/z 515 [M+H]⁺, t_{R} = 0.547 min, mode N.

### Example 310: Synthesis of (16S)-3-[(3S)-3-aminopyrrolidin-1-yl]-16-ethyl-5,12,15-trimethyl-6,7,15,16-tetrahydro-17,1-(metheno)-8λ⁶-pyrimido[6,1-f][2,1,5,7,8,11]benzothiapentaazacyclotetradecine-8,8,14(5H,9 H)-trione

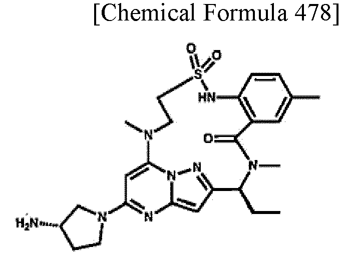

By using the same approaches as in Step 1-1 to Step 1-4, the title compound (21 mg, colorless powder) was obtained from the compound (0.60 g) obtained in Reference Example 1-15 and the compound (0.35 g) obtained in Reference Example 2-93.
LCMS (ESI) m/z 527 [M+H]⁺, t_{R} = 0.466 min, mode N.

### Example 311: Synthesis of (18S)-3-[(3S)-3-aminopyrrolidin-1-yl]-18-ethyl-5,14,17-trimethyl-6,7,8,9,17,18-hexahydro-1 9,1-(metheno)-10λ⁶-pyrimido[6,1-h][2,1,7,9,10,13]benzothiapentaazacyclohexadecine-10,10, 16(5H,11H)-trione

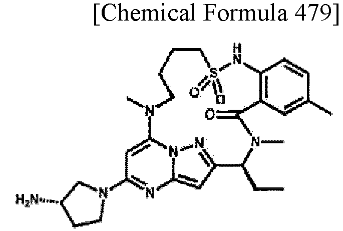

By using the same approaches as in Step 1-1, Step 202-2, Step 1-3, and Step 1-4, the title compound (22 mg, colorless amorphous) was obtained from the compound (0.25 g) obtained in Reference Example 1-15 and the compound (0.13 g) obtained in Reference Example 2-92.
LCMS (ESI) m/z 555 [M+H]⁺, t_{R} = 0.480 min, mode N.

### Example 312: Synthesis of (23aS)-18-[(3S)-3-aminopyrrolidin-1-yl]-8-chloro-16-methyl-1,3,4,13,14,15,16,23a-octahydr o-2H,6H-23,20-(metheno)-12λ⁶-pyrido[2,1-k]pyrimido[6,1-g][2,1,6,8,9,12]benzothiapentaaz acyclopentadecine-6,12,12(11H)-trione

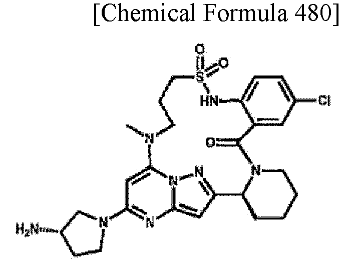

Step 312-1: By using the same approaches as in Step 1-1 to Step 1-3, (23aS)-8,18-dichloro-16-methyl-1,3,4,13,14,15,16,23a-octahydro-2H,6H-23,20-(metheno)-1 2λ⁶-pyrido[2,1-k]pyrimido[6,1-g][2,1,6,8,9,12]benzothiapentaazacyclopentadecine-6,12,12(1 1H)-trione (54 mg, colorless amorphous) was obtained from the compound (0.20 g) obtained in Reference Example 1-1 (3) and the compound (0.16 g) obtained in Reference Example 2-85.
LCMS (ESI) m/z 523 [M+H]⁺, t_{R} = 1.166 min, mode N.

Step 312-2: By using the same approach as in Step 1-4, the title compound (21 mg, colorless powder) was obtained from the compound (20 mg) obtained in Step 312-1. ¹H NMR (400 MHz, CDCl₃) δ ppm 1.51 - 2.83 (m, 10H), 2.87 - 3.14 (m, 5H), 3.14 - 3.86 (m, 9H), 4.00-4.11 (m, 0.8H), 4.41 -4.57(m, 0.2H), 5.18 (s, 1H), 5.47 (br s, 0.2H), 5.94 - 6.05 (m, 1.8H), 7.34 - 7.47 (m, 2H), 7.69 (d, J= 8.7 Hz, 0.8H), 7.81 - 7.93 (m, 0.2H). LCMS (ESI) m/z 573 [M+H]⁺, t_{R} = 0.494 min, mode N.

### Example 313: Synthesis of (23aS)-18-[(2-aminoethyl)amino]-8-chloro-16-methyl-1,3,4,13,14,15,16,23a-octahydro-2H,6 H-23,20-(metheno)-12λ⁶-pyrido[2,1-k]pyrimido[6,1-g][2,1,6,8,9,12]benzothiapentaazacyclop entadecine-6,12,12(11H)-trione

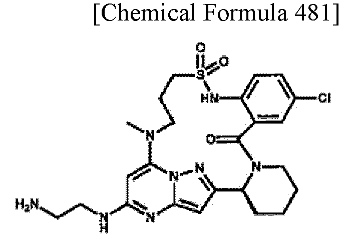

By using the same approaches as in Step 1-4 and Step 13-2, the title compound (31 mg, colorless powder) was obtained from the compound (0.10 g) obtained in Step 312-1 and tert-butyl N-(2-aminoethyl)carbamate (0.15 g).
LCMS (ESI) m/z 547 [M+H]⁺, t_{R} = 0.536 min, mode N.

### Example 314: Synthesis of (23aS)-18-[(3S)-3-aminopyrrolidin-1-yl]-8-chloro-11,16-dimethyl-1,3,4,13,14,15,16,23a-octa hydro-2H,6H-23,20-(metheno)-12λ⁶-pyrido[2,1-k]pyrimido[6,1-g][2,1,6,8,9,12]benzothiapen taazacyclopentadecine-6,12,12(11H)-trione

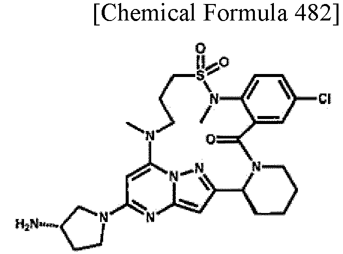

By using the same approaches as in Step 305-1 and Step 1-4, the title compound (31 mg, colorless amorphous) was obtained from the compound (30 mg) obtained in Step 312-1.
LCMS (ESI) m/z 587 [M+H]⁺, t_{R} = 0.480 min, mode N.

### Example 315: Synthesis of (23aS)-18-[(3S)-3-aminopyrrolidin-1-yl]-9-fluoro-8,16-dimethyl-1,3,4,13,14,15,16,23a-octah ydro-2H,6H-23,20-(metheno)-12λ⁶-pyrido[2,1-k]pyrimido[6,1-g][2,1,6,8,9,12]benzothiapent aazacyclopentadecine-6,12,12(11H)-trione

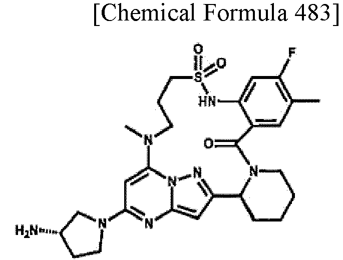

By using the same approaches as in Step 1-1 to Step 1-4, the title compound (10 mg, colorless amorphous) was obtained from the compound (0.22 g) obtained in Reference Example 1-1 (3) and the compound (0.19 g) obtained in Reference Example 2-88.
LCMS (ESI) m/z 571 [M+H]⁺, t_{R} = 0.502 min, mode N.

### Example 316: Synthesis of (22aS)-17-[(3S)-3-aminopyrrolidin-1-yl]-8,15-dimethyl-1,3,4,8,10,12,13,14,15,22a-decahydr o-2H-22,19-(metheno)-11λ⁶-pyrazolo[4,3-c]pyrido[1,2-f]pyrimido[1,6-j][1,2,6,9,10,12]thiap entaazacyclopentadecine-6,11,11-trione

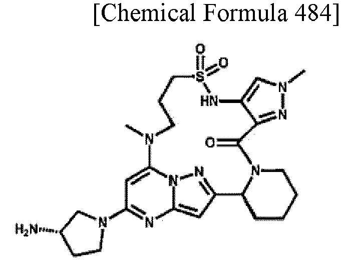

By using the same approaches as in Step 1-1 to Step 1-4, the title compound (42 mg, pink amorphous) was obtained from the compound (0.22 g) obtained in Reference Example 1-1 (3) and the compound (0.16 g) obtained in Reference Example 2-89.
LCMS (ESI) m/z 543 [M+H]⁺, t_{R} = 0.638 min, mode N.

### Example 317: Synthesis of (22aS)-17-[(3S)-3-aminopyrrolidin-1-yl]-15-methyl-1,3,4,12,13,14,15,22a-octahydro-2H,6H-22,19-(metheno)-11λ⁶-pyrido[1,2-f]pyrimido[1,6-j]thieno[3,2-c][1,2,6,9,10,12]thiapentaazac yclopentadecine-6,11,11(10H)-trione

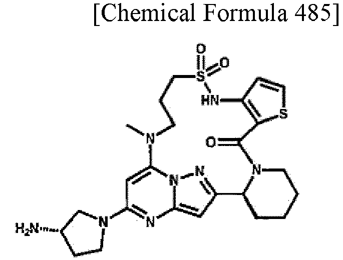

By using the same approaches as in Step 1-1 to Step 1-4, the title compound (39 mg, pink amorphous) was obtained from the compound (0.31 g) obtained in Reference Example 1-1 (3) and the compound (0.26 g) obtained in Reference Example 2-90.
LCMS (ESI) m/z 545 [M+H]⁺, t_{R} = 0.318 min, mode N.

### Example 318: Synthesis of (23aS)-18-(azetidin-1-yl)-16-ethyl-8-fluoro-1,3,4,13,14,15,16,23a-octahydro-2H,6H-23,20-( metheno)-12λ⁶-pyrido[2,1-k]pyrimido[6,1-g][2,1,6,8,9,12]benzothiapentaazacyclopentadecin e-6,12,12(11H)-trione

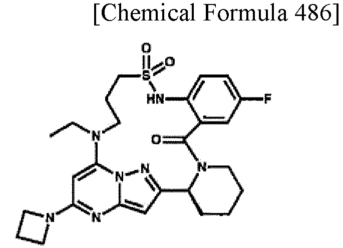

Step 318-1: By using the same approaches as in Step 1-1 to Step 1-3, (23aS)-18-chloro-16-ethyl-8-fluoro-1,3,4,13,14,15,16,23a-octahydro-2H,6H-23,20-(metheno )-12λ⁶-pyrido[2,1-k]pyrimido[6,1-g][2,1,6,8,9,12]benzothiapentaazacyclopentadecine-6,12,1 2(11H)-trione(0.36 g, light red amorphous) was obtained from the compound (0.80 g) obtained in Reference Example 1-1 (3) and the compound (0.68 g) obtained in Reference Example 2-91.
LCMS (ESI) m/z 521 [M+H]⁺, t_{R} = 1.134 min, mode N.

Step 318-2: By using the same approach as in Step 1-4, the title compound (28 mg, colorless amorphous) was obtained from the compound (60 mg) obtained in Step 318-1 and azetidine (39 µL).
LCMS (ESI) m/z 542 [M+H]⁺, t_{R} = 0.664 min, mode N.

### Example 319: Synthesis of (23aS)-18-[(2-aminoethyl)amino]-16-ethyl-8-fluoro-1,3,4,13,14,15,16,23a-octahydro-2H,6H-23,20-(metheno)-12λ⁶-pyrido[2,1-k]pyrimido[6,1-g][2,1,6,8,9,12]benzothiapentaazacyclopen tadecine-6,12,12(11H)-trione

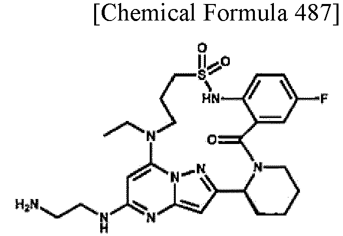

By using the same approaches as in Step 1-4 and Step 5-2, the title compound (57 mg, colorless powder) was obtained from the compound (0.15 g) obtained in Step 318-1 and tert-butyl N-(2-aminoethyl)carbamate (0.23 g).
LCMS (ESI) m/z 545 [M+H]⁺, t_{R} = 0.484 min, mode N.

### Example 320: Synthesis of (23aS)-18-(5-amino-2-azaspiro[3.3]heptan-2-yl)-16-ethyl-8-fluoro-1,3,4,13,14,15,16,23a-oct ahydro-2H,6H-23,20-(metheno)-12λ⁶-pyrido[2,1-k]pyrimido[6,1-g][2,1,6,8,9,12]benzothiape ntaazacyclopentadecine-6,12,12(11H)-trione

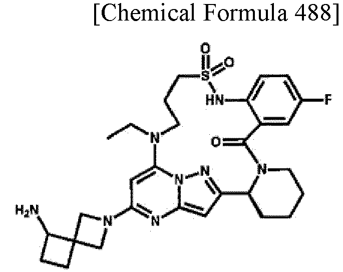

By using the same approaches as in Step 1-4 and Step 5-2, the title compound (25 mg, colorless powder) was obtained from the compound (88 mg) obtained in Step 318-1 and tert-butyl N-(2-azaspiro[3.3]heptan-5-yl)carbamate (0.18 g).
LCMS (ESI) m/z 597 [M+H]⁺, t_{R} = 0.489 min, mode N.

### Example 321: Synthesis of (23aS)-18-(6-amino-2-azaspiro[3.3]heptan-2-yl)-16-ethyl-8-fluoro-1,3,4,13,14,15,16,23a-oct ahydro-2H,6H-23,20-(metheno)-12λ⁶-pyrido[2,1-k]pyrimido[6,1-g][2,1,6,8,9,12]benzothiape ntaazacyclopentadecine-6,12,12(11H)-trione

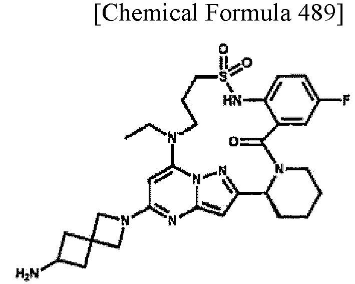

By using the same approaches as in Step 1-4 and Step 5-2, the title compound (31 mg, colorless powder) was obtained from the compound (76 mg) obtained in Step 318-1 and tert-butyl N-(2-azaspiro[3.3]heptan-6-yl)carbamate hydrochloride (0.23 g). LCMS (ESI) m/z 597 [M+H]⁺, t_{R} = 0.462 min, mode N.

### Example 322: Synthesis of (18aS)-13-(azetidin-1-yl)-2-fluoro-11-methyl-8,9,10,11,19,20,21,22-octahydro-18aH,24H-18 , 15-(metheno)-7λ⁶-pyrido[2,1-1]pyrimido[6,1-h][1,3,4,7,9,10,13]benzoxathiapentaazacyclohe xadecine-7,7,24(6H)-trione

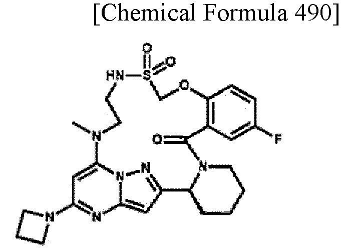

Step 322-1: By using the same approaches as in Step 1-1 to Step 1-3, (18aS)-13-chloro-2-fluoro-11-methyl-8,9,10,11,19,20,21,22-octahydro-18aH,24H-18,15-(me theno)-7λ⁶-pyrido[2,1-1]pyrimido[6,1-h][1,3,4,7,9,10,13]benzoxathiapentaazacyclohexadecin e-7,7,24(6H)-trione (0.17 g, colorless amorphous) was obtained from the compound (0.25 g) obtained in Reference Example 1-1 (3) and the compound (0.21 g) obtained in Reference Example 2-24.
LCMS (ESI) m/z 523 [M+H]⁺, t_{R} = 0.983 min, mode N.

Step 322-2: By using the same approach as in Step 1-4, the title compound (69 mg, colorless powder) was obtained from the compound (79 mg) obtained in Step 322-1 and azetidine (0.10 mL).
LCMS (ESI) m/z 544 [M+H]⁺, t_{R} = 0.571 min, mode N.

### Example 323: Synthesis of (18aS)-2-fluoro-13-(3-hydroxyazetidin-1-yl)-11-methyl-8,9,10,11,19,20,21,22-octahydro-18a H,24H-18,15-(metheno)-7λ⁶-pyrido[2,1-1]pyiimido[6,1-h][1,3,4,7,9,10,13]benzoxathiapentaa zacyclohexadecine-7,7,24(6H)-trione

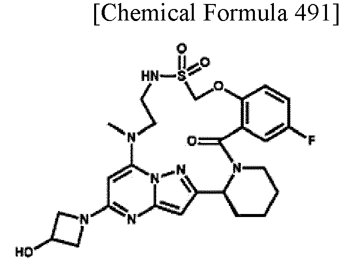

By using the same approach as in Step 3-1, the title compound (44 mg, colorless powder) was obtained from the compound (45 mg) obtained in Step 322-1 and 3-hydroxyazetidine hydrochloride (94 mg).
LCMS (ESI) m/z 560 [M+H]⁺, t_{R} = 0.519 min, mode N.

### Example 324: Synthesis of (18aS)-13-(3-aminoazetidin-1-yl)-2-fluoro-11-methyl-8,9,10,11,19,20,21,22-octahydro-18aH ,24H-18,15-(metheno)-7λ⁶-pyrido[2,1-1]pyrimido[6,1-h][1,3,4,7,9,10,13]benzoxathiapentaaz acyclohexadecine-7,7,24(6H)-trione

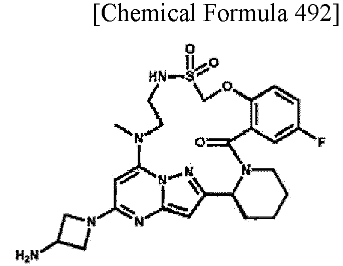

By using the same approaches as in Step 1-4 and Step 5-2, the title compound (17 mg, colorless powder) was obtained from the compound (45 mg) obtained in Step 322-1 and 3-N-BOC-amino-azetidine (0.15 g).
LCMS (ESI) m/z 559 [M+H]⁺, t_{R} = 0.763 min, mode H.

### Example 325: Synthesis of (18aS)-13-(azetidin-1-yl)-2-fluoro-8,11-dimethyl-8,9,10,11,19,20,21,22-octahydro-18aH,24 H-18,15-(metheno)-7λ⁶-pyrido[2,1-1]pyrimido[6,1-h][1,3,4,7,9,10,13]benzoxathiapentaazacy clohexadecine-7,7,24(6H)-trione

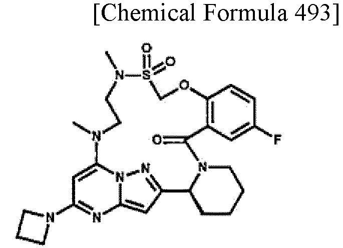

By using the same approach as in Step 7-1, the title compound (17 mg, colorless powder) was obtained from the compound (38 mg) obtained in Step 322-2.
LCMS (ESI) m/z 558 [M+H]⁺, t_{R} = 0.579 min, mode N.

### Example 326: Synthesis of (23aS)-18-[(3S)-3-aminopyrrolidin-1-yl]-8-chloro-16,17-dimethyl-1,3,4,13,14,15,16,23a-octa hydro-2H,6H-23,20-(metheno)-12λ⁶-pyrido[2,1-k]pyrimido[6,1-g][2,1,6,8,9,12]benzothiapen taazacyclopentadecine-6,12,12(11H)-trione

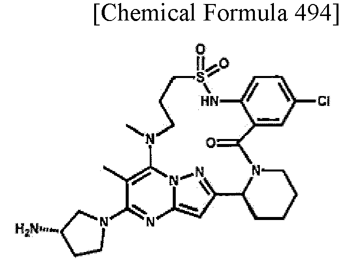

Step 326-1: By using the same approaches as in Step 1-1 to Step 1-3, (23aS)-8,18-dichloro-16,17-dimethyl-1,3,4,13,14,15,16,23a-octahydro-2H,6H-23,20-(methen o)-12λ⁶-pyrido[2,1-k]pyrimido[6,1-g][2,1,6,8,9,12]benzothiapentaazacyclopentadecine-6,12, 12(11H)-trione (0.18 g, light yellow amorphous) was obtained from the compound (0.24 g) obtained in Reference Example 1-3 and the compound (0.27 g) obtained in Reference Example 2-85.
LCMS (ESI) m/z 537 [M+H]⁺, t_{R} = 1.208 min, mode N.

Step 326-2: By using the same approach as in Step 1-4, the title compound (43 mg, colorless amorphous) was obtained from the compound (56 mg) obtained in Step 326-1. LCMS (ESI) m/z 587 [M+H]⁺, t_{R} = 0.630 min, mode N.

### Example 327: Synthesis of (23aS)-18-[(2-aminoethyl)amino]-8-chloro-16,17-dimethyl-1,3,4,13,14,15,16,23a-octahydro-2H,6H-23,20-(metheno)-12λ⁶-pyrido[2,1-k]pyrimido[6,1-g][2,1,6,8,9,12]benzothiapentaazac yclopentadecine-6,12,12(11H)-trione

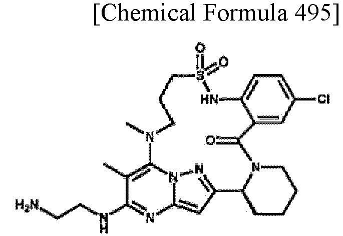

By using the same approaches as in Step 1-4 and Step 5-2, the title compound (28 mg, colorless amorphous) was obtained from the compound (72 mg) obtained in Step 326-1 and tert-butyl N-(2-aminoethyl)carbamate (0.21 g).
LCMS (ESI) m/z 561 [M+H]⁺, t_{R} = 0.644 min, mode N.

### Example 328: Synthesis of (20aS)-15-(3-aminoazetidin-1-yl)-2-chloro-9,13-dimethyl-12,13,21,22,23,24-hexahydro-6H, 11H,20aH,26H-20,17-(metheno)pyrido[2,1-1]pyrimido[6,1-h][1,2,4]triazolo[3,4-c][1,4,7,9,10 ,13]benzoxapentaazacyclohexadecin-26-one

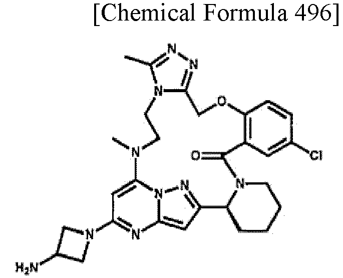

By using the same approaches as in Step 1-1 to Step 1-4 and Step 13-2, the title compound (7.0 mg, colorless powder) was obtained from the compound (27 mg) obtained in Reference Example 1-1 (3), the compound (27 mg) obtained in Reference Example 2-81, and 3-N-BOC-amino-azetidine (30 mg).
LCMS (ESI) m/z 577 [M+H]⁺, t_{R} = 0.813 min, mode H.

### Example 329: Synthesis of (20aS)-15-(azetidin-1-yl)-2-chloro-9,13-dimethyl-12,13,21,22,23,24-hexahydro-6H,11H,20a H,26H-20,17-(metheno)pyrido[2,1-1]pyrimido[6,1-h][1,2,4]triazolo[3,4-c][1,4,7,9,10,13]benz oxapentaazacyclohexadecin-26-one

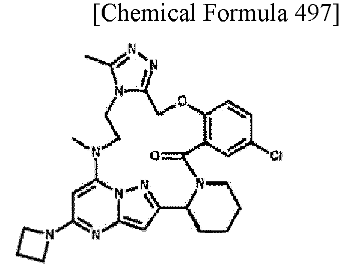

By using the same approaches as in Step 1-1 to Step 1-3 and Step 3-1, the title compound (14 mg, colorless powder) was obtained from the compound (0.22 g) obtained in Reference Example 1-1 (3), the compound (0.11 g) obtained in Reference Example 2-81, and azetidine.
LCMS (ESI) m/z 562 [M+H]⁺, t_{R} = 0.553 min, mode N.

### Example 330: Synthesis of (19aS)-14-(3-aminoazetidin-1-yl)-2-chloro-12-methyl-11,12,20,21,22,23-hexahydro-10H,19a H-6,9:19,16-di(metheno)pyrido[2,1-m]pyrimido[6,1-i] [2,3,4,8,10,11,14]benzoheptaazacyclo heptadecin-25(5H)-one

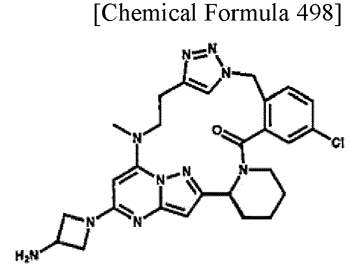

Step 330-1: By using the same approaches as in Step 1-1 to Step 1-3, (19aS)-2,14-dichloro-12-methyl-11,12,20,21,22,23-hexahydro-10H, 19aH-6,9:19,16-di(methe no)pyrido[2,1-m]pyrimido[6,1-i] [2,3,4,8,10,11,14]benzoheptaazacycloheptadecin-25(5H)-on e (0.14 g, colorless powder) was obtained from the compound (0.15 g) obtained in Reference Example 1-1 (3) and the compound (0.24 g) obtained in Reference Example 2-82.
LCMS (ESI) m/z 511 [M+H]⁺, t_{R} = 1.068 min, mode N.

Step 330-2: By using the same approaches as in Step 1-4 and Step 13-2, the title compound (53 mg, colorless amorphous) was obtained from the compound (80 mg) obtained in Step 330-1 and 3-N-BOC-amino-azetidine (80 mg).
LCMS (ESI) m/z 547 [M+H]⁺, t_{R} = 0.470 min, mode N.

### Example 331: Synthesis of (19aS)-14-(azetidin-1-yl)-2-chloro-12-methyl-11,12,20,21,22,23-hexahydro-10H,19aH-6,9:1 9,16-di(metheno)pyrido[2,1-m]pyrimido[6,1-i][2,3,4,8,10,11,14]benzoheptaazacycloheptade cin-25(5H)-one

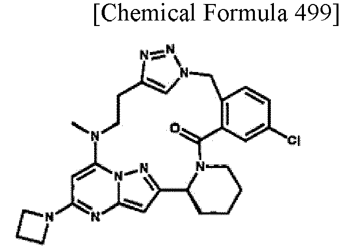

By using the same approach as in Step 1-4, the title compound (41 mg, colorless amorphous) was obtained from the compound (37 mg) obtained in Step 330-1 and azetidine (49 µL).
LCMS (ESI) m/z 532 [M+H]⁺, t_{R} = 0.642 min, mode N.

### Example 332: Synthesis of (19aS)-2-chloro-14-(3-hydroxyazetidin-1-yl)-12-methyl-11,12,20,21,22,23-hexahydro-10H,1 9aH-6,9:19,16-di(metheno)pyrido[2,1-m]pyrimido[6,1-i][2,3,4,8,10,11,14]benzoheptaazacycl oheptadecin-25(5H)-one

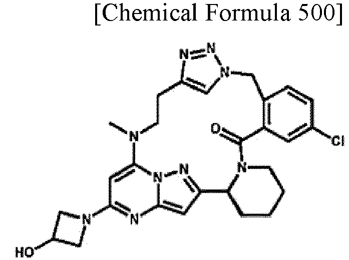

By using the same approach as in Step 3-1, the title compound (47 mg, colorless powder) was obtained from the compound (40 mg) obtained in Step 330-1 and 3-hydroxyazetidine hydrochloride (86 mg).
LCMS (ESI) m/z 548 [M+H]⁺, t_{R} = 0.602 min, mode N.

### Example 333: Synthesis of (20aS)-15-(azetidin-1-yl)-2-chloro-13-methyl-12,13,21,22,23,24-hexahydro-6H,11H,20aH,2 6H-20,17-(metheno)imidazo[2,1-c]pyrido[2,1-1]pyrimido[6,1-h][1,4,7,9,10,13]benzoxapenta azacyclohexadecin-26-one

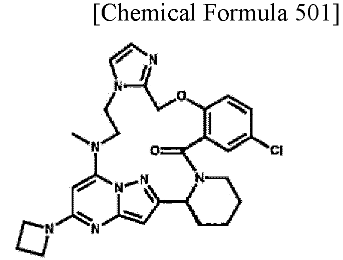

Step 333-1: By using the same approaches as in Step 1-1 to Step 1-3, (20aS)-2,15-dichloro-13-methyl-12,13,21,22,23,24-hexahydro-6H,11H,20aH,26H-20,17-(me theno)imidazo[2,1-c]pyrido[2,1-1]pyrimido[6,1-h][1,4,7,9,10,13]benzoxapentaazacyclohexad ecin-26-one (0.48 g, colorless amorphous) was obtained from the compound (0.42 g) obtained in Reference Example 1-1 (3) and the compound (0.53 g) obtained in Reference Example 2-80.
LCMS (ESI/APCI dual) m/z 526 [M+H]⁺, t_{R} = 0.579 min, mode N.

Step 333-2: By using the same approach as in Step 1-4, the title compound (0.11 g, colorless amorphous) was obtained from the compound (0.18 g) obtained in Step 333-1 and azetidine (0.23 mL).
¹H NMR (400 MHz, CDCl₃) δ ppm 1.32 - 2.03 (m, 5H), 2.27 - 2.46 (m, 4H), 2.56 (s, 3H), 2.84 - 3.13 (m, 1H), 3.24 - 3.44 (m, 2H), 3.96 - 4.23 (m, 6H), 4.94 - 5.08 (m, 1H), 5.27 - 5.36 (m, 1H), 5.63 - 5.75 (m, 1H), 6.10 (s, 1H), 6.21 - 6.28 (m, 1H), 6.84 - 6.89 (m, 1H), 6.93 - 7.11 (m, 2H), 7.16 - 7.24 (m, 1H), 7.35 - 7.40 (m, 1H).
LCMS (ESI/APCI dual) m/z 547 [M+H]⁺, t_{R} = 0.211 min, mode N.

### Example 334: Synthesis of (20aS)-2-chloro-15-(3-hydroxyazetidin-1-yl)-13-methyl-12,13,21,22,23,24-hexahydro-6H,11 H,20aH,26H-20,17-(metheno)imidazo[2,1-c]pyrido[2,1-1]pyrimido[6,1-h][1,4,7,9,10,13]benz oxapentaazacyclohexadecin-26-one

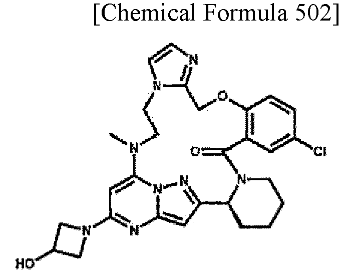

By using the same approach as in Step 3-1, the title compound (55 mg, colorless powder) was obtained from the compound (0.12 g) obtained in Step 333-1 and 3-hydroxyazetidine hydrochloride (0.25 g).
LCMS (ESI/APCI dual) m/z 563 [M+H]⁺, t_{R} = 0.224 min, mode H.

### Example 335: Synthesis of (20aS)-15-(3-aminoazetidin-1-yl)-2-chloro-13-methyl-12,13,21,22,23,24-hexahydro-6H,11H, 20aH,26H-20,17-(metheno)imidazo[2,1-c]pyrido[2,1-1]pyrimido[6,1-h][1,4,7,9,10,13]benzox apentaazacyclohexadecin-26-one

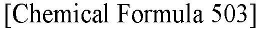

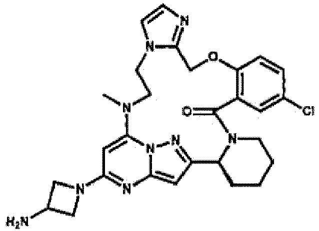

By using the same approaches as in Step 1-4 and Step 5-2, the title compound (90 mg, colorless amorphous) was obtained from the compound (0.15 g) obtained in Step 333-1 and 3-N-BOC-amino-azetidine (0.45 g).
LCMS (ESI/APCI dual) m/z 562 [M+H]⁺, t_{R} = 0.223 min, mode H.

### Example 336: Synthesis of (18aS)-13-(azetidin-1-yl)-2-fluoro-11-methyl-7-(trifluoromethyl)-6,7,8,9,10,11, 19,20,21,22-d ecahydro-18aH,24H-18, 15-(metheno)pyrido[2,1-1]pyrimido[6,1-h][1,4,7,9,10,13]benzoxapen taazacyclohexadecin-24-one

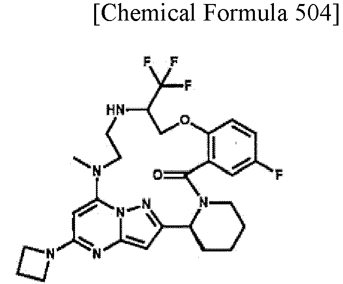

Step 336-1: By using the same approaches as in Step 1-1 to Step 1-3, (18aS)-13-chloro-2-fluoro-11-methyl-7-(trifluoromethyl)-6,7,8,9,10,11,19,20,21,22-decahydr o-18aH,24H-18,15-(metheno)pyrido[2,1-1]pyrimido[6,1-h][1,4,7,9,10,13]benzoxapentaazacy clohexadecin-24-one (0.14 g, colorless powder) was obtained from the compound (0.18 g) obtained in Reference Example 1-1 (3) and the compound (0.21 g) obtained in Reference Example 2-73.
LCMS (ESI) m/z 541 [M+H]⁺, t_{R} = 1.145 min, mode N.

Step 336-2: By using the same approach as in Step 1-4, the title compound (57 mg, colorless powder) was obtained from the compound (70 mg) obtained in Step 336-1 and azetidine (87 µL).
LCMS (ESI) m/z 562 [M+H]⁺, t_{R} = 0.675, 0.692 min, mode N.

### Example 337: Synthesis of (18aS)-13-(3-aminoazetidin-1-yl)-2-fluoro-11-methyl-7-(trifluoromethyl)-6,7,8,9,10,11,19,20 ,21,22-decahydro-18aH,24H-18, 15-(metheno)pyrido[2,1-1]pyrimido[6,1-h][1,4,7,9,10,13]ben zoxapentaazacyclohexadecin-24-one (Example 337a and Example 337b)

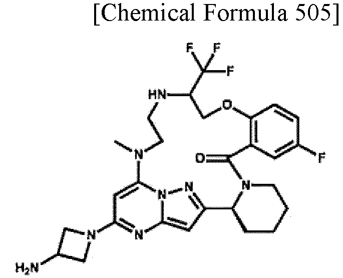

Step 337-1: By using the same approaches as in Step 1-4 and Step 5-2, (18aS)-13-(3-aminoazetidin-1-yl)-2-fluoro-11-methyl-7-(trifluoromethyl)-6,7,8,9,10,11,19,20 ,21,22-decahydro-18aH,24H-18,15-(metheno)pyrido[2,1-1]pyrimido[6,1-h][1,4,7,9,10,13]ben zoxapentaazacyclohexadecin-24-one (35 mg, colorless powder) was obtained from the compound (70 mg) obtained in Step 336-1 and 3-N-BOC-amino-azetidine (0.11 g).

Step 337-2: The compound (12 mg) obtained in Step 337-1 was preparatively purified by chiral column chromatography (preparative conditions: column: CHIRALPAK IC (Daicel trademark), column size: 20 mm × 250 mm, mobile phase: hexane/EtOH = 20/80 (v/v), flow rate: 10 mL/min) to afford Diastereomer A of the title compound (Example 337a) (6.0 mg, colorless powder) and Diastereomer B of the title compound (Example 337b) (5.3 mg, colorless powder).
Diastereomer A:
   ¹H NMR (400 MHz, CDCl₃) δ ppm 1.35 - 2.03 (m, 5H), 2.04 - 2.12 (m, 0.7H), 2.20 - 2.29 (m, 0.3H), 2.85 - 2.92 (m, 0.7H), 2.98 - 3.06 (m, 4H), 3.07 - 3.21 (m, 1.5H), 3.31 - 3.44 (m, 1.8H), 3.54 - 3.63 (m, 0.8H), 3.67 - 3.76 (m, 2.7H), 3.91 - 4.00 (m, 1H), 4.08 - 4.18 (m, 1.7H), 4.25 - 4.38 (m, 2.4H), 4.63 - 4.72 (m, 0.7H), 4.83 - 4.88 (m, 0.7H), 4.95 (s, 0.7H), 4.97 (s, 0.3H), 5.65 - 5.77 (m, 0.7H), 5.78 (s, 0.7H), 6.05 (s, 0.3H), 6.15 - 6.21 (m, 0.3H), 6.73 - 6.80 (m, 0.7H), 6.89 - 6.95 (m, 0.3H), 6.96 - 7.09 (m, 2H).
Diastereomer B:
   ¹H NMR (400 MHz, CDCl₃) δ ppm 1.05 - 2.29 (m, 6H), 2.95 - 4.41 (m, 17.8H), 4.57 - 4.67 (m, 0.1H), 4.89 (s, 0.1H), 4.97 (s, 1H), 5.14 - 5.27 (m, 0.1H), 5.78 (s, 0.1H), 6.05 (s, 0.9H), 6.20 (br s, 0.9H), 6.83 - 6.95 (m, 1H), 6.96 - 7.10 (m, 2H).

### Example 338 and Example 339: Synthesis of (18aS)-13-(3-aminoazetidin-1-yl)-2-chloro-11-methyl-7-(trifluoromethyl)-6,7,8,9,10,11,19,2 0,21,22-decahydro-18aH,24H-18,15-(metheno)pyrido[2,1-1]pyrimido[6,1-h][1,4,7,9,10,13]be nzoxapentaazacyclohexadecin-24-one

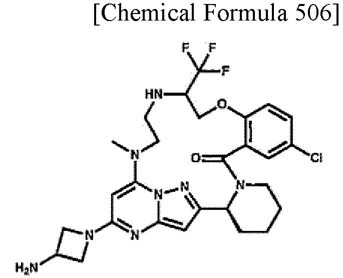

Step 338-1: By using the same approaches as in Step 1-1 to Step 1-3, (18aS)-2,13-dichloro-11-methyl-7-(trifluoromethyl)-6,7,8,9,10,11,19,20,21,22-decahydro-18 aH,24H-18,15-(metheno)pyrido[2,1-1]pyrimido[6,1-h][1,4,7,9,10,13]benzoxapentaazacycloh exadecin-24-one (0.51 g, colorless amorphous) was obtained from the compound (0.37 g) obtained in Reference Example 1-1 (3) and the compound (0.38 g) obtained in Reference Example 2-74.
LCMS (ESI) m/z 557 [M+H]⁺, t_{R} = 1.189, 1.209 min, mode N.

Step 338-2: By using the same approach as in Step 1-4, Diastereomer A (70 mg, colorless amorphous) and Diastereomer B (54 mg, colorless amorphous) of tert-butyl {1-[(18aS)-2-chloro-11-methyl-24-oxo-7-(trifluoromethyl)-6,7,8,9,10,11,19,20,21,22-decahy dro-18aH,24H-18,15-(metheno)pyrido[2,1-1]pyrimido[6,1-h][1,4,7,9,10,13]benzoxapentaaza cyclohexadecin-13-yl]azetidin-3-yl}carbamate were obtained from the compound (0.15 g) obtained in Step 338-1 and 3-N-BOC-amino-azetidine (0.46 g).
Diastereomer A: LCMS (ESI) m/z 693 [M+H]⁺, t_{R} = 0.826min, mode N.
Diastereomer B: LCMS (ESI) m/z 693 [M+H]⁺, t_{R} = 0.851min, mode N.

Step 338-3: By using the same approach as in Step 5-2, the title compound (59 mg, colorless amorphous) was obtained from Diastereomer A (70 mg) obtained in Step 338-2. LCMS (ESI) m/z 593 [M+H]⁺, t_{R} = 0.939 min, mode H.

Step 339: By using the same approach as in Step 5-2, the title compound (38 mg, colorless amorphous) was obtained from Diastereomer B (54 mg) obtained in Step 338-2. LCMS (ESI) m/z 593 [M+H]⁺, t_{R} = 0.576 min, mode N.

### Example 340: Synthesis of (18aS)-2-chloro-13-(3-hydroxyazetidin-1-yl)-11-methyl-7-(trifluoromethyl)-6,7,8,9,10,11,19, 20,21,22-decahydro-18aH,24H-18,15-(metheno)pyrido[2,1-1]pyrimido[6,1-h][1,4,7,9,10,13]b enzoxapentaazacyclohexadecin-24-one

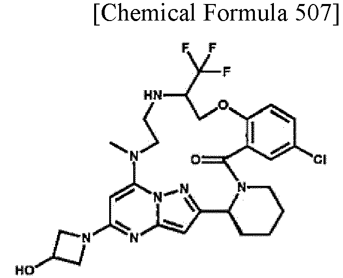

By using the same approach as in Step 3-1, the title compound (0.11 g, colorless amorphous) was obtained from the compound (0.16 g) obtained in Step 338-1 and 3-hydroxyazetidine hydrochloride (0.31 g).
LCMS (ESI) m/z 594 [M+H]⁺, t_{R} = 0.677, 0.695 min, mode N.

### Example 341: Synthesis of (18aS)-13-[(3S)-3-aminopyrrolidin-1-yl]-2-chloro-11-methyl-7-(trifluoromethyl)-6,7,8,9,10,1 1,19,20,21,22-decahydro-18aH,24H-18,15-(metheno)pyrido[2,1-1]pyrimido[6,1-h][1,4,7,9,10 ,13]benzoxapentaazacyclohexadecin-24-one

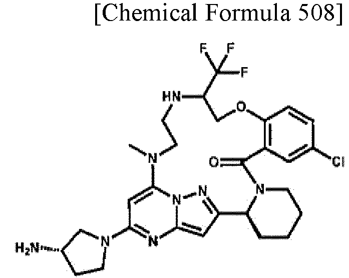

By using the same approach as in Step 1-4, the title compound (95 mg, colorless amorphous) was obtained from the compound (95 mg) obtained in Step 338-1.
LCMS (ESI) m/z 607 [M+H]⁺, t_{R} = 0.918, 0.957 min, mode H.

### Example 342 and Example 343: Synthesis of (18aS)-13-(3-aminoazetidin-1-yl)-2,11-dimethyl-7-(trifluoromethyl)-6,7,8,9,10,11,19,20,21,2 2-decahydro-18aH,24H-18,15-(metheno)pyrido[2,1-1]pyrimido[6,1-h][1,4,7,9,10,13]benzoxa pentaazacyclohexadecin-24-one

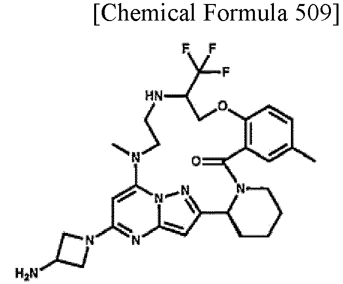

Step 342-1: By using the same approaches as in Step 1-1 to Step 1-3, (18aS)-13-chloro-2,11-dimethyl-7-(trifluoromethyl)-6,7,8,9,10,11,19,20,21,22-decahydro-18 aH,24H-18,15-(metheno)pyrido[2,1-1]pyrimido[6,1-h][1,4,7,9,10,13]benzoxapentaazacycloh exadecin-24-one (0.89 g, colorless amorphous) was obtained from the compound (0.74 g) obtained in Reference Example 1-1 (3) and the compound (0.71 g) obtained in Reference

### Example 2-75.

### LCMS (ESI) m/z 537 [M+H]⁺, t_{R} = 1.184 min, mode N.

Step 342-2: By using the same approach as in Step 1-4, Diastereomer A (58 mg, colorless amorphous) and Diastereomer B (65 mg, colorless amorphous) of tert-butyl {1-[(18aS)-2,11-dimethyl-24-oxo-7-(trifluoromethyl)-6,7,8,9,10,11,19,20,21,22-decahydro-1 8aH,24H-18,15-(metheno)pyrido[2,1-1]pyrimido[6,1-h][1,4,7,9,10,13]benzoxapentaazacyclo hexadecin-13-yl]azetidin-3-yl}carbamate were obtained from the compound (0.15 g) obtained in Step 342-1 and 3-N-BOC-amino-azetidine (0.48 g).
Diastereomer A: LCMS (ESI) m/z 673 [M+H]⁺, t_{R} = 0.834 min, mode N.
Diastereomer B: LCMS (ESI) m/z 673 [M+H]⁺, t_{R} = 0.815 min, mode N.

Step 342-3: By using the same approach as in Step 5-2, the title compound (49 mg, colorless amorphous) was obtained from Diastereomer A (58 mg) obtained in Step 342-2. LCMS (ESI) m/z 573 [M+H]⁺, t_{R} = 0.919 min, mode H.

Step 343: By using the same approach as in Step 5-2, the title compound (43 mg, colorless amorphous) was obtained from Diastereomer B (65 mg) obtained in Step 342-2. LCMS (ESI) m/z 573 [M+H]⁺, t_{R} = 0.952 min, mode H.

### Example 344: Synthesis of (18aS)-13-(3-hydroxyazetidin-1-yl)-2,11-dimethyl-7-(trifluoromethyl)-6,7,8,9,10,11,19,20,21 ,22-decahydro-18aH,24H-18,15-(metheno)pyrido[2,1-1]pyrimido[6,1-h][1,4,7,9,10,13]benzo xapentaazacyclohexadecin-24-one

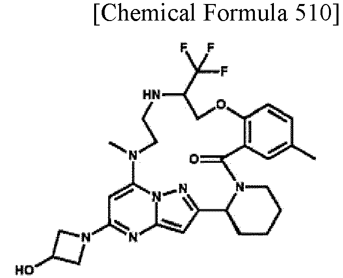

By using the same approach as in Step 3-1, the title compound (0.11 g, colorless amorphous) was obtained from the compound (0.16 g) obtained in Step 342-1 and 3-hydroxyazetidine hydrochloride (0.33 g).
LCMS (ESI) m/z 574 [M+H]⁺, t_{R} = 0.663 min, mode N.

### Example 345: Synthesis of (18aS)-13-[(3S)-3-aminopyrrolidin-1-yl]-2,11-dimethyl-7-(trifluoromethyl)-6,7,8,9,10,11,19, 20,21,22-decahydro-18aH,24H-18,15-(metheno)pyrido[2,1-1]pyrimido[6,1-h][1,4,7,9,10,13]b enzoxapentaazacyclohexadecin-24-one

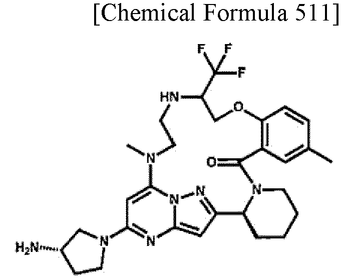

By using the same approach as in Step 1-4, the title compound (95 mg, colorless amorphous) was obtained from the compound (0.10 g) obtained in Step 342-1.
LCMS (ESI) m/z 587 [M+H]⁺, t_{R} = 0.899, 0.932 min, mode H.

### Example 346 and Example 347: Synthesis of (18aS)-13-(azetidin-1-yl)-2,11-dimethyl-9-(trifluoromethyl)-6,7,8,9,10,11,19,20,21,22-decah ydro-18aH,24H-18,15-(metheno)pyrido[2,1-1]pyrimido[6,1-h][1,4,7,9,10,13]benzoxapentaaz acyclohexadecin-24-one (Example 346a and Example 346b)

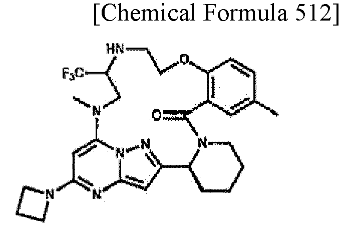

Step 346-1: By using the same approach as in Step 1-1, tert-butyl 2-[2-[[1-[[[5-chloro-2-[(2S)-2-piperidyl]pyrazolo[1,5-a]pyrimidin-7-yl]-methyl-amino]methy l]-2,2,2-trifluoro-ethyl]amino]ethoxy]-5-methyl-benzoate (0.37 g, orange oily matter) was obtained from the compound (0.43 g) obtained in Reference Example 1-1 (3) and the compound (0.43 g) obtained in Reference Example 2-79.
LCMS (ESI) m/z 611 [M+H]⁺, t_{R} = 0.895 min, mode N.

Step 346-2: By using the same approaches as in Step 1-4, Step 15-2, and Step 1-3, Diastereomer A of the title compound (Example 346a) (3.7 mg, colorless powder) and Diastereomer B of the title compound (Example 346b) (3.4 mg, colorless powder) were obtained from the compound (50 mg) obtained in Step 346-1 and azetidine (0.11 mL).
Diastereomer A: LCMS (ESI) m/z 558 [M+H]⁺, t_{R} = 0.832 min, mode N.
   Diastereomer B:
   ¹H NMR (600 MHz, CDCl₃) δ ppm 1.63 - 2.11 (m, 5H), 2.16 - 2.49 (m, 6H), 2.81 - 3.17 (m, 5.67H), 3.19 - 3.26 (m, 0.33H), 3.30 - 3.69 (m, 3H), 3.84 - 4.23 (m, 6H), 4.52 - 4.69 (m, 1H), 4.95 - 5.17 (m, 2H), 5.72 (s, 0.33H), 6.13 - 6.29 (m, 1.33H), 6.66 - 7.18 (m, 3.1H), 7.63 (br s, 0.33H).
   LCMS (ESI) m/z 558 [M+H]⁺, t_{R} = 0.756 min, mode N.

### Example 347: Synthesis of (18aS)-13-(3-aminoazetidin-1-yl)-2,11-dimethyl-9-(trifluoromethyl)-6,7,8,9,10,11,19,20,21,2 2-decahydro-18aH,24H-18,15-(metheno)pyrido[2,1-l]pyrimido[6,1-h][1,4,7,9,10,13]benzoxa pentaazacyclohexadecin-24-one

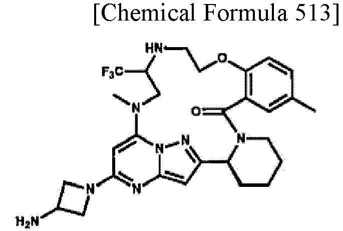

By using the same approaches as in Step 1-4, Step 15-2, Step 1-3, and Step 4-2, the title compound (23 mg, colorless amorphous) was obtained from the compound (0.13 g) obtained in Step 346-1 and benzyl N-(azetidin-3-yl)carbamate (0.17 g).
LCMS (ESI) m/z 573 [M+H]⁺, t_{R} = 0.567, 0.686 min, mode N.

### Example 348: Synthesis of (18aS)-13-(3-hydroxyazetidin-1-yl)-2,11-dimethyl-9-(trifluoromethyl)-6,7,8,9,10,11,19,20,21 ,22-decahydro-18aH,24H-18,15-(metheno)pyrido[2,1-1]pyrimido[6,1-h][1,4,7,9,10,13]benzo xapentaazacyclohexadecin-24-one

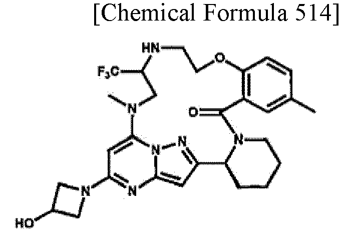

By using the same approaches as in Step 3-1, Step 15-2, and Step 1-3, the title compound (35 mg, light yellow amorphous) was obtained from the compound (0.12 g) obtained in Step 346-1 and 3-hydroxyazetidine hydrochloride (0.22 g).
LCMS (ESI) m/z 574 [M+H]⁺, t_{R} = 0.570, 0.696 min, mode N.

### Example 349 and Example 350: Synthesis of (18aS)-13-(3-aminocyclobutyl)-2,11-dimethyl-7-(trifluoromethyl)-6,7,8,9,10,11,19,20,21,22-decahydro-18aH,24H-18,15-(metheno)pyrido[2,1-l]pynmido[6,1-h][1,4,7,9,10,13]benzoxape ntaazacyclohexadecin-24-one

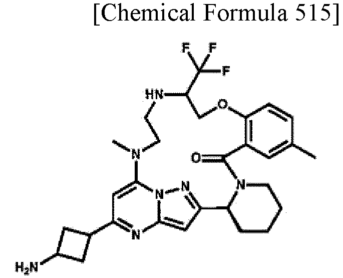

Step 349-1: By using the same approaches as in Step 1-1 to Step 1-3, Diastereomer A (0.13 g, colorless powder) and Diastereomer B (0.13 g, colorless powder) of (18aS)-13-chloro-2,11-dimethyl-7-(trifluoromethyl)-6,7,8,9,10,11,19,20,21,22-decahydro-18 aH,24H-18,15-(metheno)pyrido[2,1-l]pyrimido[6,1-h][1,4,7,9,10,13]benzoxapentaazacycloh exadecin-24-one were obtained from the compound (0.34 g) obtained in Reference Example 1-1 (3) and the compound (0.41 g) obtained in Reference Example 2-75.
Diastereomer A: LCMS (ESI) m/z 537 [M+H]⁺, t_{R} = 1.184 min, mode N.
Diastereomer B: LCMS (ESI) m/z 537 [M+H]⁺, t_{R} = 1.197 min, mode N.

Step 349-2: By using the same approaches as in Step 21-1 and Step 5-2, the title compound (29 mg, colorless powder) was obtained from Diastereomer A (0.13 g) obtained in Step 349-1, using tert-butyl N-(3-iodocyclobutyl)carbamate instead of 1-BOC-3-iodoazetidine.
LCMS (ESI) m/z 572 [M+H]⁺, t_{R} = 0.504 min, mode N.

Step 350: By using the same approaches as in Step 21-1 and Step 5-2, the title compound (9.2 mg, colorless powder) was obtained from Diastereomer B (0.13 g) obtained in Step 349-1, using tert-butyl N-(3-iodocyclobutyl)carbamate instead of 1-BOC-3-iodoazetidine.
LCMS (ESI) m/z 572 [M+H]⁺, t_{R} = 0.524 min, mode N.

### Example 351: Synthesis of (3'S)-15'-[(3S)-3-aminopyrrolidin-1-yl]-3'-ethyl-4',13'-dimethyl-3'H,4'H,5'H,7'H,8'H,9'H,10' H,11'H,12'H,13'H-spiro[cyclohexane-1,6'-[1,4,8,13,16,18]hexaaza[2,17](metheno)pyrimido[ 1,6-b][1,2,4,9,13]pentaazacyclopentadecine]-5',9'-dione

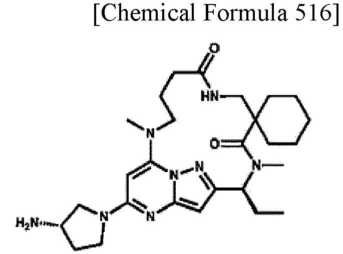

By using the same approaches as in Step 1-1 to Step 1-4, the title compound (46 mg, colorless powder) was obtained from the compound (0.41 g) obtained in Reference Example 1-15 and the compound (0.36 g) obtained in Reference Example 2-118.
LCMS (ESI) m/z 511 [M+H]⁺, t_{R} = 0.741 min, mode H.

### Example 352: Synthesis of (18aS)-3-[(3S)-3-aminopyrrolidin-1-yl]-5-methyl-11-phenyl-7,8,11,12,16,17,18,18a-octahydr o-5H,15H-19,1-(metheno)-9λ⁶-pyrido[1,2-f]pyrimido[1,6-j][1,2,6,9,10,12]thiapentaazacyclo pentadecine-9,9,13(6H,10H)-trione

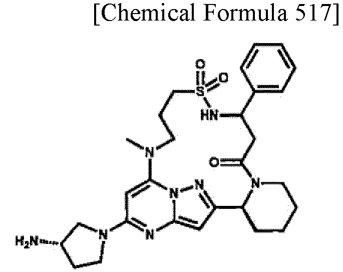

By using the same approaches as in Step 1-1 to Step 1-4, the title compound (29 mg, colorless powder) was obtained from the compound (63 mg) obtained in Reference Example 1-1 (3) and the compound (45 mg) obtained in Reference Example 2-111.
LCMS (ESI) m/z 567 [M+H]⁺, t_{R} = 0.775 min, mode H.

### Example 353: Synthesis of (18aS)-3-(azetidin-1-yl)-12-benzyl-5-methyl-6,7,8,9,11,12,16,17,18,18a-decahydro-5H,15H-19,1-(metheno)-10λ⁶-pyrido[1,2-e]pyrimido[1,6-i][1,2,5,8,9,11]thiapentaazacyclopentadecin e-10,10,13-trione

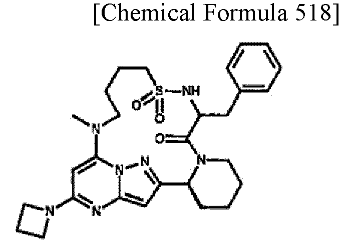

Step 353-1: By using the same approaches as in Step 1-1 to Step 1-3, (18aS)-12-benzyl-3-chloro-5-methyl-6,7,8,9,11,12,16,17,18,18a-decahydro-5H,15H-19,1-(m etheno)-10λ⁶-pyrido[1,2-e]pyrimido[1,6-i][1,2,5,8,9,11]thiapentaazacyclopentadecine-10,10, 13-trione (0.18 g, colorless powder) was obtained from the compound (0.20 g) obtained in Reference Example 1-1 (3) and the compound (0.17 g) obtained in Reference Example 2-102.
LCMS (ESI) m/z 531 [M+H]⁺, t_{R} = 1.091, 1.125 min, mode N.

Step 353-2: By using the same approach as in Step 1-4, the title compound (75 mg, colorless powder) was obtained from the compound (87 mg) obtained in Step 353-1 and azetidine (0.11 mL).
LCMS (ESI) m/z 552 [M+H]⁺, t_{R} = 1.067, 1.122 min, mode H.

### Example 354: Synthesis of (18aS)-3-[(3S)-3-aminopyrrolidin-1-yl]-12-benzyl-5-methyl-6,7,8,9,11,12,16,17,18,18a-deca hydro-5H,15H-19,1-(metheno)-10λ⁶-pyrido[1,2-e]pyrimido[1,6-i][1,2,5,8,9,11]thiapentaazac yclopentadecine-10,10,13-trione

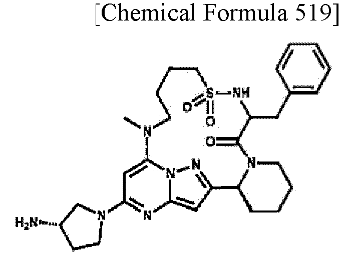

By using the same approach as in Step 1-4, the title compound (84 mg, colorless powder) was obtained from the compound (87 mg) obtained in Step 353-1.
LCMS (ESI) m/z 581 [M+H]⁺, t_{R} = 0.811, 0.860 min, mode H.

### Example 355: Synthesis of (18aS)-3-(azetidin-1-yl)-5,12-dimethyl-12-phenyl-6,7,8,9,11,12,16,17,18,18a-decahydro-5H, 15H-19,1-(metheno)-10λ⁶-pyrido[1,2-e]pyrimido[1,6-i][1,2,5,8,9,11]thiapentaazacyclopentad ecine-10,10,13-trione

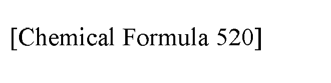

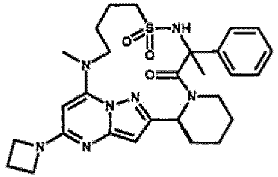

Step 355-1: By using the same approaches as in Step 1-1 to Step 1-2, 2-[4-[[5-chloro-2-[(2S)-2-piperidyl]pyrazolo[1,5-a]pyrimidin-7-yl]-methyl-amino]butylsulfo nylamino]-2-phenyl-propanoic acid (0.27 g, colorless powder) was obtained from the compound (0.25 g) obtained in Reference Example 1-1 (3) and the compound (0.21 g) obtained in Reference Example 2-98.
LCMS (ESI) m/z 549 [M+H]⁺, t_{R} = 0.616 min, mode N.

Step 355-2: To a solution of the compound (0.18 g) obtained in Step 355-1 in DMF (32 mL), Et₃N (0.22 mL), HATU (0.18 g), and DMAP (catalytic amount) were added, and the mixture was stirred at 70°C for 2.5 hours. To the reaction solution, EtOAc and a Sat. NaHCO₃ aq. were added, and the separating operation was carried out. After washing with Brine, the aqueous layer was separated using Phase Separator, and then the organic layer was concentrated under reduced pressure. The obtained residue was purified by NH type silica gel column chromatography (mobile phase: hexane/EtOAc = 40/60 to 0/100; v/v) to afford (18aS)-3-chloro-5,12-dimethyl-12-phenyl-6,7,8,9,11,12,16,17,18,18a-decahydro-5H,15H-19, 1-(metheno)-10λ⁶-pyrido[1,2-e]pyrimido[1,6-i][1,2,5,8,9,11]thiapentaazacyclopentadecine-1 0,10,13-trione (0.14 g, light yellow amorphous).
LCMS (ESI) m/z 531 [M+H]⁺, t_{R} = 1.099, 1.141 min, mode N.

Step 355-3: By using the same approach as in Step 1-4, the title compound (51 mg, colorless powder) was obtained from the compound (66 mg) obtained in Step 355-2 and azetidine (84 µL).
¹H NMR (400 MHz, CDCl₃) δ ppm 0.33 - 0.49 (m, 0.5H), 0.80 - 0.92 (m, 1H), 1.32 - 1.71 (m, 3.5H), 1.73 - 1.88 (m, 1.5H), 1.89 - 2.00 (m, 4H), 2.01 - 2.27 (m, 2.5H), 2.32 - 2.45 (m, 2H), 2.70 - 2.81 (m, 1H), 2.86 (s, 1.5H), 2.89 - 2.99 (m, 0.5H), 2.99 - 3.10 (m, 2H), 3.19 - 3.33 (m, 0.5H), 3.34 - 3.43 (m, 0.5H), 3.46 - 3.55 (m, 0.5H), 3.59 - 3.77 (m, 1H), 4.03 - 4.17 (m, 4H), 4.55 - 4.64 (m, 0.5H), 4.65 - 4.77 (m, 0.5H), 4.80 (s, 0.5H), 4.85 - 4.93 (m, 1H), 5.03 - 5.18 (m, 1H), 5.76 (s, 0.5H), 5.84 (s, 0.5H), 6.00 (s, 0.5H), 6.18 - 6.23 (m, 0.5H), 7.27 - 7.32 (m, 1H), 7.36 - 7.44 (m, 2H), 7.47 - 7.60 (m, 2H).
LCMS (ESI) m/z 552 [M+H]⁺, t_{R} = 1.077, 1.124 min, mode H.

### Example 356: Synthesis of (18aS)-3-[(3S)-3-aminopyrrolidin-1-yl]-5,12-dimethyl-12-phenyl-6,7,8,9,11,12,16,17,18,18a-decahydro-5H,15H-19,1-(metheno)-10λ⁶-pyrido[1,2-e]pyrimido[1,6-i] [1,2,5,8,9,11]thiapenta azacyclopentadecine-10,10,13-trione (Example 356a and Example 356b)

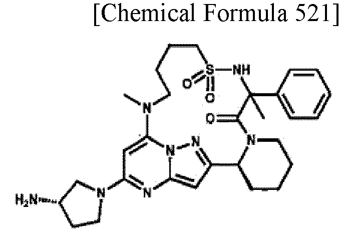

Step 356-1: By using the same approach as in Step 1-4, (18aS)-3-[(3S)-3-aminopyrrolidin-1-yl]-5,12-dimethyl-12-phenyl-6,7,8,9,11,12,16,17,18,18a-decahydro-5H,15H-19,1-(metheno)-10λ⁶-pyrido[1,2-e]pyrimido[1,6-i] [1,2,5,8,9,11]thiapenta azacyclopentadecine-10,10,13-trione (54 mg, colorless powder) was obtained from the compound (66 mg) obtained in Step 355-2.

Step 356-2: The compound (40 mg) obtained in Step 356-1 was preparatively purified by chiral column chromatography (preparative conditions: column: CHIRALPAK AD-H (Daicel trademark), column size: 20 mm × 250 mm, mobile phase: hexane/EtOH = 20/80 (v/v), flow rate: 10 mL/min) to afford Diastereomer A of the title compound (Example 356a) (19 mg, colorless powder) and Diastereomer B of the title compound (Example 356b) (15 mg, colorless powder).
Diastereomer A: LCMS (ESI) m/z 581 [M+H]⁺, t_{R} = 0.877 min, mode H.
Diastereomer B: LCMS (ESI) m/z 581 [M+H]⁺, t_{R} = 0.814 min, mode H.

### Example 357: Synthesis of (18a'S)-3'-[(3S)-3-aminopyrrolidin-1-yl]-5'-methyl-2H,3H,5'H,6'H,7'H,8'H,9'H,10'H,11'H,13' H,15'H,16'H,17'H,18'H,18a'H-spiro[indene-1,12'-[19,1](metheno)[10λ⁶]pyrido[1,2-e]pyrimid o[1,6-i][1,2,5,8,9,11]thiapentaazacyclopentadecine]-10',10',13'-trione

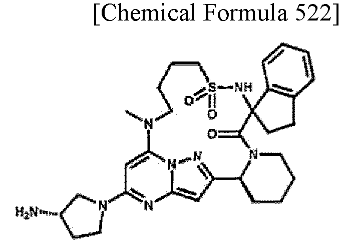

Step 357-1: By using the same approaches as in Step 1-1, Step 1-2, and Step 355-2, (18a'S)-3'-chloro-5'-methyl-2H,3H,5'H,6'H,7'H,8'H,9'H,10'H,11'H,13'H,15'H,16'H,17'H,18'H, 18a'H-spiro[indene-1,12'-[19,1](metheno)[10λ⁶]pyrido[1,2-e]pyrimido[1,6-i][1,2,5,8,9,11]thi apentaazacyclopentadecine]-10',10',13'-trione (69 mg, light yellow amorphous) was obtained from the compound (0.20 g) obtained in Reference Example 1-1 (3) and the compound (0.18 g) obtained in Reference Example 2-101.
LCMS (ESI) m/z 543 [M+H]⁺, t_{R} = 1.066, 1.115 min, mode N.

Step 357-2: By using the same approach as in Step 1-4, the title compound (8.1 mg, colorless powder) was obtained from the compound (25 mg) obtained in Step 357-1. LCMS (ESI) m/z 593 [M+H]⁺, t_{R} = 0.758, 0.835 min, mode H.

### Example 358: Synthesis of (18a'S)-3'-(azetidin-1-yl)-5'-methyl-2H,3H,5'H,6'H,7'H,8'H,9'H,10'H,11'H,13'H,15'H,16'H,17 'H,18'H,18a'H-spiro[indene-1,12'-[19,1](metheno)[10λ⁶]pyrido[1,2-e]pyrimido[1,6-i][1,2,5,8, 9,11]thiapentaazacyclopentadecine]-10',10',13'-trione

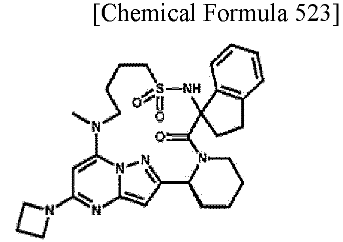

By using the same approach as in Step 1-4, the title compound (11 mg, colorless powder) was obtained from the compound (25 mg) obtained in Step 357-1 and azetidine (31 µL).
LCMS (ESI) m/z 564 [M+H]⁺, t_{R} = 1.027, 1.088 min, mode H.

### Example 359: Synthesis of (18a'S)-3'-(3-aminoazetidin-1-yl)-5'-methyl-2H,3H,5'H,6'H,7'H,8'H,9'H,10'H,11'H,13'H,15'H ,16'H,17'H,18'H,18a'H-spiro[indene-1,12'-[19,1](metheno)[10λ⁶]pyrido[1,2-e]pyrimido[1,6-i] [1,2,5,8,9,11]thiapentaazacyclopentadecine]-10',10',13'-trione

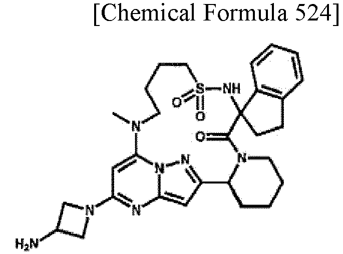

By using the same approaches as in Step 3-1 and Step 5-2, the title compound

(9.3 mg, colorless powder) was obtained from the compound (23 mg) obtained in Step 357-1 and 3-N-BOC-amino-azetidine mesylate salt (0.11 g).
LCMS (ESI) m/z 579 [M+H]⁺, t_{R} = 0.755, 0.835 min, mode H.

### Example 360: Synthesis of (24aS)-19-(3-aminoazetidin-1-yl)-17-methyl-1,3,4,14,15,16,17,24a-octahydro-2H,13H-24,21 -(metheno)pyrido[2,1-k]pyrimido[6,1-g][1,6,8,9,12]benzoxatetraazacyclohexadecin-6(7H)-o ne

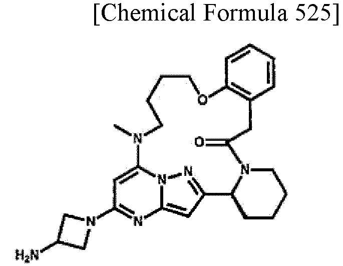

By using the same approaches as in Step 1-1 to Step 1-4 and Step 4-2, the title compound (30 mg, colorless powder) was obtained from the compound (0.18 g) obtained in Reference Example 1-1 (3) and the compound (0.19 g) obtained in Reference Example 2-110, and benzyl N-(azetidin-3-yl)carbamate (0.28 g).
LCMS (ESI) m/z 490 [M+H]⁺, t_{R} = 0.475 min, mode N.

### Example 361: Synthesis of (18aS)-3-(3-aminoazetidin-1-yl)-5,12-dimethyl-12-phenyl-6,7,8,9,11,12,16,17,18,18a-decahy dro-5H,13H,15H-19,1-(metheno)pyrido[1,2-e]pyrimido[1,6-i][1,5,8,9,11]oxatetraazacyclope ntadecin-13-one

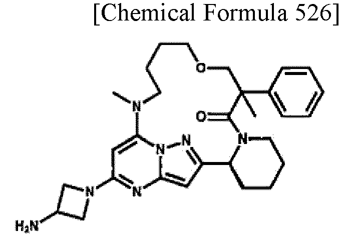

Step 361-1: By using the same approaches as in Step 1-1, Step 1-2, and Step 355-2, (18aS)-3-chloro-5,12-dimethyl-12-phenyl-6,7,8,9,11,12,16,17,18,18a-decahydro-5H,13H,15 H-19,1-(metheno)pyrido[1,2-e]pyrimido[1,6-i][1,5,8,9,11]oxatetraazacyclopentadecin-13-on e (65 mg, yellow oily matter) was obtained from the compound (0.27 g) obtained in Reference Example 1-1 (3) and the compound (0.25 g) obtained in Reference Example 2-103.
LCMS (ESI) m/z 482 [M+H]⁺, t_{R} = 1.010 min, mode L.

Step 361-2: By using the same approaches as in Step 1-4 and Step 5-2, the title compound (23 mg, colorless powder) was obtained from the compound (32 mg) obtained in Step 361-1 and 3-N-BOC-amino-azetidine (57 mg).
LCMS (ESI) m/z 518 [M+H]⁺, t_{R} = 0.602 min, mode N.

### Example 362: Synthesis of (18aS)-3-[(3S)-3-aminopyrrolidin-1-yl]-5,12-dimethyl-12-phenyl-6,7,8,9,11,12,16,17,18,18a-decahydro-5H,13H,15H-19,1-(metheno)pyrido[1,2-e]pyrimido[1,6-i][1,5,8,9,11]oxatetraazac yclopentadecin-13-one

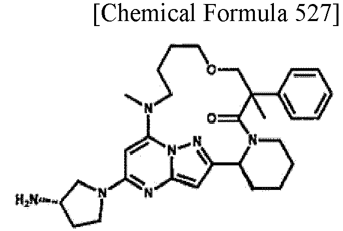

By using the same approach as in Step 1-4, the title compound (24 mg, light pink amorphous) was obtained from the compound (30 mg) obtained in Step 361-1.
LCMS (ESI) m/z 532 [M+H]⁺, t_{R} = 0.539, 0.575 min, mode N.

### Example 363: Synthesis of (19aS)-3-(3-aminoazetidin-1-yl)-5,13-dimethyl-13-phenyl-5,6,7,8,17,18,19,19a-octahydro-12 H,16H-20,1-(metheno)pyrido[2,1-l]pyrimido[6,1-h][1,4,7,9,10,13]oxapentaazacyclohexadeci ne-9,14(10H, 13H)-dione

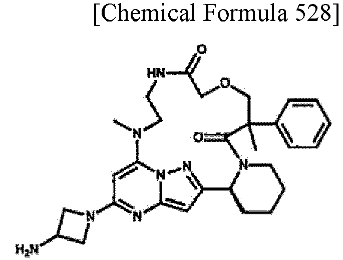

By using the same approaches as in Step 1-1, Step 1-2, Step 355-2, Step 1-4, and Step 5-2, the title compound (22 mg, colorless powder) was obtained from the compound (0.21 g) obtained in Reference Example 1-1 (3), the compound (0.27 g) obtained in Reference Example 2-106, and 3-N-BOC-amino-azetidine (55 mg).
LCMS (ESI) m/z 547 [M+H]⁺, t_{R} = 0.834, 0.882 min, mode H.

### Example 364: Synthesis of (19aS)-3-(azetidin-1-yl)-13-(4-fluorophenyl)-5,13-dimethyl-5,6,7,8,10,11,12,13,17,18,19,19a -dodecahydro-16H-20,1-(metheno)pyrido[1,2-n]pyrimido[1,6-b][1,2,4,7,10,14]hexaazacyclo hexadecine-9,14-dione (Example 364a and Example 364b)

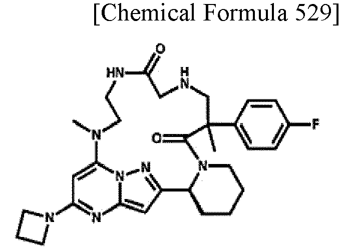

By using the same approaches as in Step 1-1, Step 1-2, Step 355-2, Step 1-4, and Step 4-2, Diastereomer A of the title compound (Example 364a) (3.1 mg, colorless powder) and Diastereomer B of the title compound (Example 364b) (3.5 mg, colorless powder) were obtained from the compound (0.24 g) obtained in Reference Example 1-1 (3), the compound (0.17 g) obtained in Reference Example 2-109, and azetidine (43 µL).
Diastereomer A: LCMS (ESI) m/z 549 [M+H]⁺, t_{R} = 0.490 min, mode N.
Diastereomer B: LCMS (ESI) m/z 549 [M+H]⁺, t_{R} = 0.589 min, mode N.

### Example 365: Synthesis of (19aS)-3-(azetidin-1-yl)-13-(4-fluorophenyl)-5,11,13-trimethyl-5,6,7,8,10,11,12,13,17,18,19, 19a-dodecahydro-16H-20,1-(metheno)pyrido[1,2-n]pyrimido[1,6-b][1,2,4,7,10,14]hexaazacy clohexadecine-9,14-dione

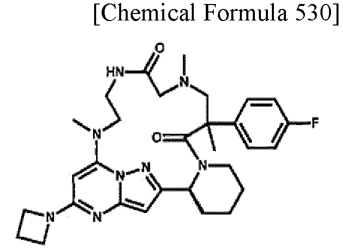

By using the same approaches as in Step 1-1, Step 1-2, Step 355-2, and Step 1-4, the title compound (5.2 mg, colorless powder) was obtained from the compound (91 mg) obtained in Reference Example 1-1 (3), the compound (0.10 g) obtained in Reference Example 2-108, and azetidine (39 µL).
LCMS (ESI) m/z 563 [M+H]⁺, t_{R} = 0.623 min, mode N.

### Example 366: Synthesis of (18aS)-3-[(3S)-3-aminopyrrolidin-1-yl]-12-(4-chlorophenyl)-5,12-dimethyl-6,7,8,9,11,12,16, 17,18,18a-decahydro-5H,15H-19,1-(metheno)-10λ⁶-pyrido[1,2-e]pyrimido[1,6-i][1,2,5,8,9,1 1]thiapentaazacyclopentadecine-10,10,13-trione

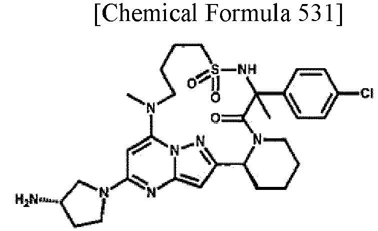

Step 366-1: By using the same approaches as in Step 1-1, Step 1-2, and Step 355-2, (18aS)-3-chloro-12-(4-chlorophenyl)-5,12-dimethyl-6,7,8,9,11,12,16,17,18,18a-decahydro-5 H,15H-19,1-(metheno)-10λ⁶-pyrido[1,2-e]pyrimido[1,6-i][1,2,5,8,9,11]thiapentaazacyclopen tadecine-10,10,13-trione (0.16 g, yellow oily matter) was obtained from the compound (0.39 g) obtained in Reference Example 1-1 (3) and the compound (0.40 g) obtained in Reference Example 2-100.
LCMS (ESI) m/z 565 [M+H]⁺, t_{R} = 1.171, 1.218 min, mode N.

Step 366-2: By using the same approach as in Step 1-4, the title compound (19 mg, light pink amorphous) was obtained from the compound (39 mg) obtained in Step 366-1. LCMS (ESI) m/z 615 [M+H]⁺, t_{R} = 0.522, 0.584 min, mode N.

### Example 367: Synthesis of (18aS)-3-(3-aminoazetidin-1-yl)-12-(4-chlorophenyl)-5,12-dimethyl-6,7,8,9,11,12,16,17,18,1 8a-decahydro-5H,15H-19,1-(metheno)-10λ⁶-pyrido[1,2-e]pyrimido[1,6-i][1,2,5,8,9,11]thiape ntaazacyclopentadecine-10,10,13-trione

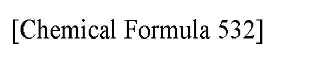

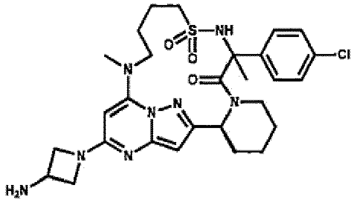

By using the same approaches as in Step 1-4 and Step 5-2, the title compound (17 mg, light yellow powder) was obtained from the compound (30 mg) obtained in Step 366-1 and 3-N-BOC-amino-azetidine (46 mg).
LCMS (ESI) m/z 601 [M+H]⁺, t_{R} = 0.539, 0.604 min, mode N.

### Example 368: Synthesis of (18aS)-3-[(3S)-3-aminopyrrolidin-1-yl]-12-(4-chlorophenyl)-5,11,12-trimethyl-6,7,8,9,11,12, 16,17,18,18a-decahydro-5H,15H-19,1-(metheno)-10λ⁶-pyrido[1,2-e]pyrimido[1,6-i][1,2,5,8, 9,11]thiapentaazacyclopentadecine-10,10,13-trione

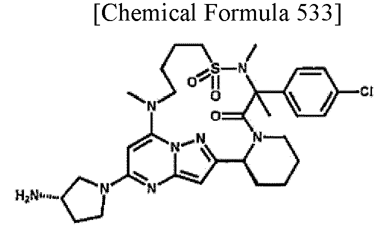

Step 368-1: To a solution of the compound (90 mg) obtained in Step 366-1 in DMF (1.6 mL), K₂CO₃ (0.13 g) and MeI (40 µL) were added, and the mixture was stirred at room temperature for 2.5 hours. After adding water to the reaction liquid and stirring the mixture, it was extracted with EtOAc. The organic layer was dried over MgSO₄ and concentrated under reduced pressure, and the resulting residue was purified by OH type silica gel chromatography (mobile phase: hexane/EtOAc = 90/10 to 0/100; v/v) to afford (18aS)-3-chloro-12-(4-chlorophenyl)-5,11,12-trimethyl-6,7,8,9,11,12,16,17,18,18a-decahydr o-5H,15H-19,1-(metheno)-10λ⁶-pyrido[1,2-e]pyrimido[1,6-i][1,2,5,8,9,11]thiapentaazacyclo pentadecine-10,10,13-trione (59 mg, colorless powder).
LCMS (ESI) m/z 579 [M+H]⁺, t_{R} = 0.923, 0.970 min, mode L.

Step 368-2: By using the same approach as in Step 1-4, the title compound (33 mg, light pink amorphous) was obtained from the compound (38 mg) obtained in Step 368-1. LCMS (ESI) m/z 629 [M+H]⁺, t_{R} = 0.578 min, mode N.

### Example 369: Synthesis of (18aS)-3-(3-aminoazetidin-1-yl)-12-(4-chlorophenyl)-5,11,12-trimethyl-6,7,8,9,11,12,16,17,1 8,18a-decahydro-5H,15H-19,1-(metheno)-10λ⁶-pyrido[1,2-e]pyrimido[1,6-i] [1,2,5,8,9,11]thi apentaazacyclopentadecine-10,10,13-trione

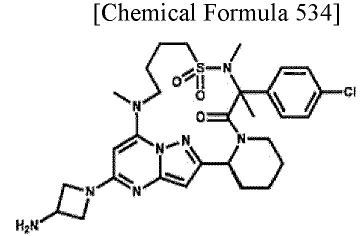

By using the same approaches as in Step 1-4 and Step 5-2, the title compound (15 mg, beige amorphous) was obtained from the compound (28 mg) obtained in Step 368-1 and 3-N-BOC-amino-azetidine (41 mg).
LCMS (ESI) m/z 615 [M+H]⁺, t_{R} = 0.556, 0.593 min, mode N.

### Example 370: Synthesis of (18aS)-3-[(3S)-3-aminopyrrolidin-1-yl]-12-(4-fluorophenyl)-5,12-dimethyl-7,8,11,12,16,17,1 8,18a-octahydro-5H,15H-19,1-(metheno)pyrido[1,2-m]pyrimido[1,6-b][1,2,4,9,13]pentaazac yclopentadecine-9,13(6H, 10H)-dione

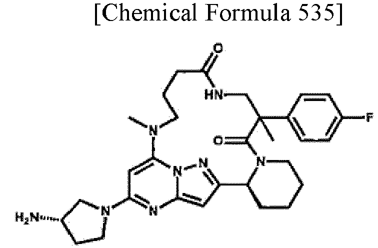

Step 370-1: By using the same approaches as in Step 1-1, Step 1-2, and Step 355-2, (18aS)-3-chloro-12-(4-fluorophenyl)-5,12-dimethyl-7,8,11,12,16,17,18,18a-octahydro-5H,15 H-19,1-(metheno)pyrido[1,2-m]pyrimido[1,6-b][1,2,4,9,13]pentaazacyclopentadecine-9,13(6 H,10H)-dione (89 mg, yellow oily matter) was obtained from the compound (0.20 g) obtained in Reference Example 1-1 (3) and the compound (0.17 g) obtained in Reference Example 2-107.
LCMS (ESI) m/z 513 [M+H]⁺, t_{R} = 0.718, 1.047 min, mode N.

Step 370-2: By using the same approach as in Step 1-4, the title compound (5.6 mg, light pink amorphous) was obtained from the compound (22 mg) obtained in Step 370-1. LCMS (ESI) m/z 563 [M+H]⁺, t_{R} = 0.838 min, mode N.

### Example 371: Synthesis of (18aS)-3-(3-aminoazetidin-1-yl)-12-(4-fluorophenyl)-5,12-dimethyl-7,8,11,12,16,17,18,18a-octahydro-5H,15H-19,1-(metheno)pyrido[1,2-m]pyrimido[1,6-b][1,2,4,9,13]pentaazacyclope ntadecine-9,13 (6H,10H)-dione

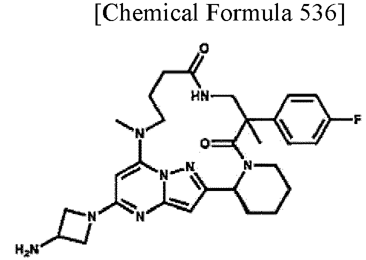

By using the same approaches as in Step 1-4 and Step 5-2, the title compound (19 mg, colorless amorphous) was obtained from the compound (55 mg) obtained in Step 372-1 and 3-N-BOC-amino-azetidine (93 mg).
LCMS (ESI) m/z 549 [M+H]⁺, t_{R} = 0.459 min, mode N.

### Example 372: Synthesis of (18aS)-3-(3-aminoazetidin-1-yl)-12-(4-fluorophenyl)-5,12-dimethyl-6,7,8,9,11,12,16,17,18,1 8a-decahydro-5H,13H,15H-19,1-(metheno)pyrido[1,2-e]pyrimido[1,6-i][1,5,8,9,11]oxatetraa zacyclopentadecin-13-one

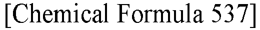

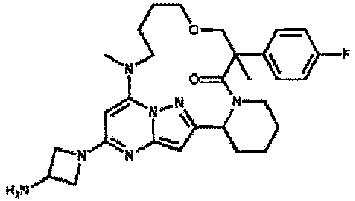

Step 372-1: By using the same approaches as in Step 1-1, Step 1-2, and Step 355-2, (18aS)-3-chloro-12-(4-fluorophenyl)-5,12-dimethyl-6,7,8,9,11,12,16,17,18,18a-decahydro-5 H,13H,15H-19,1-(metheno)pyrido[1,2-e]pyrimido[1,6-i][1,5,8,9,11]oxatetraazacyclopentade cin-13-one (94 mg, yellow oily matter) was obtained from the compound (0.30 g) obtained in Reference Example 1-1 (3) and the compound (0.24 g) obtained in Reference Example 2-104.
LCMS (ESI) m/z 500 [M+H]⁺, t_{R} = 1.009 min, mode L.

Step 372-2: By using the same approaches as in Step 1-4 and Step 5-2, the title compound (14 mg, colorless powder) was obtained from the compound (31 mg) obtained in Step 372-1 and 3-N-BOC-amino-azetidine (53 mg).
LCMS (ESI) m/z 536 [M+H]⁺, t_{R} = 0.565, 0.589 min, mode L.

### Example 373: Synthesis of (18aS)-12-(4-fluorophenyl)-3-(3-hydroxyazetidin-1-yl)-5,12-dimethyl-6,7,8,9,11,12,16,17,18 ,18a-decahydro-5H,13H,15H-19,1-(metheno)pyrido[1,2-e]pyrimido[1,6-i][1,5,8,9,11]oxatetr aazacyclopentadecin-13-one

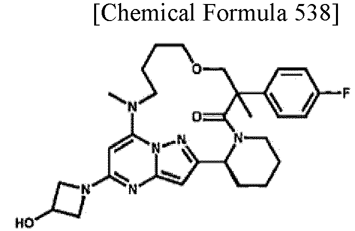

By using the same approach as in Step 3-1, the title compound (15 mg, beige amorphous) was obtained from the compound (21 mg) obtained in Step 372-1 and 3-hydroxyazetidine hydrochloride (46 mg).
LCMS (ESI) m/z 537 [M+H]⁺, t_{R} = 0.513, 0.550 min, mode N.

### Example 374: Synthesis of (18aS)-3-(azetidin-3-yl)-12-(4-fluorophenyl)-5,12-dimethyl-6,7,8,9,11,12,16,17,18,18a-deca hydro-5H,13H,15H-19,1-(metheno)pyrido[1,2-e]pyrimido[1,6-i][1,5,8,9,11]oxatetraazacyclo pentadecin-13-one

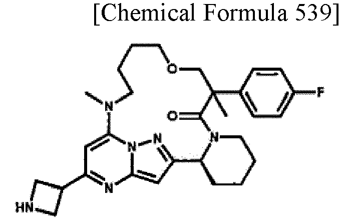

By using the same approaches as in Step 21-1 and Step 5-2, the title compound (18 mg, colorless powder) was obtained from the compound (40 mg) obtained in Step 372-1, using tert-butyl N-(3-iodocyclobutyl)carbamate instead of I-BOC-3-iodoazetidine.
LCMS (ESI) m/z 521 [M+H]⁺, t_{R} = 0.664, 0.679 min, mode N.

### Example 375: Synthesis of (18aS)-3-[(3S)-3-aminopyrrolidin-1-yl]-12-(4-fluorophenyl)-5,12-dimethyl-6,7,8,9,11,12,16, 17,18,18a-decahydro-5H,15H-19,1-(metheno)-10λ⁶-pyrido[1,2-e]pyrimido[1,6-i][1,2,5,8,9,1 1]thiapentaazacyclopentadecine-10,10,13-trione

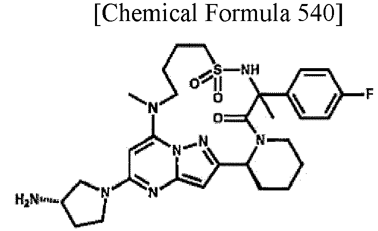

Step 375-1: By using the same approaches as in Step 1-1, Step 1-2, and Step 355-2, (18aS)-3-chloro-12-(4-fluorophenyl)-5,12-dimethyl-6,7,8,9,11,12,16,17,18,18a-decahydro-5 H,15H-19,1-(metheno)-10λ⁶-pyrido[1,2-e]pyrimido[1,6-i] [1,2,5,8,9,11]thiapentaazacyclopen tadecine-10,10,13-trione (0.18 g, yellow oily matter) was obtained from the compound (0.70 g) obtained in Reference Example 1-1 (3) and the compound (0.63 g) obtained in Reference Example 2-99.
LCMS (ESI) m/z 549 [M+H]⁺, t_{R} = 0.865, 0.900 min, mode L.

Step 375-2: By using the same approach as in Step 1-4, the title compound (42 mg, light yellow amorphous) was obtained from the compound (60 mg) obtained in Step 375-1. LCMS (ESI) m/z 599 [M+H]⁺, t_{R} = 0.463, 0.547 min, mode N.

### Example 376: Synthesis of (18aS)-3-(3-aminoazetidin-1-yl)-12-(4-fluorophenyl)-5,12-dimethyl-6,7,8,9,11,12,16,17,18,1 8a-decahydro-5H,15H-19,1-(metheno)-10λ⁶-pyrido[1,2-e]pyrimido[1,6-i][1,2,5,8,9,11]thiape ntaazacyclopentadecine-10,10,13-trione

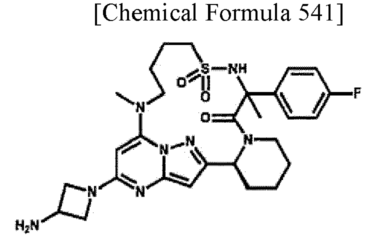

By using the same approaches as in Step 1-4 and Step 5-2, the title compound (30 mg, light yellow powder) was obtained from the compound (60 mg) obtained in Step 375-1 and 3-N-BOC-amino-azetidine (94 mg).
LCMS (ESI) m/z 585 [M+H]⁺, t_{R} = 0.485, 0.572 min, mode N.

### Example 377: Synthesis of (18aS)-12-(4-fluorophenyl)-3-(3-hydroxyazetidin-1-yl)-5,12-dimethyl-6,7,8,9,11,12,16,17,18 ,18a-decahydro-5H,15H-19,1-(metheno)-10λ⁶-pyrido[1,2-e]pyrimido[1,6-i][1,2,5,8,9,11]thia pentaazacyclopentadecine-10,10,13-trione

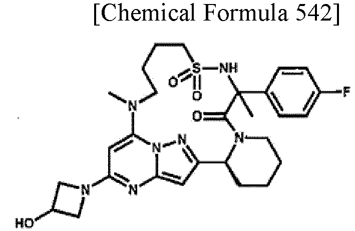

By using the same approach as in Step 3-1, the title compound (43 mg, light yellow amorphous) was obtained from the compound (57 mg) obtained in Step 375-1 and 3-hydroxyazetidine hydrochloride (0.12 g).
LCMS (ESI) m/z 586 [M+H]⁺, t_{R} = 0.661, 0.709 min, mode N.

### Example 378: Synthesis of (18aS)-3-[(3S)-3-aminopyrrolidin-1-yl]-5,12-dimethyl-12-(pyridin-3-yl)-6,7,8,9,11,12,16,17, 18,18a-decahydro-5H,13H,15H-19,1-(metheno)pyrido[1,2-e]pyrimido[1,6-i][1,5,8,9,11]oxate traazacyclopentadecin-13-one

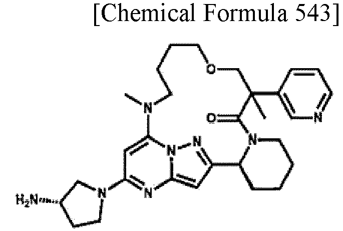

Step 378-1: By using the same approaches as in Step 1-1, Step 1-2, and Step 355-2, (18aS)-3-chloro-5,12-dimethyl-12-(pyridin-3-yl)-6,7,8,9,11,12,16,17,18,18a-decahydro-5H,1 3H,15H-19,1-(metheno)pyrido[1,2-e]pyrimido[1,6-i][1,5,8,9,11]oxatetraazacyclopentadecin-13-one (0.13 g, colorless amorphous) was obtained from the compound (0.77 g) obtained in Reference Example 1-1 (3) and the compound (0.64 g) obtained in Reference Example 2-105.
LCMS (ESI) m/z 483 [M+H]⁺, t_{R} = 0.794 min, mode N.

Step 378-2: By using the same approach as in Step 1-4, the title compound (27 mg, beige amorphous) was obtained from the compound (32 mg) obtained in Step 378-1. LCMS (ESI) m/z 533 [M+H]⁺, t_{R} = 0.631, 0.661 min, mode H.

### Example 379: Synthesis of (18aS)-3-(3-hydroxyazetidin-1-yl)-5,12-dimethyl-12-(pyridin-3-yl)-6,7,8,9,11,12,16,17,18,18 a-decahydro-5H,13H,15H-19,1-(metheno)pyrido[1,2-e]pyrimido[1,6-i][1,5,8,9,11]oxatetraaz acyclopentadecin-13-one

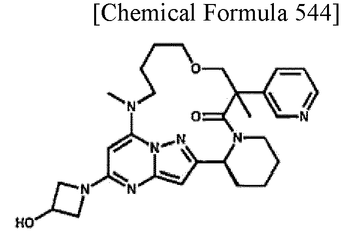

By using the same approach as in Step 3-1, the title compound (7.5 mg, beige amorphous) was obtained from the compound (25 mg) obtained in Step 378-1 and 3-hydroxyazetidine hydrochloride (57 mg).
LCMS (ESI) m/z 520 [M+H]⁺, t_{R} = 0.733, 0.784 min, mode H.

### Example 380: Synthesis of (18aS)-3-(3-aminoazetidin-1-yl)-5,12-dimethyl-12-(pyridin-3-yl)-6,7,8,9,11,12,16,17,18,18a-decahydro-5H,13H,15H-19,1-(metheno)pyrido[1,2-e]pyrimido[1,6-i][1,5,8,9,11]oxatetraazac yclopentadecin-13-one

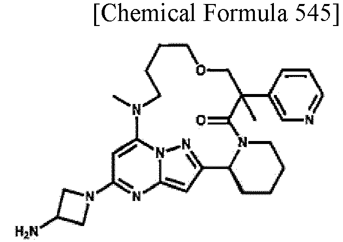

By using the same approaches as in Step 1-4 and Step 5-2, the title compound (21 mg, colorless powder) was obtained from the compound (30 mg) obtained in Step 378-1 and 3-N-BOC-amino-azetidine (54 mg).
LCMS (ESI) m/z 519 [M+H]⁺, t_{R} = 0.581, 0.607 min, mode H.

### Test Example 1: In vitro anti-RSV activity evaluation test

The antiviral activity of the present inventive compound against RSV was measured by a cytopathogenic effect (CPE) inhibition test, referring to the method described in the literature (Antimicrob Agents Chemother, 51, 3346-3353, 2007).

As the viral strain, RSV ATCC VR-1540 (A2) and its F protein D486N mutant strain were used. Before the test, the appropriate dilution factor for the viral solution of the A2 strain and D486N mutant strain, by which the desirable CPE was produced in Hep2 cells (DS Pharma Biomedical Co., Ltd.), was determined. One day before the test, Hep2 cells were seeded at a density of 1 × 10⁴ cells/well. On the very day of the test, a dilution series of the compound was fabricated using a maintenance medium and added to Hep2 cells. Subsequently, inoculation of the virus solutions of the A2 strain and the D486N mutant strain, diluted as appropriate, was conducted. For each plate, wells of infected untreated cells and uninfected cells were provided as 0% and 100% virus inhibition controls. After virus inoculation, the test plates were incubated for 4 days under conditions of 37°C and 5% CO₂. After the incubation, CPE by RSV was detected by using an XTT cell proliferation assay kit and measuring the absorbance at 450 nm using a microplate reader. The concentration of compound that inhibited CPE by 50% (EC₅₀) was determined by nonlinear regression. For some of the present inventive compounds, the EC₅₀ values against RSV A2 strain and D486N mutant strain are shown in Table 1 and Table 2.

Table 1: EC₅₀ (nmol/L) against RSV A2 strain

**[Table 1-1]**

| **Example No.** | EC₅₀ | **Example No.** | EC₅₀ | **Example** No | EC₅₀ | **Example No.** | EC₅₀ |
|---|---|---|---|---|---|---|---|
| 1 | 2.07 | 93 | 6.37 | 178 | 4.48 | 280 | 8.97 |
| 2 | 0.800 | 94 | 0.207 | 179 | 7.33 | 281 | 1.40 |
| 3 | 0.813 | 95 | 0.460 | 180 | 33.0 | 282 | 3.95 |
| 4 | 2.74 | 98 | 4.05 | 181 | 3.54 | 283 | 6.28 |
| 5 | 7.02 | 97 | 0.760 | 182 | 14.2 | 284 | 22.2 |
| 7 | 1.38 | 98 | 1.06 | 183 | 14.6 | 285 | 11.9 |
| 8 | 6.30 | 99 | 6.571 | 184 | 17.8 | 286 | 20.8 |
| 9 | 6.34 | 100 | 0.328 | 185 | 2.23 | 287 | 7.65 |
| 10 | 5.23 | 100 | 11.9 | 186 | 3.50 | 288 | 13.8 |
| 11 | 1.56 | 102 | 0.239 | 187 | 6.79 | 289 | 8.41 |
| 12 | 2.58 | 103 | 0.254 | 188 | 3.45 | 290 | 4.92 |
| 13 | 6.91 | 104 | 11.1 | 189 | 42.3 | 291 | 3.81 |
| 14 | 2.34 | 105 | 10.8 | 190 | 1.20 | 392 | 4.21 |
| 15 | 11.8 | 106 | 0.777 | 191 | 46.8 | 298 | 6.68 |
| 16 | 2.92 | 108c | 0.867 | 192 | 13.0 | 294 | 16.8 |
| 17 | 0.579 | 108 | 1.12 | 193 | 22.2 | 295 | 19.8 |
| 18 | 1.52 | 109 | 1.14 | 194 | 15.2 | 296 | 4.72 |
| 19 | 14.0 | 110 | 0.737 | 196 | 20.5 | 297 | 8.74 |
| 20 | 1.26 | 111 | 0.448 | 196 | 8.11 | 298 | 4.21 |
| 21 | 4.36 | 112 | 0.347 | 198 | 2.82 | 299 | 9.69 |
| 22 | 28.4 | 114 | 0.446 | 199 | 17.4 | 301 | 3.79 |
| 23 | 0.177 | 115 | 0.344 | 200 | 5.32 | 302 | 47.3 |
| 25 | 14.5 | 116 | 0.629 | 202 | 1.33 | 303 | 10.8 |
| 26 | 2.82 | 112 | 0.773 | 203 | 3.79 | 304 | 4.24 |
| 27 | 12.0 | 118c | 0.632 | 204 | 2.59 | 305 | 13.0 |
| 28 | 1.57 | 118 | 0.690 | 206 | 1.16 | 306 | 14.8 |
| 29 | 1.64 | 119 | 0.680 | 207 | 1.79 | 307 | 4.00 |
| 30 | 10.0 | 120 | 0.450 | 208 | 4.33 | 308 | 2.48 |

**[Table 1-2]**

| **Example No.** | EC₅₀ | **Example No.** | EC₅₀ | **Example No.** | EC₅₀ | **Example No.** | EC₅₀ |
|---|---|---|---|---|---|---|---|
| 31 | 0.538 | 121 | 11.0 | 209 | 9.00 | 309 | 1.74 |
| 33 | 0.537 | 122a | 1.64 | 210 | 1.40 | 210 | 15.8 |
| 34 | 2.48 | 122b | 14.0 | 211 | 4.66 | 311 | 5.56 |
| 35 | 7.70 | 123 | 8.51 | 212 | 7.14 | 312 | 4.06 |
| 36 | 3.36 | 124a | 0.854 | 213 | 7.61 | 313 | 3.19 |
| 37 | 5.01 | 125a | 1.08 | 214 | 41.1 | 314 | 31.9 |
| 38 | 0.785 | 125b | 13.4 | 316 | 2.11 | 315 | 4.20 |
| 39 | 0.766 | 126a | 0.610 | 217 | 8.41 | 316 | 9.49 |
| 40 | 0.856 | 127a | 18.7 | 218 | 9.89 | 318 | 22.7 |
| 41 | 0.846 | 127b | 2.56 | 220 | 18.0 | 319 | 7.58 |
| 42 | 4.72 | 126 | 3.43 | 221 | 2.81 | 324 | 20.9 |
| 43 | 4.54 | 129a | 1.43 | 222a | 7.17 | 326 | 7.73 |
| 44 | 0.843 | 129b | 13.7 | 223 | 1.34 | 327 | 1.72 |
| 45 | 2.56 | 130 | 3.13 | 227 | 8.12 | 326 | 3.48 |
| 46 | 0.872 | 131 | 3.92 | 230 | 11.8 | 329 | 1.61 |
| 47 | 3.32 | 132a | 12.1 | 231 | 6.88 | 330 | 19.8 |
| 48 | 0.861 | 132b | 3.80 | 233 | 16.7 | 331 | 23.5 |
| 49 | 3.61 | 133 | 1.36 | 233 | 39.5 | 333 | 1.94 |
| 50 | 2.18 | 134 | 15.6 | 234 | 6.77 | 334 | 3.46 |
| 51 | 1.21 | 135 | 5.44 | 236 | 40.0 | 335 | 4.16 |
| 52 | 4.77 | 136 | 4.17 | 238 | 28.0 | 336 | 20.9 |
| 53 | 0.571 | 137a | 3.49 | 239 | 5.97 | 337a | 8.54 |
| 54 | 3.20 | 137b | 18.2 | 244 | 4.01 | 337b | 5.08 |
| 57 | 3.11 | 138 | 4.59 | 245 | 4.71 | 338 | 2.37 |
| 58 | 7.91 | 140 | 18.9 | 246 | 0.759 | 339 | 3.67 |
| 59 | 1.16 | 141 | 5.08 | 247 | 1.31 | 340 | 1.76 |
| 60 | 1.34 | 142 | 17.8 | 248 | 3.90 | 341 | 3.46 |
| 61 | 1.77 | 143 | 5.29 | 249 | 2.70 | 342 | 5.57 |

**[Table 1-3]**

| **Example No.** | EC₅₀ | **Example No.** | EC₅₀ | **Example No.** | EC₅₀ | **Example No.** | EC₅₀ |
|---|---|---|---|---|---|---|---|
| 62 | 1.60 | 144 | 3.90 | 250 | 43.5 | 343 | 3.99 |
| 63 | 2.49 | 145 | 17.5 | 251 | 12.0 | 344 | 2.15 |
| 64 | 3.66 | 146 | 20.8 | 252 | 7.52 | 345 | 2.80 |
| 65 | 3.76 | 147 | 5.12 | 253 | 4.08 | 346a | 3.72 |
| 66 | 4.36 | 148 | 28.9 | 254 | 3.28 | 346b | 4.48 |
| 67 | 0.909 | 149 | 19.5 | 255 | 5.60 | 347 | 3.45 |
| 68 | 1.16 | 150 | 2.80 | 266 | 4.55 | 348 | 1.30 |
| 69 | 1.22 | 151 | 17.0 | 257 | 1.75 | 349 | 3.89 |
| 70 | 1.84 | 152 | 4.88 | 258 | 8.16 | 350 | 3.97 |
| 71 | 0.893 | 155 | 6.06 | 259 | 3.08 | 355 | 2.16 |
| 72 | 1.03 | 156 | 8.58 | 260 | 2.11 | 356a | 1.99 |
| 73 | 0.661 | 157 | 15.7 | 261 | 17.2 | 356b | 8.07 |
| 74 | 4.14 | 158 | 20.9 | 262 | 1.27 | 357 | 2.88 |
| 75 | 1.30 | 161 | 19.6 | 263 | 6.16 | 358 | 3.21 |
| 77 | 1.52 | 162 | 4.51 | 264 | 8.39 | 359 | 6.33 |
| 78 | 1.70 | 163 | 10.3 | 265 | 4.88 | 361 | 3.14 |
| 79 | 3.18 | 165 | 5.97 | 267 | 4.38 | 362 | 6.46 |
| 80 | 1.47 | 166 | 6.91 | 268 | 2.48 | 363 | 5.05 |
| 81 | 1.65 | 167 | 5.31 | 269 | 40.5 | 364a | 21.2 |
| 82 | 4.31 | 168 | 2.65 | 270 | 1.14 | 370 | 20.1 |
| 83 | 1.58 | 169 | 9.91 | 271 | 3.71 | 371 | 12.8 |
| 84 | 1.04 | 170 | 2.39 | 272 | 5.29 | 372 | 3.56 |
| 85 | 3.78 | 171 | 5.06 | 273 | 39.8 | 373 | 21.2 |
| 86 | 1.48 | 172 | 6.88 | 274 | 8.05 | 374 | 31.9 |
| 87 | 3.77 | 173 | 1.30 | 275 | 9.55 | 375 | 3.89 |
| 88 | 3.09 | 174 | 1.89 | 276 | 10.2 | 376 | 4.81 |
| 89 | 1.35 | 175 | 4.28 | 277 | 10.8 | 377 | 22.7 |
| 90 | 0.292 | 76 | 8.67 | 278 | 6.25 | 378 | 16.1 |
| 92 | 1.60 | 177 | 20.1 | 279 | 14.5 | 380 | 8.77 |

Table 2: EC₅₀ (nmol/L) against F protein D486N mutant strain of RSV A2 strain

**[Table 2]**

| **Example No.** | EC₅₀ | **Example No.** | EC₅₀ |
|---|---|---|---|
| 1 | 3.17 | 118t | 0.720 |
| 2 | 3.06 | 120 | 0.639 |
| 37 | 10.1 | 124a | 7.23 |
| 39 | 0.867 | 126a | 2.05 |
| 53 | 1.05 | 171 | 4.19 |
| 57 | 12.6 | 244 | 7.34 |
| 90 | 2.80 | 298 | 3.13 |
| 100 | 0.676 | 312 | 31.8 |
| 112 | 0.704 | 335 | 10.6 |
| 115 | 0.777 | 339 | 4.11 |
| 117 | 0.742 | 348 | 8.20 |

### INDUSTRIAL APPLICABILITY

The present inventive compound (I) or a pharmaceutically acceptable salt thereof has anti-RSV activity, and can be utilized as a drug for preventing or treating an infection in which viruses of the subfamily Pneumovirinae, including respiratory syncytial virus (RSV), are involved.

## Claims

1. A compound represented by formula (I): 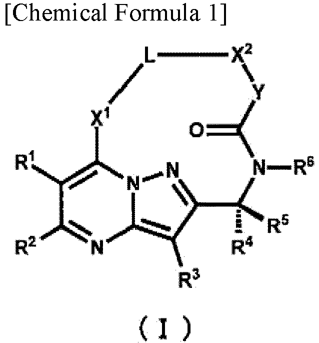 wherein
Y represents phenylene {the phenylene is optionally substituted with one to three substituents selected from the group consisting of a halogen atom, a C₁₋₆ alkyl group (the C₁₋₆ alkyl group is optionally substituted with one to five substituents selected from the group consisting of a deuterium atom, a halogen atom, an amino group, and a hydroxy group), a C₁₋₆ alkoxy group, a C₂₋₆ alkenyl group, a C₁₋₆ alkanoyl group, a C₃₋₆ cycloalkyl group, a cyano group, and a tetramethylguanidyl group}, naphthalenediyl, pyridinediyl, pyridonediyl, thiophenediyl, pyrazolediyl {the naphthalenediyl, the pyridinediyl, the pyridonediyl, the thiophenediyl, and the pyrazolediyl are optionally substituted with one to three substituents selected from the group consisting of a halogen atom, a C₁₋₆ alkyl group (the C₁₋₆ alkyl group is optionally substituted with one to five substituents selected from the group consisting of a deuterium atom, a halogen atom, an amino group, and a hydroxy group), a C₁₋₆ alkoxy group, a C₂₋₆ alkenyl group, a C₁₋₆ alkanoyl group, a C₃₋₆ cycloalkyl group, a cyano group, and a tetramethylguanidyl group}, methylene {the methylene is optionally substituted with one to two substituents selected from the group consisting of a C₁₋₆ alkyl group (the C₁₋₆ alkyl group is optionally substituted with one phenyl group) and a phenyl group (the phenyl group is optionally substituted with one to three substituents selected from the group consisting of a halogen atom and a C₁₋₆ alkyl group)}, indenediyl, or ethylene {the ethylene is optionally substituted with one to three substituents selected from the group consisting of a C₁₋₆ alkyl group (the C₁₋₆ alkyl group is optionally substituted with one phenyl group), a phenyl group (the phenyl group is optionally substituted with one to three substituents selected from the group consisting of a halogen atom and a C₁₋₆ alkyl group), and a pyridyl group, or one methylene in the ethylene is optionally replaced with C₃₋₆ cycloalkanediyl or phenylene}; X¹ represents a single bond or NR^{X11} {RX¹¹ represents a hydrogen atom, a C₁₋₆ alkyl group (the C₁₋₆ alkyl group is optionally substituted with one to five substituents selected from the group consisting of a deuterium atom, a hydroxy group, an amino group, and a C₁₋₆ alkoxy group), or a C₃₋₆ cycloalkyl group};
X² represents O, CH₂, or NR^{X21} {R^{X21} represents a hydrogen atom, a C₁₋₆ alkyl group, or a C₁₋₆ alkylsulfonyl group (the C₁₋₆ alkylsulfonyl group is optionally substituted with one to three halogen atoms)};
L represents:
CHR^{X12}-CH₂-CH₂-NHCO-CH₂, CR^{X12}=CH-CH₂-NHCO-CH₂, or CH₂-NR^{X12}-(CH₂)₃ {R^{X12} represents a hydrogen atom, a C₁₋₆ alkyl group (the C₁₋₆ alkyl group is optionally substituted with one to five substituents selected from the group consisting of a deuterium atom, a hydroxy group, an amino group, and a C₁₋₆ alkoxy group), or a C₃₋₆ cycloalkyl group}, when X¹ is a single bond; and
C₃₋₆ alkanediyl, C₂₋₄ alkanediyl-CO, C₂₋₄ alkanediyl-SO₂, or Z¹-Z²-Z³, or any of the structures represented by formula group (II) below, when X¹ is NR^{X11};
Z¹ represents C₁₋₄ alkanediyl (the C₁₋₄ alkanediyl is optionally substituted with one to three substituents selected from the group consisting of a halogen atom and oxo);
Z² represents O or NR^{Z2} {R^{Z2} represents a hydrogen atom, a C₁₋₆ alkyl group (the C₁₋₆ alkyl group is optionally substituted with one to three substituents selected from the group consisting of a phenyl group, a hydroxy group, and a C₃₋₆ cycloalkyl group), a C₁₋₆ alkylsulfonyl group, a C₁₋₆ alkanoyl group (the C₁₋₆ alkanoyl group is optionally substituted with one amino group), a sulfamoyl group, a C₃₋₆ cycloalkyl group, a pyridyl group, or a C₁₋₆ alkoxycarbonyl group (the C₁₋₆ alkoxycarbonyl group is optionally substituted with one to two phenyl groups)}; and
Z³ represents C₁₋₄ alkanediyl {the C₁₋₄ alkanediyl is optionally substituted with one to five substituents selected from the group consisting of a halogen atom, oxo, and a hydroxy group, or one methylene in the C₁₋₄ alkanediyl is optionally replaced with C₃₋₆ cycloalkanediyl (in the C₃₋₆ cycloalkanediyl, one methylene in the ring is optionally
replaced with an oxygen atom) or sulfonyl}; 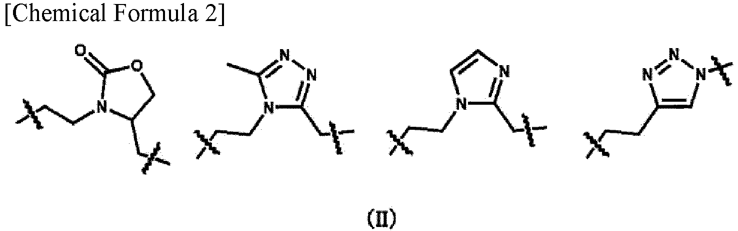
R¹ represents a hydrogen atom, a halogen atom, a C₁₋₆ alkyl group, or a C₃₋₆ cycloalkyl group; R² represents a hydrogen atom, a halogen atom, a cyano group, a C₁₋₆ alkyl group (the C₁₋₆ alkyl group is optionally substituted with one substituent selected from the group consisting of an amino group, a hydroxy group, and a di-C₁₋₆ alkylamino group), a C₂₋₆ alkenyl group (the C₂₋₆ alkenyl group is optionally substituted with one substituent selected from the group consisting of a hydroxy group and a carboxy group), a C₂₋₆ alkynyl group (the C₂₋₆ alkynyl group is optionally substituted with one substituent selected from the group consisting of a hydroxy group and an amino group), a C₁₋₆ alkoxy group (the C₁₋₆ alkoxy group is optionally substituted with one amino group), a C₁₋₆ alkylsulfanyl group (the C₁₋₆ alkylsulfanyl group is optionally substituted with one amino group), a C₃₋₆ cycloalkyl group {the C₃₋₆ cycloalkyl group is optionally substituted with one substituent selected from the group consisting of a C₁₋₆ alkyl group (the C₁₋₆ alkyl group is optionally substituted with one hydroxy group), an amino group, a hydroxy group, and a carbamoyl group}, a heterocyclyloxy group, a heterocyclylamino group, a C₁₋₆ alkylamino group {the C₁₋₆ alkylamino group is optionally substituted with one to two substituents selected from the group consisting of a hydroxy group, an amino group, a C₁₋₆ alkyl group, and a C₃₋₆ cycloalkyl group (the C₃₋₆ cycloalkyl group is optionally substituted with one amino group)}, a C₃₋₆ cycloalkylamino group (the C₃₋₆ cycloalkylamino group is optionally substituted with one amino group), or a heterocyclyl group {the heterocyclyl group is optionally substituted with one to three substituents selected from the group consisting of a halogen atom, a hydroxy group, an amino group, a C₁₋₆ alkoxy group, a mono-C₁₋₆ alkylamino group, a di-C₁₋₆ alkylamino group, a C₂₋₆ alkanoylamino group, and a C₁₋₆ alkyl group (the C₁₋₆ alkyl group is optionally substituted with one amino group or a hydroxy group)};
R³ represents a hydrogen atom or a halogen atom;
R⁴ and R⁵ are the same or different, and each represent a hydrogen atom or a C₁₋₆ alkyl group; and
R⁶ represents a C₁₋₆ alkyl group {the C₁₋₆ alkyl group is optionally substituted with one to three substituents selected from the group consisting of a halogen atom, a hydroxy group, an amino group, a C₁₋₆ alkoxy group (the C₁₋₆ alkoxy group is optionally substituted with one to three halogen atoms), an aryloxy group, a C₁₋₆ alkylsulfonylamino group, a C₁₋₆ alkylsulfonyl group, a heterocyclyl group (the heterocyclyl group is optionally substituted with one to two oxo), an aryl group, and a heteroaryl group}; or
R⁵ and R⁶ optionally form a 5- to 6-membered cyclic amine optionally containing one oxygen atom in the ring (the cyclic amine is optionally substituted with one to three halogen atoms), together with the adjacent carbon atom and nitrogen atom,
or a pharmaceutically acceptable salt thereof.

2. The compound according to claim 1, wherein:
in formula (I) above,
Y represents phenylene {the phenylene is optionally substituted with one to three substituents selected from the group consisting of a halogen atom, a C₁₋₆ alkyl group (the C₁₋₆ alkyl group is optionally substituted with one to five substituents selected from the group consisting of a deuterium atom, a halogen atom, an amino group, and a hydroxy group), a C₁₋₆ alkoxy group, a C₂₋₆ alkenyl group, a C₁₋₆ alkanoyl group, a C₃₋₆ cycloalkyl group, a cyano group, and a tetramethylguanidyl group}, naphthalenediyl, pyridinediyl, pyridonediyl, thiophenediyl,
pyrazolediyl {the naphthalenediyl, the pyridinediyl, the pyridonediyl, the thiophenediyl, and
the pyrazolediyl are optionally substituted with one to three substituents selected from the group consisting of a halogen atom, a C₁₋₆ alkyl group (the C₁₋₆ alkyl group is optionally substituted with one to five substituents selected from the group consisting of a deuterium atom, a halogen atom, an amino group, and a hydroxy group), a C₁₋₆ alkoxy group, a C₂₋₆ alkenyl group, a C₁₋₆ alkanoyl group, a C₃₋₆ cycloalkyl group, a cyano group, and a tetramethylguanidyl group}, methylene {the methylene is optionally substituted with one to two substituents selected from the group consisting of a C₁₋₆ alkyl group (the C₁₋₆ alkyl group is optionally substituted with one phenyl group) and a phenyl group (the phenyl group is optionally substituted with one to three substituents selected from the group consisting of a halogen atom and a C₁₋₆ alkyl group)}, indenediyl, or ethylene {the ethylene is optionally substituted with one to three substituents selected from the group consisting of a C₁₋₆ alkyl group, a phenyl group (the phenyl group is optionally substituted with one to three halogen atoms), and a pyridyl group, or one methylene in the ethylene is optionally replaced with C₃₋₆ cycloalkanediyl or phenylene}; and
R² represents a hydrogen atom, a halogen atom, a cyano group, a C₁₋₆ alkyl group (the C₁₋₆ alkyl group is optionally substituted with one substituent selected from the group consisting of an amino group, a hydroxy group, and a di-C₁₋₆ alkylamino group), a C₂₋₆ alkenyl group (the C₂₋₆ alkenyl group is optionally substituted with one substituent selected from the group consisting of a hydroxy group and a carboxy group), a C₂₋₆ alkynyl group (the C₂₋₆ alkynyl group is optionally substituted with one substituent selected from the group consisting of a hydroxy group and an amino group), a C₁₋₆ alkoxy group (the C₁₋₆ alkoxy group is optionally substituted with one amino group), a C₁₋₆ alkylsulfanyl group (the C₁₋₆ alkylsulfanyl group is optionally substituted with one amino group), a C₃₋₆ cycloalkyl group {the C₃₋₆ cycloalkyl group is optionally substituted with one substituent selected from the group consisting of a C₁₋₆ alkyl group (the C₁₋₆ alkyl group is optionally substituted with one hydroxy group), an amino group, a hydroxy group, and a carbamoyl group}, a heterocyclyloxy group, a heterocyclylamino group, a C₁₋₆ alkylamino group {the C₁₋₆ alkylamino group is optionally substituted with one to two substituents selected from the group consisting of an amino group, a C₁₋₆ alkyl group, and a C₃₋₆ cycloalkyl group (the C₃₋₆ cycloalkyl group is optionally substituted with one amino group)}, a C₃₋₆ cycloalkylamino group (the C₃₋₆ cycloalkylamino group is optionally substituted with one amino group), or a heterocyclyl group {the heterocyclyl group is optionally substituted with one to three substituents selected from the group consisting of a halogen atom, a hydroxy group, an amino group, a mono-C₁₋₆ alkylamino group, a di-C₁₋₆ alkylamino group, and a C₁₋₆ alkyl group (the C₁₋₆ alkyl group is optionally substituted with one amino group)},
or a pharmaceutically acceptable salt thereof.

3. The compound according to claim 1 or 2, wherein:
in formula (I) above,
Y is phenylene {the phenylene is optionally substituted with one to three substituents selected from the group consisting of a halogen atom, a C₁₋₆ alkyl group (the C₁₋₆ alkyl group is optionally substituted with one to five substituents selected from the group consisting of a deuterium atom, a halogen atom, an amino group, and a hydroxy group), a C₁₋₆ alkoxy group,
a C₂₋₆ alkenyl group, an acetyl group, a cyclopropyl group, a cyano group, and a tetramethylguanidyl group}, naphthalenediyl, pyridinediyl (the pyridinediyl is optionally substituted with one to three C₁₋₆ alkyl groups), pyridonediyl, thiophenediyl, pyrazolediyl (the pyrazolediyl is optionally substituted with one to three C₁₋₆ alkyl groups), methylene {the methylene is optionally substituted with one to two substituents selected from the group consisting of a C₁₋₆ alkyl group (the C₁₋₆ alkyl group is optionally substituted with one phenyl group) and a phenyl group (the phenyl group is optionally substituted with one to three substituents selected from the group consisting of a halogen atom and a C₁₋₆ alkyl group)}, indenediyl, or ethylene {the ethylene is optionally substituted with one to three substituents selected from the group consisting of a C₁₋₆ alkyl group, a phenyl group (the phenyl group is optionally substituted with one to three halogen atoms), and a pyridyl group, or one methylene in the ethylene is optionally replaced with cyclohexanediyl or phenylene};
X¹ is a single bond or NR^{X11} {R^{X11} represents a hydrogen atom, a C₁₋₆ alkyl group (the C₁₋₆ alkyl group is optionally substituted with one to five substituents selected from the group consisting of a deuterium atom, a hydroxy group, an amino group, and a C₁₋₆ alkoxy group),
or a cyclopropyl group};
Z³ is C₁₋₄ alkanediyl (the C₁₋₄ alkanediyl is optionally substituted with one to five substituents selected from the group consisting of a halogen atom, oxo, and a hydroxy group, or one methylene in the C₁₋₄ alkanediyl is optionally replaced with cyclopropanediyl, oxetanediyl, or
sulfonyl);
R⁴ and R⁵ are the same or different, and are each a hydrogen atom or a C₁₋₆ alkyl group; and
R⁶ is a C₁₋₆ alkyl group; or
R⁵ and R⁶ optionally form a piperidine ring (the piperidine ring is optionally substituted with one to three halogen atoms) or a morpholine ring, together with the adjacent carbon atom and
nitrogen atom,
or a pharmaceutically acceptable salt thereof.

4. The compound according to any one of claims 1 to 3, wherein:
in formula (I) above,
Y is phenylene {the phenylene is optionally substituted with one to three substituents selected from the group consisting of a halogen atom, a C₁₋₆ alkyl group (the C₁₋₆ alkyl group is optionally substituted with one to five substituents selected from the group consisting of a deuterium atom, a halogen atom, an amino group, and a hydroxy group), a C₁₋₆ alkoxy group,
a C₂₋₆ alkenyl group, a C₁₋₆ alkanoyl group, a C₃₋₆ cycloalkyl group, a cyano group, and a tetramethylguanidyl group}, naphthalenediyl, pyridinediyl (the pyridinediyl is optionally substituted with one to three C₁₋₆ alkyl groups), pyridonediyl, thiophenediyl, or pyrazolediyl (the pyrazolediyl is optionally substituted with one to three C₁₋₆ alkyl groups),
or a pharmaceutically acceptable salt thereof.

5. The compound according to any one of claims 1 to 4, wherein:
in formula (I) above,
Y is phenylene {the phenylene is optionally substituted with one to three substituents selected from the group consisting of a halogen atom, a C₁₋₆ alkyl group (the C₁₋₆ alkyl group is optionally substituted with one to five substituents selected from the group consisting of a deuterium atom, a halogen atom, an amino group, and a hydroxy group), a C₁₋₆ alkoxy group,
a C₂₋₆ alkenyl group, a C₁₋₆ alkanoyl group, a C₃₋₆ cycloalkyl group, a cyano group, and a tetramethylguanidyl group},
or a pharmaceutically acceptable salt thereof.

6. The compound according to any one of claims 1 to 5, wherein:
in formula (I) above,
X¹ is NR^{X11} {R^{X11} is a C₁₋₆ alkyl group (the C₁₋₆ alkyl group is optionally substituted with one to three deuterium atoms)};
X² is O or NH;
L is (CH₂)₂-NR^{Z2}CO-CH₂, CH₂C(CH₃)₂-NR^{Z2}CO-CH₂, (CH₂)₂-NR^{Z2}CO-CH(CH₃), CH₂-CONR^{Z2}-(CH₂)₂, (CH₂)₃-NR^{Z2}CO-CH₂, (CH₂)₂-NR^{Z2}-(CH₂)₂, CH₂CH(CF₃)-NR^{Z2}(CH₂)₂, (CH₂)₂-NR^{Z2}CH(CF₃)-CH₂, (CH₂)₂-NR^{Z2}-C(-CH₂OCH₂-)CH₂, (CH₂)₄, (CH₂)₅, (CH₂)₂-NR^{Z2}SO₂-CH₂, (CH₂)₂-O-(CH₂)₂, (CH₂)₃-SO₂, or (CH₂)₄-SO₂, or has any of the structural formulas represented by formula (III) below: 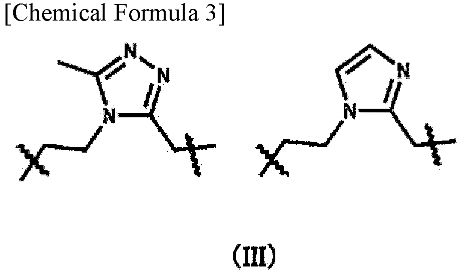 R^{Z2} is a hydrogen atom or a C₁₋₆ alkyl group;
R¹ is a hydrogen atom, a halogen atom, or a C₁₋₆ alkyl group;
R² is a C₁₋₆ alkyl group (the C₁₋₆ alkyl group is optionally substituted with one substituent selected from the group consisting of an amino group, a hydroxy group, and a di-C₁₋₆ alkylamino group), a C₂₋₆ alkenyl group (the C₂₋₆ alkenyl group is optionally substituted with one substituent selected from the group consisting of a hydroxy group and a carboxy group), a C₂₋₆ alkynyl group (the C₂₋₆ alkynyl group is optionally substituted with one substituent selected from the group consisting of a hydroxy group and an amino group), a C₁₋₆ alkoxy group (the C₁₋₆ alkoxy group is optionally substituted with one amino group), a C₃₋₆ cycloalkyl group {the C₃₋₆ cycloalkyl group is optionally substituted with one substituent selected from the group consisting of a C₁₋₆ alkyl group (the C₁₋₆ alkyl group is optionally substituted with one hydroxy group), an amino group, a hydroxy group, and a carbamoyl group}, a C₁₋₆ alkylamino group {the C₁₋₆ alkylamino group is optionally substituted with one to two substituents selected from the group consisting of an amino group, a C₁₋₆ alkyl group,
and a C₃₋₆ cycloalkyl group (the C₃₋₆ cycloalkyl group is optionally substituted with one amino group)}, or a heterocyclyl group {the heterocyclyl group is optionally substituted with one to three substituents selected from the group consisting of a halogen atom, a hydroxy group, an amino group, a mono-C₁₋₆ alkylamino group, a di-C₁₋₆ alkylamino group, and a C₁₋₆ alkyl group (the C₁₋₆ alkyl group is optionally substituted with one amino group)};
R³ is a hydrogen atom;
R⁴ is a hydrogen atom;
R⁵ is a C₁₋₆ alkyl group; and
R⁶ is a C₁₋₆ alkyl group; or
R⁵ and R⁶ optionally form a piperidine ring (the piperidine ring is optionally substituted with one to three halogen atoms) or a morpholine ring, together with the adjacent carbon atom and
nitrogen atom,
or a pharmaceutically acceptable salt thereof.

7. The compound according to any one of claims 1 to 6, wherein:
in formula (I) above,
X¹ is NR^{X11} {R^{X11} is a C₁₋₆ alkyl group (the C₁₋₆ alkyl group is optionally substituted with one to three deuterium atoms)};
X² iS O; and
L is (CH₂)₂-NR^{Z2}CO-CH₂, CH₂C(CH₃)₂-NR^{Z2}CO-CH₂, CH₂-CONR^{Z2}-(CH₂)₂, (CH₂)₃-NR^{Z2}CO-CH₂, (CH₂)₂-NR^{Z2}-(CH₂)₂, (CH₂)₄, (CH₂)₅, (CH₂)₂-NR^{Z2}SO₂-CH₂, (CH₂)₂-NR^{Z2}CH(CF₃)-CH₂, CH₂CH(CF₃)-NR^{Z2}-(CH₂)₂, (CH₂)₂-NR^{Z2}-C(-CH₂OCH₂-)CH₂, or
(CH₂)₂-O-(CH₂)₂, or has any of the structural formulas represented by formula (III) below: 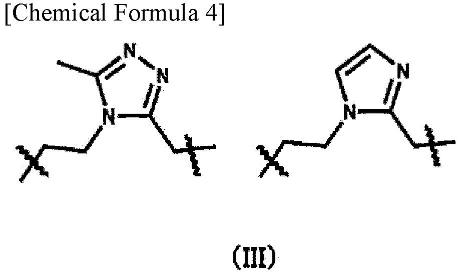 or a pharmaceutically acceptable salt thereof.

8. The compound according to any one of claims 1 to 6, wherein:
in formula (I) above,
X¹ is NR^{X11} {R^{X11} is a C₁₋₆ alkyl group (the C₁₋₆ alkyl group is optionally substituted with one to three deuterium atoms)};
X² is NH; and
L is (CH₂)₃-SO₂ or (CH₂)₄-SO₂,
or a pharmaceutically acceptable salt thereof.

9. The compound according to any one of claims 1 to 5, wherein:
in formula (I) above,
X¹ is a single bond;
X² is O;
L is CH(CH₃)-CH₂-CH₂-NHCO-CH₂ or C(CH₃)=CH-CH₂-NHCO-CH₂;
R¹ is a hydrogen atom, a halogen atom, or a C₁₋₆ alkyl group;
R² is a C₁₋₆ alkyl group (the C₁₋₆ alkyl group is optionally substituted with one substituent selected from the group consisting of an amino group, a hydroxy group, and a di-C₁₋₆ alkylamino group), a C₂₋₆ alkenyl group (the C₂₋₆ alkenyl group is optionally substituted with one substituent selected from the group consisting of a hydroxy group and a carboxy group), a C₂₋₆ alkynyl group (the C₂₋₆ alkynyl group is optionally substituted with one substituent selected from the group consisting of a hydroxy group and an amino group), a C₁₋₆ alkoxy group (the C₁₋₆ alkoxy group is optionally substituted with one amino group), a C₁₋₆ alkylsulfanyl group (the C₁₋₆ alkylsulfanyl group is optionally substituted with one amino group), a C₃₋₆ cycloalkyl group {the C₃₋₆ cycloalkyl group is optionally substituted with one substituent selected from the group consisting of a C₁₋₆ alkyl group (the C₁₋₆ alkyl group is optionally substituted with one hydroxy group), an amino group, a hydroxy group, and a carbamoyl group}, a C₁₋₆ alkylamino group {the C₁₋₆ alkylamino group is optionally substituted with one to two substituents selected from the group consisting of an amino group,
a C₁₋₆ alkyl group, and a C₃₋₆ cycloalkyl group (the C₃₋₆ cycloalkyl group is optionally substituted with one amino group)}, or a heterocyclyl group {the heterocyclyl group is optionally substituted with one to three substituents selected from the group consisting of a halogen atom, a hydroxy group, an amino group, a mono-C₁₋₆ alkylamino group, a di-C₁₋₆ alkylamino group, and a C₁₋₆ alkyl group (the C₁₋₆ alkyl group is optionally substituted with one amino group)};
R³ is a hydrogen atom;
R⁴ is a hydrogen atom;
R⁵ is a C₁₋₆ alkyl group; and
R⁶ is a C₁₋₆ alkyl group; or
R⁵ and R⁶ optionally form a piperidine ring (the piperidine ring is optionally substituted with one to three halogen atoms) or a morpholine ring, together with the adjacent carbon atom and
nitrogen atom,
or a pharmaceutically acceptable salt thereof.

10. The compound according to any one of claims 1 to 3, wherein:
in formula (I) above,
Y is methylene or ethylene {the methylene and the ethylene are optionally substituted with one to two substituents selected from the group consisting of a C₁₋₆ alkyl group (the C₁₋₆ alkyl group is optionally substituted with one phenyl group) and a phenyl group (the phenyl group is optionally substituted with one to three substituents selected from the group consisting of a halogen atom and a C₁₋₆ alkyl group)},
or a pharmaceutically acceptable salt thereof.

11. The compound according to any one of claims 1 to 10, wherein:
in formula (I) above,
R¹ is a hydrogen atom, a fluorine atom, a chlorine atom, or a methyl group,
or a pharmaceutically acceptable salt thereof.

12. The compound according to any one of claims 1 to 11, wherein:
in formula (I) above,
R² is a cyclopropyl group, a cyclobutyl group (the cyclobutyl group is optionally substituted with one amino group or hydroxyl group), an azetidinyl group (the azetidinyl group is optionally substituted with one amino group or hydroxyl group), a pyrrolidinyl group substituted with one amino group, an aminoethylamino group, or a hydroxyethylamino group,
or a pharmaceutically acceptable salt thereof.

13. The compound according to any one of claims 1 to 12, wherein:
in formula (I) above,
R³ is a hydrogen atom or a fluorine atom,
or a pharmaceutically acceptable salt thereof.

14. The compound according to any one of claims 1 to 13, wherein:
in formula (I) above,
R⁴ is a hydrogen atom; and
R⁵ and R⁶ form a piperidine ring (the piperidine ring is optionally substituted with one to three halogen atoms), together with the adjacent carbon atom and nitrogen atom,
or a pharmaceutically acceptable salt thereof.

15. The compound according to claim 1, selected from the group consisting of:
(18aS)-13 -[(3S)-3-aminopyrrolidin-1-yl]-2-fluoro-11-methyl-8,9,10,11,19,20,21,22-octahydr o-18aH,24H-18,15-(metheno)pyrido[2,1-l]pyrimido[6,1-h][1,4,7,9,10,13]benzoxapentaazacy clohexadecine-7,24(6H)-dione;
(18aS)-13-(azetidin-1-yl)-2-fluoro-11-methyl-8,9,10,11,19,20,21,22-octahydro-18aH,24H-18 ,15-(metheno)pyrido[2,1-l]pyrimido[6,1-h][1,4,7,9,10,13]benzoxapentaazacyclohexadecine-7 ,24(6H)-dione;
(18aS)-2-fluoro-13-(3-hydroxyazetidin-1-yl)-11-methyl-8,9,10,11,19,20,21,22-octahydro-18a H,24H-18,15-(metheno)pyrido[2,1-l]pyrimido[6,1-h][1,4,7,9,10,13]benzoxapentaazacyclohe xadecine-7,24(6H)-dione;
(18aS)-13-(3-aminoazetidin-1-yl)-2-fluoro-11-methyl-8,9,10,11,19,20,21,22-octahydro-18aH ,24H-18,15-(metheno)pyrido[2,1-l]pyrimido[6,1-h][1,4,7,9,10,13]benzoxapentaazacyclohexa decine-7,24(6H)-dione;
(18aS)-13-(azetidin-1-yl)-2-fluoro-8,11-dimethyl-8,9,10,11,19,20,21,22-octahydro-18aH,24 H-18,15-(metheno)pyrido[2,1-l]pyrimido[6,1-h][1,4,7,9,10,13]benzoxapentaazacyclohexadec ine-7,24(6H)-dione;
(18aS)-13-(3-aminoazetidin-1-yl)-2-fluoro-9,9,11-trimethyl-8,9,10,11,19,20,21,22-octahydro -18aH,24H-18,15-(metheno)pyrido[2,1-l]pyrimido[6,1-h][1,4,7,9,10,13]benzoxapentaazacycl ohexadecine-7,24(6H)-dione;
(18aS)-13-(3-aminoazetidin-1-yl)-2-fluoro-6,11-dimethyl-8,9,10,11,19,20,21,22-octahydro-1 8aH,24H-18,15-(metheno)pyrido[2,1-l]pyrimido[6,1-h][1,4,7,9,10,13]benzoxapentaazacyclo hexadecine-7,24(6H)-dione;
(18aS)-13-(3-aminoazetidin-1-yl)-11-cyclopropyl-2-fluoro-8,9,10,11,19,20,21,22-octahydro-18aH,24H-18,15-(metheno)pyrido[2,1-l]pyrimido[6,1-h][1,4,7,9,10, 13]benzoxapentaazacycl ohexadecine-7,24(6H)-dione;
(18aS)-13-(3-aminoazetidin-1-yl)-2-fluoro-11-methyl-9-(trifluoromethyl)-8,9,10,11,19,20,21, 22-octahydro-18aH,24H-18,15-(metheno)pyrido[2,1-l]pyrimido[6,1-h][1,4,7,9,10,13]benzox apentaazacyclohexadecine-7,24(6H)-dione;
(18aS)-13-(cyclopropylamino)-2-fluoro-11-methyl-8,9,10,11,19,20,21,22-octahydro-18aH,24 H-18,15-(metheno)pyrido[2,1-l]pyrimido[6,1-h][1,4,7,9,10,13]benzoxapentaazacyclohexadec ine-7,24(6H)-dione;
(18aS)-13-{[(1-aminocyclopropyl)methyl]amino}-2-fluoro-11-methyl-8,9,10,11,19,20,21,22-octahydro-18aH,24H-18,15-(metheno)pyrido[2,1-l]pyrimido[6,1-h][1,4,7,9,10,13]benzoxape ntaazacyclohexadecine-7,24(6H)-dione;
(18aS)-2-fluoro-13-methoxy-11-methyl-8,9,10,11,19,20,21,22-octahydro-18aH,24H-18,15-( metheno)pyrido[2,1-l]pyrimido[6,1-h][1,4,7,9,10,13]benzoxapentaazacyclohexadecine-7,24( 6H)-dione;
(18aS)-13-cyclobutyl-2-fluoro-11-methyl-8,9,10,11,19,20,21,22-octahydro-18aH,24H-18,15-(metheno)pyrido[2,1-l]pyrimido[6,1-h][1,4,7,9,10,13]benzoxapentaazacyclohexadecine-7,24( 6H)-dione;
(18aS)-13-(1-aminocyclopropyl)-2-fluoro-11-methyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione;
(18aS)-2-fluoro-13-(3-hydroxyprop-1-yn-1-yl)-11-methyl-8,9,10,11,19,20,21,22-octahydro-1 8aH,24H-18,15-(metheno)pyrido[2,1-l]pyrimido[6,1-h][1,4,7,9,10,13]benzoxapentaazacyclo hexadecine-7,24(6H)-dione;
(18aS)-2-fluoro-13-(3-hydroxypropyl)-11-methyl-8,9,10,11,19,20,21,22-octahydro-18aH,24 H-18,15-(metheno)pyrido[2,1-l]pyrimido[6,1-h][1,4,7,9,10,13]benzoxapentaazacyclohexadec ine-7,24(6H)-dione;
(18aS)-2-fluoro-13-[(1E)-3-hydroxyprop-1-en-1-yl]-11-methyl-8,9,10,11,19,20,21,22-octahy dro-18aH,24H-18,15-(metheno)pyrido[2,1-l]pyrimido[6,1-h][1,4,7,9,10,13]benzoxapentaaza cyclohexadecine-7,24(6H)-dione;
(18aS)-13-cyclopropyl-2,25-difluoro-11-methyl-8,9,10,11,19,20,21,22-octahydro-18aH,24H-18,15-(metheno)pyrido[2,1-l]pyrimido[6,1-h][1,4,7,9,10,13]benzoxapentaazacyclohexadecin e-7,24(6H)-dione;
(18aS)-13-(azetidin-1-yl)-2-fluoro-11-methyl-7,8,10,11,19,20,21,22-octahydro-18aH,24H-18 ,15-(metheno)pyrido[2,1-l]pyrimido[6,1-h][1,4,7,9,10, 13]benzoxapentaazacyclohexadecine-9 ,24(6H)-dione;
(18aS)-13-(azetidin-1-yl)-2-chloro-11-methyl-8,9,10,11,19,20,21,22-octahydro-18aH,24H-18 ,15-(metheno)pyrido[2,1-l]pyrimido[6,1-h][1,4,7,9,10, 13]benzoxapentaazacyclohexadecine-7 ,24(6H)-dione;
(18aS)-2-chloro-13-(3-hydroxyazetidin-1-yl)-11-methyl-8,9,10,11,19,20,21,22-octahydro-18 aH,24H-18,15-(metheno)pyrido[2,1-l]pyrimido[6,1-h][1,4,7,9,10,13]benzoxapentaazacycloh exadecine-7,24(6H)-dione;
(18aS)-13-(3-aminoazetidin-1-yl)-2-chloro-11-methyl-8,910,11,19,20,21,22-octahydro-18aH ,24H-18,15-(metheno)pyrido[2,1-l]pynmido[6,1-h][1,4,7,9,10,13]benzoxapentaazacyclohexa decine-7,24(6H)-dione;
(18aS)-13-(azetidin-1-yl)-2-chloro-8,11-dimethyl-8,9,10,11,19,20,21,22-octahydro-18aH,24 H-18,15-(metheno)pyrido[2,1-l]pyrimido[6,1-h][1,4,7,9,10,13]benzoxapentaazacyclohexadec ine-7,24(6H)-dione;
(18aS)-13-(3-hydroxyazetidin-1-yl)-2,11-dimethyl-8,9,10,11,19,20,21,22-octahydro-18aH,24 H-18,15-(metheno)pyrido[2,1-l]pyrimido[6,1-h][1,4,7,9,10,13]benzoxapentaazacyclohexadec ine-7,24(6H)-dione;
(18aS)-13-(3-aminoazetidin-1-yl)-2,11-dimethyl-8,9,10,11,19,20,21,22-octahydro-18aH,24H -18,15-(metheno)pyrido[2,1-l]pyrimido[6,1-h][1,4,7,9,10,13]benzoxapentaazacyclohexadecin e-7,24(6H)-dione;
(18aS)-13-(azetidin-1-yl)-2,11-dimethyl-8,9,10,11,19,20,21,22-octahydro-18aH,24H-18,15-( metheno)pyrido[2,1-l]pyrimido[6,1-h][1,4,7,9,10,13]benzoxapentaazacyclohexadecine-7,24( 6H)-dione;
(18aS)-13-(azetidin-1-yl)-2-ethyl-11-methyl-8,9,10,11,19,20,21,22-octahydro-18aH,24H-18, 15-(metheno)pyrido[2,1-l]pyrimido[6,1-h][1,4,7,9,10,13]benzoxapentaazacyclohexadecine-7, 24(6H)-dione;
(18aS)-13-(2-aminoethoxy)-2-ethyl-11-methyl-8,9,10,11,19,20,21,22-octahydro-18aH,24H-1 8,15-(metheno)pyrido[2,1-l]pyrimido[6,1-h][1,4,7,9,10,13]benzoxapentaazacyclohexadecine-7,24(6H)-dione;
(18aS)-13-(azetidin-1-yl)-2-methoxy-11-methyl-8,9,10,11,19,20,21,22-octahydro-18aH,24H-18,15-(metheno)pyrido[2,1-l]pyrimido[6,1-h][1,4,7,9,10,13]benzoxapentaazacyclohexadecin e-7,24(6H)-dione;
(18aS)-13-(3-hydroxyazetidin-1-yl)-11-methyl-2-trideuteriomethyl-8,9,10,11,19,20,21,22-oct ahydro-18aH,24H-18,15-(metheno)pyrido[2,1-l]pyrimido[6,1-h][1,4,7,9,10,13]benzoxapenta azacyclohexadecine-7,24(6H)-dione;
(19aS)-14-(3-aminoazetidin-1-yl)-2-fluoro-12-methyl-9,10,11,12,20,21,22,23-octahydro-6H, 19aH,25H-19,16-(metheno)pyrido[2,1-m]pyrimido[6,1-i][1,4,8,10,11,14]benzoxapentaazacy cloheptadecine-7,25(8H)-dione;
(18aS)-13-(azetidin-1-yl)-2-fluoro-11,12-dimethyl-8,9,10,11,19,20,21,22-octahydro-18aH,24 H-18,15-(metheno)pyrido[2,1-l]pyrimido[6,1-h][1,4,7,9,10,13]benzoxapentaazacyclohexadec ine-7,24(6H)-dione;
(18aS)-2-fluoro-13-(3-hydroxyazetidin-1-yl)-11,12-dimethyl-8,9,10,11,19,20,21,22-octahydr o-18aH,24H-18,15-(metheno)pyrido[2,1-l]pyrimido[6,1-h][1,4,7,9,10,13]benzoxapentaazacy clohexadecine-7,24(6H)-dione;
(18aS)-13-(3-aminoazetidin-1-yl)-2-fluoro-11,12-dimethyl-8,9,10,11,19,20,21,22-octahydro-18aH,24H-18,15-(metheno)pyrido[2,1-l]pyrimido[6,1-h][1,4,7,9,10,13]benzoxapentaazacycl ohexadecine-7,24(6H)-dione;
(18aS)-13-(2-aminoethoxy)-2-fluoro-11,12-dimethyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione;
(18aS)-13-(azetidin-1-yl)-2-fluoro-8,11,12-trimethyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione;
(18aS)-13-(3-aminoazetidin-1-yl)-2-fluoro-12-methyl-8,9,10,11,19,20,21,22-octahydro-18aH ,24H-18,15-(metheno)pyrido[2,1-l]pynmido[6,1-h][1,4,7,9,10,13]benzoxapentaazacyclohexa decine-7,24(6H)-dione;
(18aS)-13-(3-aminoazetidin-1-yl)-2-chloro-11,12-dimethyl-8,9,10,11,19,20,21,22-octahydro-18aH,24H-18,15-(metheno)pyrido[2,1-l]pyrimido[6,1-h][1,4,7,9,10, 13]benzoxapentaazacycl ohexadecine-7,24(6H)-dione;
(18aS)-2-chloro-13-(3-hydroxyazetidin-1-yl)-11,12-dimethyl-8,9,10,11,19,20,21,22-octahydr o-18aH,24H-18,15-(metheno)pyrido[2,1-l]pyrimido[6,1-h][1,4,7,9,10,13]benzoxapentaazacy clohexadecine-7,24(6H)-dione;
(18aS)-13-[(2-aminoethyl)amino]-2-chloro-11,12-dimethyl-8,9,10,11,19,20,21,22-octahydro-18aH,24H-18,15-(metheno)pyrido[2,1-1]pyrimido[6,1-h][1,4,7,9,10,13]benzoxapentaazacycl ohexadecine-7,24(6H)-dione;
(18aS)-13-[(2-aminoethyl)sulfanyl]-2-chloro-11,12-dimethyl-8,9,10,11,19,20,21,22-octahydr o-18aH,24H-18,15-(metheno)pyrido[2,1-1]pyrimido[6,1-h][1,4,7,9,10,13]benzoxapentaazacy clohexadecine-7,24(6H)-dione;
(18aS)-13-(2-aminoethoxy)-2-chloro-11,12-dimethyl-8,9,10,11,19,20,21,22-octahydro-18aH, 24H-18,15-(metheno)pyrido[2,1-1]pyrimido[6,1-h][1,4,7,9,10,13]benzoxapentaazacyclohexa decine-7,24(6H)-dione;
(18aS)-2-chloro-13-(3-hydroxypropyl)-11,12-dimethyl-8,9,10,11,19,20,21,22-octahydro-18a H,24H-18,15-(metheno)pyrido[2,1-1]pyrimido[6,1-h][1,4,7,9,10,13]benzoxapentaazacyclohe xadecine-7,24(6H)-dione;
(18aS)-13-(3-aminopropyl)-2-chloro-11,12-dimethyl-8,9,10,11,19,20,21,22-octahydro-18aH, 24H-18,15-(metheno)pyrido[2,1-l]pyrimido[6,1-h][1,4,7,9,10,13]benzoxapentaazacyclohexa decine-7,24(6H)-dione;
(18aS)-13-(2-aminoethyl)-2-chloro-11,12-dimethyl-8,9,10,11,19,20,21,22-octahydro-18aH,2 4H-18,15-(metheno)pyrido[2,1-1]pyrimido[6,1-h][1,4,7,9,10,13]benzoxapentaazacyclohexade cine-7,24(6H)-dione;
(18aS)-13-(4-aminobutyl)-2-chloro-11,12-dimethyl-8,9,10,11,19,20,21,22-octahydro-18aH,2 4H-18,15-(metheno)pyrido[2,1-1]pyrimido[6,1-h][1,4,7,9,10,13]benzoxapentaazacyclohexade cine-7,24(6H)-dione;
(18aS)-13-(3-aminoazetidin-1-yl)-2,11,12-trimethyl-8,9,10,11,19,20,21,22-octahydro-18aH,2 4H-18,15-(metheno)pyrido[2,1-1]pyrimido[6,1-h][1,4,7,9,10,13]benzoxapentaazacyclohexade cine-7,24(6H)-dione;
(18aS)-13-(2-aminoethoxy)-2,11,12-trimethyl-8,9,10,11,19,20,21,22-octahydro-18aH,24H-1 8,15-(metheno)pyrido[2,1-1]pyrimido[6,1-h][1,4,7,9,10,13]benzoxapentaazacyclohexadecine-7,24(6H)-dione;
(18aS)-13-(3-aminoazetidin-1-yl)-2,12-difluoro-11-methyl-8,9,10,11,19,20,21,22-octahydro-18aH,24H-18,15-(metheno)pyrido[2,1-1]pyrimido[6,1-h][1,4,7,9,10, 13]benzoxapentaazacycl ohexadecine-7,24(6H)-dione;
(18aS)-2,12-difluoro-13-(3-hydroxyazetidin-1-yl)-11-methyl-8,9,10,11,19,20,21,22-octahydr o-18aH,24H-18,15-(metheno)pyrido[2,1-1]pyrimido[6,1-h][1,4,7,9,10,13]benzoxapentaazacy clohexadecine-7,24(6H)-dione;
(18aS)-13-(3-aminoazetidin-1-yl)-2-chloro-12-fluoro-11-methyl-8,9,10,11,19,20,21,22-octah ydro-18aH,24H-18,15-(metheno)pyrido[2,1-1]pyrimido[6,1-h][1,4,7,9,10,13]benzoxapentaaz acyclohexadecine-7,24(6H)-dione;
(18aS)-13-(3-aminoazetidin-1-yl)-12-chloro-2-fluoro-11-methyl-8,9,10,11,19,20,21,22-octah ydro-18aH,24H-18,15-(metheno)pyrido[2,1-1]pyrimido[6,1-h][1,4,7,9,10,13]benzoxapentaaz acyclohexadecine-7,24(6H)-dione;
(18aS)-13-cyclopropyl-2-fluoro-11-methyl-8,9,10,11,19,20,21,22-octahydro-18aH,24H-18,1 5-(metheno)pyrido[2,1-1]pyrimido[6,1-h][1,4,7,9,10,13]benzoxapentaazacyclohexadecine-7,2 4(6H)-dione;
(18aS)-13-cyclopropyl-11-ethyl-2-fluoro-8,9,10,11,19,20,21,22-octahydro-18aH,24H-18,15-( metheno)pyrido[2,1-1]pyrimido[6,1-h][1,4,7,9,10,13]benzoxapentaazacyclohexadecine-7,24( 6H)-dione;
(18aS)-13-cyclopropyl-2-fluoro-11-trideuteriomethyl-8,9,10,11,19,20,21,22-octahydro-18aH, 24H-18,15-(metheno)pyrido[2,1-1]pyrimido[6,1-h][1,4,7,9,10,13]benzoxapentaazacyclohexa decine-7,24(6H)-dione;
(18aS)-13-cyclopropyl-2-ethenyl-11-methyl-8,9,10,11,19,20,21,22-octahydro-18aH,24H-18, 15-(metheno)pyrido[2,1-1]pyrimido[6,1-h][1,4,7,9,10,13]benzoxapentaazacyclohexadecine-7, 24(6H)-dione;
(18aS)-13-cyclopropyl-2-iodo-11-methyl-8,9,10,11,19,20,21,22-octahydro-18aH,24H-18,15-(metheno)pyrido[2,1-1]pyrimido[6,1-h][1,4,7,9,10,13]benzoxapentaazacyclohexadecine-7,24( 6H)-dione;
(18aS)-13-cyclopropyl-11-methyl-7,24-dioxo-6,7,8,9,10,11,19,20,21,22-decahydro-18aH,24 H-18,15-(metheno)pyrido[2,1-1]pyrimido[6,1-h][1,4,7,9,10,13]benzoxapentaazacyclohexadec ine-2-carbonitrile;
(18aS)-2,13-dicyclopropyl-11-methyl-8,9,10,11,19,20,21,22-octahydro-18aH,24H-18,15-(me theno)pyrido[2,1-1]pyrimido[6,1-h][1,4,7,9,10,13]benzoxapentaazacyclohexadecine-7,24(6H) -dione;
(18aS)-13-cyclopropyl-11-methyl-2-trideuteriomethyl-8,9,10,11,19,20,21,22-octahydro-18a H,24H-18,15-(metheno)pyrido[2,1-1]pyrimido[6,1-h][1,4,7,9,10,13]benzoxapentaazacyclohe xadecine-7,24(6H)-dione;
(18aS)-13-cyclopropyl-2,11-dimethyl-8,9,10,11,19,20,21,22-octahydro-18aH,24H-18,15-(me theno)pyrido[2,1-1]pyrimido[6,1-h][1,4,7,9,10,13]benzoxapentaazacyclohexadecine-7,24(6H) -dione;
(18aS)-2-chloro-13-cyclopropyl-11-methyl-8,9,10,11,19,20,21,22-octahydro-18aH,24H-18,1 5-(metheno)pyrido[2,1-1]pyrimido[6,1-h][1,4,7,9,10,13]benzoxapentaazacyclohexadecine-7,2 4(6H)-dione;
(18S)-3-cyclopropyl-18-ethyl-14-fluoro-5,17-dimethyl-5,6,7,8,17,18-hexahydro-16H-19,1-( metheno)pyrimido[6,1-h][1,4,7,9,10,13]benzoxapentaazacyclohexadecine-9,16(10H)-dione; (18aS)-13-(trans-3-aminocyclobutyl)-2-fluoro-11-methyl-8,9,10,11,19,20,21,22-octahydro-1 8aH,24H-18,15-(metheno)pyrido[2,1-1]pyrimido[6,1-h][1,4,7,9,10,13]benzoxapentaazacyclo hexadecine-7,24(6H)-dione;
(18aS)-13-(cis-3-aminocyclobutyl)-2-fluoro-11-methyl-8,9,10,11,19,20,21,22-octahydro-18a H,24H-18,15-(metheno)pyrido[2,1-1]pyrimido[6,1-h][1,4,7,9,10,13]benzoxapentaazacyclohe xadecine-7,24(6H)-dione;
(18aS)-2-fluoro-13-(cis-3-hydroxycyclobutyl)-11-methyl-8,9,10,11,19,20,21,22-octahydro-1 8aH,24H-18,15-(metheno)pyrido[2,1-1]pyrimido[6,1-h][1,4,7,9,10,13]benzoxapentaazacyclo hexadecine-7,24(6H)-dione;
(18aS)-2-fluoro-13-(trans-3-hydroxycyclobutyl)-11-methyl-8,9,10,11,19,20,21,22-octahydro-18aH,24H-18,15-(metheno)pyrido[2,1-1]pyrimido[6,1-h][1,4,7,9,10, 13]benzoxapentaazacycl ohexadecine-7,24(6H)-dione;
(18aS)-2-chloro-13-(cis-3-hydroxycyclobutyl)-11-methyl-8,9,10,11,19,20,21,22-octahydro-1 8aH,24H-18,15-(metheno)pyrido[2,1-1]pyrimido[6,1-h][1,4,7,9,10,13]benzoxapentaazacyclo hexadecine-7,24(6H)-dione;
(18aS)-2-chloro-13-(trans-3-hydroxycyclobutyl)-11-methyl-8,9,10,11,19,20,21,22-octahydro -18aH,24H-18,15-(metheno)pyrido[2,1-1]pyrimido[6,1-h][1,4,7,9,10,13]benzoxapentaazacycl ohexadecine-7,24(6H)-dione;
(18aS)-2-chloro-13-(cis-3-hydroxycyclobutyl)-11-trideuteriomethyl-8,9,10,11,19,20,21,22-oc tahydro-18aH,24H-18,15-(metheno)pyrido[2,1-1]pyrimido[6,1-h][1,4,7,9,10,13]benzoxapenta azacyclohexadecine-7,24(6H)-dione;
(18aS)-2-chloro-13-(trans-3-hydroxycyclobutyl)-11-trideuteriomethyl-8,9,10,11,19,20,21,22-octahydro-18aH,24H-18,15-(metheno)pyrido[2,1-1]pyrimido[6,1-h][1,4,7,9,10, 13]benzoxape ntaazacyclohexadecine-7,24(6H)-dione;
(18aS)-13-(cis-3-hydroxycyclobutyl)-2,11-dimethyl-8,9,10,11,19,20,21,22-octahydro-18aH,2 4H-18,15-(metheno)pyrido[2,1-1]pyrimido[6,1-h][1,4,7,9,10,13]benzoxapentaazacyclohexade cine-7,24(6H)-dione;
(18aS)-13-(trans-3-hydroxycyclobutyl)-2,11-dimethyl-8,9,10,11,19,20,21,22-octahydro-18aH ,24H-18,15-(metheno)pyrido[2,1-1]pyrimido[6,1-h][1,4,7,9,10,13]benzoxapentaazacyclohexa decine-7,24(6H)-dione;
(18aS)-13-(cis-3-hydroxycyclobutyl)-11-methyl-2-trideuteriomethyl-8,9,10,11,19,20,21,22-o ctahydro-18aH,24H-18,15-(metheno)pyrido[2,1-1]pyrimido[6,1-h][1,4,7,9,10,13]benzoxapent aazacyclohexadecine-7,24(6H)-dione;
(18aS)-13-(trans-3-hydroxycyclobutyl)-11-methyl-2-trideuteriomethyl-8,9,10,11,19,20,21,22 -octahydro-18aH,24H-18,15-(metheno)pyrido[2,1-1]pyrimido[6,1-h][1,4,7,9,10,13]benzoxap entaazacyclohexadecine-7,24(6H)-dione;
(18aS)-13-(cis-3-hydroxycyclobutyl)-2,11-bis[trideuteriomethyl]-8,9,10,11,19,20,21,22-octa hydro-18aH,24H-18,15-(metheno)pyrido[2,1-1]pyrimido[6,1-h][1,4,7,9,10,13]benzoxapentaa zacyclohexadecine-7,24(6H)-dione;
(18aS)-13-(trans-3-hydroxycyclobutyl)-2,11-bis[trideuteriomethyl]-8,9,10,11,19,20,21,22-oct ahydro-18aH,24H-18,15-(metheno)pyrido[2,1-1]pyrimido[6,1-h][1,4,7,9,10,13]benzoxapenta azacyclohexadecine-7,24(6H)-dione;
(18aS)-13 -cyclopropyl-2-fluoro-11-methyl-8,9,10,11,19,20,21,22-octahydro-18aH,24H-18,1 5-(metheno)pyrido[2,1-1]pyrimido[6,1-h][1,4,9,10,13]benzoxatetraazacyclohexadecine-7,24( 6H)-dione;
(18aS)-2-chloro-13-cyclopropyl-11-methyl-8,9,10,11,19,20,21,22-octahydro-18aH,24H-18,1 5-(metheno)pyrido[2,1-1]pyrimido[6,1-h][1,4,9,10,13]benzoxatetraazacyclohexadecine-7,24( 6H)-dione;
(18aS)-2-chloro-13-(3-hydroxyazetidin-1-yl)-11-methyl-8,9,10,11,19,20,21,22-octahydro-18 aH,24H-18,15-(metheno)pyrido[2,1-1]pyrimido[6,1-h][1,4,9,10,13]benzoxatetraazacyclohexa decine-7,24(6H)-dione;
(18aS)-13-cyclopropyl-2,11-dimethyl-8,9,10,11,19,20,21,22-octahydro-18aH,24H-18,15-(me theno)pyrido[2,1-1]pyrimido[6,1-h][1,4,9,10,13]benzoxatetraazacyclohexadecine-7,24(6H)-di one;
(18aS)-13-(3-hydroxyazetidin-1-yl)-2,11-dimethyl-8,9,10,11,19,20,21,22-octahydro-18aH,24 H-18,15-(metheno)pyrido[2,1-1]pyrimido[6,1-h][1,4,9,10,13]benzoxatetraazacyclohexadecin e-7,24(6H)-dione;
(18aS)-13-(3-hydroxyazetidin-1-yl)-11-methyl-2-trideuteriomethyl-8,9,10,11,19,20,21,22-oct ahydro-18aH,24H-18,15-(metheno)pyrido[2,1-1]pyrimido[6,1-h][1,4,9,10,13]benzoxatetraaza cyclohexadecine-7,24(6H)-dione;
(18aS)-13-(azetidin-1-yl)-2-fluoro-11-methyl-6,7,8,9,10,11,19,20,21,22-decahydro-18aH,24 H-18,15-(metheno)pyrido[2,1-1]pyrimido[6,1-h][1,4,7,9,10,13]benzoxapentaazacyclohexadec in-24-one;
(18aS)-13-(azetidin-1-yl)-2-chloro-8,11-dimethyl-6,7,8,9,10,11,19,20,21,22-decahydro-18aH ,24H-18,15-(metheno)pyrido[2,1-1]pyrimido[6,1-h][1,4,7,9,10,13]benzoxapentaazacyclohexa decin-24-one;
(18aS)-13-(azetidin-1-yl)-2,11-dimethyl-6,7,8,9,10,11,19,20,21,22-decahydro-18aH,24H-18, 15-(metheno)pyrido[2,1-1]pyrimido[6,1-h][1,4,7,9,10,13]benzoxapentaazacyclohexadecin-24 -one;
(18aS)-2,11-dimethyl-13-(pyrrolidin-1-yl)-6,7,8,9,10,11,19,20,21,22-decahydro-18aH,24H-1 8,15-(metheno)pyrido[2,1-1]pyrimido[6,1-h][1,4,7,9,10,13]benzoxapentaazacyclohexadecin-2 4-one;
(18aS)-13-(azetidin-1-yl)-2,8,11-trimethyl-6,7,8,9,10,11,19,20,21,22-decahydro-18aH,24H-1 8,15-(metheno)pyrido[2,1-1]pyrimido[6,1-h][1,4,7,9,10,13]benzoxapentaazacyclohexadecin-2 4-one;
(18aS)-13-(azetidin-1-yl)-8-ethyl-2,11-dimethyl-6,7,8,9,10,11,19,20,21,22-decahydro-18aH, 24H-18,15-(metheno)pyrido[2,1-1]pyrimido[6,1-h][1,4,7,9,10,13]benzoxapentaazacyclohexa decin-24-one;
(18aS)-13-(azetidin-1-yl)-8-(2-hydroxyethyl)-2,11-dimethyl-6,7,8,9,10,11,19,20,21,22-decah ydro-18aH,24H-18,15-(metheno)pyrido[2,1-1]pyrimido[6,1-h][1,4,7,9,10,13]benzoxapentaaz acycl ohexadecin-24-one;
(18aS)-13-cyclopropyl-2,11-dimethyl-6H,8H,9H,10H,11H,18aH,19H,20H,21H,22H,24H-spi ro[18,15-(metheno)pyrido[2,1-1]pyrimido[6,1-h][1,4,7,9,10,13]benzoxapentaazacyclohexade cine-7,3'-oxetan]-24-one;
(18aS)-13-(azetidin-1-yl)-2,12-difluoro-11-methyl-6,7,8,9,10,11,19,20,21,22-decahydro-18a H,24H-18,15-(metheno)pyrido[2,1-1]pyrimido[6,1-h][1,4,7,9,10,13]benzoxapentaazacyclohe xadecin-24-one;
(17S)-3-[(3S)-3-aminopyrrolidin-1-yl]-13-chloro-17-ethyl-5,16-dimethyl-6,7,8,9,16,17-hexah ydro-5H,15H-18,1-(metheno)pyrimido[6,1-g][1,6,8,9,12]benzoxatetraazacyclopentadecin-15 -one;
(17S)-3-[(3S)-3-aminopyrrolidin-1-yl]-13-chloro-5,17-diethyl-16-methyl-6,7,8,9,16,17-hexah ydro-5H,15H-18,1-(metheno)pyrimido[6,1-g][1,6,8,9,12]benzoxatetraazacyclopentadecin-15 -one;
(17S)-3-[(3S)-3-aminopyrrolidin-1-yl]-17-ethyl-5,13,16-trimethyl-6,7,8,9,16,17-hexahydro-5 H,15H-18,1-(metheno)pyrimido[6,1-g][1,6,8,9,12]benzoxatetraazacyclopentadecin-15-one; (17S)-3-[(2-aminoethyl)amino]-17-ethyl-5,13,16-trimethyl-6,7,8,9,16,17-hexahydro-5H,15H-18,1-(metheno)pyrimido[6,1-g][1,6,8,9,12]benzoxatetraazacyclopentadecin-15-one;
(17S)-3-(3-aminopropyl)-17-ethyl-5,13,16-trimethyl-6,7,8,9,16,17-hexahydro-5H,15H-18,1-( metheno)pyrimido[6,1-g][1,6,8,9,12]benzoxatetraazacyclopentadecin-15-one;
(17S)-3-[(3S)-3-aminopyrrolidin-1-yl]-17-ethyl-5,13,16-trimethyl-5,6,7,8,9,10,16,17-octahyd ro-15H-18,1-(metheno)pyrimido[6,1-g][1,6,8,9,12]benzopentaazacyclopentadecin-15-one;
(17S)-3-[(3S)-3-aminopyrrolidin-1-yl]-17-ethyl-4,13,16-trimethyl-6,7,8,9,16,17-hexahydro-5 H,15H-18,1-(metheno)pyrimido[6,1-g][1,6,8,9,12]benzoxatetraazacyclopentadecin-15-one;
(17S)-3-[(3S)-3-aminopyrrolidin-1-yl]-17-ethyl-4,5,13,16-tetramethyl-5,6,7,8,9,10,16,17-oct ahydro-15H-18,1-(metheno)pyrimido[6,1-g][1,6,8,9,12]benzopentaazacyclopentadecin-15-on e;
(23aS)-18-(3-aminoazetidin-1-yl)-8,16-dimethyl-1,3,4,13,14,15,16,23a-octahydro-2H,6H,12 H-23,20-(metheno)pyrido[2,1-k]pyrimido[6,1-g][1,6,8,9,12]benzoxatetraazacyclopentadecin-6-one;
(23aS)-18-(3-hydroxyazetidin-1-yl)-8,16-dimethyl-1,3,4,13,14,15,16,23a-octahydro-2H,6H,1 2H-23,20-(metheno)pyrido[2,1-k]pyrimido[6,1-g][1,6,8,9,12]benzoxatetraazacyclopentadeci n-6-one;
(23aS)-18-[(3S)-3-aminopyrrolidin-1-yl]-7-chloro-16-methyl-1,3,4,13,14,15,16,23a-octahydr o-2H,6H,12H-23,20-(metheno)pyrido[2,1-k]pyrimido[6,1-g][1,6,8,9,12]benzoxatetraazacycl opentadecin-6-one;
(23aS)-18-[(3S)-3-aminopyrrolidin-1-yl]-7-fluoro-16-methyl-1,3,4,13,14,15,16,23a-octahydr o-2H,6H,12H-23,20-(metheno)pyrido[2,1-k]pyrimido[6,1-g][1,6,8,9,12]benzoxatetraazacycl opentadecin-6-one;
(23aS)-18-(3-aminopropyl)-8-chloro-16-methyl-1,3,4,13,14,15,16,23a-octahydro-2H,6H,12H -23,20-(metheno)pyrido[2,1-k]pyrimido[6,1-g][1,6,8,9,12]benzoxatetraazacyclopentadecin-6 -one;
(23aS)-18-[(3S)-3-aminopyrrolidin-1-yl]-8-fluoro-16-methyl-1,3,4,13,14,15,16,23a-octahydr o-2H,6H,12H-23,20-(metheno)pyrido[2,1-k]pyrimido[6,1-g][1,6,8,9,12]benzoxatetraazacycl opentadecin-6-one;
(23aS)-18-(3-aminoazetidin-1-yl)-8-fluoro-16-methyl-1,3,4,13,14,15,16,23a-octahydro-2H,6 H,12H-23,20-(metheno)pyrido[2,1-k]pyrimido[6,1-g][1,6,8,9,12]benzoxatetraazacyclopentad ecin-6-one;
(23aS)-18-[(3S)-3-aminopyrrolidin-1-yl]-9-fluoro-16-methyl-1,3,4,13,14,15,16,23a-octahydr o-2H,6H,12H-23,20-(metheno)pyrido[2,1-k]pyrimido[6,1-g][1,6,8,9,12]benzoxatetraazacycl opentadecin-6-one;
(23aS)-18-[(3S)-3-aminopyrrolidin-1-yl]-16-methyl-1,3,4,13,14,15,16,23a-octahydro-2H,6H, 12H-23,20-(metheno)pyrido[2,1-k]pyrimido[6,1-g][1,6,8,9,12]benzoxatetraazacyclopentadec in-6-one;
(23aS)-18-(azetidin-1-yl)-8-chloro-15-methyl-1,3,4,13,14,15,16,23a-octahydro-2H,6H,12H-2 3,20-(metheno)pyrido[2,1-k]pyrimido[6,1-g][1,5,8,9,12]benzoxatetraazacyclopentadecin-6-o ne;
(17S)-3-[(3S)-3-aminopyrrolidin-1-yl]-17-ethyl-4,13,16-trimethyl-7,8,16,17-tetrahydro-5H-1 8,1-(metheno)-9λ⁶-pyrimido[6,1-g][2,1,6,8,9,12]benzothiapentaazacyclopentadecine-9,9,15( 6H,10H)-trione;
(17S)-3-[(2-aminoethyl)amino]-17-ethyl-4,13,16-trimethyl-7,8,16,17-tetrahydro-5H-18,1-(m etheno)-9λ⁶-pyrimido[6,1-g][2,1,6,8,9,12]benzothiapentaazacyclopentadecine-9,9,15(6H,10 H)-trione;
(23aS)-18-[(2-aminoethyl)amino]-8-chloro-16,17-dimethyl-1,3,4,13,14,15,16,23a-octahydro-2H,6H-23,20-(metheno)-12λ⁶-pyrido[2,1-k]pyrimido[6,1-g][2,1,6,8,9,12]benzothiapentaazac yclopentadecine-6,12,12(11H)-trione;
(20aS)-15-(azetidin-1-yl)-2-chloro-9,13-dimethyl-12,13,21,22,23,24-hexahydro-6H,11H,20a H,26H-20,17-(metheno)pyrido[2,1-1]pyrimido[6,1-h][1,2,4]triazolo[3,4-c][1,4,7,9,10,13]benz oxapentaazacyclohexadecin-26-one;
(20aS)-15-(azetidin-1-yl)-2-chloro-13-methyl-12,13,21,22,23,24-hexahydro-6H,11H,20aH,2 6H-20,17-(metheno)imidazo[2,1-c]pyrido[2,1-1]pyrimido[6,1-h][1,4,7,9,10,13]benzoxapenta azacyclohexadecin-26-one;
(18aS)-13-(3-aminoazetidin-1-yl)-2-chloro-11-methyl-7-(trifluoromethyl)-6,7,8,9,10,11,19,2 0,21,22-decahydro-18aH,24H-18,15-(metheno)pyrido[2,1-1]pyrimido[6,1-h][1,4,7,9,10,13]be nzoxapentaazacyclohexadecin-24-one;
(18aS)-2-chloro-13-(3-hydroxyazetidin-1-yl)-11-methyl-7-(trifluoromethyl)-6,7,8,9,10,11,19, 20,21,22-decahydro-18aH,24H-18,15-(metheno)pyrido[2,1-1]pyrimido[6,1-h][1,4,7,9,10,13]b enzoxapentaazacyclohexadecin-24-one;
(18aS)-13-(3-hydroxyazetidin-1-yl)-2,11-dimethyl-7-(trifluoromethyl)-6,7,8,9,10,11,19,20,21 ,22-decahydro-18aH,24H-18,15-(metheno)pyrido[2,1-1]pyrimido[6,1-h][1,4,7,9,10,13]benzo xapentaazacyclohexadecin-24-one;
(18aS)-13-[(3 S)-3-aminopyrrolidin-1-yl]-2,11-dimethyl-7-(trifluoromethyl)-6,7,8,9,10,11,19, 20,21,22-decahydro-18aH,24H-18,15-(metheno)pyrido[2,1-1]pyrimido[6,1-h][1,4,7,9,10,13]b enzoxapentaazacyclohexadecin-24-one;
(18aS)-13-(3-hydroxyazetidin-1-yl)-2,11-dimethyl-9-(trifluoromethyl)-6,7,8,9,10,11,19,20,21 ,22-decahydro-18aH,24H-18,15-(metheno)pyrido[2,1-1]pyrimido[6,1-h][1,4,7,9,10,13]benzo xapentaazacyclohexadecin-24-one;
(18aS)-3-(azetidin-1-yl)-5,12-dimethyl-12-phenyl-6,7,8,9,11,12,16,17,18,18a-decahydro-5H, 15H-19,1-(metheno)-10λ⁶-pyrido[1,2-e]pyrimido[1,6-i][1,2,5,8,9,11]thiapentaazacyclopentad ecine-10,10,13-trione;
(18aS)-3-[(3S)-3-aminopyrrolidin-1-yl5,12-dimethyl-12-phenyl-6,7,8,9,11,12,16,17,18,18a-decahydro-5H,15H-19,1-(metheno)-10λ⁶-pyrido[1,2-e]pyrimido[1,6-i][1,2,5,8,9,11]thiapenta azacyclopentadecine-10,10,13-trione; and
(18a'S)-3'-[(3S)-3-aminopyrrolidin-1-yl]-5'-methyl-2H,3H,5'H,6'H,7'H,8'H,9'H,10'H,11'H,13' H,15'H,16'H,17'H,18'H,18a'H-spiro[indene-1,12'-[19,1](metheno)[10λ⁶]pyrido[1,2-e]pyrimid o[1,6-i][1,2,5,8,9,11]thiapentaazacyclopentadecine]-10',10',13'-trione,
or a pharmaceutically acceptable salt thereof.

16. A medicament comprising the compound according to any one of claims 1 to 15 or a pharmaceutically acceptable salt thereof as an active ingredient.

17. A drug for preventing or treating an infection in which viruses of the subfamily Pneumovirinae, including respiratory syncytial virus (RSV), are involved, wherein the drug comprises the compound according to any one of claims 1 to 15 or a pharmaceutically acceptable salt thereof as an active ingredient.
